(19) Europäisches Patentamt
European Patent Office
Office européen des brevets

(11) **EP 4 745 137 A1**

(12) **EUROPEAN PATENT APPLICATION**
published in accordance with Art. 153(4) EPC

(43) Date of publication:
**20.05.2026 Bulletin 2026/21**

(21) Application number: **24842349.3**

(22) Date of filing: **15.07.2024**

(51) International Patent Classification (IPC):
***C07D 409/14*** (2006.01) ***C07D 401/04*** (2006.01)
***C07D 401/14*** (2006.01) ***C07D 405/14*** (2006.01)
***C07D 413/14*** (2006.01) ***C07D 417/14*** (2006.01)
***A61K 31/4545*** (2006.01) ***A61K 31/496*** (2006.01)
***A61K 31/517*** (2006.01) ***A61P 35/00*** (2006.01)

(52) Cooperative Patent Classification (CPC):
**A61K 31/4545; A61K 31/496; A61K 31/517; A61P 35/00; C07D 401/04; C07D 401/14; C07D 405/14; C07D 409/14; C07D 413/14; C07D 417/14**

(86) International application number:
**PCT/CN2024/105487**

(87) International publication number:
**WO 2025/016359 (23.01.2025 Gazette 2025/04)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **14.07.2023 CN 202310864822**

(71) Applicant: **Gluetacs Therapeutics (Shanghai) Co., Ltd.**
**Shanghai 201306 (CN)**

(72) Inventors:
- **YANG, Xiaobao**
  **Shanghai 201306 (CN)**
- **SUN, Renhong**
  **Shanghai 201306 (CN)**
- **REN, Chaowei**
  **Shanghai 201306 (CN)**
- **ZHAO, Baoyin**
  **Shanghai 201306 (CN)**
- **ZHOU, Yuedong**
  **Shanghai 201306 (CN)**
- **YANG, Sen**
  **Shanghai 201306 (CN)**
- **DENG, Meigui**
  **Shanghai 201306 (CN)**
- **REN, Yinbo**
  **Shanghai 201306 (CN)**

(74) Representative: **August Debouzy**
**7, rue de Téhéran**
**75008 Paris (FR)**

# (54) GLUTARIMIDE ISOINDOLINONE SKELETON-BASED COMPOUND

(57) The present disclosure provides a compound of Formula (I) based on a glutarimide-isoindolinone scaffold, or a salt, enantiomer, stereoisomer, solvate, isotopically enriched analog, prodrug, or polymorph thereof, and its use in the treatment and prevention of cereblon protein-related diseases or disorders, including tumors or cancers.

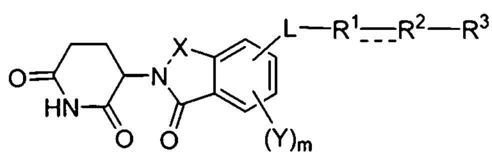

(I)



Processed by Luminess, 75001 PARIS (FR)

(Cont. next page)

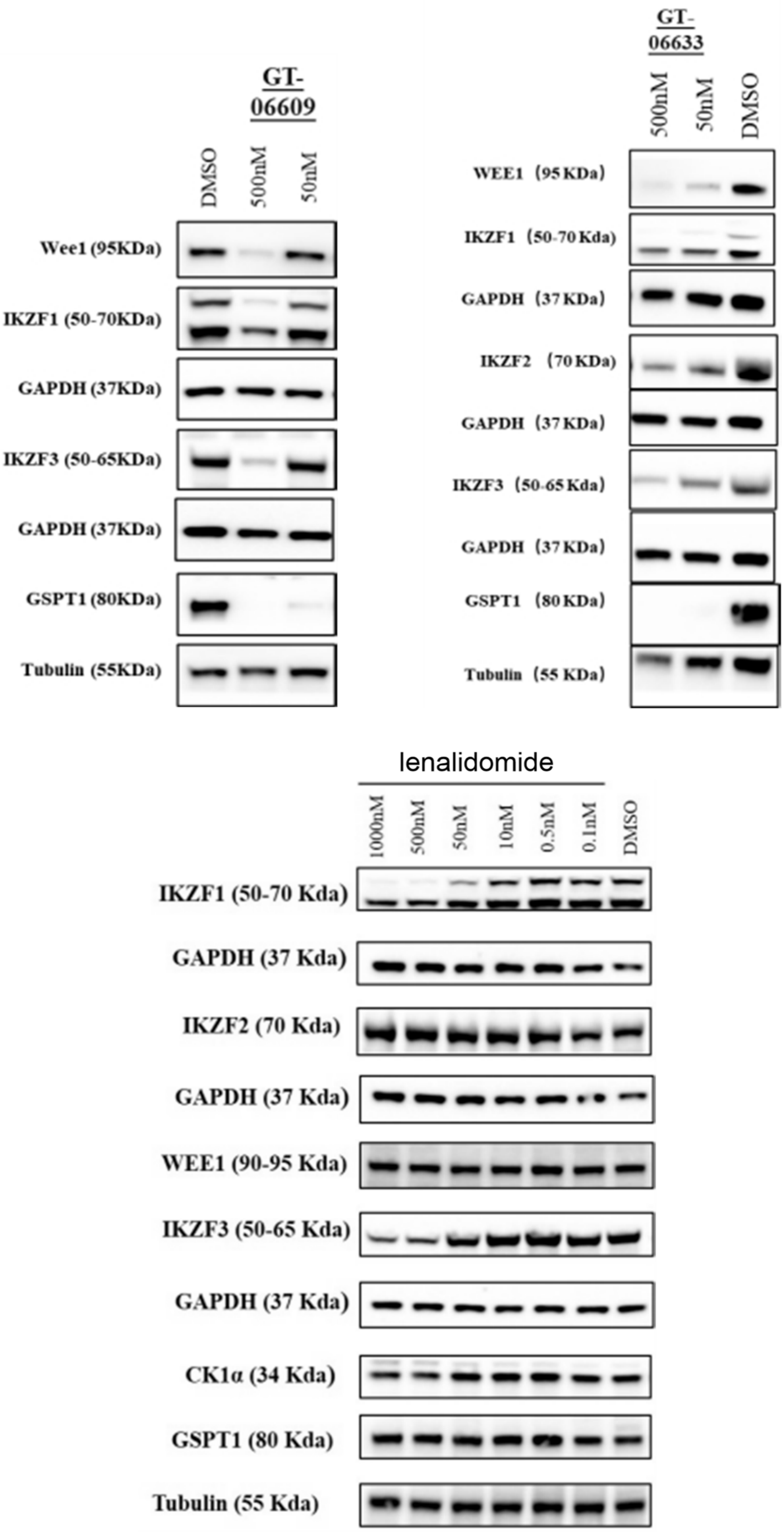
GT-06609
DMSO
500nM
50nM
Wee1 (95KDa)
IKZF1 (50-70KDa)
GAPDH (37KDa)
IKZF3 (50-65KDa)
GAPDH (37KDa)
GSPT1 (80KDa)
Tubulin (55KDa)
GT-06633
500nM
50nM
DMSO
WEE1 (95KDa)
IKZF1 (50-70Kda)
GAPDH (37KDa)
IKZF2 (70KDa)
GAPDH (37KDa)
IKZF3 (50-65Kda)
GAPDH (37KDa)
GSPT1 (80KDa)
Tubulin (55 KDa)
lenalidomide
1000nM
500nM
50nM
10nM
0.5nM
0.1nM
DMSO
IKZF1 (50-70 Kda)
GAPDH (37 Kda)
IKZF2 (70 Kda)
GAPDH (37 Kda)
WEE1 (90-95 Kda)
IKZF3 (50-65 Kda)
GAPDH (37 Kda)
CK1α (34 Kda)
GSPT1 (80 Kda)
Tubulin (55 Kda)

Figure 1

## Description

### Technical Field

The present disclosure provides a compound of Formula (I), or a salt, enantiomer, stereoisomer, solvate, isotopically enriched analog, prodrug, or polymorph thereof, and its use in the treatment of cereblon protein-related diseases or disorders, including tumors or cancers.

### Background

Immunomodulatory drugs (IMiDs) of the phthalimide class, such as thalidomide and lenalidomide, have shown remarkable efficacy in treating hematological malignancies and autoimmune diseases. However, it was not until 2010 that their direct binding target was identified as the E3 ubiquitin ligase cereblon (CRBN). Subsequent studies confirmed that upon binding to CRBN, these drugs act as molecular glues, recruiting substrate proteins (e.g., the transcription factors IKZF1/3) and inducing protein-protein interactions between CRBN and the substrates. This leads to the ubiquitination of the substrate proteins, which are then recognized and degraded by the proteasome, thereby eliciting pharmacological effects such as antitumor and immunomodulatory activities. Molecular glue degraders directly target and degrade specific proteins, offering potential advantages such as targeting "undruggable" proteins. Moreover, molecular glues typically exhibit low molecular weight and favorable drug-like properties, making their development highly challenging. Based on the CRBN E3 ubiquitin ligase and the molecular glue degradation mechanism, a series of compounds have been developed, including the approved pomalidomide and several candidates currently in clinical trials, such as Bristol Myers Squibb's CC-122, CC-220, CC-90009, CC-99282, and CC-92480; Novartis's DKY709; and C4 Therapeutics's CFT7455. The range of substrates degraded by these molecular glues has expanded from the initially identified transcription factors IKZF1/3 to include casein kinase 1$\alpha$ (CK1$\alpha$), zinc finger protein 91 (ZFP91), and the translation factor GSPT1. Degradation of these protein substrates enables molecular glues to exert immunomodulatory, anti-inflammatory, and antitumor activities. Currently, the structural novelty of designed molecular glue candidates remains quite limited, making the diversification of molecular scaffolds and the development of novel molecular glues a key research focus in the field.

Therefore, there is an urgent need for a series of novel CRBN E3 ubiquitin ligase ligand scaffolds to be applied in the development of effective molecular glue degraders for the treatment and/or prevention of diseases or disorders mediated by or associated with degradable proteins.

### Description of Invention

In view of the deficiencies in the prior art, one of the objectives of the present invention is to provide a series of novel compounds based on a glutarimide-isoindolinone scaffold, or salts, enantiomers, stereoisomers, solvates, isotopically enriched analogs, prodrugs, or polymorphs thereof. These compounds are effective in treating diseases including tumors and autoimmune diseases, thereby addressing unmet clinical needs.

In some embodiments, the present disclosure provides a compound of Formula (I):

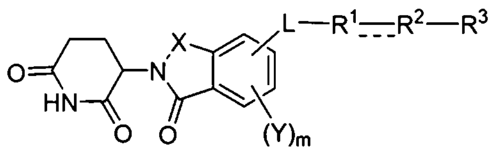

(I)

or salts, enantiomers, stereoisomers, solvates, isotopically enriched analogs, prodrugs, or polymorphs thereof, wherein represents a single or double bond;

X represents $CH_2$ or C(=O);

$(Y)_m$ indicates that benzene ring is optionally substituted with m Y groups, wherein Y represents deuterium, $C_1$-$C_4$ alkyl, halogen or halogenated $C_1$-$C_2$ alkyl, and m represents an integer of 0, 1, 2 or 3;

L represents methylene or linear or branched $C_2$-$C_6$ alkylene, wherein the methylene or linear or branched $C_2$-$C_6$ alkylene is optionally substituted with one or more substituents selected from the group consisting of: deuterium, halogen, $C_1$-$C_3$ alkyl, $C_1$-$C_3$ alkoxy, and halogenated $C_1$-$C_3$ alkyl;

$R^1$ represents a bond, O, $N(R^a)$, $N(R^a)S(O)_2$, $S(O)_2N(R^a)$, nitrogen-containing heterocyclylene or nitrogen-containing heterocyclylylidene, wherein $R^a$ represents hydrogen, deuterium or $C_1$-$C_6$ alkyl, and the nitrogen-containing heterocyclylene or nitrogen-containing heterocyclylylidene is optionally substituted with one or more substituents selected

from the group consisting of: deuterium, hydroxy, amino, mercapto, =O, halogen, cyano, $C_1$-$C_6$ alkyl, halogenated $C_1$-$C_6$ alkyl, $C_1$-$C_6$ alkoxy, and halogenated $C_1$-$C_6$ alkoxy;

$R^2$ represents heteroarylylidene, heteroarylene, heterocyclylene, $(CH_2)_n$-arylene or $(CH_2)_n$-cycloalkylene, wherein n represents an integer of 0, 1 or 2, and the heteroarylylidene, heteroarylene, heterocyclylene, arylene or cycloalkylene is optionally substituted with one or more substituents selected from the group consisting of: deuterium, hydroxy, amino, mercapto, =O, halogen, cyano, $C_1$-$C_6$ alkyl, halogenated $C_1$-$C_6$ alkyl, $C_1$-$C_6$ alkoxy, and halogenated $C_1$-$C_6$ alkoxy;

$R^3$ represents hydrogen, deuterium, $SR^b$, $R^b$, halogenated $C_1$-$C_6$ alkyl, cycloalkyl or nitrogen-containing heterocyclyl, wherein $R^b$ represents aryl, and the aryl, cycloalkyl or nitrogen-containing heterocyclyl is optionally substituted with one or more substituents selected from the group consisting of: deuterium, hydroxy, amino, mercapto, =O, halogen, cyano, $C_1$-$C_6$ alkyl, halogenated $C_1$-$C_6$ alkyl, $C_1$-$C_6$ alkoxy, and halogenated $C_1$-$C_6$ alkoxy;

wherein when ---- represents a double bond, $R^1$ represents nitrogen-containing heterocyclylylidene, and $R^2$ represents heteroarylylidene;

when ---- represents a single bond, $R^1$ represents a bond, O, $N(R^a)$, $N(R^a)S(O)_2$, $S(O)_2N(R^a)$ or nitrogen-containing heterocyclylene, and $R^2$ represents heteroarylene, heterocyclylene, $(CH_2)_n$-arylene or $(CH_2)_n$-cycloalkylene.

In some embodiments, there is provided the compound of Formula (I) or salts, enantiomers, stereoisomers, solvates, isotopically enriched analogs, prodrugs, or polymorphs thereof, wherein examples of L representing methylene or linear or branched $C_2$-$C_6$ alkylene include, but are not limited to, $-CH_2-$, $-(CH_2)_2-$, $-(CH_2)_3-$, $-(CH_2)_4-$, $-(CH_2)_5-$ or $-(CH_2)_6-$, and the methylene or linear or branched $C_2$-$C_6$ alkylene is optionally substituted with one or more substituents selected from the group consisting of: deuterium, halogen (e.g., fluorine, chlorine, bromine, or iodine), $C_1$-$C_3$ alkyl (e.g., methyl, ethyl, or propyl), $C_1$-$C_3$ alkoxy (e.g., methoxy, ethoxy, or propoxy), and halogenated $C_1$-$C_3$ alkyl (e.g., $F_3C$-, $FCH_2$-, $F_2CH$-, $ClCH_2$-, $Cl_2CH$-, $CF_3CF_2$-, $CF_3CHF$-, $CHF_2CF_2$-, $CHF_2CHF$-, $CF_3CH_2$-, and $CH_2ClCH_2$-).

In some embodiments, there is provided the compound of Formula (I) or salts, enantiomers, stereoisomers, solvates, isotopically enriched analogs, prodrugs, or polymorphs thereof, which is also represented by a structure of the following formula:

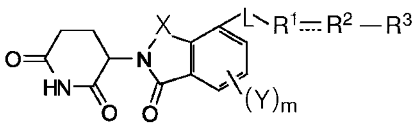

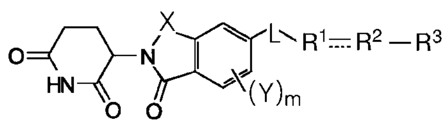

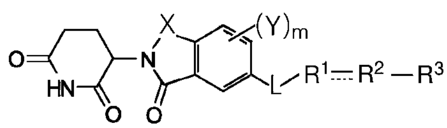

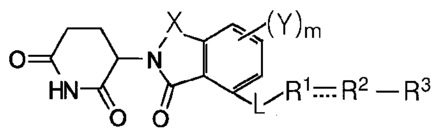

(I-c)

wherein ----, X, Y, L, m, $R^1$, $R^2$, and $R^3$ are as defined above.

In some embodiments, there is provided the compound of Formula (I) or salts, enantiomers, stereoisomers, solvates, isotopically enriched analogs, prodrugs, or polymorphs thereof, which is also represented by a structure of the following formula:

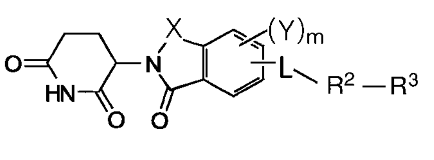

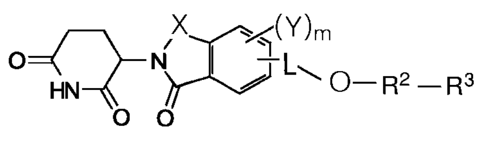

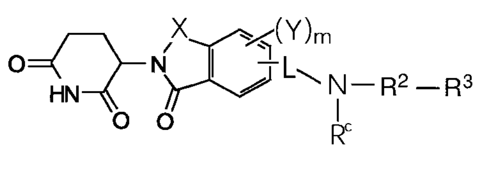

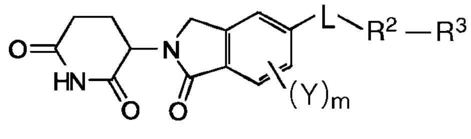

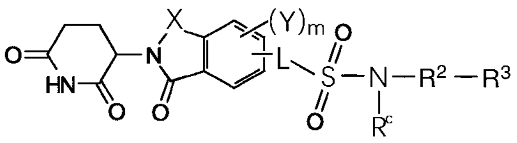 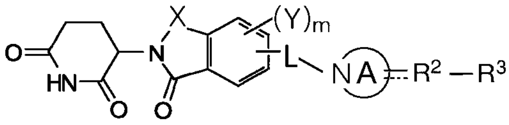

wherein , X, Y, L, m, $R^2$, and $R^3$ are as defined above;

$R^c$ represents deuterium, hydrogen or $C_1$-$C_6$ alkyl;

ring A represents nitrogen-containing heterocyclylene or nitrogen-containing heterocyclylylidene, wherein the nitrogen-containing heterocyclylene or nitrogen-containing heterocyclylylidene is optionally substituted with one or more substituents selected from the group consisting of: deuterium, hydroxy, amino, mercapto, =O, halogen, cyano, $C_1$-$C_6$ alkyl, halogenated $C_1$-$C_6$ alkyl, $C_1$-$C_6$ alkoxy, and halogenated $C_1$-$C_6$ alkoxy.

In some embodiments, there is provided the compound of Formula (I) or salts, enantiomers, stereoisomers, solvates, isotopically enriched analogs, prodrugs, or polymorphs thereof, which is also represented by a structure of the following formula:

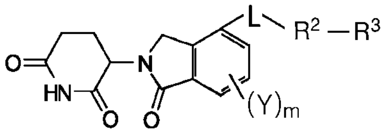 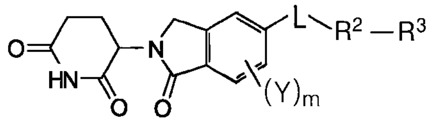

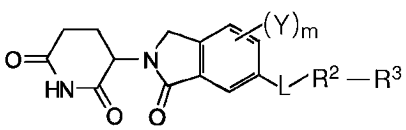 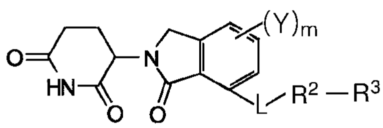

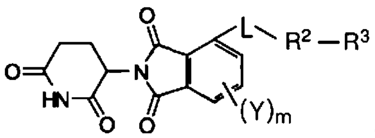 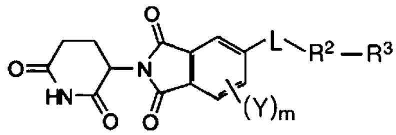

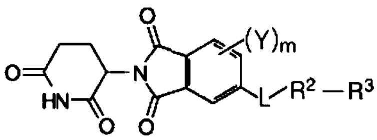 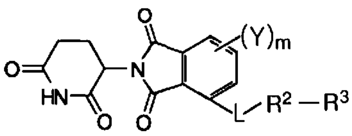

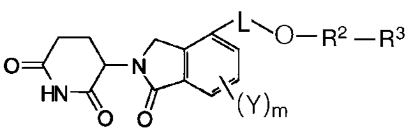 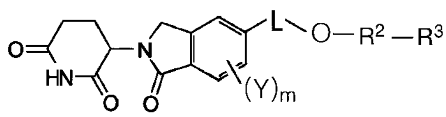

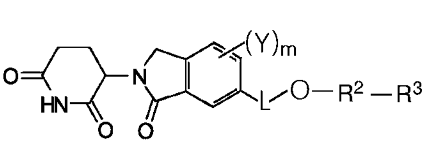 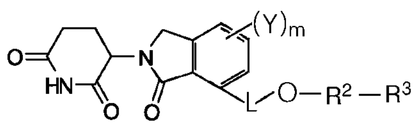

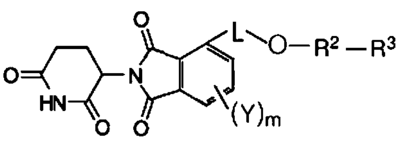 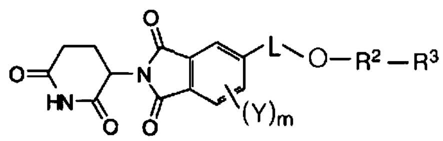

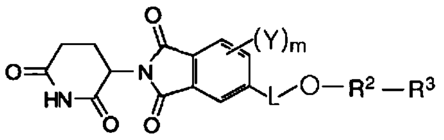

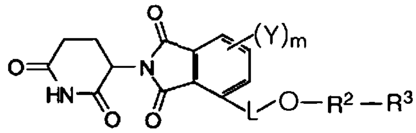

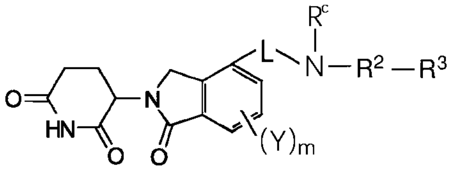

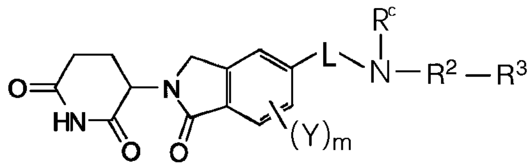

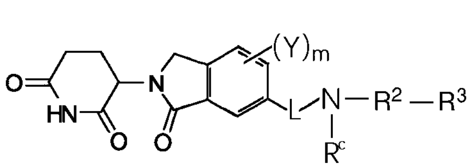

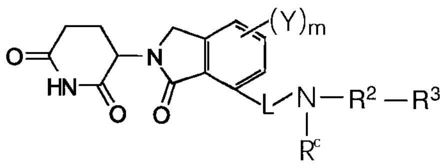

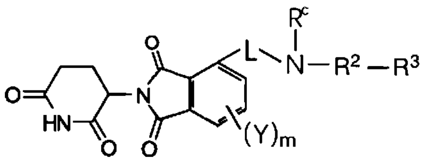

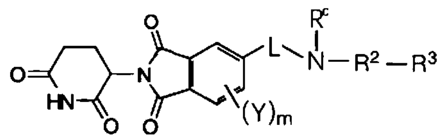

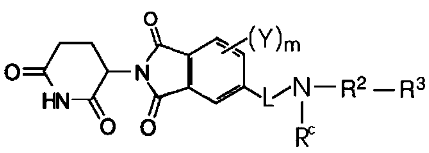

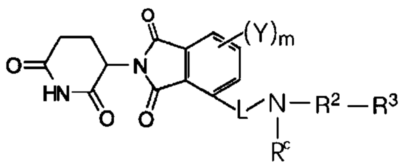

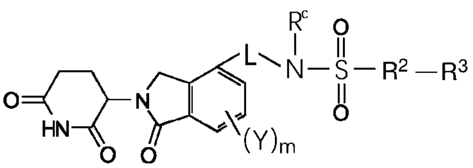

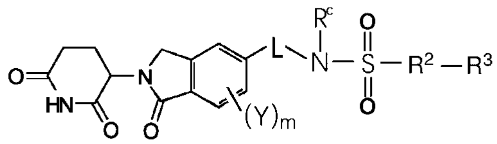

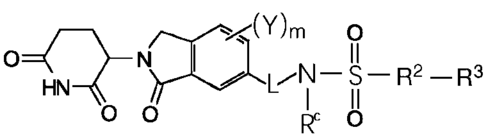

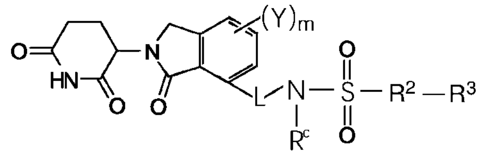

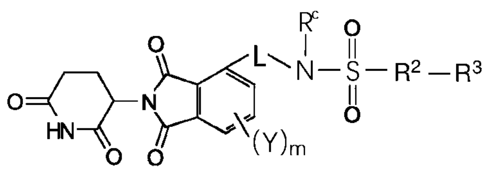

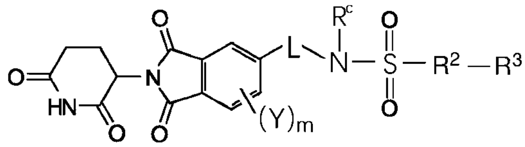

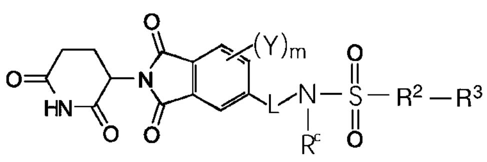

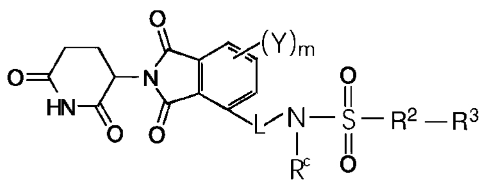

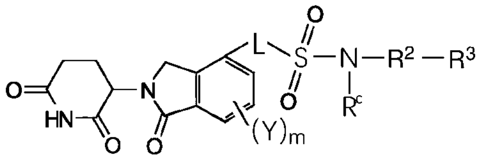

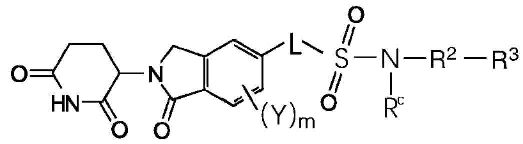

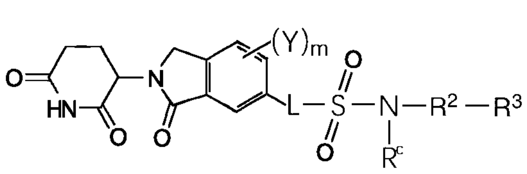

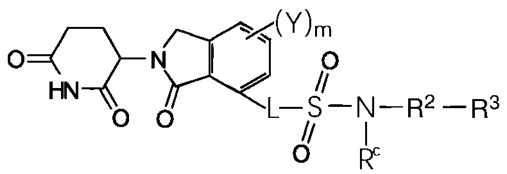

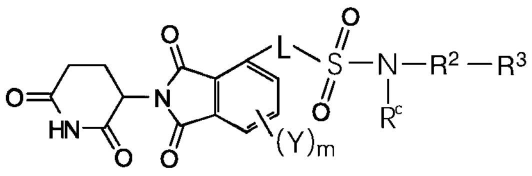

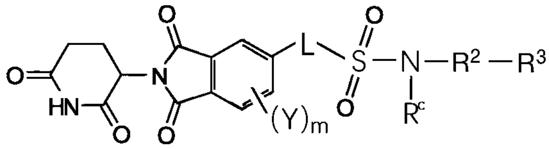

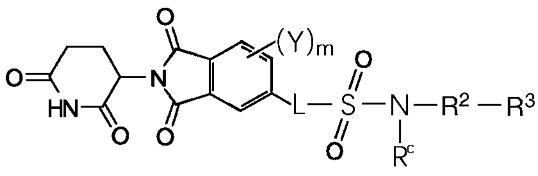

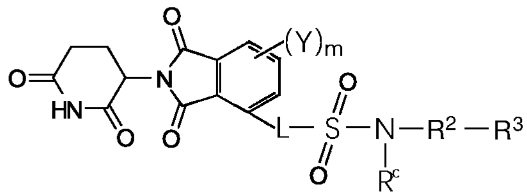

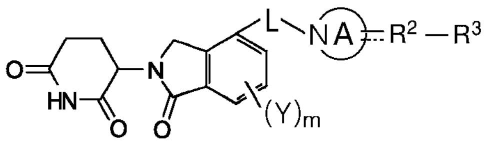

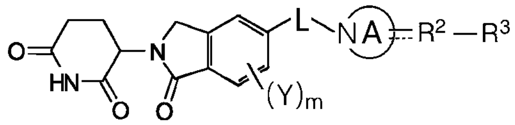

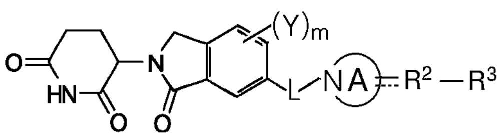

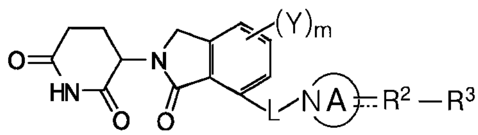

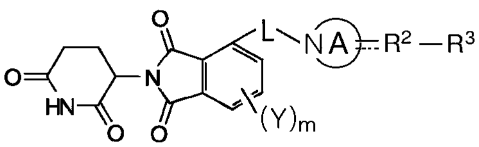

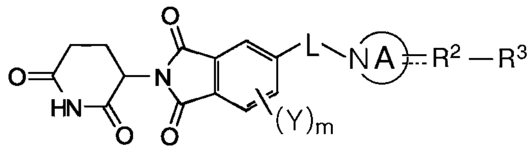

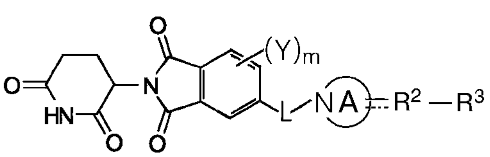

or

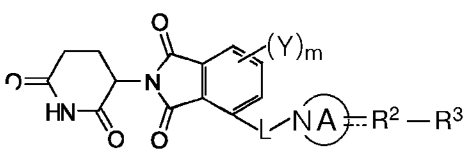

;

wherein , Y, L, m, $R^2$, and $R^3$ are as defined above;

$R^c$ represents deuterium, hydrogen or $C_1$-$C_6$ alkyl;

ring A represents a nitrogen-containing heterocyclylene or a nitrogen-containing heterocyclylylidene, wherein the nitrogen-containing heterocyclylene or nitrogen-containing heterocyclylylidene is optionally substituted with one or more substituents selected from the group consisting of: deuterium, hydroxy, amino, mercapto, =O, halogen, cyano, $C_1$-$C_6$ alkyl, halogenated $C_1$-$C_6$ alkyl, $C_1$-$C_6$ alkoxy, and halogenated $C_1$-$C_6$ alkoxy.

In some embodiments, there is provided the compound of Formula (I) or salts, enantiomers, stereoisomers, solvates, isotopically enriched analogs, prodrugs, or polymorphs thereof, wherein Y represents D, F, Cl, Br, I, $CF_3$, $CH_2F$, $CHF_2$, $CH_2Cl$, $CHCl_2$, $CF_2CF_3$, $CHFCF_3$, $CF_2CHF_2$, $CHFCHF_2$, $CH_2CF_3$ or $CH_2CH_2Cl$.

In some embodiments, there is provided the compound of Formula (I) or salts, enantiomers, stereoisomers, solvates, isotopically enriched analogs, prodrugs, or polymorphs thereof, wherein $R^1$ is ring A, which represents a nitrogen-containing heterocyclylene or a nitrogen-containing heterocyclylylidene, wherein the nitrogen-containing heterocyclylene or nitrogen-containing heterocyclylylidene is optionally substituted with one or more substituents selected from the group consisting of: deuterium, hydroxy, amino, mercapto, =O, halogen, cyano, $C_1$-$C_6$ alkyl, halogenated $C_1$-$C_6$ alkyl, $C_1$-$C_6$ alkoxy, and halogenated $C_1$-$C_6$ alkoxy.

In some embodiments, there is provided the compound of Formula (I) or salts, enantiomers, stereoisomers, solvates, isotopically enriched analogs, prodrugs, or polymorphs thereof, wherein examples of ring A representing the nitrogen-containing heterocyclylene include, but are not limited to, 4- to 30-membered nitrogen-containing heterocyclylene, 4- to 20-membered nitrogen-containing heterocyclylene, 4- to 15-membered nitrogen-containing heterocyclylene, 5- to 30-membered nitrogen-containing heterocyclylene, 5 to 20-membered nitrogen-containing heterocyclylene or 5- to 15-membered nitrogen-containing heterocyclylene, wherein the nitrogen-containing heterocyclylene is optionally substituted with one or more substituents selected from the group consisting of: deuterium, hydroxy, amino, mercapto, = O, halogen, cyano, $C_1$-$C_6$ alkyl, halogenated $C_1$-$C_6$ alkyl, $C_1$-$C_6$ alkoxy, and halogenated $C_1$-$C_6$ alkoxy.

In some embodiments, there is provided the compound of Formula (I) or salts, enantiomers, stereoisomers, solvates, isotopically enriched analogs, prodrugs, or polymorphs thereof, wherein examples of ring A representing the nitrogen-containing heterocyclylene include, but are not limited to, piperazinylene, piperidinylene, pyrrolidinylene, imidazolidinylene, pyrazolidinylene, oxazolidinylene, thiazolidinylene, morpholinylene, thiomorpholinylene, diazacycloheptanylene, diazabicyclo[3.1.1]heptanylene, diazabicyclo[2.2.1]heptanylene, diazabicyclo[2.2.2]octanylene, diazabicyclo[3.2.1]octanylene, or azaspiro-heterocyclylene (e.g., 5- to 30-membered, 5- to 20-membered, 5- to 15-membered, 6- to 20-membered, 6- to 15-membered, 7- to 30-membered, 7- to 20-membered or 7-to 15-membered azaspiro-heterocyclylene), wherein the nitrogen-containing heterocyclylene is optionally substituted with one or more substituents selected from the group consisting of: deuterium, hydroxy, amino, mercapto, =O, halogen, cyano, $C_1$-$C_6$ alkyl, halogenated $C_1$-$C_6$ alkyl, $C_1$-$C_6$ alkoxy, and halogenated $C_1$-$C_6$ alkoxy.

In some embodiments, there is provided the compound of Formula (I) or salts, enantiomers, stereoisomers, solvates, isotopically enriched analogs, prodrugs, or polymorphs thereof, wherein examples of ring A representing the nitrogen-containing heterocyclylylidene include, but are not limited to, 4- to 30-membered nitrogen-containing heterocyclylylidene, 4- to 20-membered nitrogen-containing heterocyclylylidene, 4- to 15-membered nitrogen-containing heterocyclylylidene, 5- to 30-membered nitrogen-containing heterocyclylylidene, 5 to 20-membered nitrogen-containing heterocyclylylidene or 5-to 15-membered nitrogen-containing heterocyclylylidene, wherein the nitrogen-containing heterocyclylylidene is optionally substituted with one or more substituents selected from the group consisting of: deuterium, hydroxy, amino, mercapto, =O, halogen, cyano, $C_1$-$C_6$ alkyl, halogenated $C_1$-$C_6$ alkyl, $C_1$-$C_6$ alkoxy, and halogenated $C_1$-$C_6$ alkoxy.

In some embodiments, there is provided the compound of Formula (I) or salts, enantiomers, stereoisomers, solvates, isotopically enriched analogs, prodrugs, or polymorphs thereof, wherein examples of ring A representing the nitrogen-containing heterocyclylylidene include, but are not limited to, piperazinylylidene, piperidinylylidene, pyrrolidinylylidene, imidazolidinylylidene, pyrazolidinylylidene, oxazolidinylylidene, thiazolidinylylidene, morpholinylylidene, thiomorpholinylylidene, diazacycloheptanylylidene, diazabicyclo[3.1.1]heptanylylidene, diazabicyclo[2.2.1]heptanylylidene, diazabicyclo[2.2.2]octanylylidene, diazabicyclo[3.2.1]octanylylidene or azaspiro-heterocyclylylidene, wherein the nitrogen-containing heterocyclylylidene is optionally substituted with one or more substituents selected from the group consisting of: deuterium, hydroxy, amino, mercapto, =O, halogen, cyano, $C_1$-$C_6$ alkyl, halogenated $C_1$-$C_6$ alkyl, $C_1$-$C_6$ alkoxy, and halogenated $C_1$-$C_6$ alkoxy.

In some embodiments, there is provided the compound of Formula (I) or salts, enantiomers, stereoisomers, solvates, isotopically enriched analogs, prodrugs, or polymorphs thereof, wherein examples of $R^1$ or the ring A include, but are not limited to,

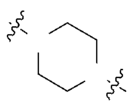 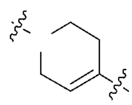 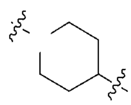 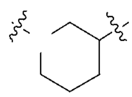 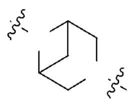 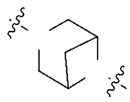

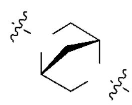 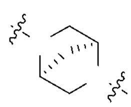 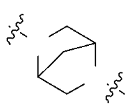 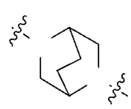 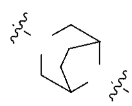 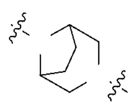

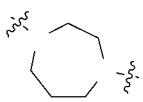 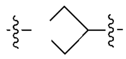 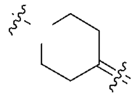 or 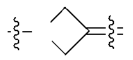 ;

wherein symbol * indicates the point of attachment to L.

In some embodiments, there is provided the compound of Formula (I) or salts, enantiomers, stereoisomers, solvates, isotopically enriched analogs, prodrugs, or polymorphs thereof, wherein $R^2$ represents heteroarylylidene, heteroarylene, heterocyclylene, arylene, cycloalkylene, $-CH_2$-arylene, $-CH_2$-cycloalkylene, $-(CH_2)_2$-arylene or $-(CH_2)_2$-cycloalkylene, wherein the heteroarylylidene, heteroarylene, heterocyclylene, arylene or cycloalkylene is optionally substituted with one or more substituents selected from the group consisting of: deuterium, hydroxy, amino, mercapto, =O, halogen, cyano, $C_1$-$C_6$ alkyl, halogenated $C_1$-$C_6$ alkyl, $C_1$-$C_6$ alkoxy, and halogenated $C_1$-$C_6$ alkoxy.

In some embodiments, there is provided the compound of Formula (I) or salts, enantiomers, stereoisomers, solvates, isotopically enriched analogs, prodrugs, or polymorphs thereof, wherein $R^2$ represents a heteroarylylidene, examples of which include, but are not limited to, 7- to 30-membered heteroarylylidene, 7- to 25-membered heteroarylylidene, 7- to 20-membered heteroarylylidene, 7- to 15-membered heteroarylylidene, 8- to 30-membered heteroarylylidene, 8- to 25-membered heteroarylylidene, 8- to 20-membered heteroarylylidene, 8- to 15-membered heteroarylylidene, 9- to 30-membered heteroarylylidene, 9- to 25-membered heteroarylylidene, 9- to 20-membered heteroarylylidene, 9- to 15-membered heteroarylylidene, 10- to 30-membered heteroarylylidene, 10- to 25-membered heteroarylylidene, 10- to 20-membered heteroarylylidene or 10- to 15-membered heteroarylylidene, wherein the heteroarylylidene is optionally substituted with one or more substituents selected from the group consisting of: deuterium, hydroxy, amino, mercapto, =O, halogen, cyano, $C_1$-$C_6$ alkyl, halogenated $C_1$-$C_6$ alkyl, $C_1$-$C_6$ alkoxy, and halogenated $C_1$-$C_6$ alkoxy.

In some embodiments, there is provided the compound of Formula (I) or salts, enantiomers, stereoisomers, solvates, isotopically enriched analogs, prodrugs, or polymorphs thereof, wherein examples of $R^2$ representing heteroarylylidene include, but are not limited to, chromanylylidene or 2,3-dihydroquinolin-4(1H)-ylylidene, wherein the heteroarylylidene is optionally substituted with one or more substituents selected from the group consisting of: deuterium, hydroxy, amino, mercapto, =O, halogen, cyano, $C_1$-$C_6$ alkyl, halogenated $C_1$-$C_6$ alkyl, $C_1$-$C_6$ alkoxy, and halogenated $C_1$-$C_6$ alkoxy.

In some embodiments, there is provided the compound of Formula (I) or salts, enantiomers, stereoisomers, solvates, isotopically enriched analogs, prodrugs, or polymorphs thereof, wherein examples of $R^2$ representing heteroarylene include, but are not limited to, 5- to 30-membered heteroarylene, 5- to 25-membered heteroarylene, 5- to 20-membered heteroarylene, 5- to 15-membered heteroarylene, 6- to 30-membered heteroarylene, 6- to 25-membered heteroarylene, 6- to 20-membered heteroarylene, 6- to 15-membered heteroarylene, 7- to 30-membered heteroarylene, 7- to 25-membered heteroarylene, 7- to 20-membered heteroarylene, 7- to 15-membered heteroarylene, 8- to 30-membered heteroarylene, 8- to 25-membered heteroarylene, 8- to 20-membered heteroarylene or 8- to 15-membered heteroarylene, wherein the heteroarylene is optionally substituted with one or more substituents selected from the group consisting of: deuterium, hydroxy, amino, mercapto, =O, halogen, cyano, $C_1$-$C_6$ alkyl, halogenated $C_1$-$C_6$ alkyl, $C_1$-$C_6$ alkoxy, and halogenated $C_1$-$C_6$ alkoxy.

In some embodiments, there is provided the compound of Formula (I) or salts, enantiomers, stereoisomers, solvates, isotopically enriched analogs, prodrugs, or polymorphs thereof, wherein examples of $R^2$ representing heteroarylene include, but are not limited to, furanylene, oxazolylene, isoxazolylene, oxadiazolylene, thienylene, isothiazolylene, thiadiazolylene, pyrrolylene, imidazolylene, pyrazolylene, triazolylene, pyridylene, pyrimidinylene, pyridazinylene, pyrazinylene, indolylene, isoindolylene, indazolylene, quinolinylene, isoquinolinylene, quinazolinylene, triazinylene, isoxazolo[4,5-c]pyridinylene, pyrrolo[2,3-b]pyridinylene, indolinylene, benzothiazolylene, benzofuranylene, isobenzofuranylene, benzothienylene, benzimidazolylene, 2,3-dihydro-1H-benzo[d]imidazolylene, pyrrolo[2,3-b]pyridinylene, chromanylene, 6,7-dihydrothieno[3,2-d]pyrimidinylene, acridinylene, benzindolylene, carbazolylene, dibenzofuranylene, xanthenylene, 3,4-dihydroquinolinylene, 2,3-dihydro-4H-benzo[b][1,4]oxazinylene, chromanylene, thieno[2,3-d]pyrimidinylene, thieno[3,2-d]pyrimidinylene, tetrahydrobenzo[4,5]thieno[2,3-d]pyrimidinylene, 6,7-dihydro-5H-cyclopenta[4,5]thieno[2,3-d]pyrimidinylene, dihydrothieno[3,2-d]pyrimidinylene, 5,6-dihydro-[1,2,4]triazolo[4,3-a]pyrazinylene, dihydrothieno[3,2-c]pyridinylene, benzoxazolylene, benzisoxazolylene, benzisothiazolylene, benzotriazolylene, benzo[2,1,3]oxadiazolylene, benzo[2,1,3]thiadiazolylene, benzo[1,2,3]thiadiazolylene, pyrazolo[1,5-a]pyridylene, pyrazolo[1,5-a]pyrimidinylene, imidazo[1,2-a]pyridylene, 1H-pyrrolo[3,2-b]thiazolylene, or benzo[2,1-b]thiazolylene. The heteroarylene is optionally substituted with one or more substituents selected from the group consisting of: deuterium, hydroxy, amino, mercapto, =O, halogen, cyano, $C_1$-$C_6$ alkyl, halogenated $C_1$-$C_6$ alkyl, $C_1$-$C_6$ alkoxy, and halogenated $C_1$-$C_6$ alkoxy.

In some embodiments, there is provided the compound of Formula (I) or salts, enantiomers, stereoisomers, solvates, isotopically enriched analogs, prodrugs, or polymorphs thereof, wherein examples of $R^2$ representing heterocyclylene include, but are not limited to, 4- to 30-membered, 4- to 25-membered, 4- to 20-membered, 4- to 15-membered, 5- to 20-membered, 5- to 15-membered, 6- to 20-membered, 6- to 15-membered, 7- to 20-membered, or 7- to 15-membered heterocyclylene. In some embodiments, examples of heterocyclylene include: 4- to 30-membered monocyclic

heterocyclylene, 4- to 25-membered monocyclic heterocyclylene, 4- to 20-membered monocyclic heterocyclylene, 4- to 15-membered monocyclic heterocyclylene, 5- to 30-membered bridged heterocyclylene, 5- to 20-membered bridged heterocyclylene, 5- to 15-membered bridged heterocyclylene, 6- to 20-membered bridged heterocyclylene, 6- to 15-membered bridged heterocyclylene, 7- to 30-membered bridged heterocyclylene, 7- to 20-membered bridged heterocyclylene, 7- to 15-membered bridged heterocyclylene, 5- to 30-membered fused heterocyclylene, 5- to 20-membered fused heterocyclylene, 5- to 15-membered fused heterocyclylene, 6-to 20-membered fused heterocyclylene, 6- to 15-membered fused heterocyclylene, 7- to 30-membered fused heterocyclylene, 7- to 20-membered fused heterocyclylene, 7- to 15-membered fused heterocyclylene, 5- to 30-membered spiro-heterocyclylene, 5- to 20-membered spiro-heterocyclylene, 5- to 15-membered spiro-heterocyclylene, 6- to 20-membered spiro-heterocyclylene, 6- to 15-membered spiro-heterocyclylene, 7- to 30-membered spiro-heterocyclylene, 7- to 20-membered spiro-heterocyclylene, or 7- to 15-membered spiro-heterocyclylene. The heterocyclylene is optionally substituted with one or more substituents selected from the group consisting of: deuterium, hydroxy, amino, mercapto, =O, halogen, cyano, $C_1$-$C_6$ alkyl, halogenated $C_1$-$C_6$ alkyl, $C_1$-$C_6$ alkoxy, and halogenated $C_1$-$C_6$ alkoxy.

In some embodiments, there is provided the compound of Formula (I) or salts, enantiomers, stereoisomers, solvates, isotopically enriched analogs, prodrugs, or polymorphs thereof, wherein examples of $R^2$ representing heterocyclylene include, but are not limited to, azetidinylene, oxetanylene, pyrrolidinylene, imidazolidinylene, pyrazolidylene, piperidinylene, tetrahydrofuranylene, tetrahydropyranylene, tetrahydrothienylene, tetrahydrothiopyranylene, oxazolidinylene, thiazolidinylene, piperidinylene, piperazinylene, morpholinylene, tetrahydrothienylene, thiomorpholinylene, dioxacyclohexylene, azetidin-1-ylene, 3,6-dihydropyridin-1(2H)-ylene, 1,4-diazepan-1-ylene, 3,6-diazabicyclo[3.1.1]heptan-6-ylene, 3,6-diazabicyclo[3.1.1]heptan-3-ylene, 2,5-diazabicyclo[2.2.1]heptan-2-ylene, 2,5-diazabicyclo[2.2.2]octan-2-ylene, 3,8-diazabicyclo[3.2.1]octan-3-ylene, 3,8-diazabicyclo[3.2.1]octan-8-ylene, and azaspiro-heterocyclylene (e.g., 5- to 30-membered, 5- to 20-membered, 5- to 15-membered, 6- to 20-membered, 6- to 15-membered, 7- to 30-membered, 7- to 20-membered or 7- to 15-membered azaspiro-heterocyclylene), wherein the heterocyclylene is optionally substituted with one or more substituents selected from the group consisting of: deuterium, hydroxy, amino, mercapto, =O, halogen, cyano, $C_1$-$C_6$ alkyl, halogenated $C_1$-$C_6$ alkyl, $C_1$-$C_6$ alkoxy, and halogenated $C_1$-$C_6$ alkoxy.

**In** some embodiments, there is provided the compound of Formula (I) or salts, enantiomers, stereoisomers, solvates, isotopically enriched analogs, prodrugs, or polymorphs thereof, wherein $R^2$ represents $(CH_2)_n$-arylene, where n represents an integer of 0, 1 or 2. Examples of arylene include, but are not limited to, $C_{5-30}$ arylene, $C_{5-20}$ arylene, $C_{5-15}$ arylene, $C_{6-30}$ arylene, $C_{6-20}$ arylene, and $C_{6-15}$ arylene. In some embodiments, Examples of $(CH_2)_n$-arylene include, but are not limited to, $C_{5-30}$ arylene, $C_{5-20}$ arylene, $C_{5-15}$ arylene, $C_{6-30}$ arylene, $C_{6-20}$ arylene, $C_{6-15}$ arylene, $C_{7-30}$ arylene, $C_{7-20}$ arylene, $C_{7-15}$ arylene, $C_{8-30}$ arylene, $C_{8-20}$ arylene, $C_{8-15}$ arylene, $CH_2$-($C_{5-30}$ arylene), $CH_2$-($C_{5-20}$ arylene), $CH_2$-($C_{5-15}$ arylene), $CH_2$-($C_{6-30}$ arylene), $CH_2$-($C_{6-20}$ arylene), $CH_2$-($C_{6-15}$ arylene), $CH_2$-($C_{7-30}$ arylene), $CH_2$-($C_{7-20}$ arylene), $CH_2$-($C_{7-15}$ arylene), $CH_2$-($C_{8-30}$ arylene), $CH_2$-($C_{8-20}$ arylene), $CH_2$-($C_{8-15}$ arylene), $CH_2$-$CH_2$-($C_{5-30}$ arylene), $CH_2$-$CH_2$-($C_{5-20}$ arylene), $CH_2$-$CH_2$-($C_{5-15}$ arylene), $CH_2$-$CH_2$-($C_{6-30}$ arylene), $CH_2$-$CH_2$-($C_{6-20}$ arylene), $CH_2$-$CH_2$-($C_{6-15}$ arylene), $CH_2$-$CH_2$-($C_{7-30}$ arylene), $CH_2$-$CH_2$-($C_{7-20}$ arylene), $CH_2$-$CH_2$-($C_{7-15}$ arylene), $CH_2$-$CH_2$-($C_{8-30}$ arylene), $CH_2$-$CH_2$-($C_{8-20}$ arylene), and $CH_2$-$CH_2$-($C_{8-15}$ arylene). The arylene is optionally substituted with one or more substituents selected from the group consisting of: deuterium, hydroxy, amino, mercapto, = O, halogen, cyano, $C_1$-$C_6$ alkyl, halogenated $C_1$-$C_6$ alkyl, $C_1$-$C_6$ alkoxy, and halogenated $C_1$-$C_6$ alkoxy.

In some embodiments, there is provided the compound of Formula (I) or salts, enantiomers, stereoisomers, solvates, isotopically enriched analogs, prodrugs, or polymorphs thereof, wherein $R^2$ represents $(CH_2)_n$-arylene, where n represents an integer of 0, 1 or 2. Examples of $R^2$ include, but are not limited to, phenylene, naphthylene, $CH_2$-phenylene, $CH_2$-naphthylene, $CH_2$-$CH_2$-phenylene or $CH_2$-$CH_2$-naphthylene, wherein the phenylene or naphthylene is optionally substituted with one or more substituents selected from the group consisting of: deuterium, hydroxy, amino, mercapto, =O, halogen, cyano, $C_1$-$C_6$ alkyl, halogenated $C_1$-$C_6$ alkyl, $C_1$-$C_6$ alkoxy, and halogenated $C_1$-$C_6$ alkoxy.

In some embodiments, there is provided the compound of Formula (I) or salts, enantiomers, stereoisomers, solvates, isotopically enriched analogs, prodrugs, or polymorphs thereof, wherein $R^2$ represents $(CH_2)_n$-cycloalkylene, where n represents an integer of 0, 1 or 2. Examples of cycloalkylene include, but are not limited to, $C_3$-$C_{30}$ cycloalkylene, $C_3$-$C_{20}$ cycloalkylene, $C_3$-$C_{15}$ cycloalkylene, $C_4$-$C_{30}$ cycloalkylene, $C_4$-$C_{20}$ cycloalkylene, $C_4$-$C_{15}$ cycloalkylene, and $C_3$-$C_{12}$ cycloalkylene. Examples of $(CH_2)_n$-cycloalkylene include, but are not limited to, $C_{3-30}$ monocyclic cycloalkylene, $C_{3-20}$ monocyclic cycloalkylene, $C_{3-15}$ monocyclic cycloalkylene, $C_{4-15}$ monocyclic cycloalkylene, $C_5$-$C_{15}$ monocyclic cycloalkylene, $C_7$-$C_{30}$ monocyclic cycloalkylene, $C_7$-$C_{20}$ monocyclic cycloalkylene, $C_7$-$C_{15}$ monocyclic cycloalkylene, $C_5$-$C_{30}$ bridged cycloalkylene, $C_5$-$C_{20}$ bridged cycloalkylene, $C_5$-$C_{15}$ bridged cycloalkylene, $C_6$-$C_{30}$ bridged cycloalkylene, $C_6$-$C_{20}$ bridged cycloalkylene, $C_6$-$C_{15}$ bridged cycloalkylene, $C_7$-$C_{30}$ bridged cycloalkylene, $C_7$-$C_{20}$ bridged cycloalkylene, $C_7$-$C_{15}$ bridged cycloalkylene, $C_5$-$C_{30}$ fused cycloalkylene, $C_5$-$C_{20}$ fused cycloalkylene, $C_5$-$C_{15}$ fused cycloalkylene, $C_6$-$C_{30}$ fused cycloalkylene, $C_6$-$C_{20}$ fused cycloalkylene, $C_6$-$C_{15}$ fused cycloalkylene, $C_7$-$C_{30}$ fused cycloalkylene, $C_7$-$C_{20}$ fused cycloalkylene, $C_7$-$C_{15}$ fused cycloalkylene, $C_5$-$C_{20}$ fused cycloalkylene, $C_5$-$C_{20}$ spiro-cycloalkylene, $C_5$-$C_{15}$ spiro-cycloalkylene, $C_6$-$C_{30}$ spiro-cycloalkylene, $C_6$-$C_{20}$ spiro-cycloalkylene, $C_6$-$C_{15}$ spiro-cycloalkylene, $C_7$-$C_{30}$ spiro-cycloalkylene, $C_7$-$C_{20}$ spiro-cycloalkylene, $C_7$-$C_{15}$ spiro-cycloalkylene, $CH_2$-($C_{3-30}$ monocyclic

cycloalkylene), $CH_2$-($C_{3-20}$ monocyclic cycloalkylene), $CH_2$-($C_{3-15}$ monocyclic cycloalkylene), $CH_2$-($C_{4-15}$ monocyclic cycloalkylene), $CH_2$-($C_{5-15}$ monocyclic cycloalkylene), $CH_2$-($C_{7-30}$ monocyclic cycloalkylene), $CH_2$-($C_{7-20}$ monocyclic cycloalkylene), $CH_2$-($C_{7-15}$ monocyclic cycloalkylene), $CH_2$-($C_{5-30}$ bridged cycloalkylene), $CH_2$-($C_{5-20}$ bridged cycloalkylene), $CH_2$-($C_{5-15}$ bridged cycloalkylene), $CH_2$-($C_{6-30}$ bridged cycloalkylene), $CH_2$-($C_{6-20}$ bridged cycloalkylene), $CH_2$-($C_{6-15}$ bridged cycloalkylene), $CH_2$-($C_{7-30}$ bridged cycloalkylene), $CH_2$-($C_{7-20}$ bridged cycloalkylene), $CH_2$-($C_{7-15}$ bridged cycloalkylene), $CH_2$-($C_{5-30}$ fused cycloalkylene), $CH_2$-($C_{5-20}$ fused cycloalkylene), $CH_2$-($C_{5-15}$ fused cycloalkylene), $CH_2$-($C_{6-30}$ fused cycloalkylene), $CH_2$-($C_{6-20}$ fused cycloalkylene), $CH_2$-($C_{6-15}$ fused cycloalkylene), $CH_2$-($C_{7-30}$ fused cycloalkylene), $CH_2$-($C_{7-20}$ fused cycloalkylene), $CH_2$-($C_{7-15}$ fused cycloalkylene), $CH_2$-($C_{5-30}$ spiro-cycloalkylene), $CH_2$-($C_{5-20}$ spiro-cycloalkylene), $CH_2$-($C_{5-15}$ spiro-cycloalkylene), $CH_2$-($C_{6-30}$ spiro-cycloalkylene), $CH_2$-($C_{6-20}$ spiro-cycloalkylene), $CH_2$-($C_{6-15}$ spiro-cycloalkylene), $CH_2$-($C_{7-30}$ spiro-cycloalkylene), $CH_2$-($C_{7-20}$ spiro-cycloalkylene), $CH_2$-($C_{7-15}$ spiro-cycloalkylene), $CH_2$-$CH_2$-($C_{3-30}$ monocyclic cycloalkylene), $CH_2$-$CH_2$-($C_{3-20}$ monocyclic cycloalkylene), $CH_2$-$CH_2$-($C_{3-15}$ monocyclic cycloalkylene), $CH_2$-$CH_2$-($C_{4-15}$ monocyclic cycloalkylene), $CH_2$-$CH_2$-($C_{5-15}$ monocyclic cycloalkylene), $CH_2$-$CH_2$-($C_{7-30}$ monocyclic cycloalkylene), $CH_2$-$CH_2$-($C_{7-20}$ monocyclic cycloalkylene), $CH_2$-$CH_2$-($C_{7-15}$ monocyclic cycloalkylene), $CH_2$-$CH_2$-($C_{5-30}$ bridged cycloalkylene), $CH_2$-$CH_2$-($C_{5-20}$ bridged cycloalkylene), $CH_2$-$CH_2$-($C_{5-15}$ bridged cycloalkylene), $CH_2$-$CH_2$-($C_{6-30}$ bridged cycloalkylene), $CH_2$-$CH_2$-($C_{6-20}$ bridged cycloalkylene), $CH_2$-$CH_2$-($C_{6-15}$ bridged cycloalkylene), $CH_2$-$CH_2$-($C_{7-30}$ bridged cycloalkylene), $CH_2$-$CH_2$-($C_{7-20}$ bridged cycloalkylene), $CH_2$-$CH_2$-($C_{7-15}$ bridged cycloalkylene), $CH_2$-$CH_2$-($C_{5-30}$ fused cycloalkylene), $CH_2$-$CH_2$-($C_{5-20}$ fused cycloalkylene), $CH_2$-$CH_2$-($C_{5-15}$ fused cycloalkylene), $CH_2$-$CH_2$-($C_{6-30}$ fused cycloalkylene), $CH_2$-$CH_2$-($C_{6-20}$ fused cycloalkylene), $CH_2$-$CH_2$-($C_{6-15}$ fused cycloalkylene), $CH_2$-$CH_2$-($C_{7-30}$ fused cycloalkylene), $CH_2$-$CH_2$-($C_{7-20}$ fused cycloalkylene), $CH_2$-$CH_2$-($C_{7-15}$ fused cycloalkylene), $CH_2$-$CH_2$-($C_{5-30}$ spiro-cycloalkylene), $CH_2$-$CH_2$-($C_{5-20}$ spiro-cycloalkylene), $CH_2$-$CH_2$-($C_{5-15}$ spiro-cycloalkylene), $CH_2$-$CH_2$-($C_{6-30}$ spiro-cycloalkylene), $CH_2$-$CH_2$-($C_{6-20}$ spiro-cycloalkylene), $CH_2$-$CH_2$-($C_{6-15}$ spiro-cycloalkylene), $CH_2$-$CH_2$-($C_{7-30}$ spiro-cycloalkylene), $CH_2$-$CH_2$-($C_{7-20}$ spiro-cycloalkylene) or $CH_2$-$CH_2$-($C_{7-15}$ spiro-cycloalkylene), wherein the cycloalkylene is optionally substituted with one or more substituents selected from the group consisting of: deuterium, hydroxy, amino, mercapto, =O, halogen, cyano, $C_1$-$C_6$ alkyl, halogenated $C_1$-$C_6$ alkyl, $C_1$-$C_6$ alkoxy, and halogenated $C_1$-$C_6$ alkoxy.

In some embodiments, there is provided the compound of Formula (I) or salts, enantiomers, stereoisomers, solvates, isotopically enriched analogs, prodrugs, or polymorphs thereof, wherein $R^2$ represents $(CH_2)_n$-cycloalkylene, where n represents an integer of 0, 1 or 2. Examples of $R^2$ include, but are not limited to, cyclopropylene, cyclobutylene, cyclopentylene, cyclopentenylene, cyclohexylene, cyclohexenylene, cycloheptylene, cyclooctylene, decalinylene, octahydropentalenylene, octahydro-1H-indenylene, $C_{5-15}$ spiro-cycloalkylene, adamantanylene, noradamantanylene, bornylene, bicyclo[2.2.1]heptylene, bicyclo[2.2.1]heptenylene, $CH_2$-cyclopropylene, $CH_2$-cyclobutylene, $CH_2$-cyclopentylene, $CH_2$-cyclopentenylene, $CH_2$-cyclohexylene, $CH_2$-cyclohexenylene, $CH_2$-cycloheptylene, $CH_2$-cyclooctylene, $CH_2$-decalinylene, $CH_2$-(octahydropentalenylene), $CH_2$-(octahydro-1H-indenylene), $CH_2$-($C_5$-$C_{15}$ spiro-cycloalkylene), $CH_2$-adamantanylene, $CH_2$-noradamantanylene, $CH_2$-bornylene, $CH_2$-(bicyclo[2.2.1]heptylene), $CH_2$-(bicyclo[2.2.1]heptenylene), $CH_2$-$CH_2$-cyclopropylene, $CH_2$-$CH_2$-cyclobutylene, $CH_2$-$CH_2$-cyclopentylene, $CH_2$-$CH_2$-cyclopentenylene, $CH_2$-$CH_2$-cyclohexylene, $CH_2$-$CH_2$-cyclohexenylene, $CH_2$-$CH_2$-cycloheptylene, $CH_2$-$CH_2$-cyclooctylene, $CH_2$-$CH_2$-decalinylene, $CH_2$-$CH_2$-(octahydropentalenylene), $CH_2$-$CH_2$-(octahydro-1H-indenylene), $CH_2$-$CH_2$-($C_5$-$C_{15}$ spiro-cycloalkylene), $CH_2$-$CH_2$-adamantanylene, $CH_2$-$CH_2$-noradamantanylene, $CH_2$-$CH_2$-bornylene, $CH_2$-$CH_2$-(bicyclo [2.2.1]heptylene) or $CH_2$-$CH_2$-(bicyclo[2.2.1]heptenylene), wherein the cycloalkylene is optionally substituted with one or more substituents selected from the group consisting of: deuterium, hydroxy, amino, mercapto, =O, halogen, cyano, $C_1$-$C_6$ alkyl, halogenated $C_1$-$C_6$ alkyl, $C_1$-$C_6$ alkoxy, and halogenated $C_1$-$C_6$ alkoxy.

In some embodiments, there is provided the compound of Formula (I) or salts, enantiomers, stereoisomers, solvates, isotopically enriched analogs, prodrugs, or polymorphs thereof, wherein $R^3$ represents $SR^b$ or $R^b$, where $R^b$ represents aryl. Examples of aryl include, but are not limited to, $C_{5-30}$ aryl, $C_{5-20}$ aryl, $C_{5-15}$ aryl, $C_{6-30}$ aryl, $C_{6-20}$ aryl, $C_{6-15}$ aryl, $C_{7-30}$ aryl, $C_{7-20}$ aryl, $C_{7-15}$ aryl, $C_{8-30}$ aryl, $C_{8-20}$ aryl, and $C_{8-15}$ aryl. Examples of $SR^b$ include, but are not limited to, S-($C_{5-30}$ aryl), S-($C_{5-20}$ aryl), S-($C_{5-15}$ aryl), S-($C_{6-30}$ aryl), S-($C_{6-20}$ aryl), S-($C_{6-15}$ aryl), S-($C_{7-30}$ aryl), S-($C_{7-20}$ aryl), S-($C_{7-15}$ aryl), S-($C_{8-30}$ aryl), S-($C_{8-20}$ aryl) or S-($C_{8-15}$ aryl) , wherein the aryl is optionally substituted with one or more substituents selected from the group consisting of: deuterium, hydroxy, amino, mercapto, = O, halogen, cyano, $C_1$-$C_6$ alkyl, halogenated $C_1$-$C_6$ alkyl, $C_1$-$C_6$ alkoxy, and halogenated $C_1$-$C_6$ alkoxy.

In some embodiments, there is provided the compound of Formula (I) or salts, enantiomers, stereoisomers, solvates, isotopically enriched analogs, prodrugs, or polymorphs thereof, wherein $R^3$ represents $SR^b$ or $R^b$, where $R^b$ represents aryl. Examples of $R^3$ include, but are not limited to, phenyl, naphthyl, S- phenyl or S-naphthyl. The aryl is optionally substituted with one or more substituents selected from the group consisting of: deuterium, hydroxy, amino, mercapto, =O, halogen, cyano, $C_1$-$C_6$ alkyl, halogenated $C_1$-$C_6$ alkyl, $C_1$-$C_6$ alkoxy, and halogenated $C_1$-$C_6$ alkoxy.

In some embodiments, there is provided the compound of Formula (I) or salts, enantiomers, stereoisomers, solvates, isotopically enriched analogs, prodrugs, or polymorphs thereof, wherein examples of $R^3$ representing halogenated $C_1$-$C_6$ alkyl include, but are not limited to, $F_3C$-, $FCH_2$-, $F_2CH$-, $ClCH_2$-, $Cl_2CH$-, $CF_3CF_2$-, $CF_3CHF$-, $CHF_2CF_2$-, $CHF_2CHF$-, $CF_3CH_2$- or $CH_2ClCH_2$-.

In some embodiments, there is provided the compound of Formula (I) or salts, enantiomers, stereoisomers, solvates, isotopically enriched analogs, prodrugs, or polymorphs thereof, wherein examples of $R^3$ representing cycloalkyl include, but are not limited to, $C_{3-30}$ monocyclic cycloalkyl, $C_{3-20}$ monocyclic cycloalkyl, $C_{3-15}$ monocyclic cycloalkyl, $C_{4-15}$ monocyclic cycloalkyl, $C_{5-15}$ monocyclic cycloalkyl, $C_{7-30}$ monocyclic cycloalkyl, $C_{7-20}$ monocyclic cycloalkyl, $C_{7-15}$ monocyclic cycloalkyl, $C_{5-30}$ bridged cycloalkyl, $C_{5-20}$ bridged cycloalkyl, $C_{5-15}$ bridged cycloalkyl, $C_{6-30}$ bridged cycloalkyl, $C_{6-20}$ bridged cycloalkyl, $C_{6-15}$ bridged cycloalkyl, $C_{7-30}$ bridged cycloalkyl, $C_{7-20}$ bridged cycloalkyl, $C_{7-15}$ bridged cycloalkyl, $C_{5-30}$ fused cycloalkyl, $C_{5-20}$ fused cycloalkyl, $C_{5-15}$ fused cycloalkyl, $C_{6-30}$ fused cycloalkyl, $C_{6-20}$ fused cycloalkyl, $C_{6-15}$ fused cycloalkyl, $C_{7-30}$ fused cycloalkyl, $C_{7-20}$ fused cycloalkyl, $C_{7-15}$ fused cycloalkyl, $C_{5-30}$ spiro-cycloalkyl, $C_{5-20}$ spiro-cycloalkyl, $C_{5-15}$ spiro-cycloalkyl, $C_{6-30}$ spiro-cycloalkyl, $C_{6-20}$ spiro-cycloalkyl, $C_{6-15}$ spiro-cycloalkyl, $C_{7-30}$ spiro-cycloalkyl, $C_{7-20}$ spiro-cycloalkyl or $C_{7-15}$ spiro-cycloalkyl, and wherein the cycloalkyl is optionally substituted with one or more substituents selected from the group consisting of: deuterium, hydroxy, amino, mercapto, =O, halogen, cyano, $C_1$-$C_6$ alkyl, halogenated $C_1$-$C_6$ alkyl, $C_1$-$C_6$ alkoxy, and halogenated $C_1$-$C_6$ alkoxy.

In some embodiments, there is provided the compound of Formula (I) or salts, enantiomers, stereoisomers, solvates, isotopically enriched analogs, prodrugs, or polymorphs thereof, wherein examples of $R^3$ representing cycloalkyl include, but are not limited to, cyclopropyl, cyclobutyl, cyclopentyl, cyclopentenyl, cyclohexyl, cyclohexenyl, cycloheptyl, cyclooctyl, decalinyl, octahydropentalenyl, octahydro-1H-indenyl, $C_{5-15}$ spiro-cycloalkyl, adamantanyl, noradamantanyl, bornyl, bicyclo[2.2.1]heptyl, and bicyclo[2.2.1]heptenyl, and wherein the cycloalkyl is optionally substituted with one or more substituents selected from the group consisting of: deuterium, hydroxy, amino, mercapto, =O, halogen, cyano, $C_1$-$C_6$ alkyl, halogenated $C_1$-$C_6$ alkyl, $C_1$-$C_6$ alkoxy, and halogenated $C_1$-$C_6$ alkoxy.

In some embodiments, there is provided the compound of Formula (I) or salts, enantiomers, stereoisomers, solvates, isotopically enriched analogs, prodrugs, or polymorphs thereof, wherein examples of $R^3$ representing nitrogen-containing heterocyclyl include, but are not limited to, 4- to 20-membered nitrogen-containing heterocyclyl, 4- to 15-membered nitrogen-containing heterocyclyl, 5- to 20-membered nitrogen-containing heterocyclyl, or 5- to 15-membered nitrogen-containing heterocyclyl, and wherein the nitrogen-containing heterocyclyl is optionally substituted with one or more substituents selected from the group consisting of: hydroxy, amino, mercapto, =O, halogen, cyano, $C_1$-$C_6$ alkyl, halogenated $C_1$-$C_6$ alkyl, $C_1$-$C_6$ alkoxy, and halogenated $C_1$-$C_6$ alkoxy.

In some embodiments, there is provided the compound of Formula (I) or salts, enantiomers, stereoisomers, solvates, isotopically enriched analogs, prodrugs, or polymorphs thereof, wherein examples of $R^3$ representing nitrogen-containing heterocyclyl include, but are not limited to, piperazinyl, piperidinyl, pyrrolidinyl, imidazolidinyl, pyrazolidyl, oxazolidinyl, morpholinyl, thiomorpholinyl, diazacycloheptanyl, diazabicyclo[3.1.1]heptanyl, diazabicyclo[2.2.1]heptanyl, diazabicyclo[2.2.2]octanyl, diazabicyclo[3.2.1]octanyl, or azaspiro-heterocyclyl, and wherein the nitrogen-containing heterocyclyl is optionally substituted with one or more substituents selected from the group consisting of: deuterium, hydroxy, amino, mercapto, =O, halogen, cyano, $C_1$-$C_6$ alkyl, halogenated $C_1$-$C_6$ alkyl, $C_1$-$C_6$ alkoxy, and halogenated $C_1$-$C_6$ alkoxy.

In some embodiments, there is provided the compound of Formula (I) or salts, enantiomers, stereoisomers, solvates, isotopically enriched analogs, prodrugs, or polymorphs thereof, wherein when $R^1$ represents O, NH, $NHS(O)_2$ or $S(O)_2NH$, $R^2$ represents $-(CH_2)_n$-cycloalkylene, and $R^3$ represents hydrogen, wherein the cycloalkylene is optionally substituted with one or more substituents selected from the group consisting of: deuterium, hydroxy, amino, mercapto, =O, halogen, cyano, $C_1$-$C_6$ alkyl, halogenated $C_1$-$C_6$ alkyl, $C_1$-$C_6$ alkoxy, and halogenated $C_1$-$C_6$ alkoxy; wherein the cycloalkylene is as defined above and is optionally substituted with one or more substituents selected from the group consisting of: deuterium, hydroxy, amino, mercapto, =O, halogen, cyano, $C_1$-$C_6$ alkyl, halogenated $C_1$-$C_6$ alkyl, $C_1$-$C_6$ alkoxy, and halogenated $C_1$-$C_6$ alkoxy.

In some embodiments, there is provided the compound of Formula (I) or salts, enantiomers, stereoisomers, solvates, isotopically enriched analogs, prodrugs, or polymorphs thereof, wherein when represents a double bond, $R^1$ represents piperidinylylidene or azetidinylylidene, $R^2$ represents chromanylylidene, and $R^3$ represents hydrogen.

In some embodiments, there is provided the compound of Formula (I) or salts, enantiomers, stereoisomers, solvates, isotopically enriched analogs, prodrugs, or polymorphs thereof, wherein when ---- represents a double bond, $-R^1-R^2-$ represents the following structure:

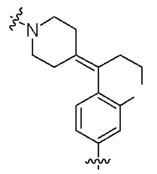
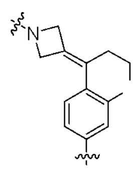
.

It should be understood by those skilled in the art that the present invention encompasses compounds obtained by any combination of various embodiments. Embodiments obtained by combining technical features or preferred

technical features from one embodiment with technical features or preferred technical features from another embodiment are also encompassed within the scope of the present invention.

In some embodiments, the following compounds of Table 1, and their salts (including pharmaceutically acceptable salts, such as their hydrochloride salts), enantiomers, stereoisomers, solvates, isotopically enriched analogs, prodrugs, or polymorphs thereof, are provided:

Table 1. The compounds of the present disclosure

| Compound No. | Structure of the compounds | The compounds' name | Chinese translation of the compounds' name |
|---|---|---|---|
| GT-04545 | 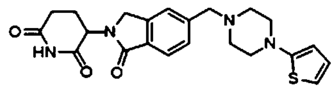 | 3-(1-oxo-5-((4-(thiophen-2-yl)piperazin-1-yl)methyl)isoindolin-2-yl)piperidine-2,6-dione | 3-(1-oxo-5-((4-(thiophen-2-yl)piperazin-1-yl)methyl)isoindolin-2-yl)piperidine-2,6-dione |
| GT-04546 | 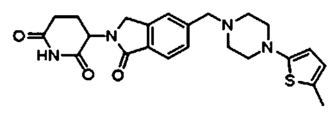 | 3-(5-((4-(5-methylthiophen-2-yl)piperazin-1-yl)methyl)-1-oxoisoindolin-2-yl)piperidine-2,6-dione | 3-(5-((4-(5-methylthiophen-2-yl)piperazin-1-yl)methyl)-1-oxoisoindolin-2-yl)piperidine-2,6-dione |
| GT-04582 | 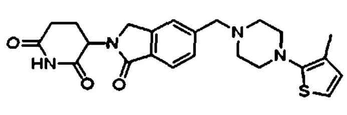 | 3-(5-((4-(3-methylthiophen-2-yl)piperazin-1-yl)methyl)-1-oxoisoindolin-2-yl)piperidine-2,6-dione | 3-(5-((4-(3-methylthiophen-2-yl)piperazin-1-yl)methyl)-1-oxoisoindolin-2-yl)piperidine-2,6-dione |
| GT-04547 | 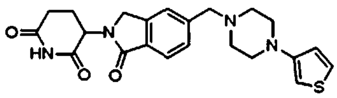 | 3-(1-oxo-5-((4-(thiophen-3-yl)piperazin-1-yl)methyl)isoindolin-2-yl)piperidine-2,6-dione | 3-(1-oxo-5-((4-(thiophen-3-yl)piperazin-1-yl)methyl)isoindolin-2-yl)piperidine-2,6-dione |
| GT-04548 | 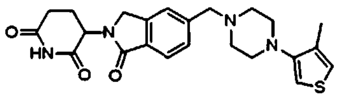 | 3-(5-((4-(4-methylthiophen-3-yl)piperazin-1-yl)methyl)-1-oxoisoindolin-2-yl)piperidine-2,6-dione | 3-(5-((4-(4-methylthiophen-3-yl)piperazin-1-yl)methyl)-1-oxoisoindolin-2-yl)piperidine-2,6-dione |
| GT-04549 | 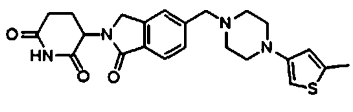 | 3-(5-((4-(5-methylthiophen-3-yl)piperazin-1-yl)methyl)-1-oxoisoindolin-2-yl)piperidine-2,6-dione | 3-(5-((4-(5-methylthiophen-3-yl)piperazin-1-yl)methyl)-1-oxoisoindolin-2-yl)piperidine-2,6-dione |
| GT-04494 | 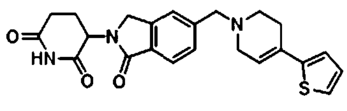 | 3-(1-oxo-5-((4-(thiophen-2-yl)-3,6-dihydropyridin-1(2H)-yl)methyl)isoindolin-2-yl)piperidine-2,6-dione | 3-(1-oxo-5-((4-(thiophen-2-yl)-3,6-dihydropyridin-1(2H)-yl)methyl)isoindolin-2-yl)piperidine-2,6-dione |
| GT-04572 | 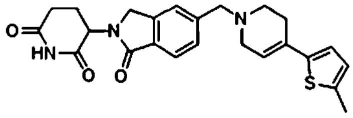 | 3-(5-((4-(5-methylthiophen-2-yl)-3,6-dihydropyridin-1(2H)-yl)methyl)-1-oxoisoindolin-2-yl)piperidine-2,6-dione | 3-(5-((4-(5-methylthiophen-2-yl)-3,6-dihydropyridin-1(2H)-yl)methyl)-1-oxoisoindolin-2-yl)piperidine-2,6-dione |
| GT-04495 | 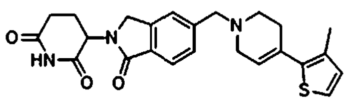 | 3-(5-((4-(3-methylthiophen-2-yl)-3,6-dihydropyridin-1(2H)-yl)methyl)-1-oxoisoindolin-2-yl)piperidine-2,6-dione | 3-(5-((4-(3-methylthiophen-2-yl)-3,6-dihydropyridin-1(2H)-yl)methyl)-1-oxoisoindolin-2-yl)piperidine-2,6-dione |
| GT-04585 | 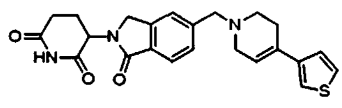 | 3-(1-oxo-5-((4-(thiophen-3-yl)-3,6-dihydropyridin-1(2H)-yl)methyl)isoindolin-2-yl)piperidine-2,6-dione | 3-(1-oxo-5-((4-(thiophen-3-yl)-3,6-dihydropyridin-1(2H)-yl)methyl)isoindolin-2-yl)piperidine-2,6-dione |
| GT-04503 | 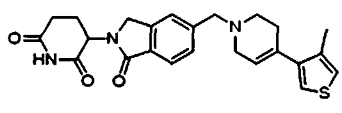 | 3-(5-((4-(4-methylthiophen-3-yl)-3,6-dihydropyridin-1(2H)-yl)methyl)-1-oxoisoindolin-2-yl)piperidine-2,6-dione | 3-(5-((4-(4-methylthiophen-3-yl)-3,6-dihydropyridin-1(2H)-yl)methyl)-1-oxoisoindolin-2-yl)piperidine-2,6-dione |

(continued)

| Compo und No. | Structure of the compounds | The compounds' name | Chinese translation of the compounds' name |
|---|---|---|---|
| GT-04587 | 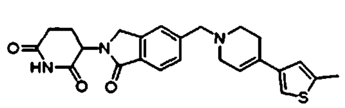 | 3-(5-((4-(5-methylthiophen-3-yl)-3,6-dihydropyridin-1(2H)-yl)methyl)-1-oxoisoindolin-2-yl)piperidine-2,6-dione | 3-(5-((4-(5-methylthiophen-3-yl)-3,6-dihydropyridin-1(2H)-yl)methyl)-1-oxoisoindolin-2-yl)piperidine-2,6-dione |
| GT-04617 | 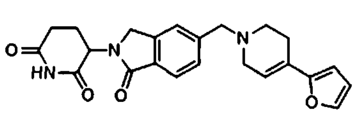 | 3-(5-((4-(furan-2-yl)-3,6-dihydropyridin-1(2H)-yl)methyl)-1-oxoisoindolin-2-yl)piperidine-2,6-dione | 3-(5-((4-(furan-2-yl)-3,6-dihydropyridin-1(2H)-yl)methyl)-1-oxoisoindolin-2-yl)piperidine-2,6-dione |
| GT-04589 | 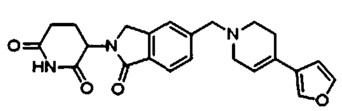 | 3-(5-((4-(furan-3-yl)-3,6-dihydropyridin-1(2H)-yl)methyl)-1-oxoisoindolin-2-yl)piperidine-2,6-dione | 3-(5-((4-(furan-3-yl)-3,6-dihydropyridin-1(2H)-yl)methyl)-1-oxoisoindolin-2-yl)piperidine-2,6-dione |
| GT-04571 | 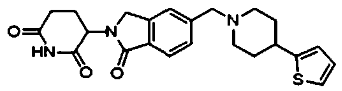 | 3-(1-oxo-5-((4-(thiophen-2-yl)piperidin-1-yl)methyl)isoindolin-2-yl)piperidine-2,6-dione | 3-(1-oxo-5-((4-(thiophen-2-yl)piperidin-1-yl)methyl)isoindolin-2-yl)piperidine-2,6-dione |
| GT-04573 | 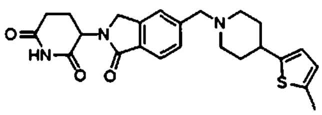 | 3-(5-((4-(5-methylthiophen-2-yl)piperidin-1-yl)methyl)-1-oxoisoindolin-2-yl)piperidine-2,6-dione | 3-(5-((4-(5-methylthiophen-2-yl)piperidin-1-yl)methyl)-1-oxoisoindolin-2-yl)piperidine-2,6-dione |
| GT-04502 | 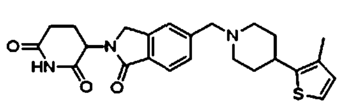 | 3-(5-((4-(3-methylthiophen-2-yl)piperidin-1-yl)methyl)-1-oxoisoindolin-2-yl)piperidine-2,6-dione | 3-(5-((4-(3-methylthiophen-2-yl)piperidin-1-yl)methyl)-1-oxoisoindolin-2-yl)piperidine-2,6-dione |
| GT-04586 | 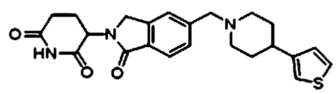 | 3-(1-oxo-5-((4-(thiophen-3-yl)piperidin-1-yl)methyl)isoindolin-2-yl)piperidine-2,6-dione | 3-(1-oxo-5-((4-(thiophen-3-yl)piperidin-1-yl)methyl)isoindolin-2-yl)piperidine-2,6-dione |
| GT-04588 | 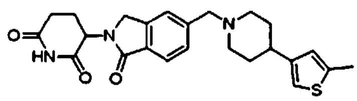 | 3-(5-((4-(5-methylthiophen-3-yl)piperidin-1-yl)methyl)-1-oxoisoindolin-2-yl)piperidine-2,6-dione | 3-(5-((4-(5-methylthiophen-3-yl)piperidin-1-yl)methyl)-1-oxoisoindolin-2-yl)piperidine-2,6-dione |
| GT-04504 | 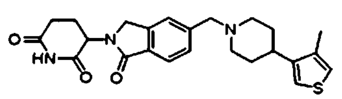 | 3-(5-((4-(4-methylthiophen-3-yl)piperidin-1-yl)methyl)-1-oxoisoindolin-2-yl)piperidine-2,6-dione | 3-(5-((4-(4-methylthiophen-3-yl)piperidin-1-yl)methyl)-1-oxoisoindolin-2-yl)piperidine-2,6-dione |
| GT-04618 | 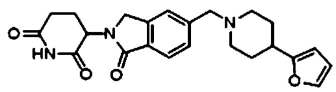 | 3-(5-((4-(furan-2-yl)piperidin-1-yl)methyl)-1-oxoisoindolin-2-yl)piperidine-2,6-dione | 3-(5-((4-(furan-2-yl)piperidin-1-yl)methyl)-1-oxoisoindolin-2-yl)piperidine-2,6-dione |
| GT-04590 | 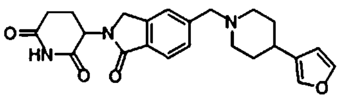 | 3-(5-((4-(furan-3-yl)piperidin-1-yl)methyl)-1-oxoisoindolin-2-yl)piperidine-2,6-dione | 3-(5-((4-(furan-3-yl)piperidin-1-yl)methyl)-1-oxoisoindolin-2-yl)piperidine-2,6-dione |
| GT-04201 | 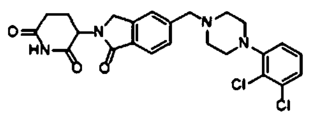 | 3-(5-((4-(2,3-dichlorophenyl)piperazin-1-yl)methyl)-1-oxoisoindolin-2-yl)piperidine-2,6-dione | 3-(5-((4-(2,3-dichlorophenyl)piperazin-1-yl)methyl)-1-oxoisoindolin-2-yl)piperidine-2,6-dione |
| GT-04446 | 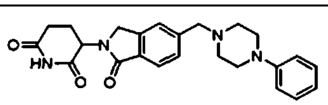 | 3-(1-oxo-5-((4-phenylpiperazin-1-yl)methyl)isoindolin-2-yl)piperidine-2,6-dione | 3-(1-oxo-5-((4-phenylpiperazin-1-yl)methyl)isoindolin-2-yl)piperidine-2,6-dione |

(continued)

| Compo und No. | Structure of the compounds | The compounds' name | Chinese translation of the compounds' name |
|---|---|---|---|
| GT-04490 | 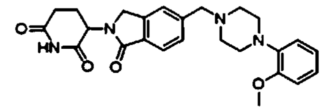 | 3-(5-((4-(2-methoxyphenyl)piperazin-1-yl)methyl)-1-oxoisoindolin-2-yl)piperidine-2,6-dione | 3-(5-((4-(2-methoxyphenyl)piperazin-1-yl)methyl)-1-oxoisoindolin-2-yl)piperidine-2,6-dione |
| GT-04448 | 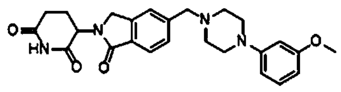 | 3-(5-((4-(3-methoxyphenyl)piperazin-1-yl)methyl)-1-oxoisoindolin-2-yl)piperidine-2,6-dione | 3-(5-((4-(3-methoxyphenyl)piperazin-1-yl)methyl)-1-oxoisoindolin-2-yl)piperidine-2,6-dione |
| GT-04447 | 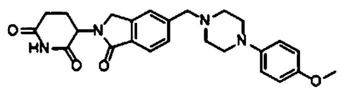 | 3-(5-((4-(4-methoxyphenyl)piperazin-1-yl)methyl)-1-oxoisoindolin-2-yl)piperidine-2,6-dione | 3-(5-((4-(4-methoxyphenyl)piperazin-1-yl)methyl)-1-oxoisoindolin-2-yl)piperidine-2,6-dione |
| GT-04451 | 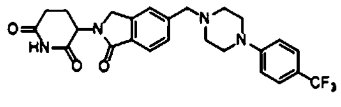 | 3-(1-oxo-5-((4-(4-(trifluoromethyl)phenyl)pip erazin-1-yl)methyl)isoindolin-2-yl)piperidine-2,6-dione | 3-(1-oxo-5-((4-(4-(trifluoromethyl)phenyl)pip erazin-1-yl)methyl)isoindolin-2-yl)piperidine-2,6-dione |
| GT-04452 | 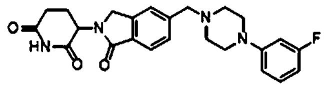 | 3-(5-((4-(3-fluorophenyl)piperazin-1-yl)methyl)-1-oxoisoindolin-2-yl)piperidine-2,6-dione | 3-(5-((4-(3-fluorophenyl)piperazin-1-yl)methyl)-1-oxoisoindolin-2-yl)piperidine-2,6-dione |
| GT-04453 | 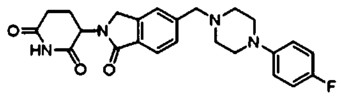 | 3-(5-((4-(4-fluorophenyl)piperazin-1-yl)methyl)-1-oxoisoindolin-2-yl)piperidine-2,6-dione | 3-(5-((4-(4-fluorophenyl)piperazin-1-yl)methyl)-1-oxoisoindolin-2-yl)piperidine-2,6-dione |
| GT-04454 | 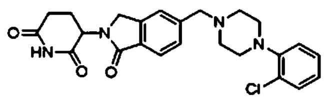 | 3-(5-((4-(2-chlorophenyl)piperazin-1-yl)methyl)-1-oxoisoindolin-2-yl)piperidine-2,6-dione | 3-(5-((4-(2-chlorophenyl)piperazin-1-yl)methyl)-1-oxoisoindolin-2-yl)piperidine-2,6-dione |
| GT-04455 | 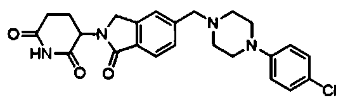 | 3-(5-((4-(4-chlorophenyl)piperazin-1-yl)methyl)-1-oxoisoindolin-2-yl)piperidine-2,6-dione | 3-(5-((4-(4-chlorophenyl)piperazin-1-yl)methyl)-1-oxoisoindolin-2-yl)piperidine-2,6-dione |
| GT-04456 | 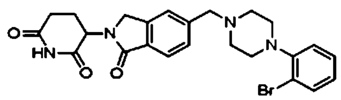 | 3-(5-((4-(2-bromophenyl)piperazin-1-yl)methyl)-1-oxoisoindolin-2-yl)piperidine-2,6-dione | 3-(5-((4-(2-bromophenyl)piperazin-1-yl)methyl)-1-oxoisoindolin-2-yl)piperidine-2,6-dione |
| GT-04467 | 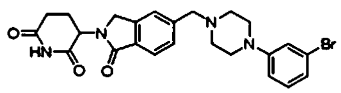 | 3-(5-((4-(3-bromophenyl)piperazin-1-yl)methyl)-1-oxoisoindolin-2-yl)piperidine-2,6-dione | 3-(5-((4-(3-bromophenyl)piperazin-1-yl)methyl)-1-oxoisoindolin-2-yl)piperidine-2,6-dione |
| GT-04485 | 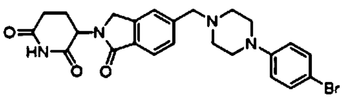 | 3-(5-((4-(4-bromophenyl)piperazin-1-yl)methyl)-1-oxoisoindolin-2-yl)piperidine-2,6-dione | 3-(5-((4-(4-bromophenyl)piperazin-1-yl)methyl)-1-oxoisoindolin-2-yl)piperidine-2,6-dione |
| GT-04461 | 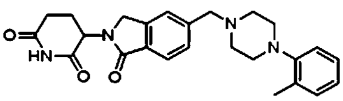 | 3-(1-oxo-5-((4-(o-tolyl)piperazin-1-yl)methyl)isoindolin-2-yl)piperidine-2,6-dione | 3-(1-oxo-5-((4-(o-tolyl)piperazin-1-yl)methyl)isoindolin-2-yl)piperidine-2,6-dione |
| GT-04462 | 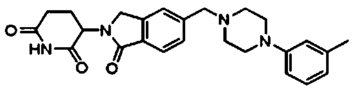 | 3-(1-oxo-5-((4-(m-tolyl)piperazin-1-yl)methyl)isoindolin-2-yl)piperidine-2,6-dione | 3-(1-oxo-5-((4-(m-tolyl)piperazin-1-yl)methyl)isoindolin-2-yl)piperidine-2,6-dione |
| GT-04463 | 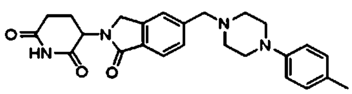 | 3-(1-oxo-5-((4-(p-tolyl)piperazin-1-yl)methyl)isoindolin-2-yl)piperidine-2,6-dione | 3-(1-oxo-5-((4-(p-tolyl)piperazin-1-yl)methyl)isoindolin-2-yl)piperidine-2,6-dione |

(continued)

| Compound No. | Structure of the compounds | The compounds' name | Chinese translation of the compounds' name |
|---|---|---|---|
| GT-04464 | 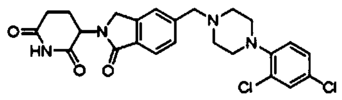 | 3-(5-((4-(2,4-dichlorophenyl)piperazin-1-yl)methyl)-1-oxoisoindolin-2-yl)piperidine-2,6-dione | 3-(5-((4-(2,4-dichlorophenyl)piperazin-1-yl)methyl)-1-oxoisoindolin-2-yl)piperidine-2,6-dione |
| GT-04487 | 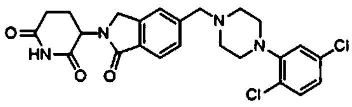 | 3-(5-((4-(2,5-dichlorophenyl)piperazin-1-yl)methyl)-1-oxoisoindolin-2-yl)piperidine-2,6-dione | 3-(5-((4-(2,5-dichlorophenyl)piperazin-1-yl)methyl)-1-oxoisoindolin-2-yl)piperidine-2,6-dione |
| GT-04613 | 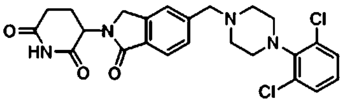 | 3-(5-((4-(2,6-dichlorophenyl)piperazin-1-yl)methyl)-1-oxoisoindolin-2-yl)piperidine-2,6-dione | 3-(5-((4-(2,6-dichlorophenyl)piperazin-1-yl)methyl)-1-oxoisoindolin-2-yl)piperidine-2,6-dione |
| GT-04437 | 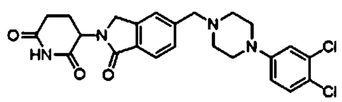 | 3-(5-((4-(3,4-dichlorophenyl)piperazin-1-yl)methyl)-1-oxoisoindolin-2-yl)piperidine-2,6-dione | 3-(5-((4-(3,4-dichlorophenyl)piperazin-1-yl)methyl)-1-oxoisoindolin-2-yl)piperidine-2,6-dione |
| GT-04614 | 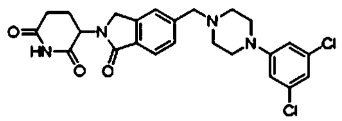 | 3-(5-((4-(3,5-dichlorophenyl)piperazin-1-yl)methyl)-1-oxoisoindolin-2-yl)piperidine-2,6-dione | 3-(5-((4-(3,5-dichlorophenyl)piperazin-1-yl)methyl)-1-oxoisoindolin-2-yl)piperidine-2,6-dione |
| GT-04515 | 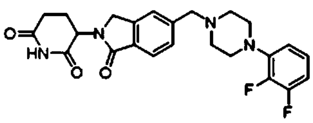 | 3-(5-((4-(2,3-difluorophenyl)piperazin-1-yl)methyl)-1-oxoisoindolin-2-yl)piperidine-2,6-dione | 3-(5-((4-(2,3-difluorophenyl)piperazin-1-yl)methyl)-1-oxoisoindolin-2-yl)piperidine-2,6-dione |
| GT-04438 | 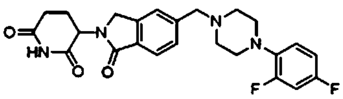 | 3-(5-((4-(2,4-difluorophenyl)piperazin-1-yl)methyl)-1-oxoisoindolin-2-yl)piperidine-2,6-dione | 3-(5-((4-(2,4-difluorophenyl)piperazin-1-yl)methyl)-1-oxoisoindolin-2-yl)piperidine-2,6-dione |
| GT-04539 | 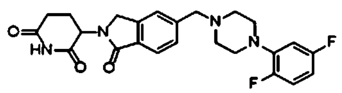 | 3-(5-((4-(2,5-difluorophenyl)piperazin-1-yl)methyl)-1-oxoisoindolin-2-yl)piperidine-2,6-dione | 3-(5-((4-(2,5-difluorophenyl)piperazin-1-yl)methyl)-1-oxoisoindolin-2-yl)piperidine-2,6-dione |
| GT-04540 | 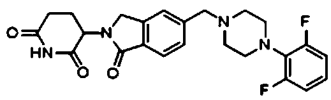 | 3-(5-((4-(2,6-difluorophenyl)piperazin-1-yl)methyl)-1-oxoisoindolin-2-yl)piperidine-2,6-dione | 3-(5-((4-(2,6-difluorophenyl)piperazin-1-yl)methyl)-1-oxoisoindolin-2-yl)piperidine-2,6-dione |
| GT-04529 | 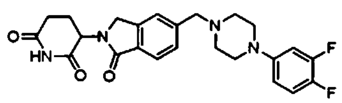 | 3-(5-((4-(3,4-difluorophenyl)piperazin-1-yl)methyl)-1-oxoisoindolin-2-yl)piperidine-2,6-dione | 3-(5-((4-(3,4-difluorophenyl)piperazin-1-yl)methyl)-1-oxoisoindolin-2-yl)piperidine-2,6-dione |
| GT-04496 | 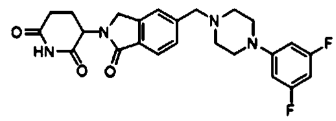 | 3-(5-((4-(3,5-difluorophenyl)piperazin-1-yl)methyl)-1-oxoisoindolin-2-yl)piperidine-2,6-dione | 3-(5-((4-(3,5-difluorophenyl)piperazin-1-yl)methyl)-1-oxoisoindolin-2-yl)piperidine-2,6-dione |
| GT-04439 | 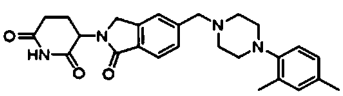 | 3-(5-((4-(2,4-dimethylphenyl)piperazin-1-yl)methyl)-1-oxoisoindolin-2-yl)piperidine-2,6-dione | 3-(5-((4-(2,4-dimethylphenyl)piperazin-1-yl)methyl)-1-oxoisoindolin-2-yl)piperidine-2,6-dione |
| GT-04440 | 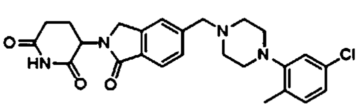 | 3-(5-((4-(5-chloro-2-methylphenyl)piperazin-1-yl)methyl)-1-oxoisoindolin-2-yl)piperidine-2,6-dione | 3-(5-((4-(5-chloro-2-methylphenyl)piperazin-1-yl)methyl)-1-oxoisoindolin-2-yl)piperidine-2,6-dione |

(continued)

| Compo und No. | Structure of the compounds | The compounds' name | Chinese translation of the compounds' name |
|---|---|---|---|
| GT-04465 | 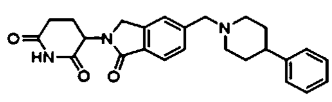 | 3-(1-oxo-5-((4-phenylpiperidin-1-yl)methyl)isoindolin-2-yl) piperidine-2,6-dione | 3-(1-oxo-5-((4-phenylpiperidin-1-yl)methyl)isoindolin-2-yl) piperidine-2,6-dione |
| GT-04466 | 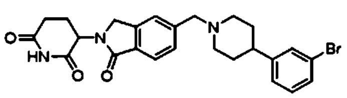 | 3-(5-((4-(3-bromophenyl)piperidin-1-yl)methyl)-1-oxoisoindolin-2-yl)piperidine-2,6-dione | 3-(5-((4-(3-bromophenyl)piperidin-1-yl)methyl)-1-oxoisoindolin-2-yl)piperidine-2,6-dione |
| GT-04493 | 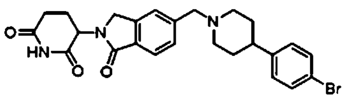 | 3-(5-((4-(4-bromophenyl)piperidin-1-yl)methyl)-1-oxoisoindolin-2-yl)piperidine-2,6-dione | 3-(5-((4-(4-bromophenyl)piperidin-1-yl)methyl)-1-oxoisoindolin-2-yl)piperidine-2,6-dione |
| GT-04468 | 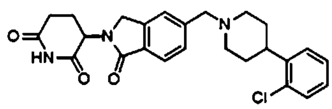 | 3-(5-((4-(2-chlorophenyl)piperidin-1-yl)methyl)-1-oxoisoindolin-2-yl)piperidine-2,6-dione | 3-(5-((4-(2-chlorophenyl)piperidin-1-yl)methyl)-1-oxoisoindolin-2-yl)piperidine-2,6-dione |
| GT-04469 | 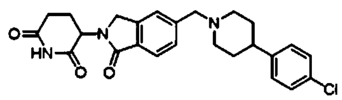 | 3-(5-((4-(4-chlorophenyl)piperidin-1-yl)methyl)-1-oxoisoindolin-2-yl)piperidine-2,6-dione | 3-(5-((4-(4-chlorophenyl)piperidin-1-yl)methyl)-1-oxoisoindolin-2-yl)piperidine-2,6-dione |
| GT-04470 | 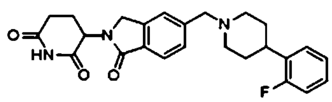 | 3-(5-((4-(2-fluorophenyl)piperidin-1-yl)methyl)-1-oxoisoindolin-2-yl)piperidine-2,6-dione | 3-(5-((4-(2-fluorophenyl)piperidin-1-yl)methyl)-1-oxoisoindolin-2-yl)piperidine-2,6-dione |
| GT-04471 | 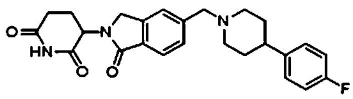 | 3-(5-((4-(4-fluorophenyl)piperidin-1-yl)methyl)-1-oxoisoindolin-2-yl)piperidine-2,6-dione | 3-(5-((4-(4-fluorophenyl)piperidin-1-yl)methyl)-1-oxoisoindolin-2-yl)piperidine-2,6-dione |
| GT-04488 | 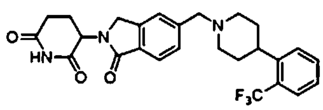 | 3-(1-oxo-5-((4-(2-(trifluoromethyl)phenyl)pip eridin-1-yl) methyl)isoindolin-2-yl)piperidine-2,6-dione | 3-(1-oxo-5-((4-(2-(trifluoromethyl)phenyl)pip eridin-1-yl) methyl)isoindolin-2-yl)piperidine-2,6-dione |
| GT-04472 | 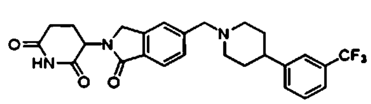 | 3-(1-oxo-5-((4-(3-(trifluoromethyl)phenyl)pip eridin-1-yl) methyl)isoindolin-2-yl)piperidine-2,6-dione | 3-(1-oxo-5-((4-(3-(trifluoromethyl)phenyl)pip eridin-1-yl) methyl)isoindolin-2-yl)piperidine-2,6-dione |
| GT-04551 | 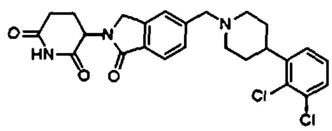 | 3-(5-((4-(2,3-dichlorophenyl)piperidin-1-yl)methyl)-1-oxoisoindolin-2-yl)piperidine-2,6-dione | 3-(5-((4-(2,3-dichlorophenyl)piperidin-1-yl)methyl)-1-oxoisoindolin-2-yl)piperidine-2,6-dione |
| GT-04553 | 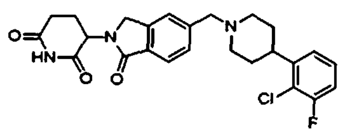 | 3-5-((4-(2-chloro-3-fluorophenyl)piperidin-1-yl)methyl)-1-oxoisoindolin-2-yl)piperidine-2,6-dione | 3-(5-((4-(2-chloro-3-fluorophenyl)piperidin-1-yl)methyl)-1-oxoisoindolin-2-yl)piperidine-2,6-dione |
| GT-04552 | 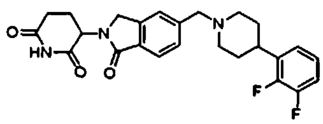 | 3-(5-((4-(2,3-difluorophenyl)piperidin-1-yl)methyl)-1-oxoisoindolin-2-yl)piperidine-2,6-dione | 3-(5-((4-(2,3-difluorophenyl)piperidin-1-yl)methyl)-1-oxoisoindolin-2-yl)piperidine-2,6-dione |
| GT-04550 | 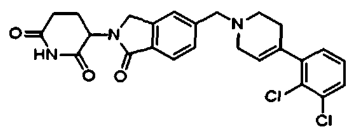 | 3-(5-((4-(2,3-dichlorophenyl)-3,6-dihydropyridin-1(2H)-yl)methyl)-1-oxoisoindolin-2-yl)piperidine-2,6-dione | 3-(5-((4-(2,3-dichlorophenyl)-3,6-dihydropyridin-1(2H)-yl) methyl)-1-oxoisoindolin-2-yl)piperidine-2,6-dione |

(continued)

| Compo und No. | Structure of the compounds | The compounds' name | Chinese translation of the compounds' name |
|---|---|---|---|
| GT-04554 | 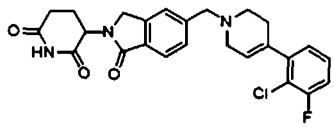 | 3-(5-((4-(2-chloro-3-fluorophenyl)-3,6-dihydropyridin-1(2H)-yl)methyl)-1-oxoisoindolin-2-yl)piperidine-2,6-dione | 3-(5-((4-(2-chloro-3-fluorophenyl)-3,6-dihydropyridin-1(2H)-yl)methyl)-1-oxoisoindolin-2-yl)piperidine-2,6-dione |
| GT-04449 | 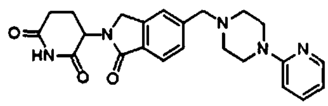 | 3-(1-oxo-5-((4-(pyridin-2-yl)piperazin-1-yl)methyl)isoindolin-2-yl)piperidine-2,6-dione | 3-(1-oxo-5-((4-(pyridin-2-yl)piperazin-1-yl)methyl)isoindolin-2-yl)piperidine-2,6-dione |
| GT-04530 | 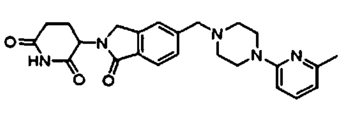 | 3-(5-((4-(6-methylpyridin-2-yl)piperazin-1-yl)methyl)-1-oxoisoindolin-2-yl)piperidine-2,6-dione | 3-(5-((4-(6-methylpyridin-2-yl)piperazin-1-yl)methyl)-1-oxoisoindolin-2-yl)piperidine-2,6-dione |
| GT-04497 | 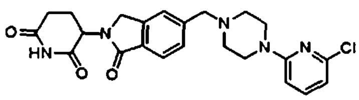 | 3-(5-((4-(6-chloropyridin-2-yl)piperazin-1-yl)methyl)-1-oxoisoindolin-2-yl)piperidine-2,6-dione | 3-(5-((4-(6-chloropyridin-2-yl)piperazin-1-yl)methyl)-1-oxoisoindolin-2-yl)piperidine-2,6-dione |
| GT-04431 | 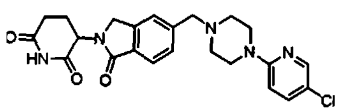 | 3-(5-((4-(5-chloropyridin-2-yl)piperazin-1-yl)methyl)-1-oxoisoindolin-2-yl)piperidine-2,6-dione | 3-(5-((4-(5-chloropyridin-2-yl)piperazin-1-yl)methyl)-1-oxoisoindolin-2-yl)piperidine-2,6-dione |
| GT-04430 | 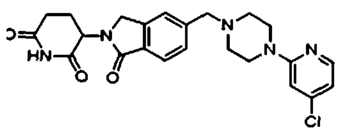 | 3-(5-((4-(4-chloropyridin-2-yl)piperazin-1-yl)methyl)-1-oxoisoindolin-2-yl)piperidine-2,6-dione | 3-(5-((4-(4-chloropyridin-2-yl)piperazin-1-yl)methyl)-1-oxoisoindolin-2-yl)piperidine-2,6-dione |
| GT-04428 | 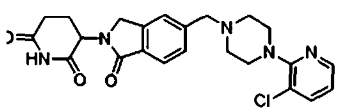 | 3-(5-((4-(3-chloropyridin-2-yl)piperazin-1-yl)methyl)-1-oxoisoindolin-2-yl)piperidine-2,6-dione | 3-(5-((4-(3-chloropyridin-2-yl)piperazin-1-yl)methyl)-1-oxoisoindolin-2-yl)piperidine-2,6-dione |
| GT-04578 | 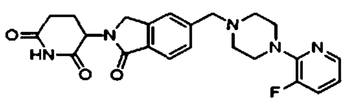 | 3-(5-((4-(3-fluoropyridin-2-yl)piperazin-1-yl)methyl)-1-oxoisoindolin-2-yl)piperidine-2,6-dione | 3-(5-((4-(3-fluoropyridin-2-yl)piperazin-1-yl)methyl)-1-oxoisoindolin-2-yl)piperidine-2,6-dione |
| GT-04498 | 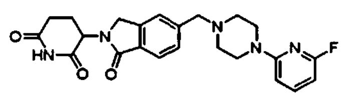 | 3-(5-((4-(6-fluoropyridin-2-yl)piperazin-1-yl)methyl)-1-oxoisoindolin-2-yl)piperidine-2,6-dione | 3-(5-((4-(6-fluoropyridin-2-yl)piperazin-1-yl)methyl)-1-oxoisoindolin-2-yl)piperidine-2,6-dione |
| GT-04531 | 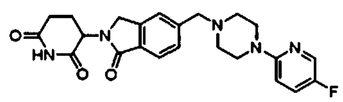 | 3-(5-((4-(5-fluoropyridin-2-yl)piperazin-1-yl)methyl)-1-oxoisoindolin-2-yl)piperidine-2,6-dione | 3-(5-((4-(5-fluoropyridin-2-yl)piperazin-1-yl)methyl)-1-oxoisoindolin-2-yl)piperidine-2,6-dione |
| GT-04434 | 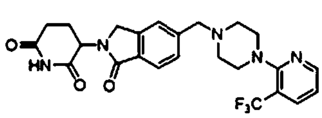 | 3-(1-oxo-5-((4-(3-(trifluoromethyl)pyridin-2-yl)piperazin-1-yl)methyl)isoindolin-2-yl)piperidine-2,6-dione | 3-(1-oxo-5-((4-(3-(trifluoromethyl)pyridin-2-yl)piperazin-1-yl)methyl)isoindolin-2-yl)piperidine-2,6-dione |
| GT-04432 | 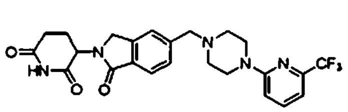 | 3-(1-oxo-5-((4-(6-(trifluoromethyl)pyridin-2-yl)piperazin-1-yl)methyl)isoindolin-2-yl)piperidine-2,6-dione | 3-(1-oxo-5-((4-(6-(trifluoromethyl)pyridin-2-yl)piperazin-1-yl)methyl)isoindolin-2-yl)piperidine-2,6-dione |
| GT-04433 | 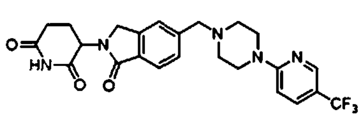 | 3-(1-oxo-5-((4-(5-(trifluoromethyl)pyridin-2-yl)piperazin-1-yl)methyl)isoindolin-2-yl)piperidine-2,6-dione | 3-(1-oxo-5-((4-(5-(trifluoromethyl)pyridin-2-yl)piperazin-1-yl)methyl)isoindolin-2-yl)piperidine-2,6-dione |

(continued)

| Compound No. | Structure of the compounds | The compounds' name | Chinese translation of the compounds' name |
|---|---|---|---|
| GT-04532 | 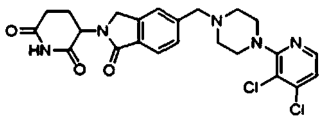 | 3-(5-((4-(3,4-dichloropyridin-2-yl)piperazin-1-yl)methyl)-1-oxoisoindolin-2-yl)piperidine-2,6-dione | 3-(5-((4-(3,4-dichloropyridin-2-yl)piperazin-1-yl)methyl)-1-oxoisoindolin-2-yl)piperidine-2,6-dione |
| GT-04479 | 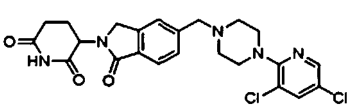 | 3-(5-((4-(3,5-dichloropyridin-2-yl)piperazin-1-yl)methyl)-1-oxoisoindolin-2-yl)piperidine-2,6-dione | 3-(5-((4-(3,5-dichloropyridin-2-yl)piperazin-1-yl)methyl)-1-oxoisoindolin-2-yl)piperidine-2,6-dione |
| GT-04533 | 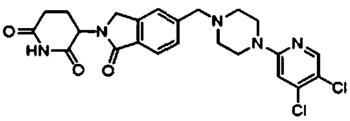 | 3-(5-((4-(4,5-dichloropyridin-2-yl)piperazin-1-yl)methyl)-1-oxoisoindolin-2-yl)piperidine-2,6-dione | 3-(5-((4-(4,5-dichloropyridin-2-yl)piperazin-1-yl)methyl)-1-oxoisoindolin-2-yl)piperidine-2,6-dione |
| GT-04579 | 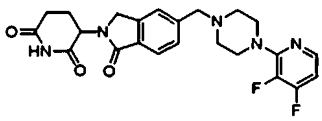 | 3-(5-((4-(3,4-difluoropyridin-2-yl)piperazin-1-yl)methyl)-1-oxoisoindolin-2-yl)piperidine-2,6-dione | 3-(5-((4-(3,4-difluoropyridin-2-yl)piperazin-1-yl)methyl)-1-oxoisoindolin-2-yl)piperidine-2,6-dione |
| GT-04610 | 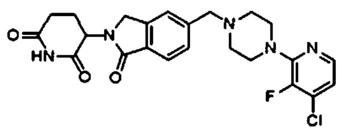 | 3-(5-((4-(4-chloro-3-fluoropyridin-2-yl)piperazin-1-yl)methyl)-1-oxoisoindolin-2-yl)piperidine-2,6-dione | 3-(5-((4-(4-chloro-3-fluoropyridin-2-yl)piperazin-1-yl)methyl)-1-oxoisoindolin-2-yl)piperidine-2,6-dione |
| GT-04557 | 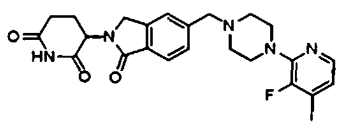 | 3-(5-((4-(3-fluoro-4-iodopyridin-2-yl)piperazin-1-yl)methyl)-1-oxoisoindolin-2-yl)piperidine-2,6-dione | 3-(5-((4-(3-fluoro-4-iodopyridin-2-yl)piperazin-1-yl)methyl)-1-oxoisoindolin-2-yl)piperidine-2,6-dione |
| GT-04615 | 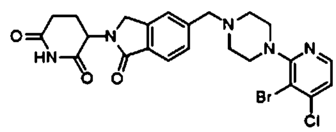 | 3-(5-((4-(3-bromo-4-chloropyridin-2-yl)piperazin-1-yl)methyl)-1-oxoisoindolin-2-yl)piperidine-2,6-dione | 3-(5-((4-(3-bromo-4-chloropyridin-2-yl)piperazin-1-yl)methyl)-1-oxoisoindolin-2-yl)piperidine-2,6-dione |
| GT-04480 | 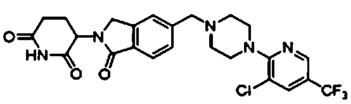 | 3-(5-((4-(3-chloro-5-(trifluoromethyl)pyridin-2-yl)piperazin-1-yl)methyl)-1-oxoisoindolin-2-yl)piperidine-2,6-dione | 3-(5-((4-(3-chloro-5-(trifluoromethyl)pyridin-2-yl)piperazin-1-yl)methyl)-1-oxoisoindolin-2-yl)piperidine-2,6-dione |
| GT-04491 | 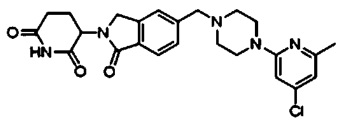 | 3-(5-((4-(4-chloro-6-methylpyridin-2-yl)piperazin-1-yl)methyl)-1-oxoisoindolin-2-yl)piperidine-2,6-dione | 3-(5-((4-(4-chloro-6-methylpyridin-2-yl)piperazin-1-yl)methyl)-1-oxoisoindolin-2-yl)piperidine-2,6-dione |
| GT-04508 | 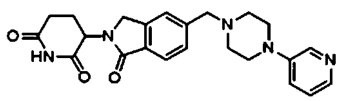 | 3-(1-oxo-5-((4-(pyridin-3-yl)piperazin-1-yl)methyl)isoindolin-2-yl)piperidine-2,6-dione | 3-(1-oxo-5-((4-(pyridin-3-yl)piperazin-1-yl)methyl)isoindolin-2-yl)piperidine-2,6-dione |
| GT-04537 | 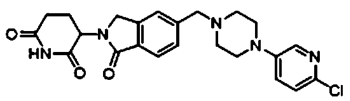 | 3-(5-((4-(6-chloropyridin-3-yl)piperazin-1-yl)methyl)-1-oxoisoindolin-2-yl)piperidine-2,6-dione | 3-(5-((4-(6-chloropyridin-3-yl)piperazin-1-yl)methyl)-1-oxoisoindolin-2-yl)piperidine-2,6-dione |
| GT-04538 | 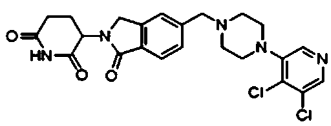 | 3-(5-((4-(4,5-dichloropyridin-3-yl)piperazin-1-yl)methyl)-1-oxoisoindolin-2-yl)piperidine-2,6-dione | 3-(5-((4-(4,5-dichloropyridin-3-yl)piperazin-1-yl)methyl)-1-oxoisoindolin-2-yl)piperidine-2,6-dione |

(continued)

| Compound No. | Structure of the compounds | The compounds' name | Chinese translation of the compounds' name |
|---|---|---|---|
| GT-04896 | 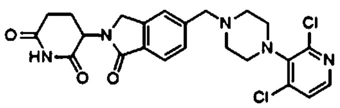 | 3-(5-((4-(2,4-dichloropyridin-3-yl)piperazin-1-yl)methyl)-1-oxoisoindolin-2-yl)piperidine-2,6-dione | 3-(5-((4-(2,4-dichloropyridin-3-yl)piperazin-1-yl)methyl)-1-oxoisoindolin-2-yl)piperidine-2,6-dione |
| GT-04450 | 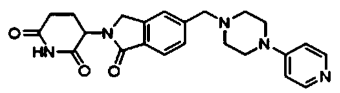 | 3-1-oxo-5-((4-(pyridin-4-yl)piperazin-1-yl)methyl)isoindolin-2-yl)piperidine-2,6-dione | 3-(1-oxo-5-((4-(pyridin-4-yl)piperazin-1-yl)methyl)isoindolin-2-yl)piperidine-2,6-dione |
| GT-04541 | 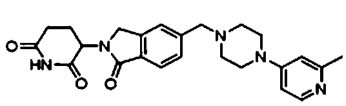 | 3-(5-((4-(2-methylpyridin-4-yl)piperazin-1-yl)methyl)-1-oxoisoindolin-2-yl)piperidine-2,6-dione | 3-(5-((4-(2-methylpyridin-4-yl)piperazin-1-yl)methyl)-1-oxoisoindolin-2-yl)piperidine-2,6-dione |
| GT-04580 | 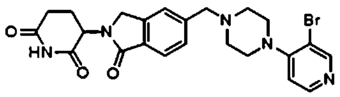 | 3-(5-((4-(3-bromopyridin-4-yl)piperazin-1-yl)methyl)-1-oxoisoindolin-2-yl)piperidine-2,6-dione | 3-(5-((4-(3-bromopyridin-4-yl)piperazin-1-yl)methyl)-1-oxoisoindolin-2-yl)piperidine-2,6-dione |
| GT-04581 | 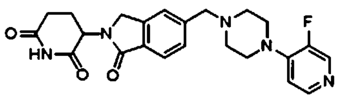 | 3-(5-((4-(3-fluoropyridin-4-yl)piperazin-1-yl)methyl)-1-oxoisoindolin-2-yl)piperidine-2,6-dione | 3-(5-((4-(3-fluoropyridin-4-yl)piperazin-1-yl)methyl)-1-oxoisoindolin-2-yl)piperidine-2,6-dione |
| GT-04481 | 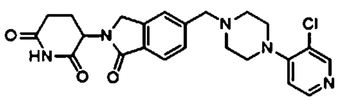 | 3-(5-((4-(3-chloropyridin-4-yl)piperazin-1-yl)methyl)-1-oxoisoindolin-2-yl)piperidine-2,6-dione | 3-(5-((4-(3-chloropyridin-4-yl)piperazin-1-yl)methyl)-1-oxoisoindolin-2-yl)piperidine-2,6-dione |
| GT-04435 | 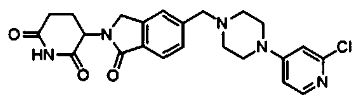 | 3-(5-((4-(2-chloropyridin-4-yl)piperazin-1-yl)methyl)-1-oxoisoindolin-2-yl)piperidine-2,6-dione | 3-(5-((4-(2-chloropyridin-4-yl)piperazin-1-yl)methyl)-1-oxoisoindolin-2-yl)piperidine-2,6-dione |
| GT-04612 | 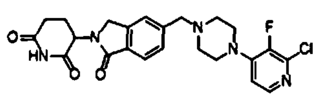 | 3-(5-((4-(2-chloro-3-fluoropyridin-4-yl)piperazin-1-yl) methyl)-1-oxoisoindolin-2-yl)piperidine-2,6-dione | 3-(5-((4-(2-chloro-3-fluoropyridin-4-yl)piperazin-1-yl) methyl)-1-oxoisoindolin-2-yl)piperidine-2,6-dione |
| GT-04928 | 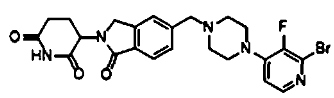 | 3-(5-((4-(2-bromo-3-fluoropyridin-4-yl)piperazin-1-yl) methyl)-1-oxoisoindolin-2-yl)piperidine-2,6-dione | 3-(5-((4-(2-bromo-3-fluoropyridin-4-yl)piperazin-1-yl) methyl)-1-oxoisoindolin-2-yl)piperidine-2,6-dione |
| GT-04616 | 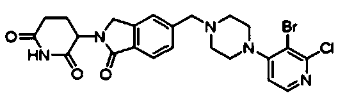 | 3-(5-((4-(3-bromo-2-chloropyridin-4-yl)piperazin-1-yl) methyl)-1-oxoisoindolin-2-yl)piperidine-2,6-dione | 3-(5-((4-(3-bromo-2-chloropyridin-4-yl)piperazin-1-yl) methyl)-1-oxoisoindolin-2-yl)piperidine-2,6-dione |
| GT-04542 | 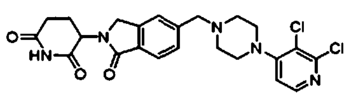 | 3-(5-((4-(2,3-dichloropyridin-4-yl)piperazin-1-yl)methyl)-1-oxoisoindolin-2-yl)piperidine-2,6-dione | 3-(5-((4-(2,3-dichloropyridin-4-yl)piperazin-1-yl)methyl)-1-oxoisoindolin-2-yl)piperidine-2,6-dione |
| GT-04543 | 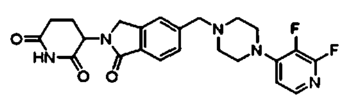 | 3-(5-((4-(2,3-difluoropyridin-4-yl)piperazin-1-yl)methyl)-1-oxoisoindolin-2-yl)piperidine-2,6-dione | 3-(5-((4-(2,3-difluoropyridin-4-yl)piperazin-1-yl)methyl)-1-oxoisoindolin-2-yl)piperidine-2,6-dione |
| GT-04929 | 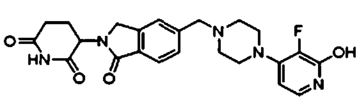 | 3-(5-((4-(3-fluoro-2-hydroxypyridin-4-yl)piperazin-1-yl) methyl)-1-oxoisoindolin-2-yl)piperidine-2,6-dione | 3-(5-((4-(3-fluoro-2-hydroxypyridin-4-yl)piperazin-1-yl) methyl)-1-oxoisoindolin-2-yl)piperidine-2,6-dione |

(continued)

| Compound No. | Structure of the compounds | The compounds' name | Chinese translation of the compounds' name |
|---|---|---|---|
| GT-04600 | 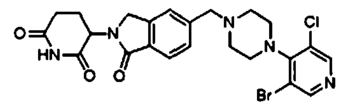 | 3-(5-((4-(3-bromo-5-chloropyridin-4-yl)piperazin-1-yl)methyl)-1-oxoisoindolin-2-yl)piperidine-2,6-dione | 3-(5-((4-(3-bromo-5-chloropyridin-4-yl)piperazin-1-yl)methyl)-1-oxoisoindolin-2-yl)piperidine-2,6-dione |
| GT-04611 | 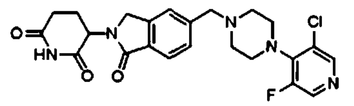 | 3-(5-((4-(3-chloro-5-fluoropyridin-4-yl)piperazin-1-yl)methyl)-1-oxoisoindolin-2-yl)piperidine-2,6-dione | 3-(5-((4-(3-chloro-5-fluoropyridin-4-yl)piperazin-1-yl)methyl)-1-oxoisoindolin-2-yl)piperidine-2,6-dione |
| GT-04436 | 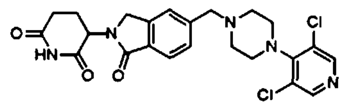 | 3-(5-((4-(3,5-dichloropyridin-4-yl)piperazin-1-yl)methyl)-1-oxoisoindolin-2-yl)piperidine-2,6-dione | 3-(5-((4-(3,5-dichloropyridin-4-yl)piperazin-1-yl)methyl)-1-oxoisoindolin-2-yl)piperidine-2,6-dione |
| GT-04492 | 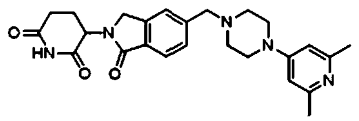 | 3-(5-((4-(2,6-dimethylpyridin-4-yl)piperazin-1-yl)methyl)-1-oxoisoindolin-2-yl)piperidine-2,6-dione | 3-(5-((4-(2,6-dimethylpyridin-4-yl)piperazin-1-yl)methyl)-1-oxoisoindolin-2-yl)piperidine-2,6-dione |
| GT-04499 | 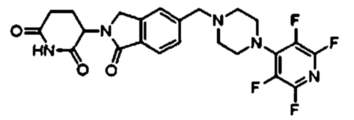 | 3-(1-oxo-5-((4-(perfluoropyridin-4-yl)piperazin-1-yl)methyl)isoindolin-2-yl)piperidine-2,6-dione | 3-(1-oxo-5-((4-(perfluoropyridin-4-yl)piperazin-1-yl)methyl)isoindolin-2-yl)piperidine-2,6-dione |
| GT-04473 | 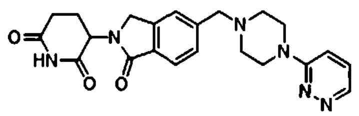 | 3-(1-oxo-5-((4-(pyridazin-3-yl)piperazin-1-yl)methyl)isoindolin-2-yl)piperidine-2,6-dione | 3-(1-oxo-5-((4-(pyridazin-3-yl)piperazin-1-yl)methyl)isoindolin-2-yl)piperidine-2,6-dione |
| GT-04482 | 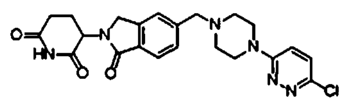 | 3-(5-((4-(6-chloropyridazin-3-yl)piperazin-1-yl)methyl)-1-oxoisoindolin-2-yl)piperidine-2,6-dione | 3-(5-((4-(6-chloropyridazin-3-yl)piperazin-1-yl)methyl)-1-oxoisoindolin-2-yl)piperidine-2,6-dione |
| GT-04484 | 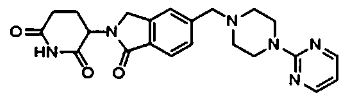 | 3-(1-oxo-5-((4-(pyrimidin-2-yl)piperazin-1-yl)methyl)isoindolin-2-yl)piperidine-2,6-dione | 3-(1-oxo-5-((4-(pyrimidin-2-yl)piperazin-1-yl)methyl)isoindolin-2-yl)piperidine-2,6-dione |
| GT-04483 | 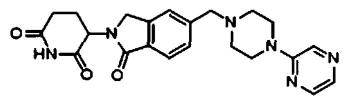 | 3-(1-oxo-5-((4-(pyrazin-2-yl)piperazin-1-yl)methyl)isoindolin-2-yl)piperidine-2,6-dione | 3-(1-oxo-5-((4-(pyrazin-2-yl)piperazin-1-yl)methyl)isoindolin-2-yl)piperidine-2,6-dione |
| GT-04501 | 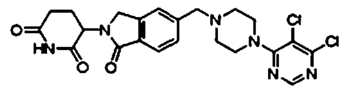 | 3-(5-((4-(5,6-dichloropyrimidin-4-yl)piperazin-1-yl)methyl)-1-oxoisoindolin-2-yl)piperidine-2,6-dione | 3-(5-((4-(5,6-dichloropyrimidin-4-yl)piperazin-1-yl)methyl)-1-oxoisoindolin-2-yl)piperidine-2,6-dione |
| GT-04500 | 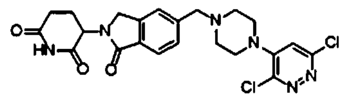 | 3-(5-((4-(3,6-dichloropyridazin-4-yl)piperazin-1-yl)methyl)-1-oxoisoindolin-2-yl)piperidine-2,6-dione | 3-(5-((4-(3,6-dichloropyridazin-4-yl)piperazin-1-yl)methyl)-1-oxoisoindolin-2-yl)piperidine-2,6-dione |
| GT-04486 | 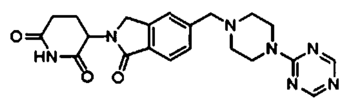 | 3-(5-((4-(1,3,5-triazin-2-yl)piperazin-1-yl)methyl)-1-oxoisoindolin-2-yl)piperidine-2,6-dione | 3-(5-((4-(1,3,5-triazin-2-yl)piperazin-1-yl)methyl)-1-oxoisoindolin-2-yl)piperidine-2,6-dione |

(continued)

| Compound No. | Structure of the compounds | The compounds' name | Chinese translation of the compounds' name |
|---|---|---|---|
| GT-04690 | 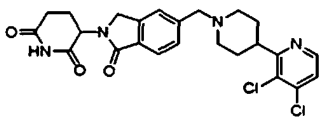 | 3-(5-((4-(3,4-dichloropyridin-2-yl)piperidin-1-yl)methyl)-1-oxoisoindolin-2-yl)piperidine-2,6-dione | 3-(5-((4-(3,4-dichloropyridin-2-yl)piperidin-1-yl)methyl)-1-oxoisoindolin-2-yl)piperidine-2,6-dione |
| GT-04560 | 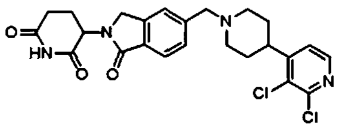 | 3-(5-((4-(2,3-dichloropyridin-4-yl)piperidin-1-yl)methyl)-1-oxoisoindolin-2-yl)piperidine-2,6-dione | 3-(5-((4-(2,3-dichloropyridin-4-yl)piperidin-1-yl)methyl)-1-oxoisoindolin-2-yl)piperidine-2,6-dione |
| GT-04556 | 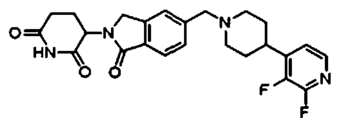 | 3-(5-((4-(2,3-difluoropyridin-4-yl)piperidin-1-yl)methyl)-1-oxoisoindolin-2-yl)piperidine-2,6-dione | 3-(5-((4-(2,3-difluoropyridin-4-yl)piperidin-1-yl)methyl)-1-oxoisoindolin-2-yl)piperidine-2,6-dione |
| GT-04591 | 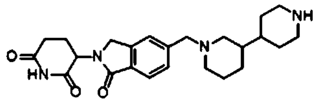 | 3-(5-([3,4'-bipiperidin]-1-ylmethyl)-1-oxoisoindolin-2-yl)piperidine-2,6-dione | 3-(5-([3,4'-bipiperidin]-1-ylmethyl)-1-oxoisoindolin-2-yl)piperidine-2,6-dione |
| GT-04570 | 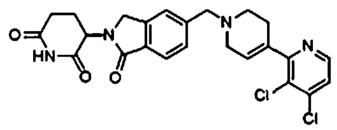 | 3-(5-((3,4-dichloro-3',6'-dihydro-[2,4'-bipyridin]-1'(2'H)-yl)methyl)-1-oxoisoindolin-2-yl)piperidine-2,6-dione | 3-(5-((3,4-dichloro-3',6'-dihydro-[2,4'-bipyridin]-1'(2'H)-yl)methyl)-1-oxoisoindolin-2-yl)piperidine-2,6-dione |
| GT-04558 | 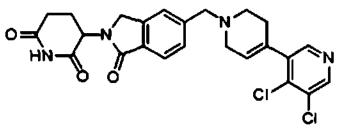 | 3-(5-((4,5-dichloro-3',6'-dihydro-[3,4'-bipyridin]-1'(2'H)-yl)methyl)-1-oxoisoindolin-2-yl)piperidine-2,6-dione | 3-(5-((4,5-dichloro-3',6'-dihydro-[3,4'-bipyridin]-1'(2'H)-yl)methyl)-1-oxoisoindolin-2-yl)piperidine-2,6-dione |
| GT-04620 | 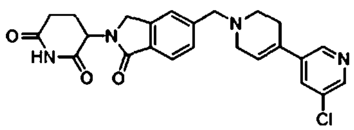 | 3-(5-((5-chloro-3',6'-dihydro-[3,4'-bipyridin]-1'(2'H)-yl)methyl)-1-oxoisoindolin-2-yl)piperidine-2,6-dione | 3-(5-((5-chloro-3',6'-dihydro-[3,4'-bipyridin]-1'(2'H)-yl)methyl)-1-oxoisoindolin-2-yl)piperidine-2,6-dione |
| GT-04559 | 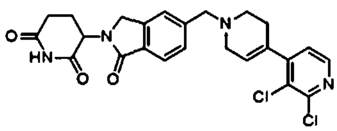 | 3-(5-((2',3'-dichloro-3,6-dihydro-[4,4'-bipyridin]-1(2H)-yl)methyl)-1-oxoisoindolin-2-yl)piperidine-2,6-dione | 3-(5-((2',3'-dichloro-3,6-dihydro-[4,4'-bipyridin]-1(2H)-yl)methyl)-1-oxoisoindolin-2-yl)piperidine-2,6-dione |
| GT-04689 | 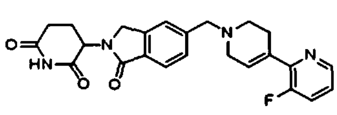 | 3-(5-((3-fluoro-3',6'-dihydro-[2,4'-bipyridin]-1'(2'H)-yl)methyl)-1-oxoisoindolin-2-yl)piperidine-2,6-dione | 3-(5-((3-fluoro-3',6'-dihydro-[2,4'-bipyridin]-1'(2'H)-yl)methyl)-1-oxoisoindolin-2-yl)piperidine-2,6-dione |
| GT-04555 | 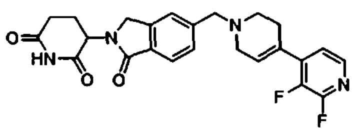 | 3-(5-((2',3'-difluoro-3,6-dihydro-[4,4'-bipyridin]-1(2H)-yl)methyl)-1-oxoisoindolin-2-yl)piperidine-2,6-dione | 3-(5-((2',3'-difluoro-3,6-dihydro-[4,4'-bipyridin]-1(2H)-yl)methyl)-1-oxoisoindolin-2-yl)piperidine-2,6-dione |
| GT-04712 | 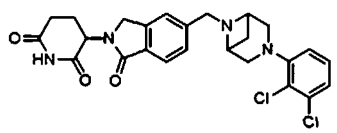 | 3-(5-((3-(2,3-dichlorophenyl)-3,6-diazabicyclo[3.1.1]heptan-6-yl)methyl)-1-oxoisoindolin-2-yl)piperidine-2,6-dione | 3-(5-((3-(2,3-dichlorophenyl)-3,6-diazabicyclo[3.1.1]heptan-6-yl)methyl)-1-oxoisoindolin-2-yl)piperidine-2,6-dione |

(continued)

| Compo und No. | Structure of the compounds | The compounds' name | Chinese translation of the compounds' name |
|---|---|---|---|
| GT-04897 | 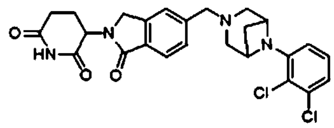 | 3-(5-((6-(2,3-dichlorophenyl)-3,6-diazabicyclo[3.1.1]heptan-3-yl)methyl)-1-oxoisoindolin-2-yl)piperidine-2,6-dione | 3-(5-((6-(2,3-dichlorophenyl)-3,6-diazabicyclo[3.1.1]heptan-3-yl)methyl)-1-oxoisoindolin-2-yl)piperidine-2,6-dione |
| GT-04724 | 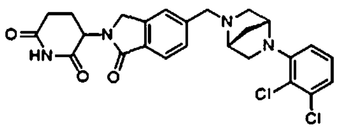 | 3-(5-(((1R,4R)-5-(2,3-dichlorophenyl)-2,5-diazabicyclo[2.2. 1]heptan-2-yl)methyl)-1-oxoisoindolin-2-yl)piperidine-2,6-dione | 3-(5-(((1R,4R)-5-(2,3-dichlorophenyl)-2,5-diazabicyclo[2.2.1]heptan-2-yl)methyl)-1-oxoisoindolin-2-yl)piperidine-2,6-dione |
| GT-04898 | 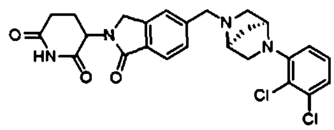 | 3-(5-(((1S,4S)-5-(2,3-dichlorophenyl)-2,5-diazabicyclo[2.2. 1]heptan-2-yl)methyl)-1-oxoisoindolin-2-yl)piperidine-2,6-dione | 3-(5-(((1S,4S)-5-(2,3-dichlorophenyl)-2,5-diazabicyclo[2.2.1]heptan-2-yl)methyl)-1-oxoisoindolin-2-yl)piperidine-2,6-dione |
| GT-04930 | 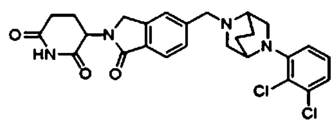 | 3-(5-((5-(2,3-dichlorophenyl)-2,5-diazabicyclo[2.2.2]octan-2-yl)methyl)-1-oxoisoindolin-2-yl)piperidine-2,6-dione | 3-(5-((5-(2,3-dichlorophenyl)-2,5-diazabicyclo[2.2.2]octan-2-yl)methyl)-1-oxoisoindolin-2-yl)piperidine-2,6-dione |
| GT-04899 | 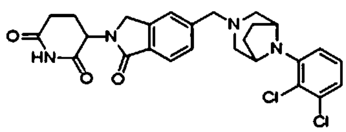 | 3-(5-((8-(2,3-dichlorophenyl)-3,8-diazabicyclo[3.2.1]octan-3-yl)methyl)-1-oxoisoindolin-2-yl)piperidine-2,6-dione | 3-(5-((8-(2,3-dichlorophenyl)-3,8-diazabicyclo[3.2.1]octan-3-yl)methyl)-1-oxoisoindolin-2-yl)piperidine-2,6-dione |
| GT-04855 | 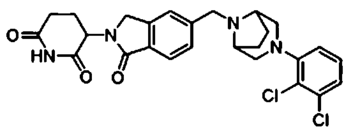 | 3-(5-((3-(2,3-dichlorophenyl)-3,8-diazabicyclo[3.2.1]octan-8-yl)methyl)-1-oxoisoindolin-2-yl)piperidine-2,6-dione | 3-(5-((3-(2,3-dichlorophenyl)-3,8-diazabicyclo[3.2.1]octan-8-yl)methyl)-1-oxoisoindolin-2-yl)piperidine-2,6-dione |
| GT-04856 | 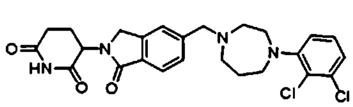 | 3-(5-((4-(2,3-dichlorophenyl)-1,4-diazepan-1-yl)methyl)-1-oxoisoindolin-2-yl)piperidine-2,6-dione | 3-(5-((4-(2,3-dichlorophenyl)-1,4-diazepan-1-yl)methyl)-1-oxoisoindolin-2-yl)piperidine-2,6-dione |
| GT-04254 | 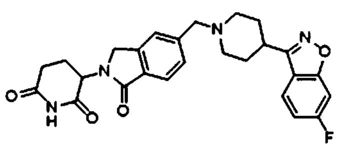 | 3-(5-((4-(6-fluorobenzo[d]isoxazol-3-yl)piperidin-1-yl)methyl)-1-oxoisoindolin-2-yl)piperidine-2,6-dione | 3-(5-((4-(6-fluorobenzo[d]isoxazol-3-yl)piperidin-1-yl)methyl)-1-oxoisoindolin-2-yl)piperidine-2,6-dione |
| GT-04907 | 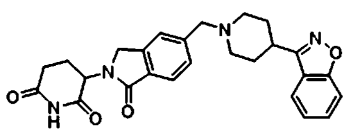 | 3-(5-((4-(benzo[d]isoxazol-3-yl)piperidin-1-yl)methyl)-1-oxoisoindolin-2-yl)piperidine-2,6-dione | 3-(5-((4-(benzo[d]isoxazol-3-yl)piperidin-1-yl)methyl)-1-oxoisoindolin-2-yl)piperidine-2,6-dione |
| GT-04715 | 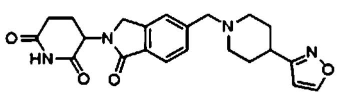 | 3-(5-((4-(isoxazol-3-yl)piperidin-1-yl)methyl)-1-oxoisoindolin-2-yl)piperidine-2,6-dione | 3-(5-((4-(isoxazol-3-yl)piperidin-1-yl)methyl)-1-oxoisoindolin-2-yl)piperidine-2,6-dione |
| GT-04667 | 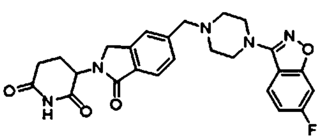 | 3-(5-((4-(6-fluorobenzo[d]isoxazol-3-yl)piperazin-1-yl)methyl)-1-oxoisoindolin-2-yl)piperidine-2,6-dione | 3-(5-((4-(6-fluorobenzo[d]isoxazol-3-yl)piperazin-1-yl)methyl)-1-oxoisoindolin-2-yl)piperidine-2,6-dione |

(continued)

| Compo und No. | Structure of the compounds | The compounds' name | Chinese translation of the compounds' name |
|---|---|---|---|
| GT-04904 | 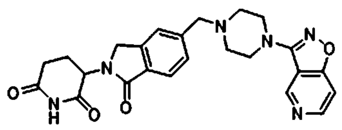 | 3-(5-((4-(isoxazolo[4,5-c]pyridin-3-yl)piperazin-1-yl)methyl)-1-oxoisoindolin-2-yl)piperidine-2,6-dione | 3-(5-((4-(isoxazolo[4,5-c]pyridin-3-yl)piperazin-1-yl)methyl)-1-oxoisoindolin-2-yl)piperidine-2,6-dione |
| GT-05332 | 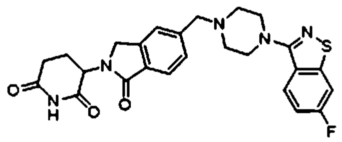 | 3-(5-((4-(6-fluorobenzo[d]isothiazol-3-yl)piperazin-1-yl)methyl)-1-oxoisoindolin-2-yl)piperidine-2,6-dione | 3-(5-((4-(6-fluorobenzo[d]isothiazol-3-yl)piperazin-1-yl)methyl)-1-oxoisoindolin-2-yl)piperidine-2,6-dione |
| GT-05264 | 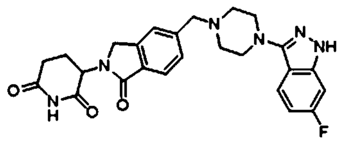 | 3-(5-((4-(6-fluoro-1H-indazol-3-yl)piperazin-1-yl)methyl)-1-oxoisoindolin-2-yl)piperidine-2,6-dione | 3-(5-((4-(6-fluoro-1H-indazol-3-yl)piperazin-1-yl)methyl)-1-oxoisoindolin-2-yl)piperidine-2,6-dione |
| GT-04601 | 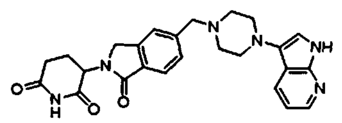 | 3-(5-((4-(1H-pyrrolo[2,3-b]pyridin-3-yl)piperazin-1-yl)methyl)-1-oxoisoindolin-2-yl)piperidine-2,6-dione | 3-(5-((4-(1H-pyrrolo[2,3-b]pyridin-3-yl)piperazin-1-yl)methyl)-1-oxoisoindolin-2-yl)piperidine-2,6-dione |
| GT-04713 | 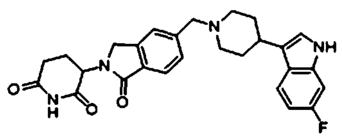 | 3-(5-((4-(6-fluoro-1H-indol-3-yl)piperidin-1-yl)methyl)-1-oxoisoindolin-2-yl)piperidine-2,6-dione | 3-(5-((4-(6-fluoro-1H-indol-3-yl)piperidin-1-yl)methyl)-1-oxoisoindolin-2-yl)piperidine-2,6-dione |
| GT-04858 | 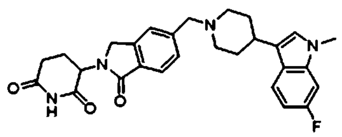 | 3-(5-((4-(6-fluoro-1-methyl-1H-indol-3-yl)piperidin-1-yl)methyl)-1-oxoisoindolin-2-yl)piperidine-2,6-dione | 3-(5-((4-(6-fluoro-1-methyl-1H-indol-3-yl)piperidin-1-yl)methyl)-1-oxoisoindolin-2-yl)piperidine-2,6-dione |
| GT-04650 | 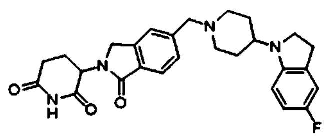 | 3-(5-((4-(5-fluoroindolin-1-yl)piperidin-1-yl)methyl)-1-oxoisoindolin-2-yl)piperidine-2,6-dione | 3-(5-((4-(5-fluoroindolin-1-yl)piperidin-1-yl)methyl)-1-oxoisoindolin-2-yl)piperidine-2,6-dione |
| GT-04905 | 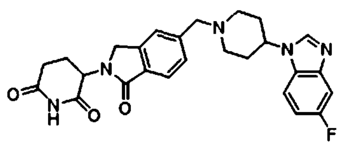 | 3-(5-((4-(5-fluoro-1H-benzo[d]imidazol-1-yl)piperidin-1-yl)methyl)-1-oxoisoindolin-2-yl)piperidine-2,6-dione | 3-(5-((4-(5-fluoro-1H-benzo[d]imidazol-1-yl)piperidin-1-yl)methyl)-1-oxoisoindolin-2-yl)piperidine-2,6-dione |
| GT-04714 | 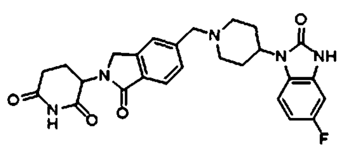 | 3-(5-((4-(5-fluoro-2-oxo-2,3-dihydro-1H-benzo[d]imidazol-1-yl)piperidin-1-yl)methyl)-1-oxoisoindolin-2-yl)piperidine-2,6-dione | 3-(5-((4-(5-fluoro-2-oxo-2,3-dihydro-1H-benzo[d]imidazol-1-yl)piperidin-1-yl)methyl)-1-oxoisoindolin-2-yl)piperidine-2,6-dione |
| GT-04603 | 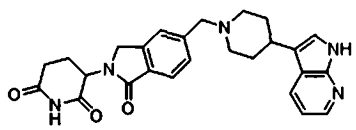 | 3-(5-((4-(1H-pyrrolo[2,3-b]pyridin-3-yl)piperidin-1-yl)methyl)-1-oxoisoindolin-2-yl)piperidine-2,6-dione | 3-(5-((4-(1H-pyrrolo[2,3-b]pyridin-3-yl)piperidin-1-yl)methyl)-1-oxoisoindolin-2-yl)piperidine-2,6-dione |
| GT-04602 | 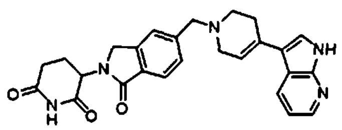 | 3-(5-((4-(1H-pyrrolo[2,3-b]pyridin-3-yl)-3,6-dihydropyridin-1(2H)-yl)methyl)-1-oxoisoindolin-2-yl)piperidine-2,6-dione | 3-(5-((4-(1H-pyrrolo[2,3-b]pyridin-3-yl)-3,6-dihydropyridin-1(2H)-yl)methyl)-1-oxoisoindolin-2-yl)piperidine-2,6-dione |

(continued)

| Compo und No. | Structure of the compounds | The compounds' name | Chinese translation of the compounds' name |
|---|---|---|---|
| GT-04722 | 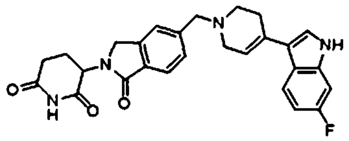 | 3-(5-((4-(6-fluoro-1H-indol-3-yl)-3,6-dihydropyridin-1(2H)-yl) methyl)-1-oxoisoindolin-2-yl)piperidine-2,6-dione | 3-(5-((4-(6-fluoro-1H-indol-3-yl)-3,6-dihydropyridin-1(2H)-yl) methyl)-1-oxoisoindolin-2-yl)piperidine-2,6-dione |
| GT-04723 | 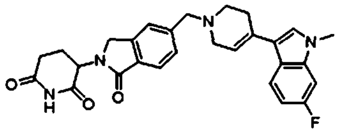 | 3-(5-((4-(6-fluoro-1-methyl-1H-indol-3-yl)-3,6-dihydropyridin-1(2H)-yl)methyl)-1-oxoisoindolin-2-yl)piperidine-2,6-dione | 3-(5-((4-(6-fluoro-1-methyl-1H-indol-3-yl)-3,6-dihydropyridin-1(2H)-yl)methyl)-1-oxoisoindolin-2-yl)piperidine-2,6-dione |
| GT-04691 | 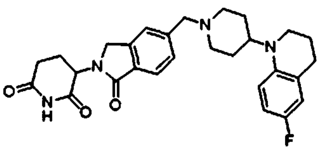 | 3-(5-((4-(6-fluoro-3,4-dihydroquinolin-1(2H)-yl)piperidin-1-yl) methyl)-1-oxoisoindolin-2-yl)piperidine-2,6-dione | 3-(5-((4-(6-fluoro-3,4-dihydroquinolin-1(2H)-yl)piperidin-1-yl) methyl)-1-oxoisoindolin-2-yl)piperidine-2,6-dione |
| GT-04859 | 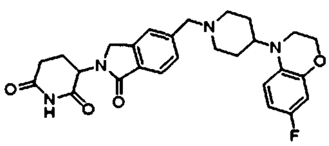 | 3-(5-((4-(7-fluoro-2,3-dihydro-4H-benzo[b][1,4]oxazin-4-yl)piperidin-1-yl)methyl)-1-oxoisoindolin-2-yl)piperidine-2,6-dione | 3-(5-((4-(7-fluoro-2,3-dihydro-4H-benzo[b][1,4]oxazin-4-yl)piperidin-1-yl)methyl)-1-oxoisoindolin-2-yl)piperidine-2,6-dione |
| GT-04860 | 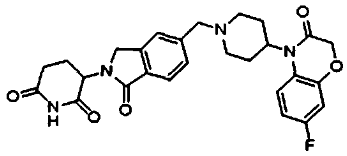 | 3-(5-((4-(7-fluoro-3-oxo-2,3-dihydro-4H-benzo[b][1,4]oxazin-4-yl)piperidin-1-yl) methyl)-1-oxoisoindolin-2-yl)piperidine-2,6-dione | 3-(5-((4-(7-fluoro-3-oxo-2,3-dihydro-4H-benzo[b][1,4]oxazin-4-yl)piperidin-1-yl)methyl)-1-oxoisoindolin-2-yl)piperidine-2,6-dione |
| GT-04901 | 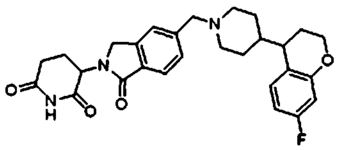 | 3-(5-((4-(7-fluorochroman-4-yl) piperidin-1-yl)methyl)-1-oxoisoindolin-2-yl)piperidine-2,6-dione | 3-(5-((4-(7-fluorochroman-4-yl) piperidin-1-yl)methyl)-1-oxoisoindolin-2-yl)piperidine-2,6-dione |
| GT-04649 | 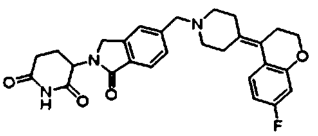 | 3-(5-((4-(7-fluorochroman-4-ylidene)piperidin-1-yl)methyl)-1-oxoisoindolin-2-yl)piperidine-2,6-dione | 3-(5-((4-(7-fluorochroman-4-ylidene)piperidin-1-yl)methyl)-1-oxoisoindolin-2-yl)piperidine-2,6-dione |
| GT-04664 | 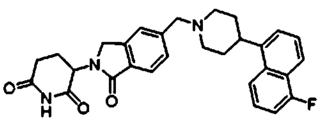 | 3-(5-((4-(5-fluoronaphthalen-1-yl)piperidin-1-yl)methyl)-1-oxoisoindolin-2-yl)piperidine-2,6-dione | 3-(5-((4-(5-fluoronaphthalen-1-yl)piperidin-1-yl)methyl)-1-oxoisoindolin-2-yl)piperidine-2,6-dione |
| GT-04648 | 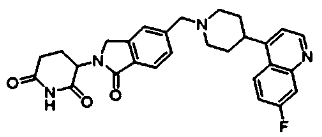 | 3-(5-((4-(7-fluoroquinolin-4-yl) piperidin-1-yl)methyl)-1-oxoisoindolin-2-yl)piperidine-2,6-dione | 3-(5-((4-(7-fluoroquinolin-4-yl) piperidin-1-yl)methyl)-1-oxoisoindolin-2-yl)piperidine-2,6-dione |
| GT-04665 | 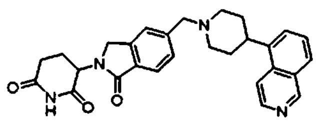 | 3-(5-((4-(isoquinolin-5-yl)piperidin-1-yl)methyl)-1-oxoisoindolin-2-yl)piperidine-2,6-dione | 3-(5-((4-(isoquinolin-5-yl)piperidin-1-yl)methyl)-1-oxoisoindolin-2-yl)piperidine-2,6-dione |

(continued)

| Compound No. | Structure of the compounds | The compounds' name | Chinese translation of the compounds' name |
|---|---|---|---|
| GT-04902 | 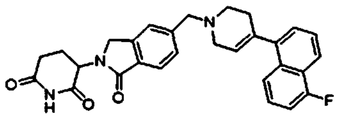 | 3-(5-((4-(5-fluoronaphthalen-1-yl)-3,6-dihydropyridin-1(2H)-yl) methyl)-1-oxoisoindolin-2-yl)piperidine-2,6-dione | 3-(5-((4-(5-fluoronaphthalen-1-yl)-3,6-dihydropyridin-1(2H)-yl) methyl)-1-oxoisoindolin-2-yl)piperidine-2,6-dione |
| GT-04666 | 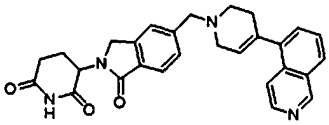 | 3-(5-((4-(isoquinolin-5-yl)-3,6-dihydropyridin-1(2H)-yl) methyl)-1-oxoisoindolin-2-yl)piperidine-2,6-dione | 3-(5-((4-(isoquinolin-5-yl)-3,6-dihydropyridin-1(2H)-yl) methyl)-1-oxoisoindolin-2-yl)piperidine-2,6-dione |
| GT-04700 | 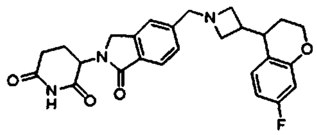 | 3-(5-((3-(7-fluorochroman-4-yl) azetidin-1-yl)methyl)-1-oxoisoindolin-2-yl)piperidine-2,6-dione | 3-(5-((3-(7-fluorochroman-4-yl) azetidin-1-yl)methyl)-1-oxoisoindolin-2-yl)piperidine-2,6-dione |
| GT-04701 | 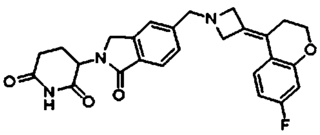 | 3-(5-((3-(7-fluorochroman-4-ylidene)azetidin-1-yl)methyl)-1-oxoisoindolin-2-yl)piperidine-2,6-dione | 3-(5-((3-(7-fluorochroman-4-ylidene)azetidin-1-yl)methyl)-1-oxoisoindolin-2-yl)piperidine-2,6-dione |
| GT-04651 | 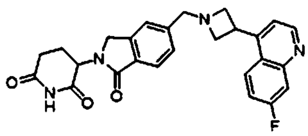 | 3-(5-((3-(7-fluoroquinolin-4-yl) azetidin-1-yl)methyl)-1-oxoisoindolin-2-yl)piperidine-2,6-dione | 3-(5-((3-(7-fluoroquinolin-4-yl) azetidin-1-yl)methyl)-1-oxoisoindolin-2-yl)piperidine-2,6-dione |
| GT-04677 | 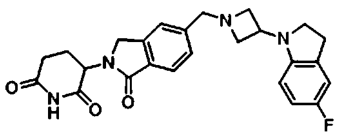 | 3-(5-((3-(5-fluoroindolin-1-yl) azetidin-1-yl)methyl)-1-oxoisoindolin-2-yl)piperidine-2,6-dione | 3-(5-((3-(5-fluoroindolin-1-yl) azetidin-1-yl)methyl)-1-oxoisoindolin-2-yl)piperidine-2,6-dione |
| GT-04716 | 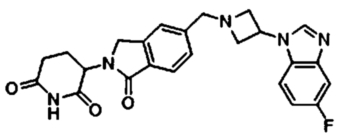 | 3-(5-((3-(5-fluoro-1H-benzo[d] imidazol-1-yl)azetidin-1-yl) methyl)-1-oxoisoindolin-2-yl)piperidine-2,6-dione | 3-(5-((3-(5-fluoro-1H-benzo[d] imidazol-1-yl)azetidin-1-yl) methyl)-1-oxoisoindolin-2-yl)piperidine-2,6-dione |
| GT-04678 | 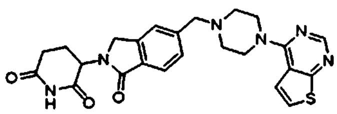 | 3-(1-oxo-5-((4-(thieno[2,3-d] pyrimidin-4-yl)piperazin-1-yl) methyl)isoindolin-2-yl)piperidine-2,6-dione | 3-(1-oxo-5-((4-(thieno[2,3-d]pyrimidin-4-yl)piperazin-1-yl) methyl)isoindolin-2-yl)piperidine-2,6-dione |
| GT-04679 | 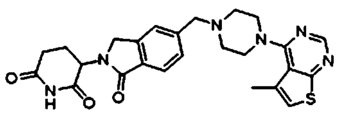 | 3-(5-((4-(5-methylthieno[2,3-d] pyrimidin-4-yl)piperazin-1-yl) methyl)-1-oxoisoindolin-2-yl)piperidine-2,6-dione | 3-(5-((4-(5-methylthieno[2,3-d] pyrimidin-4-yl)piperazin-1-yl) methyl)-1-oxoisoindolin-2-yl)piperidine-2,6-dione |
| GT-04680 | 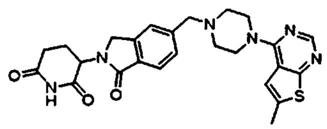 | 3-(5-((4-(6-methylthieno[2,3-d] pyrimidin-4-yl)piperazin-1-yl) methyl)-1-oxoisoindolin-2-yl)piperidine-2,6-dione | 3-(5-((4-(6-methylthieno[2,3-d] pyrimidin-4-yl)piperazin-1-yl) methyl)-1-oxoisoindolin-2-yl)piperidine-2,6-dione |
| GT-04705 | 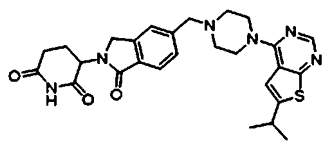 | 3-(5-((4-(6-isopropylthieno[2,3-d]pyrimidin-4-yl)piperazin-1-yl) methyl)-1-oxoisoindolin-2-yl)piperidine-2,6-dione | 3-(5-((4-(6-isopropylthieno[2,3-d]pyrimidin-4-yl)piperazin-1-yl) methyl)-1-oxoisoindolin-2-yl)piperidine-2,6-dione |

(continued)

| Compo und No. | Structure of the compounds | The compounds' name | Chinese translation of the compounds' name |
|---|---|---|---|
| GT-04706 | 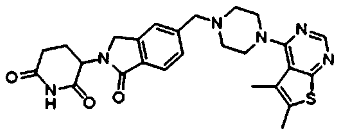 | 3-(5-((4-(5,6-dimethylthieno [2,3-d]pyrimidin-4-yl)piperazin-1-yl)methyl)-1-oxoisoindolin-2-yl)piperidine-2,6-dione | 3-(5-((4-(5,6-dimethylthieno [2,3-d]pyrimidin-4-yl)piperazin-1-yl)methyl)-1-oxoisoindolin-2-yl)piperidine-2,6-dione |
| GT-04862 | 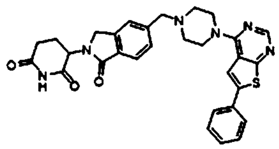 | 3-(1-oxo-5-((4-(6-phenylthieno [2,3-d]pyrimidin-4-yl)piperazin-1-yl)methyl)isoindolin-2-yl) piperidine-2,6-dione | 3-(1-oxo-5-((4-(6-phenylthieno [2,3-d]pyrimidin-4-yl)piperazin-1-yl)methyl)isoindolin-2-yl) piperidine-2,6-dione |
| GT-04861 | 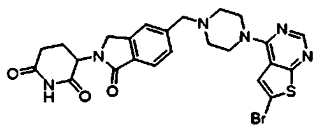 | 3-(5-((4-(6-bromothieno[2,3-d] pyrimidin-4-yl)piperazin-1-yl) methyl)-1-oxoisoindolin-2-yl)piperidine-2,6-dione | 3-(5-((4-(6-bromothieno[2,3-d] pyrimidin-4-yl)piperazin-1-yl) methyl)-1-oxoisoindolin-2-yl)piperidine-2,6-dione |
| GT-04709 | 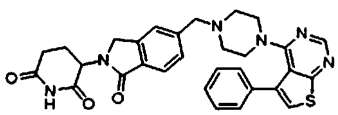 | 3-(1-oxo-5-((4-(5-phenylthieno [2,3-d]pyrimidin-4-yl)piperazin-1-yl)methyl)isoindolin-2-yl) piperidine-2,6-dione | 3-(1-oxo-5-((4-(5-phenylthieno [2,3-d]pyrimidin-4-yl)piperazin-1-yl)methyl)isoindolin-2-yl) piperidine-2,6-dione |
| GT-05209 | 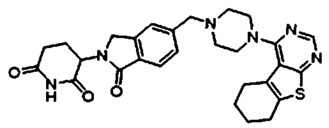 | 3-(1-oxo-5-((4-(5,6,7,8-tetrahydrobenzo[4,5]thieno [2,3-d]pyrimidin-4-yl)piperazin-1-yl) methyl)isoindolin-2-yl)piperidine-2,6-dione | 3-(1-oxo-5-((4-(5,6,7,8-tetrahydrobenzo[4,5]thieno [2,3-d]pyrimidin-4-yl)piperazin-1-yl) methyl)isoindolin-2-yl)piperidine-2,6-dione |
| GT-04996 | 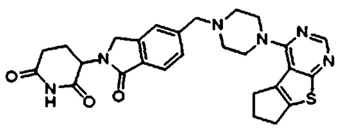 | 3-(5-((4-(6,7-dihydro-5H-cyclopenta[4,5]thieno[2,3-d]pyrimidin-4-yl)piperazin-1-yl) methyl)-1-oxoisoindolin-2-yl)piperidine-2,6-dione | 3-(5-((4-(6,7-dihydro-5H-cyclopenta[4,5]thieno[2,3-d]pyrimidin-4-yl)piperazin-1-yl) methyl)-1-oxoisoindolin-2-yl)piperidine-2,6-dione |
| GT-05100 | 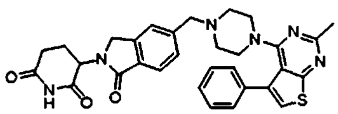 | 3-(5-((4-(2-methyl-5-phenylthieno[2,3-d]pyrimidin-4-yl)piperazin-1-yl)methyl)-1-oxoisoindolin-2-yl)piperidine-2,6-dione | 3-(5-((4-(2-methyl-5-phenylthieno[2,3-d]pyrimidin-4-yl)piperazin-1-yl)methyl)-1-oxoisoindolin-2-yl)piperidine-2,6-dione |
| GT-04900 | 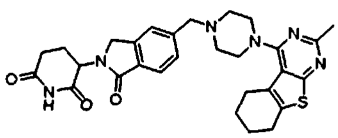 | 3-(5-((4-(2-methyl-5,6,7,8-tetrahydrobenzo[4,5]thieno [2,3-d] pyrimidin-4-yl)piperazin-1-yl) methyl)-1-oxoisoindolin-2-yl)piperidine-2,6-dione | 3-(5-((4-(2-methyl-5,6,7,8-tetrahydrobenzo[4,5]thieno [2,3-d] pyrimidin-4-yl)piperazin-1-yl) methyl)-1-oxoisoindolin-2-yl)piperidine-2,6-dione |
| GT-05157 | 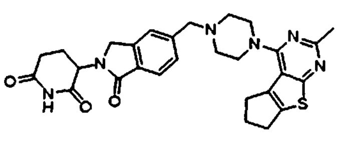 | 3-(5-((4-(2-methyl-6,7-dihydro-5H-cyclopenta[4,5]thieno [2,3-d]pyrimidin-4-yl)piperazin-1-yl)methyl)-1-oxoisoindolin-2-yl)piperidine-2,6-dione | 3-(5-((4-(2-methyl-6,7-dihydro-5H-cyclopenta[4,5]thieno [2,3-d]pyrimidin-4-yl)piperazin-1-yl)methyl)-1-oxoisoindolin-2-yl)piperidine-2,6-dione |
| GT-04693 | 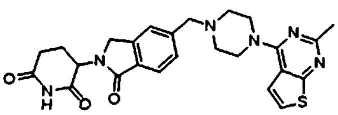 | 3-(5-((4-(2-methylthieno[2,3-d] pyrimidin-4-yl)piperazin-1-yl) methyl)-1-oxoisoindolin-2-yl)piperidine-2,6-dione | 3-(5-((4-(2-methylthieno[2,3-d] pyrimidin-4-yl)piperazin-1-yl) methyl)-1-oxoisoindolin-2-yl)piperidine-2,6-dione |
| GT-04725 | 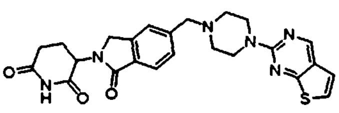 | 3-(1-oxo-5-((4-(thieno[2,3-d] pyrimidin-2-yl)piperazin-1-yl) methyl)isoindolin-2-yl)piperidine-2,6-dione | 3-(1-oxo-5-((4-(thieno[2,3-d]pyrimidin-2-yl)piperazin-1-yl) methyl)isoindolin-2-yl)piperidine-2,6-dione |

(continued)

| Compound No. | Structure of the compounds | The compounds' name | Chinese translation of the compounds' name |
|---|---|---|---|
| GT-04707 | 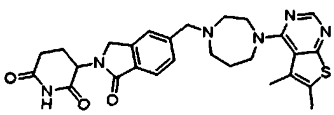 | 3-(5-((4-(5,6-dimethylthieno [2,3-d]pyrimidin-4-yl)-1,4-diazepan-1-yl)methyl)-1-oxoisoindolin-2-yl)piperidine-2,6-dione | 3-(5-((4-(5,6-dimethylthieno [2,3-d]pyrimidin-4-yl)-1,4-diazepan-1-yl)methyl)-1-oxoisoindolin-2-yl)piperidine-2,6-dione |
| GT-04702 | 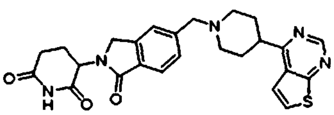 | 3-(1-oxo-5-((4-(thieno[2,3-d] pyrimidin-4-yl)piperidin-1-yl) methyl)isoindolin-2-yl)piperidine-2,6-dione | 3-(1-oxo-5-((4-(thieno[2,3-d]pyrimidin-4-yl)piperidin-1-yl) methyl)isoindolin-2-yl)piperidine-2,6-dione |
| GT-04717 | 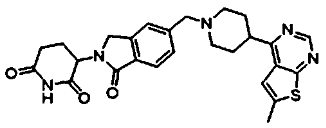 | 3-(5-((4-(6-methylthieno[2,3-d] pyrimidin-4-yl)piperidin-1-yl) methyl)-1-oxoisoindolin-2-yl)piperidine-2,6-dione | 3-(5-((4-(6-methylthieno[2,3-d] pyrimidin-4-yl)piperidin-1-yl) methyl)-1-oxoisoindolin-2-yl)piperidine-2,6-dione |
| GT-05034 | 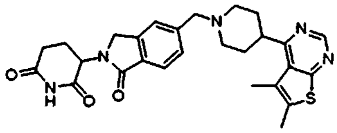 | 3-(5-((4-(5,6-dimethylthieno [2,3-d]pyrimidin-4-yl)piperidin-1-yl)methyl)-1-oxoisoindolin-2-yl)piperidine-2,6-dione | 3-(5-((4-(5,6-dimethylthieno [2,3-d]pyrimidin-4-yl)piperidin-1-yl)methyl)-1-oxoisoindolin-2-yl) piperidine-2,6-dione |
| GT-05159 | 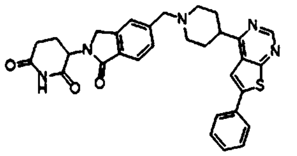 | 3-(1-oxo-5-((4-(6-phenylthieno [2,3-d]pyrimidin-4-yl)piperidin-1-yl)methyl)isoindolin-2-yl) piperidine-2,6-dione | 3-(1-oxo-5-((4-(6-phenylthieno [2,3-d]pyrimidin-4-yl)piperidin-1-yl)methyl)isoindolin-2-yl)piperidine-2,6-dione |
| GT-04995 | 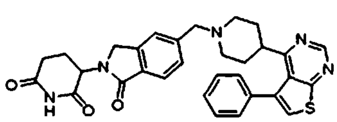 | 3-(1-oxo-5-((4-(5-phenylthieno [2,3-d]pyrimidin-4-yl)piperidin-1-yl)methyl)isoindolin-2-yl) piperidine-2,6-dione | 3-(1-oxo-5-((4-(5-phenylthieno [2,3-d]pyrimidin-4-yl)piperidin-1-yl)methyl)isoindolin-2-yl)piperidine-2,6-dione |
| GT-05265 | 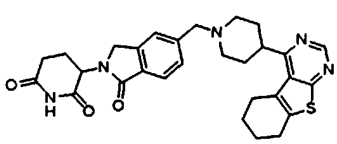 | 3-(1-oxo-5-((4-(5,6,7,8-tetrahydrobenzo[4,5]thieno [2,3-d]pyrimidin-4-yl)piperidin-1-yl)methyl) isoindolin-2-yl)piperidine-2,6-dione | 3-(1-oxo-5-((4-(5,6,7,8-tetrahydrobenzo[4,5]thieno [2,3-d]pyrimidin-4-yl)piperidin-1-yl)methyl) isoindolin-2-yl)piperidine-2,6-dione |
| GT-04994 | 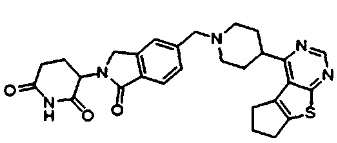 | 3-(5-((4-(6,7-dihydro-5H-cyclopenta[4,5]thieno[2,3-d]pyrimidin-4-yl)piperidin-1-yl) methyl)-1-oxoisoindolin-2-yl)piperidine-2,6-dione | 3-(5-((4-(6,7-dihydro-5H-cyclopenta[4,5]thieno[2,3-d]pyrimidin-4-yl)piperidin-1-yl) methyl)-1-oxoisoindolin-2-yl)piperidine-2,6-dione |
| GT-05158 | 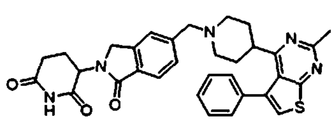 | 3-(5-((4-(2-methyl-5-phenylthieno[2,3-d]pyrimidin-4-yl)piperidin-1-yl)methyl)-1-oxoisoindolin-2-yl)piperidine-2,6-dione | 3-(5-((4-(2-methyl-5-phenylthieno[2,3-d]pyrimidin-4-yl)piperidin-1-yl)methyl)-1-oxoisoindolin-2-yl)piperidine-2,6-dione |
| GT-04993 | 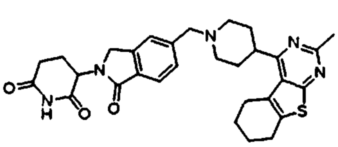 | 3-(5-((4-(2-methyl-5,6,7,8-tetrahydrobenzo[4,5]thieno [2,3-d] pyrimidin-4-yl)piperidin-1-yl) methyl)-1-oxoisoindolin-2-yl)piperidine-2,6-dione | 3-(5-((4-(2-methyl-5,6,7,8-tetrahydrobenzo[4,5]thieno [2,3-d] pyrimidin-4-yl)piperidin-1-yl) methyl)-1-oxoisoindolin-2-yl)piperidine-2,6-dione |
| GT-05210 | 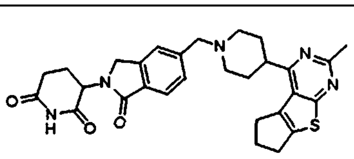 | 3-(5-((4-(2-methyl-6,7-dihydro-5H-cyclopenta[4,5]thieno [2,3-d]pyrimidin-4-yl)piperidin-1-yl)methyl)-1-oxoisoindolin-2-yl)piperidine-2,6-dione | 3-(5-((4-(2-methyl-6,7-dihydro-5H-cyclopenta[4,5]thieno [2,3-d]pyrimidin-4-yl)piperidin-1-yl)methyl)-1-oxoisoindolin-2-yl) piperidine-2,6-dione |

(continued)

| Compo und No. | Structure of the compounds | The compounds' name | Chinese translation of the compounds' name |
|---|---|---|---|
| GT-04726 | 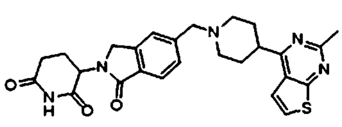 | 3-(5-((4-(2-methylthieno[2,3-d]pyrimidin-4-yl)piperidin-1-yl)methyl)-1-oxoisoindolin-2-yl)piperidine-2,6-dione | 3-(5-((4-(2-methylthieno[2,3-d]pyrimidin-4-yl)piperidin-1-yl)methyl)-1-oxoisoindolin-2-yl)piperidine-2,6-dione |
| GT-04933 | 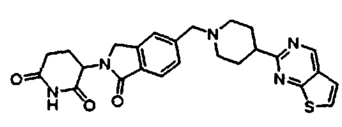 | 3-(1-oxo-5-((4-(thieno[2,3-d]pyrimidin-2-yl)piperidin-1-yl)methyl)isoindolin-2-yl)piperidine-2,6-dione | 3-(1-oxo-5-((4-(thieno[2,3-d]pyrimidin-2-yl)piperidin-1-yl)methyl)isoindolin-2-yl)piperidine-2,6-dione |
| GT-04936 | 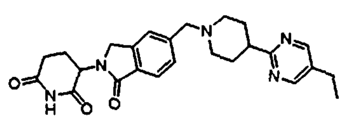 | 3-(5-((4-(5-ethylpyrimidin-2-yl)piperidin-1-yl)methyl)-1-oxoisoindolin-2-yl)piperidine-2,6-dione | 3-(5-((4-(5-ethylpyrimidin-2-yl)piperidin-1-yl)methyl)-1-oxoisoindolin-2-yl)piperidine-2,6-dione |
| GT-04692 | 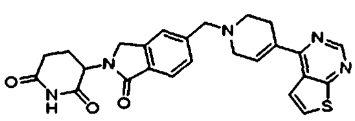 | 3-(1-oxo-5-((4-(thieno[2,3-d]pyrimidin-4-yl)-3,6-dihydropyridin-1(2H)-yl)methyl)isoindolin-2-yl)piperidine-2,6-dione | 3-(1-oxo-5-((4-(thieno[2,3-d]pyrimidin-4-yl)-3,6-dihydropyridin-1(2H)-yl)methyl)isoindolin-2-yl)piperidine-2,6-dione |
| GT-04703 | 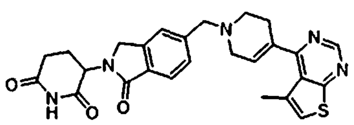 | 3-(5-((4-(5-methylthieno[2,3-d]pyrimidin-4-yl)-3,6-dihydropyridin-1(2H)-yl)methyl)-1-oxoisoindolin-2-yl)piperidine-2,6-dione | 3-(5-((4-(5-methylthieno[2,3-d]pyrimidin-4-yl)-3,6-dihydropyridin-1(2H)-yl)methyl)-1-oxoisoindolin-2-yl)piperidine-2,6-dione |
| GT-04704 | 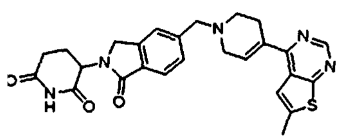 | 3-(5-((4-(6-methylthieno[2,3-d]pyrimidin-4-yl)-3,6-dihydropyridin-1(2H)-yl)methyl)-1-oxoisoindolin-2-yl)piperidine-2,6-dione | 3-(5-((4-(6-methylthieno[2,3-d]pyrimidin-4-yl)-3,6-dihydropyridin-1(2H)-yl)methyl)-1-oxoisoindolin-2-yl)piperidine-2,6-dione |
| GT-04935 | 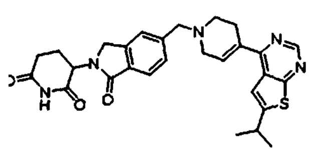 | 3-(5-((4-(6-isopropylthieno[2,3-d]pyrimidin-4-yl)-3,6-dihydropyridin-1(2H)-yl)methyl)-1-oxoisoindolin-2-yl)piperidine-2,6-dione | 3-(5-((4-(6-isopropylthieno[2,3-d]pyrimidin-4-yl)-3,6-dihydropyridin-1(2H)-yl)methyl)-1-oxoisoindolin-2-yl)piperidine-2,6-dione |
| GT-04727 | 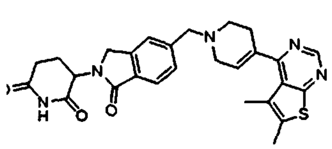 | 3-(5-((4-(5,6-dimethylthieno [2,3-d]pyrimidin-4-yl)-3,6-dihydropyridin-1(2H)-yl)methyl)-1-oxoisoindolin-2-yl)piperidine-2,6-dione | 3-(5-((4-(5,6-dimethylthieno [2,3-d]pyrimidin-4-yl)-3,6-dihydropyridin-1(2H)-yl)methyl)-1-oxoisoindolin-2-yl)piperidine-2,6-dione |
| GT-05101 | 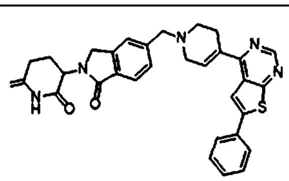 | 3-(1-oxo-5-((4-(6-phenylthieno [2,3-d]pyrimidin-4-yl)-3,6-dihydropyridin-1(2H)-yl)methyl)isoindolin-2-yl)piperidine-2,6-dione | 3-(1-oxo-5-((4-(6-phenylthieno [2,3-d]pyrimidin-4-yl)-3,6-dihydropyridin-1(2H)-yl)methyl)isoindolin-2-yl)piperidine-2,6-dione |
| GT-04908 | 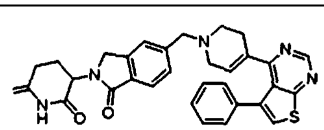 | 3-(1-oxo-5-((4-(5-phenylthieno [2,3-d]pyrimidin-4-yl)-3,6-dihydropyridin-1(2H)-yl)methyl)isoindolin-2-yl)piperidine-2,6-dione | 3-(1-oxo-5-((4-(5-phenylthieno [2,3-d]pyrimidin-4-yl)-3,6-dihydropyridin-1(2H)-yl)methyl)isoindolin-2-yl)piperidine-2,6-dione |
| GT-05266 | 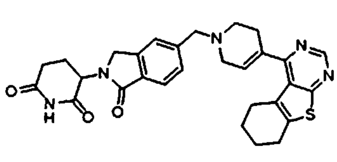 | 3-(1-oxo-5-((4-(5,6,7,8-tetrahydrobenzo[4,5]thieno [2,3-d]pyrimidin-4-yl)-3,6-dihydropyridin-1(2H)-yl)methyl)isoindolin-2-yl)piperidine-2,6-dione | 3-(1-oxo-5-((4-(5,6,7,8-tetrahydrobenzo[4,5]thieno [2,3-d]pyrimidin-4-yl)-3,6-dihydropyridin-1(2H)-yl)methyl)isoindolin-2-yl)piperidine-2,6-dione |

(continued)

| Compo und No. | Structure of the compounds | The compounds' name | Chinese translation of the compounds' name |
|---|---|---|---|
| GT-04934 | 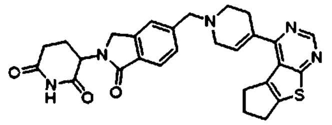 | 3-(5-((4-(6,7-dihydro-5H-cyclopenta[4,5]thieno[2,3-d]pyrimidin-4-yl)-3,6-dihydropyridin-1(2H)-yl)methyl)-1-oxoisoindolin-2-yl)piperidine-2,6-dione | 3-(5-((4-(6,7-dihydro-5H-cyclopenta[4,5]thieno[2,3-d]pyrimidin-4-yl)-3,6-dihydropyridin-1(2H)-yl)methyl)-1-oxoisoindolin-2-yl)piperidine-2,6-dione |
| GT-05102 | 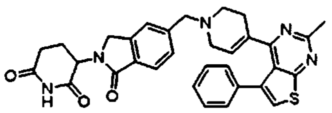 | 3-(5-((4-(2-methyl-5-phenylthieno[2,3-d]pyrimidin-4-yl)-3,6-dihydropyridin-1(2H)-yl) methyl)-1-oxoisoindolin-2-yl)piperidine-2,6-dione | 3-(5-((4-(2-methyl-5-phenylthieno[2,3-d]pyrimidin-4-yl)-3,6-dihydropyridin-1(2H)-yl) methyl)-1-oxoisoindolin-2-yl)piperidine-2,6-dione |
| GT-04863 | 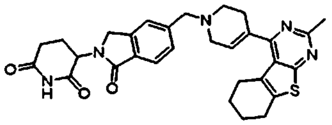 | 3-(5-((4-(2-methyl-5,6,7,8-tetrahydrobenzo[4,5]thieno [2,3-d] pyrimidin-4-yl)-3,6-dihydropyridin-1(2H)-yl)methyl)-1-oxoisoindolin-2-yl)piperidine-2,6-dione | 3-(5-((4-(2-methyl-5,6,7,8-tetrahydrobenzo[4,5]thieno [2,3-d] pyrimidin-4-yl)-3,6-dihydropyridin-1(2H)-yl)methyl)-1-oxoisoindolin-2-yl)piperidine-2,6-dione |
| GT-05211 | 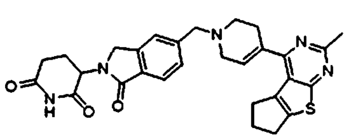 | 3-(5-((4-(2-methyl-6,7-dihydro-5H-cyclopenta[4,5]thieno [2,3-d]pyrimidin-4-yl)-3,6-dihydropyridin-1(2H)-yl)methyl)-1-oxoisoindolin-2-yl)piperidine-2,6-dione | 3-(5-((4-(2-methyl-6,7-dihydro-5H-cyclopenta[4,5]thieno [2,3-d]pyrimidin-4-yl)-3,6-dihydropyridin-1(2H)-yl)methyl)-1-oxoisoindolin-2-yl)piperidine-2,6-dione |
| GT-04718 | 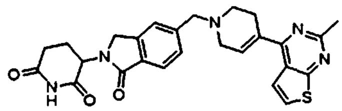 | 3-(5-((4-(2-methylthieno[2,3-d] pyrimidin-4-yl)-3,6-dihydropyridin-1(2H)-yl)methyl)-1-oxoisoindolin-2-yl)piperidine-2,6-dione | 3-(5-((4-(2-methylthieno[2,3-d] pyrimidin-4-yl)-3,6-dihydropyridin-1(2H)-yl)methyl)-1-oxoisoindolin-2-yl)piperidine-2,6-dione |
| GT-04708 | 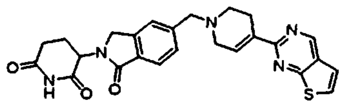 | 3-(1-oxo-5-((4-(thieno[2,3-d] pyrimidin-2-yl)-3,6-dihydropyridin-1(2H)-yl)methyl)isoindolin-2-yl)piperidine-2,6-dione | 3-(1-oxo-5-((4-(thieno[2,3-d]pyrimidin-2-yl)-3,6-dihydropyridin-1(2H)-yl)methyl)isoindolin-2-yl)piperidine-2,6-dione |
| GT-04683 | 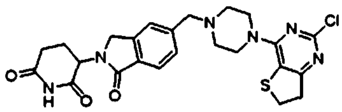 | 3-(5-((4-(2-chloro-6,7-dihydrothieno[3,2-d]pyrimidin-4-yl) piperazin-1-yl)methyl)-1-oxoisoindolin-2-yl)piperidine-2,6-dione | 3-(5-((4-(2-chloro-6,7-dihydrothieno[3,2-d]pyrimidin-4-yl) piperazin-1-yl)methyl)-1-oxoisoindolin-2-yl)piperidine-2,6-dione |
| GT-04710 | 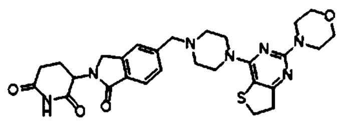 | 3-(5-((4-(2-morpholino-6,7-dihydrothieno[3,2-d]pyrimidin-4-yl) piperazin-1-yl)methyl)-1-oxoisoindolin-2-yl)piperidine-2,6-dione | 3-(5-((4-(2-morpholino-6,7-dihydrothieno[3,2-d]pyrimidin-4-yl) piperazin-1-yl)methyl)-1-oxoisoindolin-2-yl)piperidine-2,6-dione |
| GT-04720 | 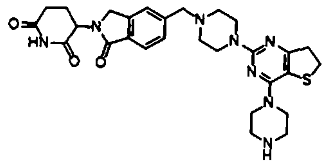 | 3-(1-oxo-5-((4-(4-(piperazin-1-yl)-6,7-dihydrothieno[3,2-d]pyrimidin-2-yl)piperazin-1-yl) methyl)isoindolin-2-yl)piperidine-2,6-dione | 3-(1-oxo-5-((4-(4-(piperazin-1-yl)-6,7-dihydrothieno[3,2-d]pyrimidin-2-yl)piperazin-1-yl) methyl)isoindolin-2-yl)piperidine-2,6-dione |
| GT-04694 | 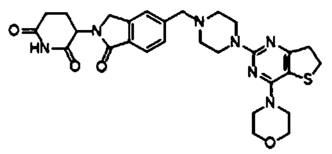 | 3-(5-((4-(4-morpholino-6,7-dihydrothieno[3,2-d]pyrimidin-2-yl) piperazin-1-yl)methyl)-1-oxoisoindolin-2-yl)piperidine-2,6-dione | 3-(5-((4-(4-morpholino-6,7-dihydrothieno[3,2-d]pyrimidin-2-yl) piperazin-1-yl)methyl)-1-oxoisoindolin-2-yl)piperidine-2,6-dione |

(continued)

| Compo und No. | Structure of the compounds | The compounds' name | Chinese translation of the compounds' name |
|---|---|---|---|
| GT-05380 | 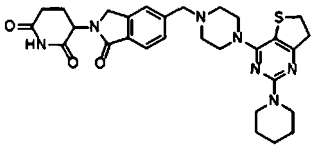 | 3-(1-oxo-5-((4-(2-(piperidin-1-yl)-6,7-dihydrothieno[3,2-d]pyrimidin-4-yl)piperazin-1-yl)methyl)isoindolin-2-yl)piperidine-2,6-dione | 3-(1-oxo-5-((4-(2-(piperidin-1-yl)-6,7-dihydrothieno[3,2-d]pyrimidin-4-yl)piperazin-1-yl)methyl)isoindolin-2-yl)piperidine-2,6-dione |
| GT-05389 | 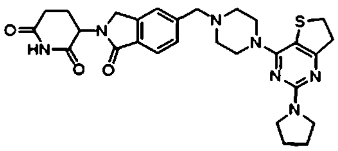 | 3-(1-oxo-5-((4-(2-(pyrrolidin-1-yl)-6,7-dihydrothieno[3,2-d]pyrimidin-4-yl)piperazin-1-yl)methyl)isoindolin-2-yl)piperidine-2,6-dione | 3-(1-oxo-5-((4-(2-(pyrrolidin-1-yl)-6,7-dihydrothieno[3,2-d]pyrimidin-4-yl)piperazin-1-yl)methyl)isoindolin-2-yl)piperidine-2,6-dione |
| GT-05900 | 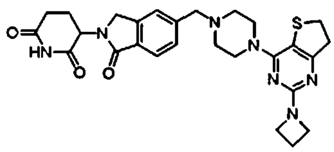 | 3-(5-((4-(2-(azetidin-1-yl)-6,7-dihydrothieno[3,2-d]pyrimidin-4-yl)piperazin-1-yl)methyl)-1-oxoisoindolin-2-yl)piperidine-2,6-dione | 3-(5-((4-(2-(azetidin-1-yl)-6,7-dihydrothieno[3,2-d]pyrimidin-4-yl)piperazin-1-yl)methyl)-1-oxoisoindolin-2-yl)piperidine-2,6-dione |
| GT-04681 | 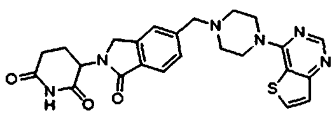 | 3-(1-oxo-5-((4-(thieno[3,2-d]pyrimidin-4-yl)piperazin-1-yl)methyl)isoindolin-2-yl)piperidine-2,6-dione | 3-(1-oxo-5-((4-(thieno[3,2-d]pyrimidin-4-yl)piperazin-1-yl)methyl)isoindolin-2-yl)piperidine-2,6-dione |
| GT-04682 | 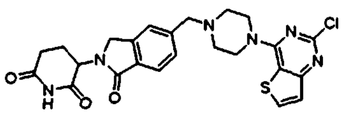 | 3-(5-((4-(2-chlorothieno[3,2-d]pyrimidin-4-yl)piperazin-1-yl)methyl)-1-oxoisoindolin-2-yl)piperidine-2,6-dione | 3-(5-((4-(2-chlorothieno[3,2-d]pyrimidin-4-yl)piperazin-1-yl)methyl)-1-oxoisoindolin-2-yl)piperidine-2,6-dione |
| GT-04903 | 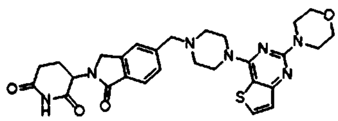 | 3-(5-((4-(2-morpholinothieno[3,2-d]pyrimidin-4-yl)piperazin-1-yl)methyl)-1-oxoisoindolin-2-yl)piperidine-2,6-dione | 3-(5-((4-(2-morpholinothieno[3,2-d]pyrimidin-4-yl)piperazin-1-yl)methyl)-1-oxoisoindolin-2-yl)piperidine-2,6-dione |
| GT-04698 | 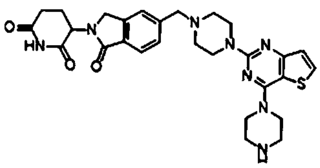 | 3-(1-oxo-5-((4-(4-(piperazin-1-yl)thieno[3,2-d]pyrimidin-2-yl)piperazin-1-yl)methyl)isoindolin-2-yl)piperidine-2,6-dione | 3-(1-oxo-5-((4-(4-(piperazin-1-yl)thieno[3,2-d]pyrimidin-2-yl)piperazin-1-yl)methyl)isoindolin-2-yl)piperidine-2,6-dione |
| GT-04906 | 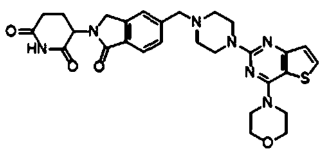 | 3-(5-((4-(4-morpholinothieno[3,2-d]pyrimidin-2-yl)piperazin-1-yl)methyl)-1-oxoisoindolin-2-yl)piperidine-2,6-dione | 3-(5-((4-(4-morpholinothieno[3,2-d]pyrimidin-2-yl)piperazin-1-yl)methyl)-1-oxoisoindolin-2-yl)piperidine-2,6-dione |
| GT-04202 | 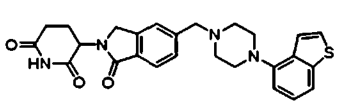 | 3-(5-((4-(benzo[b]thiophen-4-yl)piperazin-1-yl)methyl)-1-oxoisoindolin-2-yl)piperidine-2,6-dione | 3-(5-((4-(benzo[b]thiophen-4-yl)piperazin-1-yl)methyl)-1-oxoisoindolin-2-yl)piperidine-2,6-dione |
| GT-04199 | 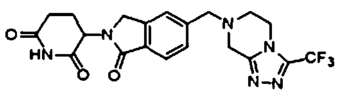 | 3-(1-oxo-5-((3-(trifluoromethyl)-5,6-dihydro-[1,2,4]triazolo[4,3-a]pyrazin-7(8H)-yl)methyl)isoindolin-2-yl)piperidine-2,6-dione | 3-(1-oxo-5-((3-(trifluoromethyl)-5,6-dihydro-[1,2,4]triazolo[4,3-a]pyrazin-7(8H)-yl)methyl)isoindolin-2-yl)piperidine-2,6-dione |

(continued)

| Compound No. | Structure of the compounds | The compounds' name | Chinese translation of the compounds' name |
|---|---|---|---|
| GT-04203 | 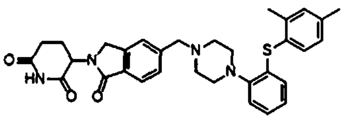 | 3-(5-((4-(2-((2,4-dimethylphenyl)thio)pheny 1)piperazin-1-yl)methyl)-1-oxoisoindolin-2-yl)piperidine-2,6-dione | 3-(5-((4-(2-((2,4-dimethylphenyl)thio)pheny 1)piperazin-1-yl)methyl)-1-oxoisoindolin-2-yl)piperidine-2,6-dione |
| GT-04200 | 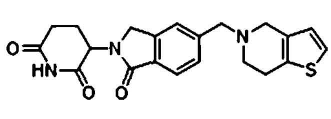 | 3-(5-((6,7-dihydrothieno[3,2-c]pyridin-5(4H)-yl)methyl)-1-oxoisoindolin-2-yl)piperidine-2,6-dione | 3-(5-((6,7-dihydrothieno[3,2-c]pyridin-5(4H)-yl)methyl)-1-oxoisoindolin-2-yl)piperidine-2,6-dione |
| GT-04255 | 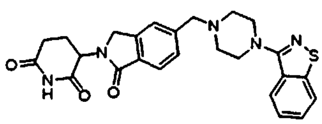 | 3-(5-((4-(benzo[d]isothiazol-3-yl)piperazin-1-yl)methyl)-1-oxoisoindolin-2-yl)piperidine-2,6-dione | 3-(5-((4-(benzo[d]isothiazol-3-yl)piperazin-1-yl)methyl)-1-oxoisoindolin-2-yl)piperidine-2,6-dione |
| GT-03026 | 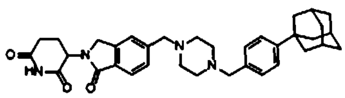 | 3-(5-((4-(4-((3r,5r,7r)-adamantan-1-yl)benzyl)piperazin-1-yl)methyl)-1-oxoisoindolin-2-yl)piperidine-2,6-dione | 3-(5-((4-(4-((3r,5r,7r)-adamantan-1-yl)benzyl)piperazin-1-yl)methyl)-1-oxoisoindolin-2-yl)piperidine-2,6-dione |
| GT-06913 | 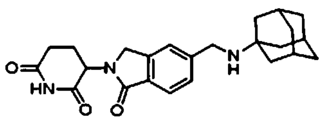 | 3-(5-((((3s,5s,7s)-adamantan-1-yl)amino)methyl)-1-oxoisoindolin-2-yl)piperidine-2,6-dione | 3-(5-((((3s,5s,7s)-adamantan-1-yl)amino)methyl)-1-oxoisoindolin-2-yl)piperidine-2,6-dione |
| GT-06927 | 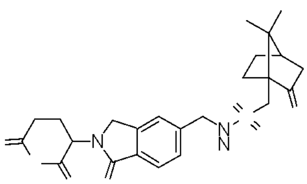 | (1-((1R,4R)-7,7-dimethyl-2-oxobicyclo[2.2.1]heptan-1-yl)-N-((2-(2,6-dioxopiperidin-3-yl)-1-oxoisoindolin-5-yl)methyl)methanesulfona mide | (1-((1R,4R)-7,7-dimethyl-2-oxobicyclo[2.2.1]heptan-1-yl)-N-((2-(2,6-dioxopiperidin-3-yl)-1-oxoisoindolin-5-yl)methyl)methanesulfona mide |
| | 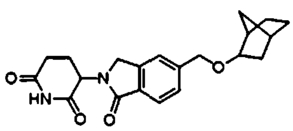 | 3-(5-((((1R,4S)-bicyclo[2.2.1]heptan-2-yl)oxy)methyl)-1-oxoisoindolin-2-yl)piperidine-2,6-dione | 3-(5-((((1R,4S)-bicyclo[2.2.1]heptan-2-yl)oxy)methyl)-1-oxoisoindolin-2-yl)piperidine-2,6-dione |
| GT-06212 | 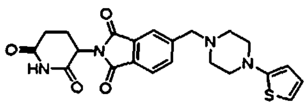 | 2-(2,6-dioxopiperidin-3-yl)-5-((4-(thiophen-2-yl)piperazin-1-yl)methyl)isoindoline-1,3-dione | 2-(2,6-dioxopiperidin-3-yl)-5-((4-(thiophen-2-yl)piperazin-1-yl)methyl)isoindoline-1,3-dione |
| GT-06213 | 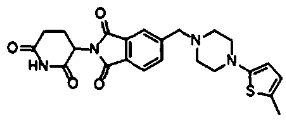 | 2-(2,6-dioxopiperidin-3-yl)-5-((4-(5-methylthiophen-2-yl)piperazin-1-yl)methyl)isoindoline-1,3-dione | 2-(2,6-dioxopiperidin-3-yl)-5-((4-(5-methylthiophen-2-yl)piperazin-1-yl)methyl)isoindoline-1,3-dione |
| GT-06214 | 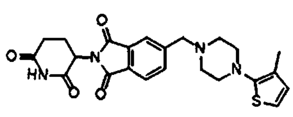 | 2-(2,6-dioxopiperidin-3-yl)-5-((4-(3-methylthiophen-2-yl)piperazin-1-yl)methyl)isoindoline-1,3-dione | 2-(2,6-dioxopiperidin-3-yl)-5-((4-(3-methylthiophen-2-yl)piperazin-1-yl)methyl)isoindoline-1,3-dione |
| GT-06215 | 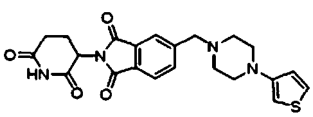 | 2-(2,6-dioxopiperidin-3-yl)-5-((4-(thiophen-3-yl)piperazin-1-yl)methyl)isoindoline-1,3-dione | 2-(2,6-dioxopiperidin-3-yl)-5-((4-(thiophen-3-yl)piperazin-1-yl)methyl)isoindoline-1,3-dione |

(continued)

| Compo und No. | Structure of the compounds | The compounds' name | Chinese translation of the compounds' name |
|---|---|---|---|
| GT-06260 | 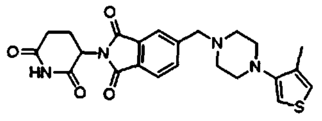 | 2-(2,6-dioxopiperidin-3-yl)-5-((4-(4-methylthiophen-3-yl)piperazin-1-yl)methyl)isoindoline-1,3-dione | 2-(2,6-dioxopiperidin-3-yl)-5-((4-(4-methylthiophen-3-yl)piperazin-1-yl)methyl)isoindoline-1,3-dione |
| GT-06218 | 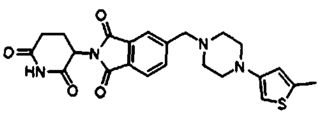 | 2-(2,6-dioxopiperidin-3-yl)-5-((4-(5-methylthiophen-3-yl)piperazin-1-yl)methyl)isoindoline-1,3-dione | 2-(2,6-dioxopiperidin-3-yl)-5-((4-(5-methylthiophen-3-yl)piperazin-1-yl)methyl)isoindoline-1,3-dione |
| GT-06219 | 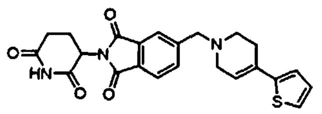 | 2-(2,6-dioxopiperidin-3-yl)-5-((4-(thiophen-2-yl)-3,6-dihydropyridin-1 (2H)-yl)methyl) isoindoline-1,3-dione | 2-(2,6-dioxopiperidin-3-yl)-5-((4-(thiophen-2-yl)-3,6-dihydropyridin-1 (2H)-yl)methyl) isoindoline-1,3-dione |
| GT-06220 | 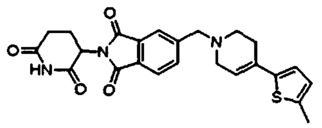 | 2-(2,6-dioxopiperidin-3-yl)-5-((4-(5-methylthiophen-2-yl)-3,6-dihydropyridin-1(2H)-yl) methyl)isoindoline-1,3-dione | 2-(2,6-dioxopiperidin-3-yl)-5-((4-(5-methylthiophen-2-yl)-3,6-dihydropyridin-1(2H)-yl) methyl)isoindoline-1,3-dione |
| GT-04732 | 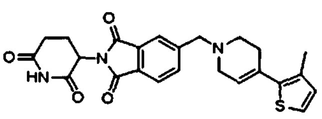 | 2-(2,6-dioxopiperidin-3-yl)-5-((4-(3-methylthiophen-2-yl)-3,6-dihydropyridin-1 (2H)-yl) methyl)isoindoline-1,3-dione | 2-(2,6-dioxopiperidin-3-yl)-5-((4-(3-methylthiophen-2-yl)-3,6-dihydropyridin-1 (2H)-yl) methyl)isoindoline-1,3-dione |
| GT-06261 | 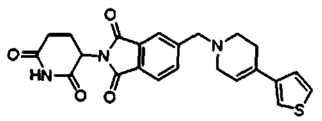 | 2-(2,6-dioxopiperidin-3-yl)-5-((4-(thiophen-3-yl)-3,6-dihydropyridin-1(2H)-yl)methyl) isoindoline-1,3-dione | 2-(2,6-dioxopiperidin-3-yl)-5-((4-(thiophen-3-yl)-3,6-dihydropyridin-1(2H)-yl)methyl) isoindoline-1,3-dione |
| GT-06221 | 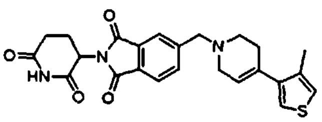 | 2-(2,6-dioxopiperidin-3-yl)-5-((4-(4-methylthiophen-3-yl)-3,6-dihydropyridin-1(2H)-yl) methyl)isoindoline-1,3-dione | 2-(2,6-dioxopiperidin-3-yl)-5-((4-(4-methylthiophen-3-yl)-3,6-dihydropyridin-1(2H)-yl) methyl)isoindoline-1,3-dione |
| GT-06279 | 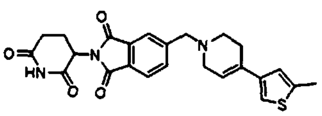 | 2-(2,6-dioxopiperidin-3-yl)-5-((4-(5-methylthiophen-3-yl)-3,6-dihydropyridin-1(2H)-yl) methyl)isoindoline-1,3-dione | 2-(2,6-dioxopiperidin-3-yl)-5-((4-(5-methylthiophen-3-yl)-3,6-dihydropyridin-1(2H)-yl) methyl)isoindoline-1,3-dione |
| GT-06222 | 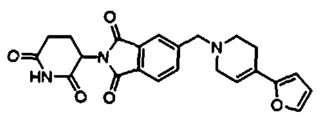 | 2-(2,6-dioxopiperidin-3-yl)-5-((4-(furan-2-yl)-3,6-dihydropyridin-1(2H)-yl)methyl)isoindoline-1,3-dione | 2-(2,6-dioxopiperidin-3-yl)-5-((4-(furan-2-yl)-3,6-dihydropyridin-1(2H)-yl)methyl)isoindoline-1,3-dione |
| GT-06223 | 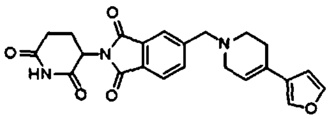 | 2-(2,6-dioxopiperidin-3-yl)-5-((4-(furan-3-yl)-3,6-dihydropyridin-1(2H)-yl)methyl)isoindoline-1,3-dione | 2-(2,6-dioxopiperidin-3-yl)-5-((4-(furan-3-yl)-3,6-dihydropyridin-1(2H)-yl)methyl)isoindoline-1,3-dione |
| GT-06224 | 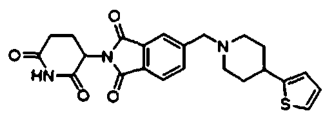 | 2-(2,6-dioxopiperidin-3-yl)-5-((4-(thiophen-2-yl)piperidin-1-yl)methyl)isoindoline-1,3-dione | 2-(2,6-dioxopiperidin-3-yl)-5-((4-(thiophen-2-yl)piperidin-1-yl)methyl)isoindoline-1,3-dione |
| GT-06262 | 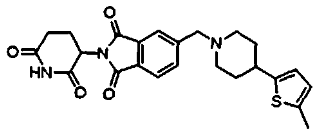 | 2-(2,6-dioxopiperidin-3-yl)-5-((4-(5-methylthiophen-2-yl)piperidin-1-yl)methyl)isoindoline-1,3-dione | 2-(2,6-dioxopiperidin-3-yl)-5-((4-(5-methylthiophen-2-yl)piperidin-1-yl)methyl)isoindoline-1,3-dione |

(continued)

| Compound No. | Structure of the compounds | The compounds' name | Chinese translation of the compounds' name |
|---|---|---|---|
| GT-06280 | 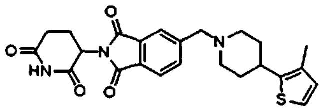 | 2-(2,6-dioxopiperidin-3-yl)-5-((4-(3-methylthiophen-2-yl)piperidin-1-yl)methyl)isoindoline-1,3-dione | 2-(2,6-dioxopiperidin-3-yl)-5-((4-(3-methylthiophen-2-yl)piperidin-1-yl)methyl)isoindoline-1,3-dione |
| GT-06281 | 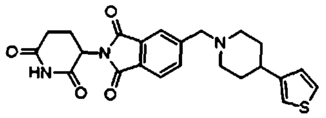 | 2-(2,6-dioxopiperidin-3-yl)-5-((4-(thiophen-3-yl)piperidin-1-yl)methyl)isoindoline-1,3-dione | 2-(2,6-dioxopiperidin-3-yl)-5-((4-(thiophen-3-yl)piperidin-1-yl)methyl)isoindoline-1,3-dione |
| GT-06283 | 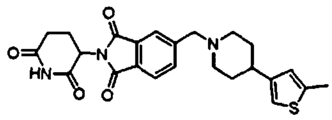 | 2-(2,6-dioxopiperidin-3-yl)-5-((4-(5-methylthiophen-3-yl)piperidin-1-yl)methyl)isoindoline-1,3-dione | 2-(2,6-dioxopiperidin-3-yl)-5-((4-(5-methylthiophen-3-yl)piperidin-1-yl)methyl)isoindoline-1,3-dione |
| GT-06282 | 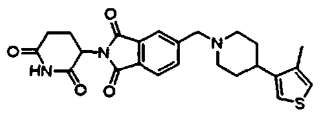 | 2-(2,6-dioxopiperidin-3-yl)-5-((4-(4-methylthiophen-3-yl)piperidin-1-yl)methyl)isoindoline-1,3-dione | 2-(2,6-dioxopiperidin-3-yl)-5-((4-(4-methylthiophen-3-yl)piperidin-1-yl)methyl)isoindoline-1,3-dione |
| GT-06284 | 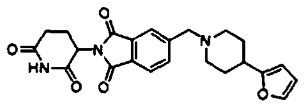 | 2-(2,6-dioxopiperidin-3-yl)-5-((4-(furan-2-yl)piperidin-1-yl)methyl)isoindoline-1,3-dione | 2-(2,6-dioxopiperidin-3-yl)-5-((4-(furan-2-yl)piperidin-1-yl)methyl)isoindoline-1,3-dione |
| GT-06285 | 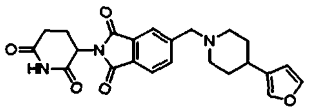 | 2-(2,6-dioxopiperidin-3-yl)-5-((4-(furan-3-yl)piperidin-1-yl)methyl)isoindoline-1,3-dione | 2-(2,6-dioxopiperidin-3-yl)-5-((4-(furan-3-yl)piperidin-1-yl)methyl)isoindoline-1,3-dione |
| GT-06273 | 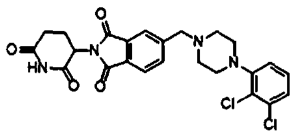 | 5-((4-(2,3-dichlorophenyl)piperazin-1-yl)methyl)-2-(2,6-dioxopiperidin-3-yl)isoindoline-1,3-dione | 5-((4-(2,3-dichlorophenyl)piperazin-1-yl)methyl)-2-(2,6-dioxopiperidin-3-yl)isoindoline-1,3-dione |
| GT-06286 | 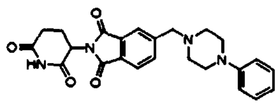 | 2-(2,6-dioxopiperidin-3-yl)-5-((4-phenylpiperazin-1-yl)methyl)isoindoline-1,3-dione | 2-(2,6-dioxopiperidin-3-yl)-5-((4-phenylpiperazin-1-yl)methyl)isoindoline-1,3-dione |
| GT-06287 | 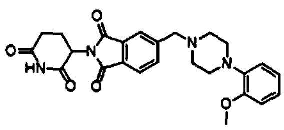 | 2-(2,6-dioxopiperidin-3-yl)-5-((4-(2-methoxyphenyl)piperazin-1-yl)methyl)isoindoline-1,3-dione | 2-(2,6-dioxopiperidin-3-yl)-5-((4-(2-methoxyphenyl)piperazin-1-yl)methyl)isoindoline-1,3-dione |
| GT-06288 | 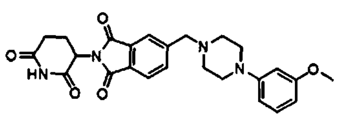 | 2-(2,6-dioxopiperidin-3-yl)-5-((4-(3-methoxyphenyl)piperazin-1-yl)methyl)isoindoline-1,3-dione | 2-(2,6-dioxopiperidin-3-yl)-5-((4-(3-methoxyphenyl)piperazin-1-yl)methyl)isoindoline-1,3-dione |
| GT-06294 | 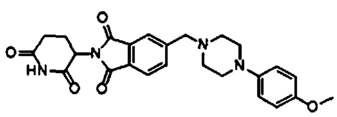 | 2-(2,6-dioxopiperidin-3-yl)-5-((4-(4-methoxyphenyl)piperazin-1-yl)methyl)isoindoline-1,3-dione | 2-(2,6-dioxopiperidin-3-yl)-5-((4-(4-methoxyphenyl)piperazin-1-yl)methyl)isoindoline-1,3-dione |
| GT-06290 | 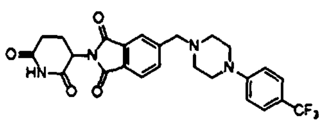 | 2-(2,6-dioxopiperidin-3-yl)-5-((4-(4-(trifluoromethyl)phenyl)pip erazin-1-yl)methyl)isoindoline-1,3-dione | 2-(2,6-dioxopiperidin-3-yl)-5-((4-(4-(trifluoromethyl)phenyl)pip erazin-1-yl)methyl)isoindoline-1,3-dione |

(continued)

| Compound No. | Structure of the compounds | The compounds' name | Chinese translation of the compounds' name |
|---|---|---|---|
| GT-06291 | 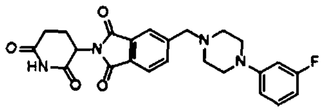 | 2-(2,6-dioxopiperidin-3-yl)-5-((4-(3-fluorophenyl)piperazin-1-yl)methyl)isoindoline-1,3-dione | 2-(2,6-dioxopiperidin-3-yl)-5-((4-(3-fluorophenyl)piperazin-1-yl)methyl)isoindoline-1,3-dione |
| GT-06292 | 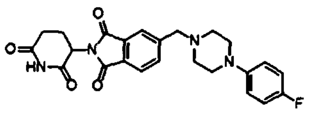 | 2-(2,6-dioxopiperidin-3-yl)-5-((4-(4-fluorophenyl)piperazin-1-yl)methyl)isoindoline-1,3-dione | 2-(2,6-dioxopiperidin-3-yl)-5-((4-(4-fluorophenyl)piperazin-1-yl)methyl)isoindoline-1,3-dione |
| GT-06274 | 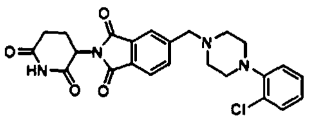 | 5-((4-(2-chlorophenyl)piperazin-1-yl)methyl)-2-(2,6-dioxopiperidin-3-yl)isoindoline-1,3-dione | 5-((4-(2-chlorophenyl)piperazin-1-yl)methyl)-2-(2,6-dioxopiperidin-3-yl)isoindoline-1,3-dione |
| GT-06275 | 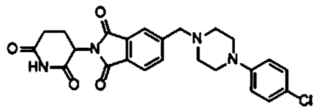 | 5-((4-(4-chlorophenyl)piperazin-1-yl)methyl)-2-(2,6-dioxopiperidin-3-yl)isoindoline-1,3-dione | 5-((4-(4-chlorophenyl)piperazin-1-yl)methyl)-2-(2,6-dioxopiperidin-3-yl)isoindoline-1,3-dione |
| GT-06293 | 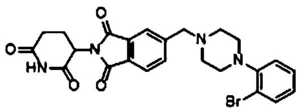 | 5-((4-(2-bromophenyl)piperazin-1-yl)methyl)-2-(2,6-dioxopiperidin-3-yl)isoindoline-1,3-dione | 5-((4-(2-bromophenyl)piperazin-1-yl)methyl)-2-(2,6-dioxopiperidin-3-yl)isoindoline-1,3-dione |
| GT-06289 | 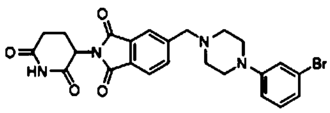 | 5-((4-(3-bromophenyl)piperazin-1-yl)methyl)-2-(2,6-dioxopiperidin-3-yl)isoindoline-1,3-dione | 5-((4-(3-bromophenyl)piperazin-1-yl)methyl)-2-(2,6-dioxopiperidin-3-yl)isoindoline-1,3-dione |
| GT-06295 | 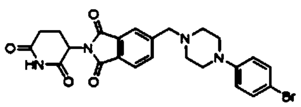 | 5-((4-(4-bromophenyl)piperazin-1-yl)methyl)-2-(2,6-dioxopiperidin-3-yl)isoindoline-1,3-dione | 5-((4-(4-bromophenyl)piperazin-1-yl)methyl)-2-(2,6-dioxopiperidin-3-yl)isoindoline-1,3-dione |
| GT-06296 | 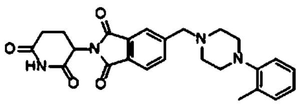 | 2-(2,6-dioxopiperidin-3-yl)-5-((4-(o-tolyl)piperazin-1-yl)methyl)isoindoline-1,3-dione | 2-(2,6-dioxopiperidin-3-yl)-5-((4-(o-tolyl)piperazin-1-yl)methyl)isoindoline-1,3-dione |
| GT-06276 | 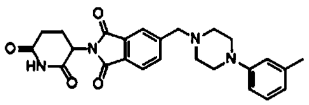 | 2-(2,6-dioxopiperidin-3-yl)-5-((4-(m-tolyl)piperazin-1-yl)methyl)isoindoline-1,3-dione | 2-(2,6-dioxopiperidin-3-yl)-5-((4-(m-tolyl)piperazin-1-yl)methyl)isoindoline-1,3-dione |
| GT-06277 | 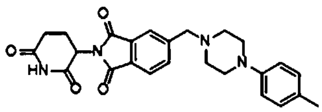 | 2-(2,6-dioxopiperidin-3-yl)-5-((4-(p-tolyl)piperazin-1-yl)methyl)isoindoline-1,3-dione | 2-(2,6-dioxopiperidin-3-yl)-5-((4-(p-tolyl)piperazin-1-yl)methyl)isoindoline-1,3-dione |
| GT-06355 | 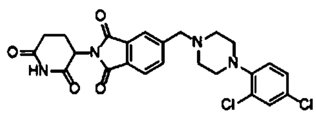 | 5-((4-(2,4-dichlorophenyl)piperazin-1-yl)methyl)-2-(2,6-dioxopiperidin-3-yl)isoindoline-1,3-dione | 5-((4-(2,4-dichlorophenyl)piperazin-1-yl)methyl)-2-(2,6-dioxopiperidin-3-yl)isoindoline-1,3-dione |
| GT-06356 | 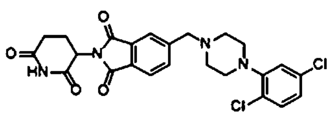 | 5-((4-(2,5-dichlorophenyl)piperazin-1-yl)methyl)-2-(2,6-dioxopiperidin-3-yl)isoindoline-1,3-dione | 5-((4-(2,5-dichlorophenyl)piperazin-1-yl)methyl)-2-(2,6-dioxopiperidin-3-yl)isoindoline-1,3-dione |

(continued)

| Compo und No. | Structure of the compounds | The compounds' name | Chinese translation of the compounds' name |
|---|---|---|---|
| GT-06297 | 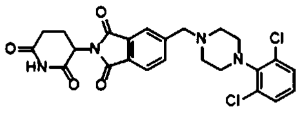 | 5-((4-(2,6-dichlorophenyl)piperazin-1-yl)methyl)-2-(2,6-dioxopiperidin-3-yl)isoindoline-1,3-dione | 5-((4-(2,6-dichlorophenyl)piperazin-1-yl)methyl)-2-(2,6-dioxopiperidin-3-yl)isoindoline-1,3-dione |
| GT-06298 | 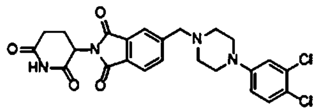 | 5-((4-(3,4-dichlorophenyl)piperazin-1-yl)methyl)-2-(2,6-dioxopiperidin-3-yl)isoindoline-1,3-dione | 5-((4-(3,4-dichlorophenyl)piperazin-1-yl)methyl)-2-(2,6-dioxopiperidin-3-yl)isoindoline-1,3-dione |
| GT-06299 | 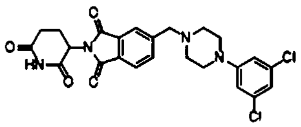 | 5-((4-(3,5-dichlorophenyl)piperazin-1-yl)methyl)-2-(2,6-dioxopiperidin-3-yl)isoindoline-1,3-dione | 5-((4-(3,5-dichlorophenyl)piperazin-1-yl)methyl)-2-(2,6-dioxopiperidin-3-yl)isoindoline-1,3-dione |
| GT-06300 | 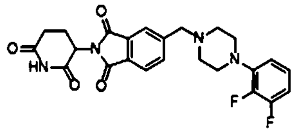 | 5-((4-(2,3-difluorophenyl)piperazin-1-yl)methyl)-2-(2,6-dioxopiperidin-3-yl)isoindoline-1,3-dione | 5-((4-(2,3-difluorophenyl)piperazin-1-yl)methyl)-2-(2,6-dioxopiperidin-3-vl)isoindoline-1,3-dione |
| GT-06361 | 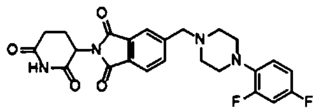 | 5-((4-(2,4-difluorophenyl)piperazin-1-yl)methyl)-2-(2,6-dioxopiperidin-3-yl)isoindoline-1,3-dione | 5-((4-(2,4-difluorophenyl)piperazin-1-yl)methyl)-2-(2,6-dioxopiperidin-3-yl)isoindoline-1,3-dione |
| GT-06362 | 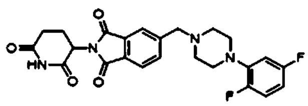 | 5-((4-(2,5-difluorophenyl)piperazin-1-yl)methyl)-2-(2,6-dioxopiperidin-3-yl)isoindoline-1,3-dione | 5-((4-(2,5-difluorophenyl)piperazin-1-yl)methyl)-2-(2,6-dioxopiperidin-3-yl)isoindoline-1,3-dione |
| GT-06363 | 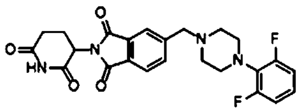 | 5-((4-(2,6-difluorophenyl)piperazin-1-yl)methyl)-2-(2,6-dioxopiperidin-3-yl)isoindoline-1,3-dione | 5-((4-(2,6-difluorophenyl)piperazin-1-yl)methyl)-2-(2,6-dioxopiperidin-3-yl)isoindoline-1,3-dione |
| GT-06364 | 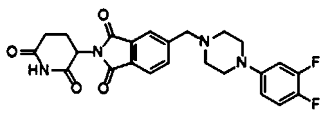 | 5-((4-(3,4-difluorophenyl)piperazin-1-yl)methyl)-2-(2,6-dioxopiperidin-3-yl)isoindoline-1,3-dione | 5-((4-(3,4-difluorophenyl)piperazin-1-yl)methyl)-2-(2,6-dioxopiperidin-3-yl)isoindoline-1,3-dione |
| GT-06365 | 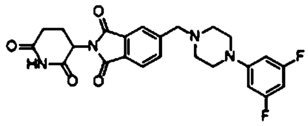 | 5-((4-(3,5-difluorophenyl)piperazin-1-yl)methyl)-2-(2,6-dioxopiperidin-3-yl)isoindoline-1,3-dione | 5-((4-(3,5-difluorophenyl)piperazin-1-yl)methyl)-2-(2,6-dioxopiperidin-3-yl)isoindoline-1,3-dione |
| GT-06366 | 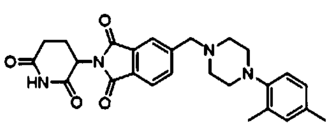 | 5-((4-(2,4-dimethylphenyl)piperazin-1-yl)methyl)-2-(2,6-dioxopiperidin-3-yl)isoindoline-1,3-dione | 5-((4-(2,4-dimethylphenyl)piperazin-1-yl)methyl)-2-(2,6-dioxopiperidin-3-yl)isoindoline-1,3-dione |
| GT-06367 | 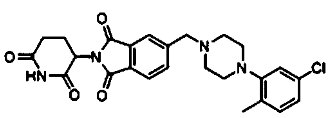 | 5-((4-(5-chloro-2-methylphenyl) piperazin-1-yl)methyl)-2-(2,6-di-oxopiperidin-3-yl)isoindo-line-1,3-dione | 5-((4-(5-chloro-2-methylphenyl) piperazin-1-yl)methyl)-2-(2,6-di-oxopiperidin-3-yl)isoindo-line-1,3-dione |
| GT-06368 | 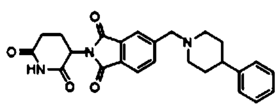 | 2-(2,6-dioxopiperidin-3-yl)-5-((4-phenylpiperidin-1-yl) methyl)isoindoline-1,3-dione | 2-(2,6-dioxopiperidin-3-yl)-5-((4-phenylpiperidin-1-yl) methyl)isoindoline-1,3-dione |

(continued)

| Compo und No. | Structure of the compounds | The compounds' name | Chinese translation of the compounds' name |
|---|---|---|---|
| GT-06369 | 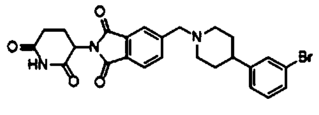 | 5-((4-(3-bromophenyl)piperidin-1-yl)methyl)-2-(2,6-dioxopiperidin-3-yl)isoindoline-1,3-dione | 5-((4-(3-bromophenyl)piperidin-1-yl)methyl)-2-(2,6-dioxopiperidin-3-yl)isoindoline-1,3-dione |
| GT-06370 | 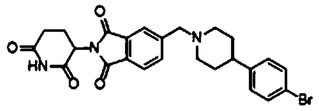 | 5-((4-(4-bromophenyl)piperidin-1-yl)methyl)-2-(2,6-dioxopiperidin-3-yl)isoindoline-1,3-dione | 5-((4-(4-bromophenyl)piperidin-1-yl)methyl)-2-(2,6-dioxopiperidin-3-yl)isoindoline-1,3-dione |
| GT-06371 | 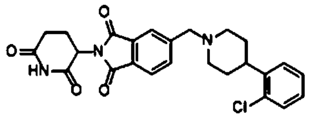 | 5-((4-(2-chlorophenyl)piperidin-1-yl)methyl)-2-(2,6-dioxopiperidin-3-yl)isoindoline-1,3-dione | 5-((4-(2-chlorophenyl)piperidin-1-yl)methyl)-2-(2,6-dioxopiperidin-3-yl)isoindoline-1,3-dione |
| GT-06372 | 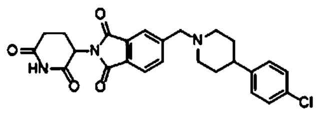 | 5-((4-(4-chlorophenyl)piperidin-1-yl)methyl)-2-(2,6-dioxopiperidin-3-yl)isoindoline-1,3-dione | 5-((4-(4-chlorophenyl)piperidin-1-yl)methyl)-2-(2,6-dioxopiperidin-3-yl)isoindoline-1,3-dione |
| GT-06373 | 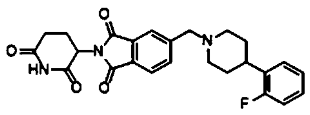 | 2-(2,6-dioxopiperidin-3-yl)-5-((4-(2-fluorophenyl)piperidin-1-yl)methyl)isoindoline-1,3-dione | 2-(2,6-dioxopiperidin-3-yl)-5-((4-(2-fluorophenyl)piperidin-1-yl)methyl)isoindoline-1,3-dione |
| GT-06374 | 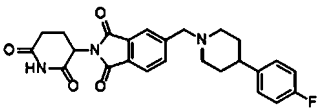 | 2-(2,6-dioxopiperidin-3-yl)-5-((4-(4-fluorophenyl)piperidin-1-yl)methyl)isoindoline-1,3-dione | 2-(2,6-dioxopiperidin-3-yl)-5-((4-(4-fluorophenyl)piperidin-1-yl)methyl)isoindoline-1,3-dione |
| GT-06375 | 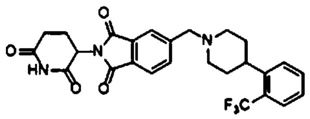 | 2-(2,6-dioxopiperidin-3-yl)-5-((4-(2-(trifluoromethyl)phenyl)pip eridin-1-yl)methyl)isoindoline-1,3-dione | 2-(2,6-dioxopiperidin-3-yl)-5-((4-(2-(trifluoromethyl)phenyl)pip eridin-1-yl)methyl)isoindoline-1,3-dione |
| GT-06357 | 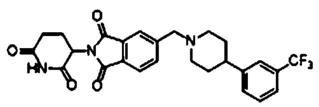 | 2-(2,6-dioxopiperidin-3-yl)-5-((4-(3-(trifluoromethyl)phenyl)pip eridin-1-yl)methyl)isoindoline-1,3-dione | 2-(2,6-dioxopiperidin-3-yl)-5-((4-(3-(trifluoromethyl)phenyl)pip eridin-1-yl)methyl)isoindoline-1,3-dione |
| GT-06358 | 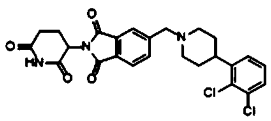 | 5-((4-(2,3-dichlorophenyl)piperidin-1-yl)methyl)-2-(2,6-dioxopiperidin-3-yl)isoindoline-1,3-dione | 5-((4-(2,3-dichlorophenyl)piperidin-1-yl)methyl)-2-(2,6-dioxopiperidin-3-yl)isoindoline-1,3-dione |
| GT-06360 | 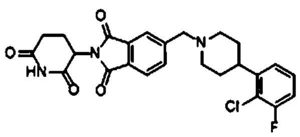 | 5-((4-(2-chloro-3-fluorophenyl) piperidin-1-yl)methyl)-2-(2,6-dioxopiperidin-3-yl)isoindoline-1,3-dione | 5-((4-(2-chloro-3-fluorophenyl) piperidin-1-yl)methyl)-2-(2,6-dioxopiperidin-3-yl)isoindoline-1,3-dione |
| GT-06359 | 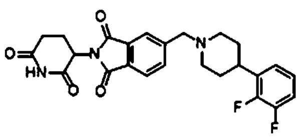 | 5-((4-(2,3-difluorophenyl)piperidin-1-yl)methyl)-2-(2,6-dioxopiperidin-3-yl)isoindoline-1,3-dione | 5-((4-(2,3-difluorophenyl)piperidin-1-yl)methyl)-2-(2,6-dioxopiperidin-3-yl)isoindoline-1,3-dione |
| GT-07842 | 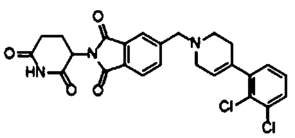 | 5-((4-(2,3-dichlorophenyl)-3,6-dihydropyridin-1(2H)-yl) methyl)-2-(2,6-dioxopiperidin-3-yl)isoindoline-1,3-dione | 5-((4-(2,3-dichlorophenyl)-3,6-dihydropyridin-1(2H)-yl) methyl)-2-(2,6-dioxopiperidin-3-yl)isoindoline-1,3-dione |

(continued)

| Compo und No. | Structure of the compounds | The compounds' name | Chinese translation of the compounds' name |
|---|---|---|---|
| GT-06429 | 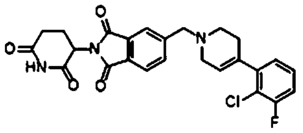 | 5-((4-(2-chloro-3-fluorophenyl)-3,6-dihydropyridin-1(2H)-yl)methyl)-2-(2,6-dioxopiperidin-3-yl)isoindoline-1,3-dione | 5-((4-(2-chloro-3-fluorophenyl)-3,6-dihydropyridin-1(2H)-yl)methyl)-2-(2,6-dioxopiperidin-3-yl)isoindoline-1,3-dione |
| GT-06521 | 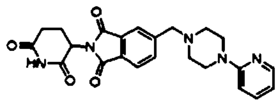 | 2-(2,6-dioxopiperidin-3-yl)-5-((4-(pyridin-2-yl)piperazin-1-yl)methyl)isoindoline-1,3-dione | 2-(2,6-dioxopiperidin-3-yl)-5-((4-(pyridin-2-yl)piperazin-1-yl)methyl)isoindoline-1,3-dione |
| GT-06522 | 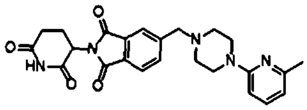 | 2-(2,6-dioxopiperidin-3-yl)-5-((4-(6-methylpyridin-2-yl)piperazin-1-yl)methyl)isoindoline-1,3-dione | 2-(2,6-dioxopiperidin-3-yl)-5-((4-(6-methylpyridin-2-yl)piperazin-1-yl)methyl)isoindoline-1,3-dione |
| GT-06523 | 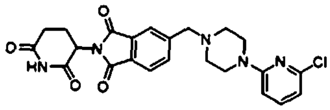 | 5-((4-(6-chloropyridin-2-yl)piperazin-1-yl)methyl)-2-(2,6-dioxopiperidin-3-yl)isoindoline-1,3-dione | 5-((4-(6-chloropyridin-2-yl)piperazin-1-yl)methyl)-2-(2,6-dioxopiperidin-3-yl)isoindoline-1,3-dione |
| GT-06524 | 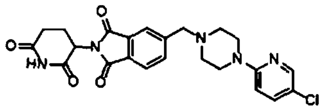 | 5-((4-(5-chloropyridin-2-yl)piperazin-1-yl)methyl)-2-(2,6-dioxopiperidin-3-yl)isoindoline-1,3-dione | 5-((4-(5-chloropyridin-2-yl)piperazin-1-yl)methyl)-2-(2,6-dioxopiperidin-3-yl)isoindoline-1,3-dione |
| GT-06525 | 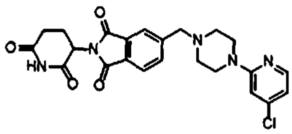 | 5-((4-(4-chloropyridin-2-yl)piperazin-1-yl)methyl)-2-(2,6-dioxopiperidin-3-yl)isoindoline-1,3-dione | 5-((4-(4-chloropyridin-2-yl)piperazin-1-yl)methyl)-2-(2,6-dioxopiperidin-3-yl)isoindoline-1,3-dione |
| GT-07843 | 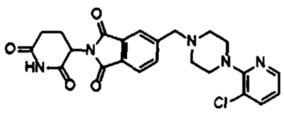 | 5-((4-(3-chloropyridin-2-yl)piperazin-1-yl)methyl)-2-(2,6-dioxopiperidin-3-yl)isoindoline-1,3-dione | 5-((4-(3-chloropyridin-2-yl)piperazin-1-yl)methyl)-2-(2,6-dioxopiperidin-3-yl)isoindoline-1,3-dione |
| GT-06526 | 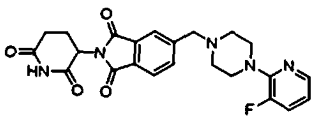 | 2-(2,6-dioxopiperidin-3-yl)-5-((4-(3-fluoropyridin-2-yl)piperazin-1-yl)methyl)isoindoline-1,3-dione | 2-(2,6-dioxopiperidin-3-yl)-5-((4-(3-fluoropyridin-2-yl)piperazin-1-yl)methyl)isoindoline-1,3-dione |
| GT-06527 | 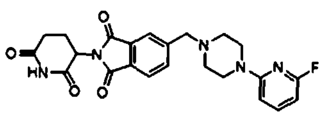 | 2-(2,6-dioxopiperidin-3-yl)-5-((4-(6-fluoropyridin-2-yl)piperazin-1-yl)methyl)isoindoline-1,3-dione | 2-(2,6-dioxopiperidin-3-yl)-5-((4-(6-fluoropyridin-2-yl)piperazin-1-yl)methyl)isoindoline-1,3-dione |
| GT-06528 | 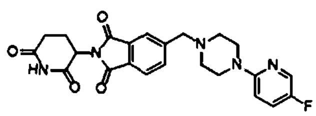 | 2-(2,6-dioxopiperidin-3-yl)-5-((4-(5-fluoropyridin-2-yl)piperazin-1-yl)methyl)isoindoline-1,3-dione | 2-(2,6-dioxopiperidin-3-yl)-5-((4-(5-fluoropyridin-2-yl)piperazin-1-yl)methyl)isoindoline-1,3-dione |
| GT-06529 | 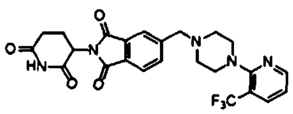 | 2-(2,6-dioxopiperidin-3-yl)-5-((4-(3-(trifluoromethyl)pyridin-2-yl)piperazin-1-yl)methyl)isoindoline-1,3-dione | 2-(2,6-dioxopiperidin-3-yl)-5-((4-(3-(trifluoromethyl)pyridin-2-yl)piperazin-1-yl)methyl)isoindoline-1,3-dione |
| GT-06530 | 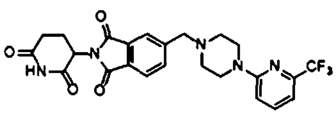 | 2-(2,6-dioxopiperidin-3-yl)-5-((4-(6-(trifluoromethyl)pyridin-2-yl)piperazin-1-yl)methyl)isoindoline-1,3-dione | 2-(2,6-dioxopiperidin-3-yl)-5-((4-(6-(trifluoromethyl)pyridin-2-yl)piperazin-1-yl)methyl)isoindoline-1,3-dione |

(continued)

| Compo und No. | Structure of the compounds | The compounds' name | Chinese translation of the compounds' name |
|---|---|---|---|
| GT-06531 | 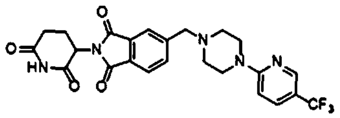 | 2-(2,6-dioxopiperidin-3-yl)-5-((4-(5-(trifluoromethyl)pyridin-2-yl)piperazin-1-yl)methyl) isoindoline-1,3-dione | 2-(2,6-dioxopiperidin-3-yl)-5-((4-(5-(trifluoromethyl)pyridin-2-yl)piperazin-1-yl)methyl) isoindoline-1,3-dione |
| | 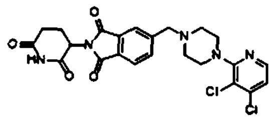 | 5-((4-(3,4-dichloropyridin-2-yl) piperazin-1-yl)methyl)-2-(2,6-dioxopiperidin-3-yl)isoindoline-1,3-dione | 5-((4-(3,4-dichloropyridin-2-yl) piperazin-1-yl)methyl)-2-(2,6-dioxopiperidin-3-yl)isoindoline-1,3-dione |
| GT-06532 | 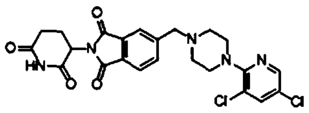 | 5-((4-(3,5-dichloropyridin-2-yl) piperazin-1-yl)methyl)-2-(2,6-dioxopiperidin-3-yl)isoindoline-1,3-dione | 5-((4-(3,5-dichloropyridin-2-yl) piperazin-1-yl)methyl)-2-(2,6-dioxopiperidin-3-yl)isoindoline-1,3-dione |
| | 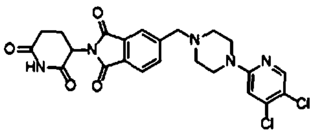 | 5-((4-(4,5-dichloropyridin-2-yl) piperazin-1-yl)methyl)-2-(2,6-dioxopiperidin-3-yl)isoindoline-1,3-dione | 5-((4-(4,5-dichloropyridin-2-yl) piperazin-1-yl)methyl)-2-(2,6-dioxopiperidin-3-yl)isoindoline-1,3-dione |
| GT-06555 | 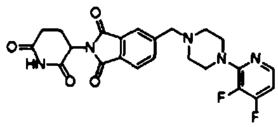 | 5-((4-(3,4-difluoropyridin-2-yl) piperazin-1-yl)methyl)-2-(2,6-dioxopiperidin-3-yl)isoindoline-1,3-dione | 5-((4-(3,4-difluoropyridin-2-yl)piperazin-1-yl)methyl)-2-(2,6-dioxopiperidin-3-yl)isoindoline-1,3-dione |
| GT-08135 | 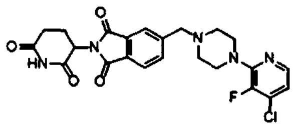 | 5-((4-(4-chloro-3-fluoropyridin-2-yl)piperazin-1-yl) methyl)-2-(2,6-dioxopiperidin-3-yl)isoindoline-1,3-dione | 5-((4-(4-chloro-3-fluoropyridin-2-yl)piperazin-1-yl) methyl)-2-(2,6-dioxopiperidin-3-yl)isoindoline-1,3-dione |
| GT-06533 | 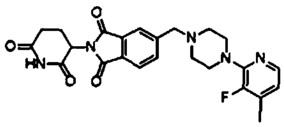 | 2-(2,6-dioxopiperidin-3-yl)-5-((4-(3-fluoro-4-iodopyridin-2-yl)piperazin-1-yl)methyl) isoindoline-1,3-dione | 2-(2,6-dioxopiperidin-3-yl)-5-((4-(3-fluoro-4-iodopyridin-2-yl)piperazin-1-yl)methyl) isoindoline-1,3-dione |
| GT-06601 | 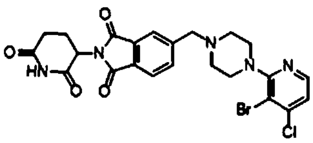 | 5-((4-(3-bromo-4-chloropyridin-2-yl)piperazin-1-yl) methyl)-2-(2,6-dioxopiperidin-3-yl)isoindoline-1,3-dione | 5-((4-(3-bromo-4-chloropyridin-2-yl)piperazin-1-yl) methyl)-2-(2,6-dioxopiperidin-3-yl)isoindoline-1,3-dione |
| GT-06548 | 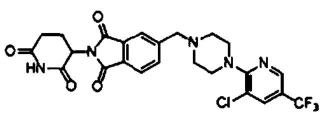 | 5-((4-(3-chloro-5-(trifluoromethyl)pyridin-2-yl)piperazin-1-yl)methyl)-2-(2,6-dioxopiperidin-3-yl)isoindoline-1,3-dione | 5-((4-(3-chloro-5-(trifluoromethyl)pyridin-2-yl)piperazin-1-yl)methyl)-2-(2,6-dioxopiperidin-3-yl)isoindoline-1,3-dione |
| | 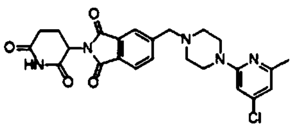 | 5-((4-(4-chloro-6-methylpyridin-2-yl)piperazin-1-yl) methyl)-2-(2,6-dioxopiperidin-3-yl)isoindoline-1,3-dione | 5-((4-(4-chloro-6-methylpyridin-2-yl)piperazin-1-yl) methyl)-2-(2,6-dioxopiperidin-3-yl)isoindoline-1,3-dione |
| GT-06549 | 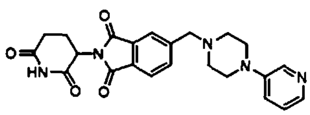 | 2-(2,6-dioxopiperidin-3-yl)-5-((4-(pyridin-3-yl)piperazin-1-yl)methyl)isoindoline-1,3-dione | 2-(2,6-dioxopiperidin-3-yl)-5-((4-(pyridin-3-yl)piperazin-1-yl)methyl)isoindoline-1,3-dione |

(continued)

| Compound No. | Structure of the compounds | The compounds' name | Chinese translation of the compounds' name |
|---|---|---|---|
| GT-06550 | 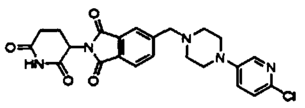 | 5-((4-(6-chloropyridin-3-yl)piperazin-1-yl)methyl)-2-(2,6-dioxopiperidin-3-yl)isoindoline-1,3-dione | 5-((4-(6-chloropyridin-3-yl)piperazin-1-yl)methyl)-2-(2,6-dioxopiperidin-3-vl)isoindoline-1,3-dione |
| GT-06551 | 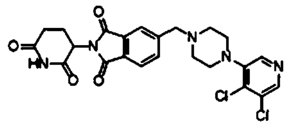 | 5-((4-(4,5-dichloropyridin-3-yl) piperazin-1-yl)methyl)-2-(2,6-dioxopiperidin-3-yl)isoindoline-1,3-dione | 5-((4-(4,5-dichloropyridin-3-yl) piperazin-1-yl)methyl)-2-(2,6-dioxopiperidin-3-vl)isoindoline-1,3-dione |
| GT-06552 | 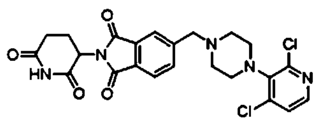 | 5-((4-(2,4-dichloropyridin-3-yl) piperazin-1-yl)methyl)-2-(2,6-dioxopiperidin-3-yl)isoindoline-1,3-dione | 5-((4-(2,4-dichloropyridin-3-yl) piperazin-1-yl)methyl)-2-(2,6-dioxopiperidin-3-vl)isoindoline-1,3-dione |
| GT-06553 | 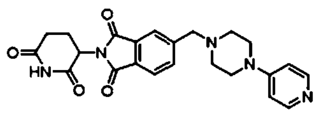 | 2-(2,6-dioxopiperidin-3-yl)-5-((4-(pyridin-4-yl)piperazin-1-yl)methyl)isoindoline-1,3-dione | 2-(2,6-dioxopiperidin-3-yl)-5-((4-(pyridin-4-yl)piperazin-1-yl)methyl)isoindoline-1,3-dione |
| GT-06554 | 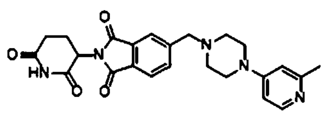 | 2-(2,6-dioxopiperidin-3-yl)-5-((4-(2-methylpyridin-4-yl) piperazin-1-yl)methyl)isoindoline-1,3-dione | 2-(2,6-dioxopiperidin-3-yl)-5-((4-(2-methylpyridin-4-yl) piperazin-1-yl)methyl)isoindoline-1,3-dione |
| GT-06556 | 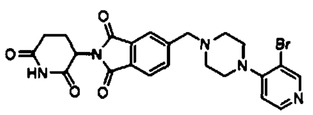 | 5-((4-(3-bromopyridin-4-yl)piperazin-1-yl)methyl)-2-(2,6-dioxopiperidin-3-yl)isoindoline-1,3-dione | 5-((4-(3-bromopyridin-4-yl)piperazin-1-yl)methyl)-2-(2,6-dioxopiperidin-3-vl)isoindoline-1,3-dione |
| GT-06617 | 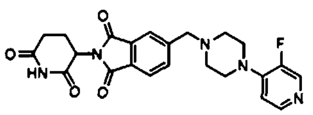 | 2-(2,6-dioxopiperidin-3-yl)-5-((4-(3-fluoropyridin-4-yl)piperazin-1-yl)methyl)isoindoline-1,3-dione | 2-(2,6-dioxopiperidin-3-yl)-5-((4-(3-fluoropyridin-4-yl)piperazin-1-yl)methyl)isoindoline-1,3-dione |
| GT-06618 | 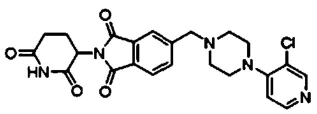 | 5-((4-(3-chloropyridin-4-yl)piperazin-1-yl)methyl)-2-(2,6-dioxopiperidin-3-yl)isoindoline-1,3-dione | 5-((4-(3-chloropyridin-4-yl)piperazin-1-yl)methyl)-2-(2,6-dioxopiperidin-3-vl)isoindoline-1,3-dione |
| GT-06619 | 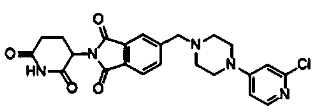 | 5-((4-(2-chloropyridin-4-yl)piperazin-1-yl)methyl)-2-(2,6-dioxopiperidin-3-yl)isoindoline-1,3-dione | 5-((4-(2-chloropyridin-4-yl)piperazin-1-yl)methyl)-2-(2,6-dioxopiperidin-3-yl)isoindoline-1,3-dione |
| GT-06620 | 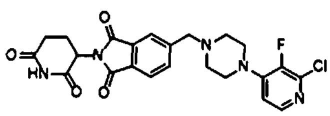 | 5-((4-(2-chloro-3-fluoropyridin-4-yl)piperazin-1-yl) methyl)-2-(2,6-dioxopiperidin-3-yl)isoindoline-1,3-dione | 5-((4-(2-chloro-3-fluoropyridin-4-yl)piperazin-1-yl) methyl)-2-(2,6-dioxopiperidin-3-yl)isoindoline-1,3-dione |
| GT-06621 | 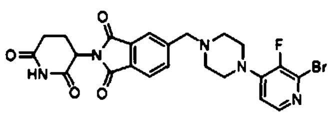 | 5-((4-(2-bromo-3-fluoropyridin-4-yl)piperazin-1-yl) methyl)-2-(2,6-dioxopiperidin-3-yl)isoindoline-1,3-dione | 5-((4-(2-bromo-3-fluoropyridin-4-yl)piperazin-1-yl) methyl)-2-(2,6-dioxopiperidin-3-yl)isoindoline-1,3-dione |
| GT-06622 | 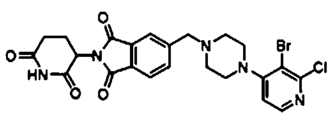 | 5-((4-(3-bromo-2-chloropyridin-4-yl)piperazin-1-yl) methyl)-2-(2,6-dioxopiperidin-3-yl)isoindoline-1,3-dione | 5-((4-(3-bromo-2-chloropyridin-4-yl)piperazin-1-yl) methyl)-2-(2,6-dioxopiperidin-3-vl)isoindoline-1,3-dione |

(continued)

| Compo und No. | Structure of the compounds | The compounds' name | Chinese translation of the compounds' name |
|---|---|---|---|
| GT-06534 | 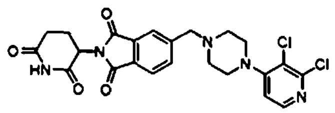 | 5-((4-(2,3-dichloropyridin-4-yl) piperazin-1-yl)methyl)-2-(2,6-di-oxopiperidin-3-yl)isoindo-line-1,3-dione | 5-((4-(2,3-dichloropyridin-4-yl) piperazin-1-yl)methyl)-2-(2,6-di-oxopiperidin-3-yl)isoindo-line-1,3-dione |
| GT-06535 | 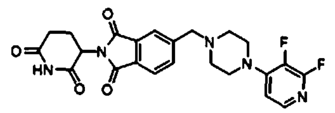 | 5-((4-(2,3-difluoropyridin-4-yl) piperazin-1-yl)methyl)-2-(2,6-di-oxopiperidin-3-yl)isoindo-line-1,3-dione | 5-((4-(2,3-difluoropyridin-4-yl)piperazin-1-yl)methyl)-2-(2,6-di-oxopiperidin-3-yl)isoindo-line-1,3-dione |
| | 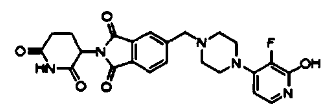 | 2-(2,6-dioxopiperidin-3-yl)-5-((4-(3-fluoro-2-hydroxypyridin-4-yl)piperazin-1-yl)methyl) isoindoline-1,3-dione | 2-(2,6-dioxopiperidin-3-yl)-5-((4-(3-fluoro-2-hydroxypyridin-4-yl)piperazin-1-yl)methyl) isoindoline-1,3-dione |
| GT-06599 | 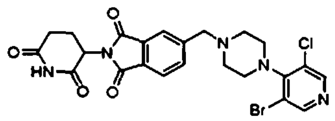 | 5-((4-(3-bromo-5-chloropyridin-4-yl)piperazin-1-yl) methyl)-2-(2,6-dioxopiperidin-3-yl)isoindoline-1,3-dione | 5-((4-(3-bromo-5-chloropyridin-4-yl)piperazin-1-yl) methyl)-2-(2,6-dioxopiperidin-3-yl)isoindoline-1,3-dione |
| GT-06600 | 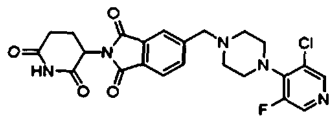 | 5-((4-(3-chloro-5-fluoropyridin-4-yl)piperazin-1-yl) methyl)-2-(2,6-dioxopiperidin-3-yl)isoindoline-1,3-dione | 5-((4-(3-chloro-5-fluoropyridin-4-yl)piperazin-1-yl) methyl)-2-(2,6-dioxopiperidin-3-yl)isoindoline-1,3-dione |
| GT-06602 | 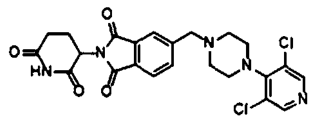 | 5-((4-(3,5-dichloropyridin-4-yl) piperazin-1-yl)methyl)-2-(2,6-di-oxopiperidin-3-yl)isoindo-line-1,3-dione | 5-((4-(3,5-dichloropyridin-4-yl) piperazin-1-yl)methyl)-2-(2,6-di-oxopiperidin-3-yl)isoindo-line-1,3-dione |
| GT-06603 | 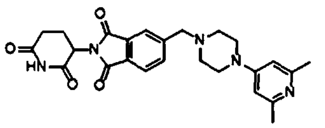 | 5-((4-(2,6-dimethylpyridin-4-yl) piperazin-1-yl)methyl)-2-(2,6-di-oxopiperidin-3-yl)isoindo-line-1,3-dione | 5-((4-(2,6-dimethylpyridin-4-yl) piperazin-1-yl)methyl)-2-(2,6-di-oxopiperidin-3-yl)isoindo-line-1,3-dione |
| GT-06604 | 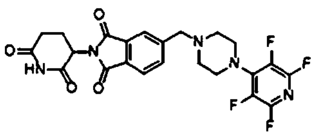 | 2-(2,6-dioxopiperidin-3-yl)-5-((4-(perfluoropyridin-4-yl) piperazin-1-yl)methyl)isoindoline-1,3-dione | 2-(2,6-dioxopiperidin-3-yl)-5-((4-(perfluoropyridin-4-yl) piperazin-1-yl)methyl)isoindoline-1,3-dione |
| GT-07844 | 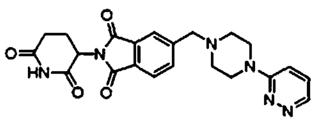 | 2-(2,6-dioxopiperidin-3-yl)-5-((4-(pyridazin-3-yl)piperazin-1-yl)methyl)isoindoline-1,3-dione | 2-(2,6-dioxopiperidin-3-yl)-5-((4-(pyridazin-3-yl)piperazin-1-yl)methyl)isoindoline-1,3-dione |
| GT-06605 | 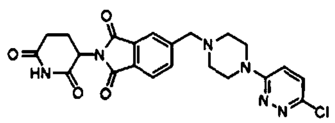 | 5-((4-(6-chloropyridazin-3-yl)piperazin-1-yl)methyl)-2-(2,6-di-oxopiperidin-3-yl)isoindo-line-1,3-dione | 5-((4-(6-chloropyridazin-3-yl)piperazin-1-yl)methyl)-2-(2,6-di-oxopiperidin-3-yl)isoindo-line-1,3-dione |
| GT-06606 | 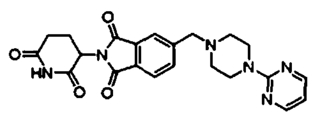 | 2-(2,6-dioxopiperidin-3-yl)-5-((4-(pyrimidin-2-yl)piperazin-1-yl)methyl)isoindoline-1,3-dione | 2-(2,6-dioxopiperidin- 3-yl)-5-((4-(pyrimidin-2-yl)piperazin-1-yl)methyl)isoindoline-1,3-dione |
| GT-07060 | 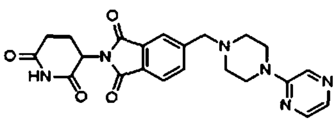 | 2-(2,6-dioxopiperidin-3-yl)-5-((4-(pyrazin-2-yl)piperazin-1-yl)methyl)isoindoline-1,3-dione | 2-(2,6-dioxopiperidin-3-yl)-5-((4-(pyrazin-2-yl)piperazin-1-yl)methyl)isoindoline-1,3-dione |

(continued)

| Compo und No. | Structure of the compounds | The compounds' name | Chinese translation of the compounds' name |
|---|---|---|---|
| GT-06623 | 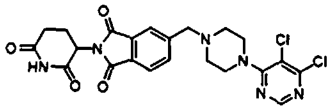 | 5-((4-(5,6-dichloropyrimidin-4-yl)piperazin-1-yl)methyl)-2-(2,6-dioxopiperidin-3-yl)isoindoline-1,3-dione | 5-((4-(5,6-dichloropyrimidin-4-yl)piperazin-1-yl)methyl)-2-(2,6-dioxopiperidin-3-yl)isoindoline-1,3-dione |
| GT-06625 | 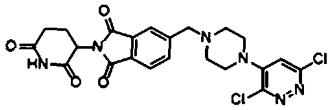 | 5-((4-(3,6-dichloropyridazin-4-yl)piperazin-1-yl)methyl)-2-(2,6-dioxopiperidin-3-yl)isoindoline-1,3-dione | 5-((4-(3,6-dichloropyridazin-4-yl)piperazin-1-yl)methyl)-2-(2,6-dioxopiperidin-3-yl)isoindoline-1,3-dione |
| GT-06626 | 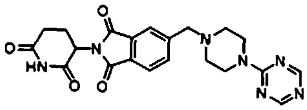 | 5-((4-(1,3,5-triazin-2-yl)piperazin-1-yl)methyl)-2-(2,6-dioxopiperidin-3-yl)isoindoline-1,3-dione | 5-((4-(1,3,5-triazin-2-yl)piperazin-1-yl)methyl)-2-(2,6-dioxopiperidin-3-yl)isoindoline-1,3-dione |
| GT-06607 | 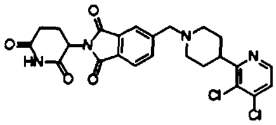 | 5-((4-(3,4-dichloropyridin-2-yl) piperidin-1-yl)methyl)-2-(2,6-dioxopiperidin-3-yl)isoindoline-1,3-dione | 5-((4-(3,4-dichloropyridin-2-yl) piperidin-1-yl)methyl)-2-(2,6-dioxopiperidin-3-yl)isoindoline-1,3-dione |
| GT-06615 | 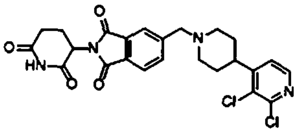 | 5-((4-(2,3-dichloropyridin-4-yl) piperidin-1-yl)methyl)-2-(2,6-dioxopiperidin-3-yl)isoindoline-1,3-dione | 5-((4-(2,3-dichloropyridin-4-yl) piperidin-1-yl)methyl)-2-(2,6-dioxopiperidin-3-yl)isoindoline-1,3-dione |
| GT-06613 | 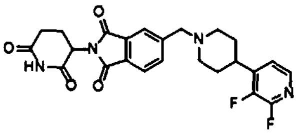 | 5-((4-(2,3-difluoropyridin-4-yl) piperidin-1-yl)methyl)-2-(2,6-dioxopiperidin-3-yl)isoindoline-1,3-dione | 5-((4-(2,3-difluoropyridin-4-yl)piperidin-1-yl)methyl)-2-(2,6-dioxopiperidin-3-yl)isoindoline-1,3-dione |
| GT-06881 | 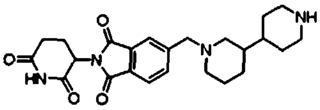 | 5-([3,4'-bipiperidin]-1-ylmethyl)-2-(2,6-dioxopiperidin-3-yl)isoindoline-1,3-dione | 5-([3,4'-bipiperidin]-1-ylmethyl)-2-(2,6-dioxopiperidin-3-yl)isoindoline-1,3-dione |
| GT-06608 | 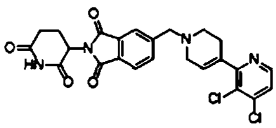 | 5-((3,4-dichloro-3',6'-dihydro-[2,4'-bipyridin]-1'(2'H)-yl) methyl)-2-(2,6-dioxopiperidin-3-yl)isoindoline-1,3-dione | 5-((3,4-dichloro-3',6'-dihydro-[2,4'-bipyridin]-1'(2'H)-yl) methyl)-2-(2,6-dioxopiperidin-3-yl)isoindoline-1,3-dione |
| GT-06609 | 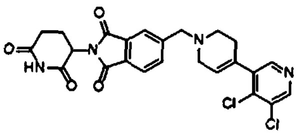 | 5-((4,5-dichloro-3',6'-dihydro-[3,4'-bipyridin]-1'(2'H)-yl) methyl)-2-(2,6-dioxopiperidin-3-yl)isoindoline-1,3-dione | 5-((4,5-dichloro-3',6'-dihydro-[3,4'-bipyridin]-1'(2'H)-yl) methyl)-2-(2,6-dioxopiperidin-3-yl)isoindoline-1,3-dione |
| GT-06611 | 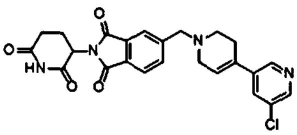 | 5-((5-chloro-3',6'-dihydro-[3,4'-bipyridin]-1'(2'H)-yl) methyl)-2-(2,6-dioxopiperidin-3-yl)isoindoline-1,3-dione | 5-((5-chloro-3',6'-dihydro-[3,4'-bipyridin]-1'(2'H)-yl) methyl)-2-(2,6-dioxopiperidin-3-yl)isoindoline-1,3-dione |
| GT-06616 | 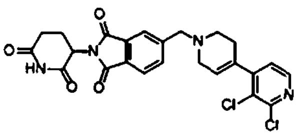 | 5-((2',3'-dichloro-3,6-dihydro-[4,4'-bipyridin]-1(2H)-yl) methyl)-2-(2,6-dioxopiperidin-3-yl)isoindoline-1,3-dione | 5-((2',3'-dichloro-3,6-dihydro-[4,4'-bipyridin]-1(2H)-yl) methyl)-2-(2,6-dioxopiperidin-3-yl)isoindoline-1,3-dione |

(continued)

| Compound No. | Structure of the compounds | The compounds' name | Chinese translation of the compounds' name |
|---|---|---|---|
| GT-06612 | 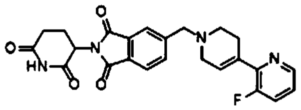 | 2-(2,6-dioxopiperidin-3-yl)-5-((3-fluoro-3',6'-dihydro-[2,4'-bipyridin]-1'(2'H)-yl)methyl)isoindoline-1,3-dione | 2-(2,6-dioxopiperidin-3-yl)-5-((3-fluoro-3',6'-dihydro-[2,4'-bipyridin]-1'(2'H)-yl)methyl)isoindoline-1,3-dione |
| GT-06614 | 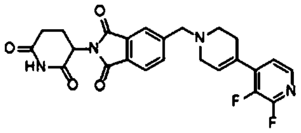 | 5-((2',3'-difluoro-3,6-dihydro-[4,4'-bipyridin]-1(2H)-yl)methyl)-2-(2,6-dioxopiperidin-3-yl)isoindoline-1,3-dione | 5-((2',3'-difluoro-3,6-dihydro-[4,4'-bipyridin]-1(2H)-yl)methyl)-2-(2,6-dioxopiperidin-3-yl)isoindoline-1,3-dione |
| GT-06688 | 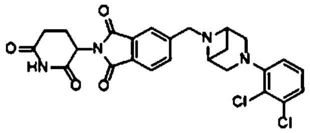 | 5-((3-(2,3-dichlorophenyl)-3,6-diazabicyclo[3.1.1]heptan-6-yl)methyl)-2-(2,6-dioxopiperidin-3-yl)isoindoline-1,3-dione | 5-((3-(2,3-dichlorophenyl)-3,6-diazabicyclo[3.1.1]heptan-6-yl)methyl)-2-(2,6-dioxopiperidin-3-yl)isoindoline-1,3-dione |
| GT-06689 | 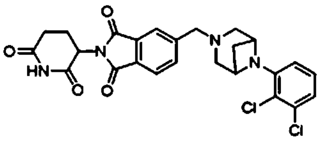 | 5-((6-(2,3-dichlorophenyl)-3,6-diazabicyclo[3.1.1]heptan-3-yl)methyl)-2-(2,6-dioxopiperidin-3-yl)isoindoline-1,3-dione | 5-((6-(2,3-dichlorophenyl)-3,6-diazabicyclo[3.1.1]heptan-3-yl)methyl)-2-(2,6-dioxopiperidin-3-yl)isoindoline-1,3-dione |
| GT-06690 | 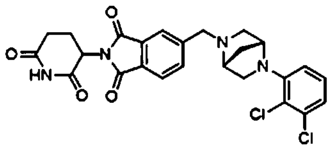 | 5-(((1R,4R)-5-(2,3-dichlorophenyl)-2,5-diazabicyclo[2.2.1]heptan-2-yl)methyl)-2-(2,6-dioxopiperidin-3-yl)isoindoline-1,3-dione | 5-(((1R,4R)-5-(2,3-dichlorophenyl)-2,5-diazabicyclo[2.2.1]heptan-2-yl)methyl)-2-(2,6-dioxopiperidin-3-yl)isoindoline-1,3-dione |
| GT-06691 | 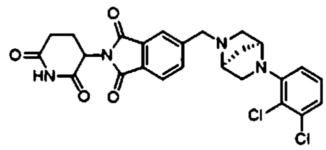 | 5-(((1S,4S)-5-(2,3-dichlorophenyl)-2,5-diazabicyclo[2.2.1]heptan-2-yl)methyl)-2-(2,6-dioxopiperidin-3-yl)isoindoline-1,3-dione | 5-(((1S,4S)-5-(2,3-dichlorophenyl)-2,5-diazabicyclo[2.2.1]heptan-2-yl)methyl)-2-(2,6-dioxopiperidin-3-yl)isoindoline-1,3-dione |
| GT-06692 | 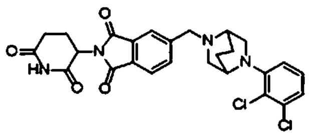 | 5-((5-(2,3-dichlorophenyl)-2,5-diazabicyclo[2.2.2]octan-2-yl)methyl)-2-(2,6-dioxopiperidin-3-yl)isoindoline-1,3-dione | 5-((5-(2,3-dichlorophenyl)-2,5-diazabicyclo[2.2.2]octan-2-yl)methyl)-2-(2,6-dioxopiperidin-3-yl)isoindoline-1,3-dione |
| GT-06693 | 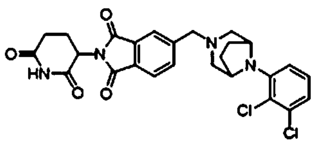 | 5-((8-(2,3-dichlorophenyl)-3,8-diazabicyclo[3.2.1]octan-3-yl)methyl)-2-(2,6-dioxopiperidin-3-yl)isoindoline-1,3-dione | 5-((8-(2,3-dichlorophenyl)-3,8-diazabicyclo[3.2.1]octan-3-yl)methyl)-2-(2,6-dioxopiperidin-3-yl)isoindoline-1,3-dione |
| GT-06694 | 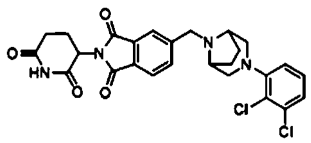 | 5-((3-(2,3-dichlorophenyl)-3,8-diazabicyclo[3.2.1]octan-8-yl)methyl)-2-(2,6-dioxopiperidin-3-yl)isoindoline-1,3-dione | 5-((3-(2,3-dichlorophenyl)-3,8-diazabicyclo[3.2.1]octan-8-yl)methyl)-2-(2,6-dioxopiperidin-3-yl)isoindoline-1,3-dione |
| GT-06695 | 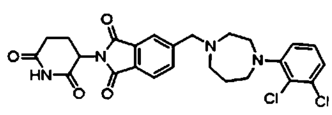 | 5-((4-(2,3-dichlorophenyl)-1,4-diazepan-1-yl)methyl)-2-(2,6-dioxopiperidin-3-yl)isoindoline-1,3-dione | 5-((4-(2,3-dichlorophenyl)-1,4-diazepan-1-yl)methyl)-2-(2,6-dioxopiperidin-3-yl)isoindoline-1,3-dione |
| GT-04771 | 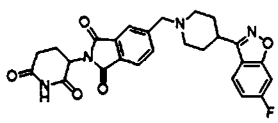 | 2-(2,6-dioxopiperidin-3-yl)-5-((4-(6-fluorobenzo[d]isoxazol-3-yl)piperidin-1-yl)methyl)isoindoline-1,3-dione | 2-(2,6-dioxopiperidin-3-yl)-5-((4-(6-fluorobenzo[d]isoxazol-3-yl)piperidin-1-yl)methyl)isoindoline-1,3-dione |

(continued)

| Compound No. | Structure of the compounds | The compounds' name | Chinese translation of the compounds' name |
| --- | --- | --- | --- |
| GT-06627 | 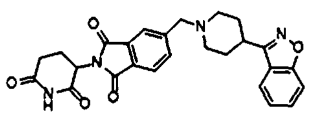 | 5-((4-(benzo[d]isoxazol-3-yl)piperidin-1-yl)methyl)-2-(2,6-dioxopiperidin-3-yl)isoindoline-1,3-dione | 5-((4-(benzo[d]isoxazol-3-yl)piperidin-1-yl)methyl)-2-(2,6-dioxopiperidin-3-yl)isoindoline-1,3-dione |
| GT-06628 | 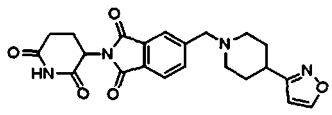 | 2-(2,6-dioxopiperidin-3-yl)-5-((4-(isoxazol-3-yl)piperidin-1-yl)methyl)isoindoline-1,3-dione | 2-(2,6-dioxopiperidin-3-yl)-5-((4-(isoxazol-3-yl)piperidin-1-yl)methyl)isoindoline-1,3-dione |
| GT-06629 | 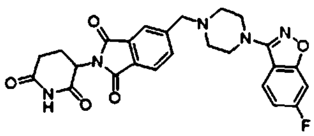 | 2-(2,6-dioxopiperidin-3-yl)-5-((4-(6-fluorobenzo[d]isoxazol-3-yl)piperazin-1-yl)methyl) isoindoline-1,3-dione | 2-(2,6-dioxopiperidin-3-yl)-5-((4-(6-fluorobenzo[d]isoxazol-3-yl)piperazin-1-yl)methyl) isoindoline-1,3-dione |
| GT-06630 | 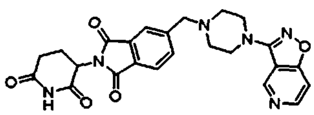 | 2-(2,6-dioxopiperidin-3-yl)-5-((4-(isoxazolo[4,5-c]pyridin-3-yl)piperazin-1-yl)methyl) isoindoline-1,3-dione | 2-(2,6-dioxopiperidin-3-yl)-5-((4-(isoxazolo[4,5-c]pyridin-3-yl)piperazin-1-yl)methyl) isoindoline-1,3-dione |
| GT-06631 | 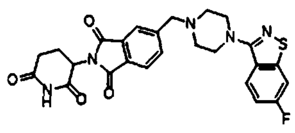 | 2-(2,6-dioxopiperidin-3-yl)-5-((4-(6-fluorobenzo[d]isothiazol-3-yl)piperazin-1-yl) methyl)isoindoline-1,3-dione | 2-(2,6-dioxopiperidin-3-yl)-5-((4-(6-fluorobenzo[d]isothiazol-3-yl)piperazin-1-yl) methyl)isoindoline-1,3-dione |
| GT-06632 | 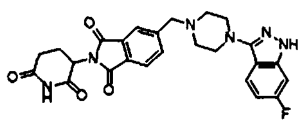 | 2-(2,6-dioxopiperidin-3-yl)-5-((4-(6-fluoro-1H-indazol-3-yl)piperazin-1-yl)methyl)isoindoline-1,3-dione | 2-(2,6-dioxopiperidin-3-yl)-5-((4-(6-fluoro-1H-indazol-3-yl)piperazin-1-yl)methyl)isoindoline-1,3-dione |
| GT-06707 | 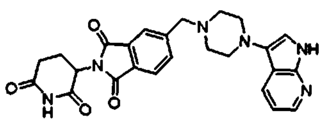 | 5-((4-(1H-pyrrolo[2,3-b]pyridin-3-yl)piperazin-1-yl) methyl)-2-(2,6-dioxopiperidin-3-yl)isoindoline-1,3-dione | 5-((4-(1H-pyrrolo[2,3-b]pyridin-3-yl)piperazin-1-yl) methyl)-2-(2,6-dioxopiperidin-3-yl)isoindoline-1,3-dione |
| GT-06633 | 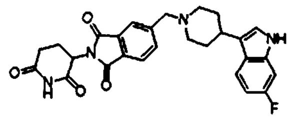 | 2-(2,6-dioxopiperidin-3-yl)-5-((4-(6-fluoro-1H-indol-3-yl) piperidin-1-yl)methyl)isoindoline-1,3-dione | 2-(2,6-dioxopiperidin-3-yl)-5-((4-(6-fluoro-1H-indol-3-yl) piperidin-1-yl)methyl)isoindoline-1,3-dione |
| GT-06635 | 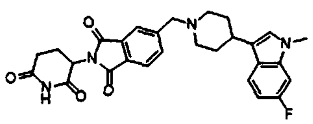 | 2-(2,6-dioxopiperidin-3-yl)-5-((4-(6-fluoro-1-methyl-1H-indol-3-yl)piperidin-1-yl)methyl) isoindoline-1,3-dione | 2-(2,6-dioxopiperidin-3-yl)-5-((4-(6-fluoro-1-methyl-1H-indol-3-yl)piperidin-1-yl)methyl) isoindoline-1,3-dione |
| GT-06637 | 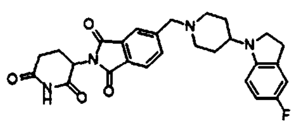 | 2-(2,6-dioxopiperidin-3-yl)-5-((4-(5-fluoroindolin-1-yl)piperidin-1-yl)methyl)isoindoline-1,3-dione | 2-(2,6-dioxopiperidin-3-yl)-5-((4-(5-fluoroindolin-1-yl)piperidin-1-yl)methyl)isoindoline-1,3-dione |
| GT-06638 | 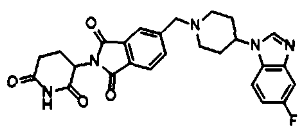 | 2-(2,6-dioxopiperidin-3-yl)-5-((4-(5-fluoro-1H-benzo[d] imidazol-1-yl)piperidin-1-yl) methyl)isoindoline-1,3-dione | 2-(2,6-dioxopiperidin-3-yl)-5-((4-(5-fluoro-1H-benzo[d] imidazol-1-yl)piperidin-1-yl) methyl)isoindoline-1,3-dione |

(continued)

| Compo und No. | Structure of the compounds | The compounds' name | Chinese translation of the compounds' name |
|---|---|---|---|
| GT-06704 | 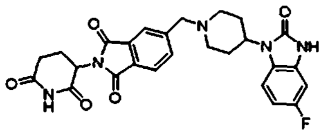 | 2-(2,6-dioxopiperidin-3-yl)-5-((4-(5-fluoro-2-oxo-2,3-dihydro-1H-benzo[d]imidazol-1-yl)piperidin-1-yl)methyl)isoindoline-1,3-dione | 2-(2,6-dioxopiperidin-3-yl)-5-((4-(5-fluoro-2-oxo-2,3-dihydro-1H-benzo[d]imidazol-1-yl)piperidin-1-yl)methyl)isoindoline-1,3-dione |
| GT-06705 | 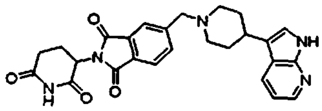 | 5-((4-(1H-pyrrolo[2,3-b]pyridin-3-yl)piperidin-1-yl) methyl)-2-(2,6-dioxopiperidin-3-yl)isoindoline-1,3-dione | 5-((4-(1H-pyrrolo[2,3-b]pyridin-3-yl)piperidin-1-yl) methyl)-2-(2,6-dioxopiperidin-3-yl)isoindoline-1,3-dione |
| GT-06706 | 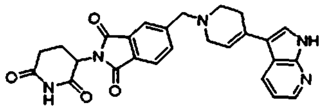 | 5-((4-(1H-pyrrolo[2,3-b]pyridin-3-yl)-3,6-dihydropyridin-1(2H)-yl)methyl)-2-(2,6-dioxopiperidin-3-yl)isoindoline-1,3-dione | 5-((4-(1H-pyrrolo[2,3-b]pyridin-3-yl)-3,6-dihydropyridin-1(2H)-yl)methyl)-2-(2,6-dioxopiperidin-3-yl)isoindoline-1,3-dione |
| GT-06634 | 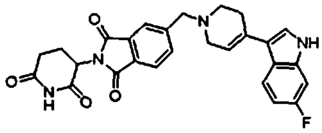 | 2-(2,6-dioxopiperidin-3-yl)-5-((4-(6-fluoro-1H-indol-3-yl)-3,6-dihydropyridin-1(2H)-yl) methyl)isoindoline-1,3-dione | 2-(2,6-dioxopiperidin-3-yl)-5-((4-(6-fluoro-1H-indol-3-yl)-3,6-dihydropyridin-1(2H)-yl) methyl)isoindoline-1,3-dione |
| GT-06636 | 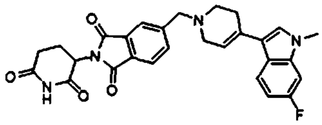 | 2-(2,6-dioxopiperidin-3-yl)-5-((4-(6-fluoro-1-methyl-1H-indol-3-yl)-3,6-dihydropyridin-1(2H)-yl)methyl)isoindoline-1,3-dione | 2-(2,6-dioxopiperidin-3-yl)-5-((4-(6-fluoro-1-methyl-1H-indol-3-yl)-3,6-dihydropyridin-1(2H)-yl)methyl)isoindoline-1,3-dione |
| GT-06234 | 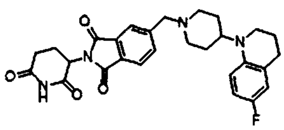 | 2-(2,6-dioxopiperidin-3-yl)-5-((4-(6-fluoro-3,4-dihydroquinolin-1(2H)-yl)piperidin-1-yl) methyl)isoindoline-1,3-dione | 2-(2,6-dioxopiperidin-3-yl)-5-((4-(6-fluoro-3,4-dihydroquinolin-1(2H)-yl)piperidin-1-yl) methyl)isoindoline-1,3-dione |
| GT-06235 | 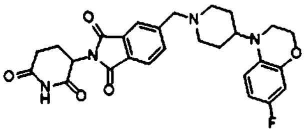 | 2-(2,6-dioxopiperidin-3-yl)-5-((4-(7-fluoro-2,3-dihydro-4H-benzo[b][1,4]oxazin-4-yl)piperidin-1-yl)methyl)isoindoline-1,3-dione | 2-(2,6-dioxopiperidin-3-yl)-5-((4-(7-fluoro-2,3-dihydro-4H-benzo[b][1,4]oxazin-4-yl)piperidin-1-yl)methyl)isoindoline-1,3-dione |
| GT-06236 | 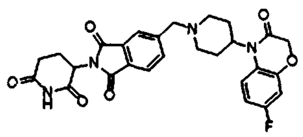 | 2-(2,6-dioxopiperidin-3-yl)-5-((4-(7-fluoro-3-oxo-2,3-dihydro-4H-benzo[b][1,4]oxazin-4-yl)piperidin-1-yl)methyl) isoindoline-1,3-dione | 2-(2,6-dioxopiperidin-3-yl)-5-((4-(7-fluoro-3-oxo-2,3-dihydro-4H-benzo[b][1,4]oxazin-4-yl)piperidin-1-yl)methyl) isoindoline-1,3-dione |
| GT-06237 | 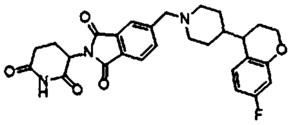 | 2-(2,6-dioxopiperidin-3-yl)-5-((4-(7-fluorochroman-4-yl) piperidin-1-yl)methyl)isoindoline-1,3-dione | 2-(2,6-dioxopiperidin-3-yl)-5-((4-(7-fluorochroman-4-yl) piperidin-1-yl)methyl)isoindoline-1,3-dione |
| GT-06238 | 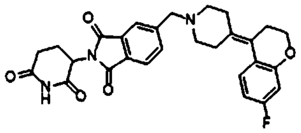 | 2-(2,6-dioxopiperidin-3-yl)-5-((4-(7-fluorochroman-4-ylidene)piperidin-1-yl)methyl)isoindoline-1,3-dione | 2-(2,6-dioxopiperidin-3-yl)-5-((4-(7-fluorochroman-4-ylidene)piperidin-1-yl)methyl)isoindoline-1,3-dione |

(continued)

| Compound No. | Structure of the compounds | The compounds' name | Chinese translation of the compounds' name |
|---|---|---|---|
| GT-06263 | 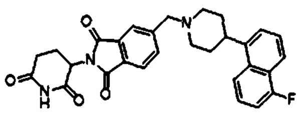 | 2-(2,6-dioxopiperidin-3-yl)-5-((4-(5-fluoronaphthalen-1-yl)piperidin-1-yl)methyl)isoindoline-1,3-dione | 2-(2,6-dioxopiperidin-3-yl)-5-((4-(5-fluoronaphthalen-1-yl)piperidin-1-yl)methyl)isoindoline-1,3-dione |
| GT-06265 | 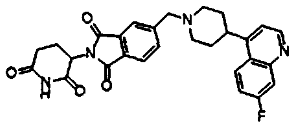 | 2-(2,6-dioxopiperidin-3-yl)-5-((4-(7-fluoroquinolin-4-yl)piperidin-1-yl)methyl)isoindoline-1,3-dione | 2-(2,6-dioxopiperidin-3-yl)-5-((4-(7-fluoroquinolin-4-yl)piperidin-1-yl)methyl)isoindoline-1,3-dione |
| GT-06266 | 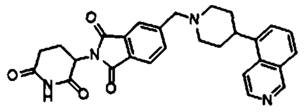 | 2-(2,6-dioxopiperidin-3-yl)-5-((4-(isoquinolin-5-yl)piperidin-1-yl)methyl)isoindoline-1,3-dione | 2-(2,6-dioxopiperidin-3-yl)-5-((4-(isoquinolin-5-yl)piperidin-1-yl)methyl)isoindoline-1,3-dione |
| GT-06264 | 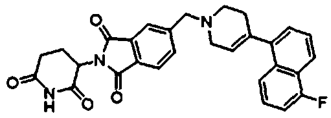 | 2-(2,6-dioxopiperidin-3-yl)-5-((4-(5-fluoronaphthalen-1-yl)-3,6-dihydropyridin-1(2H)-yl)methyl)isoindoline-1,3-dione | 2-(2,6-dioxopiperidin-3-yl)-5-((4-(5-fluoronaphthalen-1-yl)-3,6-dihydropyridin-1(2H)-yl)methyl)isoindoline-1,3-dione |
| GT-06268 | 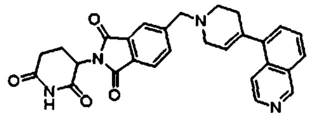 | 2-(2,6-dioxopiperidin-3-yl)-5-((4-(isoquinolin-5-yl)-3,6-dihydropyridin-1(2H)-yl)methyl)isoindoline-1,3-dione | 2-(2,6-dioxopiperidin-3-yl)-5-((4-(isoquinolin-5-yl)-3,6-dihydropyridin-1(2H)-yl)methyl)isoindoline-1,3-dione |
| GT-06269 | 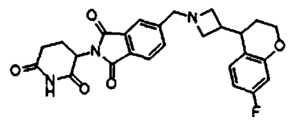 | 2-(2,6-dioxopiperidin-3-yl)-5-((3-(7-fluorochroman-4-yl)azetidin-1-yl)methyl)isoindoline-1,3-dione | 2-(2,6-dioxopiperidin-3-yl)-5-((3-(7-fluorochroman-4-yl)azetidin-1-yl)methyl)isoindoline-1,3-dione |
| GT-08134 | 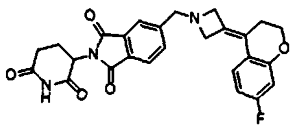 | 2-(2,6-dioxopiperidin-3-yl)-5-((3-(7-fluorochroman-4-ylidene)azetidin-1-yl)methyl)isoindoline-1,3-dione | 2-(2,6-dioxopiperidin-3-yl)-5-((3-(7-fluorochroman-4-ylidene)azetidin-1-yl)methyl)isoindoline-1,3-dione |
| GT-06270 | 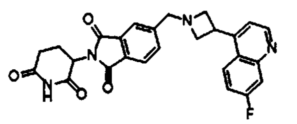 | 2-(2,6-dioxopiperidin-3-yl)-5-((3-(7-fluoroquinolin-4-yl)azetidin-1-yl)methyl)isoindoline-1,3-dione | 2-(2,6-dioxopiperidin-3-yl)-5-((3-(7-fluoroquinolin-4-yl)azetidin-1-yl)methyl)isoindoline-1,3-dione |
| GT-06271 | 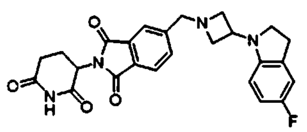 | 2-(2,6-dioxopiperidin-3-yl)-5-((3-(5-fluoroindolin-1-yl)azetidin-1-yl)methyl)isoindoline-1,3-dione | 2-(2,6-dioxopiperidin-3-yl)-5-((3-(5-fluoroindolin-1-yl)azetidin-1-yl)methyl)isoindoline-1,3-dione |
| GT-06272 | 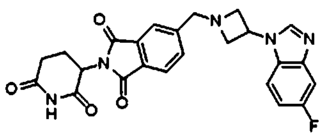 | 2-(2,6-dioxopiperidin-3-yl)-5-((3-(5-fluoro-1H-benzo[d]imidazol-1-yl)azetidin-1-yl)methyl)isoindoline-1,3-dione | 2-(2,6-dioxopiperidin-3-yl)-5-((3-(5-fluoro-1H-benzo[d]imidazol-1-yl)azetidin-1-yl)methyl)isoindoline-1,3-dione |
| GT-06303 | 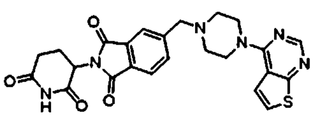 | 2-(2,6-dioxopiperidin-3-yl)-5-((4-(thieno[2,3-d]pyrimidin-4-yl)piperazin-1-yl)methyl)isoindoline-1,3-dione | 2-(2,6-dioxopiperidin-3-yl)-5-((4-(thieno[2,3-d]pyrimidin-4-yl)piperazin-1-yl)methyl)isoindoline-1,3-dione |
| GT-06304 | 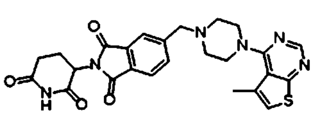 | 2-(2,6-dioxopiperidin-3-yl)-5-((4-(5-methylthieno[2,3-d]pyrimidin-4-yl)piperazin-1-yl)methyl)isoindoline-1,3-dione | 2-(2,6-dioxopiperidin-3-yl)-5-((4-(5-methylthieno[2,3-d]pyrimidin-4-yl)piperazin-1-yl)methyl)isoindoline-1,3-dione |

(continued)

| Compound No. | Structure of the compounds | The compounds' name | Chinese translation of the compounds' name |
|---|---|---|---|
| GT-06305 | 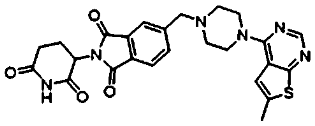 | 2-(2,6-dioxopiperidin-3-yl)-5-((4-(6-methylthieno[2,3-d]pyrimidin-4-yl)piperazin-1-yl)methyl)isoindoline-1,3-dione | 2-(2,6-dioxopiperidin-3-yl)-5-((4-(6-methylthieno[2,3-d]pyrimidin-4-yl)piperazin-1-yl)methyl)isoindoline-1,3-dione |
| GT-06306 | 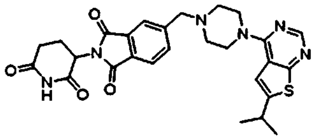 | 2-(2,6-dioxopiperidin-3-yl)-5-((4-(6-isopropylthieno[2,3-d]pyrimidin-4-yl)piperazin-1-yl)methyl)isoindoline-1,3-dione | 2-(2,6-dioxopiperidin-3-yl)-5-((4-(6-isopropylthieno[2,3-d]pyrimidin-4-yl)piperazin-1-yl)methyl)isoindoline-1,3-dione |
| GT-06307 | 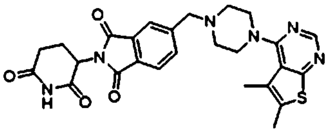 | 5-((4-(5,6-dimethylthieno[2,3-d]pyrimidin-4-yl)piperazin-1-yl)methyl)-2-(2,6-dioxopiperidin-3-yl)isoindoline-1,3-dione | 5-((4-(5,6-dimethylthieno[2,3-d]pyrimidin-4-yl)piperazin-1-yl)methyl)-2-(2,6-dioxopiperidin-3-yl)isoindoline-1,3-dione |
| GT-06310 | 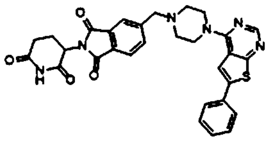 | 2-(2,6-dioxopiperidin-3-yl)-5-((4-(6-phenylthieno[2,3-d]pyrimidin-4-yl)piperazin-1-yl)methyl)isoindoline-1,3-dione | 2-(2,6-dioxopiperidin-3-yl)-5-((4-(6-phenylthieno[2,3-d]pyrimidin-4-yl)piperazin-1-yl)methyl)isoindoline-1,3-dione |
| GT-06309 | 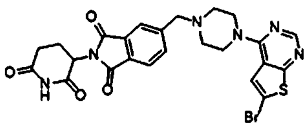 | 5-((4-(6-bromothieno[2,3-d]pyrimidin-4-yl)piperazin-1-yl)methyl)-2-(2,6-dioxopiperidin-3-yl)isoindoline-1,3-dione | 5-((4-(6-bromothieno[2,3-d]pyrimidin-4-yl)piperazin-1-yl)methyl)-2-(2,6-dioxopiperidin-3-yl)isoindoline-1,3-dione |
| GT-06345 | 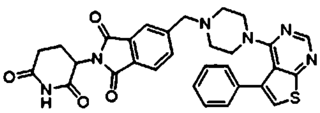 | 2-(2,6-dioxopiperidin-3-yl)-5-((4-(5-phenylthieno[2,3-d]pyrimidin-4-yl)piperazin-1-yl)methyl)isoindoline-1,3-dione | 2-(2,6-dioxopiperidin-3-yl)-5-((4-(5-phenylthieno[2,3-d]pyrimidin-4-yl)piperazin-1-yl)methyl)isoindoline-1,3-dione |
| GT-06564 | 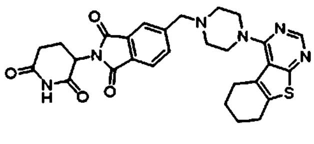 | 2-(2,6-dioxopiperidin-3-yl)-5-((4-(5,6,7,8-tetrahydrobenzo[4,5]thieno [2,3-d]pyrimidin-4-yl)piperazin-1-yl)methyl)isoindoline-1,3-dione | 2-(2,6-dioxopiperidin-3-yl)-5-((4-(5,6,7,8-tetrahydrobenzo[4,5]thieno [2,3-d]pyrimidin-4-yl)piperazin-1-yl)methyl)isoindoline-1,3-dione |
| GT-06565 | 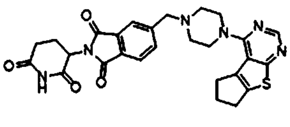 | 5-((4-(6,7-dihydro-5H-cyclopenta[4,5]thieno[2,3-d]pyrimidin-4-yl)piperazin-1-yl)methyl)-2-(2,6-dioxopiperidin-3-yl)isoindoline-1,3-dione | 5-((4-(6,7-dihydro-5H-cyclopenta[4,5]thieno[2,3-d]pyrimidin-4-yl)piperazin-1-yl)methyl)-2-(2,6-dioxopiperidin-3-yl)isoindoline-1,3-dione |
| GT-06346 | 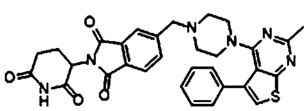 | 2-(2,6-dioxopiperidin-3-yl)-5-((4-(2-methyl-5-phenylthieno[2,3-d]pyrimidin-4-yl)piperazin-1-yl)methyl)isoindoline-1,3-dione | 2-(2,6-dioxopiperidin-3-yl)-5-((4-(2-methyl-5-phenylthieno[2,3-d]pyrimidin-4-yl)piperazin-1-yl)methyl)isoindoline-1,3-dione |
| GT-06348 | 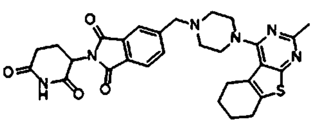 | 2-(2,6-dioxopiperidin-3-yl)-5-((4-(2-methyl-5,6,7,8-tetrahydrobenzo[4,5]thieno [2,3-d]pyrimidin-4-yl)piperazin-1-yl)methyl)isoindoline-1,3-dione | 2-(2,6-dioxopiperidin-3-yl)-5-((4-(2-methyl-5,6,7,8-tetrahydrobenzo[4,5]thieno [2,3-d]pyrimidin-4-yl)piperazin-1-yl)methyl)isoindoline-1,3-dione |

(continued)

| Compo und No. | Structure of the compounds | The compounds' name | Chinese translation of the compounds' name |
|---|---|---|---|
| GT-06566 | 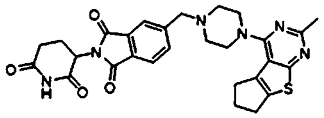 | 2-(2,6-dioxopiperidin-3-yl)-5-((4-(2-methyl-6,7-dihydro-5H-cyclopenta[4,5]thieno [2,3-d]pyrimidin-4-yl)piperazin-1-yl)methyl)isoindoline-1,3-dione | 2-(2,6-dioxopiperidin-3-yl)-5-((4-(2-methyl-6,7-dihydro-5H-cyclopenta[4,5]thieno [2,3-d]pyrimidin-4-yl)piperazin-1-yl)methyl)isoindoline-1,3-dione |
| GT-06347 | 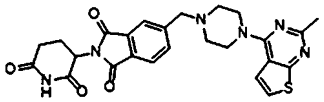 | 2-(2,6-dioxopiperidin-3-yl)-5-((4-(2-methylthieno[2,3-d] pyrimidin-4-yl)piperazin-1-yl) methyl)isoindoline-1,3-dione | 2-(2,6-dioxopiperidin-3-yl)-5-((4-(2-methylthieno[2,3-d] pyrimidin-4-yl)piperazin-1-yl) methyl)isoindoline-1,3-dione |
| GT-06567 | 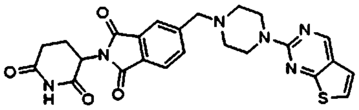 | 2-(2,6-dioxopiperidin-3-yl)-5-((4-(thieno[2,3-d]pyrimidin-2-yl)piperazin-1-yl)methyl) isoindoline-1,3-dione | 2-(2,6-dioxopiperidin-3-yl)-5-((4-(thieno[2,3-d]pyrimidin-2-yl)piperazin-1-yl)methyl) isoindoline-1,3-dione |
| GT-06308 | 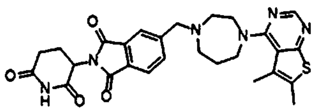 | 5-((4-(5,6-dimethylthieno[2,3-d] pyrimidin-4-yl)-1,4-diazepan-1-yl)methyl)-2-(2,6-dioxopiperidin-3-yl)isoindoline-1,3-dione | 5-((4-(5,6-dimethylthieno[2,3-d] pyrimidin-4-yl)-1,4-diazepan-1-yl)methyl)-2-(2,6-dioxopiperidin-3-yl)isoindoline-1,3-dione |
| GT-06591 | 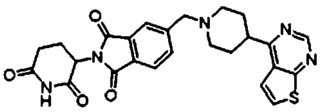 | 2-(2,6-dioxopiperidin-3-yl)-5-((4-(thieno[2,3-d]pyrimidin-4-yl)piperidin-1-yl)methyl) isoindoline-1,3-dione | 2-(2,6-dioxopiperidin-3-yl)-5-((4-(thieno[2,3-d]pyrimidin-4-yl)piperidin-1-yl)methyl) isoindoline-1,3-dione |
| GT-06592 | 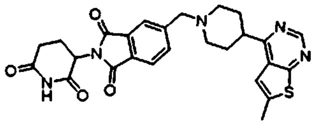 | 2-(2,6-dioxopiperidin-3-yl)-5-((4-(6-methylthieno[2,3-d] pyrimidin-4-yl)piperidin-1-yl) methyl)isoindoline-1,3-dione | 2-(2,6-dioxopiperidin-3-yl)-5-((4-(6-methylthieno[2,3-d] pyrimidin-4-yl)piperidin-1-yl) methyl)isoindoline-1,3-dione |
| GT-06593 | 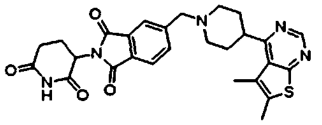 | 5-((4-(5,6-dimethylthieno[2,3-d] pyrimidin-4-yl)piperidin-1-yl) methyl)-2-(2,6-dioxopiperidin-3-yl)isoindoline-1,3-dione | 5-((4-(5,6-dimethylthieno[2,3-d] pyrimidin-4-yl)piperidin-1-yl) methyl)-2-(2,6-dioxopiperidin-3-yl)isoindoline-1,3-dione |
| GT-06594 | 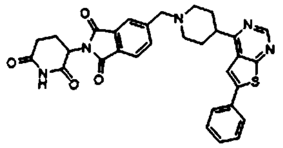 | 2-(2,6-dioxopiperidin-3-yl)-5-((4-(6-phenylthieno[2,3-d] pyrimidin-4-yl)piperidin-1-yl) methyl)isoindoline-1,3-dione | 2-(2,6-dioxopiperidin-3-yl)-5-((4-(6-phenylthieno[2,3-d] pyrimidin-4-yl)piperidin-1-yl) methyl)isoindoline-1,3-dione |
| GT-06595 | 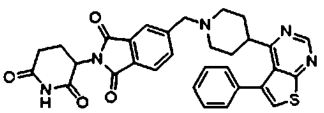 | 2-(2,6-dioxopiperidin-3-yl)-5-((4-(5-phenylthieno[2,3-d] pyrimidin-4-yl)piperidin-1-yl) methyl)isoindoline-1,3-dione | 2-(2,6-dioxopiperidin-3-yl)-5-((4-(5-phenylthieno[2,3-d] pyrimidin-4-yl)piperidin-1-yl) methyl)isoindoline-1,3-dione |
| GT-06649 | 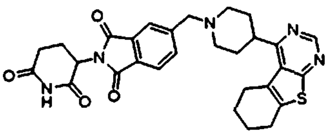 | 2-(2,6-dioxopiperidin-3-yl)-5-((4-(5,6,7,8-tetrahydrobenzo[4,5]thieno [2,3-d]pyrimidin-4-yl)piperidin-1-yl)methyl)isoindoline-1,3-dione | 2-(2,6-dioxopiperidin-3-yl)-5-((4-(5,6,7,8-tetrahydrobenzo[4,5]thieno [2,3-d]pyrimidin-4-yl)piperidin-1-yl)methyl)isoindoline-1,3-dione |
| GT-06650 | 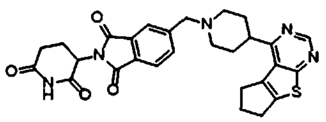 | 5-((4-(6,7-dihydro-5H-cyclopenta[4,5]thieno[2,3-d]pyrimidin-4-yl)piperidin-1-yl)methyl)-2-(2,6-dioxopiperidin-3-yl)isoindoline-1,3-dione | 5-((4-(6,7-dihydro-5H-cyclopenta[4,5]thieno[2,3-d]pyrimidin-4-yl)piperidin-1-yl)methyl)-2-(2,6-dioxopiperidin-3-yl)isoindoline-1,3-dione |

(continued)

| Compo und No. | Structure of the compounds | The compounds' name | Chinese translation of the compounds' name |
|---|---|---|---|
| GT-06646 | 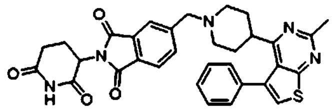 | 2-(2,6-dioxopiperidin-3-yl)-5-((4-(2-methyl-5-phenylthieno[2,3-d]pyrimidin-4-yl)piperidin-1-yl)methyl)isoindoline-1,3-dione | 2-(2,6-dioxopiperidin-3-yl)-5-((4-(2-methyl-5-phenylthieno[2,3-d]pyrimidin-4-yl)piperidin-1-yl)methyl)isoindoline-1,3-dione |
| GT-06648 | 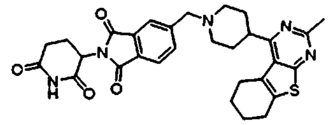 | 2-(2,6-dioxopiperidin-3-yl)-5-((4-(2-methyl-5,6,7,8-tetrahydrobenzo[4,5]thieno [2,3-d]pyrimidin-4-yl)piperidin-1-yl)methyl)isoindoline-1,3-dione | 2-(2,6-dioxopiperidin-3-yl)-5-((4-(2-methyl-5,6,7,8-tetrahydrobenzo[4,5]thieno [2,3-d]pyrimidin-4-yl)piperidin-1-yl)methyl)isoindoline-1,3-dione |
| GT-06651 | 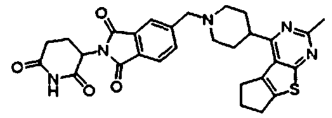 | 2-(2,6-dioxopiperidin-3-yl)-5-((4-(2-methyl-6,7-dihydro-5H-cyclopenta[4,5]thieno [2,3-d]pyrimidin-4-yl)piperidin-1-yl)methyl)isoindoline-1,3-dione | 2-(2,6-dioxopiperidin-3-yl)-5-((4-(2-methyl-6,7-dihydro-5H-cyclopenta[4,5]thieno [2,3-d]pyrimidin-4-yl)piperidin-1-yl)methyl)isoindoline-1,3-dione |
| GT-06647 | 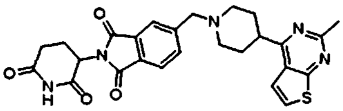 | 2-(2,6-dioxopiperidin-3-yl)-5-((4-(2-methylthieno[2,3-d]pyrimidin-4-yl)piperidin-1-yl)methyl)isoindoline-1,3-dione | 2-(2,6-dioxopiperidin-3-yl)-5-((4-(2-methylthieno[2,3-d]pyrimidin-4-yl)piperidin-1-yl)methyl)isoindoline-1,3-dione |
| GT-06652 | 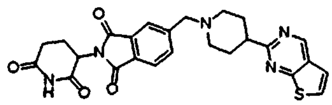 | 2-(2,6-dioxopiperidin-3-yl)-5-((4-(thieno[2,3-d]pyrimidin-2-yl)piperidin-1-yl)methyl)isoindoline-1,3-dione | 2-(2,6-dioxopiperidin-3-yl)-5-((4-(thieno[2,3-d]pyrimidin-2-yl)piperidin-1-yl)methyl)isoindoline-1,3-dione |
| | 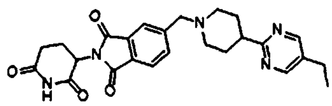 | 2-(2,6-dioxopiperidin-3-yl)-5-((4-(5-ethylpyrimidin-2-yl)piperidin-1-yl)methyl)isoindoline-1,3-dione | 2-(2,6-dioxopiperidin-3-yl)-5-((4-(5-ethylpyrimidin-2-yl)piperidin-1-yl)methyl)isoindoline-1,3-dione |
| GT-06653 | 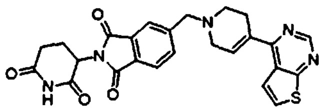 | 2-(2,6-dioxopiperidin-3-yl)-5-((4-(thieno[2,3-d]pyrimidin-4-yl)-3,6-dihydropyridin-1 (2H)-yl)methyl)isoindoline-1,3-dione | 2-(2,6-dioxopiperidin-3-yl)-5-((4-(thieno[2,3-d]pyrimidin-4-yl)-3,6-dihydropyridin-1(2H)-yl)methyl)isoindoline-1,3-dione |
| GT-06654 | 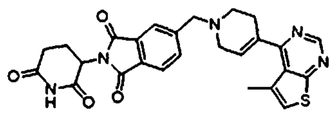 | 2-(2,6-dioxopiperidin-3-yl)-5-((4-(5-methylthieno[2,3-d]pyrimidin-4-yl)-3,6-dihydropyridin-1(2H)-yl)methyl)isoindoline-1,3-dione | 2-(2,6-dioxopiperidin-3-yl)-5-((4-(5-methylthieno[2,3-d]pyrimidin-4-yl)-3,6-dihydropyridin-1(2H)-yl)methyl)isoindoline-1,3-dione |
| GT-06655 | 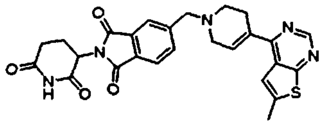 | 2-(2,6-dioxopiperidin-3-yl)-5-((4-(6-methylthieno[2,3-d]pyrimidin-4-yl)-3,6-dihydropyridin-1(2H)-yl)methyl)isoindoline-1,3-dione | 2-(2,6-dioxopiperidin-3-yl)-5-((4-(6-methylthieno[2,3-d]pyrimidin-4-yl)-3,6-dihydropyridin-1(2H)-yl)methyl)isoindoline-1,3-dione |
| GT-06656 | 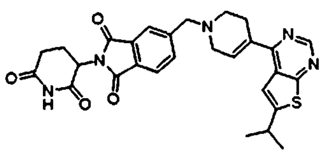 | 2-(2,6-dioxopiperidin-3-yl)-5-((4-(6-isopropylthieno[2,3-d]pyrimidin-4-yl)-3,6-dihydropyridin-1(2H)-yl)methyl)isoindoline-1,3-dione | 2-(2,6-dioxopiperidin-3-yl)-5-((4-(6-isopropylthieno[2,3-d]pyrimidin-4-yl)-3,6-dihydropyridin-1(2H)-yl)methyl)isoindoline-1,3-dione |

(continued)

| Compo und No. | Structure of the compounds | The compounds' name | Chinese translation of the compounds' name |
|---|---|---|---|
| GT-06657 | 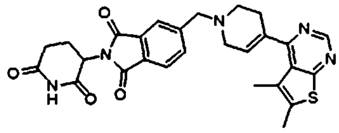 | 5-((4-(5,6-dimethylthieno[2,3-d] pyrimidin-4-yl)-3,6-dihydropyridin-1(2H)-yl)methyl)-2-(2,6-dioxopiperidin-3-yl)isoindoline-1,3-dione | 5-((4-(5,6-dimethylthieno[2,3-d] pyrimidin-4-yl)-3,6-dihydropyridin-1(2H)-yl)methyl)-2-(2,6-dioxopiperidin-3-yl)isoindoline-1,3-dione |
| GT-06675 | 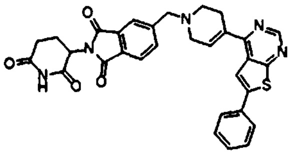 | 2-(2,6-dioxopiperidin-3-yl)-5-((4-(6-phenylthieno[2,3-d] pyrimidin-4-yl)-3,6-dihydropyridin-1(2H)-yl)methyl)isoindoline-1,3-dione | 2-(2,6-dioxopiperidin-3-yl)-5-((4-(6-phenylthieno[2,3-d] pyrimidin-4-yl)-3,6-dihydropyridin-1(2H)-yl)methyl)isoindoline-1,3-dione |
| GT-06676 | 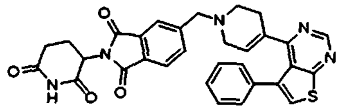 | 2-(2,6-dioxopiperidin-3-yl)-5-((4-(5-phenylthieno[2,3-d] pyrimidin-4-yl)-3,6-dihydropyridin-1(2H)-yl)methyl)isoindoline-1,3-dione | 2-(2,6-dioxopiperidin-3-yl)-5-((4-(5-phenylthieno[2,3-d] pyrimidin-4-yl)-3,6-dihydropyridin-1(2H)-yl)methyl)isoindoline-1,3-dione |
| GT-06680 | 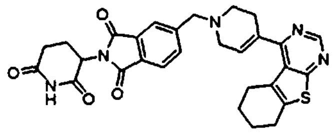 | 2-(2,6-dioxopiperidin-3-yl)-5-((4-(5,6,7,8-tetrahydrobenzo[4,5]thieno [2,3-d]pyrimidin-4-yl)-3,6-dihydropyridin-1(2H)-yl) methyl)isoindoline-1,3-dione | 2-(2,6-dioxopiperidin-3-yl)-5-((4-(5,6,7,8-tetrahydrobenzo[4,5]thieno [2,3-d]pyrimidin-4-yl)-3,6-dihydropyridin-1(2H)-yl) methyl)isoindoline-1,3-dione |
| GT-06681 | 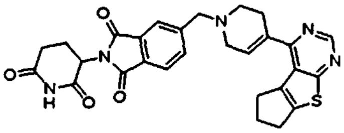 | 5-((4-(6,7-dihydro-5H-cyclopenta[4,5]thieno[2,3-d]pyrimidin-4-yl)-3,6-dihydropyridin-1(2H)-yl) methyl)-2-(2,6-dioxopiperidin-3-yl)isoindoline-1,3-dione | 5-((4-(6,7-dihydro-5H-cyclopenta[4,5]thieno[2,3-d]pyrimidin-4-yl)-3,6-dihydropyridin-1(2H)-yl) methyl)-2-(2,6-dioxopiperidin-3-yl)isoindoline-1,3-dione |
| GT-06677 | 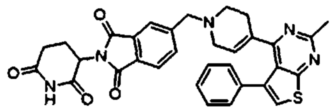 | 2-(2,6-dioxopiperidin-3-yl)-5-((4-(2-methyl-5-phenylthieno[2,3-d]pyrimidin-4-yl)-3,6-dihydropyridin-1(2H)-yl) methyl)isoindoline-1,3-dione | 2-(2,6-dioxopiperidin-3-yl)-5-((4-(2-methyl-5-phenylthieno[2,3-d]pyrimidin-4-yl)-3,6-dihydropyridin-1(2H)-yl)methyl) isoindoline-1,3-dione |
| GT-06679 | 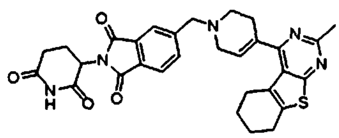 | 2-(2,6-dioxopiperidin-3-yl)-5-((4-(2-methyl-5,6,7,8-tetrahydrobenzo[4,5]thieno [2,3-d] pyrimidin-4-yl)-3,6-dihydropyridin-1(2H)-yl)methyl)isoindoline-1,3-dione | 2-(2,6-dioxopiperidin-3-yl)-5-((4-(2-methyl-5,6,7,8-tetrahydrobenzo[4,5]thieno [2,3-d] pyrimidin-4-yl)-3,6-dihydropyridin-1(2H)-yl)methyl)isoindoline-1,3-dione |
| GT-06682 | 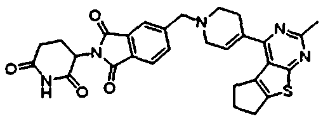 | 2-(2,6-dioxopiperidin-3-yl)-5-((4-(2-methyl-6,7-dihydro-5H-cyclopenta[4,5]thieno [2,3-d]pyrimidin-4-yl)-3,6-dihydropyridin-1(2H)-yl)methyl)isoindoline-1,3-dione | 2-(2,6-dioxopiperidin-3-yl)-5-((4-(2-methyl-6,7-dihydro-5H-cyclopenta[4,5]thieno [2,3-d]pyrimidin-4-yl)-3,6-dihydropyridin-1(2H)-yl)methyl)isoindoline-1,3-dione |
| GT-06678 | 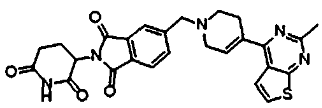 | 2-(2,6-dioxopiperidin-3-yl)-5-((4-(2-methylthieno[2,3-d] pyrimidin-4-yl)-3,6-dihydropyridin-1(2H)-yl)methyl)isoindoline-1,3-dione | 2-(2,6-dioxopiperidin-3-yl)-5-((4-(2-methylthieno[2,3-d] pyrimidin-4-yl)-3,6-dihydropyridin-1(2H)-yl)methyl)isoindoline-1,3-dione |

(continued)

| Compound No. | Structure of the compounds | The compounds' name | Chinese translation of the compounds' name |
|---|---|---|---|
| GT-06683 | 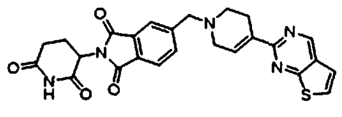 | 2-(2,6-dioxopiperidin-3-yl)-5-((4-(thieno[2,3-d]pyrimidin-2-yl)-3,6-dihydropyridin-1(2H)-yl)methyl)isoindoline-1,3-dione | 2-(2,6-dioxopiperidin-3-yl)-5-((4-(thieno[2,3-d]pyrimidin-2-yl)-3,6-dihydropyridin-1(2H)-yl)methyl)isoindoline-1,3-dione |
| GT-06710 | 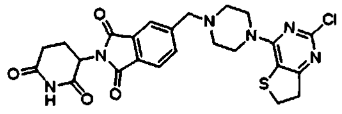 | 5-((4-(2-chloro-6,7-dihydrothieno[3,2-d]pyrimidin-4-yl)piperazin-1-yl)methyl)-2-(2,6-dioxopiperidin-3-yl)isoindoline-1,3-dione | 5-((4-(2-chloro-6,7-dihydrothieno[3,2-d]pyrimidin-4-yl)piperazin-1-yl)methyl)-2-(2,6-dioxopiperidin-3-yl)isoindoline-1,3-dione |
| GT-06712 | 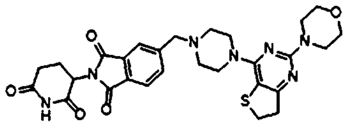 | 2-(2,6-dioxopiperidin-3-yl)-5-((4-(2-morpholino-6,7-dihydrothieno[3,2-d]pyrimidin-4-yl)piperazin-1-yl)methyl)isoindoline-1,3-dione | 2-(2,6-dioxopiperidin-3-yl)-5-((4-(2-morpholino-6,7-dihydrothieno[3,2-d]pyrimidin-4-yl)piperazin-1-yl)methyl)isoindoline-1,3-dione |
| GT-06799 | 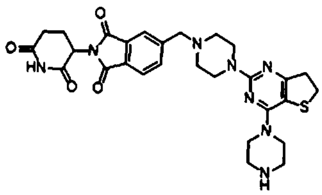 | 2-(2,6-dioxopiperidin-3-yl)-5-((4-(4-(piperazin-1-yl)-6,7-dihydrothieno[3,2-d]pyrimidin-2-yl)piperazin-1-yl)methyl)isoindoline-1,3-dione | 2-(2,6-dioxopiperidin-3-yl)-5-((4-(4-(piperazin-1-yl)-6,7-dihydrothieno[3,2-d]pyrimidin-2-yl)piperazin-1-yl)methyl)isoindoline-1,3-dione |
| GT-06713 | 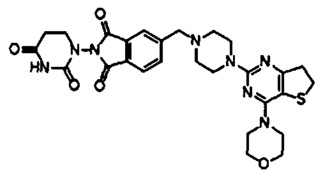 | 2-(2,6-dioxopiperidin-3-yl)-5-((4-(4-morpholino-6,7-dihydrothieno[3,2-d]pyrimidin-2-yl)piperazin-1-yl)methyl)isoindoline-1,3-dione | 2-(2,6-dioxopiperidin-3-yl)-5-((4-(4-morpholino-6,7-dihydrothieno[3,2-d]pyrimidin-2-yl)piperazin-1-yl)methyl)isoindoline-1,3-dione |
| GT-08003 | 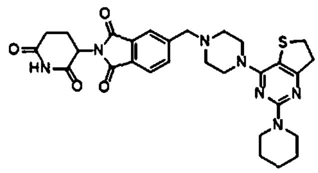 | 2-(2,6-dioxopiperidin-3-yl)-5-((4-(2-(piperidin-1-yl)-6,7-dihydrothieno[3,2-d]pyrimidin-4-yl)piperazin-1-yl)methyl)isoindoline-1,3-dione | 2-(2,6-dioxopiperidin-3-yl)-5-((4-(2-(piperidin-1-yl)-6,7-dihydrothieno[3,2-d]pyrimidin-4-yl)piperazin-1-yl)methyl)isoindoline-1,3-dione |
| GT-08004 | 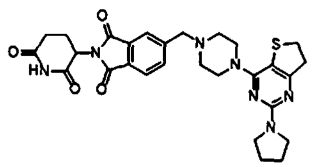 | 2-(2,6-dioxopiperidin-3-yl)-5-((4-(2-(pyrrolidin-1-yl)-6,7-dihydrothieno[3,2-d]pyrimidin-4-yl)piperazin-1-yl)methyl)isoindoline-1,3-dione | 2-(2,6-dioxopiperidin-3-yl)-5-((4-(2-(pyrrolidin-1-yl)-6,7-dihydrothieno[3,2-d]pyrimidin-4-yl)piperazin-1-yl)methyl)isoindoline-1,3-dione |
| GT-08005 | 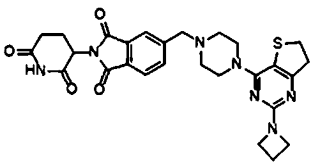 | 5-((4-(2-(azetidin-1-yl)-6,7-dihydrothieno[3,2-d]pyrimidin-4-yl) piperazin-1-yl)methyl)-2-(2,6-dioxopiperidin-3-yl)isoindoline-1,3-dione | 5-((4-(2-(azetidin-1-yl)-6,7-dihydrothieno[3,2-d]pyrimidin-4-yl) piperazin-1-yl)methyl)-2-(2,6-dioxopiperidin-3-yl)isoindoline-1,3-dione |
| GT-06714 | 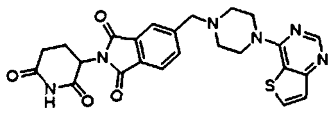 | 2-(2,6-dioxopiperidin-3-yl)-5-((4-(thieno[3,2-d]pyrimidin-4-yl)piperazin-1-yl)methyl) isoindoline-1,3-dione | 2-(2,6-dioxopiperidin-3-yl)-5-((4-(thieno[3,2-d]pyrimidin-4-yl)piperazin-1-yl)methyl) isoindoline-1,3-dione |

(continued)

| Compo und No. | Structure of the compounds | The compounds' name | Chinese translation of the compounds' name |
|---|---|---|---|
| GT-06715 | 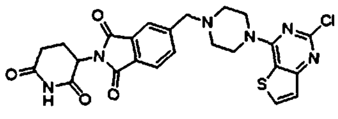 | 5-((4-(2-chlorothieno[3,2-d]pyrimidin-4-yl)piperazin-1-yl) methyl)-2-(2,6-dioxopiperidin-3-yl)isoindoline-1,3-dione | 5-((4-(2-chlorothieno[3,2-d]pyrimidin-4-yl)piperazin-1-yl) methyl)-2-(2,6-dioxopiperidin-3-yl)isoindoline-1,3-dione |
| GT-06717 | 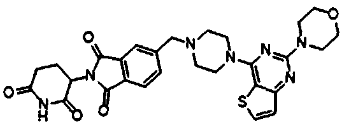 | 2-(2,6-dioxopiperidin-3-yl)-5-((4-(2-morpholinothieno [3,2-d]pyrimidin-4-yl)piperazin-1-yl)methyl)isoindoline-1,3-dione | 2-(2,6-dioxopiperidin-3-yl)-5-((4-(2-morpholinothieno [3,2-d]pyrimidin-4-yl)piperazin-1-yl)methyl)isoindoline-1,3-dione |
| GT-06800 | 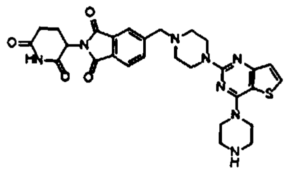 | 2-(2,6-dioxopiperidin-3-yl)-5-((4-(4-(piperazin-1-yl)thieno[3,2-d]pyrimidin-2-yl)piperazin-1-yl)methyl)isoindoline-1,3-dione | 2-(2,6-dioxopiperidin-3-yl)-5-((4-(4-(piperazin-1-yl)thieno[3,2-d]pyrimidin-2-yl)piperazin-1-yl)methyl)isoindoline-1,3-dione |
| GT-06718 | 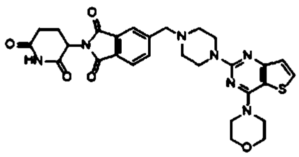 | 2-(2,6-dioxopiperidin-3-yl)-5-((4-(4-morpholinothieno [3,2-d]pyrimidin-2-yl)piperazin-1-yl)methyl)isoindoline-1,3-dione | 2-(2,6-dioxopiperidin-3-yl)-5-((4-(4-morpholinothieno [3,2-d]pyrimidin-2-yl)piperazin-1-yl)methyl)isoindoline-1,3-dione |
| GT-04915 | 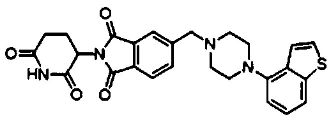 | 5-((4-(benzo[b]thiophen-4-yl)piperazin-1-yl)methyl)-2-(2,6-dioxopiperidin-3-yl)isoindoline-1,3-dione | 5-((4-(benzo[b]thiophen-4-yl)piperazin-1-yl)methyl)-2-(2,6-dioxopiperidin-3-yl)isoindoline-1,3-dione |
| GT-04921 | 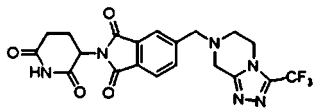 | 2-(2,6-dioxopiperidin-3-yl)-5-((3-(trifluoromethyl)-5,6-dihydro-[1,2,4]triazolo[4,3-a]pyrazin-7(8H)-yl)methyl)isoindoline-1,3-dione | 2-(2,6-dioxopiperidin-3-yl)-5-((3-(trifluoromethyl)-5,6-dihydro-[1,2,4]triazolo[4,3-a]pyrazin-7(8H)-yl)methyl)isoindoline-1,3-dione |
| GT-06761 | 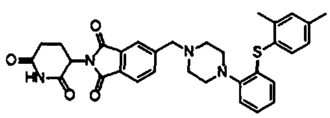 | 5-((4-(2-((2,4-dimethylphenyl) thio)pheny 1)piperazin-1-yl) methyl)-2-(2,6-dioxopiperidin-3-vl)isoindoline-1,3-dione | 5-((4-(2-((2,4-dimethylphenyl) thio)pheny l)piperazin-1-yl) methyl)-2-(2,6-dioxopiperidin-3-yl)isoindoline-1,3-dione |
| GT-06762 | 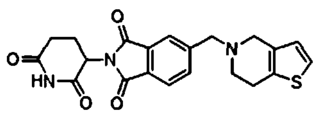 | 5-((6,7-dihydrothieno[3,2-c]pyridin-5(4H)-yl)methyl)-2-(2,6-dioxopiperidin-3-yl)isoindoline-1,3-dione | 5-((6,7-dihydrothieno[3,2-c]pyridin-5(4H)-yl)methyl)-2-(2,6-dioxopiperidin-3-yl)isoindoline-1,3-dione |
| GT-06763 | 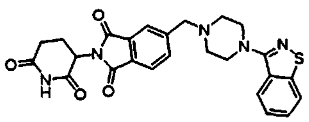 | 5-((4-(benzo[d]isothiazol-3-yl) piperazin-1-yl)methyl)-2-(2,6-dioxopiperidin-3-yl)isoindoline-1,3-dione | 5-((4-(benzo[d]isothiazol-3-yl)piperazin-1-yl)methyl)-2-(2,6-dioxopiperidin-3-yl)isoindoline-1,3-dione |
| GT-06810 | 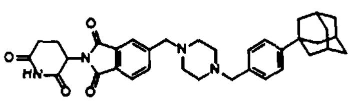 | 5-((4-(4-((3r,5r,7r)-adamantan-1-yl)benzyl)piperazin-1-yl) methyl)-2-(2,6-dioxopiperidin-3-yl)isoindoline-1,3-dione | 5-((4-(4-((3r,5r,7r)-adamantan-1-yl)benzyl)piperazin-1-yl) methyl)-2-(2,6-dioxopiperidin-3-yl)isoindoline-1,3-dione |
| GT-06912 | 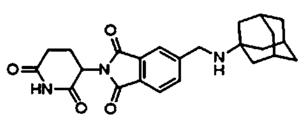 | 5-((((3s,5s,7s)-adamantan-1-yl) amino)methyl)-2-(2,6-dioxopiperidin-3-yl)isoindoline-1,3-dione | 5-((((3s,5s,7s)-adamantan-1-yl) amino)methyl)-2-(2,6-dioxopiperidin-3-yl)isoindoline-1,3-dione |

(continued)

| Compo und No. | Structure of the compounds | The compounds' name | Chinese translation of the compounds' name |
|---|---|---|---|
| GT-07997 | 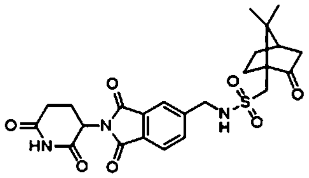 | 1-((1R,4R)-7,7-dimethyl-2-oxobicyclo[2.2.1]heptan-1-yl)-N-((2-(2,6-dioxopiperidin-3-yl)-1,3-dioxoisoindolin-5-yl) methyl)methanesulfona mide | 1-((1R,4R)-7,7-dimethyl-2-oxobicyclo[2.2.1]heptan-1-yl)-N-((2-(2,6-dioxopiperidin-3-yl)-1,3-dioxoisoindolin-5-yl) methyl)methanesulfona mide |
| | 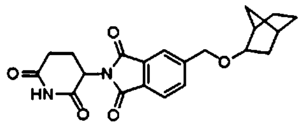 | 5-((((1R,4S)-bicyclo[2.2.1]heptan-2-yl)oxy)methyl)-2-(2,6-dioxopiperidin-3-yl)isoindoline-1,3-dione | 5-((((1R,4S)-bicyclo[2.2.1]heptan-2-yl)oxy)methyl)-2-(2,6-dioxopiperidin-3-yl)isoindoline-1,3-dione |
| GT-04728 | 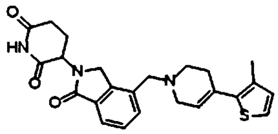 | 3-(4-((4-(3-methylthiophen-2-yl)-3,6-dihydropyridin-1(2H)-yl) methyl)-1-oxoisoindolin-2-yl)piperidine-2,6-dione | 3-(4-((4-(3-methylthiophen-2-yl)-3,6-dihydropyridin-1(2H)-yl) methyl)-1-oxoisoindolin-2-yl)piperidine-2,6-dione |
| GT-04729 | 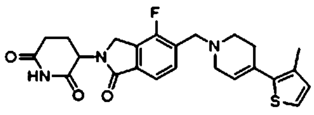 | 3-(4-fluoro-5-((4-(3-methylthiophen-2-yl)-3,6-dihydropyridin-1(2H)-yl)methyl)-1-oxoisoindolin-2-yl)piperidine-2,6-dione | 3-(4-fluoro-5-((4-(3-methylthiophen-2-yl)-3,6-dihydropyridin-1(2H)-yl)methyl)-1-oxoisoindolin-2-yl)piperidine-2,6-dione |
| GT-04730 | 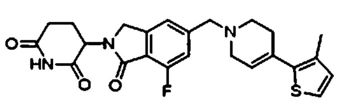 | 3-(7-fluoro-5-((4-(3-methylthiophen-2-yl)-3,6-dihydropyridin-1(2H)-yl)methyl)-1-oxoisoindolin-2-yl)piperidine-2,6-dione | 3-(7-fluoro-5-((4-(3-methylthiophen-2-yl)-3,6-dihydropyridin-1(2H)-yl)methyl)-1-oxoisoindolin-2-yl)piperidine-2,6-dione |
| GT-04731 | 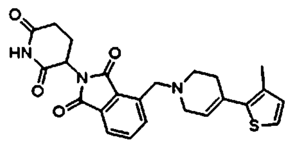 | 2-(2,6-dioxopiperidin-3-yl)-4-((4-(3-methylthiophen-2-yl)-3,6-dihydropyridin-1(2H)-yl) methyl)isoindoline-1,3-dione | 2-(2,6-dioxopiperidin-3-yl)-4-((4-(3-methylthiophen-2-yl)-3,6-dihydropyridin-1(2H)-yl) methyl)isoindoline-1,3-dione |
| GT-04528 | 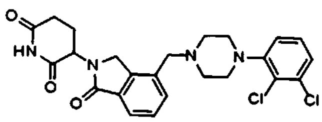 | 3-(4-((4-(2,3-dichlorophenyl)piperazin-1-yl)methyl)-1-oxoisoindolin-2-yl)piperidine-2,6-dione | 3-(4-((4-(2,3-dichlorophenyl)piperazin-1-yl)methyl)-1-oxoisoindolin-2-yl)piperidine-2,6-dione |
| GT-04526 | 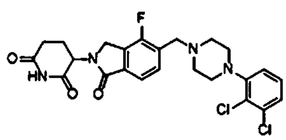 | 3-(5-((4-(2,3-dichlorophenyl)piperazin-1-yl)methyl)-4-fluoro-1-oxoisoindolin-2-yl)piperidine-2,6-dione | 3-(5-((4-(2,3-dichlorophenyl)piperazin-1-yl)methyl)-4-fluoro-1-oxoisoindolin-2-yl)piperidine-2,6-dione |
| GT-04527 | 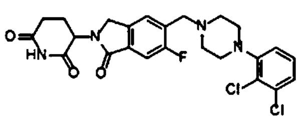 | 3-(5-((4-(2,3-dichlorophenyl)piperazin-1-yl)methyl)-6-fluoro-1-oxoisoindolin-2-yl)piperidine-2,6-dione | 3-(5-((4-(2,3-dichlorophenyl)piperazin-1-yl)methyl)-6-fluoro-1-oxoisoindolin-2-yl)piperidine-2,6-dione |
| GT-04445 | 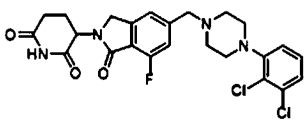 | 3-(5-((4-(2,3-dichlorophenyl)piperazin-1-yl)methyl)-7-fluoro-1-oxoisoindolin-2-yl)piperidine-2,6-dione | 3-(5-((4-(2,3-dichlorophenyl)piperazin-1-yl)methyl)-7-fluoro-1-oxoisoindolin-2-yl)piperidine-2,6-dione |
| GT-04764 | 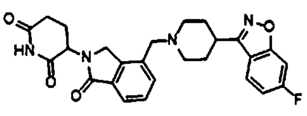 | 3-(4-((4-(6-fluorobenzo[d]isoxazol-3-yl)piperidin-1-yl) methyl)-1-oxoisoindolin-2-yl)piperidine-2,6-dione | 3-(4-((4-(6-fluorobenzo[d]isoxazol-3-yl)piperidin-1-yl)methyl)-1-oxoisoindolin-2-yl)piperidine-2,6-dione |

(continued)

| Compound No. | Structure of the compounds | The compounds' name | Chinese translation of the compounds' name |
|---|---|---|---|
| GT-04768 | 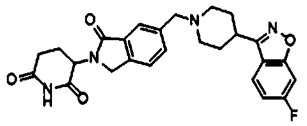 | 3-(6-((4-(6-fluorobenzo[d]isoxazol-3-yl)piperidin-1-yl) methyl)-1-oxoisoindolin-2-yl)piperidine-2,6-dione | 3-(6-((4-(6-fluorobenzo[d]isoxazol-3-yl)piperidin-1-yl)methyl)-1-oxoisoindolin-2-yl)piperidine-2,6-dione |
| GT-04765 | 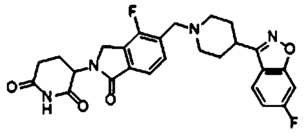 | 3-(4-fluoro-5-((4-(6-fluorobenzo [d]isoxazol-3-yl)piperidin-1-yl) methyl)-1-oxoisoindolin-2-yl)piperidine-2,6-dione | 3-(4-fluoro-5-((4-(6-fluorobenzo [d]isoxazol-3-yl)piperidin-1-yl) methyl)-1-oxoisoindolin-2-yl)piperidine-2,6-dione |
| GT-04766 | 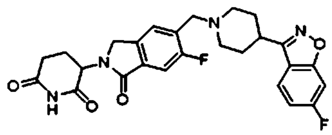 | 3-(6-fluoro-5-((4-(6-fluorobenzo [d]isoxazol-3-yl)piperidin-1-yl) methyl)-1-oxoisoindolin-2-yl)piperidine-2,6-dione | 3-(6-fluoro-5-((4-(6-fluorobenzo [d]isoxazol-3-yl)piperidin-1-yl) methyl)-1-oxoisoindolin-2-yl)piperidine-2,6-dione |
| GT-04767 | 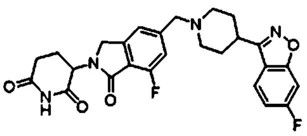 | 3-(7-fluoro-5-((4-(6-fluorobenzo [d]isoxazol-3-yl)piperidin-1-yl) methyl)-1-oxoisoindolin-2-yl)piperidine-2,6-dione | 3-(7-fluoro-5-((4-(6-fluorobenzo [d]isoxazol-3-yl)piperidin-1-yl) methyl)-1-oxoisoindolin-2-yl)piperidine-2,6-dione |
| GT-04770 | 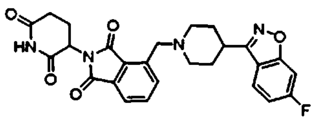 | 2-(2,6-dioxopiperidin-3-yl)-4-((4-(6-fluorobenzo[d]isoxazol-3-yl)piperidin-1-yl)methyl) isoindoline-1,3-dione | 2-(2,6-dioxopiperidin-3-yl)-4-((4-(6-fluorobenzo[d]isoxazol-3-yl)piperidin-1-yl)methyl) isoindoline-1,3-dione |
| GT-04914 | 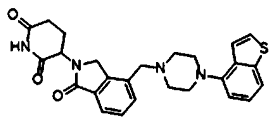 | 3-(4-((4-(benzo[b]thiophen-4-yl) piperazin-1-yl)methyl)-1-oxoisoindolin-2-yl)piperidine-2,6-dione | 3-(4-((4-(benzo[b]thiophen-4-yl) piperazin-1-yl)methyl)-1-oxoisoindolin-2-yl)piperidine-2,6-dione |
| GT-04920 | 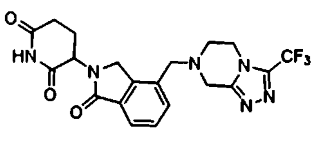 | 3-(1-oxo-4-((3-(trifluoromethyl)-5,6-dihydro-[1,2,4]triazolo[4,3-a]pyrazin-7(8H)-yl) methyl)isoindolin-2-yl)piperidine-2,6-dione | 3-(1-oxo-4-((3-(trifluoromethyl)-5,6-dihydro-[1,2,4]triazolo[4,3-a]pyrazin-7(8H)-yl) methyl)isoindolin-2-yl)piperidine-2,6-dione |
| GT-04544 | 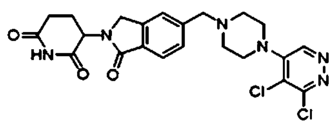 | 3-(5-((4-(5,6-dichloropyridazin-4-yl)piperazin-1-yl) methyl)-1-oxoisoindolin-2-yl)piperidine-2,6-dione | 3-(5-((4-(5,6-dichloropyridazin-4-yl)piperazin-1-yl) methyl)-1-oxoisoindolin-2-yl)piperidine-2,6-dione |
| GT-06267 | 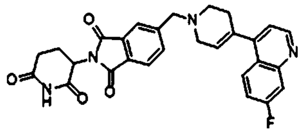 | 2-(2,6-dioxopiperidin-3-yl)-5-((4-(7-fluoroquinolin-4-yl)-3,6-dihydropyridin-1(2H)-yl) methyl)isoindoline-1,3-dione | 2-(2,6-dioxopiperidin-3-yl)-5-((4-(7-fluoroquinolin-4-yl)-3,6-dihydropyridin-1(2H)-yl) methyl)isoindoline-1,3-dione |
| GT-06624 | 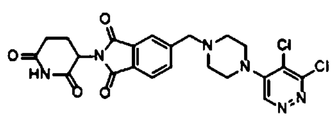 | 5-((4-(5,6-dichloropyridazin-4-yl)piperazin-1-yl)methyl)-2-(2,6-dioxopiperidin-3-yl)isoindoline-1,3-dione | 5-((4-(5,6-dichloropyridazin-4-yl)piperazin-1-yl)methyl)-2-(2,6-dioxopiperidin-3-vl)isoindoline-1,3-dione |

In some embodiments, polymorph forms or salts of the compounds of the present disclosure are also provided. Salts of the compounds of the present disclosure can be pharmaceutically acceptable salts including, but not limited to, hydrochlorides, hydrobromides, sulfates, citrates, maleates, sulfonates, methanesulfonates, ethanesulfonates, edisylates, formates, acetates, 2,2-dichloroacetates, trimethylacetates, propionates, valerates, citrates, lactates, lactobionates, L-tartrates, fumarates, L-malates, L-lactates, $\alpha$-ketoglutarates, hippurates, D-glucuronates, D-gluconates, $\alpha$-D-

glucoheptonates, glycolates, mucates, L-ascorbates, orotates, picrates, glycinates, alaninates, arginates, cinnamates, laurates, pamoates, sebacates, besylates, palmitates, triphenylacetates, 2-ethyl-butanedioates, iodates, nicotinates, L-pyroglutamates, L-prolinates, ferulates, 2-hydroxyethanesulfonates, undecenoates, camphorates, camphorsulfonates, dodecylsulfonates, phosphates, thiocyanates, dihydrophosphates, pyrophosphates, metaphosphates, oxalates, malonates, carbonates, malonates, benzoates, mandelates, succinates, trifluoroacetates, pyruvates, p-chlorobenzenesulfonates, 1,5-naphthalenedisulfonates, 3-hydroxy-2-naphthoates, 1-hydroxy-2-naphthoates, 2-naphthalenesulfonates, glycolates, or p-toluenesulfonates, etc. The compounds of the present disclosure can exist as non-solvated or solvated forms in pharmaceutically acceptable solvents such as water, ethanol, and the like.

In some embodiments, the compounds of the present disclosure can be prepared as prodrugs or precursor drugs. Prodrugs can be converted into parent drugs in the body to play their role. In some embodiments, isotopically-labeled compounds of the present disclosure are also provided, examples of which include deuterium (D or $^2H$).

In some embodiments, the present disclosure provides a pharmaceutical composition comprising, as an active ingredient, the compound of Formula (I) of the present disclosure or a salt, enantiomer, stereoisomer, solvate, isotopically enriched analog, prodrug, or polymorph thereof, and at least one pharmaceutically acceptable carrier.

In some embodiments, pharmaceutically acceptable carriers include, but are not limited to, fillers, stabilizers, dispersants, suspending agents, diluents, excipients, thickeners, colorants, solvents, or encapsulating materials. Each carrier must be "acceptable" in the sense of being compatible with the other ingredients of the formulation (including the compounds useful in the present disclosure) and not injurious to the patient. Some examples of materials that can be used as pharmaceutically acceptable carriers include: sugars such as lactose, glucose, and sucrose; starches such as corn starch and potato starch; cellulose and its derivatives such as sodium carboxymethyl cellulose, ethyl cellulose and cellulose acetate; powdered tragacanth; malt; gelatin; talc; excipients such as cocoa butter and suppository wax; oils such as peanut oil, cottonseed oil, safflower oil, sesame oil, olive oil, corn oil and soybean oil; glycols such as propylene glycol; polyols such as glycerol, sorbitol, mannitol, and polyethylene glycol; esters such as ethyl oleate and ethyl laurate; agar; buffers such as magnesium hydroxide and aluminum hydroxide; surfactant phosphate buffer solution; polyethylene oxide, polyvinylpyrrolidone, polyacrylamide, poloxamer; and other common nontoxic compatible substances used in pharmaceutical formulations.

The pharmaceutical composition of the present disclosure can further comprise at least one second therapeutic agent, e.g., a therapeutic agent for treating tumors or cancer. The second therapeutic agent may be used in combination with the compounds of Formula (I) of the present disclosure to treat the tumors or cancers. The second therapeutic agent includes, but is not limited to, chemotherapeutic agents, immunotherapeutic agents, gene therapy agents, and the like.

In some embodiments, the tumors or cancers includes, but is not limited to, myeloma, including multiple myeloma, plasma cell myeloma, smoldering myeloma, smoldering multiple myeloma; myelofibrosis; bone marrow disease; myelodysplastic syndrome (MDS); previously treated myelodysplastic syndrome; transplantation-related cancer; neutropenia; leukemia, including acute myeloid leukemia (AML), chronic myeloid leukemia (CML), chronic myelogenous leukemia, acute lymphoblastic leukemia (ALL), chronic lymphocytic leukemia (CLL), B-cell chronic lymphocytic leukemia, leukemia-associated anemia, monocytic leukemia, myelomonocytic leukemia, T-lymphocytic leukemia, acute T-lymphocytic leukemia; lymphoma, including diffuse large B-cell lymphoma, non-Hodgkin’s lymphoma, Hodgkin’s lymphoma, anaplastic lymphoma, anaplastic large cell lymphoma, immunoblastic T-Cell lymphoma, CD20 positive lymphoma, mantle cell lymphoma, follicular lymphoma (FL), Burkitt’s lymphoma, marginal zone lymphoma (MZL), primary lymphoma, B-cell lymphoma, recurrent B-cell non-Hodgkin’s lymphoma, recurrent diffuse large B-cell lymphoma, recurrent mediastinal (thymic) large B-cell lymphoma, primary mediastinal (thymic) large B-cell lymphoma, recurrent transformed non-Hodgkin’s lymphoma, refractory B-cell non-Hodgkin’s lymphoma, refractory diffuse large B-cell lymphoma, refractory primary mediastinal (thymic) large B-cell lymphoma, refractory transformed non-Hodgkin’s lymphoma; thyroid cancer; melanoma; lung cancer, including lung adenocarcinoma, lung squamous cell carcinoma, non-small cell lung cancer, and small cell lung cancer; inflammatory myofibroblastoma; colorectal cancer; intestinal cancer; brain glioma; astroblastoma; ovarian cancer; bronchial cancer; prostate cancer; breast cancer, including triple negative breast cancer, sporadic breast cancer, ductal carcinoma of the breast, and patients with Cowden syndrome; pancreatic cancer; central nervous system tumor; neuroblastoma; glioma; peripheral neuroepithelioma; extramedullary plasmacytoma; plasmacytoma; gastric cancer; gastrointestinal stromal tumors; esophageal cancer; colorectal adenocarcinoma; esophageal squamous cell carcinoma; liver cancer; renal cell carcinoma; bladder cancer; endometrial cancer; metrocarcinoma; head and neck cancer; brain cancer; oral cancer; sarcoma, including rhabdomyosarcoma, various lipogenic tumors, Ewing’s sarcoma/primitive neuroectodermal tumors (Ewing/PNETs), and leiomyosarcoma; urothelial carcinoma; basal cell carcinoma; oral squamous cell carcinoma; cholangiocarcinoma; bone cancer; cervical cancer; skin cancer; and Richter syndrome (RS).

In some embodiments, the pharmaceutical composition of the present disclosure comprising, as an active ingredient, the compounds of Formula (I) of the present disclosure or a pharmaceutically acceptable salt thereof can be formulated into any suitable formulations such as sprays, patches, tablets (such as conventional tablets, dispersible tablets, orally disintegrating tablets), capsules (such as soft capsules, hard capsules, enteric-coated capsules), dragees,

troches, powders, granules, powder injections, suppositories, or liquid formulations (such as suspensions (e.g., aqueous or oily suspensions), solutions, emulsions, or syrups), or conventional injection dosage forms such as injectable solutions (e.g., sterile injectable solutions formulated according to methods known in the art using water, Ringer's solution, or isotonic sodium chloride solution or the like as a vehicle or solvent) or lyophilized injectable formulation and the like, depending upon a suitable route of administration (including, but not limited to, nasal administration, inhalation administration, topical administration, oral administration, oral mucosal administration, rectal administration, intrapleural administration, intraperitoneal administration, vaginal administration, intramuscular administration, subcutaneous administration, transdermal administration, epidural administration, intrathecal administration, and intravenous administration). Those skilled in the art can also formulate the compounds of Formula (I) of the present disclosure into conventional, dispersible, chewable, orally disintegrating or rapidly dissolving formulations, or sustained-release capsules or controlled-release capsules as needed.

In some embodiments, the present disclosure provides a kit or reagent kit comprising: the compound of Formula (I) of the present disclosure or a pharmaceutically acceptable salt, enantiomer, stereoisomer, solvate, isotopically enriched analog, prodrug, or polymorph thereof; or the pharmaceutical composition of the present disclosure. The kit/reagent kit may include a package or container including, but not limited to, ampoules, blister packs, pharmaceutical plastic bottles, vials, pharmaceutical glass bottles, containers, syringes, laminated flexible packaging, co-extruded film infusion containers, test tubes and dispensing devices, and the like. The kit/reagent kit may contain instructions for use of the product.

In some embodiments, the present disclosure provides the compound of Formula (I), or a pharmaceutically acceptable salt, enantiomer, stereoisomer, solvate, isotopically enriched analog, prodrug, or polymorph thereof, which is for use in the manufacture of a medicament for the treatment of a disease or disorder. In some embodiments, the disease or disorder comprises a disease or disorder associated with cereblon protein. In some embodiments, the disease or disorder includes, but is not limited to: tumors or cancers, infectious diseases, inflammatory diseases, autoimmune diseases, anemia, hemorrhagic shock, transplant rejection, multiple organ dysfunction syndrome (MODS), sarcoidosis, adult respiratory distress syndrome, cardiovascular diseases, Richter syndrome (RS), acute liver failure, and diabetes.

In some embodiments, the present disclosure provides a method for treating or preventing a disease or disorder in a subject, comprising administering to the subject a therapeutically effective amount of: the compound of Formula (I) of the present disclosure or a pharmaceutically acceptable salt, enantiomer, stereoisomer, solvate, isotopically enriched analog, prodrug, or polymorph thereof; or the pharmaceutical composition of the present disclosure. In some embodiments, the disease or disorder comprises a disease or disorder associated with cereblon protein. In some embodiments, the disease or disorder includes, but is not limited to: tumors or cancers, infectious diseases, inflammatory diseases, autoimmune diseases, anemia, hemorrhagic shock, transplant rejection, multiple organ dysfunction syndrome (MODS), sarcoidosis, adult respiratory distress syndrome, cardiovascular diseases, Richter syndrome (RS), acute liver failure, and diabetes.

In some embodiments, the disease or disorder includes, but is not limited to: myeloma, including multiple myeloma, plasma cell myeloma, smoldering myeloma, smoldering multiple myeloma; myelofibrosis; bone marrow disease; myelodysplastic syndrome (MDS); previously treated myelodysplastic syndrome; transplantation-related cancer; neutropenia; leukemia, including acute myeloid leukemia (AML), chronic myeloid leukemia (CML), chronic myelogenous leukemia, acute lymphoblastic leukemia (ALL), chronic lymphocytic leukemia (CLL), B-cell chronic lymphocytic leukemia, leukemia-associated anemia, monocytic leukemia, myelomonocytic leukemia, T-lymphocytic leukemia, acute T-lymphocytic leukemia; lymphoma, including diffuse large B-cell lymphoma, non-Hodgkin's lymphoma, Hodgkin's lymphoma, anaplastic lymphoma, anaplastic large cell lymphoma, immunoblastic T-Cell lymphoma, CD20 positive lymphoma, mantle cell lymphoma, follicular lymphoma (FL), Burkitt's lymphoma, marginal zone lymphoma (MZL), primary lymphoma, B-cell lymphoma, recurrent B-cell non-Hodgkin's lymphoma, recurrent diffuse large B-cell lymphoma, recurrent mediastinal (thymic) large B-cell lymphoma, primary mediastinal (thymic) large B-cell lymphoma, recurrent transformed non-Hodgkin's lymphoma, refractory B-cell non-Hodgkin's lymphoma, refractory diffuse large B-cell lymphoma, refractory primary mediastinal (thymic) large B-cell lymphoma, refractory transformed non-Hodgkin's lymphoma; thyroid cancer; melanoma; lung cancer, including lung adenocarcinoma, lung squamous cell carcinoma, non-small cell lung cancer, and small cell lung cancer; inflammatory myofibroblastoma; colorectal cancer; intestinal cancer; brain glioma; astroblastoma; ovarian cancer; bronchial cancer; prostate cancer; breast cancer, including triple negative breast cancer, sporadic breast cancer, ductal carcinoma of the breast, and patients with Cowden syndrome; pancreatic cancer; central nervous system tumor; neuroblastoma; glioma; peripheral neuroepithelioma; extramedullary plasmacytoma; plasmacytoma; gastric cancer; gastrointestinal stromal tumors; esophageal cancer; colorectal adenocarcinoma; esophageal squamous cell carcinoma; liver cancer; renal cell carcinoma; bladder cancer; endometrial cancer; metrocarcinoma; head and neck cancer; brain cancer; oral cancer; sarcoma, including rhabdomyosarcoma, various lipogenic tumors, Ewing's sarcoma/primitive neuroectodermal tumors (Ewing/PNETs), and leiomyosarcoma; urothelial carcinoma; basal cell carcinoma; oral squamous cell carcinoma; cholangiocarcinoma; bone cancer; cervical cancer; skin cancer; Richter syndrome (RS); sepsis syndrome; autoimmune diseases, including rheumatoid arthritis, autoimmune encephalomyelitis,

ankylosing spondylitis, psoriasis, systemic lupus erythematosus, multiple sclerosis, recurrent oral ulcers, Kawasaki disease, polymyositis/dermatomyositis, Sjogren's syndrome, and atopic dermatitis; keratoconjunctivitis; inflammatory diseases, including Crohn's disease and ulcerative colitis, pneumonia, osteoarthritis, synovitis, systemic inflammatory response syndrome, airway inflammation, bronchitis; cerebral malaria; infectious diseases, including viral pneumonia, Acquired immunodeficiency syndrome (AIDS), COVID-19 novel coronavirus infection, gram-negative bacteria infection, gram-positive bacteria infection, tuberculosis, etc; septic shock; tuberculosis; bacterial meningitis; chronic obstructive pulmonary disease; asthma; hemorrhagic shock; organ (including kidney, heart, lung) or tissue transplantation rejection; diabetes; sarcoidosis; adult respiratory distress syndrome; anemia; pediatric aplastic anemia; cardiovascular diseases (e.g., coronary heart disease, congestive heart failure, myocardial infarction, atherosclerosis); multiple organ dysfunction caused by cachexia and septic shock; and acute liver failure.

In the method for treating diseases or disorders in a subject, the compound of Formula (I) of the present disclosure or the pharmaceutical composition of the present disclosure is administered to the subject through at least one mode of administration selected from the group consisting of nasal administration, inhalation administration, topical administration, oral administration, oral mucosal administration, rectal administration, pleural cavity administration, peritoneal administration, vaginal administration, intramuscular administration, subcutaneous, transdermal, epidural, intrathecal, and intravenous administration.

The term "treatment" or "treating" refers to the administration of the compound of Formula (I) or a pharmaceutically acceptable salt thereof according to the present disclosure, or the pharmaceutical composition containing, as an active ingredient, the compound of Formula (I) or a pharmaceutically acceptable salt thereof, to a subject to mitigate (alleviate) undesirable diseases or conditions, such as the development of a cancer or tumor. The beneficial or desired clinical results of the present disclosure include, but are not limited to: alleviating symptoms, reducing the severity of the disease, stabilizing the state of the disease, slowing down or delaying the progression of the disease, improving or alleviating the condition, and alleviating the disease.

A "therapeutic effective amount" of the compound of the present disclosure depends on a variety of factors, including the activity of the specific compound used, the metabolic stability of the compound and the duration of its action, the age, sex and weight of the patient, the patient's current medical condition, the route and duration of administration, the excretion rate, the combined administration of additional drugs, and the progression of the diseases or conditions of the patient being treated. Those skilled in the art will be able to determine appropriate dosages based on these and other factors.

As used herein, the term "patient" or "subject" to be treated refers to animal, for example mammal, including but not limited to primate (such as human being), cow, sheep, goat, horse, dog, cat, rabbit, guinea pig, rat, mice, etc.

Term Explanations and Definitions

Unless otherwise specified, the following words, phrases, and symbols used in this specification and the claims generally have the meanings as described below.

As used herein, the term "compound" includes all stereoisomers, geometric isomers, tautomers, and isotopically labeled compounds.

The compounds of the present invention may have a stereo-configuration and thus can exist in more than one stereoisomeric form. Unless otherwise specified, all stereoisomers are included, such as enantiomers and diastereomers. The compounds of the present invention containing an asymmetric carbon atom can be isolated in an optically pure form or in a racemic form. The optically active pure form can be resolved from a racemic mixture or synthesized using a chiral starting material or a chiral reagent.

The compounds of the present invention also include tautomeric forms. Tautomeric forms result from the exchange of a single bond with an adjacent double bond together with the concomitant migration of a proton.

As used herein, isotopes refer to two or more atoms of the same chemical element that have the same atomic number but different mass numbers. For example, hydrogen isotopes include tritium and deuterium.

As used herein, the term "stereoisomer" refers to compounds that have the same chemical structural formula but differ in the spatial arrangement of their atoms or groups. Stereoisomers include enantiomers, diastereomers, conformational isomers (rotamers), geometric isomers, (cis/trans) isomers, atropisomers, and the like. Herein, enantiomers refer to two non-superimposable mirror image isomers of a compound. Diastereomers refer to stereoisomers that have two or more chiral centers but are not mirror images of each other. Diastereomers have different physical properties, such as melting point, boiling point, spectral properties, and reactivity. Mixtures of diastereomers can be separated by high-resolution analytical techniques such as electrophoresis and chromatography, for example, HPLC.

As used herein, the term "solvate" refers to an association or complex formed by the interaction between one or more solvent molecules and the compounds of the present invention. Examples of solvents include water, isopropanol, ethanol, methanol, DMSO, ethyl acetate, acetic acid and ethanolamine. The term "hydrate" means that a complex formed with water.

In general, the nomenclature used herein (including the IUPAC nomenclature) and the laboratory procedures described below (including those used in cell culture, organic chemistry, analytical chemistry, and pharmacology, etc.) are those well-known and commonly used in the art. Unless otherwise defined, all scientific and technical terms used herein in connection with the present disclosure described herein have the same meaning as commonly understood by one skill in the art. In addition, the use of the word "a" or "an" when used in conjunction with the term "comprising" or a noun in the claims and/or the specification may mean "one", but it is also consistent with the meaning of "one or more", "at least one", and "one or more than one". Similarly, the terms "another" or "other" can mean at least a second or more.

It should be understood that whenever the term "comprise" or "include" is used herein to describe various aspects, other similar aspects described by "consisting of" and/or "consisting essentially of" are also provided.

As used herein, the wording "...represents a bond" means that the referenced group is a bond linker (that is, the referenced group is absent). For example, the wording "$R^1$ represents a bond" means that $R^1$ is a bond linker. In other words, when $R^1$ represents a bond, the group $R^2$ in the structure of Formula (I) is directly connected to the group L in the structure of Formula (I).

Herein, a bond interrupted by a wavy line shows the point of attachment of the depicted group to the rest of the molecule.

Herein, the wording "optionally substituted with..." and "unsubstituted or substituted" can be used interchangeably. The term "substituted" generally indicates that one or more hydrogens in the referenced structure are replaced by the same or different specific substituents.

As used herein, the term "halogen atom" or "halogen", used alone or in combination, refers to fluorine, chlorine, bromine, or iodine.

As used herein, the term "hydroxy" refers to -OH.

As used herein, the term "sulfonyl" refers to $-SO_2-$.

As used herein, the term "oxo", used alone or in combination, refers to =O.

As used herein, the term "alkyl", used alone or in combination, refers to a saturated, linear or branched aliphatic hydrocarbon group consisting of carbon and hydrogen atoms. The term "$C_x$-$C_y$ alkyl" or "$C_{x-y}$ alkyl" (x and y each being an integer) refers to a saturated, linear or branched aliphatic hydrocarbon group containing from x to y carbon atoms. The term "$C_1$-$C_6$ alkyl" used alone or in combination in the present disclosure refers to a linear or branched alkyl group containing from 1 to 6 carbon atoms. $C_1$-$C_6$ alkyl of the present disclosure include $C_1$-$C_5$ alkyl, $C_1$-$C_4$ alkyl, and $C_1$-$C_3$ alkyl. Representative examples include methyl, ethyl, n-propyl, isopropyl, n-butyl, isobutyl, sec-butyl, tert-butyl, pentyl, isopentyl, neopentyl, tert-pentyl, and hexyl. In the present disclosure, the "alkyl" or "$C_1$-$C_6$ alkyl" may be unsubstituted or substituted with one or more substituents selected from the group consisting of deuterium, hydroxy, halogen, cyano, amino, mercapto, $C_1$-$C_3$ alkyl, $C_1$-$C_3$ alkoxy, halogenated $C_1$-$C_3$ alkyl, halogenated $C_1$-$C_3$ alkoxy, or a combination thereof.

As used herein, the term "halogenated alkyl" or "haloalkyl", used alone or in combination, refers to a linear or branched alkyl group substituted with one or more halogens, wherein one or more hydrogen atom(s) of the alkyl group are replaced with one or more halogens. The term "halogenated $C_{x-Cy}$ alkyl" or "halogenated $C_{x-y}$ alkyl" (x and y are each an integer) refers to a linear or branched alkyl containing from x to y carbon atoms substituted with one or more halogens. The term "halogenated $C_{1-6}$ alkyl" used alone or in combination in the present disclosure refers to a linear or branched alkyl group containing from 1 to 6 carbon atoms substituted with one or more halogens. The halogenated $C_{1-6}$ alkyl group of the present disclosure includes, for example, halogenated $C_{1-5}$ alkyl group, halogenated $C_{1-4}$ alkyl group, and halogenated $C_{1-3}$ alkyl, with representative examples including halomethyl, haloethyl, halo-n-propyl, haloisopropyl, halo-n-butyl, haloisobutyl, halo-sec-butyl, halo-tert-butyl, halopentyl, haloisoamyl, haloneopentyl, halo-tert-pentyl, and halohexyl.

As used herein, the term "alkoxy", used alone or in combination, refers to a linear or branched alkoxy group having structural formula of alkyl-O-. The term "$C_1$-$C_6$ alkoxy", used alone or in combination, refers to a linear or branched alkoxy group may contain 1-6 carbon atoms. $C_1$-$C_6$ alkoxy of the present disclosure include $C_1$-$C_5$ alkoxy, $C_1$-$C_4$ alkoxy, and $C_1$-$C_3$ alkoxy. Representative examples of "$C_1$-$C_6$ alkoxy" include methoxy, ethoxy, propoxy, isopropoxy, n-butoxy, isobutoxy, tert-butoxy, pentyloxy, isopentyloxy, 2-pentyloxy, neopentyloxy, hexyloxy, 2-hexyloxy, 3-hexyloxy, 3-methylpentyloxy, etc.

As used herein, the term "halogenated alkoxy" used alone or in combination refers to a linear or branched alkoxy substituted with one or more halogens, wherein one or more hydrogens in said alkoxy are replaced by halogens. The term "$C_x$-$C_y$ halogenated alkoxy" (where x and y are each integers) refers to a linear or branched alkoxy containing x to y carbon atoms, substituted with one or more halogens. In the present invention, the term "$C_1$-$C_6$ halogenated alkoxy" used alone or in combination refers to a linear or branched alkoxy containing 1 to 6 carbon atoms, substituted with one or more halogens. The $C_1$-$C_6$ halogenated alkoxy of the present disclosure includes, for example, $C_1$-$C_5$ halogenated alkoxy, $C_1$-$C_4$ halogenated alkoxy, or $C_1$-$C_3$ halogenated alkoxy. Representative examples of the $C_1$-$C_6$ halogenated alkoxy include halogenated methoxy, halogenated ethoxy, halogenated n-propoxy, halogenated isopropoxy, halogenated n-butoxy, halogenated isobutoxy, halogenated sec-butoxy, halogenated tert-butoxy, halogenated pentyloxy, halogenated isopentyloxy, halogenated neopentyloxy, halogenated tert-pentyloxy, and halogenated hexyloxy, among others.

As used herein, the term "alkylene" used alone or in combination refers to a linear or branched divalent saturated hydrocarbon group consisting of carbon and hydrogen atoms. The term "$C_x$-$C_y$ alkylene" (where x and y are each integers) refers to a linear or branched alkylene containing x to y carbon atoms. The $C_1$-$C_6$ alkylene of the present disclosure includes, for example, $C_1$-$C_5$ alkylene, $C_1$-$C_4$ alkylene, or $C_1$-$C_3$ alkylene. Representative examples of the $C_1$-$C_6$ alkylene include, but are not limited to, methylene, ethylene, propylene, isopropylene, butylene, isobutylene, sec-butylene, tert-butylene, pentylene, isopentylene, neopentylene, tert-pentylene, and hexylene. The "alkylene" or "$C_1$-$C_6$ alkylene" may be unsubstituted or substituted with one or more substituents selected from deuterium, hydroxy, halogen, cyano, amino, mercapto, $C_1$-$C_3$ alkyl, $C_1$-$C_3$ alkoxy, halogenated $C_1$-$C_3$ alkyl, halogenated $C_1$-$C_3$ alkoxy, or a combination thereof.

As used herein, the term "aryl" used alone or in combination refers to a monovalent aromatic hydrocarbon group containing 5 to 30 (e.g., 5 to 20, 5 to 15, 6 to 20, 6 to 15, 7 to 20, 7 to 15, 8 to 20, 8 to 15, 9 to 20, 9 to 15, 10 to 20, 10 to 15) carbon atoms and optionally containing one or more fused rings, such as phenyl, naphthyl, or fluorenyl. In the present invention, said "aryl" is optionally substituted aryl. Substituted aryl refers to aryl substituted one or more times (e.g., 1-4, 1-3, or 1-2 times) with a substituent(s). For example, aryl may be mono-, di-, or tri-substituted by substituents which are optionally selected from, for example: deuterium, halogen, hydroxy, cyano, amino, mercapto, $C_1$-$C_6$ alkyl, halogenated $C_1$-$C_6$ alkyl, $C_1$-$C_6$ alkoxy, halogenated $C_1$-$C_6$ alkoxy, or any combination thereof.

As used herein, the term "arylene" used alone or in combination refers to a divalent aromatic hydrocarbon group containing 5 to 30 (e.g., 5 to 20, 5 to 15, 6 to 20, 6 to 15, 7 to 20, 7 to 15, 8 to 20, 8 to 15, 9 to 20, 9 to 15, 10 to 20, 10 to 15) carbon atoms and optionally containing one or more fused rings, such as phenylene, naphthylene, or fluorenylene. In the present invention, said "arylene" is optionally substituted arylene. Substituted arylene refers to arylene substituted one or more times (e.g., 1 to 4, 1 to 3, or 1 to 2 times) with a substituent(s). For example, arylene may be mono-, di-, or tri-substituted by substituents which are optionally selected from, for example: deuterium, halogen, hydroxy, cyano, amino, mercapto, $C_1$-$C_6$ alkyl, halogenated $C_1$-$C_6$ alkyl, $C_1$-$C_6$ alkoxy, halogenated $C_1$-$C_6$ alkoxy, or any combination thereof.

As used herein, the term "cycloalkyl" used alone or in combination refers to a saturated or partially unsaturated (i.e., having one or more double bonds, but not fully conjugated) monocyclic, bicyclic, or polycyclic ring hydrocarbon cyclic hydrocarbon radical, which in some embodiments contains 3 to 30 carbon atoms (i.e., $C_3$-$C_{30}$ cycloalkyl), 3 to 20 carbon atoms (i.e., $C_3$-$C_{20}$ cycloalkyl), 3 to 15 carbon atoms (i.e., $C_3$-$C_{15}$ cycloalkyl), 3 to 12 carbon atoms (i.e., $C_3$-$C_{12}$ cycloalkyl), 3 to 11 carbon atoms (i.e., $C_3$-$C_{11}$ cycloalkyl), 3 to 10 carbon atoms (i.e., $C_3$-$C_{10}$ cycloalkyl), 3 to 9 carbon atoms (i.e., $C_3$-$C_9$ cycloalkyl), 3 to 8 carbon atoms (i.e., $C_3$-$C_8$ cycloalkyl), 3 to 7 carbon atoms (i.e., $C_3$-$C_7$ cycloalkyl), 3 to 6 carbon atoms (i.e., $C_3$-$C_6$ cycloalkyl), 4 to 20 carbon atoms (i.e., $C_4$-$C_{20}$ cycloalkyl), or 4 to 15 carbon atoms (i.e., $C_4$-$C_{15}$ cycloalkyl). The term "cycloalkyl" includes monocyclic, bicyclic, tricyclic, or polycyclic cycloalkyls with 3 to 30 carbon atoms. Examples of the term "cycloalkyl" include but are not limited to monocyclic cycloalkyl (e.g., $C_3$-$C_{30}$ monocyclic cycloalkyl, $C_3$-$C_{20}$ monocyclic cycloalkyl, $C_3$-$C_{15}$ monocyclic cycloalkyl, $C_4$-$C_{15}$ monocyclic cycloalkyl, $C_5$-$C_{15}$ monocyclic cycloalkyl, $C_7$-$C_{30}$ monocyclic cycloalkyl, $C_7$-$C_{20}$ monocyclic cycloalkyl, $C_7$-$C_{15}$ monocyclic cycloalkyl), bridged cycloalkyl (e.g., $C_5$-$C_{30}$ bridged cycloalkyl, $C_5$-$C_{20}$ bridged cycloalkyl, $C_5$-$C_{15}$ bridged cycloalkyl, $C_6$-$C_{30}$ bridged cycloalkyl, $C_6$-$C_{20}$ bridged cycloalkyl, $C_6$-$C_{15}$ bridged cycloalkyl, $C_7$-$C_{30}$ bridged cycloalkyl, $C_7$-$C_{20}$ bridged cycloalkyl, $C_7$-$C_{15}$ bridged cycloalkyl), fused cycloalkyl (e.g., $C_5$-$C_{30}$ fused cycloalkyl, $C_5$-$C_{20}$ fused cycloalkyl, $C_5$-$C_{15}$ fused cycloalkyl, $C_6$-$C_{30}$ fused cycloalkyl, $C_6$-$C_{20}$ fused cycloalkyl, $C_6$-$C_{15}$ fused cycloalkyl, $C_7$-$C_{30}$ fused cycloalkyl, $C_7$-$C_{20}$ fused cycloalkyl, $C_7$-$C_{15}$ fused cycloalkyl), and spiro-cycloalkyl (e.g., $C_5$-$C_{30}$ spiro-cycloalkyl, $C_5$-$C_{20}$ spiro-cycloalkyl, $C_5$-$C_{15}$ spiro-cycloalkyl, $C_6$-$C_{30}$ spiro-cycloalkyl, $C_6$-$C_{20}$ spiro-cycloalkyl, $C_6$-$C_{15}$ spiro-cycloalkyl, $C_7$-$C_{30}$ spiro-cycloalkyl, $C_7$-$C_{20}$ spiro-cycloalkyl, $C_7$-$C_{15}$ spiro-cycloalkyl). Representative examples of monocyclic cycloalkyl include, but are not limited to, cyclopropyl, cyclobutyl, cyclopentyl, cyclopentenyl, cyclohexyl, cyclohexenyl, cycloheptyl, and cyclooctyl. Examples of bridged cycloalkyl, fused cycloalkyl, and spiro-cycloalkyl include, but are not limited to, decalinyl, octahydropentalenyl, $C_5$-$C_{20}$ spiro-cycloalkyl, adamantanyl, noradamantanyl, bornyl, norbornyl (also named as bicyclo[2.2.1]heptyl by the IUPAC system). As used herein, the "cycloalkyl" is optionally mono- or poly-substituted, such as, but not limited to, 2,2-, 2,3-, 2,4-, 2,5-, or 2,6-disubstituted cyclohexyl. The substituents of the substituted "cycloalkyl" are optionally one or more (e.g., 1-5, 1-4, 1-3, 1-2, or 1) substituents selected from: deuterium, =O, halogen, hydroxy, cyano, amino, mercapto, $C_1$-$C_6$ alkyl, halogenated $C_1$-$C_6$ alkyl, $C_1$-$C_6$ alkoxy, halogenated $C_1$-$C_6$ alkoxy or any combination thereof.

As used herein, the term "cycloalkylene", used alone or in combination, refers to a saturated or partially unsaturated (i.e., having one or more double bonds, but not fully conjugated) monocyclic, bicyclic, or polycyclic cyclic hydrocarbon divalent group having 3 to 30 carbon atoms (e.g., 3 to 25, 3 to 20, 3 to 15, 3 to 12, 3 to 10, 3 to 9, 3 to 8, 3 to 7, 3 to 6, 4 to 20, 4 to 15, 4 to 12, 4 to 10, 4 to 9, 4 to 8, 4 to 7, or 4 to 6 carbon atoms). The term "cycloalkylene" includes monocyclic, bicyclic, tricyclic, or polycyclic cycloalkylenes having 3 to 30 carbon atoms. Examples of the term cycloalkylene include, but are not limited to, monocyclic cycloalkylene (e.g., $C_3$-$C_{30}$ monocyclic cycloalkylene, $C_3$-$C_{20}$ monocyclic cycloalkylene, $C_3$-$C_{15}$ monocyclic cycloalkylene, $C_4$-$C_{15}$ monocyclic cycloalkylene, $C_5$-$C_{15}$ monocyclic cycloalkylene, $C_7$-$C_{30}$ monocyclic cycloalkylene, $C_7$-$C_{20}$ monocyclic cycloalkylene, $C_7$-$C_{15}$ monocyclic cycloalkylene), bridged cycloalkylene (e.g., $C_5$-$C_{30}$ bridged cycloalkylene, $C_5$-$C_{20}$ bridged cycloalkylene, $C_5$-$C_{15}$ bridged cycloalkylene, $C_6$-$C_{30}$ bridged cycloalkylene, $C_6$-$C_{20}$ bridged cycloalkylene, $C_6$-$C_{15}$ bridged cycloalkylene, $C_7$-$C_{30}$ bridged cycloalkylene, $C_7$-$C_{20}$ bridged cycloalkylene, $C_7$-$C_{15}$ bridged cycloalkylene), fused cycloalkylene (e.g., $C_5$-$C_{30}$ fused cycloalkylene, $C_5$-$C_{20}$ fused

cycloalkylene, $C_5$-$C_{15}$ fused cycloalkylene, $C_6$-$C_{30}$ fused cycloalkylene, $C_6$-$C_{20}$ fused cycloalkylene, $C_6$-$C_{15}$ fused cycloalkylene, $C_7$-$C_{30}$ fused cycloalkylene, $C_7$-$C_{20}$ fused cycloalkylene, $C_7$-$C_{15}$ fused cycloalkylene), and spiro-cycloalkylene (e.g., $C_5$-$C_{30}$ spiro-cycloalkylene, $C_5$-$C_{20}$ spiro-cycloalkylene, $C_5$-$C_{15}$ spiro-cycloalkylene, $C_6$-$C_{30}$ spiro-cycloalkylene, $C_6$-$C_{20}$ spiro-cycloalkylene, $C_6$-$C_{15}$ spiro-cycloalkylene, $C_7$-$C_{30}$ spiro-cycloalkylene, $C_7$-$C_{20}$ spiro-cycloalkylene, $C_7$-$C_{15}$ spiro-cycloalkylene). Representative examples of monocyclic cycloalkylene include, but are not limited to, cyclopropylene, cyclobutylene, cyclopentylene, cyclopentenylene, cyclohexylene, cyclohexenylene, cycloheptylene, and cyclooctylene. Examples of bridged cycloalkylene, fused cycloalkylene, and spiro-cycloalkylene include, but are not limited to, decalinylene, octahydropentalenylene, $C_5$-$C_{20}$ spiro-cycloalkylene, adamantanylene, noradamantanylene, norbomylene (also named as bicyclo[2.2.1]heptylene by the IUPAC system). As used herein, the "cycloalkylene" is optionally mono- or poly-substituted, such as, but not limited to: 2,2-, 2,3-, 2,4-, 2,5- or 2,6-disubstituted cyclohexylene. The substituents of the substituted "cycloalkylene" can be optionally one or more (e.g., 1-5, 1-4, 1-3, 1-2, or 1) substituents selected from the group consisting of deuterium, = O, halogen, hydroxy, cyano, amino, mercapto, $C_1$-$C_6$ alkyl, halogenated $C_1$-$C_6$ alkyl, $C_1$-$C_6$ alkoxy, halogenated $C_1$-$C_6$ alkoxy or any combination thereof.

As used herein, the term "heteroaryl", used alone or in combination, refers to a 5- to 30-membered (e.g., 5- to 20-membered, 5- to 15-membered, 5- to 12-membered, 5- to 11-membered, 5- to 10-membered, 5- to 9-membered, 5- to 8-membered, 5- to 7-membered, 5- to 6-membered, 6- to 15-membered, or 6- to 9-membered) monovalent monocyclic, bicyclic, or polycyclic cyclic hydrocarbon radical containing at least one aromatic ring having one or more (e.g., from 1 to 6, or from 1 to 5, or from 1 to 4, or from 1 to 3) heteroatoms independently selected from the group consisting of oxygen, nitrogen, and sulfur. Bicyclic or polycyclic heteroaryl groups include bicyclic, tricyclic, tetracyclic or polycyclic heteroaryl groups, which contain one aromatic ring having one or more heteroatoms independently selected from O, S and N, and the remaining rings which may be a saturated, partially unsaturated or aromatic ring and can be carbocyclic ring or contain one or more heteroatoms independently selected from O, S and N. Representative examples of heteroaryl groups include, but are not limited to, furanyl, oxazolyl, isoxazolyl, oxadiazolyl, thienyl, thiazolyl, isothiazolyl, thiadiazolyl, pyrrolyl, imidazolyl, pyrazolyl, triazolyl, triazinyl, pyridyl, pyrimidinyl, pyridazinyl, pyrazinyl, indolyl, isoindolyl, isoindolinyl, benzofuranyl, isobenzofuranyl, benzothienyl, indazolyl, benzimidazolyl, benzoxazolyl, benzisoxazolyl, benzothiazolyl, benzisothiazolyl, benzotriazolyl, chromanyl, benzo[2,1,3]oxadiazolyl, benzo[2,1,3]thiadiazolyl, benzo[1,2,3]thiadiazolyl, benzo[b]thien-4-yl, quinolinyl, isoquinolinyl, dihydroquinolinyl, isoxazolo[4,5-c]pyridinyl, 2,3-dihydro-1H-benzo[d]imidazol-1-yl, 3,4-dihydroquinolin-1(2H)-yl, dihydro-4H-benzo[b][1,4]oxazin-4-yl, chroman-4-yl, thieno[2,3-d]pyrimidin-4-yl, 5,6,7,8-tetrahydrobenzo[4,5]thieno[2,3-d]pyrimidin-4-yl, 6,7-dihydro-5H-cyclopenta[4,5]thieno[2,3-d]pyrimidin-4-yl, 6,7-dihydrothieno[3,2-c]pyridin-5(4H)-yl, thieno[2,3-d]pyrimidin-4-yl, thieno[2,3-d]pyrimidin-2-yl, 5,6-dihydro-[1,2,4]triazolo[4,3-a]pyrazin-7(8H)-yl, pyrazolo[1,5]thiadiazolyl, pyrazolo[1,5-a]pyrimidinyl, imidazo[1,2-a]pyridyl, 1H-pyrrolo[3,2-b]pyridyl, 1H-pyrrolo[2,3-b]pyridyl, pyrrolo[2,1-b]thiazolyl, imidazo[2,1-b]thiazolyl, chromanyl, 6,7-dihydrothieno[3,2-d]pyrimidinyl, acridinyl, benzindolyl, carbazolyl, dibenzofuranyl, xanthenyl, 5,6,7,8-tetrahydrobenzo[4,5]thieno[2,3-d]pyrimidinyl, and 6,7-dihydro-5H-cyclopenta[4,5]thieno[2,3-d]pyrimidinyl. The heteroaryl group may be unsubstituted or substituted. A substituted heteroaryl refers to a heteroaryl substituted one or more times (e.g., 1-4, 1-3, or 1-2 times) by a substituent(s) optionally selected from the group consisting of deuterium, = O, halogen, hydroxy, cyano, amino, mercapto, $C_1$-$C_6$ alkyl, halogenated $C_1$-$C_6$ alkyl, $C_1$-$C_6$ alkoxy, halogenated $C_1$-$C_6$ alkoxy or any combination thereof.

As used herein, the term "heteroarylene", used alone or in combination, refers to a 5- to 30-membered (e.g., 5- to 20-membered, 5- to 15-membered, 5- to 12-membered, 5- to 11-membered, 5- to 10-membered, 5- to 9-membered, 5- to 8-membered, 5- to 7-membered, 5- to 6-membered, 6- to 15-membered, or 6- to 9-membered) bivalent monocyclic, bicyclic, or polycyclic cyclic hydrocarbon radical containing at least one aromatic ring having one or more (e.g., from 1 to 6, or from 1 to 5, or from 1 to 4, or from 1 to 3) heteroatoms independently selected from the group consisting of oxygen, nitrogen, and sulfur. Bicyclic or polycyclic heteroarylene groups include bicyclic, tricyclic, tetracyclic or polycyclic heteroarylene groups, which contain one aromatic ring having one or more heteroatoms independently selected from O, S and N, and the remaining ring(s) which may be a saturated, partially unsaturated or aromatic ring and can be carbocyclic ring or contain one or more heteroatoms independently selected from O, S and N. Representative examples of heteroarylene include, but are not limited to, furanylene, oxazolylene, isoxazolylene, oxadiazolylene, thienylene, isothiazolylene, thiadiazolylene, pyrrolylene, imidazolylene, pyrazolylene, triazolylene, pyridylene, pyrimidinylene, pyridazinylene, pyrazinylene, indolylene, isoindolylene, indazolylene, quinolinylene, isoquinolinylene, quinazolinylene, triazinylene, isoxazolo[4,5-c]pyridinylene, pyrrolo[2,3-b]pyridinylene, indolinylene, benzothiazolylene, benzofuranylene, isobenzofuranylene, benzothienylene, benzimidazolylene, 2,3-dihydro-1H-benzo[d]imidazolylene, pyrrolo[2,3-b]pyridinylene, chromanylene, 6,7-dihydrothieno[3,2-d]pyrimidinylene, acridinylene, benzindolylene, carbazolyl, dibenzofuranylene, xanthenylene, 3,4-dihydroquinolinylene, 2,3-dihydro-4H-benzo[b][1,4]oxazinylene, chromanylene, thieno[2,3-d]pyrimidinylene, thieno[3,2-d]pyrimidinylene, tetrahydrobenzo[4,5]thieno[2,3-d]pyrimidinylene, 6,7-dihydro-5H-cyclopenta[4,5]thieno[2,3-d]pyrimidinylene, dihydrothieno[3,2-d]pyrimidinylene, 5,6-dihydro-[1,2,4]triazolo[4,3-a]pyrazinylene, dihydrothieno[3,2-c]pyridinylene, benzoxazolylene, benzisoxazolylene, benzisothiazolylene, benzotriazolylene, benzo[2,1,3]oxadiazolylene, benzo[2,1,3]thiadiazolylene, benzo[1,2,3]thiadiazolylene, pyrazolo[1,5-a]pyridylene, pyrazolo[1,5-a]pyrimidinylene, imidazo[1,2-a]pyridylene, 1H-pyrrolo[3,2-b]thiazolylene, or benzo[2,1-b]thiazolylene. The heteroarylene group may be unsubstituted or substituted. A substituted heteroarylene refers to a heteroarylene substituted one or more times (e.g., 1-4, 1-3, or 1-2 times) by a substituent(s) optionally selected from the group consisting of deuterium, =O, halogen, hydroxy, cyano, amino, mercapto, $C_1$-$C_6$ alkyl, halogenated $C_1$-$C_6$ alkyl, $C_1$-$C_6$ alkoxy, halogenated $C_1$-$C_6$ alkoxy or any combination thereof.

As used herein, the term "heterocyclyl" or "heterocyclic group", used alone or in combination, refers to a 4- to 30-membered (e.g., 4- to 25-membered, 4- to 20-membered, 4- to 15-membered, 5- to 20-membered, 5- to 15-membered, 6- to 20-membered, 6- to 15-membered, 7- to 20-membered, or 7- to 15-membered) saturated or partially unsaturated (i.e., containing one or more double bonds, but not fully conjugated) monocyclic, bicyclic, tricyclic or polycyclic cyclic hydrocarbon group containing one or more (e.g., from 1 to 5, or from 1 to 4, from 1 to 3, from 1 to 2, or 1) heteroatoms independently selected from sulfur, oxygen, and nitrogen. Representative examples of the heterocyclyl groups include, but not limited to, monocyclic heterocyclyl (e.g., 4- to 30-membered, 4- to 25-membered, 4- to 20-membered, or 4- to 15-membered monocyclic heterocyclyl), bridged heterocyclyl (e.g., 5- to 30-membered, 5- to 20-membered, 5- to 15-membered, 6- to 20-membered, 6- to 15-membered, 7- to 30-membered, 7- to 20-membered, or 7-to 15-membered bridged heterocyclyl), fused heterocyclyl (e.g., 5- to 30-membered, 5- to 20-membered, 5- to 15-membered, 6- to 20-membered, 6- to 15-membered, 7- to 30-membered, 7- to 20-membered, or 7-to 15-membered fused heterocyclyl), and spiro-heterocyclyl groups (e.g., 5- to 30-membered, 5- to 20-membered, 5- to 15-membered, 6- to 20-membered, 6- to 15-membered, 7- to 30-membered, 7- to 20-membered, or 7- to 15-membered spiro-heterocyclyl). Representative examples of heterocyclyl include, but are not limited to, azetidinyl, oxetanyl, pyrrolidinyl, imidazolidinyl, pyrazolidyl, piperidinyl, tetrahydrofuranyl, tetrahydropyranyl, tetrahydrothienyl, tetrahydrothiopyranyl, oxazolidinyl, thiazolidinyl, piperidinyl, piperazinyl, morpholinyl, thiomorpholinyl, dioxacyclohexyl, azetidin-1-yl, 3,6-dihydropyridin-1(2H)-yl, 1,4-diazepan-1-yl, 3,6-diazabicyclo[3.1.1]heptan-6-yl, 3,6-diazabicyclo[3.1.1]heptan-3-yl, 2,5-diazabicyclo[2.2.1]heptan-2-yl, 2,5-diazabicyclo[2.2.2]octan-2-yl, 3,8-diazabicyclo[3.2.1]octan-3-yl, 3,8-diazabicyclo[3.2.1]octan-8-yl, and azaspiro-heterocyclyl. The heterocyclyl group may be unsubstituted or substituted. A substituted heterocyclyl refers to a heterocyclyl substituted one or more times (e.g., 1-4, 1-3, or 1-2 times) by a substituent(s) optionally selected from the group consisting of deuterium, = O, halogen, hydroxy, cyano, amino, mercapto, $C_1$-$C_6$ alkyl, halogenated $C_1$-$C_6$ alkyl, $C_1$-$C_6$ alkoxy, halogenated $C_1$-$C_6$ alkoxy or any combination thereof.

As used herein, the term "heterocyclylene", used alone or in combination, refers to a 4- to 30-membered (e.g., 4- to 25-membered, 4- to 20-membered, 4- to 15-membered, 5- to 20-membered, 5- to 15-membered, 6- to 20-membered, 6- to 15-membered, 7- to 20-membered, or 7- to 15-membered) saturated or partially unsaturated (i.e., containing one or more double bonds, but not fully conjugated) monocyclic, bicyclic, tricyclic or polycyclic bivalent cyclic hydrocarbon group containing one or more (e.g., from 1 to 5, or from 1 to 4, from 1 to 3, from 1 to 2, or 1) heteroatoms independently selected from sulfur, oxygen, and nitrogen. Representative examples of the heterocyclylene include, but not limited to, monocyclic heterocyclylene (e.g., 4- to 30-membered, 4- to 25-membered, 4- to 20-membered, or 4- to 15-membered monocyclic heterocyclylene), bridged heterocyclylene (e.g., 5- to 30-membered, 5- to 20-membered, 5- to 15-membered, 6- to 20-membered, 6- to 15-membered, 7- to 30-membered, 7- to 20-membered, or 7- to 15-membered bridged heterocyclylene), fused heterocyclylene (e.g., 5- to 30-membered, 5- to 20-membered, 5- to 15-membered, 6- to 20-membered, 6- to 15-membered, 7- to 30-membered, 7- to 20-membered, or 7-to 15-membered fused heterocyclylene), and spiro-heterocyclylene (e.g., 5- to 30-membered, 5- to 20-membered, 5- to 15-membered, 6- to 20-membered, 6- to 15-membered, 7- to 30-membered, 7- to 20-membered, or 7- to 15-membered spiro-heterocyclylene). Representative examples of the heterocyclylene include, but not limited to, azetidinylene, oxetanylene, pyrrolidinylene, imidazolidinylene, pyrazolidylene, piperidinylene, tetrahydrofuranylene, tetrahydropyranylene, tetrahydrothienylene, tetrahydrothiopyranylene, oxazolidinylene, thiazolidinylene, piperidinylene, piperazinylene, morpholinylene, tetrahydrothienylene, thiomorpholinylene, dioxacyclohexylene, azetidin-1-ylene, 3,6-dihydropyridin-1(2H)-ylene, 1,4-diazepan-1-ylene, 3,6-diazabicyclo[3.1.1]heptan-6-ylene, 3,6-diazabicyclo[3.1.1]heptan-3-ylene, 2,5-diazabicyclo[2.2.1]heptan-2-ylene, 2,5-diazabicyclo[2.2.2]octan-2-ylene, 3,8-diazabicyclo[3.2.1]octan-3-ylene, 3,8-diazabicyclo[3.2.1]octan-8-ylene, and azaspiro-heterocyclylene. The "heterocyclylene" may be unsubstituted or substituted. A substituted heterocyclylene refers to a heterocyclylene substituted one or more times (e.g., 1-4, 1-3, or 1-2 times) by a substituent(s) optionally selected from the group consisting of deuterium, = O, halogen, hydroxy, $C_1$-$C_6$ alkyl, halogenated $C_1$-$C_6$ alkyl, $C_1$-$C_6$ alkoxy, or any combination thereof.

As used herein, the suffix "ylylidene" for a group name indicates that the mentioned group is connected to the rest of the molecule through one single bond and one double bond, constituting a trivalent group.

As used herein, the term "heterocyclylylidene", used alone or in combination, refers to a 4- to 30-membered (e.g., 4- to 25-membered, 4- to 20-membered, 4- to 15-membered, 5- to 20-membered, 5- to 15-membered, 6- to 20-membered, 6- to 15-membered, 7- to 20-membered, or 7- to 15-membered) saturated or partially unsaturated (i.e., containing one or more double bonds, but not fully conjugated) monocyclic, bicyclic, tricyclic or polycyclic trivalent cyclic hydrocarbon group containing one or more (e.g., from 1 to 5, or from 1 to 4, from 1 to 3, from 1 to 2, or 1) heteroatoms independently selected from sulfur, oxygen, and nitrogen. Representative examples of the heterocyclylylidene include, but not limited to, monocyclic heterocyclylylidene (e.g., 4- to 30-membered, 4- to 25-membered, 4- to 20-membered, or 4- to 15-membered monocyclic heterocyclylylidene), bridged heterocyclylylidene (e.g., 5- to 30-membered, 5- to 20-membered, 5- to 15-membered, 7- to 30-membered, 7- to 20-membered, or 7- to 15-membered bridged heterocyclylylidene), fused heterocyclylylidene (e.g., 5- to 30-membered, 5- to 20-membered, 5- to 15-membered, 7- to 30-membered, 7- to 20-membered, or 7- to 15-membered fused heterocyclylylidene), and spiro-heterocyclylylidene (e.g., 5- to 30-membered, 5- to 20-membered, 5- to 15-membered, 7- to 30-membered, 7- to 20-membered, or 7- to 15-membered spiro-heterocyclylylidene). Representative examples of the heterocyclylylidene include, but not limited to, azetidinylylidene, oxetanylylidene, pyrrolidinylylidene, imidazolidinylylidene, pyrazolidinylylidene, piperidinylylidene, tetrahydrofuranylylidene, tetrahydropyranylylidene, tetrahydrothienylylidene, tetrahydrothiopyranylylidene, oxazolidinylylidene, thiazolidinylylidene, piperidinylylidene, piperazinylylidene, morpholinylylidene, tetrahydrothienylylidene, thiomorpholinylylidene, dioxacyclohexylylidene, azetidin-1-ylylidene, 3,6-dihydropyridin-1(2H)-ylylidene, 1,4-diazepan-1-ylylidene, 3,6-diazabicyclo[3.1.1]heptan-6-ylylidene, 3,6-diazabicyclo[3.1.1]heptan-3-ylylidene, 2,5-diazabicyclo[2.2.1]heptan-2-ylylidene, 2,5-diazabicyclo[2.2.2]octan-2-ylylidene, 3,8-diazabicyclo[3.2.1]octan-3-ylylidene, 3,8-diazabicyclo[3.2.1]octan-8-ylylidene, and azaspiro-heterocyclylylidene. The "heterocyclylylidene" may be unsubstituted or substituted. A substituted heterocyclylylidene refers to a heterocyclylylidene substituted one or more times (e.g., 1-4, 1-3, or 1-2 times) by a substituent(s) optionally selected from the group consisting of deuterium, =O, halogen, hydroxy, $C_1$-$C_6$ alkyl, halogenated $C_1$-$C_6$ alkyl, $C_1$-$C_6$ alkoxy, or any combination thereof.

As used herein, the term "heteroarylylidene", used alone or in combination, refers to a 7- to 30-membered (e.g., 7- to 30-membered, 7- to 20-membered, 7- to 15-membered, 7- to 12-membered, 7- to 11-membered, 7- to 10-membered, 8- to 30-membered, 8- to 20-membered, 8- to 15-membered, 8- to 12-membered, 8- to 11-membered, 8- to 10-membered, 9- to 30-membered, 9- to 20-membered, 9- to 15-membered, 10- to 30-membered, 10- to 20-membered, 10- to 15-membered, 11- to 30-membered, 11- to 20-membered, 11- to 15-membered, 12- to 30-membered, 12- to 20-membered, 15- to 30-membered, or 15- to 20-membered) trivalent bicyclic or polycyclic cyclic hydrocarbon radical containing at least one aromatic ring having one or more (e.g., from 1 to 6, or from 1 to 5, or from 1 to 4, or from 1 to 3) heteroatoms independently selected from the group consisting of oxygen, nitrogen, and sulfur. Bicyclic or polycyclic heteroarylylidene include bicyclic, tricyclic, tetracyclic or polycyclic heteroarylylidene, which contain one aromatic ring having one or more heteroatoms independently selected from O, S and N, and the remaining ring(s) which may be a saturated or partially unsaturated and can be carbocyclic ring or contain one or more heteroatoms independently selected from O, S and N. Representative examples of the heteroarylylidene include, but not limited to, chromanylylidene (e.g.,

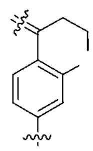

)

or 2,3-dihydroquinolin-4(1H)-ylylidene (e.g.,

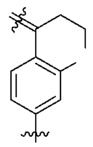

).

The "heteroarylylidene" may be unsubstituted or substituted. A substituted heteroarylylidene refers to a heteroarylylidene substituted one or more times (e.g., 1-4, 1-3, or 1-2 times) by a substituent(s) optionally selected from the group consisting of deuterium, = O, halogen, hydroxy, cyano, amino, mercapto, $C_1$-$C_6$ alkyl, halogenated $C_1$-$C_6$ alkyl, $C_1$-$C_6$ alkoxy, halogenated $C_1$-$C_6$ alkoxy or any combination thereof.

As used herein, "bornylane" or "bornane" (also known as 1,7,7-trimethylbicyclo[2.2.1]heptane; camphane; bornylane) has a definition known to those skilled in the art. As used herein, "camphanyl" or "bornyl" refers to a monovalent group of bornane, i.e., the group remaining after any one of the hydrogens in bornane is removed. Representative examples of "bornyl" include, but are not limited to, 1,7,7-trimethylbicyclo[2.2.1]heptan-2-yl, 1,7,7-trimethylbicyclo[2.2.1]heptan-3-yl, 1,7,7-trimethylbicyclo[2.2.1]heptan-4-yl, 1,7,7-trimethylbicyclo[2.2.1]heptan-5-yl, 1,7,7-trimethylbicyclo[2.2.1]heptan-6-yl,

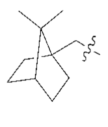

, or

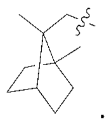

.

As used herein, "bicyclo[2.2.1]heptane" also known as "norbornane", has a definition known to those skilled in the art. As used herein, "bicyclo[2.2.1]heptyl" or "norbornyl" refers to a monovalent group of bicyclo[2.2.1]heptane, i.e., the group remaining after any one hydrogen in bicyclo[2.2.1]heptane is removed. Representative examples of "bicyclo[2.2.1]heptyl" include, but are not limited to, bicyclo[2.2.1]heptan-1-yl, bicyclo[2.2.1]heptan-2-yl, bicyclo[2.2.1]heptan-3-yl, bicyclo[2.2.1]heptan-4-yl, bicyclo[2.2.1]heptan-5-yl, or bicyclo[2.2.1]heptan-6-yl.

As used herein, the term "adamantane" has a definition known to those skilled in the art, and its structural formula is e.g., as follows:

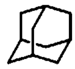.

As used herein, "adamantanyl" refers to a monovalent group of adamantane, that is, the group remaining after any one hydrogen in adamantane is removed. Representative examples of "adamantanyl" include, but are not limited to, 1-adamantanyl, 2-adamantanyl, 3-adamantanyl, 4-adamantanyl, 5-adamantanyl, 6-adamantanyl, 7-adamantanyl, 8-adamantanyl, 9-adamantanyl, or 10-adamantanyl.

As used herein, the term "noradamantane" (also known as octahydro-2,5-methanopentalene) has a definition known to those skilled in the art, and its structural formula is e.g., as follows:

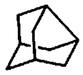 or 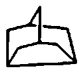.

As used herein, "noradamantanyl" refers to a monovalent group of noradamantane, that is, the group remaining after any one hydrogen in noradamantane is removed. Representative examples of "noradamantanyl" include, but are not limited to, 1-noradamantanyl, 2-noradamantanyl, 3-noradamantanyl, 4-noradamantanyl, 5-noradamantanyl, 6-noradamantanyl, 7-noradamantanyl, 8-noradamantanyl or 9-noradamantanyl.

In all embodiments of the present disclosure, the salts or pharmaceutically acceptable salts of the compounds of Formula (I) refer to non-toxic inorganic acid and/or base addition salts. Examples include: sulfate, hydrohalides (including hydrochloride and hydrobromide), maleate, sulfonate, citrate, lactate, lactobionate, L-tartrate, fumarate, L-malate, L-lactate, α-ketoglutarate, hippurate, D-glucuronate, D-gluconate, α-D-glucoheptonate, glycolate, mucate, L-ascorbate, orotate, picrate, glycinate, alaninate, arginate, cinnamate, laurate, pamoate, sebacate, besylate, methanesulfonate, ethanesulfonate, edisylate, formate, acetate, 2,2-dichloroacetate, trimethylacetate, propionate, valerate, palmitate, triphenylacetate, 2-ethyl-butanedioate, iodate, nicotinate, L-pyroglutamate, L-prolinate, ferulate, 2-hydroxyethanesulfonate, nitrate, gentisate, cholate, salicylate, terephthalate, glutarate, adipate, stearate, oleate, undecenoate, camphorate, camphorsulfonate, dodecylsulfonate, phosphate, thiocyanate, dihydrophosphate, pyrophosphate, metaphosphate, oxalate, carbonate, malonate, benzoate, mandelate, succinate, pyruvate, p-chlorobenzenesulfonate, 1,5-naphthalenedisulfonate, 3-hydroxy-2-naphthoate, 1-hydroxy-2-naphthoate, 2-naphthalenesulfonate, glycolate, and tosylate etc.

As used herein, the term "pharmaceutically acceptable carrier" refers to a pharmaceutically acceptable material, such as a filler, stabilizer, dispersant, suspending agent, diluent, excipient, thickener, solvent, or encapsulating material, with which the useful compounds according to the present disclosure are carried or transported into or administered to a patient so that they can perform their intended function. Generally, such constructs are carried or transported from one organ or part of the body to another organ or part of the body. The carrier is compatible with the other ingredients of the formulation, including the compounds useful in the present disclosure, and is not harmful to the patient, and the carrier must be "acceptable". Some examples of materials that can be used as pharmaceutically acceptable carriers include sugars such as lactose, glucose, and sucrose; starches such as corn starch and potato starch; cellulose and its derivatives such as sodium carboxymethyl cellulose, ethyl cellulose and cellulose acetate; powdered tragacanth; malt; gelatin; talc; excipients such as cocoa butter and suppository wax; oils such as peanut oil, cottonseed oil, safflower oil, sesame oil, olive oil, corn oil and soybean oil; glycols such as propylene glycol; polyols such as glycerol, sorbitol, mannitol, and polyethylene glycol; esters such as ethyl oleate and ethyl laurate; agar; buffers such as magnesium hydroxide and aluminum hydroxide; surfactant phosphate buffer solution; and other common non-toxic compatible substances used in pharmaceutical formulations.

As used herein, the term "room temperature" refers to the ambient temperature, such as 20-30°C.

The compounds of the present disclosure exhibit strong binding affinity to CRBN, can significantly inhibit the proliferation of tumor cells, and their inhibitory effect is far superior to that of the positive control drugs lenalidomide and pomalidomide. In Western blot assay, the compounds of the present disclosure demonstrate the ability to degrade substrate proteins (e.g., IKZF 1/2/3/4 proteins, WEE1 protein, CK1α protein, GSPT1 protein, ZFP91 protein, etc.). The results of pharmacokinetic tests indicate that the novel compounds of the present disclosure, administered via the oral route, possess favorable pharmacokinetic (DMPK) properties and can be used as therapeutic agents for cancer patients.

## Description of the Drawings

Fig. 1 shows the Western blot assay results of the degradation of target substrate proteins by the compounds of the present disclosure in cells.

## Examples

To better illustrate the present application and facilitate the understanding of its technical solutions, further detailed descriptions are provided below. It should be clarified that the described embodiments represent only a part of the embodiments of the present invention, rather than all embodiments. Based on the embodiments of the present invention, all other embodiments obtained by those of ordinary skill in the art without creative effort shall fall within the scope of protection of the present invention.

The terminology used in the embodiments of the present invention is for the purpose of describing specific embodiments only and is not intended to limit the present invention. As used in the embodiments of the present invention and the appended claims, the singular forms "a," "the," and "said" are also intended to include the plural forms, unless the context clearly indicates otherwise.

The following abbreviations are used throughout the specification and examples:

| | |
|---|---|
| DIEA | N, N-diisopropylethylamine |
| DMF | N,N-dimethylformamide |
| Eq | equivalent |
| OMs | Methanesulfonyloxy |
| OTs | Tosyloxy |
| ONs | p-Nitrobenzenesulfonyl |
| $^1$H NMR | Proton nuclear magnetic resonance |

## Synthesis methods

Compounds and/or salts thereof of the present disclosure can be synthesized using commercially available raw materials by synthetic techniques known in the art. The synthetic schemes described below illustrate the preparation of most compounds. The starting materials or reagents used in each scheme can be purchased from commercial sources or prepared by methods known to those skilled in the art. One skilled in the art can prepare the salts, enantiomers, stereoisomers, solvates, isotopically enriched analogs, prodrugs, or polymorphs forms of the compounds of Formula (I) of the present disclosure according to common techniques in the art.

Scheme 1

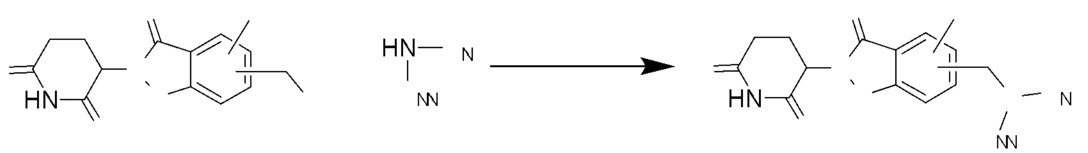

In Scheme 1, group X in substrate 1 represents $CH_2$ or C(=O); Y represents fluorine or hydrogen; and group LE represents Cl, Br, I, OMs, OTs, or ONs. Group $R_{b1}$ in substrate 2 represents hydrogen, and group $R_{b2}$ represents optionally substituted heterocyclyl or cycloalkyl; alternatively, groups $R_{b1}$ and $R_{b2}$, together with the nitrogen atom to which they are attached, form an optionally substituted nitrogen-containing heterocycle.

The aminoalkylation reaction in Scheme 1 can be performed under conventional conditions well-known to those skilled in the art. For example, the aminoalkylation may be conducted in the presence of DIEA and sodium iodide, or triethylamine and sodium iodide, at a temperature ranging from room temperature to 80°C (e.g., 40°C-60°C, 40°C-50°C, or 50°C-60°C). The molar ratio of substrate 1 to substrate 2 can be, for example, 1.1-2:1, 1.1-1.5:1, 1:1.1-2, 1:1.1-1.5, 1:1.1-1.2, or 1:1.2-1.3, etc. As an example, mesylate substrate 1 (1.1 eq.) and substrate 2 (1.0 eq.) are dissolved in 3 mL of anhydrous DMF, followed by the addition of triethylamine (3.0 eq.) and sodium iodide (1.0 eq.). The reaction mixture is stirred at room temperature for 18 hours. Upon completion as monitored by LCMS, the mixture is filtered, and the filtrate is purified by high-performance liquid chromatography to afford the target compound.

Depending upon the target compounds, the various schemes mentioned above and their reaction substrates,

reaction conditions (including reaction dosage, temperature, duration, etc.), work up, etc. can be appropriately modified and adjusted by techniques and methods well known to those skilled in the art to obtain the desired target compounds. The obtained target compounds can be further modified by the substituents and the like to obtain subsequent target compounds through methods well known to those skilled in the art.

Examples

Example 1: Preparation of 3-(1-oxo-5-((4-(thiophen-2-yl)piperazin-1-yl)methyl)isoindolin-2-yl)piperidine-2,6-dione (GT-04545)

Referring to the method of Scheme 1, the target compound (GT-04545) was prepared under appropriate conditions as would be understood by those skilled in the art (GT-04545) (white solid, 57 mg, yield 51%). $^1$H NMR (400 MHz, DMSO) δ 11.01 (s, 1H), 9.64 (s, 1H), 7.91 (s, 1H), 7.87 - 7.75 (m, 4H), 6.13 (t, J = 3.8 Hz, 1H), 5.14 (dd, J = 13.3, 5.0 Hz, 1H), 4.66 - 4.34 (m, 4H), 3.45 - 3.39 (m, 5H), 3.27 (brs, 2H), 2.99 - 2.81 (m, 2H), 2.61 (d, J = 16.8 Hz, 1H), 2.43 (dd, J = 12.9, 4.3 Hz, 1H), 2.06 - 1.96 (m, 1H). LCMS (ESI) calcd for $C_{22}H_{25}N_4O_3S^+$ [M+H]$^+$: 425.16, found, 425.2.

Example 2: Preparation of 3-(5-((4-(5-methylthiophen-2-yl)piperazin-1-yl)methyl)-1-oxoisoindolin-2-yl)piperidine-2,6-dione (GT-04546)

Referring to the method of Scheme 1, the target compound (GT-04546) was prepared under appropriate conditions as would be understood by those skilled in the art (white solid, 43 mg, yield 40%). $^1$H NMR (400 MHz, DMSO) δ 11.01 (s, 1H), 9.65 (s, 1H), 7.91 (s, 1H), 7.87 - 7.74 (m, 3H), 5.14 (dd, *J* = 13.3, 5.1 Hz, 1H), 4.53 - 4.36 (m, 4H), 3.39 - 3.27 (m, 5H), 3.28 - 3.04 (m, 2H), 2.93 - 2.88(m, 1H), 2.84 - 2.72 (m, 1H), 2.61 (d, *J* = 16.6 Hz, 1H), 2.50 (s, 3H), 2.44 (dd, J= 13.1, 4.5 Hz, 1H), 2.05 - 2.01(m, 1H). LCMS (ESI) calcd for $C_{23}H_{27}N_4O_3S^+$ [M+H]$^+$: 439.18, found, 439.2.

Example 3: Preparation of 3-(5-((4-(3-methylthiophen-2-yl)piperazin-1-yl)methyl)-1-oxoisoindolin-2-yl)piperidine-2,6-dione (GT-04582)

Referring to the method of Scheme 1, the target compound (GT-04582) was prepared under appropriate conditions as would be understood by those skilled in the art (white solid, 39 mg, yield 36%). $^1$H NMR (400 MHz, MeOD) δ 7.97 (d, J = 7.8 Hz, 1H), 7.82 (s, 1H), 7.75 (d, J = 7.8 Hz, 1H), 7.02 (d, J = 5.6 Hz, 1H), 6.77 (d, J = 5.6 Hz, 1H), 5.21 (dd, J = 13.3, 5.2 Hz, 1H), 4.70 - 4.52 (m, 4H), 3.56 (brs, 2H), 3.50 - 3.45 (m, 2H), 3.32 - 3.20 (m, 2H), 3.03 - 2.96 (m, 2H), 3.00 - 2.88 (m, 1H), 2.84 - 2.80 (m, 1H), 2.60 - 2.49 (m, 1H), 2.30 - 2.19 (m, 1H), 2.19 (s, 3H). LCMS (ESI) calcd for $C_{23}H_{27}N_4O_3S^+$ [M+H]$^+$: 439.18, found, 439.2.

Example 4: Preparation of 3-(1-oxo-5-((4-(thiophen-3-yl)piperazin-1-yl)methyl)isoindolin-2-yl)piperidine-2,6-dione (GT-04547)

Referring to the method of Scheme 1, the target compound (GT-04547) was prepared under appropriate conditions as would be understood by those skilled in the art (white solid, 39 mg, yield 35%). $^1$H NMR (400 MHz, DMSO) δ 11.39 (s, 1H), 11.01 (s, 1H), 9.71 (s, 1H), 7.92 (s, 1H), 7.87 - 7.75 (m, 2H), 7.50 - 7.38 (m, 1H), 7.00 - 6.96 (m, 1H), 5.14 (dd, *J* = 13.4, 4.8 Hz, 1H), 4.65 - 4.32 (m, 4H), 3.48 - 3.42 (m, 3H), 3.37 (d, *J* = 10.3 Hz, 2H), 3.29 - 3.08 (m, 3H), 3.00 - 2.84 (m, 1H), 2.61 (d, *J* = 17.5 Hz, 1H), 2.43 (dd, *J*= 13.1, 4.4 Hz, 1H), 2.08 - 1.97 (m, 1H). LCMS (ESI) calcd for $C_{22}H_{25}N_4O_3S^+$ [M+H]$^+$: 425.16, found, 425.2.

Example 5: Preparation of 3-(5-((4-(4-methylthiophen-3-yl)piperazin-1-yl)methyl)-1-oxoisoindolin-2-yl)piperidine-2,6-dione (GT-04548)

Referring to the method of Scheme 1, the target compound (GT-04548) was prepared under appropriate conditions as would be understood by those skilled in the art (white solid, 77 mg, yield 71%). $^1$H NMR (400 MHz, DMSO) δ 11.21 (s, 1H), 11.01 (s, 1H), 7.91 (s, 1H), 7.82 (q, *J* = 7.8 Hz, 2H), 7.16 - 7.09 (m, 1H), 6.80 (d, *J* = 3.2 Hz, 1H), 5.15 (dd, *J* = 13.3, 5.1 Hz, 1H), 4.54 - 4.37 (m, 4H), 3.38 (d, *J* = 11.7 Hz, 2H), 3.29 (d, *J* = 13.6 Hz, 2H), 3.23 (d, *J* = 10.8 Hz, 2H), 3.05 - 2.97 (m, 2H), 2.95 - 2.86 (m, 1H), 2.61 (d, *J*= *16.6* Hz, 1H), 2.43 (dd, *J* = 13.2, 4.4 Hz, 1H), 2.10 (s, 3H), 2.06 - 1.95 (m, 1H). LCMS (ESI) calcd for $C_{23}H_{27}N_4O_3S^+$ [M+H]$^+$: 439.18, found, 439.2.

Example 6: Preparation of 3-(5-((4-(5-methylthiophen-3-yl)piperazin-1-yl)methyl)-1-oxoisoindolin-2-yl)piperidine-2,6-dione (GT-04549)

Referring to the method of Scheme 1, the target compound (GT-04549) was prepared under appropriate conditions as would be understood by those skilled in the art (white solid, 73 mg, yield 67%). $^{1}$H NMR (400 MHz, DMSO) δ 11.00 (s, 1H), 7.91 (d, *J* = 7.3 Hz, 1H), 7.87 - 7.73 (m, 2H), 6.68 (s, 1H), 6.19 (s, 1H), 5.14 (dd, *J* = 13.2, 5.1 Hz, 1H), 4.59 - 4.33 (m, 3H), 3.61 - 3.59 (m, 2H), 3.47 - 3.42 (m, 2H), 3.35 (d, *J* = 10.3 Hz, 2H), 3.17 (s, 3H), 3.11 - 3.08 (m, 2H), 2.94 - 2.90 (m, 1H), 2.61 (d, *J* = 16.8 Hz, 1H), 2.46 - 2.41 (m, 1H), 2.38 - 2.32 (m, 1H), 2.06 - 1.95 (m, 1H). LCMS (ESI) calcd for $C_{23}H_{27}N_4O_3S^+$ [M+H]$^+$: 439.18, found, 439.2.

Example 7: Preparation of 3-(1-oxo-5-((4-(thiophen-2-yl)-3,6-dihydropyridin-1(2H)-yl)methyl)isoindolin-2-yl)piperidine-2,6-dione (GT-04494)

Referring to the method of Scheme 1, the target compound (GT-04494) was prepared under appropriate conditions as would be understood by those skilled in the art (white solid, 26 mg, yield 23%). $^{1}$H NMR (400 MHz, DMSO) δ 11.01 (s, 1H), 10.58 (s, 1H), 7.85 (d, *J* = 7.2 Hz, 2H), 7.74 (d, *J* = 7.7 Hz, 1H), 7.49 (d, *J*= 4.8 Hz, 1H), 7.17 (s, 1H), 7.10 - 7.01 (m, 1H), 6.06 (s, 1H), 5.15 (dd, *J*= 13.2, 5.0 Hz, 1H), 4.56 - 4.37 (m, 4H), 3.88 - 3.68 (m, 2H), 3.65 - 3.61 (m, 1H), 3.27 - 3.22 (m, 1H), 2.96 - 2.88 (m, 1H), 2.82 (brs, 2H), 2.67 - 2.59 (m, 1H), 2.43 (d, *J= 8.7* Hz, 1H), 2.10 - 1.94 (m, 1H). LCMS (ESI) calcd for $C_{23}H_{24}N_3O_3S^+$ [M+H]$^+$: 422.15, found, 422.2.

Example 8: Preparation of 3-(5-((4-(5-methylthiophen-2-yl)-3,6-dihydropyridin-1(2H)-yl)methyl)-1-oxoisoindolin-2-yl)piperidine-2,6-dione (GT-04572)

Referring to the method of Scheme 1, the target compound (GT-04572) was prepared under appropriate conditions as would be understood by those skilled in the art (white solid, 53 mg, yield 48%). $^{1}$H NMR (400 MHz, DMSO) δ 11.00 (s, 1H), 10.82 (s, 1H), 7.93 - 7.81 (m, 2H), 7.76 *(d, J=* 7.3 Hz, 1H), 6.94 (s, 1H), 6.74 (s, 1H), 5.90 (s, 1H), 5.14 (dd, *J* = 13.2, 4.0 Hz, 1H), 4.54 - 4.36 (m, 4H), 3.82 - 3.69 (m, 2H), 3.63 - 3.46 (m, 1H), 3.46 - 3.43 (m, 2H), 2.82 - 2.71 (m, 2H), 2.61 (d, *J* = 16.4 Hz, 2H), 2.41 (s, sH), 2.07 - 1.99 (m, 1H). LCMS (ESI) calcd for $C_{24}H_{26}N_3O_3S^+$ [M+H]$^+$: 436.17, found, 436.2.

Example 9: Preparation of 3-(5-((4-(3-methylthiophen-2-yl)-3,6-dihydropyridin-1(2H)-yl)methyl)-1-oxoisoindolin-2-yl)piperidine-2,6-dione (GT-04495)

Referring to the method of Scheme 1, the target compound (GT-04495) was prepared under appropriate conditions as would be understood by those skilled in the art (white solid, 25 mg, yield 23%). $^{1}$H NMR (400 MHz, DMSO) δ 11.01 (s, 1H), 10.67 (s, 1H), 7.85 (d, *J* = 6.4 Hz, 2H), 7.75 (d, *J* = 8.0 Hz, 1H), 7.39 (d, *J* = 5.1 Hz, 1H), 6.91 (d, *J* = 5.1 Hz, 1H), 5.84 (s, 1H), 5.15 (dd, *J* = 13.2, 5.0 Hz, 1H), 4.54 - 4.37(m, 4H), 3.92 - 3.69 (m, 2H), 3.64 - 3.59 (m, 1H), 3.02 - 2.73 (m, 2H), 2.72 - 2.55 (m, 2H), 2.45 - 2.39 (m, 2H), 2.22 (s, 3H), 2.05 - 2.01(m, 1H). LCMS (ESI) calcd for $C_{24}H_{26}N_3O_3S^+$ [M+H]$^+$: 436.17, found, 436.3.

Example 10: Preparation of 3-(1-oxo-5-((4-(thiophen-3-yl)-3,6-dihydropyridin-1(2H)-yl)methyl)isoindolin-2-yl)piperidine-2,6-dione (GT-04585)

Referring to the method of Scheme 1, the target compound (GT-04585) was prepared under appropriate conditions as would be understood by those skilled in the art (white solid, 55 mg, yield 48%). $^{1}$H NMR (400 MHz, MeOD) δ 7.97 (d, J = 7.8 Hz, $^{1}$H), 7.82 (s, 1H), 7.75 (d, J = 7.8 Hz, 1H), 7.02 (d, J = 5.6 Hz, 1H), 6.77 (d, J = 5.6 Hz, 1H), 5.21 (dd, J = 13.3, 5.2 Hz, 1H), 4.70 - 4.52 (m, 4H), 3.56 (brs, 2H), 3.50 - 3.45 (m, 2H), 3.32 - 3.20 (m, 2H), 3.03 - 2.96 (m, 2H), 3.00 - 2.88 (m, 1H), 2.84 - 2.80 (m, 1H), 2.60 - 2.49 (m, 1H), 2.30 - 2.19 (m, 1H), 2.19 (s, 3H). LCMS (ESI) calcd for $C_{23}H_{24}N_3O_3S^+$ [M+H]$^+$: 422.15, found, 422.2.

Example 11: Preparation of 3-(5-((4-(4-methylthiophen-3-yl)-3,6-dihydropyridin-1(2H)-yl)methyl)-1-oxoisoindolin-2-yl) piperidine-2,6-dione (GT-04503)

Referring to the method of Scheme 1, the target compound (GT-04503) was prepared under appropriate conditions as would be understood by those skilled in the art (white solid, 20 mg, yield 18%). $^{1}$H NMR (400 MHz, DMSO) δ 11.01 (s, 1H), 10.77 (s, 1H), 7.91 - 7.82 (m, 2H), 7.77 (d, *J* = 7.7 Hz, 1H), 7.42 (d, *J* = 3.0 Hz, 1H), 7.20 (d, *J* = 2.9 Hz, 1H), 5.79 (s, 1H), 5.15 (dd, *J* = 13.3, 5.0 Hz, 1H), 4.54 - 4.50 (m, 3H), 4.39 (d, *J* = 17.4 Hz, 1H), 3.87 - 3.66 (m, 2H), 3.63 - 3.59 (m, 1H), 3.28 - 3.19 (m, 1H), 2.94 - 2.88 (m, 2H), 2.62 (d, *J* = 16.8 Hz, 2H), 2.46 - 2.37 (m, 1H), 2.23 (s, 3H), 2.09 - 1.94 (m, 1H). LCMS (ESI) calcd for $C_{24}H_{26}N_3O_3S^+$ [M+H]$^+$: 436.17, found, 436.2.

Example 12: Preparation of 3-(5-((4-(5-methylthiophen-3-yl)-3,6-dihydropyridin-1(2H)-yl)methyl)-1-oxoisoindolin-2-yl) piperidine-2,6-dione (GT-04587)

Referring to the method of Scheme 1, the target compound (GT-04587) was prepared under appropriate conditions as would be understood by those skilled in the art (white solid, 66 mg, yield 60%). $^1$H NMR (400 MHz, MeOD) δ 8.34 (d, *J* = 5.2 Hz, 1H), 7.94 (dd, *J* = 17.5, 7.5 Hz, 1H), 7.84 - 7.51 (m, 2H), 7.20 - 7.01 (m, 1H), 6.00 (d, *J* = 76.8 Hz, 1H), 5.22 (d, *J* = 7.9 Hz, 1H), 4.77 - 4.36 (m, 4H), 3.87 (d, *J* = 31.1 Hz, 2H), 3.53 - 3.35 (m, 2H), 3.00 - 2.70 (m, 4H), 2.63 - 2.44 (m, 4H), 2.21 (s, 1H). LCMS (ESI) calcd for $C_{24}H_{26}N_3O_3S^+$ [M+H]$^+$: 436.17, found, 436.2.

Example 13: Preparation of 3-(5-((4-(furan-2-yl)-3,6-dihydropyridin-1(2H)-yl)methyl)-1-oxoisoindolin-2-yl)piperidine-2,6-dione (GT-04617)

Referring to the method of Scheme 1, the target compound (GT-04617) was prepared under appropriate conditions as would be understood by those skilled in the art (white solid, 44 mg, yield 46%). $^1$H NMR (400 MHz, MeOD) δ 7.95 (t, *J* = 9.2 Hz, 1H), 7.80 (d, *J* = 10.6 Hz, 1H), 7.74 (d, *J* = 8.2 Hz, 1H), 7.52 (s, 1H), 6.89 - 5.86 (m, 3H), 5.21 (dd, *J* = 13.3, 5.2 Hz, 1H), 4.67 - 4.47 (m, 4H), 3.86 (d, *J* = 63.6 Hz, 2H), 3.52 - 3.34 (m, 2H), 3.00 - 2.89 (m, 1H), 2.82 (d, *J* = 12.8 Hz, 3H), 2.62 - 2.45 (m, 1H), 2.30 - 2.15 (m, 1H). LCMS (ESI) calcd for $C_{23}H_{24}N_3O_4^+$ [M+H]$^+$: 406.18, found, 406.2.

Example 14: Preparation of 3-(5-((4-(furan-3-yl)-3,6-dihydropyridin-1(2H)-yl)methyl)-1-oxoisoindolin-2-yl)piperidine-2,6-dione (GT-04589)

Referring to the method of Scheme 1, the target compound (GT-04589) was prepared under appropriate conditions as would be understood by those skilled in the art (white solid, 67 mg, yield 56%). $^1$H NMR (400 MHz, MeOD) δ 7.96 (d, *J* = 7.8 Hz, 1H), 7.81 (s, 1H), 7.74 (d, *J* = 7.8 Hz, 1H), 7.68 (s, 1H), 7.52 (t, *J* = 1.6 Hz, 1H), 6.68 (s, 1H), 5.96 (d, *J* = 39.6 Hz, 1H), 5.21 (dd, *J* = 13.3, 5.2 Hz, 1H), 4.68 - 4.50 (m, 4H), 3.94 (d, *J* = 23.1 Hz, 2H), 3.76 (s, 1H), 3.39 (d, *J* = 18.9 Hz, 1H), 3.01 - 2.88 (m, 1H), 2.83 (dd, *J* = 11.0, 8.7 Hz, 3H), 2.54 (qd, *J* = 13.5, 4.9 Hz, 1H), 2.22 (dd, *J* = 10.1, 4.9 Hz, 1H). LCMS (ESI) calcd for $C_{23}H_{24}N_3O_4^+$ [M+H]$^+$: 406.18, found, 406.2.

Example 15: Preparation of 3-(1-oxo-5-((4-(thiophen-2-yl)piperidin-1-yl)methyl)isoindolin-2-yl)piperidine-2,6-dione (GT-04571)

Referring to the method of Scheme 1, the target compound (GT-04571) was prepared under appropriate conditions as would be understood by those skilled in the art (white solid, 51 mg, yield 45%). $^1$H NMR (400 MHz, DMSO) δ 11.02 (s, 1H), 10.80 (s, 1H), 7.88 (s, 1H), 7.83 (t, *J* = 6.5 Hz, 1H), 7.76 (d, *J* = 7.9 Hz, 1H), 7.38 (dd, *J* = 5.1, 1.2 Hz, 1H), 6.97 (dd, *J* = 5.1, 3.5 Hz, 1H), 6.90 (d, *J* = 3.4 Hz, 1H), 5.15 (dd, *J* = 13.3, 5.1 Hz, 1H), 4.65 - 4.39 (m, 4H), 3.49 - 3.39 (m, 2H), 3.11 - 3.05 (m, 3H), 2.94 - 2.89 (m, 1H), 2.61 (d, *J* = 17.6 Hz, 1H), 2.43 (dd, *J*= 13.0, 4.4 Hz, 1H), 2.17 - 1.97 (m, 5H). LCMS (ESI) calcd for $C_{23}H_{26}N_3O_3S^+$ [M+H]$^+$: 424.17, found, 424.2.

Example 16: Preparation of 3-(5-((4-(5-methylthiophen-2-yl)piperidin-1-yl)methyl)-1-oxoisoindolin-2-yl)piperidine-2,6-dione (GT-04573)

Referring to the method of Scheme 1, the target compound (GT-04573) was prepared under appropriate conditions as would be understood by those skilled in the art (white solid, 59 mg, yield 54%). $^1$H NMR (400 MHz, MeOD) δ 7.83 (d, J = 7.8 Hz, 1H), 7.68 (s, 1H), 7.60 (d, J = 7.8 Hz, 1H), 6.56 (s, 1H), 6.49 (s, 1H), 5.09 (dd, J = 13.3, 5.1 Hz, 1H), 4.48 (q, J = 17.4 Hz, 2H), 4.38 (s, 2H), 3.49 (d, J = 11.3 Hz, 2H), 3.16 - 2.95 (m, 3H), 2.92 - 2.75 (m, 1H), 2.75 - 2.64 (m, 1H), 2.42 (qd, J = 13.2, 4.6 Hz, 1H), 2.31 (s, 3H), 2.24 - 2.02 (m, 3H), 1.91-1.69 (m, 2H). LCMS (ESI) calcd for C24H28N3O3S+ [M+H]+: 438.18, found, 438.2.

Example 17: Preparation of 3-(5-((4-(3-methylthiophen-2-yl)piperidin-1-yl)methyl)-1-oxoisoindolin-2-yl)piperidine-2,6-dione (GT-04502)

Referring to the method of Scheme 1, the target compound (GT-04502) was prepared under appropriate conditions as would be understood by those skilled in the art (white solid, 31 mg, yield 28%). $^1$H NMR (400 MHz, DMSO) δ 11.01 (s, 1H), 10.42 (s, 1H), 7.85 (d, *J* = 7.3 Hz, 2H), 7.73 (d, *J* = 8.1 Hz, 1H), 7.26 (d, *J* = 5.1 Hz, 1H), 6.82 (d, *J* = 5.0 Hz, 1H), 5.15 (dd, *J* = 13.2, 5.1 Hz, 1H), 4.54 - 4.37 (m, 4H), 3.44 (d, *J* = 11.4 Hz, 2H), 3.20 - 3.06 (m, 3H), 3.02 - 2.85 (m, 1H), 2.61 (d, *J* = 18.0 Hz, 1H), 2.43 (dd, *J* = 13.0, 4.3 Hz, 1H), 2.14 (s, 3H), 2.08 - 1.87 (m, 5H). LCMS (ESI) calcd for $C_{24}H_{28}N_3O_3S^+$ [M+H]$^+$: 438.18, found, 438.2.

Example 18: Preparation of 3-(1-oxo-5-((4-(thiophen-3-yl)piperidin-1-yl)methyl)isoindolin-2-yl)piperidine-2,6-dione (GT-04586)

Referring to the method of Scheme 1, the target compound (GT-04586) was prepared under appropriate conditions as would be understood by those skilled in the art (white solid, 62 mg, yield 55%). $^1$H NMR (400 MHz, MeOD) δ 7.96 (d, J = 7.8 Hz, 1H), 7.82 (s, 1H), 7.74 (d, J = 7.8 Hz, 1H), 7.40 (dd, J = 5.0, 2.9 Hz, 1H), 7.17 (d, J = 2.6 Hz, 1H), 7.07 (dd, J = 5.0, 1.1 Hz, 1H), 5.21 (dd, J = 13.4, 5.2 Hz, 1H), 4.69 - 4.55 (m, 2H), 4.52 (s, 2H), 3.63 (d, J = 12.6 Hz, 2H), 3.26 - 3.20 (m, 2H), 3.11 - 2.98 (m, 1H), 2.98 - 2.87 (m, 1H), 2.86 - 2.77 (m, 1H), 2.60 - 2.49 (m, 1H), 2.32 - 2.16 (m, 3H), 1.99 - 1.88 (m, 2H). LCMS (ESI) calcd for $C_{23}H_{26}N_3O_3S^+$ [M+H]$^+$: 424.17, found, 424.2.

Example 19: Preparation of 3-(5-((4-(5-methylthiophen-3-yl)piperidin-1-yl)methyl)-1-oxoisoindolin-2-yl)piperidine-2,6-dione (GT-04588)

Referring to the method of Scheme 1, the target compound (GT-04588) was prepared under appropriate conditions as would be understood by those skilled in the art (white solid, 52 mg, yield 48%). $^1$H NMR (400 MHz, MeOD) δ 7.95 (d, *J* = 7.8 Hz, 1H), 7.81 (s, 1H), 7.73 (d, *J* = 7.8 Hz, 1H), 6.87 (s, 1H), 6.73 (s, 1H), 5.21 (dd, *J* = 13.3, 5.2 Hz, 1H), 4.69 - 4.46 (m, 4H), 3.54 (dd, *J* = 57.9, 21.8 Hz, 2H), 3.28 - 3.12 (m, 2H), 3.02 - 2.76 (m, 3H), 2.54 (ddd, *J* = 26.4, 13.2, 4.6 Hz, 1H), 2.52 - 2.40 (m, 3H), 2.28 - 2.06 (m, 3H), 1.89 (dd, *J* = 24.9, 11.7 Hz, 2H). LCMS (ESI) calcd for $C_{24}H_{28}N_3O_3S^+$ [M+H]$^+$: 438.18, found, 438.2.

Example 20: Preparation of 3-(5-((4-(4-methylthiophen-3-yl)piperidin-1-yl)methyl)-1-oxoisoindolin-2-yl)piperidine-2,6-dione (GT-04504)

Referring to the method of Scheme 1, the target compound (GT-04504) was prepared under appropriate conditions as would be understood by those skilled in the art (white solid, 27 mg, yield 25%). $^1$H NMR (400 MHz, DMSO) δ 11.01 (s, 1H), 10.81 (s, 1H), 7.89 (s, 1H), 7.83 (d, *J* = 7.7 Hz, 1H), 7.77 (d, *J*= 7.8 Hz, 1H), 7.11 (d, *J* = 4.9 Hz, 2H), 5.15 (dd, *J* = 13.2, 5.0 Hz, 1H), 4.54 - 4.37 (m, 4H), 3.50 - 3.46 (m, 2H), 3.11 - 3.05 (m, 2H), 3.00 - 2.85 (m, 1H), 2.85 - 2.72 (m, 1H), 2.61 (d, *J* = 17.2 Hz, 1H), 2.43 (dd, *J*= 13.2, 4.4 Hz, 1H), 2.17 (s, 3H), 2.08 - 1.87 (m, 5H). LCMS (ESI) calcd for $C_{24}H_{28}N_3O_3S^+$ [M+H]$^+$: 438.18, found, 438.2.

Example 21: Preparation of 3-(5-((4-(furan-2-yl)piperidin-1-yl)methyl)-1-oxoisoindolin-2-yl)piperidine-2,6-dione (GT-04618)

Referring to the method of Scheme 1, the target compound (GT-04618) was prepared under appropriate conditions as would be understood by those skilled in the art (white solid, 49 mg, yield 52%). $^1$H NMR (400 MHz, MeOD) δ 7.95 (d, *J* = 7.7 Hz, 1H), 7.80 (s, 1H), 7.72 (d, *J* = 7.9 Hz, 1H), 7.42 (s, 1H), 6.35 (s, 1H), 6.15 (s, 1H), 5.21 (dd, *J* = 13.4, 5.1 Hz, 1H), 4.66 - 4.46 (m, 4H), 3.58 (t, *J* = 27.2 Hz, 2H), 3.20 (dd, *J* = 23.8, 12.1 Hz, 2H), 3.09 - 2.87 (m, 2H), 2.87 - 2.75 (m, 1H), 2.54 (qd, *J* = 13.4, 4.7 Hz, 1H), 2.39 - 2.15 (m, 3H), 1.93 (t, *J* = 11.8 Hz, 2H). LCMS (ESI) calcd for $C_{23}H_{26}N_3O_4^+$ [M+H]$^+$: 408.19, found, 408.3.

Example 22: Preparation of 3-(5-((4-(furan-3-yl)piperidin-1-yl)methyl)-1-oxoisoindolin-2-yl)piperidine-2,6-dione (GT-04590)

Referring to the method of Scheme 1, the target compound (GT-04590) was prepared under appropriate conditions as would be understood by those skilled in the art (white solid, 59 mg, yield 50%). $^1$H NMR (400 MHz, MeOD) δ 7.94 (t, *J* = 7.4 Hz, 1H), 7.86 - 7.78 (m, 1H), 7.72 (t, *J* = 7.6 Hz, 1H), 7.48 (dd, *J* = 16.2, 14.6 Hz, 1H), 7.40 (s, 1H), 6.46 (d, *J* = 35.8 Hz, 1H), 5.20 (dt, *J* = 19.1, 9.6 Hz, 1H), 4.67 - 4.54 (m, 2H), 4.51 (s, 2H), 3.61 (d, *J* = 11.8 Hz, 2H), 3.28 - 3.13 (m, 2H), 2.98 - 2.75 (m, 3H), 2.54 (qd, *J* = 13.3, 4.7 Hz, 1H), 2.22 (dd, *J* = 10.1, 4.7 Hz, 3H), 1.85 (td, *J* = 16.5, 3.6 Hz, 2H). LCMS (ESI) calcd for $C_{23}H_{26}N_3O_4^+$ [M+H]$^+$: 408.19, found, 408.3.

Example 23: Preparation of 3-(5-((4-(2,3-dichlorophenyl)piperazin-1-yl)methyl)-1-oxoisoindolin-2-yl)piperidine-2,6-dione (GT-04201)

Referring to the method of Scheme 1, the target compound (GT-04201) was prepared under appropriate conditions as would be understood by those skilled in the art (white solid, 22 mg, yield 30%). $^1$H NMR (400 MHz, MeOD-d4) δ 7.95 (d, *J* = 7.8 Hz, 1H), 7.81 (s, 1H), 7.74 (d, *J* = 7.9 Hz, 1H), 7.32 - 7.27 (m, 2H), 7.18 - 7.11 (m, 1H), 5.19 (dd, *J* = 13.4, 5.1 Hz, 1H), 4.66 - 4.51 (m, 4H), 3.62 - 3.43 (m, 6H), 3.17 - 3.07 (m, 2H), 3.02 - 2.85 (m, 1H), 2.82 - 2.77 (m, 1H), 2.60 - 2.44 (m, 1H), 2.27 - 2.12 (m, 1H). LCMS (ESI) calcd for $C_{24}H_{25}Cl_2N_4O_3^+$ [M+H]$^+$: 487.13, found, 487.1.

Example 24: Preparation of 3-(1-oxo-5-((4-phenylpiperazin-1-yl)methyl)isoindolin-2-yl)piperidine-2,6-dione (GT-04446)

Referring to the method of Scheme 1, the target compound (GT-04446) was prepared under appropriate conditions as would be understood by those skilled in the art (white solid, 91 mg, yield 65%). $^1$H NMR (400 MHz, DMSO) δ 11.63 (s, 1H), 11.02 (s, 1H), 7.95 (s, 1H), 7.83 (s, 2H), 7.26 (dd, *J*= 8.7, 7.3 Hz, 2H), 6.98 (d, *J* = 7.9 Hz, 2H), 6.86 (t, *J* = 7.3 Hz, 1H), 5.15 (dd, *J* = 13.3, 5.1 Hz, 1H), 4.52 - 4.37 (m, 4H), 3.82- 3.78 (m, 2H), 3.39 (d, *J* = 8.9 Hz, 2H), 3.29 - 3.09 (m, 4H), 2.93 - 2.89 (m, 1H), 2.62 (d, *J* = 16.5 Hz, 1H), 2.46 - 2.38 (m, 1H), 2.05 - 2.01 (m, 1H). LCMS (ESI) calcd for $C_{24}H_{27}N_4O_3^+$ $[M+H]^+$: 419.21, found, 419.3.

Example 25: Preparation of 3-(5-((4-(2-methoxyphenyl)piperazin-1-yl)methyl)-1-oxoisoindolin-2-yl)piperidine-2,6-dione (GT-04490)

Referring to the method of Scheme 1, the target compound (GT-04490) was prepared under appropriate conditions as would be understood by those skilled in the art (white solid, 46 mg, yield 36%). $^1$H NMR (400 MHz, DMSO) δ 11.02 (s, 1H), 7.90 (s, 1H), 7.85 - 7.78 (m, 2H), 7.04 - 6.98(m, 2H), 6.95 - 6.85 (m, 2H), 5.15 (dd, *J* = 13.2, 5.2 Hz, 1H), 4.54 - 4.37 (m, 4H), 3.78 (s, 3H), 3.47 (d, *J* = 12.8 Hz, 2H), 3.39 (d, *J* = 12.8 Hz, 2H), 3.25 - 5.22 (m, 2H), 3.08 - 3.02 (m, 2H), 2.99 - 2.85 (m, 1H), 2.64 - 2.59 (m, 1H), 2.49 - 2.41 (m, 1H), 2.07 - 1.95 (m, 1H). LCMS (ESI) calcd for $C_{25}H_{29}N_4O_4^+$ $[M+H]^+$: 449.22, found, 449.3.

Example 26: Preparation of 3-(5-((4-(3-methoxyphenyl)piperazin-1-yl)methyl)-1-oxoisoindolin-2-yl)piperidine-2,6-dione (GT-04448)

Referring to the method of Scheme 1, the target compound (GT-04448) was prepared under appropriate conditions as would be understood by those skilled in the art (white solid, 105 mg, yield 83%). $^1$H NMR (400 MHz, DMSO) δ 11.16 (s, 1H), 11.02 (s, 1H), 7.90 (s, 1H), 7.85 (d, *J* = 7.8 Hz, 1H), 7.78 (d, *J* = 7.8 Hz, 1H), 7.16 (t, *J* = 8.2 Hz, 1H), 6.56 (dd, *J* = 8.2, 1.9 Hz, 1H), 6.50 (t, *J* = 2.2 Hz, 1H), 6.45 (dd, *J* = 8.0, 2.0 Hz, 1H), 5.15 (dd, *J* = 13.3, 5.1 Hz, 1H), 4.59 - 4.33 (m, 4H), 3.81 (d, *J* = 9.2 Hz, 2H), 3.72 (s, 3H), 3.41 -3.38 (m, 2H), 3.15 (d, *J* = 8.8 Hz, 4H), 3.02 - 2.85 (m, 1H), 2.62 (d, *J* = 16.7 Hz, 1H), 2.48 - 2.39 (m, 1H), 2.07 - 1.93 (m, 1H). LCMS (ESI) calcd for $C_{25}H_{29}N_4O_4^+$ $[M+H]^+$: 449.22, found, 449.3.

Example 27: Preparation of 3-(5-((4-(4-methoxyphenyl)piperazin-1-yl)methyl)-1-oxoisoindolin-2-yl)piperidine-2,6-dione (GT-04447)

Referring to the method of Scheme 1, the target compound (GT-04447) was prepared under appropriate conditions as would be understood by those skilled in the art (white solid, 81 mg, yield 64%). $^1$H NMR (400 MHz, DMSO) δ 11.13 (s, 1H), 11.02 (s, 1H), 7.91 (s, 1H), 7.85 (d, *J* = 7.8 Hz, 1H), 7.79 (d, *J* = 7.9 Hz, 1H), 6.94 (d, *J* = 9.1 Hz, 2H), 6.90 - 6.83 (m, 2H), 5.15 (dd, *J* = 13.3, 5.1 Hz, 1H), 4.59 - 4.35 (m, 4H), 3.70 (s, 3H), 3.62 - 3.60(m, 2H), 3.40 (d, *J* = 10.8 Hz, 2H), 3.23 - 3.19 (m, 2H), 3.08 (t, *J* = 11.9 Hz, 2H), 3.01 - 2.87 (m, 1H), 2.65 (dd, *J* = 23.9, 9.3 Hz, 1H), 2.48 - 2.38 (m, 1H), 2.10 - 1.94 (m, 1H). LCMS (ESI) calcd for $C_{25}H_{29}N_4O_4^+$ $[M+H]^+$: 449.22, found, 449.3.

Example 28: Preparation of 3-(1-oxo-5-((4-(4-(trifluoromethyl)phenyl)piperazin-1-yl)methyl)isoindolin-2-yl)piperidine-2,6-dione (GT-04451)

Referring to the method of Scheme 1, the target compound (GT-04451) was prepared under appropriate conditions as would be understood by those skilled in the art (white solid, 63 mg, yield 56%). $^1$H NMR (400 MHz, DMSO) δ 11.03 (s, 1H), 10.79 (s, 1H), 7.86 (d, *J* = 9.2 Hz, 2H), 7.74 (d, *J* = 7.1 Hz, 1H), 7.58 (d, *J* = 8.5 Hz, 2H), 7.13 (d, *J* = 8.8 Hz, 2H), 5.16 (dd, *J* = 13.3, 5.2 Hz, 1H), 4.55 - 4.50 (m, 2H), 4.40 (d, *J* = 17.4 Hz, 1H), 4.00 (s, 1H), 3.49 - 3.38 (m, 2H), 3.30 - 3.26 (m, 1H), 3.22 (d, *J* = 11.5 Hz, 4H), 2.93 (dd, *J* = 21.7, 9.0 Hz, 1H), 2.62 (d, *J* = 16.2 Hz, 1H), 2.40 (dd, *J* = 31.1, 17.5 Hz, 2H), 2.11 - 1.98 (m, 1H). LCMS (ESI) calcd for $C_{25}H_{26}F_3N_4O_3^+$ $[M+H]^+$: 487.20, found, 487.2.

Example 29: Preparation of 3-(5-((4-(3-fluorophenyl)piperazin-1-yl)methyl)-1-oxoisoindolin-2-yl)piperidine-2,6-dione (GT-04452)

Referring to the method of Scheme 1, the target compound (GT-04452) was prepared under appropriate conditions as would be understood by those skilled in the art (white solid, 71 mg, yield 54%). $^1$H NMR (400 MHz, DMSO) δ 11.03 (s, 1H), 10.86 (s, 1H), 7.86 (d, *J* = 7.2 Hz, 2H), 7.75 (d, *J* = 7.6 Hz, 1H), 7.27 (dd, *J* = 16.0, 7.9 Hz, 1H), 6.82 (t, *J* = 10.1 Hz, 2H), 6.64 (t, *J* = 8.3 Hz, 1H), 5.16 (dd, *J* = 13.3, 5.1 Hz, 1H), 4.63 - 4.44 (m, 3H), 4.40 (d, *J* = 17.6 Hz, 1H), 3.88 (d, *J* = 9.4 Hz, 2H), 3.16 (d, *J* = 8.9 Hz, 4H), 2.93 (dd, *J* = 21.8, 9.5 Hz, 1H), 2.64 (t, *J* = 15.9 Hz, 1H), 2.42 (dd, *J* = 13.1, 8.8 Hz, 1H), 2.04

(dd, $J$ = 16.7, 11.2 Hz, 1H). LCMS (ESI) calcd for $C_{24}H_{26}FN_4O_3^+$ [M+H]$^+$: 437.20, found, 437.2.

Example 30: Preparation of 3-(5-((4-(4-fluorophenyl)piperazin-1-yl)methyl)-1-oxoisoindolin-2-yl)piperidine-2,6-dione (GT-04453)

Referring to the method of Scheme 1, the target compound (GT-04453) was prepared under appropriate conditions as would be understood by those skilled in the art (white solid, 85 mg, yield 65%). $^1$H NMR (400 MHz, DMSO) δ 11.36 (s, 1H), 11.03 (s, 1H), 7.92 (s, 1H), 7.82 (dd, $J$ = 17.3, 7.8 Hz, 2H), 7.11 (t, $J$ = 8.9 Hz, 2H), 7.06 - 6.93 (m, 2H), 5.16 (dd, $J$ = 13.2, 5.0 Hz, 1H), 4.62 - 4.46 (m, 3H), 4.39 (d, $J$ = 17.6 Hz, 1H), 3.71 (d, $J$ = 10.5 Hz, 2H), 3.40 (d, $J$ = 9.6 Hz, 2H), 3.25 - 3.07 (m, 4H), 2.99 - 2.85 (m, 1H), 2.62 (d, $J$ = 16.9 Hz, 1H), 2.47 - 2.31 (m, 1H), 2.08 - 1.94 (m, 1H). LCMS (ESI) calcd for $C_{24}H_{26}FN_4O_3^+$ [M+H]$^+$: 437.20, found, 437.2.

Example 31: Preparation of 3-(5-((4-(2-chlorophenyl)piperazin-1-yl)methyl)-1-oxoisoindolin-2-yl)piperidine-2,6-dione (GT-04454)

Referring to the method of Scheme 1, the target compound (GT-04454) was prepared under appropriate conditions as would be understood by those skilled in the art (white solid, 48 mg, yield 39%). $^1$H NMR (400 MHz, DMSO) δ 11.03 (s, 1H), 10.69 (s, 1H), 7.86 (d, $J$ = 7.6 Hz, 2H), 7.77 (d, $J$ = 7.3 Hz, 1H), 7.46 (d, $J$ = 6.7 Hz, 1H), 7.35 (t, $J$ = 7.1 Hz, 1H), 7.21 (d, $J$ = 8.0 Hz, 1H), 7.12 (t, $J$ = 7.5 Hz, 1H), 5.16 (dd, $J$ = 13.3, 5.1 Hz, 1H), 4.53 (d, $J$ = 18.2 Hz, 3H), 4.40 (d, $J$ = 17.6 Hz, 1H), 3.43 (d, $J$ = 12.6 Hz, 4H), 3.28 (d, $J$ = 12.3 Hz, 2H), 3.12 (t, $J$ = 11.4 Hz, 2H), 2.98 - 2.87 (m, 1H), 2.62 (d, $J$ = 18.1 Hz, 1H), 2.43 (dd, $J$ = 17.7, 9.1 Hz, 1H), 2.07 - 1.98 (m, 1H). LCMS (ESI) calcd for $C_{24}H_{26}ClN_4O_3^+$ [M+H]$^+$: 453.17, found, 453.2.

Example 32: Preparation of 3-(5-((4-(4-chlorophenyl)piperazin-1-yl)methyl)-1-oxoisoindolin-2-yl)piperidine-2,6-dione (GT-04455)

Referring to the method of Scheme 1, the target compound (GT-04455) was prepared under appropriate conditions as would be understood by those skilled in the art (white solid, 64 mg, yield 51%). $^1$H NMR (400 MHz, DMSO) δ 11.20 (s, 1H), 11.03 (s, 1H), 7.92 - 7.81 (m, 2H), 7.78 (d, $J$= 7.7 Hz, 1H), 7.29 (d, $J$= *9.0* Hz, 2H), 7.00 (d, $J$= *9.1* Hz, 2H), 5.16 (dd, $J$= 13.3, 5.1 Hz, 1H), 4.66 - 4.45 (m, 3H), 4.39 (d, $J$= 17.6 Hz, 1H), 3.81 (d, $J$= *9.1* Hz, 2H), 3.39 (s, 2H), 3.16 (d, $J$ = 8.6 Hz, 4H), 3.01 - 2.85 (m, 1H), 2.64 (t, $J$ = 16.0 Hz, 1H), 2.48 - 2.34 (m, 1H), 2.07 - 1.97 (m, 1H). LCMS (ESI) calcd for $C_{24}H_{26}ClN_4O_3^+$ [M+H]$^+$: 453.17, found, 453.2.

Example 33: Preparation of 3-(5-((4-(2-bromophenyl)piperazin-1-yl)methyl)-1-oxoisoindolin-2-yl)piperidine-2,6-dione (GT-04456)

Referring to the method of Scheme 1, the target compound (GT-04456) was prepared under appropriate conditions as would be understood by those skilled in the art (white solid, 59 mg, yield 53%). $^1$H NMR (400 MHz, DMSO) δ 11.03 (s, 1H), 10.61 (s, 1H), 7.86 (d, $J$ = 6.3 Hz, 2H), 7.77 (d, $J$= 7.7 Hz, 1H), 7.64 (d, $J$ = 7.8 Hz, 1H), 7.39 (t, $J$ = 7.3 Hz, 1H), 7.22 (d, $J$ = 6.9 Hz, 1H), 7.06 (t, $J$ = 7.6 Hz, 1H), 5.16 (dd, $J$ = 13.4, 5.1 Hz, 1H), 4.71 - 4.46 (m, 3H), 4.40 (d, $J$ = 17.8 Hz, 1H), 3.50 - 3.38 (m, 4H), 3.28 (s, 3H), 3.11 (t, $J$ = 11.8 Hz, 2H), 2.99 - 2.89 (m, 1H), 2.68 - 2.60 (m, 1H), 2.11 - 1.95 (m, 1H). LCMS (ESI) calcd for $C_{24}H_{26}BrN_4O_3^+$ [M+H]$^+$: 497.12, found, 497.1.

Example 34: Preparation of 3-(5-((4-(3-bromophenyl)piperazin-1-yl)methyl)-1-oxoisoindolin-2-yl)piperidine-2,6-dione (GT-04467)

Referring to the method of Scheme 1, the target compound (GT-04467) was prepared under appropriate conditions as would be understood by those skilled in the art (white solid, 58 mg, yield 52%). $^1$H NMR (400 MHz, DMSO) δ 11.05 (s, 1H), 11.01 (s, 1H), 7.96 - 7.79 (m, 2H), 7.76 (d, $J$ = 7.8 Hz, 1H), 7.19 (dd, $J$ = 17.0, 8.9 Hz, 2H), 6.98 (dd, $J$ = 14.5, 5.7 Hz, 2H), 5.15 (dd, $J$ = 13.3, 5.1 Hz, 1H), 4.69 - 4.45 (m, 3H), 4.39 (d, $J$ = 17.6 Hz, 1H), 3.87 (d, $J$ = 9.4 Hz, 2H), 3.38 (d, $J$ = 9.0 Hz, 2H), 3.18 (d, $J$ = 9.6 Hz, 4H), 2.97 - 2.85 (m, 1H), 2.63 (t, $J$ = 15.4 Hz, 1H), 2.46 - 2.36 (m, 1H), 2.06 - 1.97 (m, 1H). LCMS (ESI) calcd for $C_{24}H_{26}BrN_4O_3^+$ [M+H]$^+$: 497.12, found, 497.1.

Example 35: Preparation of 3-(5-((4-(3-bromophenyl)piperazin-1-yl)methyl)-1-oxoisoindolin-2-yl)piperidine-2,6-dione (GT-04485)

Referring to the method of Scheme 1, the target compound (GT-04485) was prepared under appropriate conditions as would be understood by those skilled in the art (white solid, 59 mg, yield 53%). $^1$H NMR (400 MHz, DMSO) δ 11.39 (s, 1H), 11.01 (s, 1H), 7.91 (s, 1H), 7.81 (dd, $J$ = 19.6, 7.8 Hz, 2H), 7.40 (d, $J$ = 8.9 Hz, 2H), 6.94 (d, $J$ = 9.0 Hz, 2H), 5.15

(dd, $J$ = 13.2, 5.0 Hz, 1H), 4.45 (dd, $J$ = 52.4, 17.4 Hz, 4H), 3.80 (d, $J$ = 10.3 Hz, 2H), 3.38 (d, $J$ = 8.1 Hz, 2H), 3.25 - 3.09 (m, 4H), 3.01 - 2.84 (m, 1H), 2.61 (d, $J$ = 16.7 Hz, 1H), 2.43 (dd, $J$ = 13.1, 4.4 Hz, 1H), 2.08 - 1.93 (m, 1H). LCMS (ESI) calcd for $C_{24}H_{26}BrN_4O_3^+$ [M+H]$^+$: 497.12, found, 497.1.

Example 36: Preparation of 3-(1-oxo-5-((4-(o-tolyl)piperazin-1-yl)methyl)isoindolin-2-yl)piperidine-2,6-dione (GT-04461)

Referring to the method of Scheme 1, the target compound (GT-04461) was prepared under appropriate conditions as would be understood by those skilled in the art (white solid, 77 mg, yield 58%). $^1$H NMR (400 MHz, DMSO) δ 11.03 (s, 1H), 10.81 (s, 1H), 7.92 - 7.83 (m, 2H), 7.79 (d, $J$ = 8.0 Hz, 1H), 7.19 (t, $J$ = 8.1 Hz, 2H), 7.03 (dd, $J$ = 12.4, 5.0 Hz, 2H), 5.16 (dd, $J$ = 13.3, 5.2 Hz, 1H), 4.53 (d, $J$ = 17.6 Hz, 3H), 4.40 (d, $J$ = 17.6 Hz, 1H), 3.26 (d, $J$ = 11.4 Hz, 2H), 3.19 (d, $J$ = 13.0 Hz, 2H), 3.08 (t, $J$ = 12.1 Hz, 2H), 2.98 - 2.88 (m, 1H), 2.64 (t, $J$ = 15.5 Hz, 1H), 2.44 (dd, $J$ = 13.3, 4.5 Hz, 1H), 2.26 (s, 3H), 2.06 - 1.98 (m, 1H). LCMS (ESI) calcd for $C_{25}H_{29}N_4O_3^+$ [M+H]$^+$: 433.2, found, 433.3.

Example 37: Preparation of 3-(1-oxo-5-((4-(m-tolyl)piperazin-1-yl)methyl)isoindolin-2-yl)piperidine-2,6-dione (GT-04462)

Referring to the method of Scheme 1, the target compound (GT-04462) was prepared under appropriate conditions as would be understood by those skilled in the art (white solid, 71 mg, yield 53%). $^1$H NMR (400 MHz, DMSO) δ 11.12 (s, 1H), 11.01 (s, 1H), 7.95 - 7.82 (m, 2H), 7.78 (d, $J$ = 7.9 Hz, 1H), 7.14 (*t*, $J$ = 7.8 Hz, 1H), 6.82 - 6.73 (m, 2H), 6.68 (d, $J$= *7.4* Hz, 1H), 5.15 (dd, $J$ = 13.3, 5.0 Hz, 1H), 4.61 - 4.46 (m, 3H), 4.40 (d, $J$ = 17.6 Hz, 1H), 3.79 (d, $J$ = 10.4 Hz, 2H), 3.40 (d, $J$ = 10.1 Hz, 2H), 3.23 - 3.07 (m, 4H), 2.98 - 2.88 (m, 1H), 2.64 (t, $J$ = 15.6 Hz, 1H), 2.47 - 2.38 (m, 1H), 2.26 (s, 3H), 2.07 - 1.99 (m, 1H). LCMS (ESI) calcd for $C_{25}H_{29}N_4O_3^+$ [M+H]$^+$: 433.2, found, 433.3.

Example 38: Preparation of 3-(1-oxo-5-((4-(p-tolyl)piperazin-1-yl)methyl)isoindolin-2-yl)piperidine-2,6-dione (GT-04463)

Referring to the method of Scheme 1, the target compound (GT-04463) was prepared under appropriate conditions as would be understood by those skilled in the art (white solid, 74 mg, yield 56%). $^1$H NMR (400 MHz, DMSO) δ 11.16 (s, 1H), 11.01 (s, 1H), 7.90 (s, 1H), 7.84 (d, $J$ = 7.8 Hz, 1H), 7.79 (d, $J$ = 7.9 Hz, 1H), 7.07 (d, $J$ = 8.4 Hz, 2H), 6.88 (d, $J$ = 8.5 Hz, 2H), 5.15 (dd, $J$ = 13.3, 5.1 Hz, 1H), 4.52 (d, $J$ = 17.2 Hz, 3H), 4.39 (d, $J$ = 17.6 Hz, 1H), 3.72 (d, $J$ = 12.0 Hz, 2H), 3.39 (d, $J$ = 12.4 Hz, 2H), 3.12 (dd, J= 25.6, 13.1 Hz, 3H), 3.00 - 2.87 (m, 1H), 2.62 (d, $J$ = 16.9 Hz, 1H), 2.47 - 2.36 (m, 1H), 2.21 (s, 3H), 2.06 - 1.98 (m, 1H). LCMS (ESI) calcd for $C_{25}H_{29}N_4O_3$ [M+H]$^+$: 433.2, found, 433.3.

Example 39: Preparation of 3-(5-((4-(2,4-dichlorophenyl)piperazin-1-yl)methyl)-1-oxoisoindolin-2-yl)piperidine-2,6-dione (GT-04464)

Referring to the method of Scheme 1, the target compound (GT-04464) was prepared under appropriate conditions as would be understood by those skilled in the art (white solid, 44 mg, yield 39%). $^1$H NMR (400 MHz, DMSO) δ 11.01 (s, 1H), 10.96 (s, 1H), 7.92 - 7.80 (m, 2H), 7.77 (d, $J$ = 7.6 Hz, 1H), 7.60 (d, $J$ = 2.3 Hz, 1H), 7.41 (dd, $J$ = 8.6, 2.3 Hz, 1H), 7.22 (t, $J$ = 7.3 Hz, 1H), 5.15 (dd, $J$ = 13.2, 5.1 Hz, 1H), 4.52 (d, $J$ = 18.0 Hz, 3H), 4.39 (d, $J$ = 17.4 Hz, 1H), 3.42 (t, $J$ = 12.6 Hz, 4H), 3.25 (d, $J$ = 10.5 Hz, 2H), 3.19 - 3.10 (m, 2H), 2.98 - 2.87 (m, 1H), 2.63 (t, $J$ = 15.9 Hz, 1H), 2.46 - 2.37 (m, 1H), 2.08 - 1.95 (m, 1H). LCMS (ESI) calcd for $C_{24}H_{25}Cl_2N_4O_3^+$ [M+H]$^+$: 487.13, found, 487.1.

Example 40: Preparation of 3-(5-((4-(2,5-dichlorophenyl)piperazin-1-yl)methyl)-1-oxoisoindolin-2-yl)piperidine-2,6-dione (GT-04487)

Referring to the method of Scheme 1, the target compound (GT-04487) was prepared under appropriate conditions as would be understood by those skilled in the art (white solid, 51 mg, yield 52%). $^1$H NMR (400 MHz, DMSO) δ 11.01 (s, 1H), 10.83 (s, 1H), 7.85 (d, $J$ = 10.7 Hz, 2H), 7.77 (d, $J$ = 8.1 Hz, 1H), 7.48 (d, $J$ = 8.5 Hz, 1H), 7.23 (s, 1H), 7.18 (d, $J$ = 8.3 Hz, 1H), 5.15 (dd, $J$ = 13.4, 5.0 Hz, 1H), 4.46 (dd, $J$ = 51.6, 17.7 Hz, 4H), 3.53 - 3.37 (m, 4H), 3.15 (s, 2H), 3.01 - 2.86 (m, 2H), 2.63 (t, $J$ = 16.1 Hz, 1H), 2.37 (*d*, $J$ = 30.0 Hz, 2H), 2.09 - 1.93 (m, 1H). LCMS (ESI) calcd for $C_{24}H_{25}Cl_2N_4O_3^+$ [M+H]$^+$: 487.13, found, 487.1.

Example 41: Preparation of 3-(5-((4-(2,6-dichlorophenyl)piperazin-1-yl)methyl)-1-oxoisoindolin-2-yl)piperidine-2,6-dione (GT-04613)

Referring to the method of Scheme 1, the target compound (GT-04613) was prepared under appropriate

conditions as would be understood by those skilled in the art (white solid, 57 mg, yield 58%). $^{1}H$ NMR (400 MHz, MeOD) δ 7.94 (d, *J* = 7.8 Hz, 1H), 7.81 (s, 1H), 7.74 (d, *J* = 8.1 Hz, 1H), 7.40 (d, *J* = 32.2 Hz, 2H), 7.18 (*t, J* = 8.1 Hz, 1H), 5.19 (dd, *J* = 13.3, 5.1 Hz, 1H), 4.66 - 4.48 (m, 4H), 3.79 (s, 2H), 3.44 (d, *J* = 33.6 Hz, 4H), 3.13 (s, 2H), 2.98 - 2.84 (m, 1H), 2.84 - 2.72 (m, 1H), 2.52 (qd, *J* = 13.2, 4.8 Hz, 1H), 2.28 - 2.13 (m, 1H). LCMS (ESI) calcd for $C_{24}H_{25}Cl_2N_4O_3^+$ [M+H]$^+$: 487.13, found, 487.1.

Example 42: Preparation of 3-(5-((4-(3,4-dichlorophenyl)piperazin-1-yl)methyl)-1-oxoisoindolin-2-yl)piperidine-2,6-dione (GT-04437)

Referring to the method of Scheme 1, the target compound (GT-04437) was prepared under appropriate conditions as would be understood by those skilled in the art (white solid, 72 mg, yield 64%). $^{1}H$ NMR (400 MHz, DMSO) δ 11.01 (s, 1H), 11.00 - 10.89 (m, 1H), 7.85 (d, *J*= 7.8 Hz, 2H), 7.75 (d, *J* = 7.6 Hz, 1H), 7.47 (d, *J= 9.0* Hz, 1H), 7.23 (d, *J*= 2.6 Hz, 1H), 6.98 (dd, *J= 9.0,* 2.7 Hz, 1H), 5.15 (dd, *J* = 13.3, 5.1 Hz, 1H), 4.52 (d, *J* = 17.3 Hz, 3H), 4.40 (d, *J* = 17.6 Hz, 1H), 3.90 (d, *J* = 10.2 Hz, 2H), 3.37 (s, 2H), 3.26 - 3.08 (m, 4H), 3.00 - 2.89 (m, 1H), 2.62 (d, *J* = 17.7 Hz, 1H), 2.47 - 2.33 (m, 1H), 2.07 - 1.98 (m, 1H). LCMS (ESI) calcd for $C_{24}H_{25}Cl_2N_4O_3^+$ [M+H]$^+$: 487.13, found, 487.1.

Example 43: Preparation of 3-(5-((4-(3,5-dichlorophenyl)piperazin-1-yl)methyl)-1-oxoisoindolin-2-yl)piperidine-2,6-dione (GT-04614)

Referring to the method of Scheme 1, the target compound (GT-04614) was prepared under appropriate conditions as would be understood by those skilled in the art (white solid, 77 mg, yield 68%). $^{1}H$ NMR (400 MHz, MeOD) δ 7.94 (d, *J* = 7.9 Hz, 1H), 7.78 (s, 1H), 7.71 (d, *J* = 7.9 Hz, 1H), 6.98 (s, 2H), 6.93 (s, 1H), 5.19 (dd, *J* = 13.3, 5.2 Hz, 1H), 4.58 (q, *J* = 17.1 Hz, 4H), 3.91 (s, 2H), 3.40 (dd, *J* = 48.0, 7.4 Hz, 4H), 3.13 (s, 2H), 2.98 - 2.86 (m, 1H), 2.79 (d, *J* = 15.3 Hz, 1H), 2.58 - 2.42 (m, 1H), 2.26 - 2.12 (m, 1H). LCMS (ESI) calcd for $C_{24}H_{25}Cl_2N_4O_3^+$ [M+H]$^+$: 487.13, found, 487.1.

Example 44: Preparation of 3-(5-((4-(2,3-difluorophenyl)piperazin-1-yl)methyl)-1-oxoisoindolin-2-yl)piperidine-2,6-dione (GT-04515)

Referring to the method of Scheme 1, the target compound (GT-04515) was prepared under appropriate conditions as would be understood by those skilled in the art (white solid, 55 mg, yield 53%). $^{1}H$ NMR (400 MHz, DMSO) δ 11.01 (s, 1H), 10.97 (s, 1H), 7.94 - 7.80 (m, 2H), 7.76 (d, *J* = 7.9 Hz, 1H), 7.10 (dq, *J* = 16.6, 7.9 Hz, 2H), 6.92 (t, *J* = 7.8 Hz, 1H), 5.15 (dd, *J* = 13.3, 5.1 Hz, 1H), 4.46 (dd, *J* = 51.1, 17.5 Hz, 4H), 3.53 (d, *J* = 10.1 Hz, 2H), 3.41 (s, 2H), 3.22 (d, *J* = 11.8 Hz, 3H), 2.94 (dd, *J* = 23.1, 10.3 Hz, 1H), 2.62 (d, *J*= 17.4 Hz, 1H), 2.43 (dd, *J*= 13.3, 4.4 Hz, 1H), 2.10 - 1.92 (m, 1H), 1.19 (t, *J* = 7.2 Hz, 1H). LCMS (ESI) calcd for $C_{24}H_{25}F_2N_4O_3^+$ [M+H]$^+$: 455.19, found, 455.2.

Example 45: Preparation of 3-(5-((4-(2,4-difluorophenyl)piperazin-1-yl)methyl)-1-oxoisoindolin-2-yl)piperidine-2,6-dione (GT-04438)

Referring to the method of Scheme 1, the target compound (GT-04438) was prepared under appropriate conditions as would be understood by those skilled in the art (white solid, 77 mg, yield 62%). $^{1}H$ NMR (400 MHz, DMSO) δ 11.01 (s, 2H), 7.92 - 7.83 (m, 2H), 7.78 (d, *J* = 7.8 Hz, 1H), 7.26 (ddd, *J* = 12.1, 9.0, 2.8 Hz, 1H), 7.14 (*td, J* = 9.4, 5.9 Hz, 1H), 7.05 (dd, *J* = 11.4, 5.3 Hz, 1H), 5.15 (dd, *J* = 13.3, 5.1 Hz, 1H), 4.60 - 4.47 (m, 3H), 4.40 (d, *J* = 17.5 Hz, 1H), 3.39 (s, 4H), 3.26 (d, *J* = 9.9 Hz, 2H), 3.16 (t, *J* = 11.2 Hz, 2H), 3.00 - 2.88 (m, 1H), 2.64 (t, *J* = 15.6 Hz, 1H), 2.47 - 2.31 (m, 1H), 2.07 - 1.97 (m, 1H). LCMS (ESI) calcd for $C_{24}H_{25}F_2N_4O_3^+$ [M+H]$^+$: 455.19, found, 455.2.

Example 46: Preparation of 3-(5-((4-(2,5-difluorophenyl)piperazin-1-yl)methyl)-1-oxoisoindolin-2-yl)piperidine-2,6-dione (GT-04539)

Referring to the method of Scheme 1, the target compound (GT-04539) was prepared under appropriate conditions as would be understood by those skilled in the art (white solid, 37 mg, yield 35%). $^{1}H$ NMR (400 MHz, DMSO) δ 11.01 (s, 1H), 10.85 (s, 1H), 7.85 (d, *J* = 7.7 Hz, 2H), 7.75 (d, *J* = 7.0 Hz, 1H), 7.27 - 7.15 (m, 1H), 7.01 - 6.95 (m, 1H), 6.85 - 6.82 (m, 1H), 5.15 (dd, *J* = 13.3, 5.1 Hz, 1H), 4.51 - 4.37 (m, 4H), 3.54 (d, *J* = 11.0 Hz, 2H), 3.43 - 3.40 (m, 2H), 3.31 - 3.25 (m, 2H), 3.19 - 3.15 (m, 2H), 3.00 - 2.82 (m, 1H), 2.61 (d, *J* = 18.4 Hz, 1H), 2.43 (dd, *J* = 13.3, 4.5 Hz, 1H), 2.12 - 1.89 (m, 1H). LCMS (ESI) calcd for $C_{24}H_{25}F_2N_4O_3^+$ [M+H]$^+$: 455.19, found, 455.2.

Example 47: Preparation of 3-(5-((4-(2,6-difluorophenyl)piperazin-1-yl)methyl)-1-oxoisoindolin-2-yl)piperidine-2,6-dione (GT-04540)

Referring to the method of Scheme 1, the target compound (GT-04540) was prepared under appropriate conditions as would be understood by those skilled in the art (white solid, 47 mg, yield 45%). $^1$H NMR (400 MHz, DMSO) δ 11.01 (s, 1H), 10.87 (s, 1H), 7.93 - 7.81 (m, 2H), 7.76 (d, *J* = 8.0 Hz, 1H), 7.19 = 7.15 (m, 1H), 7.10 - 7.05 (m, 2H), 5.15 (dd, *J* = 13.2, 5.1 Hz, 1H), 4.54 - 4.37 (m, 4H), 3.52 (t, *J* = 11.7 Hz, 2H), 3.41 - 3.37 (m, 2H), 3.33 - 3.29 (m, 2H), 3.21 - 3.17 (m, 2H), 3.01 - 2.83 (m, 1H), 2.61 (d, *J* = 17.9 Hz, 1H), 2.43 (dd, *J* = 13.1, 4.5 Hz, 1H), 2.09 - 1.92 (m, 1H). LCMS (ESI) calcd for $C_{24}H_{25}F_2N_4O_3^+$ [M+H]$^+$: 455.19, found, 455.2.

Example 48: Preparation of 3-(5-((4-(3,4-difluorophenyl)piperazin-1-yl)methyl)-1-oxoisoindolin-2-yl)piperidine-2,6-dione (GT-04529)

Referring to the method of Scheme 1, the target compound (GT-04529) was prepared under appropriate conditions as would be understood by those skilled in the art (white solid, 51 mg, yield 49%). $^1$H NMR (400 MHz, DMSO) δ 11.01 (s, 1H), 10.72 (s, 1H), 7.85 (d, *J* = 7.9 Hz, 2H), 7.73 (d, *J* = 7.7 Hz, 1H), 7.31 (dd, *J* = 19.7, 9.5 Hz, 1H), 7.08 (ddd, *J* = 13.8, 6.9, 2.8 Hz, 1H), 6.80 - 6.76 (m, 1H), 5.15 (dd, *J* = 13.2, 5.0 Hz, 1H), 4.60 - 4.33 (m, 4H), 3.80 (d, *J* = 11.4 Hz, 2H), 3.51 - 3.39 (m, 2H), 3.22 - 3.07 (m, 4H), 3.01 - 2.82 (m, 1H), 2.67 - 2.60 (m, 1H), 2.46 - 2.36 (m, 1H), 2.09 - 1.86 (m, 1H). LCMS (ESI) calcd for $C_{24}H_{25}F_2N_4O_3^+$ [M+H]$^+$: 455.19, found, 455.2.

Example 49: Preparation of 3-(5-((4-(3,5-difluorophenyl)piperazin-1-yl)methyl)-1-oxoisoindolin-2-yl)piperidine-2,6-dione (GT-04496)

Referring to the method of Scheme 1, the target compound (GT-04496) was prepared under appropriate conditions as would be understood by those skilled in the art (white solid, 68 mg, yield 55%). $^1$H NMR (400 MHz, DMSO) δ 11.11 (s, 1H), 11.02 (s, 1H), 7.92 - 7.80 (m, 2H), 7.75 (d, *J* = 7.8 Hz, 1H), 6.70 (d, *J* = 9.1 Hz, 2H), 6.58 (t, *J* = 9.2 Hz, 1H), 5.15 (dd, *J* = 13.3, 5.1 Hz, 1H), 4.54 - 4.37 (m, 4H), 3.93 (d, *J* = 12.9 Hz, 2H), 3.41- 3.38 (m, 2H), 3.27 - 3.15 (m, 4H), 3.02 - 2.85 (m, 1H), 2.61 (d, *J* = 16.4 Hz, 1H), 2.43 (dd, *J* = 13.0, 4.5 Hz, 1H), 2.05 - 2.01 (m, 1H). LCMS (ESI) calcd for $C_{24}H_{25}F_2N_4O_3^+$ [M+H]$^+$: 455.19, found, 455.2.

Example 50: Preparation of 3-(5-((4-(2,4-dimethylphenyl)piperazin-1-yl)methyl)-1-oxoisoindolin-2-yl)piperidine-2,6-dione (GT-04439)

Referring to the method of Scheme 1, the target compound (GT-04439) was prepared under appropriate conditions as would be understood by those skilled in the art (white solid, 87 mg, yield 68%). $^1$H NMR (400 MHz, DMSO) δ 11.13 (s, 1H), 11.01 (s, 1H), 7.93 (s, 1H), 7.83 (q, *J* = 7.9 Hz, 2H), 7.04 - 6.95 (m, 2H), 6.92 (d, *J* = 8.0 Hz, 1H), 5.15 (dd, *J* = 13.3, 5.0 Hz, 1H), 4.58 - 4.48 (m, 3H), 4.40 (d, *J* = 17.6 Hz, 1H), 3.38 (d, *J* = 11.2 Hz, 2H), 3.23 (d, *J* = 7.8 Hz, 2H), 3.11 (s, 4H), 3.00 - 2.87 (m, 1H), 2.64 (t, *J* = 16.0 Hz, 1H), 2.47 - 2.33 (m, 1H), 2.21 (s, 6H), 2.07 - 1.98 (m, 1H). LCMS (ESI) calcd for $C_{26}H_{31}N_4O_3^+$ [M+H]$^+$: 447.24, found, 447.3.

Example 51: Preparation of 3-(5-((4-(5-chloro-2-methylphenyl)piperazin-1-yl)methyl)-1-oxoisoindolin-2-yl)piperidine-2,6-dione (GT-04440)

Referring to the method of Scheme 1, the target compound (GT-04440) was prepared under appropriate conditions as would be understood by those skilled in the art (white solid, 58 mg, yield 49%). $^1$H NMR (400 MHz, DMSO) δ 11.02 (s, 1H), 10.78 (s, 1H), 7.92 - 7.81 (m, 2H), 7.78 (d, *J* = 7.9 Hz, 1H), 7.22 (d, *J* = 8.2 Hz, 1H), 7.06 (dd, *J* = 14.2, 5.0 Hz, 2H), 5.16 (dd, *J* = 13.2, 5.1 Hz, 1H), 4.53 (d, *J* = 17.4 Hz, 3H), 4.40 (d, *J* = 17.7 Hz, 1H), 3.41 (d, *J* = 11.8 Hz, 2H), 3.31 - 3.19 (m, 4H), 3.08 (t, *J* = 11.7 Hz, 2H), 2.99 - 2.86 (m, 1H), 2.64 (t, *J* = 15.5 Hz, 1H), 2.47 - 2.37 (m, 1H), 2.23 (s, 3H), 2.07 - 1.99 (m, 1H). LCMS (ESI) calcd for $C_{25}H_{28}ClN_4O_3^+$ [M+H]$^+$: 467.18, found, 467.2.

Example 52: Preparation of 3-(1-oxo-5-((4-phenylpiperidin-1-yl)methyl)isoindolin-2-yl)piperidine-2,6-dione (GT-04465)

Referring to the method of Scheme 1, the target compound (GT-04465) was prepared under appropriate conditions as would be understood by those skilled in the art (white solid, 60 mg, yield 46%). $^1$H NMR (400 MHz, DMSO) δ 11.01 (s, 1H), 10.49 (s, 1H), 7.89 - 7.81 (m, 2H), 7.75 (d, *J* = 8.0 Hz, 1H), 7.37 - 7.30 (m, 2H), 7.23 (t, *J* = 6.3 Hz, 3H), 5.15 (dd, *J* = 13.2, 5.1 Hz, 1H), 4.46 (dt, *J* = 32.3, 17.6 Hz, 4H), 3.46 (d, *J* = 12.3 Hz, 2H), 3.15 - 3.01 (m, 2H), 2.98 - 2.89 (m, 1H), 2.80 (t, *J* = 11.4 Hz, 1H), 2.63 (t, *J* = 15.3 Hz, 1H), 2.46 - 2.37 (m, 1H), 1.99 (dd, *J* = 26.0, 12.6 Hz, 4H). LCMS (ESI) calcd for $C_{25}H_{28}N_3O_3^+$ [M+H]$^+$: 418.21, found, 418.3.

Example 53: Preparation of 3-(5-((4-(3-bromophenyl)piperidin-1-yl)methyl)-1-oxoisoindolin-2-yl)piperidine-2,6-dione (GT-04466)

Referring to the method of Scheme 1, the target compound (GT-04466) was prepared under appropriate conditions as would be understood by those skilled in the art (white solid, 34 mg, yield 31%). $^1H$ NMR (400 MHz, DMSO) δ 11.01 (s, 1H), 10.43 (s, 1H), 7.85 (d, *J* = 7.4 Hz, 2H), 7.77 - 7.69 (m, 1H), 7.47 - 7.39 (m, 2H), 7.31 (t, *J* = 7.8 Hz, 1H), 7.24 (d, *J* = 7.8 Hz, 1H), 5.15 (dd, *J* = 13.3, 5.0 Hz, 1H), 4.46 (dt, *J* = 34.1, 17.6 Hz, 4H), 3.46 (d, *J* = 12.4 Hz, 2H), 3.06 (s, 2H), 2.97 - 2.80 (m, 2H), 2.63 (t, *J* = 15.1 Hz, 1H), 2.46 - 2.36 (m, 1H), 2.08 - 1.93 (m, 5H). LCMS (ESI) calcd for $C_{25}H_{27}BrN_3O_3^+$ $[M+H]^+$: 496.12, found, 496.1.

Example 54: Preparation of 3-(5-((4-(4-bromophenyl)piperidin-1-yl)methyl)-1-oxoisoindolin-2-yl)piperidine-2,6-dione (GT-04493)

Referring to the method of Scheme 1, the target compound (GT-04493) was prepared under appropriate conditions as would be understood by those skilled in the art (white solid, 45 mg, yield 41%). $^1H$ NMR (400 MHz, DMSO) δ 11.02 (s, 1H), 10.54 (s, 1H), 7.85 (d, *J* = 10.2 Hz, 2H), 7.75 (d, *J* = 7.8 Hz, 1H), 7.53 (d, *J* = 8.4 Hz, 2H), 7.19 (d, *J* = 8.5 Hz, 2H), 5.15 (dd, *J* = 13.3, 5.1 Hz, 1H), 4.54 - 4.37 (m, 4H), 3.46 (d, *J* = 10.8 Hz, 2H), 3.05 (d, *J* = 11.1 Hz, 2H), 2.99 - 2.87 (m, 1H), 2.85 - 2.78 (m, 1H), 2.63 (t, *J* = 15.5 Hz, 1H), 2.46 - 2.39 (m, 1H), 2.05 - 2.01 (m, 5H). LCMS (ESI) calcd for $C_{25}H_{27}BrN_3O_3^+$ $[M+H]^+$: 496.12, found, 496.1.

Example 55: Preparation of 3-(5-((4-(2-chlorophenyl)piperidin-1-yl)methyl)-1-oxoisoindolin-2-yl)piperidine-2,6-dione (GT-04468)

Referring to the method of Scheme 1, the target compound (GT-04468) was prepared under appropriate conditions as would be understood by those skilled in the art (white solid, 51 mg, yield 41%). $^1H$ NMR (400 MHz, DMSO) δ 11.01 (s, 1H), 10.70 (s, 1H), 7.91 - 7.79 (m, 2H), 7.76 (d, *J* = 7.9 Hz, 1H), 7.45 (d, *J* = 7.9 Hz, 1H), 7.37 (t, *J* = 7.0 Hz, 1H), 7.33 - 7.22 (m, 2H), 5.15 (dd, *J* = 13.3, 5.1 Hz, 1H), 4.46 (dt, *J* = 31.1, 17.6 Hz, 4H), 3.47 (d, *J* = 11.6 Hz, 2H), 3.19 (dt, *J* = 31.7, 10.8 Hz, 3H), 2.98 - 2.88 (m, 1H), 2.61 (d, *J* = 17.3 Hz, 1H), 2.43 (dd, *J* = 13.0, 4.4 Hz, 1H), 2.04 (dd, *J* = 21.3, 8.7 Hz, 3H), 1.95 (d, *J* = 12.8 Hz, 2H). LCMS (ESI) calcd for $C_{25}H_{27}ClN_3O_3^+$ $[M+H]^+$: 452.17, found, 452.2.

Example 56: Preparation of 3-(5-((4-(4-chlorophenyl)piperidin-1-yl)methyl)-1-oxoisoindolin-2-yl)piperidine-2,6-dione (GT-04469)

Referring to the method of Scheme 1, the target compound (GT-04469) was prepared under appropriate conditions as would be understood by those skilled in the art (white solid, 73 mg, yield 58%). $^1H$ NMR (400 MHz, DMSO) δ 11.01 (s, 1H), 10.46 (s, 1H), 7.85 (d, *J* = 7.6 Hz, 2H), 7.74 (d, *J* = 8.0 Hz, 1H), 7.39 (d, *J* = 8.4 Hz, 2H), 7.25 (d, *J* = 8.5 Hz, 2H), 5.15 (dd, *J* = 13.3, 5.2 Hz, 1H), 4.55 - 4.38 (m, 4H), 3.46 (d, *J* = 11.4 Hz, 2H), 3.06 (d, *J* = 9.1 Hz, 2H), 2.96 - 2.88 (m, 1H), 2.82 (s, 1H), 2.63 (t, *J* = 15.5 Hz, 1H), 2.43 (dd, *J* = 13.0, 4.3 Hz, 1H), 2.06 - 1.94 (m, 5H). LCMS (ESI) calcd for $C_{25}H_{27}ClN_3O_3^+$ $[M+H]^+$: 452.17, found, 452.2.

Example 57: Preparation of 3-(5-((4-(2-fluorophenyl)piperidin-1-yl)methyl)-1-oxoisoindolin-2-yl)piperidine-2,6-dione (GT-04470)

Referring to the method of Scheme 1, the target compound (GT-04470) was prepared under appropriate conditions as would be understood by those skilled in the art (white solid, 35 mg, yield 27%). $^1H$ NMR (400 MHz, DMSO) δ 11.01 (s, 1H), 10.48 (s, 1H), 7.85 (d, *J* = 7.3 Hz, 2H), 7.74 (d, *J* = 8.1 Hz, 1H), 7.29 (dd, *J* = 17.0, 7.6 Hz, 2H), 7.23 - 7.15 (m, 2H), 5.15 (dd, *J* = 13.3, 5.0 Hz, 1H), 4.46 (dt, *J* = 29.3, 17.6 Hz, 4H), 3.46 (d, *J* = 11.2 Hz, 2H), 3.20 - 3.06 (m, 3H), 2.97 - 2.87 (m, 1H), 2.61 (d, *J* = 16.8 Hz, 1H), 2.43 (dd, *J* = 13.1, 4.1 Hz, 1H), 2.14 - 1.99 (m, 3H), 1.93 (d, *J* = 13.4 Hz, 2H). LCMS (ESI) calcd for $C_{25}H_{27}FN_3O_3^+$ $[M+H]^+$: 436.20, found, 436.2.

Example 58: Preparation of 3-(5-((4-(4-fluorophenyl)piperidin-1-yl)methyl)-1-oxoisoindolin-2-yl)piperidine-2,6-dione (GT-04471)

Referring to the method of Scheme 1, the target compound (GT-04471) was prepared under appropriate conditions as would be understood by those skilled in the art (white solid, 57 mg, yield 43%). $^1H$ NMR (400 MHz, DMSO) δ 11.01 (s, 1H), 10.61 (s, 1H), 7.88 - 7.80 (m, 2H), 7.76 (d, *J* = 7.8 Hz, 1H), 7.26 (dd, *J* = 8.6, 5.6 Hz, 2H), 7.16 (t, *J* = 8.9 Hz, 2H), 5.15 (dd, *J* = 13.2, 5.1 Hz, 1H), 4.46 (dt, *J* = 31.7, 17.6 Hz, 4H), 3.45 (d, *J* = 11.8 Hz, 2H), 3.11 - 2.99 (m, 2H), 2.98 - 2.89 (m, 1H), 2.82 (t, *J* = 11.7 Hz, 1H), 2.63 (t, *J* = 15.4 Hz, 1H), 2.47 - 2.38 (m, 1H), 1.98 (dd, *J* = 27.8, 12.4 Hz, 5H). LCMS (ESI) calcd

for $C_{25}H_{27}FN_3O_3^+$ [M+H]$^+$: 436.20, found, 436.2.

Example 59: Preparation of 3-(1-oxo-5-((4-(2-(trifluoromethyl)phenyl)piperidin-1-yl)methyl)isoindolin-2-yl)piperidine-2,6-dione (GT-04488)

Referring to the method of Scheme 1, the target compound (GT-04488) was prepared under appropriate conditions as would be understood by those skilled in the art (white solid, 46 mg, yield 40%). $^1$H NMR (400 MHz, DMSO) δ 11.01 (s, 1H), 10.48 (s, 1H), 7.85 (d, *J* = 8.3 Hz, 2H), 7.72 (*t, J* = 8.9 Hz, 3H), 7.53 (d, *J* = 8.2 Hz, 1H), 7.46 (t, *J* = 7.7 Hz, 1H), 5.15 (dd, *J* = 13.2, 5.0 Hz, 1H), 4.46 (dt, *J* = 32.0, 17.6 Hz, 4H), 3.46 (d, *J* = 11.8 Hz, 2H), 3.26 - 3.09 (m, 3H), 3.00 - 2.85 (m, 1H), 2.70 - 2.57 (m, 1H), 2.46 - 2.38 (m, 1H), 2.24 - 2.09 (m, 2H), 2.09 - 1.96 (m, 1H), 1.88 (d, *J* = 13.3 Hz, 2H). LCMS (ESI) calcd for $C_{26}H_{27}F_3N_3O_3^+$ [M+H]$^+$: 486.20, found, 486.2.

Example 60: Preparation of 3-(1-oxo-5-((4-(3-(trifluoromethyl)phenyl)piperidin-1-yl)methyl)isoindolin-2-yl)piperidine-2,6-dione (GT-04472)

Referring to the method of Scheme 1, the target compound (GT-04472) was prepared under appropriate conditions as would be understood by those skilled in the art (white solid, 52 mg, yield 46%). $^1$H NMR (400 MHz, DMSO) δ 11.01 (s, 1H), 10.60 (s, 1H), 7.89 - 7.82 (m, 2H), 7.76 (d, *J* = 8.1 Hz, 1H), 7.63 - 7.54 (m, 4H), 5.15 (dd, *J* = 13.3, 5.1 Hz, 1H), 4.47 (dt, *J* = 38.7, 17.6 Hz, 4H), 3.48 (d, *J* = 11.0 Hz, 2H), 3.07 (dd, *J* = 21.7, 9.6 Hz, 2H), 2.99 - 2.89 (m, 2H), 2.61 (d, *J* = 17.6 Hz, 1H), 2.43 (dd, *J* = 12.9, 4.1 Hz, 1H), 2.11 - 1.97 (m, 5H). LCMS (ESI) calcd for $C_{26}H_{27}F_3N_3O_3^+$ [M+H]$^+$: 486.20, found, 486.2.

Example 61: Preparation of 3-(5-((4-(2,3-dichlorophenyl)piperidin-1-yl)methyl)-1-oxoisoindolin-2-yl)piperidine-2,6-dione (GT-04551)

Referring to the method of Scheme 1, the target compound (GT-04551) was prepared under appropriate conditions as would be understood by those skilled in the art (white solid, 59 mg, yield 60%). $^1$H NMR (400 MHz, DMSO) δ 11.01 (s, 1H), 10.18 (s, 1H), 7.86 (*d, J* = 7.8 Hz, 1H), 7.82 (s, 1H), 7.72 (d, *J* = 7.6 Hz, 1H), 7.56 (d, *J* = 7.9 Hz, 1H), 7.40 (t, *J* = 8.0 Hz, 1H), 7.29 (d, *J* = 7.5 Hz, 1H), 5.15 (dd, *J* = 13.2, 5.3 Hz, 1H), 4.54 - 4.38 (m, 5H), 4.19 - 3.98 (m, 4H), 3.03 - 2.84 (m, 2H), 2.73 - 2.58 (m, 2H), 2.44 (d, *J* = 13.1 Hz, 1H), 2.04 - 1.93 (m, 4H). LCMS (ESI) calcd for $C_{25}H_{26}Cl_2N_3O_3^+$ [M+H]$^+$: 486.13, found, 486.1.

Example 62: Preparation of 3-(5-((4-(2-chloro-3-fluorophenyl)piperidin-1-yl)methyl)-1-oxoisoindolin-2-yl)piperidine-2,6-dione (GT-04553)

Referring to the method of Scheme 1, the target compound (GT-04553) was prepared under appropriate conditions as would be understood by those skilled in the art (white solid, 60 mg, yield 59%). $^1$H NMR (400 MHz, DMSO) δ 11.01 (s, 1H), 10.00(s, 1H), 7.87 - 7.84 (m, 2H), 7.74 (d, *J* = 7.8 Hz, 1H), 7.47 - 7.38 (m, 1H), 7.34 - 7.30 (m, 1H), 7.18 - 7.12 (m, 1H), 5.15 (dd, *J* = 13.3, 5.0 Hz, 1H), 4.55 - 4.46 (m, 5H), 3.52 - 3.47 (m, 2H), 3.24 - 3.20 (m, 3H), 3.00 - 2.84 (m, 2H), 2.63 (t, *J* = 15.2 Hz, 1H), 2.43 (dd, *J* = 13.2, 4.5 Hz, 1H), 2.05 - 2.01 (m, 5H). LCMS (ESI) calcd for $C_{25}H_{26}ClFN_3O_3{}^+$ [M+H]$^+$: 470.16, found, 470.2.

Example 63: Preparation of 3-(5-((4-(2,3-difluorophenyl)piperidin-1-yl)methyl)-1-oxoisoindolin-2-yl)piperidine-2,6-dione (GT-04552)

Referring to the method of Scheme 1, the target compound (GT-04552) was prepared under appropriate conditions as would be understood by those skilled in the art (white solid, 59 mg, yield 56%). $^1$H NMR (400 MHz, DMSO) δ 11.01 (s, 1H), 10.28 (s, 1H), 7.89 - 7.81 (m, 2H), 7.73 (d, *J* = 8.0 Hz, 1H), 7.35 - 7.31 (m, 1H), 7.24 - 7.19 (m, 1H), 7.11 - 7.08 (m, 1H), 5.15 (dd, *J* = 13.2, 4.9 Hz, 1H), 4.52 - 4.42 (m, 5H), 3.52 - 3.48 (m, 2H), 3.15 - 3.12 (m, 3H), 3.02 - 2.85 (m, 2H), 2.70 - 2.57 (m, 2H), 2.46 - 2.38 (m, 1H), 2.10 - 1.90 (m, 5H). LCMS (ESI) calcd for $C_{25}H_{26}F_2N_3O_3^+$ [M+H]$^+$: 454.19, found, 454.2.

Example 64: Preparation of 3-(5-((4-(2,3-dichlorophenyl)-3,6-dihydropyridin-1(2H)-yl)methyl)-1-oxoisoindolin-2-yl)piperidine-2,6-dione (GT-04550)

Referring to the method of Scheme 1, the target compound (GT-04550) was prepared under appropriate conditions as would be understood by those skilled in the art (white solid, 57 mg, yield 58%). $^1$H NMR (400 MHz, DMSO) δ 11.01 (s, 1H), 10.85 (s, 1H), 7.94 - 7.82 (m, 2H), 7.78 (d, *J*=7.5 Hz, 1H), 7.63 (dd, *J* = 8.1, 1.3 Hz, 1H), 7.40 (t, *J* = 7.9 Hz, 1H), 7.25 (dd, *J* = 7.7, 1.5 Hz, 1H), 5.75 (s, 1H), 5.15 (dd, *J* = 13.3, 5.1 Hz, 1H), 4.66 - 4.34 (m, 4H), 3.91 - 3.71 (m, 2H), 3.69 - 3.56 (m, 1H), 3.01 - 2.75 (m, 2H), 2.69 - 2.53 (m, 3H), 2.43 (dd, *J* = 13.4, 4.5 Hz, 1H), 2.09 - 1.92 (m, 1H). LCMS (ESI) calcd for $C_{25}H_{24}Cl_2N_3O_3^+$ [M+H]$^+$: 484.12, found, 484.1.

Example 65: Preparation of 3-(5-((4-(2-chloro-3-fluorophenyl)-3,6-dihydropyridin-1(2H)-yl)methyl)-1-oxoisoindolin-2-yl)piperidine-2,6-dione (GT-04554)

Referring to the method of Scheme 1, the target compound (GT-04554) was prepared under appropriate conditions as would be understood by those skilled in the art (white solid, 57 mg, yield 56%). $^1$H NMR (400 MHz, DMSO) δ 11.01 (s, 1H), 10.61 (s, 1H), 7.86 (d, *J* = 8.1 Hz, 2H), 7.76 (d, *J* = 7.0 Hz, 1H), 7.43 - 7.40 (m, 2H), 7.18 - 7.10 (m, 1H), 5.79 (s, 1H), 5.15 (dd, *J* = 13.2, 5.1 Hz, 1H), 4.64 - 4.35 (m, 5H), 3.87 - 3.78 (m, 2H), 3.65 - 3.60 (m, 1H), 2.97 - 2.79 (m, 2H), 2.72 - 2.58 (m, 2H), 2.43 (dd, *J* = 13.2, 4.6 Hz, 1H), 2.10 - 1.92 (m, 1H). LCMS (ESI) calcd for $C_{25}H_{24}ClFN_3O_3^+$ [M+H]$^+$: 468.15, found, 468.2.

Example 66: Preparation of 3-(1-oxo-5-((4-(pyridin-2-yl)piperazin-1-yl)methyl)isoindolin-2-yl)piperidine-2,6-dione (GT-04449)

Referring to the method of Scheme 1, the target compound (GT-04449) was prepared under appropriate conditions as would be understood by those skilled in the art (white solid, 75 mg, yield 54%). $^1$H NMR (400 MHz, DMSO) δ 12.06 (s, 1H), 11.02 (s, 1H), 8.12 (dd, *J*= 5.8, 1.4 Hz, 1H), 7.97 (t, *J*= 7.9 Hz, 1H), 7.93 (s, 1H), 7.86 - 7.76 (m, 2H), 7.30 (d, *J* = 9.0 Hz, 1H), 6.98 (t, *J* = 6.4 Hz, 1H), 5.15 (dd, *J* = 13.3, 5.1 Hz, 1H), 4.54 - 4.50 (m, 4H), 4.39 (d, *J* = 17.6 Hz, 3H), 3.6br6 (s, 2H), 3.44 (brs, 2H), 3.22 (brs, 2H), 2.94 - 2.89 (m, 1H), 2.62 (d, *J* = 16.8 Hz, 1H), 2.47 - 2.39 (m, 1H), 2.08 - 1.93 (m, 1H). LCMS (ESI) calcd for $C_{23}H_{26}N_5O_3^+$ [M+H]$^+$: 420.20, found, 420.2.

Example 67: Preparation of 3-(5-((4-(6-methylpyridin-2-yl)piperazin-1-yl)methyl)-1-oxoisoindolin-2-yl)piperidine-2,6-dione (GT-04530)

Referring to the method of Scheme 1, the target compound (GT-04530) was prepared under appropriate conditions as would be understood by those skilled in the art (white solid, 20 mg, yield 19%). $^1$H NMR (400 MHz, DMSO) δ 12.05 (s, 1H), 11.01 (s, 1H), 7.93 (s, 1H), 7.91 - 7.76 (m, 3H), 7.09 (d, *J* = 8.7 Hz, 1H), 6.85 (d, *J* = 7.2 Hz, 1H), 5.14 (dd, *J* = 13.3, 5.1 Hz, 1H), 4.62 - 4.35 (m, 6H), 3.71 - 3.60 (m, 2H), 3.50 - 3.37 (m, 3H), 3.31 - 3.12 (m, 2H), 2.93 - 2.89 (m, 1H), 2.61 (d, *J* = 17.6 Hz, 1H), 2.53 (s, 3H), 2.48 - 2.41 (m, 1H), 2.04 - 2.01 (m, 1H). LCMS (ESI) calcd for $C_{24}H_{28}N_5O_3^+$ [M+H]$^+$: 434.22, found, 434.2.

Example 68: Preparation of 3-(5-((4-(6-chloropyridin-2-yl)piperazin-1-yl)methyl)-1-oxoisoindolin-2-yl)piperidine-2,6-dione (GT-04497)

Referring to the method of Scheme 1, the target compound (GT-04497) was prepared under appropriate conditions as would be understood by those skilled in the art (white solid, 31 mg, yield 30%). $^1$H NMR (400 MHz, DMSO) δ 11.59 (s, 1H), 11.02 (s, 1H), 7.90 (s, 1H), 7.83 (d, *J* = 7.8 Hz, 1H), 7.78 (d, *J* = 7.8 Hz, 1H), 7.64 (dd, *J* = 8.3, 7.6 Hz, 1H), 6.89 (d, *J* = 8.4 Hz, 1H), 6.78 (d, *J* = 7.5 Hz, 1H), 5.15 (dd, *J* = 13.3, 5.1 Hz, 1H), 4.54 - 4.31 (m, 6H), 3.44 - 3.36 (m, 4H), 3.13 - 3.08 (m, 2H), 3.00 - 2.85 (m, 1H), 2.61 (d, *J* = 16.5 Hz, 1H), 2.43 (dd, *J* = 13.1, 4.5 Hz, 1H), 2.09 - 1.91 (m, 1H). LCMS (ESI) calcd for $C_{23}H_{25}ClN_5O_3^+$ [M+H]$^+$: 454.16, found, 454.2.

Example 69: Preparation of 3-(5-((4-(5-chloropyridin-2-yl)piperazin-1-yl)methyl)-1-oxoisoindolin-2-yl)piperidine-2,6-dione (GT-04431)

Referring to the method of Scheme 1, the target compound (GT-04431) was prepared under appropriate conditions as would be understood by those skilled in the art (white solid, 91 mg, yield 73%). $^1$H NMR (400 MHz, DMSO) δ 11.37 (s, 1H), 11.02 (s, 1H), 8.18 (d, *J* = 2.6 Hz, 1H), 7.89 (s, 1H), 7.84 (d, *J* = 7.8 Hz, 1H), 7.77 (d, *J* = 8.0 Hz, 1H), 7.71 (dd, *J* = 9.1, 2.7 Hz, 1H), 6.98 (d, *J* = 9.1 Hz, 1H), 5.15 (dd, *J* = 13.3, 5.1 Hz, 1H), 4.43 (dt, *J* = 33.2, 16.6 Hz, 6H), 3.35 (dd, *J* = 25.9, 12.5 Hz, 4H), 3.10 (d, *J* = 11.2 Hz, 2H), 3.00 - 2.86 (m, 1H), 2.62 (d, *J* = 16.6 Hz, 1H), 2.47 - 2.37 (m, 1H), 2.07 - 1.98 (m, 1H). LCMS (ESI) calcd for $C_{23}H_{25}ClN_5O_3^+$ [M+H]$^+$: 454.16, found, 454.

Example 70: Preparation of 3-(5-((4-(4-chloropyridin-2-yl)piperazin-1-yl)methyl)-1-oxoisoindolin-2-yl)piperidine-2,6-dione (GT-04430)

Referring to the method of Scheme 1, the target compound (GT-04430) was prepared under appropriate conditions as would be understood by those skilled in the art (white solid, 89 mg, yield 72%). $^1$H NMR (400 MHz, DMSO) δ 11.86 (s, 1H), 11.02 (s, 1H), 8.12 (d, *J* = 5.6 Hz, 1H), 7.92 (s, 1H), 7.81 (q, *J* = 8.1 Hz, 2H), 7.15 (d, *J* = 1.3 Hz, 1H), 6.87 (dd, *J* = 5.6, 1.6 Hz, 1H), 5.15 (dd, *J* = 13.3, 5.1 Hz, 1H), 4.44 (dt, *J* = 24.4, 17.8 Hz, 6H), 3.57 - 3.28 (m, 4H), 3.11 (s, 2H), 2.99 - 2.86 (m, 1H), 2.61 (d, *J* = 17.3 Hz, 1H), 2.44 (dd, *J* = 13.1, 4.4 Hz, 1H), 2.02 (dd, *J* = 9.0, 3.6 Hz, 1H). LCMS (ESI) calcd for

$C_{23}H_{25}ClN_5O_3^+$ [M+H]+: 454.16, found, 454.2.

Example 71: Preparation of 3-(5-((4-(3-chloropyridin-2-yl)piperazin-1-yl)methyl)-1-oxoisoindolin-2-yl)piperidine-2,6-dione (GT-04428)

Referring to the method of Scheme 1, the target compound (GT-04428) was prepared under appropriate conditions as would be understood by those skilled in the art (white solid, 82 mg, yield 66%). $^1$H NMR (400 MHz, DMSO) δ 11.70 (s, 1H), 11.02 (s, 1H), 8.26 (dd, *J* = 4.7, 1.6 Hz, 1H), 7.95 (s, 1H), 7.87 (dd, *J* = 7.8, 1.6 Hz, 1H), 7.83 (s, 2H), 7.10 (dd, *J* = 7.8, 4.7 Hz, 1H), 5.15 (dd, *J* = 13.3, 5.1 Hz, 1H), 4.54 - 4.36 (m, 4H), 3.83 (d, *J* = 13.5 Hz, 2H), 3.39 (t, *J* = 13.9 Hz, 4H), 3.29 - 3.16 (m, 2H), 2.93 (ddd, *J* = 17.3, 9.5, 4.1 Hz, 1H), 2.62 (d, *J* = 16.6 Hz, 1H), 2.49 - 2.38 (m, 1H), 2.10 - 1.94 (m, 1H). LCMS (ESI) calcd for $C_{23}H_{25}ClN_5O_3^+$ [M+H]+: 454.16, found, 454.2.

Example 72: Preparation of 3-(5-((4-(3-fluoropyridin-2-yl)piperazin-1-yl)methyl)-1-oxoisoindolin-2-yl)piperidine-2,6-dione (GT-04578)

Referring to the method of Scheme 1, the target compound (GT-04578) was prepared under appropriate conditions as would be understood by those skilled in the art (white solid, 33 mg, yield 30%). $^1$H NMR (400 MHz, MeOD) δ 8.07 (dd, J = 5.4, 1.3 Hz, 1H), 7.95 (d, J = 7.8 Hz, 1H), 7.90 (s, 1H), 7.81 - 7.73 (m, 2H), 7.20 - 7.09 (m, 1H), 5.20 (dd, J = 13.3, 5.2 Hz, 1H), 4.80 - 4.54 (m, 4H), 4.27 (s, 2H), 3.77 - 3.40 (m, 6H), 2.99 - 2.90 (m, 1H), 2.85 - 2.74 (m, 1H), 2.60 - 2.49 (m, 1H), 2.25 - 2.19 (m, 1H). LCMS (ESI) calcd for $C_{23}H_{25}FN_5O_3^+$ [M+H]+: 438.19, found, 438.3.

Example 73: Preparation of 3-(5-((4-(6-fluoropyridin-2-yl)piperazin-1-yl)methyl)-1-oxoisoindolin-2-yl)piperidine-2,6-dione (GT-04498)

Referring to the method of Scheme 1, the target compound (GT-04498) was prepared under appropriate conditions as would be understood by those skilled in the art (white solid, 23 mg, yield 21%). $^1$H NMR (400 MHz, DMSO) δ 11.47 (s, 1H), 11.01 (s, 1H), 7.89 (s, 1H), 7.83 (d, *J* = 7.8 Hz, 1H), 7.76 (dt, *J* = 11.8, 6.7 Hz, 2H), 6.79 (dd, *J* = 8.2, 2.3 Hz, 1H), 6.40 (dd, *J* = 7.7, 2.5 Hz, 1H), 5.15 (dd, *J* = 13.3, 5.0 Hz, 1H), 4.45 (q, *J* = 17.7 Hz, 4H), 4.31 (d, *J* = 13.4 Hz, 2H), 3.37 (t, *J* = 13.1 Hz, 4H), 3.13 - 3.09 (m, 2H), 3.01 - 2.85 (m, 1H), 2.61 (d, *J* = 17.2 Hz, 1H), 2.43 (dd, *J* = 13.1, 4.4 Hz, 1H), 2.09 - 1.92 (m, 1H). LCMS (ESI) calcd for $C_{23}H_{25}FN_5O_3^+$ [M+H]+: 438.19, found, 438.3.

Example 74: Preparation of 3-(5-((4-(5-fluoropyridin-2-yl)piperazin-1-yl)methyl)-1-oxoisoindolin-2-yl)piperidine-2,6-dione (GT-04531)

Referring to the method of Scheme 1, the target compound (GT-04531) was prepared under appropriate conditions as would be understood by those skilled in the art (white solid, 40 mg, yield 37%). $^1$H NMR (400 MHz, DMSO) δ 11.68 (s, 1H), 11.01 (s, 1H), 8.15 (d, *J*= 3.0 Hz, 1H), 7.93 (s, 1H), 7.86 - 7.76 (m, 2H), 7.66 - 7.57 (m, 1H), 6.99 (dd, *J* = 9.3, 3.4 Hz, 1H), 5.14 (dd, *J* = 13.3, 5.1 Hz, 1H), 4.70 - 4.34 (m, 5H), 4.28 (d, *J* = 13.2 Hz, 2H), 3.39 - 3.32 (m, 4H), 3.12 - 3.05 (m, 2H), 3.01 - 2.82 (m, 1H), 2.61 (d, *J= 17.3* Hz, 1H), 2.47 - 2.35 (m, 1H), 2.05 - 2.01 (m, 1H). LCMS (ESI) calcd for $C_{23}H_{25}FN_5O_3^+$ [M+H]+: 438.19, found, 438.3.

Example 75: Preparation of 3-(1-oxo-5-((4-(3-(trifluoromethyl)pyridin-2-yl)piperazin-1-yl)methyl)isoindolin-2-yl)piperidine-2,6-dione (GT-04434)

Referring to the method of Scheme 1, the target compound (GT-04434) was prepared under appropriate conditions as would be understood by those skilled in the art (white solid, 59 mg, yield 52%). $^1$H NMR (400 MHz, DMSO) δ 11.29 (s, 1H), 11.02 (s, 1H), 8.60 (d, *J* = 3.5 Hz, 1H), 8.15 (dd, *J* = 7.9, 1.6 Hz, 1H), 7.93 (s, 1H), 7.82 (q, *J* = 8.0 Hz, 2H), 7.33 (dd, *J* = 7.5, 5.1 Hz, 1H), 5.15 (dd, *J* = 13.2, 5.1 Hz, 1H), 4.63 - 4.39 (m, 4H), 3.54 - 3.35 (m, 6H), 3.21 (d, *J* = 10.9 Hz, 2H), 2.99 - 2.87 (m, 1H), 2.62 (d, *J* = 17.2 Hz, 1H), 2.44 (dd, *J* = 13.1, 4.4 Hz, 1H), 2.11 - 1.92 (m, 1H). LCMS (ESI) calcd for $C_{24}H_{25}F_3N_5O_3^+$ [M+H]+: 488.19, found, 488.2.

Example 76: Preparation of 3-(1-oxo-5-((4-(6-(trifluoromethyl)pyridin-2-yl)piperazin-1-yl)methyl)isoindolin-2-yl)piperidine-2,6-dione (GT-04432)

Referring to the method of Scheme 1, the target compound (GT-04432) was prepared under appropriate conditions as would be understood by those skilled in the art (white solid, 60 mg, yield 53%). $^1$H NMR (400 MHz, DMSO) δ 11.02 (s, 1H), 10.93 (s, 1H), 7.84 (s, 3H), 7.73 (d, *J* = 7.8 Hz, 1H), 7.20 (dd, *J* = 24.1, 8.0 Hz, 2H), 5.16 (dd, *J* = 13.1, 5.1 Hz, 1H), 4.60 - 4.34 (m, 6H), 3.45 (d, *J* = 12.6 Hz, 2H), 3.37 (s, 1H), 3.33 - 3.29 (m, 1H), 3.15 (s, 2H), 3.00 - 2.89 (m, 1H), 2.62 (d, *J*

= 15.6 Hz, 1H), 2.44 (dd, *J* = 13.6, 4.2 Hz, 1H), 2.08 - 1.99 (m, 1H). LCMS (ESI) calcd for $C_{24}H_{25}F_3N_5O_3^+$ $[M+H]^+$: 488.19, found, 488.2.

Example 77: Preparation of 3-(1-oxo-5-((4-(5-(trifluoromethyl)pyridin-2-yl)piperazin-1-yl)methyl)isoindolin-2-yl)piperidine-2,6-dione (GT-04433)

Referring to the method of Scheme 1, the target compound (GT-04433) was prepared under appropriate conditions as would be understood by those skilled in the art (white solid, 86 mg, yield 76%). $^1H$ NMR (400 MHz, DMSO) δ 11.18 (s, 1H), 11.02 (s, 1H), 8.48 (s, 1H), 7.93 (dd, *J* = 9.2, 2.4 Hz, 1H), 7.85 (d, *J* = 8.0 Hz, 2H), 7.74 (d, *J* = 8.4 Hz, 1H), 7.08 (d, *J* = 9.1 Hz, 1H), 5.15 (dd, *J* = 13.3, 5.1 Hz, 1H), 4.46 (dd, *J* = 51.1, 17.7 Hz, 6H), 3.42 (d, *J* = 11.4 Hz, 4H), 3.13 (s, 2H), 3.00 - 2.85 (m, 1H), 2.66 (d, *J* = 14.8 Hz, 1H), 2.47 - 2.39 (m, 1H), 2.12 - 1.97 (m, 1H). LCMS (ESI) calcd for $C_{24}H_{25}F_3N_5O_3^+$ $[M+H]^+$: 488.19, found, 488.2.

Example 78: Preparation of 3-(5-((4-(3,4-dichloropyridin-2-yl)piperazin-1-yl)methyl)-1-oxoisoindolin-2-yl)piperidine-2,6-dione (GT-04532)

Referring to the method of Scheme 1, the target compound (GT-04532) was prepared under appropriate conditions as would be understood by those skilled in the art (white solid, 19 mg, yield 19%). $^1H$ NMR (400 MHz, DMSO) δ 11.38 (s, 1H), 11.01 (s, 1H), 8.21 (d, *J* = 5.3 Hz, 1H), 7.91 (s, 1H), 7.81 (q, *J* = 7.9 Hz, 2H), 7.38 (d, *J* = 5.3 Hz, 1H), 5.14 (dd, *J* = 13.3, 5.1 Hz, 1H), 4.60 - 4.33 (m, 4H), 3.86 (d, *J* = 13.3 Hz, 2H), 3.39 (dd, *J* = 25.0, 12.5 Hz, 5H), 3.23 (d, *J* = 10.2 Hz, 2H), 3.00 - 2.85 (m, 1H), 2.61 (d, *J* = 17.0 Hz, 1H), 2.43 (dd, *J*= 13.1, 4.4 Hz, 1H), 2.05 - 2.01 (m, 1H). LCMS (ESI) calcd for $C_{23}H_{24}Cl_2N_5O_3^+$ $[M+H]^+$: 488.13, found, 488.2.

Example 79: Preparation of 3-(5-((4-(3,5-dichloropyridin-2-yl)piperazin-1-yl)methyl)-1-oxoisoindolin-2-yl)piperidine-2,6-dione (GT-04479)

Referring to the method of Scheme 1, the target compound (GT-04479) was prepared under appropriate conditions as would be understood by those skilled in the art (white solid, 54 mg, yield 48%). $^1H$ NMR (400 MHz, DMSO) δ 11.01 (s, 1H), 10.95 (s, 1H), 8.33 (d, *J* = 2.1 Hz, 1H), 8.13 (d, *J* = 1.9 Hz, 1H), 7.89 - 7.79 (m, 2H), 7.76 (d, *J* = 7.8 Hz, 1H), 5.14 (dd, *J* = 13.2, 5.0 Hz, 1H), 4.52 (d, *J* = 17.2 Hz, 3H), 4.39 (d, *J* = 17.6 Hz, 1H), 3.85 (d, *J* = 11.8 Hz, 2H), 3.43 (d, *J* = 9.6 Hz, 2H), 3.31 - 3.17 (m, 4H), 3.00 - 2.87 (m, 1H), 2.63 (t, *J* = 15.8 Hz, 1H), 2.46 - 2.36 (m, 1H), 2.05 - 1.97 (m, 1H). LCMS (ESI) calcd for $C_{23}H_{24}Cl_2N_5O_3^+$ $[M+H]^+$: 488.13, found, 488.2.

Example 80: Preparation of 3-(5-((4-(4,5-dichloropyridin-2-yl)piperazin-1-yl)methyl)-1-oxoisoindolin-2-yl)piperidine-2,6-dione (GT-04533)

Referring to the method of Scheme 1, the target compound (GT-04533) was prepared under appropriate conditions as would be understood by those skilled in the art (white solid, 19 mg, yield 19%). $^1H$ NMR (400 MHz, DMSO) δ 11.44 (s, 1H), 11.01 (s, 1H), 8.30 (s, 1H), 7.88 (s, 1H), 7.79 (dd, *J* = 29.5, 7.8 Hz, 2H), 7.29 (s, 1H), 5.14 (dd, *J* = 13.3, 5.1 Hz, 1H), 4.60 - 4.31 (m, 6H), 3.38 (t, *J* = 12.5 Hz, 4H), 3.15 - 3.01 (m, 2H), 3.00 - 2.85 (m, 1H), 2.61 (d, *J* = 17.5 Hz, 1H), 2.43 (dd, *J* = 13.2, 4.5 Hz, 1H), 2.08 - 1.86 (m, 1H). LCMS (ESI) calcd for $C_{23}H_{24}Cl_2N_5O_3^+$ $[M+H]^+$: 488.13, found, 488.2.

Example 81: Preparation of 3-(5-((4-(3,4-difluoropyridin-2-yl)piperazin-1-yl)methyl)-1-oxoisoindolin-2-yl)piperidine-2,6-dione (GT-04579)

Referring to the method of Scheme 1, the target compound (GT-04579) was prepared under appropriate conditions as would be understood by those skilled in the art (white solid, 37 mg, yield 35%). $^1H$ NMR (400 MHz, MeOD) δ 8.03 (t, J = 6.4 Hz, 1H), 7.95 (d, J = 7.9 Hz, 1H), 7.88 (s, 1H), 7.77 (d, J = 7.9 Hz, 1H), 7.01 - 6.96 (m, 1H), 5.20 (dd, J = 13.2, 5.2 Hz, 1H), 4.69 - 4.52 (m, 4H), 4.40 - 4.18 (m, 2H), 3.60 (brs, 2H), 3.51 - 3.38 (m, 4H), 2.99 - 2.90 (m, 1H), 2.85 - 2.79 (m, 1H), 2.60 - 2.49 (m, 1H), 2.24 - 2.19 (m, 1H). LCMS (ESI) calcd for $C_{23}H_{24}F_2N_5O_3^+$ $[M+H]^+$: 456.18, found, 456.2.

Example 82: Preparation of 3-(5-((4-(4-chloro-3-fluoropyridin-2-yl)piperazin-1-yl)methyl)-1-oxoisoindolin-2-yl)piperidine-2,6-dione (GT-04610)

Referring to the method of Scheme 1, the target compound (GT-04610) was prepared under appropriate conditions as would be understood by those skilled in the art (white solid, 45 mg, yield 45%). $^1H$ NMR (400 MHz, MeOD) δ 8.03 (d, *J* = 5.3 Hz, 1H), 7.97 (d, *J* = 7.8 Hz, 1H), 7.88 (s, 1H), 7.79 (d, *J* = 7.8 Hz, 1H), 7.14 (t, *J* = 5.0 Hz, 1H), 5.23 (dd, *J* = 13.3, 5.1 Hz, 1H), 4.57 (d, *J* = 13.5 Hz, 4H), 4.26 (d, *J* = 11.4 Hz, 2H), 3.57 (s, 2H), 3.39 (dd, *J* = 23.5, 11.0 Hz, 4H), 2.98 (ddd,

*J* = 18.7, 11.8, 5.0 Hz, 1H), 2.88 - 2.76 (m, 1H), 2.61 - 2.50 (m, 1H), 2.30 - 2.15 (m, 1H). LCMS (ESI) calcd for $C_{23}H_{24}ClFN_5O_3^+$ $[M+H]^+$: 472.15, found, 472.2.

Example 83: Preparation of 3-(5-((4-(3-fluoro-4-iodopyridin-2-yl)piperazin-1-yl)methyl)-1-oxoisoindolin-2-yl)piperidine-2,6-dione (GT-04557)

Referring to the method of Scheme 1, the target compound (GT-04557) was prepared under appropriate conditions as would be understood by those skilled in the art (white solid, 42 mg, yield 48%). $^1$H NMR (400 MHz, DMSO) δ 11.53 (s, 1H), 11.01 (s, 1H), 7.91 (s, 1H), 7.80 (dd, *J*= 16.9, 7.9 Hz, 2H), 7.72 (d, *J* = 5.1 Hz, 1H), 7.39 (dd, *J* = 5.0, 3.7 Hz, 1H), 5.14 (dd, *J* = 13.3, 5.1 Hz, 1H), 4.51 - 4.36 (m, 4H), 4.04 (d, *J*= 14.0 Hz, 3H), 3.53 - 3.33 (m, 4H), 3.21 - 3.17 (m, 2H), 3.00 - 2.84 (m, 1H), 2.61 (d, *J* = 17.2 Hz, 1H), 2.48 - 2.38 (m, 1H), 2.10 - 1.91 (m, 1H). LCMS (ESI) calcd for $C_{23}H_{24}FIN_5O_3^+$ $[M+H]^+$: 564.09, found, 564.1.

Example 84: Preparation of 3-(5-((4-(3-bromo-4-chloropyridin-2-yl)piperazin-1-yl)methyl)-1-oxoisoindolin-2-yl)piperidine-2,6-dione (GT-04615)

Referring to the method of Scheme 1, the target compound (GT-04615) was prepared under appropriate conditions as would be understood by those skilled in the art (white solid, 47 mg, yield 52%). $^1$H NMR (400 MHz, MeOD) δ 8.23 (d, *J* = 5.3 Hz, 1H), 7.97 (d, *J* = 7.8 Hz, 1H), 7.86 (s, 1H), 7.78 (d, *J* = 8.1 Hz, 1H), 7.32 (d, *J* = 5.2 Hz, 1H), 5.22 (dd, *J* = 13.4, 5.1 Hz, 1H), 4.67 - 4.51 (m, 4H), 3.99 (*d, J* = 13.9 Hz, 2H), 3.61 (d, *J* = 11.9 Hz, 2H), 3.44 (t, *J* = 11.2 Hz, 2H), 3.28 (s, 2H), 3.04 - 2.90 (m, 1H), 2.85 - 2.74 (m, 1H), 2.55 (qd, *J* = 13.4, 4.9 Hz, 1H), 2.28 - 2.11 (m, 1H). LCMS (ESI) calcd for $C_{23}H_{24}BrClN_5O_3^+$ $[M+H]^+$: 532.07, found, 532.1.

Example 85: Preparation of 3-(5-((4-(3-chloro-5-(trifluoromethyl)pyridin-2-yl)piperazin-1-yl)methyl)-1-oxoisoindolin-2-yl)piperidine-2,6-dione (GT-04480)

Referring to the method of Scheme 1, the target compound (GT-04480) was prepared under appropriate conditions as would be understood by those skilled in the art (white solid, 54 mg, yield 51%). $^1$H NMR (400 MHz, DMSO) δ 11.17 (s, 1H), 11.01 (s, 1H), 8.62 (s, 1H), 8.29 (s, 1H), 7.90 - 7.80 (m, 2H), 7.76 (d, *J* = *7.5* Hz, 1H), 5.15 (dd, *J* = 13.3, 5.1 Hz, 1H), 4.52 (d, *J* = 17.2 Hz, 3H), 4.39 (d, *J* = 17.5 Hz, 1H), 4.11 (d, *J* = 13.2 Hz, 2H), 3.43 (t, *J*= 12.2 Hz, 4H), 3.25 (s, 2H), 2.99 - 2.87 (m, 1H), 2.61 (d, *J*= 17.6 Hz, 1H), 2.48 - 2.35 (m, 1H), 2.06 - 1.98 (m, 1H). LCMS (ESI) calcd for $C_{24}H_{24}ClF_3N_5O_3^+$ $[M+H]^+$: 522.15, found, 522.2.

Example 86: Preparation of 3-(5-((4-(4-chloro-6-methylpyridin-2-yl)piperazin-1-yl)methyl)-1-oxoisoindolin-2-yl)piperidine-2,6-dione (GT-04491)

Referring to the method of Scheme 1, the target compound (GT-04491) was prepared under appropriate conditions as would be understood by those skilled in the art (white solid, 69 mg, yield 62%). $^1$H NMR (400 MHz, DMSO) δ 11.67 (s, 1H), 11.01 (s, 1H), 7.90 (s, 1H), 7.82 *(d, J*= 7.8 Hz, 1H), 7.78 (d, *J* = 7.9 Hz, 1H), 6.91 (s, 1H), 6.75 (s, 1H), 5.15 (dd, *J* = 13.3, 5.1 Hz, 1H), 4.57 - 4.38 (m, 6H), 3.43 - 3.35 (m, 4H), 3.11 - 3.04 (m, 2H), 3.01 - 2.85 (m, 1H), 2.61 (d, *J* = 16.9 Hz, 1H), 2.43 (dd, *J* = 13.0, 4.5 Hz, 1H), 2.34 (s, 3H), 2.05 - 2.01 (m, 1H). LCMS (ESI) calcd for $C_{24}H_{27}ClN_5O_3^+$ $[M+H]^+$: 468.18, found, 468.2.

Example 87: Preparation of 3-(1-oxo-5-((4-(pyridin-3-yl)piperazin-1-yl)methyl)isoindolin-2-yl)piperidine-2,6-dione (GT-04508)

Referring to the method of Scheme 1, the target compound (GT-04508) was prepared under appropriate conditions as would be understood by those skilled in the art (white solid, 45 mg, yield 22%). $^1$H NMR (400 MHz, DMSO) δ 11.97 (s, 1H), 11.01 (s, 1H), 8.53 (d, *J* = 2.6 Hz, 1H), 8.26 (d, *J* = 5.3 Hz, 1H), 8.08 (dd, *J* = 8.9, 2.3 Hz, 1H), 7.93 (s, 1H), 7.90 - 7.76 (m, 3H), 5.15 (dd, *J* = 13.2, 5.0 Hz, 1H), 4.58 - 4.34 (m, 4H), 4.10 (brs, 2H), 3.66 - 3.57 (m, 2H), 3.23 (brs, 2H), 3.18 - 3.06 (m, 2H), 2.99 - 2.86 (m, 1H), 2.62 (d, *J* = 16.7 Hz, 1H), 2.44 (dd, *J* = 13.1, 4.4 Hz, 1H), 2.08 - 1.95 (m, 1H). LCMS (ESI) calcd for $C_{23}H_{26}N_5O_3^+$ $[M+H]^+$: 420.20, found, 420.3.

Example 88: Preparation of 3-(5-((4-(6-chloropyridin-3-yl)piperazin-1-yl)methyl)-1-oxoisoindolin-2-yl)piperidine-2,6-dione (GT-04537)

Referring to the method of Scheme 1, the target compound (GT-04537) was prepared under appropriate conditions as would be understood by those skilled in the art (white solid, 25 mg, yield 24%). $^1$H NMR (400 MHz, DMSO) δ

11.44 (s, 1H), 11.01 (s, 1H), 8.13 (d, *J* = 3.1 Hz, 1H), 7.90 (s, 1H), 7.81 (dd, *J* = 22.9, 7.9 Hz, 2H), 7.48 (dd, *J* = 8.9, 3.2 Hz, 1H), 7.36 (d, *J* = 8.8 Hz, 1H), 5.15 (dd, *J* = 13.3, 5.1 Hz, 1H), 4.68 - 4.34 (m, 4H), 3.89 (d, *J* = 12.7 Hz, 2H), 3.39 (d, *J* = 11.3 Hz, 2H), 3.22 (dt, *J* = 19.7, 12.6 Hz, 4H), 3.01 - 2.85 (m, 1H), 2.61 (d, *J* = 17.1 Hz, 1H), 2.43 (dd, *J* = 13.2, 4.6 Hz, 1H), 2.05 - 2.01 (m, 1H). LCMS (ESI) calcd for $C_{23}H_{25}ClN_5O_3^+$ [M+H]$^+$: 454.16, found, 454.2.

Example 89: Preparation of 3-(5-((4-(4,5-dichloropyridin-3-yl)piperazin-1-yl)methyl)-1-oxoisoindolin-2-yl)piperidine-2,6-dione (GT-04538)

Referring to the method of Scheme 1, the target compound (GT-04538) was prepared under appropriate conditions as would be understood by those skilled in the art (white solid, 50 mg, yield 51%). $^1$H NMR (400 MHz, DMSO) δ 11.87 (s, 1H), 11.01 (s, 1H), 8.52 (s, 1H), 8.40 (s, 1H), 7.97 (s, 1H), 7.84 (q, *J* = 7.9 Hz, 2H), 5.15 (dd, *J* = 13.2, 5.1 Hz, 1H), 4.61 - 4.48 (m, 4H), 3.55 (d, *J* = 12.4 Hz, 2H), 3.42 (t, *J* = 10.7 Hz, 4H), 3.26 (s, 2H), 3.00 - 2.85 (m, 1H), 2.62 (d, *J* = 17.3 Hz, 1H), 2.47 - 2.39 (m, 1H), 2.10 - 1.92 (m, 1H). LCMS (ESI) calcd for $C_{23}H_{24}Cl_2N_5O_3^+$ [M+H]$^+$: 488.13, found, 488.1.

Example 90: Preparation of 3-(5-((4-(2,4-dichloropyridin-3-yl)piperazin-1-yl)methyl)-1-oxoisoindolin-2-yl)piperidine-2,6-dione (GT-04896)

Referring to the method of Scheme 1, the target compound (GT-04896) was prepared under appropriate conditions as would be understood by those skilled in the art (white solid, 30 mg, yield 40%). $^1$H NMR (400 MHz, DMSO) δ 7.36 (s, 1H), 7.13 (d, *J* = 8.3 Hz, 1H), 7.05 (s, 1H), 6.97 (s, 1H), 6.73 (s, 1H), 4.41 - 4.29 (m, 2H), 3.92 - 3.76 (m, 4H), 3.02 - 2.99 (m, 2H), 2.77 (d, *J* = 12.0 Hz, 2H), 2.69 - 2.60 (m, 2H), 2.21 - 1.98 (m, 1H), 1.78 - 1.56 (m, 2H), 1.51 - 1.39 (m, 1H). LCMS (ESI) calcd for $C_{23}H_{24}Cl_2N_5O_3^+$ [M+H]$^+$: 488.13, found, 488.1.

Example 91: Preparation of 3-(1-oxo-5-((4-(pyridin-4-yl)piperazin-1-yl)methyl)isoindolin-2-yl)piperidine-2,6-dione (GT-04450)

Referring to the method of Scheme 1, the target compound (GT-04450) was prepared under appropriate conditions as would be understood by those skilled in the art (white solid, 36 mg, yield 29%). $^1$H NMR (400 MHz, $CDCl_3$) δ 11.01 (s, 1H), 8.35 (d, *J* = 7.3 Hz, 2H), 7.90 (s, 1H), 7.82 (d, *J* = 7.7 Hz, 1H), 7.76 (d, *J* = 7.5 Hz, 1H), 7.25 (d, *J* = 7.6 Hz, 2H), 5.14 (dd, *J* = 13.3, 5.1 Hz, 1H), 4.54 - 4.35 (m, 6H), 3.71 (brs, 2H), 3.44 (brs, 2H), 3.16 (brs, 2H), 3.01 - 2.89 (m, 1H), 2.61 (d, *J* = 17.3 Hz, 1H), 2.48 - 2.40 (m, 1H), 2.05 - 2.01 (m, 1H). LCMS (ESI) calcd for $C_{23}H_{26}N_5O_3^+$ [M+H]$^+$: 420.20, found, 420.2.

Example 92: Preparation of 3-(5-((4-(2-methylpyridin-4-yl)piperazin-1-yl)methyl)-1-oxoisoindolin-2-yl)piperidine-2,6-dione (GT-04541)

Referring to the method of Scheme 1, the target compound (GT-04541) was prepared under appropriate conditions as would be understood by those skilled in the art (white solid, 20 mg, yield 18%). $^1$H NMR (400 MHz, DMSO) δ 14.23 (s, 1H), 12.36 (s, 1H), 11.01 (s, 1H), 8.23 (d, *J* = 7.1 Hz, 1H), 7.92 (s, 1H), 7.80 (dd, *J* = 13.9, 7.3 Hz, 2H), 7.19 (s, 1H), 7.13 (d, *J* = 7.4 Hz, 1H), 5.14 (dd, *J* = 13.2, 4.9 Hz, 1H), 4.44 (dd, *J* = 52.6, 17.4 Hz, 7H), 3.72 (s, 4H), 3.20 (s, 2H), 3.02 - 2.83 (m, 2H), 2.61 (d, *J* = 16.9 Hz, 2H), 2.46 - 2.37 (m, 1H), 2.09 - 1.90 (m, 1H). LCMS (ESI) calcd for $C_{24}H_{28}N_5O_3^+$ [M+H]$^+$: 434.22, found, 434.3.

Example 93: Preparation of 3-(5-((4-(3-bromopyridin-4-yl)piperazin-1-yl)methyl)-1-oxoisoindolin-2-yl)piperidine-2,6-dione (GT-04580)

Referring to the method of Scheme 1, the target compound (GT-04580) was prepared under appropriate conditions as would be understood by those skilled in the art (white solid, 20 mg, yield 21%). $^1$H NMR (400 MHz, MeOD) δ 8.86 (d, J = 1.1 Hz, 1H), 8.54 (dd, J = 6.9, 1.2 Hz, 1H), 8.00 - 7.91 (m, 2H), 7.80 (d, J = 7.8 Hz, 1H), 7.54 (d, J = 6.9 Hz, 1H), 5.21 (dd, J = 13.3, 5.2 Hz, 1H), 4.70 - 4.52 (m, 4H), 4.36 (brs, 2H), 3.62 (brs, 6H), 3.02 - 2.83 (m, 1H), 2.83 - 2.79 (m, 1H), 2.60 - 2.49 (m, 1H), 2.24 - 2.19 (m, 1H). LCMS (ESI) calcd for $C_{23}H_{25}BrN_5O_3^+$ [M+H]$^+$: 498.11, found, 498.1.

Example 94: Preparation of 3-(5-((4-(3-fluoropyridin-4-yl)piperazin-1-yl)methyl)-1-oxoisoindolin-2-yl)piperidine-2,6-dione (GT-04581)

Referring to the method of Scheme 1, the target compound (GT-04581) was prepared under appropriate conditions as would be understood by those skilled in the art (white solid, 21 mg, yield 19%). $^1$H NMR (400 MHz, MeOD) δ 8.62 (dd, J = 8.5, 1.1 Hz, 1H), 8.32 (dd, J = 7.1, 1.1 Hz, 1H), 7.96 (d, J = 7.8 Hz, 1H), 7.92 (s, 1H), 7.78 (d, J = 8.9 Hz, 1H), 7.49 (dd, J = 8.7, 7.1 Hz, 1H), 5.21 (dd, J = 13.3, 5.1 Hz, 1H), 4.70 - 4.51 (m, 4H), 4.16 - 3.83 (m, 4H), 3.59 (s, 4H), 3.02 - 2.85 (m,

1H), 2.84 - 2.79 (m, 1H), 2.60 - 2.49 (m, 1H), 2.22 - 2.19 (m, 1H). LCMS (ESI) calcd for $C_{23}H_{25}FN_5O_3^+$ [M+H]$^+$: 438.19, found, 438.2.

Example 95: Preparation of 3-(5-((4-(3-chloropyridin-4-yl)piperazin-1-yl)methyl)-1-oxoisoindolin-2-yl)piperidine-2,6-dione (GT-04481)

Referring to the method of Scheme 1, the target compound (GT-04481) was prepared under appropriate conditions as would be understood by those skilled in the art (white solid, 30 mg, yield 24%). $^1$H NMR (400 MHz, DMSO) δ 12.41 (s, 1H), 11.01 (s, 1H), 8.81 (s, 1H), 8.53 (d, *J* = 6.8 Hz, 1H), 7.97 (s, 1H), 7.88 - 7.72 (m, 2H), 7.51 (d, *J* = 6.8 Hz, 1H), 5.15 (dd, *J* = 13.3, 5.1 Hz, 1H), 4.56 (s, 2H), 4.49 (s, 1H), 4.38 (d, *J* = 17.6 Hz, 1H), 4.18 (s, 2H), 3.77 (s, 2H), 3.44 (s, 2H), 3.31 (s, 2H), 2.99 - 2.88 (m, 1H), 2.61 (d, *J* = 16.9 Hz, 1H), 2.43 (tt, *J* = 13.2, 6.7 Hz, 1H), 2.07 - 1.98 (m, 1H). LCMS (ESI) calcd for $C_{23}H_{25}ClN_5O_3^+$ [M+H]$^+$: 454.16, found, 454.1.

Example 96: Preparation of 3-(5-((4-(2-chloropyridin-4-yl)piperazin-1-yl)methyl)-1-oxoisoindolin-2-yl)piperidine-2,6-dione (GT-04435)

Referring to the method of Scheme 1, the target compound (GT-04435) was prepared under appropriate conditions as would be understood by those skilled in the art (white solid, 72 mg, yield 58%). $^1$H NMR (400 MHz, DMSO) δ 12.12 (s, 1H), 11.02 (s, 1H), 8.12 (d, *J* = 6.5 Hz, 1H), 7.92 (s, 1H), 7.81 (q, *J* = 7.8 Hz, 2H), 7.15 (d, *J* = 2.3 Hz, 1H), 7.02 (dd, *J* = 6.6, 2.4 Hz, 1H), 5.15 (dd, *J* = 13.3, 5.1 Hz, 1H), 4.50 (s, 2H), 4.40 (s, 2H), 4.24 (d, *J* = 12.6 Hz, 2H), 3.56 (s, 2H), 3.36 (s, 2H), 3.27 - 3.09 (m, 2H), 2.93 (ddd, *J* = 17.4, 9.5, 4.2 Hz, 1H), 2.61 (d, *J*= 16.7 Hz, 1H), 2.47 - 2.34 (m, 1H), 2.02 (dd, *J* = 9.0, 3.5 Hz, 1H). LCMS (ESI) calcd for $C_{23}H_{25}ClN_5O_3^+$ [M+H]$^+$: 454.16, found, 454.2.

Example 97: Preparation of 3-(5-((4-(2-chloro-3-fluoropyridin-4-yl)piperazin-1-yl)methyl)-1-oxoisoindolin-2-yl)piperidine-2,6-dione (GT-04612)

Referring to the method of Scheme 1, the target compound (GT-04612) was prepared under appropriate conditions as would be understood by those skilled in the art (white solid, 47 mg, yield 47%). $^1$H NMR (400 MHz, MeOD) δ 8.02 (d, *J* = 5.8 Hz, 1H), 7.92 (t, *J* = 8.4 Hz, 1H), 7.86 (s, 1H), 7.76 (d, *J* = 7.9 Hz, 1H), 7.09 (t, *J* = 6.2 Hz, 1H), 5.18 (dd, *J* = 13.3, 5.1 Hz, 1H), 4.66 - 4.48 (m, 4H), 4.01 (s, 2H), 3.44 (t, *J* = 47.5 Hz, 6H), 2.92 (ddd, *J* = 18.5, 13.5, 5.3 Hz, 1H), 2.84 - 2.69 (m, 1H), 2.51 (tt, *J* = 13.3, 6.5 Hz, 1H), 2.23 - 2.11 (m, 1H). LCMS (ESI) calcd for $C_{23}H_{24}ClFN_5O_3^+$ [M+H]$^+$: 472.15, found, 472.1.

Example 98: Preparation of 3-(5-((4-(2-bromo-3-fluoropyridin-4-yl)piperazin-1-yl)methyl)-1-oxoisoindolin-2-yl)piperidine-2,6-dione (GT-04928)

Referring to the method of Scheme 1, the target compound (GT-04928) was prepared under appropriate conditions as would be understood by those skilled in the art (white solid, 24 mg, yield 31%). $^1$H NMR (400 MHz, MeOD-d4) δ 8.07 (d, *J* = 6.2 Hz, 1H), 7.92 (t, *J* = 7.8 Hz, 1H), 7.87 (s, 1H), 7.75 (d, *J* = 6.7 Hz, 1H), 7.19 (t, *J* = 6.6 Hz, 1H), 5.18 (dd, *J* = 13.3, 5.1 Hz, 1H), 4.68 - 4.52 (m, 4H), 4.15 (brs, 2H), 3.75 - 3.37 (m, 6H), 2.97 - 2.88 (m, 1H), 2.85 - 2.73 (m, 1H), 2.58 - 2.47 (m, 1H), 2.26 - 2.12 (m, 1H). LCMS (ESI) calcd for $C_{23}H_{24}BrFN_5O_3^+$ [M+H]$^+$: 516.10, found, 516.1.

Example 99: Preparation of 3-(5-((4-(3-bromo-2-chloropyridin-4-yl)piperazin-1-yl)methyl)-1-oxoisoindolin-2-yl)piperidine-2,6-dione (GT-04616)

Referring to the method of Scheme 1, the target compound (GT-04616) was prepared under appropriate conditions as would be understood by those skilled in the art (white solid, 46 mg, yield 51%). $^1$H NMR (400 MHz, MeOD) δ 8.25 (d, *J* = 5.5 Hz, 1H), 7.96 (d, *J* = 7.8 Hz, 1H), 7.88 (s, 1H), 7.78 (d, *J* = 7.9 Hz, 1H), 7.13 (d, *J* = 5.5 Hz, 1H), 5.21 (dd, *J* = 13.3, 5.1 Hz, 1H), 4.71 - 4.51 (m, 4H), 3.84 (d, *J* = 13.2 Hz, 2H), 3.66 (d, *J* = 11.5 Hz, 2H), 3.47 (t, *J* = 11.0 Hz, 2H), 3.28 (d, *J* = 12.0 Hz, 2H), 2.94 (ddd, *J* = 18.4, 13.4, 5.3 Hz, 1H), 2.88 - 2.76 (m, 1H), 2.55 (qd, *J* = 13.2, 4.7 Hz, 1H), 2.29 - 2.15 (m, 1H). LCMS (ESI) calcd for $C_{23}H_{24}BrClN_5O_3^+$ [M+H]$^+$: 532.07, found, 532.1.

Example 100: Preparation of 3-(5-((4-(2,3-dichloropyridin-4-yl)piperazin-1-yl)methyl)-1-oxoisoindolin-2-yl)piperidine-2,6-dione (GT-04542)

Referring to the method of Scheme 1, the target compound (GT-04542) was prepared under appropriate conditions as would be understood by those skilled in the art (white solid, 65 mg, yield 67%). $^1$H NMR (400 MHz, DMSO) δ 11.93 (s, 1H), 11.01 (s, 1H), 8.22 (d, *J* = 5.5 Hz, 1H), 7.96 (s, 1H), 7.83 (s, 2H), 7.20 (d, *J* = 5.5 Hz, 1H), 5.15 (dd, *J* = 13.2, 5.1

Hz, 1H), 4.54 - 4.37 (m, 4H), 3.71 (d, *J* = 12.4 Hz, 2H), 3.41 (t, *J* = 12.5 Hz, 4H), 3.27 - 3.22 (m, 2H), 2.98 - 2.92 (m, 1H), 2.62 (d, *J* = 16.7 Hz, 1H), 2.48 - 2.35 (m, 1H), 2.05 - 2.01 (m, 1H). LCMS (ESI) calcd for $C_{23}H_{24}Cl_2N_5O_3^+$ $[M+H]^+$: 488.13, found, 488.1.

Example 101: Preparation of 3-(5-((4-(2,3-difluoropyridin-4-yl)piperazin-1-yl)methyl)-1-oxoisoindolin-2-yl)piperidine-2,6-dione (GT-04543)

Referring to the method of Scheme 1, the target compound (GT-04543) was prepared under appropriate conditions as would be understood by those skilled in the art (white solid, 62 mg, yield 59%). $^1$H NMR (400 MHz, DMSO) δ 11.90 (s, 1H), 11.02 (s, 1H), 7.94 (s, 1H), 7.82 (s, 2H), 7.80 (d, *J* = 5.7 Hz, 1H), 7.03 (t, *J* = 6.0 Hz, 1H), 5.15 (dd, *J* = 13.3, 5.1 Hz, 1H), 4.66 - 4.35 (m, 4H), 4.01 - 3.84 (m, 2H), 3.65 - 3.46 (m, 2H), 3.38 (brs, 2H), 3.25 (brs, 2H), 3.01 - 2.82 (m, 1H), 2.62 (d, *J* = 17.1 Hz, 1H), 2.48 - 2.40 (m, 1H), 2.09 - 1.90 (m, 1H). LCMS (ESI) calcd for $C_{23}H_{24}F_2N_5O_3^+$ $[M+H]^+$: 456.18, found, 456.2.

Example 102: Preparation of 3-(5-((4-(3-fluoro-2-hydroxypyridin-4-yl)piperazin-1-yl)methyl)-1-oxoisoindolin-2-yl)piperidine-2,6-dione (GT-04929)

Referring to the method of Scheme 1, the target compound (GT-04929) was prepared under appropriate conditions as would be understood by those skilled in the art (white solid, 14 mg, yield 20%). $^1$H NMR (400 MHz, MeOD) LCMS (ESI) calcd for $C_{23}H_{25}FN_5O_4^+$ $[M+H]^+$: 454.19, found, 454.2.

Example 103: Preparation of 3-(5-((4-(3-bromo-5-chloropyridin-4-yl)piperazin-1-yl)methyl)-1-oxoisoindolin-2-yl)piperidine-2,6-dione (GT-04600)

Referring to the method of Scheme 1, the target compound (GT-04600) was prepared under appropriate conditions as would be understood by those skilled in the art (white solid, 40 mg, yield 44%). $^1$H NMR (400 MHz, MeOD) δ 8.78 (s, 1H), 8.68 (s, 1H), 7.94 (d, J = 7.9 Hz, 1H), 7.89 (s, 1H), 7.78 (d, J = 8.1 Hz, 1H), 5.19 (dd, J = 13.2, 5.2 Hz, 1H), 4.69 - 4.55 (m, 4H), 3.89 - 3.76 (m, 4H), 3.64 - 3.61 (m, 2H), 3.51 - 3.43 (m, 2H), 3.02 - 2.85 (m, 1H), 2.84 - 2.74 (m, 1H), 2.58 - 2.48 (m, 1H), 2.29 - 2.09 (m, 1H). LCMS (ESI) calcd for $C_{23}H_{24}BrClN_5O_3^+$ $[M+H]^+$: 532.07, found, 532.1.

Example 104: Preparation of 3-(5-((4-(3-chloro-5-fluoropyridin-4-yl)piperazin-1-yl)methyl)-1-oxoisoindolin-2-yl)piperidine-2,6-dione (GT-04611)

Referring to the method of Scheme 1, the target compound (GT-04611) was prepared under appropriate conditions as would be understood by those skilled in the art (white solid, 25 mg, yield 25%). $^1$H NMR (400 MHz, MeOD) δ 8.77 - 8.58 (m, 2H), 7.94 (q, *J* = 8.2 Hz, 2H), 7.79 (d, *J* = 8.0 Hz, 1H), 5.20 (dt, *J* = 13.4, 6.7 Hz, 1H), 4.67 - 4.53 (m, 4H), 4.09 (s, 2H), 3.85 (s, 2H), 3.65 (s, 2H), 3.49 (d, *J* = 9.3 Hz, 2H), 3.02 - 2.88 (m, 1H), 2.88 - 2.74 (m, 1H), 2.55 (dt, *J* = 13.2, 8.5 Hz, 1H), 2.28 - 2.14 (m, 1H). LCMS (ESI) calcd for $C_{23}H_{24}ClFN_5O_3^+$ $[M+H]^+$: 472.15, found, 472.1.

Example 105: Preparation of 3-(5-((4-(3,5-dichloropyridin-4-yl)piperazin-1-yl)methyl)-1-oxoisoindolin-2-yl)piperidine-2,6-dione (GT-04436)

Referring to the method of Scheme 1, the target compound (GT-04436) was prepared under appropriate conditions as would be understood by those skilled in the art (white solid, 45 mg, yield 40%). $^1$H NMR (400 MHz, DMSO) δ 11.81 (s, 1H), 10.99 (d, *J* = 12.5 Hz, 1H), 8.52 (s, 2H), 7.98 (s, 1H), 7.84 (q, *J* = 8.0 Hz, 2H), 5.15 (dd, *J* = 13.3, 5.1 Hz, 1H), 4.53 (d, *J* = 20.8 Hz, 3H), 4.40 (t, *J* = 12.1 Hz, 1H), 3.84 (t, *J* = 12.3 Hz, 2H), 3.43 (dd, *J* = 26.7, 12.2 Hz, 4H), 3.20 (d, *J* = 10.2 Hz, 2H), 2.96 - 2.88 (m, 1H), 2.61 (d, *J* = 17.1 Hz, 1H), 2.47 - 2.37 (m, 1H), 2.05 - 1.98 (m, 1H). LCMS (ESI) calcd for $C_{23}H_{24}Cl_2N_5O_3^+$ $[M+H]^+$: 488.13, found, 488.1.

Example 106: Preparation of 3-(5-((4-(2,6-dimethylpyridin-4-yl)piperazin-1-yl)methyl)-1-oxoisoindolin-2-yl)piperidine-2,6-dione (GT-04492)

Referring to the method of Scheme 1, the target compound (GT-04492) was prepared under appropriate conditions as would be understood by those skilled in the art (white solid, 43 mg, yield 34%). $^1$H NMR (400 MHz, DMSO) δ 13.92 (s, 1H), 12.29 (s, 1H), 11.01 (s, 1H), 7.92 (s, 1H), 7.82 (d, *J* = 7.7 Hz, 1H), 7.79 - 7.71 (m, 1H), 7.05 (s, 2H), 5.15 (dd, *J* = 13.3, 5.1 Hz, 1H), 4.57 - 4.31 (m, 6H), 3.71 (brs, 2H), 3.42 (brs, 2H), 3.16 (brs, 2H), 3.00 - 2.81 (m, 1H), 2.61 (d, *J* = 17.1 Hz, 1H), 2.48 (s, 6H), 2.45 - 2.41 (m, 1H), 2.08 - 1.91 (m, 1H). LCMS (ESI) calcd for $C_{25}H_{30}N_5O_3^+$ $[M+H]^+$: 448.23, found, 448.3.

Example 107: Preparation of 3-(1-oxo-5-((4-(perfluoropyridin-4-yl)piperazin-1-yl)methyl)isoindolin-2-yl)piperidine-2,6-dione (GT-04499)

Referring to the method of Scheme 1, the target compound (GT-04499) was prepared under appropriate conditions as would be understood by those skilled in the art (white solid, 41 mg, yield 42%). $^1$H NMR (400 MHz, DMSO) δ 11.80 (s, 1H), 11.01 (s, 1H), 7.92 (s, 1H), 7.81 (q, *J* = 7.9 Hz, 2H), 5.14 (dd, *J* = 13.3, 5.1 Hz, 1H), 4.60 - 4.33 (m, 4H), 3.87 - 3.76 (m, 4H), 3.41 (d, *J* = 10.9 Hz, 2H), 3.23 (brs, 2H), 3.01 - 2.83 (m, 1H), 2.61 (d, *J* = 17.7 Hz, 1H), 2.44 (dd, *J* = 13.1, 4.4 Hz, 1H), 2.10 - 1.91 (m, 1H). LCMS (ESI) calcd for $C_{23}H_{22}F_4N_5O_3^+$ [M+H]$^+$: 492.17, found, 492.2.

Example 108: Preparation of 3-(1-oxo-5-((4-(pyridazin-3-yl)piperazin-1-yl)methyl)isoindolin-2-yl)piperidine-2,6-dione (GT-04473)

Referring to the method of Scheme 1, the target compound (GT-04473) was prepared under appropriate conditions as would be understood by those skilled in the art (white solid, 6 mg, yield 4%). $^1$H NMR (400 MHz, DMSO) δ 11.67 (s, 1H), 11.01 (s, 1H), 8.80 (d, *J* = 4.5 Hz, 1H), 7.89 (s, 1H), 7.87 - 7.79 (m, 2H), 7.76 (t, *J* = 8.8 Hz, 2H), 5.15 (dd, *J* = 13.2, 5.1 Hz, 1H), 4.52 (d, *J* = 14.8 Hz, 5H), 4.39 (d, *J* = 17.6 Hz, 1H), 3.50 (d, *J* = 36.9 Hz, 4H), 3.18 (s, 2H), 2.97 - 2.88 (m, 1H), 2.61 (d, *J* = 17.4 Hz, 1H), 2.47 - 2.32 (m, 1H), 2.06 - 1.98 (m, 1H). LCMS (ESI) calcd for $C_{22}H_{25}N_6O_3^+$ [M+H]$^+$: 421.20, found, 421.2.

Example 109: Preparation of 3-(5-((4-(6-chloropyridazin-3-yl)piperazin-1-yl)methyl)-1-oxoisoindolin-2-yl)piperidine-2,6-dione (GT-04482)

Referring to the method of Scheme 1, the target compound (GT-04482) was prepared under appropriate conditions as would be understood by those skilled in the art (white solid, 53 mg, yield 43%). $^1$H NMR (400 MHz, DMSO) δ 11.37 (s, 1H), 10.99 (d, *J* = 13.7 Hz, 1H), 7.89 - 7.82 (m, 2H), 7.75 (d, *J* = 7.8 Hz, 1H), 7.66 (d, *J* = 9.6 Hz, 1H), 7.48 (d, *J* = 9.6 Hz, 1H), 5.15 (dd, *J* = 13.2, 5.1 Hz, 1H), 4.55 - 4.34 (m, 6H), 3.50 - 3.38 (m, 4H), 3.16 (s, 2H), 2.99 - 2.85 (m, 1H), 2.63 (t, *J* = 15.5 Hz, 1H), 2.47 - 2.36 (m, 1H), 2.07 - 1.98 (m, 1H). LCMS (ESI) calcd for $C_{22}H_{24}ClN_6O_3^+$ [M+H]$^+$: 455.16, found, 455.2.

Example 110: Preparation of 3-(1-oxo-5-((4-(pyrimidin-2-yl)piperazin-1-yl)methyl)isoindolin-2-yl)piperidine-2,6-dione (GT-04484)

Referring to the method of Scheme 1, the target compound (GT-04484) was prepared under appropriate conditions as would be understood by those skilled in the art (white solid, 57 mg, yield 41%). $^1$H NMR (400 MHz, DMSO) δ 11.65 (s, 1H), 11.01 (s, 1H), 8.44 (d, *J* = 4.8 Hz, 2H), 7.91 (s, 1H), 7.80 (dd, *J* = 17.4, 7.9 Hz, 2H), 6.77 (t, *J* = 4.8 Hz, 1H), 5.14 (dd, *J* = 13.3, 5.1 Hz, 1H), 4.69 (d, *J* = 13.9 Hz, 2H), 4.48 (dd, *J* = 29.4, 22.4 Hz, 4H), 3.43 (dd, *J* = 24.5, 12.2 Hz, 4H), 3.08 (d, *J* = 10.1 Hz, 2H), 3.00 - 2.83 (m, 1H), 2.61 (d, *J* = 17.3 Hz, 1H), 2.44 (dd, *J* = 13.2, 4.4 Hz, 1H), 2.09 - 1.94 (m, 1H). LCMS (ESI) calcd for $C_{22}H_{25}N_6O_3^+$ [M+H]$^+$: 421.20, found, 421.2.

Example 111: Preparation of 3-(1-oxo-5-((4-(pyrazin-2-yl)piperazin-1-yl)methyl)isoindolin-2-yl)piperidine-2,6-dione (GT-04483)

Referring to the method of Scheme 1, the target compound (GT-04483) was prepared under appropriate conditions as would be understood by those skilled in the art (white solid, 75 mg, yield 54%). $^1$H NMR (400 MHz, DMSO) δ 11.99 (s, 1H), 11.01 (s, 1H), 8.43 (s, 1H), 8.20 (d, *J* = 1.3 Hz, 1H), 7.99 - 7.87 (m, 2H), 7.80 (d, *J* = 8.0 Hz, 2H), 5.13 (d, *J* = 5.1 Hz, 1H), 4.50 (t, *J* = 14.5 Hz, 4H), 4.40 (t, *J* = 13.4 Hz, 2H), 3.49 (t, *J* = 12.7 Hz, 2H), 3.40 (d, *J* = 11.8 Hz, 2H), 3.13 (d, *J*= 9.3 Hz, 2H), 2.99 - 2.87 (m, 1H), 2.61 (d, *J* = 17.1 Hz, 1H), 2.48 - 2.35 (m, 1H), 2.08 - 1.94 (m, 1H). LCMS (ESI) calcd for $C_{22}H_{25}N_6O_3^+$ [M+H]$^+$: 421.20, found, 421.2.

Example 112: Preparation of 3-(5-((4-(5,6-dichloropyrimidin-4-yl)piperazin-1-yl)methyl)-1-oxoisoindolin-2-yl)piperidine-2,6-dione (GT-04501)

Referring to the method of Scheme 1, the target compound (GT-04501) was prepared under appropriate conditions as would be understood by those skilled in the art (white solid, 41 mg, yield 42%). $^1$H NMR (400 MHz, DMSO) δ 11.65 (s, 1H), 11.01 (s, 1H), 8.52 (s, 1H), 7.89 (s, 1H), 7.83 (d, *J* = 7.8 Hz, 1H), 7.77 (d, *J* = 7.9 Hz, 1H), 5.14 (dd, *J* = 13.3, 5.1 Hz, 1H), 4.53 - 4.36 (m, 6H), 3.57 (t, *J* = 12.9 Hz, 2H), 3.44 - 3.39 (m, 2H), 3.20 (brs, 2H), 3.03 - 2.82 (m, 1H), 2.61 (d, *J* = 16.8 Hz, 1H), 2.43 (dd, *J* = 13.1, 4.4 Hz, 1H), 2.10 - 1.91 (m, 1H). LCMS (ESI) calcd for $C_{22}H_{23}Cl_2N_6O_3^+$ [M+H]$^+$: 489.12, found, 489.2.

Example 113: Preparation of 3-(5-((4-(3,6-dichloropyridazin-4-yl)piperazin-1-yl)methyl)-1-oxoisoindolin-2-yl)piperidine-2,6-dione (GT-04500)

Referring to the method of Scheme 1, the target compound (GT-04500) was prepared under appropriate conditions as would be understood by those skilled in the art (white solid, 41 mg, yield 42%). $^1$H NMR (400 MHz, DMSO) δ 11.92 (s, 1H), 11.01 (s, 1H), 7.93 (s, 1H), 7.82 (s, 2H), 7.58 (s, 1H), 5.15 (dd, *J* = 13.3, 5.0 Hz, 1H), 4.56 - 4.39 (m, 4H), 3.96 - 3.86 (m, 2H), 3.51 (t, *J* = 11.8 Hz, 2H), 3.43 - 3.34 (m, 2H), 3.25 (brs, 2H), 3.00 - 2.83 (m, 1H), 2.61 (d, *J*= 17.4 Hz, 1H), 2.44 (dd, *J* = 13.0, 4.3 Hz, 1H), 2.10 - 1.93 (m, 1H). LCMS (ESI) calcd for $C_{22}H_{23}Cl_2N_6O_3^+$ [M+H]$^+$: 489.12, found, 489.2.

Example 114: Preparation of 3-(5-((4-(1,3,5-triazin-2-yl)piperazin-1-yl)methyl)-1-oxoisoindolin-2-yl)piperidine-2,6-dione (GT-04486)

Referring to the method of Scheme 1, the target compound (GT-04486) was prepared under appropriate conditions as would be understood by those skilled in the art (white solid, 45 mg, yield 32%). $^1$H NMR (400 MHz, DMSO) δ 11.53 (s, 1H), 11.01 (s, 1H), 8.70 (s, 2H), 7.87 (s, 1H), 7.83 (d, *J* = 7.8 Hz, 1H), 7.75 (d, *J* = 7.7 Hz, 1H), 5.14 (dd, *J* = 13.2, 5.0 Hz, 1H), 4.72 (d, *J* = 13.4 Hz, 2H), 4.56 - 4.34 (m, 4H), 3.56 - 3.37 (m, 4H), 3.12 (s, 2H), 3.01 - 2.85 (m, 1H), 2.61 (d, *J* = 17.0 Hz, 1H), 2.43 (dd, *J* = 13.0, 4.4 Hz, 1H), 2.09 - 1.93 (m, 1H). LCMS (ESI) calcd for $C_{21}H_{24}N_7O_3^+$ [M+H]$^+$: 422.19, found, 422.2.

Example 115: Preparation of 3-(5-((4-(3,4-dichloropyridin-2-yl)piperidin-1-yl)methyl)-1-oxoisoindolin-2-yl)piperidine-2,6-dione (GT-04690)

Referring to the method of Scheme 1, the target compound (GT-04690) was prepared under appropriate conditions as would be understood by those skilled in the art (white solid, 22 mg, yield 22%). $^1$H NMR (400 MHz, MeOD) δ 8.30 (d, *J* = 5.2 Hz, 1H), 7.83 (d, *J* = 7.8 Hz, 1H), 7.75 (s, 1H), 7.64 (d, *J* = 7.8 Hz, 1H), 7.42 (d, *J* = 5.2 Hz, 1H), 5.09 (dd, *J* = 13.3, 5.1 Hz, 1H), 4.55 - 4.43 (m, 5H), 3.56 (d, *J* = 14.0 Hz, 3H), 3.19 - 3.07 (m, 1H), 2.83 - 2.79 (m, 1H), 2.75 - 2.64 (m, 1H), 2.43 - 2.38 (m, 1H), 2.14 - 2.03 (m, 5H). LCMS (ESI) calcd for $C_{24}H_{25}Cl_2N_4O_3^+$ [M+H]$^+$: 487.13, found, 487.1.

Example 116: Preparation of 3-(5-((4-(2,3-dichloropyridin-4-yl)piperidin-1-yl)methyl)-1-oxoisoindolin-2-yl)piperidine-2,6-dione (GT-04560)

Referring to the method of Scheme 1, the target compound (GT-04560) was prepared under appropriate conditions as would be understood by those skilled in the art (white solid, 36 mg, yield 37%). $^1$H NMR (400 MHz, DMSO) δ 11.12 (s, 1H), 11.00 (s, 1H), 8.39 (d, J = 5.0 Hz, 1H), 7.89 (s, 1H), 7.84 - 7.76 (m, 2H), 7.37 (d, J = 5.1 Hz, 1H), 5.14 (dd, J = 13.2, 5.1 Hz, 1H), 4.54 - 4.37 (m, 4H), 3.40 - 3.37 (m, 1H), 3.33 - 3.27 (m, 1H), 3.20 - 3.12 (m, 2H), 3.02 - 2.83 (m, 1H), 2.64 - 2.59 (m, 1H), 2.46 - 2.41 (m, 1H), 2.23 - 2.07 (m, 2H), 2.03 - 1.97 (m, 3H). LCMS (ESI) calcd for $C_{24}H_{25}Cl_2N_4O_3^+$ [M+H]$^+$: 487.13, found, 487.1.

Example 117: Preparation of 3-(5-((4-(2,3-difluoropyridin-4-yl)piperidin-1-yl)methyl)-1-oxoisoindolin-2-yl)piperidine-2,6-dione (GT-04556)

Referring to the method of Scheme 1, the target compound (GT-04556) was prepared under appropriate conditions as would be understood by those skilled in the art (white solid, 72 mg, yield 69%). $^1$H NMR (400 MHz, DMSO) δ 11.18 (s, 1H), 11.01 (s, 1H), 8.04 (d, *J* = 5.0 Hz, 1H), 7.91 (s, 1H), 7.81 (dd, *J* = 19.0, 7.9 Hz, 2H), 7.29 (t, *J* = 4.8 Hz, 1H), 5.15 (dd, *J* = 13.3, 5.0 Hz, 1H), 4.54 - 4.37(m, 5H), 3.53 - 3.40 (m, 3H), 3.28 - 3.21 (m, 1H), 3.16 - 3.04 (m, 2H), 2.99 - 2.85 (m, 1H), 2.61 (d, *J* = 16.6 Hz, 1H), 2.44 (dd, *J* = 13.1, 4.4 Hz, 1H), 2.05 - 2.01 (m, 3H). LCMS (ESI) calcd for $C_{24}H_{25}F_2N_4O_3^+$ [M+H]$^+$: 455.19, found, 455.2.

Example 118: Preparation of 3-(5-([3,4'-bipiperidin]-1-ylmethyl)-1-oxoisoindolin-2-yl)piperidine-2,6-dione (GT-04591)

Referring to the method of Scheme 1, the target compound (GT-04591) was prepared under appropriate conditions as would be understood by those skilled in the art (white solid, 34 mg, yield 72%). $^1$H NMR (400 MHz, MeOD) δ 7.98 - 7.84 (m, 2H), 7.76 (d, *J* = 7.9 Hz, 1H), 5.20 (dd, *J* = 13.3, 5.1 Hz, 1H), 4.69 - 4.40 (m, 4H), 3.57 (d, *J* = 12.4 Hz, 1H), 3.45 (t, *J* = 13.1 Hz, 3H), 3.09 - 2.76 (m, 6H), 2.55 (qd, *J* = 13.3, 4.7 Hz, 1H), 2.30 - 2.15 (m, 1H), 1.90 (ddd, *J* = 40.7, 32.6, 15.1 Hz, 6H), 1.71 - 1.41 (m, 3H), 1.41 - 1.27 (m, 1H). LCMS (ESI) calcd for $C_{24}H_{33}N_4O_3^+$ [M+H]$^+$: 425.25, found, 425.3.

Example 119: Preparation of 3-(5-((3,4-dichloro-3',6'-dihydro-[2,4'-bipyridin]-1'(2'H)-yl)methyl)-1-oxoisoindolin-2-yl)piperidine-2,6-dione (GT-04570)

Referring to the method of Scheme 1, the target compound (GT-04570) was prepared under appropriate conditions as would be understood by those skilled in the art (white solid, 44 mg, yield 45%). $^1$H NMR (400 MHz, DMSO) δ 11.19 (s, 1H), 11.00 (s, 1H), 8.49 (d, *J* = 5.2 Hz, 1H), 7.92 (s, 1H), 7.83 (q, *J* = 7.9 Hz, 2H), 7.71 (d, *J* = 5.2 Hz, 1H), 6.20 (s, 1H), 5.15 (dd, *J* = 13.3, 5.0 Hz, 1H), 4.66 - 4.33 (m, 4H), 3.87 - 3.79 (m, 3H), 3.28 - 3.17 (m, 1H), 3.03 - 2.86 (m, 2H), 2.66 (dd, *J* = 35.3, 19.1 Hz, 2H), 2.44 (dd, *J* = 13.1, 4.5 Hz, 1H), 2.10 - 1.92 (m, 1H). LCMS (ESI) calcd for $C_{24}H_{23}Cl_2N_4O_3^+$ [M+H]$^+$: 485.11, found, 485.2.

Example 120: Preparation of 3-(5-((4,5-dichloro-3',6'-dihydro-[3,4'-bipyridin]-1'(2'H)-yl)methyl)-1-oxoisoindolin-2-yl)piperidine-2,6-dione (GT-04558)

Referring to the method of Scheme 1, the target compound (GT-04558) was prepared under appropriate conditions as would be understood by those skilled in the art (white solid, 24 mg, yield 24%). $^1$H NMR (400 MHz, DMSO) δ 11.56 (s, 1H), 11.01 (s, 1H), 8.75 (s, 1H), 8.39 (s, 1H), 7.96 (s, 1H), 7.84 (s, 2H), 5.91 (s, 1H), 5.15 (dd, *J* = 13.3, 5.1 Hz, 1H), 4.51 (dd, *J* = 41.0, 23.4 Hz, 4H), 3.88 - 3.78 (m, 2H), 3.65 - 3.56 (m, 1H), 3.32 - 3.24 (m, 1H), 2.99 - 2.90 (m, 2H), 2.64 - 2.57 (m, 2H), 2.44 (dd, *J*= 13.1, 4.5 Hz, 1H), 2.10 - 1.94 (m, 1H). LCMS (ESI) calcd for $C_{24}H_{23}Cl_2N_4O_3^+$ [M+H]$^+$: 485.11, found, 485.2.

Example 121: Preparation of 3-(5-((5-chloro-3',6'-dihydro-[3,4'-bipyridin]-1'(2'H)-yl)methyl)-1-oxoisoindolin-2-yl)piperidine-2,6-dione (GT-04620)

Referring to the method of Scheme 1, the target compound (GT-04620) was prepared under appropriate conditions as would be understood by those skilled in the art (white solid, 29 mg, yield 28%). $^1$H NMR (400 MHz, MeOD) δ 8.89 (dt, *J* = 6.3, 3.1 Hz, 2H), 8.62 - 8.57 (m, 1H), 7.97 (d, *J* = 7.9 Hz, 1H), 7.90 (d, *J* = 6.5 Hz, 1H), 7.78 (dd, *J* = 17.6, 13.8 Hz, 1H), 6.52 (s, 1H), 5.21 (dd, *J* = 13.4, 5.2 Hz, 1H), 4.72 - 4.58 (m, 4H), 4.04 (d, *J* = 2.5 Hz, 2H), 3.91 - 3.86 (m, 1H), 3.56 - 3.42 (m, 1H), 3.07 - 2.96 (m, 2H), 2.97 - 2.87 (m, 1H), 2.85 - 2.79 (m, 1H), 2.64 - 2.46 (m, 1H), 2.29 - 2.15 (m, 1H). LCMS (ESI) calcd for $C_{24}H_{24}ClN_4O_3^+$ [M+H]$^+$: 451.15, found, 451.2.

Example 122: Preparation of 3-(5-((2',3'-dichloro-3,6-dihydro-[4,4'-bipyridin]-1(2H)-yl)methyl)-1-oxoisoindolin-2-yl)piperidine-2,6-dione (GT-04559)

Referring to the method of Scheme 1, the target compound (GT-04559) was prepared under appropriate conditions as would be understood by those skilled in the art (white solid, 52 mg, yield 53%). $^1$H NMR (400 MHz, DMSO) δ 11.59 (s, 1H), 11.00 (s, 1H), 8.40 (d, J = 4.9 Hz, 1H), 7.95 (s, 1H), 7.84 (s, 2H), 7.35 (d, J = 4.9 Hz, 1H), 5.91 (s, 1H), 5.15 (dd, J = 13.2, 5.1 Hz, 1H), 4.56 (d, J = 10.8 Hz, 2H), 4.55 - 4.34 (m, 2H), 3.86 - 3.79 (m, 2H), 3.28 (brs, 1H), 3.06 - 2.85 (m, 2H), 2.64 - 2.55 (m, 2H), 2.46 - 2.42 (m, 1H), 2.12 - 1.88 (m, 1H). LCMS (ESI) calcd for $C_{24}H_{23}Cl_2N_4O_3^+$ [M+H]$^+$: 485.11, found, 485.1.

Example 123: Preparation of 3-(5-((3-fluoro-3',6'-dihydro-[2,4'-bipyridin]-1'(2'H)-yl)methyl)-1-oxoisoindolin-2-yl)piperidine-2,6-dione (GT-04689)

Referring to the method of Scheme 1, the target compound (GT-04689) was prepared under appropriate conditions as would be understood by those skilled in the art (white solid, 17 mg, yield 25%). $^1$H NMR (400 MHz, MeOD) δ 8.47 (d, *J* = 4.0 Hz, 1H), 7.97 (d, *J* = 7.8 Hz, 1H), 7.88 (s, 1H), 7.79 (d,*J* = 7.8 Hz, 1H), 7.73 (dd, *J* = 11.7, 8.4 Hz, 1H), 7.47 (dt, *J* = 8.4, 4.2 Hz, 1H), 6.63 (s, 1H), 5.22 (dd, *J* = 13.4, 5.1 Hz, 1H), 4.67 - 4.60 (m, 4H), 4.04 (s, 2H), 3.84 (brs, 1H), 3.50 - 3.43(m, 1H), 3.13 - 2.96(m, 2H), 2.94 - 2.84(m, 1H), 2.87 - 2.76 (m, 1H), 2.62 - 2.49 (m, 1H), 2.28 - 2.16 (m, 1H). LCMS (ESI) calcd for $C_{24}H_{24}FN_4O_3^+$ [M+H]$^+$: 435.18, found, 435.2.

Example 124: Preparation of 3-(5-((2',3'-difluoro-3,6-dihydro-[4,4'-bipyridin]-1(2H)-yl)methyl)-1-oxoisoindolin-2-yl)piperidine-2,6-dione (GT-04555)

Referring to the method of Scheme 1, the target compound (GT-04555) was prepared under appropriate conditions as would be understood by those skilled in the art (white solid, 75 mg, yield 71%). $^1$H NMR (400 MHz, DMSO) δ 11.01 (s, 1H), 10.85 (s, 1H), 8.05 (d, *J* = 5.0 Hz, 1H), 7.85 (d, *J* = 7.8 Hz, 2H), 7.76 (d, *J* = 7.4 Hz, 1H), 7.42 (t, *J* = 5.1 Hz, 1H), 6.34 (s, 1H), 5.15 (dd, *J* = 13.2, 5.1 Hz, 1H), 4.62 - 4.37 (m, 4H), 3.98 - 3.77 (m, 2H), 3.67 - 3.62 (m, 1H), 3.30 - 3.19 (m, 1H), 3.02 - 2.86 (m, 2H), 2.70 (d, *J* = 24.2 Hz, 1H), 2.62 (d, *J* = 16.9 Hz, 1H), 2.43 (dd, *J* = 13.3, 4.6 Hz, 1H), 2.09 - 1.92 (m, 1H).

LCMS (ESI) calcd for $C_{24}H_{23}F_2N_4O_3^+$ [M+H]+: 453.17, found, 453.2.

Example 125: Preparation of 3-(5-((3-(2,3-dichlorophenyl)-3,6-diazabicyclo[3.1.1]heptan-6-yl)methyl)-1-oxoisoindolin-2-yl)piperidine-2,6-dione (GT-04712)

Referring to the method of Scheme 1, the target compound (GT-04712) was prepared under appropriate conditions as would be understood by those skilled in the art (white solid, 29 mg, yield 30%). $^1$H NMR (400 MHz, MeOD) δ 7.93 (d, *J* = 14.0 Hz, 1H), 7.76 (dd, *J* = 39.9, 18.4 Hz, 2H), 7.47 - 7.12 (m, 3H), 5.20 (dd, *J* = 13.4, 5.1 Hz, 1H), 4.90 (s, 2H), 4.85 - 4.81 (m, 2H), 4.69 - 4.48 (m, 5H), 3.98 - 3.74 (m, 3H), 2.99 - 2.89 (m, 1H), 2.86 - 2.78 (m, 1H), 2.53 (dd, *J* = 13.2, 4.7 Hz, 1H), 2.21 (dd, *J* = 8.8, 3.8 Hz, 1H). LCMS (ESI) calcd for $C_{25}H_{25}Cl_2N_4O_3^+$ [M+H]+: 499.13, found, 499.1.

Example 126: Preparation of 3-(5-((6-(2,3-dichlorophenyl)-3,6-diazabicyclo[3.1.1]heptan-3-yl)methyl)-1-oxoisoindolin-2-yl)piperidine-2,6-dione (GT-04897)

Referring to the method of Scheme 1, the target compound (GT-04897) was prepared under appropriate conditions as would be understood by those skilled in the art (white solid, 48 mg, yield 63%). $^1$H NMR (400 MHz, DMSO) δ 6.97 (d, *J* = 7.7 Hz, 1H), 6.83 - 6.77 (m, 2H), 6.27 (t, *J* = 8.2 Hz, 1H), 6.14 (d, *J* = 7.6 Hz, 1H), 5.69 (d, *J* = 7.6 Hz, 1H), 4.44 (dd, *J* = 13.3, 4.7 Hz, 1H), 4.01 - 3.93 (m, 1H), 3.89 - 3.85 (m, 1H), 3.70 (brs, 2H), 3.58 (brs, 2H), 3.07 - 2.81 (m, 4H), 2.26 - 2.10 (m, 2H), 2.03 (d, *J* = 13.9 Hz, 1H), 1.77 (dd, *J* = 13.2, 4.6 Hz, 1H), 1.50 - 1.32 (m, 2H). LCMS (ESI) calcd for $C_{25}H_{25}Cl_2N_4O_3^+$ [M+H]+: 499.13, found, 499.1.

Example 127: Preparation of 3-(5-(((1R,4R)-5-(2,3-dichlorophenyl)-2,5-diazabicyclo[2.2.1]heptan-2-yl)methyl)-1-oxoisoindolin-2-yl)piperidine-2,6-dione (GT-04724)

Referring to the method of Scheme 1, the target compound (GT-04724) was prepared under appropriate conditions as would be understood by those skilled in the art (white solid, 47 mg, yield 49%). $^1$H NMR (400 MHz, MeOD) δ 7.93 (d, *J* = 7.8 Hz, 1H), 7.86 (s, 1H), 7.77 (d, *J*= 7.9 Hz, 1H), 7.20 (t, *J* = 8.0 Hz, 1H), 7.14 (s, 1H), 6.99 (d, *J* = 7.5 Hz, 1H), 5.20 (dd, *J* = 13.3, 5.2 Hz, 1H), 4.71 (d, *J* = 13.1 Hz, 1H), 4.61 (t, *J* = 12.6 Hz, 3H), 4.52 (d, *J* = 13.1 Hz, 2H), 3.88 (dd, *J* = 30.8, 11.0 Hz, 2H), 3.65 (d, *J* = 10.5 Hz, 1H), 3.49 (d, *J* = 12.7 Hz, 1H), 2.94 (ddd, *J* = 18.5, 13.4, 5.3 Hz, 1H), 2.86 - 2.76 (m, 1H), 2.54 (ddd, *J* = 26.6, 13.2, 4.7 Hz, 2H), 2.33 (d, *J* = 11.3 Hz, 1H), 2.27 - 2.13 (m, 1H). LCMS (ESI) calcd for $C_{25}H_{25}Cl_2N_4O_3^+$ [M+H]+: 499.13, found, 499.1.

Example 128: Preparation of3-(5-(((1S,4S)-5-(2,3-dichlorophenyl)-2,5-diazabicyclo[2.2.1]heptan-2-yl)methyl)-1-oxoisoindolin-2-yl)piperidine-2,6-dione (GT-04898)

Referring to the method of Scheme 1, the target compound (GT-04898) was prepared under appropriate conditions as would be understood by those skilled in the art (white solid, 42 mg, yield 55%). $^1$H NMR (400 MHz, DMSO) δ 7.20 - 6.79 (m, 3H), 6.51 - 6.08 (m, 3H), 4.46 - 4.26 (m, 1H), 3.82 - 3.64 (m, 5H), 3.18 - 2.88 (m, 2H), 2.84 - 2.77 (m, 1H), 2.69 - 2.61 (m, 1H), 2.19 - 1.87 (m, 2H), 1.83 - 1.60 (m, 2H), 1.57 - 1.45 (m, 1H), 1.42 - 1.28 (m, 2H). LCMS (ESI) calcd for $C_{25}H_{25}Cl_2N_4O_3^+$ [M+H]+: 499.13, found, 499.1.

Example 129: Preparation of 3-(5-((5-(2,3-dichlorophenyl)-2,5-diazabicyclo[2.2.2]octan-2-yl)methyl)-1-oxoisoindolin-2-yl)piperidine-2,6-dione (GT-04930)

Referring to the method of Scheme 1, the target compound (GT-04930) was prepared under appropriate conditions as would be understood by those skilled in the art (white solid, 26 mg, yield 33%). $^1$H NMR (400 MHz, MeOD-d4) δ 7.93 (d, *J* = 7.8 Hz, 1H), 7.86 (s, 1H), 7.78 (d, *J* = 7.6 Hz, 1H), 7.31 - 7.22 (m, 3H), 5.19 (dd, *J* = 13.2, 5.0 Hz, 1H), 4.90 - 4.70 (m, 2H), 4.65 - 4.49 (m, 2H), 3.90 - 3.55 (m, 5H), 2.97 - 2.91 (m, 1H), 2.85 - 2.74 (m, 1H), 2.57 - 2.47 (m, 2H), 2.38 - 2.29 (m, 1H), 2.21 - 2.11 (m, 3H), 1.95 - 1.87 (m, 1H). LCMS (ESI) calcd for $C_{26}H_{27}Cl_2N_4O_3^+$ [M+H]+: 513.15, found, 513.2.

Example 130: Preparation of 3-(5-((8-(2,3-dichlorophenyl)-3,8-diazabicyclo[3.2.1]octan-3-yl)methyl)-1-oxoisoindolin-2-yl)piperidine-2,6-dione (GT-04899)

Referring to the method of Scheme 1, the target compound (GT-04899) was prepared under appropriate conditions as would be understood by those skilled in the art (white solid, 41 mg, yield 53%). $^1$H NMR (400 MHz, DMSO) δ 7.19 - 7.11 (m, 1H), 7.07 - 7.00 (m, 1H), 7.00 - 6.92 (m, 1H), 6.45 - 6.39 (m, 2H), 6.26 - 6.21 (m, 1H), 4.44 - 4.39 (m, 1H), 3.84 - 3.75 (m, 3H), 3.47 - 3.35 (m, 2H), 2.87 - 2.79 (m, 2H), 2.73 - 2.69 (m, 2H), 2.21 - 2.11 (m, 1H), 2.02 - 1.98 (m, 1H), 1.77 - 1.71 (m, 1H), 1.51 - 1.39 (m, 3H), 1.26 - 1.21 (m, 3H). LCMS (ESI) calcd for $C_{26}H_{27}Cl_2N_4O_3^+$ [M+H]+: 513.15, found, 513.2.

Example 131: Preparation of 3-(5-((3-(2,3-dichlorophenyl)-3,8-diazabicyclo[3.2.1]octan-8-yl)methyl)-1-oxoisoindolin-2-yl)piperidine-2,6-dione (GT-04855)

Referring to the method of Scheme 1, the target compound (GT-04855) was prepared under appropriate conditions as would be understood by those skilled in the art (white solid, 21 mg, yield 22%). $^{1}$H NMR (400 MHz, MeOD) δ 7.97 (d, *J* = 7.8 Hz, 1H), 7.87 (s, 1H), 7.79 (d, *J* = 7.7 Hz, 1H), 7.32 (dt, *J* = 12.4, 8.0 Hz, 2H), 7.18 (d, *J* = 6.8 Hz, 1H), 5.24 - 5.19 (m, 2H), 4.61 (d, *J* = 6.9 Hz, 4H), 4.47 (s, 2H), 4.13 (s, 2H), 3.39 (s, 2H), 2.92 (dd, *J* = 13.5, 5.3 Hz, 1H), 2.82 (d, *J* = 18.0 Hz, 1H), 2.58 - 2.45 (m, 4H), 2.26 - 2.15 (m, 1H). LCMS (ESI) calcd for $C_{26}H_{27}Cl_2N_4O_3^+$ [M+H]$^+$: 513.15, found, 513.2.

Example 132: Preparation of 3-(5-((4-(2,3-dichlorophenyl)-1,4-diazepan-1-yl)methyl)-1-oxoisoindolin-2-yl)piperidine-2,6-dione (GT-04856)

Referring to the method of Scheme 1, the target compound (GT-04856) was prepared under appropriate conditions as would be understood by those skilled in the art (white solid, 23 mg, yield 24%). $^{1}$H NMR (400 MHz, MeOD) δ 7.96 (d, *J* = 7.8 Hz, 1H), 7.83 (s, 1H), 7.76 (d, *J* = 7.7 Hz, 1H), 7.33 - 7.22 (m, 3H), 5.21 (dd, *J* = 13.2, 5.1 Hz, 2H), 4.65 - 4.59 (m, 4H), 3.64 (s, 4H), 3.51 (s, 3H), 3.35 (s, 1H), 2.92 (dd, *J* = 13.2, 5.2 Hz, 1H), 2.84 (s, 1H), 2.54 (dd, *J* = 13.3, 4.8 Hz, 1H), 2.23 (s, 2H). LCMS (ESI) calcd for $C_{25}H_{27}Cl_2N_4O_3^+$ [M+H]$^+$: 501.15, found, 501.2.

Example 133: Preparation of 3-(5-((4-(6-fluorobenzo[d]isoxazol-3-yl)piperidin-1-yl)methyl)-1-oxoisoindolin-2-yl)piperidine-2,6-dione (GT-04254)

Referring to the method of Scheme 1, the target compound (GT-04254) was prepared under appropriate conditions as would be understood by those skilled in the art (white solid, 28 mg, yield 38%). $^{1}$H NMR (400 MHz, MeOD-d4) δ 7.94 (d, *J* = 7.8 Hz, 1H), 7.89 (dd, *J* = 8.8, 5.1 Hz, 1H), 7.80 (s, 1H), 7.72 (d, *J* = 7.8 Hz, 1H), 7.43 (d, *J* = 6.8 Hz, 1H), 7.21 (td, *J* = 9.0, 2.1 Hz, 1H), 5.19 (dd, *J* = 13.5, 5.2 Hz, 1H), 4.62 - 4.49 (m, 4H), 3.73 - 3.63 (m, 2H), 3.61 - 3.42 (m, 2H), 2.99 - 2.86 (m, 1H), 2.82 - 2.76 (m, 1H), 2.61 - 2.47 (m, 1H), 2.46 - 2.38 (m, 2H), 2.25 - 2.16 (m, 3H). LCMS (ESI) calcd for $C_{26}H_{26}FN_4O_4^+$ [M+H]$^+$: 477.19, found, 477.2.

Example 134: Preparation of 3-(5-((4-(benzo[d]isoxazol-3-yl)piperidin-1-yl)methyl)-1-oxoisoindolin-2-yl)piperidine-2,6-dione (GT-04907)

Referring to the method of Scheme 1, the target compound (GT-04907) was prepared under appropriate conditions as would be understood by those skilled in the art (white solid, 27 mg, yield 38%). $^{1}$H NMR (400 MHz, MeOD-d4) δ 7.93 (dd, *J* = 7.9, 2.0 Hz, 2H), 7.84 (s, 1H), 7.76 (d, *J* = 7.7 Hz, 1H), 7.65 (d, *J* = 3.0 Hz, 2H), 7.40 (ddd, *J* = 8.0, 4.9, 3.0 Hz, 1H), 5.18 (dd, *J* = 13.3, 5.1 Hz, 1H), 4.55 - 4.5 1(m, 4H), 3.67 (d, *J* = 11.7 Hz, 2H), 3.57 - 3.44 (m, 2H), 3.00 - 2.86 (m, 1H), 2.81 - 2.70 (m, 1H), 2.53 - 2.47 (m, 1H), 2.40 (d, *J* = 14.4 Hz, 3H), 2.30 - 2.23 (m, 2H), 2.21 - 2.13 (m, 1H). LCMS (ESI) calcd for $C_{26}H_{27}N_4O_4^+$ [M+H]$^+$: 459.20, found, 459.2.

Example 135: Preparation of 3-(5-((4-(isoxazol-3-yl)piperidin-1-yl)methyl)-1-oxoisoindolin-2-yl)piperidine-2,6-dione (GT-04715)

Referring to the method of Scheme 1, the target compound (GT-04715) was prepared under appropriate conditions as would be understood by those skilled in the art (white solid, 21 mg, yield 25%). $^{1}$H NMR (400 MHz, MeOD) δ 8.65 (t, *J* = 11.6 Hz, 1H), 7.95 (t, *J* = 8.2 Hz, 1H), 7.82 (s, 1H), 7.73 (d, *J* = 8.3 Hz, 1H), 6.51 (t, *J* = 13.1 Hz, 1H), 5.21 (dd, *J* = 13.3, 5.2 Hz, 1H), 4.61 - 4.52 (m, 5H), 3.66 (d, *J* = 12.0 Hz, 2H), 3.28 - 3.20 (m, 2H), 2.94 (dd, *J* = 15.3, 10.5 Hz, 1H), 2.82 (d, *J* = 15.4 Hz, 1H), 2.53 (dt, *J* = 13.5, 6.8 Hz, 1H), 2.34 (d, *J* = 14.4 Hz, 2H), 2.25 - 2.19 (m, 1H), 2.00 (dd, *J* = 25.1, 11.7 Hz, 2H). LCMS (ESI) calcd for $C_{22}H_{25}N_4O_4^+$ [M+H]$^+$: 409.19, found, 409.3.

Example 136: Preparation of 3-(5-((4-(6-fluorobenzo[d]isoxazol-3-yl)piperazin-1-yl)methyl)-1-oxoisoindolin-2-yl)piperidine-2,6-dione (GT-04667)

Referring to the method of Scheme 1, the target compound (GT-04667) was prepared under appropriate conditions as would be understood by those skilled in the art (white solid, 62 mg, yield 62%). $^{1}$H NMR (400 MHz, MeOD) δ 8.01 - 7.91 (m, 2H), 7.84 (s, 1H), 7.77 (d, *J* = 7.8 Hz, 1H), 7.38 (d, *J* = 8.8 Hz, 1H), 7.18 (t, *J* = 8.1 Hz, 1H), 5.22 (dd, *J* = 13.4, 5.1 Hz, 1H), 4.77 (s, 1H), 4.68 -4.53 (m, 4H), 4.24 (s, 1H), 3.50 (s, 6H), 3.03 - 2.90 (m, 1H), 2.81 (d, *J* = 17.8 Hz, 1H), 2.62 - 2.47 (m, 1H), 2.27 - 2.15 (m, 1H). LCMS (ESI) calcd for $C_{25}H_{25}FN_5O_4^+$ [M+H]$^+$: 478.19, found, 478.2.

Example 137: Preparation of 3-(5-((4-(isoxazolo[4,5-c]pyridin-3-yl)piperazin-1-yl)methyl)-1-oxoisoindolin-2-yl)piperidine-2,6-dione (GT-04904)

Referring to the method of Scheme 1, the target compound (GT-04904) was prepared under appropriate conditions as would be understood by those skilled in the art (white solid, 27 mg, yield 38%). $^1$H NMR (400 MHz, MeOD) δ 9.80 (s, 1H), 8.87 (d, *J* = 6.9 Hz, 1H), 8.20 (d, *J* = 6.9 Hz, 1H), 7.93 (d, *J* = 8.4 Hz, 2H), 7.78 (d, *J* = 7.9 Hz, 1H), 5.18 (dd, *J* = 13.3, 5.1 Hz, 1H), 4.69 - 4.53 (m, 4H), 4.43 - 4.32 (m, 2H), 3.89 - 3.44 (m, 6H), 3.00 - 2.86 (m, 1H), 2.79 (dd, *J* = 15.3, 2.3 Hz, 1H), 2.53 (qd, *J* = 13.2, 4.6 Hz, 1H), 2.29 - 2.10 (m, 1H). LCMS (ESI) calcd for $C_{24}H_{25}N_6O_4^+$ [M+H]$^+$: 461.19, found, 461.2.

Example 138: Preparation of 3-(5-((4-(6-fluorobenzo[d]isothiazol-3-yl)piperazin-1-yl)methyl)-1-oxoisoindolin-2-yl)piperidine-2,6-dione (GT-05332)

Referring to the method of Scheme 1, the target compound (GT-05332) was prepared under appropriate conditions as would be understood by those skilled in the art (white solid, 18 mg, yield 20%). $^1$H NMR (400 MHz, MeOD-d4) δ 8.10 - 8.03 (m, 1H), 7.95 (d, *J* = 7.8 Hz, 1H), 7.83 (s, 1H), 7.77 - 7.69 (m, 2H), 7.25 (td, *J* = 8.8, 2.3 Hz, 1H), 5.19 (dd, *J* = 13.4, 5.2 Hz, 1H), 4.66 - 4.54 (m, 4H), 4.17 (d, *J* = 12.5 Hz, 2H), 3.63 - 3.41 (m, 6H), 2.99 - 2.85 (m, 2H), 2.85 - 2.74 (m, 1H), 2.61 - 2.44 (m, 1H), 2.25 - 2.14 (m, 1H). LCMS (ESI) calcd for $C_{25}H_{25}FN_5O_3S^+$ [M+H]$^+$: 494.17, found, 494.1.

Example 139: Preparation of 3-(5-((4-(6-fluoro-1H-indazol-3-yl)piperazin-1-yl)methyl)-1-oxoisoindolin-2-yl)piperidine-2,6-dione (GT-05264)

Referring to the method of Scheme 1, the target compound (GT-05264) was prepared under appropriate conditions as would be understood by those skilled in the art (white solid, 37 mg, yield 42%). $^1$H NMR (400 MHz, MeOD-d4) δ 7.94 (d, *J* = 7.9 Hz, 1H), 7.85 (s, 1H), 7.81 - 7.73 (m, 2H), 7.08 (dd, *J* = 9.5, 2.1 Hz, 1H), 6.88 (td, *J* = 9.1, 2.2 Hz, 1H), 5.19 (dd, *J* = 13.4, 5.2 Hz, 1H), 4.66 - 4.50 (m, 4H), 4.05 (d, *J* = 11.4 Hz, 2H), 3.63 - 3.57 (m, 2H), 3.47 - 3.44 (m, 2H), 3.38 - 3.35 (m, 1H), 2.92 - 2.88(m, 1H), 2.84 - 2.73 (m, 1H), 2.62 - 2.61(m, 1H), 2.52 - 2.37(m, 1H), 2.25 - 2.13 (m, 1H). LCMS (ESI) calcd for $C_{25}H_{26}FN_6O_3^+$ [M+H]$^+$: 477.20, found, 477.3.

Example 140: Preparation of 3-(5-((4-(1H-pyrrolo[2,3-b]pyridin-3-yl)piperazin-1-yl)methyl)-1-oxoisoindolin-2-yl)piperidine-2,6-dione (GT-04601)

Referring to the method of Scheme 1, the target compound (GT-04601) was prepared under appropriate conditions as would be understood by those skilled in the art (white solid, 10 mg, yield 10%). 1H NMR (400 MHz, MeOD) δ 8.78 (s, 1H), 8.68 (s, 1H), 7.94 (d, J = 7.9 Hz, 1H), 7.89 (s, 1H), 7.78 (d, J = 8.1 Hz, 1H), 5.19 (dd, J = 13.2, 5.2 Hz, 1H), 4.69 - 4.55 (m, 4H), 3.89 - 3.76 (m, 4H), 3.64 - 3.61 (m, 2H), 3.51 - 3.43 (m, 2H), 3.02 - 2.85 (m, 1H), 2.84 - 2.74 (m, 1H), 2.58 - 2.48 (m, 1H), 2.29 - 2.09 (m, 1H). LCMS (ESI) calcd for $C_{25}H_{27}N_6O_3^+$ [M+H]$^+$: 459.21, found, 459.3.

Example 141: Preparation of 3-(5-((4-(6-fluoro-1H-indol-3-yl)piperidin-1-yl)methyl)-1-oxoisoindolin-2-yl)piperidine-2,6-dione (GT-04713)

Referring to the method of Scheme 1, the target compound (GT-04713) was prepared under appropriate conditions as would be understood by those skilled in the art (white solid, 60 mg, yield 60%). $^1$H NMR (400 MHz, MeOD) δ 7.97 (d, *J* = 7.8 Hz, 1H), 7.83 (s, 1H), 7.75 (d, *J* = 7.9 Hz, 1H), 7.57 (dd, *J* = 8.7, 5.3 Hz, 1H), 7.06 (d, *J* = 10.6 Hz, 2H), 6.83 (dd, *J* = 12.9, 5.6 Hz, 1H), 5.21 (dd, *J* = 13.3, 5.1 Hz, 1H), 4.91 - 4.89 (m, 1H), 4.63 (t, *J* = 12.2 Hz, 2H), 4.54 (s, 2H), 3.65 (d, *J* = 11.5 Hz, 2H), 3.32 - 3.13 (m, 3H), 2.94 (ddd, *J* = 21.8, 13.4, 6.8 Hz, 1H), 2.86 - 2.77 (m, 1H), 2.62 - 2.47 (m, 1H), 2.33 (d, *J* = 13.7 Hz, 2H), 2.26 - 2.16 (m, 1H), 2.03 (d, *J* = 14.0 Hz, 2H). LCMS (ESI) calcd for $C_{27}H_{28}FN_4O_3^+$ [M+H]$^+$: 475.21, found, 475.2.

Example 142: Preparation of 3-(5-((4-(6-fluoro-1-methyl-1H-indol-3-yl)piperidin-1-yl)methyl)-1-oxoisoindolin-2-yl)piperidine-2,6-dione (GT-04858)

Referring to the method of Scheme 1, the target compound (GT-04858) was prepared under appropriate conditions as would be understood by those skilled in the art (white solid, 41 mg, yield 42%). $^1$H NMR (400 MHz, MeOD) δ 7.97 (d, *J* = 7.8 Hz, 1H), 7.82 (s, 1H), 7.75 (d, *J* = 7.8 Hz, 1H), 7.57 (dd, *J* = 8.8, 5.1 Hz, 1H), 7.09 (d, *J* = 10.0 Hz, 1H), 7.03 (d, *J* = 6.6 Hz, 1H), 6.84 (dd, *J* = 14.8, 5.6 Hz, 1H), 5.21 (dd, *J* = 13.3, 5.2 Hz, 1H), 4.60 (dd, *J* = 28.2, 21.1 Hz, 4H), 3.70 (t, *J* = 22.0 Hz, 5H), 3.30 - 3.15 (m, 3H), 2.92 (dd, *J* = 13.4, 5.4 Hz, 1H), 2.83 (d, *J* = 2.5 Hz, 1H), 2.54 (dd, *J* = 13.1, 4.6 Hz, 1H), 2.32 (d, *J* = 14.8 Hz, 2H), 2.22 (dd, *J* = 10.2, 4.8 Hz, 1H), 2.01 (d, *J* = 12.8 Hz, 2H). LCMS (ESI) calcd for $C_{28}H_{30}FN_4O_3^+$ [M+H]$^+$: 489.23, found, 489.3.

Example 143: Preparation of 3-(5-((4-(5-fluoroindolin-1-yl)piperidin-1-yl)methyl)-1-oxoisoindolin-2-yl)piperidine-2,6-dione (GT-04650)

Referring to the method of Scheme 1, the target compound (GT-04650) was prepared under appropriate conditions as would be understood by those skilled in the art (white solid, 33 mg, yield 33%). $^1$H NMR (400 MHz, MeOD) δ 8.00 - 7.86 (m, 2H), 7.79 - 7.70 (m, 1H), 7.43 (dd, *J* = 8.8, 4.2 Hz, 1H), 7.19 (ddd, *J* = 12.7, 11.2, 5.3 Hz, 2H), 5.19 (dd, *J* = 13.3, 5.2 Hz, 1H), 4.59 (dd, *J* = 28.0, 18.6 Hz, 4H), 4.26 - 4.12 (m, 1H), 3.95 (t, *J* = 7.7 Hz, 2H), 3.67 (d, *J* = 12.4 Hz, 2H), 3.33 - 3.25 (m, 4H), 2.94 (ddd, *J* = 18.5, 13.5, 5.4 Hz, 1H), 2.86 - 2.73 (m, 1H), 2.54 (qd, *J* = 13.3, 4.7 Hz, 1H), 2.41 - 2.07 (m, 5H). LCMS (ESI) calcd for $C_{27}H_{30}FN_4O_3^+$ [M+H]$^+$: 477.23, found, 477.3.

Example 144: Preparation of 3-(5-((4-(5-fluoro-1H-benzo[d]imidazol-1-yl)piperidin-1-yl)methyl)-1-oxoisoindolin-2-yl)piperidine-2,6-dione (GT-04905)

Referring to the method of Scheme 1, the target compound (GT-04905) was prepared under appropriate conditions as would be understood by those skilled in the art (white solid, 9 mg, yield 12%). $^1$H NMR (400 MHz, MeOD) δ 9.63 (s, 1H), 8.20 (dd, *J* = 9.3, 4.0 Hz, 1H), 7.98 - 7.90 (m, 2H), 7.80 (d, *J* = 9.0 Hz, 1H), 7.65 (dd, *J* = 7.9, 2.1 Hz, 1H), 7.52 (td, *J* = 9.2, 2.4 Hz, 1H), 5.21 - 5.15 (m, 2H), 4.69 - 4.52 (m, 4H), 3.78 (d, *J* = 12.7 Hz, 2H), 3.56 - 3.41 (m, 2H), 3.00 - 2.86 (m, 1H), 2.79 (ddd, *J* = 17.6, 4.6, 2.4 Hz, 1H), 2.74 - 2.46 (m, 5H), 2.27 - 2.10 (m, 1H). LCMS (ESI) calcd for $C_{26}H_{27}FN_5O_3^+$ [M+H]$^+$: 476.21, found, 476.2.

Example 145: Preparation of 3-(5-((4-(5-fluoro-2-oxo-2,3-dihydro-1H-benzo[d]imidazol-1-yl)piperidin-1-yl)methyl)-1-oxoisoindolin-2-yl)piperidine-2,6-dione (GT-04714)

Referring to the method of Scheme 1, the target compound (GT-04714) was prepared under appropriate conditions as would be understood by those skilled in the art (white solid, 5 mg, yield 5%). $^1$H NMR (400 MHz, MeOD) δ 7.97 (d, *J* = 7.8 Hz, 1H), 7.85 (s, 1H), 7.76 (d, *J* = 8.1 Hz, 1H), 7.27 (dd, *J* = 8.7, 4.4 Hz, 1H), 6.86 (t, *J* = 8.9 Hz, 2H), 5.21 (dd, *J* = 13.3, 5.1 Hz, 1H), 4.92 - 4.88 (m, 1H), 4.61 (dd, *J* = 26.9, 19.7 Hz, 5H), 3.70 (d, *J* = 12.2 Hz, 2H), 3.31 - 3.19 (m, 1H), 3.00 - 2.78 (m, 4H), 2.55 (qd, *J* = 13.2, 4.7 Hz, 1H), 2.27 - 2.17 (m, 1H), 2.09 (t, *J* = 13.4 Hz, 2H). LCMS (ESI) calcd for $C_{26}H_{27}FN_5O_4^+$ [M+H]$^+$: 492.20, found, 492.2.

Example 146: Preparation of 3-(5-((4-(1H-pyrrolo[2,3-b]pyridin-3-yl)piperidin-1-yl)methyl)-1-oxoisoindolin-2-yl)piperidine-2,6-dione (GT-04603)

Referring to the method of Scheme 1, the target compound (GT-04603) was prepared under appropriate conditions as would be understood by those skilled in the art (white solid, 12 mg, yield 12%). $^1$H NMR (400 MHz, MeOD) δ 8.78 (s, 1H), 8.68 (s, 1H), 7.94 (d, J = 7.9 Hz, 1H), 7.89 (s, 1H), 7.78 (d, J = 8.1 Hz, 1H), 5.19 (dd, J = 13.2, 5.2 Hz, 1H), 4.69 - 4.55 (m, 4H), 3.89 - 3.76 (m, 4H), 3.64 - 3.61 (m, 2H), 3.51 - 3.43 (m, 2H), 3.02 - 2.85 (m, 1H), 2.84 - 2.74 (m, 1H), 2.58 - 2.48 (m, 1H), 2.29 - 2.09 (m, 1H). LCMS (ESI) calcd for $C_{26}H_{28}N_5O_3^+$ [M+H]$^+$: 458.22, found, 458.3.

Example 147: Preparation of 3-(5-((4-(1H-pyrrolo[2,3-b]pyridin-3-yl)-3,6-dihydropyridin-1(2H)-yl)methyl)-1-oxoisoindolin-2-yl)piperidine-2,6-dione (GT-04602)

Referring to the method of Scheme 1, the target compound (GT-04602) was prepared under appropriate conditions as would be understood by those skilled in the art (white solid, 22 mg, yield 21%). $^1$H NMR (400 MHz, MeOD) δ 8.78 (s, 1H), 8.68 (s, 1H), 7.94 (d, J = 7.9 Hz, 1H), 7.89 (s, 1H), 7.78 (d, J = 8.1 Hz, 1H), 5.19 (dd, J = 13.2, 5.2 Hz, 1H), 4.69 - 4.55 (m, 4H), 3.89 - 3.76 (m, 4H), 3.64 - 3.61 (m, 2H), 3.51 - 3.43 (m, 2H), 3.02 - 2.85 (m, 1H), 2.84 - 2.74 (m, 1H), 2.58 - 2.48 (m, 1H), 2.29 - 2.09 (m, 1H). LCMS (ESI) calcd for $C_{26}H_{26}N_5O_3^+$ [M+H]$^+$: 456.20, found, 456.2.

Example 148: Preparation of 3-(5-((4-(6-fluoro-1H-indol-3-yl)-3,6-dihydropyridin-1(2H)-yl)methyl)-1-oxoisoindolin-2-yl) piperidine-2,6-dione (GT-04722)

Referring to the method of Scheme 1, the target compound (GT-04722) was prepared under appropriate conditions as would be understood by those skilled in the art (white solid, 45 mg, yield 45%). $^1$H NMR (400 MHz, MeOD) δ 7.98 - 7.75 (m, 4H), 7.30 - 7.10 (m, 2H), 6.69 (t, *J* = 9.2 Hz, 1H), 5.29 - 5.13 (m, 2H), 4.59 (d, *J* = 17.0 Hz, 4H), 2.94 (s, 3H), 2.82 (d, *J* = 17.2 Hz, 3H), 2.55 (s, 2H), 2.22 (s, 2H). LCMS (ESI) calcd for $C_{27}H_{26}FN_4O_3^+$ [M+H]$^+$: 473.20, found, 473.2.

Example 149: Preparation of 3-(5-((4-(6-fluoro-1-methyl-1H-indol-3-yl)-3,6-dihydropyridin-1(2H)-yl)methyl)-1-oxoisoindolin-2-yl)piperidine-2,6-dione (GT-04723)

Referring to the method of Scheme 1, the target compound (GT-04723) was prepared under appropriate

conditions as would be understood by those skilled in the art (white solid, 45 mg, yield 46%). $^1$H NMR (400 MHz, MeOD) δ 7.98 (d, *J* = 7.9 Hz, 1H), 7.81 (dd, *J* = 16.9, 8.6 Hz, 3H), 7.18 (t, *J* = 8.8 Hz, 1H), 6.96 (dd, *J* = 19.8, 8.4 Hz, 1H), 6.23 (d, *J* = 34.2 Hz, 1H), 5.21 (dd, *J* = 13.3, 5.1 Hz, 2H), 5.01 (d, *J* = 12.8 Hz, 1H), 4.75 (s, 1H), 4.61 (d, *J* = 4.6 Hz, 3H), 3.79 (d, *J* = 7.4 Hz, 3H), 2.92 (d, *J* = 12.9 Hz, 2H), 2.81 (d, *J* = 16.5 Hz, 2H), 2.53 (d, *J* = 7.2 Hz, 2H), 2.22 (s, 2H). LCMS (ESI) calcd for $C_{28}H_{28}FN_4O_3^+$ [M+H]$^+$: 487.21, found, 487.2.

Example 150: Preparation of 3-(5-((4-(6-fluoro-3,4-dihydroquinolin-1(2H)-yl)piperidin-1-yl)methyl)-1-oxoisoindolin-2-yl)piperidine-2,6-dione (GT-04691)

Referring to the method of Scheme 1, the target compound (GT-04691) was prepared under appropriate conditions as would be understood by those skilled in the art (white solid, 42 mg, yield 43%). $^1$H NMR (400 MHz, MeOD) δ 7.93 (d, *J* = 7.8 Hz, 1H), 7.86 (s, 1H), 7.74 (d, *J* = 7.7 Hz, 1H), 7.31 - 7.21 (m, 1H), 7.04 (t, *J* = 8.3 Hz, 2H), 5.20 (dd, *J* = 13.3, 5.1 Hz, 1H), 4.59 (q, *J* = 17.5 Hz, 2H), 4.51 (s, 2H), 4.11 - 4.05 (m, 1H), 3.66 (d, *J* = 12.1 Hz, 2H), 3.58 - 3.47 (m, 2H), 3.23 (t, *J* = 12.0 Hz, 2H), 3.03 - 2.87 (m, 3H), 2.87 -2.76 (m, 1H), 2.58 - 2.50 (m, 1H), 2.33 - 2.05 (m, 7H). LCMS (ESI) calcd for $C_{28}H_{32}FN_4O_3^+$ [M+H]$^+$: 491.25, found, 491.2.

Example 151: Preparation of 3-(5-((4-(7-fluoro-2,3-dihydro-4H-benzo[b][1,4]oxazin-4-yl)piperidin-1-yl)methyl)-1-oxoisoindolin-2-yl)piperidine-2,6-dione (GT-04859)

Referring to the method of Scheme 1, the target compound (GT-04859) was prepared under appropriate conditions as would be understood by those skilled in the art (white solid, 40 mg, yield 41%). $^1$H NMR (400 MHz, DMSO) δ 11.16 (s, 1H), 11.01 (s, 1H), 7.89 (s, 1H), 7.83 (d, *J* = 7.8 Hz, 1H), 7.77 (d, *J* = 7.8 Hz, 1H), 6.86 - 6.79 (m, 1H), 6.62 - 6.57 (m, 2H), 5.14 (dd, *J*= 13.3, 5.0 Hz, 1H), 4.45 (q, *J* = 17.7 Hz, 4H), 4.22 - 4.12 (m, 2H), 3.91 (t, *J* = 11.7 Hz, 2H), 3.46 (d, *J* = 9.4 Hz, 2H), 3.19 - 3.12 (m, 2H), 3.11 - 3.02 (m, 2H), 2.98 - 2.84 (m, 1H), 2.61 (d, *J* = 17.2 Hz, 1H), 2.44 (dd, *J* = 13.2, 4.3 Hz, 1H), 2.22 - 2.16 (m, 2H), 2.09 - 1.96 (m, 1H), 1.78 (d, *J* = 11.9 Hz, 2H). LCMS (ESI) calcd for $C_{27}H_{30}FN_4O_4^+$ [M+H]$^+$: 493.22, found, 493.2.

Example 152: Preparation of 3-(5-((4-(7-fluoro-3-oxo-2,3-dihydro-4H-benzo[b][1,4]oxazin-4-yl)piperidin-1-yl)methyl)-1-oxoisoindolin-2-yl)piperidine-2,6-dione (GT-04860)

Referring to the method of Scheme 1, the target compound (GT-04860) was prepared under appropriate conditions as would be understood by those skilled in the art (white solid, 46 mg, yield 49%). $^1$H NMR (400 MHz, DMSO) δ 11.01 (s, 1H), 10.14 (s, 1H), 7.86 (d, *J* = 7.7 Hz, 1H), 7.80 (s, 1H), 7.70 (d, *J* = 7.7 Hz, 1H), 7.47 (dd, *J* = 9.0, 5.5 Hz, 1H), 7.00 (dd, *J* = 9.2, 2.9 Hz, 1H), 6.96 - 6.89 (m, 1H), 5.15 (dd, *J* = 13.1, 5.0 Hz, 1H), 4.57 (s, 2H), 4.46 (dd, *J* = 48.7, 17.8 Hz, 5H), 3.50 (d, *J* = 11.6 Hz, 2H), 3.15 (d, *J* = 11.4 Hz, 2H), 2.97 - 2.79 (m, 3H), 2.66 (d, *J* = 12.4 Hz, 1H), 2.45 - 2.42 (m, 1H), 2.06 - 1.98 (m, 1H), 1.94 (d, *J*= 13.2 Hz, 2H). LCMS (ESI) calcd for $C_{27}H_{28}FN_4O_5^+$ [M+H]$^+$: 507.20, found, 507.3.

Example 153: Preparation of 3-(5-((4-(7-fluorochroman-4-yl)piperidin-1-yl)methyl)-1-oxoisoindolin-2-yl)piperidine-2,6-dione (GT-04901)

Referring to the method of Scheme 1, the target compound (GT-04901) was prepared under appropriate conditions as would be understood by those skilled in the art (white solid, 24 mg, yield 32%). $^1$H NMR (400 MHz, MeOD) δ 7.91 (d, *J* = 7.8 Hz, 1H), 7.76 (s, 1H), 7.68 (d, *J* = 7.9 Hz, 1H), 7.11 (dd, *J* = 8.4, 6.7 Hz, 1H), 6.58 (td, *J* = 8.4, 2.7 Hz, 1H), 6.48 (dd, *J* = 10.4, 2.7 Hz, 1H), 5.18 (dd, *J* = 13.3, 5.2 Hz, 1H), 4.64 - 4.50 (m, 2H), 4.43 (s, 2H), 4.27 - 4.21 (m, 1H), 4.19 - 4.08 (m, 1H), 3.54 (t, *J* = 14.0 Hz, 2H), 3.08 - 3.01 (m, 2H), 2.97 - 2.84 (m, 1H), 2.84 - 2.74 (m, 2H), 2.66 - 2.47 (m, 1H), 2.22 - 2.17 (m, 1H), 2.13 - 2.01 (m, 1H), 2.00 - 1.84 (m, 4H), 1.82 - 1.70 (m, 1H), 1.61 - 1.49 (m, 1H). LCMS (ESI) calcd for $C_{28}H_{31}FN_3O_4^+$ [M+H]$^+$: 492.23, found, 492.3.

Example 154: Preparation of 3-(5-((4-(7-fluorochroman-4-ylidene)piperidin-1-yl)methyl)-1-oxoisoindolin-2-yl)piperidine-2,6-dione (GT-04649)

Referring to the method of Scheme 1, the target compound (GT-04649) was prepared under appropriate conditions as would be understood by those skilled in the art (white solid, 28 mg, yield 29%). $^1$H NMR (400 MHz, MeOD) δ 7.94 (t, *J* = 8.8 Hz, 1H), 7.79 (d, *J* = 10.6 Hz, 1H), 7.71 (t, *J* = 9.1 Hz, 1H), 7.19 (dd, *J* = 8.6, 6.5 Hz, 1H), 6.70 - 6.46 (m, 2H), 5.21 (dd, *J* = 13.4, 5.2 Hz, 1H), 4.67 - 4.44 (m, 4H), 4.41 - 4.19 (m, 2H), 3.56 (d, *J* = 46.1 Hz, 2H), 3.35 (s, 2H), 3.12 (t, *J* = 13.9 Hz, 2H), 3.02 - 2.89 (m, 1H), 2.87 - 2.73 (m, 2H), 2.73 - 2.30 (m, 4H), 2.27 - 2.14 (m, 1H). LCMS (ESI) calcd for $C_{28}H_{29}FN_3O_4^+$ [M+H]$^+$: 490.21, found, 490.2.

Example 155: Preparation of 3-(5-((4-(5-fluoronaphthalen-1-yl)piperidin-1-yl)methyl)-1-oxoisoindolin-2-yl)piperidine-2,6-dione (GT-04664)

Referring to the method of Scheme 1, the target compound (GT-04664) was prepared under appropriate conditions as would be understood by those skilled in the art (white solid, 51 mg, yield 52%). $^1$H NMR (400 MHz, MeOD) δ 8.10 - 7.93 (m, 3H), 7.85 (s, 1H), 7.77 (d, *J* = 7.9 Hz, 1H), 7.55 (dt, *J* = 17.5, 7.6 Hz, 3H), 7.25 (dd, *J* = 10.5, 7.8 Hz, 1H), 5.22 (dd, *J* = 13.4, 5.2 Hz, 1H), 4.74 - 4.49 (m, 4H), 3.70 (dd, *J* = 49.6, 37.0 Hz, 3H), 3.41 (dd, *J* = 23.7, 11.0 Hz, 2H), 2.95 (ddd, *J* = 18.5, 13.5, 5.4 Hz, 1H), 2.87 - 2.76 (m, 1H), 2.61 - 2.46 (m, 1H), 2.34 - 2.00 (m, 5H). LCMS (ESI) calcd for $C_{29}H_{29}FN_3O_3^+$ $[M+H]^+$: 486.22, found, 486.2.

Example 156: Preparation of 3-(5-((4-(7-fluoroquinolin-4-yl)piperidin-1-yl)methyl)-1-oxoisoindolin-2-yl)piperidine-2,6-dione (GT-04648)

Referring to the method of Scheme 1, the target compound (GT-04648) was prepared under appropriate conditions as would be understood by those skilled in the art (white solid, 24 mg, yield 25%). $^1$H NMR (400 MHz, MeOD) δ 9.21 (d, *J* = 5.9 Hz, 1H), 8.83 (dd, *J* = 9.5, 5.3 Hz, 1H), 8.04 (d, *J* = 6.0 Hz, 1H), 8.00 - 7.85 (m, 4H), 7.81 (d, *J* = 8.1 Hz, 1H), 5.19 (dd, *J* = 13.3, 5.1 Hz, 1H), 4.69 - 4.52 (m, 4H), 4.18 (t, *J* = 11.9 Hz, 1H), 3.74 (d, *J*= 12.6 Hz, 2H), 3.48 (t, *J* = 11.2 Hz, 2H), 2.98 - 2.87 (m, 1H), 2.84 - 2.74 (m, 1H), 2.53 (qd, *J* = 13.3, 4.7 Hz, 1H), 2.45 - 2.25 (m, 4H), 2.24 - 2.14 (m, 1H). LCMS (ESI) calcd for $C_{28}H_{28}FN_4O_3^+$ $[M+H]^+$: 487.21, found, 487.2.

Example 157: Preparation of 3-(5-((4-(isoquinolin-5-yl)piperidin-1-yl)methyl)-1-oxoisoindolin-2-yl)piperidine-2,6-dione (GT-04665)

Referring to the method of Scheme 1, the target compound (GT-04665) was prepared under appropriate conditions as would be understood by those skilled in the art (white solid, 22 mg, yield 22%). $^1$H NMR (400 MHz, MeOD) δ 9.83 (s, 1H), 8.88 (d, *J* = 6.9 Hz, 1H), 8.66 (t, *J* = 12.4 Hz, 1H), 8.47 (d, *J* = 8.2 Hz, 1H), 8.28 - 8.17 (m, 1H), 8.09 (q, *J* = 7.7 Hz, 1H), 8.00 - 7.90 (m, 2H), 7.82 (d, *J* = 7.1 Hz, 1H), 5.19 (td, *J* = 13.0, 4.9 Hz, 1H), 4.71 - 4.51 (m, 4H), 4.02 - 3.86 (m, 1H), 3.74 (d, *J* = 12.4 Hz, 2H), 3.49 (dt, *J* = 20.1, 10.4 Hz, 2H), 2.95 (tt, *J* = 12.5, 8.5 Hz, 1H), 2.86 - 2.76 (m, 1H), 2.56 (qd, *J* = 13.1, 4.7 Hz, 1H), 2.42 - 2.08 (m, 5H). LCMS (ESI) calcd for $C_{28}H_{29}N_4O_3^+$ $[M+H]^+$: 469.22, found, 469.2.

Example 158: Preparation of 3-(5-((4-(5-fluoronaphthalen-1-yl)-3,6-dihydropyridin-1(2H)-yl)methyl)-1-oxoisoindolin-2-yl)piperidine-2,6-dione (GT-04902)

Referring to the method of Scheme 1, the target compound (GT-04902) was prepared under appropriate conditions as would be understood by those skilled in the art (white solid, 38 mg, yield 52%). $^1$H NMR (400 MHz, MeOD) δ 8.08 (d, *J* = 8.4 Hz, 1H), 7.98 (d, *J* = 7.9 Hz, 1H), 7.85 (s, 1H), 7.83 - 7.78 (m, 2H), 7.57 (t, *J* = 7.8 Hz, 1H), 7.53 - 7.41 (m, 3H), 7.23 (dd, *J* = 10.8, 7.3 Hz, 1H), 5.83 (s, 1H), 5.20 (dd, *J* = 13.3, 5.2 Hz, 1H), 4.68 - 4.59 (m, 4H), 4.01 (brs, 2H), 3.88 - 3.83 (m, 1H), 3.58 - 3.55 (m, 1H), 2.94 - 2.88 (m, 1H), 2.81 - 2.76 (m, 3H), 2.55 - 2.51 (m, 1H), 2.23 - 2.17 (m, 1H). LCMS (ESI) calcd for $C_{29}H_{27}FN_3O_3^+$ $[M+H]^+$: 484.20, found, 484.2.

Example 159: Preparation of 3-(5-((4-(isoquinolin-5-yl)-3,6-dihydropyridin-1(2H)-yl)methyl)-1-oxoisoindolin-2-yl)piperidine-2,6-dione (GT-04666)

Referring to the method of Scheme 1, the target compound (GT-04666) was prepared under appropriate conditions as would be understood by those skilled in the art (white solid, 21 mg, yield 21%). $^1$H NMR (400 MHz, MeOD) δ 9.84 (s, 1H), 8.80 (d, *J* = 6.8 Hz, 1H), 8.62 (d, *J* = 6.8 Hz, 1H), 8.53 (d, *J* = 8.3 Hz, 1H), 8.18 (d, *J* = 7.2 Hz, 1H), 8.09 (t, *J* = 7.8 Hz, 1H), 8.03 - 7.95 (m, 2H), 7.88 (d, *J* = 7.7 Hz, 1H), 5.96 (s, 1H), 5.21 (dd, *J* = 13.3, 5.1 Hz, 1H), 4.94 - 4.88 (m, 1H) 4.71 (t, *J* = 9.6 Hz, 2H), 4.61 (t,*J* = 12.8 Hz, 2H), 4.07 (s, 2H), 3.88 (s, 1H), 3.63 (d, *J* = 8.9 Hz, 1H), 3.15 (s, 1H), 3.01 - 2.88 (m, 1H), 2.84 (d, *J* = 2.3 Hz, 1H), 2.56 (qd, *J* = 13.1, 4.6 Hz, 1H), 2.29 - 2.18 (m, 1H). LCMS (ESI) calcd for $C_{28}H_{27}N_4O_3^+$ $[M+H]^+$: 467.21, found, 467.2.

Example 160: Preparation of 3-(5-((3-(7-fluorochroman-4-yl)azetidin-1-yl)methyl)-1-oxoisoindolin-2-yl)piperidine-2,6-dione (GT-04700)

Referring to the method of Scheme 1, the target compound (GT-04700) was prepared under appropriate conditions as would be understood by those skilled in the art (white solid, 24 mg, yield 25%). $^1$H NMR (400 MHz, MeOD) δ 7.93 (d, J = 7.8 Hz, 1H), 7.73 (s, 1H), 7.66 (d, J = 7.5 Hz, 1H), 7.02 (t, J = 7.4 Hz, 1H), 6.59 (d, J = 8.5 Hz, 1H), 6.54 (dd, J = 10.3, 2.7 Hz, 1H), 5.20 (dd, J = 13.0, 4.8 Hz, 1H), 4.56 (t, J = 12.7 Hz, 5H), 4.42 - 4.31 (m, 1H), 4.21 (s, 5H), 3.15 (s, 2H), 2.94

(dd, J = 29.9, 16.6 Hz, 2H), 2.81 (d, J = 15.2 Hz, 2H), 2.63 - 2.45 (m, 1H), 2.25 - 2.19 (m, 1H), 2.10 - 2.04 (m, 1H). LCMS (ESI) calcd for $C_{26}H_{27}FN_3O_4^+$ [M+H]+: 464.20, found, 464.2.

Example 161: Preparation of 3-(5-((3-(7-fluorochroman-4-ylidene)azetidin-1-yl)methyl)-1-oxoisoindolin-2-yl)piperidine-2,6-dione (GT-04701)

Referring to the method of Scheme 1, the target compound (GT-04701) was prepared under appropriate conditions as would be understood by those skilled in the art (white solid, 32 mg, yield 33%). $^1$H NMR (400 MHz, MeOD) δ 7.95 (d, J = 7.8 Hz, 1H), 7.79 (s, 1H), 7.72 (d, J = 7.6 Hz, 1H), 7.01 (dd, J = 8.7, 6.3 Hz, 1H), 6.72 (td, J = 8.5, 2.7 Hz, 1H), 6.65 (dd, J = 10.1, 2.6 Hz, 1H), 5.21 (dd, J = 13.1, 5.3 Hz, 4H), 4.72 (s, 2H), 4.59 (d, J = 7.1 Hz, 3H), 4.24 (s, 2H), 3.01 - 2.90 (m, 1H), 2.86 - 2.75 (m, 1H), 2.61 - 2.49 (m, 3H), 2.27 - 2.15 (m, 1H). LCMS (ESI) calcd for $C_{26}H_{25}FN_3O_4^+$ [M+H]+: 462.18, found, 462.2.

Example 162: Preparation of 3-(5-((3-(7-fluoroquinolin-4-yl)azetidin-1-yl)methyl)-1-oxoisoindolin-2-yl)piperidine-2,6-dione (GT-04651)

Referring to the method of Scheme 1, the target compound (GT-04651) was prepared under appropriate conditions as would be understood by those skilled in the art (white solid, 25 mg, yield 24%). $^1$H NMR (400 MHz, MeOD) δ 9.30 (t, *J* = 7.7 Hz, 1H), 8.40 - 8.07 (m, 2H), 8.00 (dd, *J* = 8.6, 2.5 Hz, 1H), 7.85 (dt, *J* = 51.8, 25.7 Hz, 4H), 5.42 - 5.05 (m, 2H), 4.61 (dt, *J* = 26.7, 24.7 Hz, 8H), 2.92 (ddd, *J* = 18.5, 13.5, 5.4 Hz, 1H), 2.85 - 2.72 (m, 1H), 2.52 (qd, *J* = 13.1, 4.5 Hz, 1H), 2.27 - 2.12 (m, 1H). LCMS (ESI) calcd for $C_{26}H_{24}FN_4O_3^+$ [M+H]+: 459.18, found, 459.2.

Example 163: Preparation of 3-(5-((3-(5-fluoroindolin-1-yl)azetidin-1-yl)methyl)-1-oxoisoindolin-2-yl)piperidine-2,6-dione (GT-04677)

Referring to the method of Scheme 1, the target compound (GT-04677) was prepared under appropriate conditions as would be understood by those skilled in the art (white solid, 33 mg, yield 26%). $^1$H NMR (400 MHz, MeOD) δ 7.92 (d, *J* = 7.8 Hz, 1H), 7.75 (s, 1H), 7.67 (d, *J* = 7.9 Hz, 1H), 6.89 (d, *J* = 8.3 Hz, 1H), 6.75 (t, *J* = 8.5 Hz, 1H), 6.40 - 6.29 (m, 1H), 5.18 (dd, *J* = 13.3, 5.2 Hz, 1H), 4.67 - 4.53 (m, 4H), 4.44 - 4.31 (m, 4H), 4.30 - 4.28 (m, 1H), 3.43 (t, *J* = 8.1 Hz, 2H), 3.01 - 2.99 (m, 2H), 2.95 - 2.86 (m, 1H), 2.84 - 2.74 (m, 1H), 2.56 - 2.46 (m, 1H), 2.23 - 2.13 (m, 1H). LCMS (ESI) calcd for $C_{25}H_{26}FN_4O_3^+$ [M+H]+: 449.20, found, 449.2.

Example 164: Preparation of 3-(5-((3-(5-fluoro-1H-benzo[d]imidazol-1-yl)azetidin-1-yl)methyl)-1-oxoisoindolin-2-yl)piperidine-2,6-dione (GT-04716)

Referring to the method of Scheme 1, the target compound (GT-04716) was prepared under appropriate conditions as would be understood by those skilled in the art (white solid, 79 mg, yield 78%). $^1$H NMR (400 MHz, MeOD) δ 9.80 (d, *J* = 80.3 Hz, 1H), 7.96 - 7.89 (m, 2H), 7.81 (dd, *J* = 23.1, 6.2 Hz, 1H), 7.72 - 7.65 (m, 1H), 7.51 (td, *J* = 9.2, 2.3 Hz, 1H), 5.91 (s, 1H), 5.19 (dt, *J* = 13.6, 6.8 Hz, 1H), 4.97 (dd, *J* = 23.4, 16.2 Hz, 3H), 4.83 (s, 3H), 4.68 - 4.47 (m, 2H), 2.94 (ddd, *J* = 18.6, 13.6, 5.3 Hz, 1H), 2.85 - 2.76 (m, 1H), 2.54 (qd, *J* = 13.1, 4.7 Hz, 1H), 2.27 - 2.13 (m, 1H). LCMS (ESI) calcd for $C_{24}H_{23}FN_5O_3^+$ [M+H]+: 448.18, found, 448.2.

Example 165: Preparation of 3-(1-oxo-5-((4-(thieno[2,3-d]pyrimidin-4-yl)piperazin-1-yl)methyl)isoindolin-2-yl)piperidine-2,6-dione (GT-04678)

Referring to the method of Scheme 1, the target compound (GT-04678) was prepared under appropriate conditions as would be understood by those skilled in the art (white solid, 37 mg, yield 48%). $^1$H NMR (400 MHz, DMSO) δ 11.84 (s, 1H), 11.02 (s, 1H), 8.57 (s, 1H), 7.92 (s, 1H), 7.86 - 7.74 (m, 3H), 7.70 (d, *J* = 6.2 Hz, 1H), 5.15 (dd, *J* = 13.3, 5.1 Hz, 1H), 4.70 (d, *J* = 12.7 Hz, 2H), 4.65 - 4.29 (m, 5H), 3.85 - 3.80(m, 2H), 3.46 (d, *J* = 11.0 Hz, 2H), 3.24 (brs, 2H), 3.01 - 2.82 (m, 1H), 2.62 (d, *J* = 17.7 Hz, 1H), 2.48 - 2.39 (m, 1H), 2.11 - 1.90 (m, 1H). LCMS (ESI) calcd for $C_{24}H_{25}N_6O_3S^+$ [M+H]+: 477.17, found, 477.2.

Example 166: Preparation of 3-(5-((4-(5-methylthieno[2,3-d]pyrimidin-4-yl)piperazin-1-yl)methyl)-1-oxoisoindolin-2-yl) piperidine-2,6-dione (GT-04679)

Referring to the method of Scheme 1, the target compound (GT-04679) was prepared under appropriate conditions as would be understood by those skilled in the art (white solid, 50 mg, yield 51%). $^1$H NMR (400 MHz, MeOD) δ 8.70 (s, 1H), 7.94 (d, *J* = 8.1 Hz, 1H), 7.83 (s, 1H), 7.73 (d, *J* = 8.5 Hz, 1H), 7.44 (d, *J* = 1.1 Hz, 1H), 5.19 (dd, *J* = 13.3, 5.1 Hz,

1H), 4.57 (d, *J* = 4.8 Hz, 4H), 4.39 - 4.31 (m, 1H), 3.72 - 3.59 (m, 5H), 3.39 - 3.34 (m, 3H), 2.98 - 2.85 (m, 1H), 2.79 (d, *J* = 15.6 Hz, 1H), 2.61 (s, 3H), 2.52 (d, *J* = 14.5 Hz, 1H), 2.23 - 2.17 (m, 1H). LCMS (ESI) calcd for $C_{25}H_{27}N_6O_3S^+$ [M+H]$^+$: 491.19, found, 491.2.

Example 167: Preparation of 3-(5-((4-(6-methylthieno[2,3-d]pyrimidin-4-yl)piperazin-1-yl)methyl)-1-oxoisoindolin-2-yl) piperidine-2,6-dione (GT-04680)

Referring to the method of Scheme 1, the target compound (GT-04680) was prepared under appropriate conditions as would be understood by those skilled in the art (white solid, 67 mg, yield 69%). $^1$H NMR (400 MHz, MeOD) δ 8.70 (s, 1H), 7.96 *(d, J* = 7.9 Hz, 1H), 7.91 (s, 1H), 7.78 (d, *J* = 7.8 Hz, 1H), 7.50 (d, *J* = 1.3 Hz, 1H), 5.21 (dd, *J* = 13.4, 5.2 Hz, 1H), 4.69 - 4.54 (m, 4H), 3.91 - 3.84 (m, 1H), 3.75 - 3.45 (m, 4H), 3.35 - 3.32(m, 3H), 2.99 - 2.90 (m, 1H), 2.82 (ddd, *J* = 17.6, 4.6, 2.4 Hz, 1H), 2.69 (d, *J* = 1.2 Hz, 3H), 2.55 (qd, *J* = 13.3, 4.7 Hz, 1H), 2.25 - 2.19 (m, 1H). LCMS (ESI) calcd for $C_{25}H_{27}N_6O_3S^+$ [M+H]$^+$: 491.19, found, 491.2.

Example 168: Preparation of 3-(5-((4-(6-isopropylthieno[2,3-d]pyrimidin-4-yl)piperazin-1-yl)methyl)-1-oxoisoindolin-2-yl)piperidine-2,6-dione (GT-04705)

Referring to the method of Scheme 1, the target compound (GT-04705) was prepared under appropriate conditions as would be understood by those skilled in the art (white solid, 22 mg, yield 30%). $^1$H NMR (400 MHz, MeOD) δ 8.76 (s, 1H), 7.98 - 7.91 (m, 2H), 7.80 (d, J = 7.9 Hz, 1H), 7.53 (s, 1H), 5.21 (dd, J = 13.2, 5.2 Hz, 1H), 5.03- 4.97 (m, 1H), 4.74 - 4.47 (m, 5H), 4.13 - 3.99 (m, 2H), 3.59 (brs, 4H), 3.46 - 3.39 (m, 1H), 2.95 - 2.90 (m, 1H), 2.86 - 2.75 (m, 1H), 2.57 - 2.51 (m, 1H), 2.28 - 2.15 (m, 1H), 1.45 (d, J = 6.9 Hz, 6H). LCMS (ESI) calcd for $C_{27}H_{31}N_6O_3S^+$ [M+H]$^+$: 519.22, found, 519.2.

Example 169: Preparation of 3-(5-((4-(5,6-dimethylthieno[2,3-d]pyrimidin-4-yl)piperazin-1-yl)methyl)-1-oxoisoindolin-2-yl)piperidine-2,6-dione (GT-04706)

Referring to the method of Scheme 1, the target compound (GT-04706) was prepared under appropriate conditions as would be understood by those skilled in the art (white solid, 26 mg, yield 34%). $^1$H NMR (400 MHz, MeOD) δ 8.63 (s, 1H), 7.84 (d, J = 7.7 Hz, 1H), 7.77 (s, 1H), 7.64 (d, J = 7.6 Hz, 1H), 5.09 (dd, J = 13.6, 5.1 Hz, 1H), 4.49 (d, J = 6.5 Hz, 4H), 4.36 - 4.31 (m, 2H), 3.74 - 3.69 (m, 2H), 3.56 - 3.47 (m, 3H), 3.39 - 3.31 (m, 3H), 2.88 - 2.75 (m, 1H), 2.75 - 2.62 (m, 1H), 2.45 (s, 3H), 2.37 (s, 3H), 2.13 - 2.07 (m, 1H). LCMS (ESI) calcd for $C_{26}H_{29}N_6O_3S^+$ [M+H]$^+$: 505.20, found, 505.3.

Example 170: Preparation of 3-(1-oxo-5-((4-(6-phenylthieno[2,3-d]pyrimidin-4-yl)piperazin-1-yl)methyl)isoindolin-2-yl) piperidine-2,6-dione (GT-04862)

Referring to the method of Scheme 1, the target compound (GT-04862) was prepared under appropriate conditions as would be understood by those skilled in the art (white solid, 49 mg, yield 55%). $^1$H NMR (400 MHz, DMSO) δ 11.01 (s, 1H), 10.97 (s, 1H), 8.52 (s, 1H), 8.00 (s, 1H), 7.88 - 7.83 (m, 4H), 7.76 (d, *J* = 8.0 Hz, 1H), 7.51 (t, *J* = 7.6 Hz, 2H), 7.43 (t, *J*= 7.4 Hz, 1H), 5.15 (dd, *J* = 13.2, 5.1 Hz, 1H), 4.74 (d, *J* = 14.2 Hz, 2H), 4.55 - 4.36 (m, 4H), 3.66 - 3.58 (m, 2H), 3.51 - 3.43 (m, 2H), 3.30 - 3.22 (m, 2H), 2.96 - 2.89 (m, 1H), 2.62 (d, *J* = 17.1 Hz, 1H), 2.51 - 2.43 (m, 1H), 2.05 - 2.01 (m, 1H). LCMS (ESI) calcd for $C_{30}H_{29}N_6O_3S^+$ [M+H]$^+$: 553.20, found, 553.2.

Example 171: Preparation of 3-(5-((4-(6-bromothieno[2,3-d]pyrimidin-4-yl)piperazin-1-yl)methyl)-1-oxoisoindolin-2-yl) piperidine-2,6-dione (GT-04861)

Referring to the method of Scheme 1, the target compound (GT-04861) was prepared under appropriate conditions as would be understood by those skilled in the art (white solid, 28 mg, yield 32%). $^1$H NMR (400 MHz, DMSO) δ 11.40 (s, 1H), 11.01 (s, 1H), 8.49 (s, 1H), 7.93 (s, 1H), 7.89 - 7.82 (m, 2H), 7.76 (d, *J* = 7.9 Hz, 1H), 5.15 (dd, *J* = 13.2, 5.0 Hz, 1H), 4.65 - 4.57 (m, 2H), 4.55 - 4.35 (m, 4H), 3.67 - 3.58 (m, 2H), 3.46 - 3.43 (m, 2H), 3.27 - 3.18 (m, 2H), 3.00 - 2.86 (m, 1H), 2.63 (t, *J* = 16.0 Hz, 1H), 2.47 - 2.39 (m, 1H), 2.11 - 1.95 (m, 1H). LCMS (ESI) calcd for $C_{24}H_{24}BrN_6O_3S^+$ [M+H]$^+$: 555.08, found, 552.2.

Example 172: Preparation of 3-(1-oxo-5-((4-(5-phenylthieno[2,3-d]pyrimidin-4-yl)piperazin-1-yl)methyl)isoindolin-2-yl) piperidine-2,6-dione (GT-04709)

Referring to the method of Scheme 1, the target compound (GT-04709) was prepared under appropriate conditions as would be understood by those skilled in the art (white solid, 22 mg, yield 31%). $^1$H NMR (400 MHz, MeOD) δ 8.79 (s, 1H), 7.93 (d, J = 7.8 Hz, 1H), 7.73 (d, J = 5.2 Hz, 2H), 7.62 (d, J = 7.8 Hz, 1H), 7.51 - 7.48 (m, 5H), 5.21 (dd, J = 13.2,

5.2 Hz, 1H), 4.68 - 4.52 (m, 2H), 4.41 (s, 2H), 4.15 (brs, 2H), 3.32 - 3.06 (m, 4H), 2.99 - 2.90 (m, 3H), 2.75 - 2.64 (m, 1H), 2.60 - 2.49 (m, 1H), 2.27 - 2.16 (m, 1H). LCMS (ESI) calcd for $C_{30}H_{29}N_6O_3S^+$ [M+H]$^+$: 553.20, found, 553.3.

Example 173: Preparation of 3-(1-oxo-5-((4-(5,6,7,8-tetrahydrobenzo[4,5]thieno[2,3-d]pyrimidin-4-yl)piperazin-1-yl)methyl)isoindolin-2-yl)piperidine-2,6-dione (GT-05209)

Referring to the method of Scheme 1, the target compound (GT-05209) was prepared under appropriate conditions as would be understood by those skilled in the art (white solid, 35 mg, yield 43%). $^1$H NMR (400 MHz, MeOD-d4) δ 8.63 (s, 1H), 7.94 (d, *J* = 7.8 Hz, 1H), 7.84 (s, 1H), 7.74 (d, *J* = 7.8 Hz, 1H), 5.19 (dd, *J* = 13.3, 5.2 Hz, 1H), 4.66 - 4.51 (m, 4H), 4.32 - 4.21 (m, 2H), 3.63 - 3.43 (m, 6H), 3.04 - 2.86 (m, 5H), 2.82 - 2.77 (m, 1H), 2.56 - 2.47 (m, 1H), 2.25 - 2.14 (m, 1H), 2.03 - 1.97 (m, 2H), 1.88 - 1.83 (m, 2H). LCMS (ESI) calcd for $C_{28}H_{31}N_6O_3S^+$ [M+H]$^+$: 531.22, found, 531.3.

Example 174: Preparation of 3-(5-((4-(6,7-dihydro-5H-cyclopenta[4,5]thieno[2,3-d]pyrimidin-4-yl)piperazin-1-yl)methyl)-1-oxoisoindolin-2-yl)piperidine-2,6-dione (GT-04996)

Referring to the method of Scheme 1, the target compound (GT-04996) was prepared under appropriate conditions as would be understood by those skilled in the art (white solid, 44 mg, yield 56%). $^1$H NMR (400 MHz, MeOD-d4) δ 8.63 (s, 1H), 7.95 (d, *J* = 7.9 Hz, 1H), 7.84 (s, 1H), 7.74 (d, *J* = 6.8 Hz, 1H), 5.19 (dd, *J* = 13.2, 5.1 Hz, 1H), 4.66 - 4.47 (m, 6H), 3.78 - 3.48 (m, 5H), 3.16 - 3.07 (m, 5H), 2.98 - 2.86 (m, 1H), 2.81 - 2.77 (m, 1H), 2.55 - 2.50 (m, 3H), 2.23 - 2.16 (m, 1H). LCMS (ESI) calcd for $C_{27}H_{29}N_6O_3S^+$ [M+H]$^+$: 517.20, found, 517.3.

Example 175: Preparation of 3-(5-((4-(2-methyl-5-phenylthieno[2,3-d]pyrimidin-4-yl)piperazin-1-yl)methyl)-1-oxoisoindolin-2-yl)piperidine-2,6-dione (GT-05100)

Referring to the method of Scheme 1, the target compound (GT-05100) was prepared under appropriate conditions as would be understood by those skilled in the art (white solid, 50 mg, yield 58%). $^1$H NMR (400 MHz, MeOD-d4) δ 7.91 (d, *J* = 7.8 Hz, 1H), 7.71 (s, 1H), 7.64 (s, 1H), 7.59 (d, *J* = 7.8 Hz, 1H), 7.51 - 7.45 (m, 5H), 5.19 (dd, *J* = 13.3, 5.2 Hz, 1H), 4.64 - 4.49 (m, 2H), 4.39 (s, 2H), 4.18 (brs, 2H), 3.29 - 3.08 (m, 4H), 2.93 - 2.88 (m, 2H), 2.83 - 2.74 (m, 2H), 2.74 (s, 3H), 2.58 - 2.47 (m, 1H), 2.25 - 2.15 (m, 1H). LCMS (ESI) calcd for $C_{31}H_{31}N_6O_3S^+$ [M+H]$^+$: 567.22, found, 567.3.

Example 176: Preparation of 3-(5-((4-(2-methyl-5,6,7,8-tetrahydrobenzo[4,5]thieno[2,3-d]pyrimidin-4-yl)piperazin-1-yl)methyl)-1-oxoisoindolin-2-yl)piperidine-2,6-dione (GT-04900)

Referring to the method of Scheme 1, the target compound (GT-04900) was prepared under appropriate conditions as would be understood by those skilled in the art (white solid, 60 mg, yield 73%). $^1$H NMR (400 MHz, MeOD) δ 7.93 (d, *J* = 7.8 Hz, 1H), 7.87 (s, 1H), 7.75 (d, *J* = 7.9 Hz, 1H), 5.18 (dd, *J* = 13.3, 5.2 Hz, 1H), 4.66 - 4.52 (m, 4H), 4.47 - 4.40 (m, 1H), 3.84 - 3.32 (m, 7H), 3.00 - 2.86 (m, 5H), 2.82 - 2.76 (m, 1H), 2.69 (s, 3H), 2.58 - 2.48 (m, 1H), 2.22 - 2.17 (m, 1H), 2.02 - 1.97 (m, 2H), 1.87 - 1.81 (m, 2H). LCMS (ESI) calcd for $C_{29}H_{33}N_6O_3S^+$ [M+H]$^+$: 545.23, found, 545.3.

Example 177: Preparation of 3-(5-((4-(2-methyl-6,7-dihydro-5H-cyclopenta[4,5]thieno[2,3-d]pyrimidin-4-yl)piperazin-1-yl)methyl)-1-oxoisoindolin-2-yl)piperidine-2,6-dione (GT-05157)

Referring to the method of Scheme 1, the target compound (GT-05157) was prepared under appropriate conditions as would be understood by those skilled in the art (white solid, 46 mg, yield 57%). $^1$H NMR (400 MHz, MeOD-d4) δ 7.94 (d, *J* = 7.9 Hz, 1H), 7.88 (s, 1H), 7.76 (d, *J* = 7.9 Hz, 1H), 5.19 (dd, *J* = 13.4, 5.2 Hz, 1H), 4.65 - 4.53 (m, 5H), 3.94 - 3.76 (m, 2H), 3.69 - 3.47 (m, 4H), 3.16 - 3.07 (m, 5H), 2.95 - 2.86 (m, 1H), 2.84 - 2.74 (m, 1H), 2.70 (s, 3H), 2.57 - 2.50 (m, 3H), 2.21 - 2.18 (m, 1H). LCMS (ESI) calcd for $C_{28}H_{31}N_6O_3S^+$ [M+H]$^+$: 531.22, found, 531.2.

Example 178: Preparation of 3-(5-((4-(2-methylthieno[2,3-d]pyrimidin-4-yl)piperazin-1-yl)methyl)-1-oxoisoindolin-2-yl)piperidine-2,6-dione (GT-04693)

Referring to the method of Scheme 1, the target compound (GT-04693) was prepared under appropriate conditions as would be understood by those skilled in the art (white solid, 24 mg, yield 25%). $^1$H NMR (400 MHz, MeOD) δ 7.95 (d, *J* = 8.1 Hz, 2H), 7.85 - 7.78 (m, 3H), 5.39 - 4.94 (m, 3H), 4.72 - 4.55 (m, 4H), 4.11 - 4.38 (m, 1H), 3.70 - 3.56 (m, 4H), 3.01 - 2.88 (m, 1H), 2.87 - 2.77 (m, 1H), 2.74 (s, 3H), 2.55 (qd, *J* = 13.1, 4.5 Hz, 1H), 2.28 - 2.14 (m, 1H). LCMS (ESI) calcd for $C_{25}H_{27}N_6O_3S^+$ [M+H]$^+$: 491.19, found, 491.2.

Example 179: Preparation of 3-(1-oxo-5-((4-(thieno[2,3-d]pyrimidin-2-yl)piperazin-1-yl)methyl)isoindolin-2-yl)piperidine-2,6-dione (GT-04725)

Referring to the method of Scheme 1, the target compound (GT-04725) was prepared under appropriate conditions as would be understood by those skilled in the art (white solid, 43 mg, yield 43%). $^1$H NMR (400 MHz, MeOD) δ 8.96 (s, 1H), 7.98 (d, *J* = 7.8 Hz, 1H), 7.87 (s, 1H), 7.80 (d, *J* = 7.8 Hz, 1H), 7.36 (dd, *J* = 23.1, 6.0 Hz, 2H), 5.24 (dd, *J* = 13.2, 5.2 Hz, 1H), 5.01 (d, *J* = 11.8 Hz, 2H), 4.58 (s, 4H), 3.61 (s, 2H), 3.52 - 3.40 (m, 2H), 3.32 - 3.21 (m, 2H), 3.02 - 2.93 (m, 1H), 2.86 - 2.76 (m, 1H), 2.61 - 2.45 (m, 1H), 2.25 - 2.17 (m, 1H). LCMS (ESI) calcd for $C_{24}H_{25}N_6O_3S^+$ [M+H]$^+$: 477.14, found, 477.2.

Example 180: Preparation of 3-(5-((4-(5,6-dimethylthieno[2,3-d]pyrimidin-4-yl)-1,4-diazepan-1-yl)methyl)-1-oxoisoindolin-2-yl)piperidine-2,6-dione (GT-04707)

Referring to the method of Scheme 1, the target compound (GT-04707) was prepared under appropriate conditions as would be understood by those skilled in the art (white solid, 32 mg, yield 43%). $^1$H NMR (400 MHz, MeOD) δ 8.65 (s, 1H), 7.95 - 7.89 (m, 2H), 7.76 (d, J = 8.0 Hz, 1H), 5.20 (dd, J = 13.2, 5.2 Hz, 1H), 4.68 - 4.53 (m, 5H), 4.28 (brs, 1H), 4.14 (brs, 1H), 3.96 (brs, 1H), 3.74 (brs, 2H), 3.62 (brs, 1H), 3.01 - 2.87 (m, 1H), 2.87 - 2.76 (m, 1H), 2.61 - 2.51 (m, 2H), 2,54 (s, 3H), 2.50 - 2.41 (m, 1H), 2.45(s, 3H), 2.37- 2.32 (m, 1H), 2.26 - 2.15 (m, 1H). LCMS (ESI) calcd for $C_{27}H_{31}N_6O_3S^+$ [M+H]$^+$: 519.22, found, 519.2.

Example 181: Preparation of 3-(1-oxo-5-((4-(thieno[2,3-d]pyrimidin-4-yl)piperidin-1-yl)methyl)isoindolin-2-yl)piperidine-2,6-dione (GT-04702)

Referring to the method of Scheme 1, the target compound (GT-04702) was prepared under appropriate conditions as would be understood by those skilled in the art (white solid, 25 mg, yield 31%). $^1$H NMR (400 MHz, MeOD) δ 9.00 (s, 1H), 7.97 (d, J = 7.8 Hz, 1H), 7.88 (d, J = 6.1 Hz, 1H), 7.85 (s, 1H), 7.76 (d, J = 7.8 Hz, 1H), 7.73 (d, J = 6.1 Hz, 1H), 5.22 (dd, J = 13.3, 5.1 Hz, 1H), 4.82 (s, 1H), 4.68 - 4.54 (m, 4H), 3.74 (t, J = 13.9 Hz, 3H), 2.95 (ddd, J = 18.7, 13.5, 5.4 Hz, 1H), 2.88 - 2.76 (m, 1H), 2.55 (qd, J = 13.1, 4.6 Hz, 1H), 2.36 (dd, J = 24.2, 11.7 Hz, 2H), 2.28 - 2.14 (m, 3H). LCMS (ESI) calcd for $C_{25}H_{26}N_5O_3S^+$ [M+H]$^+$: 476.18, found, 476.2.

Example 182: Preparation of 3-(5-((4-(6-methylthieno[2,3-d]pyrimidin-4-yl)piperidin-1-yl)methyl)-1-oxoisoindolin-2-yl) piperidine-2,6-dione (GT-04717)

Referring to the method of Scheme 1, the target compound (GT-04717) was prepared under appropriate conditions as would be understood by those skilled in the art (white solid, 28 mg, yield 29%). $^1$H NMR (400 MHz, MeOD) δ 9.00 (d, *J* = 10.8 Hz, 1H), 7.96 (d, *J* = 7.8 Hz, 1H), 7.90 (s, 1H), 7.79 (d, *J* = 7.3 Hz, 1H), 7.57 (s, 1H), 5.21 (dd, *J* = 13.3, 5.1 Hz, 1H), 4.71 - 4.48 (m, 5H), 3.72 (d, *J* = 12.2 Hz, 2H), 3.40 (s, 2H), 2.99 - 2.90 (m, 1H), 2.86 - 2.79 (m, 1H), 2.74 (s, 3H), 2.55 (dd, *J* = 13.0, 4.5 Hz, 1H), 2.40 (t, *J* = 12.4 Hz, 2H), 2.25 (d, *J* = 14.7 Hz, 3H). LCMS (ESI) calcd for $C_{26}H_{28}N_5O_3S^+$ [M+H]$^+$: 490.19, found, 490.3.

Example 183: Preparation of 3-(5-((4-(5,6-dimethylthieno[2,3-d]pyrimidin-4-yl)piperidin-1-yl)methyl)-1-oxoisoindolin-2-yl)piperidine-2,6-dione (GT-05034)

Referring to the method of Scheme 1, the target compound (GT-05034) was prepared under appropriate conditions as would be understood by those skilled in the art (white solid, 39 mg, yield 51%). $^1$H NMR (400 MHz, MeOD-d4) δ 8.93 (s, 1H), 7.93 (t, *J* = 8.1 Hz, 1H), 7.86 (s, 1H), 7.76 (d, *J* = 7.9 Hz, 1H), 5.18 (dd, *J* = 13.3, 5.2 Hz, 1H), 4.64 - 4.51 (m, 4H), 4.07 - 4.00 (m, 1H), 3.68 (d, *J* = 12.2 Hz, 2H), 3.42 - 3.35 (m, 2H), 2.99 - 2.85 (m, 1H), 2.82 - 2.78 (m, 1H), 2.62 (s, 3H), 2.59 (s, 3H), 2.47 - 2.35 (m, 3H), 2.24 - 2.18 (m, 3H). LCMS (ESI) calcd for $C_{27}H_{30}N_5O_3S^+$ [M+H]$^+$: 504.21, found, 504.2.

Example 184: Preparation of 3-(1-oxo-5-((4-(6-phenylthieno[2,3-d]pyrimidin-4-yl)piperidin-1-yl)methyl)isoindolin-2-yl) piperidine-2,6-dione (GT-05159)

Referring to the method of Scheme 1, the target compound (GT-05159) was prepared under appropriate conditions as would be understood by those skilled in the art (white solid, 31 mg, yield 37%). $^1$H NMR (400 MHz, MeOD-d4) δ 8.93 (s, 1H), 8.00 (s, 1H), 7.96 (d, *J* = 7.9 Hz, 1H), 7.88 - 7.81 (m, 3H), 7.75 (d, *J* = 7.8 Hz, 1H), 7.56 - 7.44 (m, 3H), 5.19 (dd, *J* = 13.3, 5.2 Hz, 1H), 4.67 - 4.54 (m, 4H), 3.77 - 3.70 (m, 3H), 3.37 - 3.33 (m, 2H), 2.99 - 2.87 (m, 1H), 2.83 - 2.77 (m, 1H), 2.60 - 2.45 (m, 1H), 2.36 - 2.19 (m, 5H). LCMS (ESI) calcd for $C_{31}H_{30}N_5O_3S^+$ [M+H]$^+$: 552.21, found, 552.2.

Example 185: Preparation of 3-(1-oxo-5-((4-(5-phenylthieno[2,3-d]pyrimidin-4-yl)piperidin-1-yl)methyl)isoindolin-2-yl) piperidine-2,6-dione (GT-04995)

Referring to the method of Scheme 1, the target compound (GT-04995) was prepared under appropriate conditions as would be understood by those skilled in the art (white solid, 48 mg, yield 58%). $^1$H NMR (400 MHz, MeOD-d4) δ 8.99 (s, 1H), 7.92 (d, *J* = 7.8 Hz, 1H), 7.72 (s, 1H), 7.66 - 7.62 (m, 2H), 7.56 - 7.46 (m, 5H), 5.20 (dd, *J* = 13.4, 5.2 Hz, 1H), 4.64 - 4.55 (m, 2H), 4.39 (s, 2H), 3.49 (d, *J* = 11.8 Hz, 2H), 3.00 - 2.88 (m, 2H), 2.84 - 2.75 (m, 1H), 2.59 - 2.48 (m, 1H), 2.42 - 2.36 (m, 2H), 2.24 - 2.14 (m, 3H), 1.91 (d, *J* = 13.9 Hz, 2H). LCMS (ESI) calcd for $C_{31}H_{30}N_5O_3S^+$ [M+H]$^+$: 552.21, found, 552.2.

Example 186: Preparation of 3-(1-oxo-5-((4-(5,6,7,8-tetrahydrobenzo[4,5]thieno[2,3-d]pyrimidin-4-yl)piperidin-1-yl) methyl)isoindolin-2-yl)piperidine-2,6-dione (GT-05265)

Referring to the method of Scheme 1, the target compound (GT-05265) was prepared under appropriate conditions as would be understood by those skilled in the art (white solid, 34 mg, yield 35%). $^1$H NMR (400 MHz, MeOD-d4) δ 8.91 (s, 1H), 7.93 (d, *J* = 7.8 Hz, 1H), 7.87 (s, 1H), 7.76 (d, *J* = 7.8 Hz, 1H), 5.18 (dd, *J* = 13.3, 5.1 Hz, 1H), 4.64 - 4.52 (m, 4H), 3.90 (t, *J* = 12.0 Hz, 1H), 3.67 (d, *J* = 12.4 Hz, 2H), 3.39 (dd, *J* = 12.9, 10.5 Hz, 2H), 3.13 - 3.09 (m, 2H), 2.98 - 2.95 (m, 2H), 2.94 - 2.85 (m, 1H), 2.82 - 2.79 (m, 1H), 2.55 - 2.51 (m, 1H), 2.47 - 2.36 (m, 2H), 2.25 - 2.17 (m, 3H), 2.01 - 1.96 (m, 5H). LCMS (ESI) calcd for $C_{29}H_{32}N_5O_3S^+$ [M+H]$^+$: 530.22, found, 530.2.

Example 187: Preparation of 3-(5-((4-(6,7-dihydro-5H-cyclopenta[4,5]thieno[2,3-d]pyrimidin-4-yl)piperidin-1-yl) methyl)-1-oxoisoindolin-2-yl)piperidine-2,6-dione (GT-04994)

Referring to the method of Scheme 1, the target compound (GT-04994) was prepared under appropriate conditions as would be understood by those skilled in the art (white solid, 40 mg, yield 51%). $^1$H NMR (400 MHz, MeOD-d4) δ 8.94 (s, 1H), 7.92 (d, *J* = 7.8 Hz, 1H), 7.89 (s, 1H), 7.77 (d, *J* = 7.8 Hz, 1H), 5.18 (dd, *J* = 13.3, 5.1 Hz, 1H), 4.67 - 4.52 (m, 4H), 3.72 - 3.67(m, 3H), 3.43 - 3.37 (m, 2H), 3.26 - 3.22 (m, 2H), 3.17 - 3.13 (m, 3H), 2.97 - 2.87 (m, 1H), 2.82 - 2.77 (m, 1H), 2.66 - 2.59 (m, 2H), 2.42 - 2.38 (m, 2H), 2.30 - 2.14 (m, 4H). LCMS (ESI) calcd for $C_{28}H_{30}N_5O_3S^+$ [M+H]$^+$: 516.21, found, 516.3.

Example 188: Preparation of 3-(5-((4-(2-methyl-5-phenylthieno[2,3-d]pyrimidin-4-yl)piperidin-1-yl)methyl)-1-oxoisoindolin-2-yl)piperidine-2,6-dione (GT-05158)

Referring to the method of Scheme 1, the target compound (GT-05158) was prepared under appropriate conditions as would be understood by those skilled in the art (white solid, 52 mg, yield 61%). $^1$H NMR (400 MHz, MeOD-d4) δ 7.92 (d, *J* = 7.8 Hz, 1H), 7.75 (s, 1H), 7.64 (d, *J* = 8.1 Hz, 2H), 7.52 - 7.45 (m, 5H), 5.20 (dd, *J* = 13.3, 5.1 Hz, 1H), 4.59 (q, *J* = 17.6 Hz, 2H), 4.39 (s, 2H), 3.50 (d, *J* = 11.7 Hz, 2H), 3.02 - 2.91 (m, 2H), 2.91 - 2.85 (m, 1H), 2.81 (s, 3H), 2.59 - 3.52 (m, 1H), 2.38 - 2.19 (m, 5H), 1.92 (d, *J* = 14.1 Hz, 2H). LCMS (ESI) calcd for $C_{32}H_{32}N_5O_3S^+$ [M+H]$^+$: 566.22, found, 566.3.

Example 189: Preparation of 3-(5-((4-(2-methyl-5,6,7,8-tetrahydrobenzo[4,5]thieno[2,3-d]pyrimidin-4-yl)piperidin-1-yl) methyl)-1-oxoisoindolin-2-yl)piperidine-2,6-dione (GT-04993)

Referring to the method of Scheme 1, the target compound (GT-04993) was prepared under appropriate conditions as would be understood by those skilled in the art (white solid, 15 mg, yield 18%). $^1$H NMR (400 MHz, MeOD-d4) δ 7.93 (d, *J* = 7.8 Hz, 1H), 7.88 (s, 1H), 7.76 (d, *J* = 7.2 Hz, 1H), 5.18 (dd, *J*= 13.3, 5.2 Hz, 1H), 4.64 - 4.55 (m, 4H), 3.92 (t, *J* = 12.0 Hz, 1H), 3.67 (d, *J* = 12.4 Hz, 2H), 3.37 (t,*J* = 11.8 Hz, 2H), 3.12 - 3.05 (m, 2H), 2.97 - 2.92 (m, 3H), 2.89 - 2.84 (m, 1H), 2.81 (s, 3H), 2.55 - 2.43 (m, 3H), 2.25 - 2.18 (m, 3H), 2.02 - 1.95 (m, 4H). LCMS (ESI) calcd for $C_{30}H_{34}N_5O_3S^+$ [M+H]$^+$: 544.24, found, 544.3.

Example 190: Preparation of 3-(5-((4-(2-methyl-6,7-dihydro-5H-cyclopenta[4,5]thieno[2,3-d]pyrimidin-4-yl)piperidin-1-yl)methyl)-1-oxoisoindolin-2-yl)piperidine-2,6-dione (GT-05210)

Referring to the method of Scheme 1, the target compound (GT-05210) was prepared under appropriate conditions as would be understood by those skilled in the art (white solid, 31 mg, yield 39%). $^1$H NMR (400 MHz, MeOD-d4) δ 7.94 - 7.90 (m, 2H), 7.78 (d, *J* = 7.7 Hz, 1H), 5.18 (dd, *J* = 13.3, 5.2 Hz, 1H), 4.64 - 4.51 (m, 4H), 3.83 - 3.67 (m, 3H), 3.43 - 3.37 (m, 2H), 3.27 - 3.23 (m, 2H), 3.16 - 3.13 (m, 2H), 2.95 - 2.90 (m, 1H), 2.87 (s, 3H), 2.84 - 2.74 (m, 1H), 2.67 - 2.59 (m, 2H), 2.56 - 2.46 (m, 3H), 2.41 - 2.38 (m, 1H), 2.25 - 2.18 (m, 2H). LCMS (ESI) calcd for $C_{29}H_{32}N_5O_3S^+$ [M+H]$^+$: 530.22, found, 530.3.

Example 191: Preparation of 3-(5-((4-(2-methylthieno[2,3-d]pyrimidin-4-yl)piperidin-1-yl)methyl)-1-oxoisoindolin-2-yl) piperidine-2,6-dione (GT-04726)

Referring to the method of Scheme 1, the target compound (GT-04726) was prepared under appropriate conditions as would be understood by those skilled in the art (white solid, 19 mg, yield 20%). $^1$H NMR (400 MHz, MeOD) δ 7.99 - 7.87 (m, 4H), 7.77 (dd, *J* = 20.5, 6.9 Hz, 1H), 5.21 (dd, *J* = 13.3, 5.1 Hz, 1H), 4.68 - 4.50 (m, 4H), 3.77 (dd, *J* = 31.8, 12.4 Hz, 3H), 3.39 (d, *J* = 13.0 Hz, 2H), 3.02 - 2.89 (m, 1H), 2.89 - 2.79 (m, 4H), 2.61 - 2.43 (m, 3H), 2.24 (dd, *J* = 18.5, 10.7 Hz, 3H). LCMS (ESI) calcd for $C_{26}H_{28}N_5O_3S^+$ [M+H]$^+$: 490.19, found, 490.2.

Example 192: Preparation of 3-(1-oxo-5-((4-(thieno[2,3-d]pyrimidin-2-yl)piperidin-1-yl)methyl)isoindolin-2-yl)piperidine-2,6-dione (GT-04933)

Referring to the method of Scheme 1, the target compound (GT-04933) was prepared under appropriate conditions as would be understood by those skilled in the art (white solid, 16 mg, yield 22%). $^1$H NMR (400 MHz, MeOD-d4) δ 9.36 (s, 1H), 7.96 - 7.93 (m, 2H), 7.85 (s, 1H), 7.75 (d, *J* = 7.5 Hz, 1H), 7.61 (d, *J* = 6.0 Hz, 1H), 5.19 (dd, *J* = 13.3, 5.1 Hz, 1H), 4.60 - 4.54 (m, 4H), 3.70 (d, *J* = 12.4 Hz, 2H), 3.54 - 3.39 (m, 2H), 3.01 - 2.86 (m, 1H), 2.85 - 2.74 (m, 1H), 2.59 - 2.48 (m, 1H), 2.41 - 2.33(m, 3H), 2.35 - 2.24 (m, 2H), 2.21 - 2.18 (m, 1H). LCMS (ESI) calcd for $C_{25}H_{26}N_5O_3S^+$ [M+H]$^+$: 476.18, found, 476.2.

Example 193: Preparation of 3-(5-((4-(5-ethylpyrimidin-2-yl)piperidin-1-yl)methyl)-1-oxoisoindolin-2-yl)piperidine-2,6-dione (GT-04936)

Referring to the method of Scheme 1, the target compound (GT-04936) was prepared under appropriate conditions as would be understood by those skilled in the art (white solid, 25 mg, yield 36%). $^1$H NMR (400 MHz, MeOD-d4) δ 8.95 (s, 2H), 7.91 (t, *J* = 7.5 Hz, 1H), 7.87 (s, 1H), 7.75 (d, *J* = 7.4 Hz, 1H), 5.18 (dd, *J* = 13.1, 4.9 Hz, 1H), 4.64 - 4.49 (m, 4H), 3.67 (d, *J* = 11.6 Hz, 2H), 3.48 - 3.34 (M, 2H), 2.98 - 2.85 (m, 1H), 2.84 - 2.78 (m, 3H), 2.60 - 2.45 (m, 1H), 2.44 - 2.13 (m, 6H), 1.32 (t, *J* = 7.5 Hz, 3H). LCMS (ESI) calcd for $C_{25}H_{30}N_5O_3S^+$ [M+H]$^+$: 448.23, found, 448.3.

Example 194: Preparation of 3-(1-oxo-5-((4-(thieno[2,3-d]pyrimidin-4-yl)-3,6-dihydropyridin-1(2H)-yl)methyl)isoindolin-2-yl)piperidine-2,6-dione (GT-04692)

Referring to the method of Scheme 1, the target compound (GT-04692) was prepared under appropriate conditions as would be understood by those skilled in the art (white solid, 23 mg, yield 23%). $^1$H NMR (400 MHz, MeOD) δ 9.08 (s, 1H), 7.97 (dd, *J* = 15.6, 7.0 Hz, 2H), 7.92 (s, 1H), 7.84 (d, *J* = 8.3 Hz, 1H), 7.79 (d, *J* = 6.1 Hz, 1H), 6.71 (s, 1H), 5.22 (dd, *J* = 17.8, 8.9 Hz, 1H), 4.59 - 4.51 (m, 4H), 4.11 (s, 2H), 3.96 - 3.90 (m, 1H), 3.59 - 3.50 (m, 1H), 3.22 - 3.18 (m, 3H), 3.00 - 2.90 (m, 1H), 2.84 (d, *J* = 2.3 Hz, 1H), 2.57 (dd, *J* = 13.2, 4.4 Hz, 1H), 2.26 - 2.18 (m, 1H). LCMS (ESI) calcd for $C_{25}H_{24}N_5O_3S^+$ [M+H]$^+$: 474.16, found, 474.1.

Example 195: Preparation of 3-(5-((4-(5-methylthieno[2,3-d]pyrimidin-4-yl)-3,6-dihydropyridin-1(2H)-yl)methyl)-1-oxoisoindolin-2-yl)piperidine-2,6-dione (GT-04703)

Referring to the method of Scheme 1, the target compound (GT-04703) was prepared under appropriate conditions as would be understood by those skilled in the art (white solid, 31 mg, yield 40%). $^1$H NMR (400 MHz, MeOD) δ 9.04 (s, 1H), 7.98 (d, J = 7.8 Hz, 1H), 7.91 (s, 1H), 7.82 (d, J = 8.1 Hz, 1H), 7.59 (s, 1H), 6.01 (s, 1H), 5.22 (dd, J = 13.3, 5.2 Hz, 1H), 4.71 (d, J = 3.7 Hz, 2H), 4.62 (q, J = 17.5 Hz, 2H), 4.08 (s, 2H), 3.89 (s, 1H), 3.57 (s, 1H), 3.19 - 3.01 (m, 2H), 3.00 - 2.88 (m, 1H), 2.88 - 2.76 (m, 1H), 2.61 - 2.53 (m, 1H), 2.52 (s, 3H), 2.28 - 2.16 (m, 1H). LCMS (ESI) calcd for $C_{26}H_{26}N_5O_3S^+$ [M+H]$^+$: 488.18, found, 488.2.

Example 196: Preparation of 3-(5-((4-(6-methylthieno[2,3-d]pyrimidin-4-yl)-3,6-dihydropyridin-1(2H)-yl)methyl)-1-oxoisoindolin-2-yl)piperidine-2,6-dione (GT-04704)

Referring to the method of Scheme 1, the target compound (GT-04704) was prepared under appropriate conditions as would be understood by those skilled in the art (white solid, 32 mg, yield 41%). $^1$H NMR (400 MHz, MeOD) δ 8.95 (s, 1H), 7.99 (d, J = 7.8 Hz, 1H), 7.88 (s, 1H), 7.80 (d, J = 8.3 Hz, 1H), 7.43 (s, 1H), 6.65 (s, 1H), 5.22 (dd, J = 13.3, 5.2 Hz, 1H), 4.69 (s, 2H), 4.62 (d, J = 7.3 Hz, 2H), 4.10 (s, 2H), 3.90 (s, 1H), 3.15 (s, 2H), 3.00 - 2.89 (m, 1H), 2.84 (s, 1H), 2.79 (d, J = 11.7 Hz, 1H), 2.70 (s, 3H), 2.62 - 2.50 (m, 1H), 2.26 - 2.20 (m, 1H). LCMS (ESI) calcd for $C_{26}H_{26}N_5O_3S^+$ [M+H]$^+$: 488.18, found, 488.2.

Example 197: Preparation of 3-(5-((4-(6-isopropylthieno[2,3-d]pyrimidin-4-yl)-3,6-dihydropyridin-1(2H)-yl)methyl)-1-oxoisoindolin-2-yl)piperidine-2,6-dione (GT-04935)

Referring to the method of Scheme 1, the target compound (GT-04935) was prepared under appropriate conditions as would be understood by those skilled in the art (white solid, 38 mg, yield 49%). $^1$H NMR (400 MHz, MeOD-d4) δ 8.99 (s, 1H), 7.99 - 7.88 (m, 2H), 7.80 (d, *J* = 6.6 Hz, 1H), 7.49 (s, 1H), 6.63 (s, 1H), 5.19 (dd, *J* = 13.3, 5.1 Hz, 1H), 4.68 (s, 2H), 4.58 (q, *J* = 12.9 Hz, 2H), 4.11 (d, *J* = 2.4 Hz, 2H), 3.92 - 3.85 (m, 1H), 3.57 - 3.50 (m, 1H), 3.44 - 3.35 (m, 1H), 3.15 (brs, 2H), 3.02 - 2.85 (m, 1H), 2.85 - 2.73 (m, 1H), 2.59 - 2.51 (m, 2H), 2.46 - 2.35 (m, 1H), 2.26 - 2.15 (m, 1H), 1.44 (s, 3H), 1.43 (s, 3H). LCMS (ESI) calcd for $C_{28}H_{30}N_4O_3S^+$ [M+H]$^+$: 516.21, found, 516.2.

Example 198: Preparation of 3-(5-((4-(5,6-dimethylthieno[2,3-d]pyrimidin-4-yl)-3,6-dihydropyridin-1(2H)-yl)methyl)-1-oxoisoindolin-2-yl)piperidine-2,6-dione (GT-04727)

Referring to the method of Scheme 1, the target compound (GT-04727) was prepared under appropriate conditions as would be understood by those skilled in the art (white solid, 34 mg, yield 36%). $^1$H NMR (400 MHz, MeOD) δ 8.95 (s, 1H), 7.97 (t, *J* = 9.5 Hz, 1H), 7.89 (s, 1H), 7.82 (t, *J* = 7.9 Hz, 2H), 7.70 (s, 1H), 7.62 (d, *J* = 8.0 Hz, 1H), 5.96 (s, 1H), 5.52 (s, 1H), 5.20 (td, *J* = 14.0, 5.1 Hz, 2H), 4.69 (d, *J* = 11.7 Hz, 2H), 4.61 (t, *J* = 9.0 Hz, 2H), 4.51 (t, *J* = 12.6 Hz, 2H), 4.06 (s, 2H), 2.94 (ddd, *J* = 23.8, 12.7, 6.0 Hz, 3H), 2.87 - 2.77 (m, 2H), 2.39 (s, 3H), 2.21 (dd, *J* = 12.9, 5.1 Hz, 2H). LCMS (ESI) calcd for $C_{27}H_{28}N_5O_3S^+$ [M+H]$^+$: 502.19, found, 502.2.

Example 199: Preparation of 3-(1-oxo-5-((4-(6-phenylthieno[2,3-d]pyrimidin-4-yl)-3,6-dihydropyridin-1(2H)-yl)methyl) isoindolin-2-yl)piperidine-2,6-dione (GT-05101)

Referring to the method of Scheme 1, the target compound (GT-05101) was prepared under appropriate conditions as would be understood by those skilled in the art (white solid, 51 mg, yield 61%). $^1$H NMR (400 MHz, MeOD-d4) δ 8.99 (s, 1H), 7.96 (d, *J* = 6.9 Hz, 2H), 7.85 (d, *J* = 7.0 Hz, 3H), 7.80 (d, *J* = 7.6 Hz, 1H), 7.53 - 7.48 (m, 3H), 6.74 (s, 1H), 5.20 (dd, *J* = 13.3, 5.2 Hz, 1H), 4.64 - 4.53 (m, 4H), 4.09 (s, 2H), 3.93 - 3.84 (m, 1H), 3.56 - 3.46 (m, 1H), 3.23 - 3.07 (m, 2H), 3.01 - 2.86 (m, 1H), 2.80 - 2.79 (m, 1H), 2.54 - 2.51 (m, 1H), 2.20 - 2.17 (m, 1H). LCMS (ESI) calcd for $C_{31}H_{28}N_5O_3S^+$ [M+H]$^+$: 550.19, found, 550.2.

Example 200: Preparation of 3-(1-oxo-5-((4-(5-phenylthieno[2,3-d]pyrimidin-4-yl)-3,6-dihydropyridin-1(2H)-yl)methyl) isoindolin-2-yl)piperidine-2,6-dione (GT-04908)

Referring to the method of Scheme 1, the target compound (GT-04908) was prepared under appropriate conditions as would be understood by those skilled in the art (white solid, 26 mg, yield 31%). $^1$H NMR (400 MHz, MeOD) δ 9.07 (s, 1H), 7.96 (d, *J* = 7.8 Hz, 1H), 7.80 (s, 1H), 7.75 (d, *J* = 5.9 Hz, 1H), 7.67 (d, *J* = 7.9 Hz, 1H), 7.44 - 7.33 (m, 4H), 7.24 (t, *J* = 7.1 Hz, 1H), 5.48 (s, 1H), 5.22 (dd, *J* = 13.4, 5.3 Hz, 1H), 4.69 - 4.53 (m, 2H), 4.46 (s, 2H), 3.52 - 3.47 (m, 2H), 3.07 - 2.87 (m, 3H), 2.85 - 2.77 (m, 1H), 2.74 - 2.66 (m, 2H), 2.54 (qd, *J* = 13.2, 4.7 Hz, 1H), 2.29 - 2.13 (m, 1H). LCMS (ESI) calcd for $C_{31}H_{28}N_5O_3S^+$ [M+H]$^+$: 550.19, found, 550.2.

Example 201: Preparation of 3-(1-oxo-5-((4-(5,6,7,8-tetrahydrobenzo[4,5]thieno[2,3-d]pyrimidin-4-yl)-3,6-dihydropyridin-1(2H)-yl)methyl)isoindolin-2-yl)piperidine-2,6-dione (GT-05266)

Referring to the method of Scheme 1, the target compound (GT-05266) was prepared under appropriate conditions as would be understood by those skilled in the art (white solid, 49 mg, yield 51%). $^1$H NMR (400 MHz, MeOD-d4) δ 9.12 (s, 1H), 7.97 (s, 1H), 7.94 (d, *J* = 7.8 Hz, 1H), 7.84 (d, *J* = 7.3 Hz, 1H), 6.18 (s, 1H), 5.19 (dd, *J* = 13.3, 5.1 Hz, 1H), 4.71 (s, 2H), 4.60 (q, *J* = 17.5 Hz, 2H), 4.10 (s, 2H), 3.96 - 3.78 (m, 1H), 3.62 - 3.54 (m, 1H), 3.05 - 3.01 (m, 3H), 2.98 - 2.70 (m, 5H), 2.58 - 2.49 (m, 1H), 2.26 - 2.15 (m, 1H), 2.03 - 1.87 (m, 4H). LCMS (ESI) calcd for $C_{29}H_{30}N_5O_3S^+$ [M+H]$^+$: 528.21, found, 528.3.

Example 202: Preparation of 3-(5-((4-(6,7-dihydro-5H-cyclopenta[4,5]thieno[2,3-d]pyrimidin-4-yl)-3,6-dihydropyridin-1(2H)-yl)methyl)-1-oxoisoindolin-2-yl)piperidine-2,6-dione (GT-04934)

Referring to the method of Scheme 1, the target compound (GT-04934) was prepared under appropriate conditions as would be understood by those skilled in the art (white solid, 45 mg, yield 58%). $^1$H NMR (400 MHz, MeOD-d4) δ 8.95 (s, 1H), 7.95 (d, *J* = 7.9 Hz, 1H), 7.91 (d, *J* = 7.0 Hz, 1H), 7.80 (d, *J* = 7.3 Hz, 1H), 6.20 (s, 1H), 5.19 (dd, *J* = 13.3, 5.1 Hz, 1H), 4.68 (s, 2H), 4.65 - 4.52 (m, 2H), 4.08 (s, 2H), 3.95 - 3.77 (m, 1H), 3.65 - 3.43 (m, 1H), 3.15 - 3.09 (m, 4H), 2.98 (brs, 2H), 2.95 - 2.85 (m, 1H), 2.86 - 2.74 (m, 1H), 2.63 - 2.46 (m, 3H), 2.27 - 2.13 (m, 1H). LCMS (ESI) calcd for $C_{28}H_{28}N_5O_3S^+$

$[M+H]^+$: 514.19, found, 514.2.

Example 203: Preparation of 3-(5-((4-(2-methyl-5-phenylthieno[2,3-d]pyrimidin-4-yl)-3,6-dihydropyridin-1(2H)-yl) methyl)-1-oxoisoindolin-2-yl)piperidine-2,6-dione (GT-05102)

Referring to the method of Scheme 1, the target compound (GT-05102) was prepared under appropriate conditions as would be understood by those skilled in the art (white solid, 53 mg, yield 62%). $^1$H NMR (400 MHz, MeOD-d4) δ 7.96 (d, $J$ = 7.8 Hz, 1H), 7.77 (s, 1H), 7.73 (s, 1H), 7.68 (d, $J$ = 7.8 Hz, 1H), 7.43 - 7.33 (m, 4H), 7.24 (t, $J$ = 7.2 Hz, 1H), 5.60 (s, 1H), 5.22 (dd, $J$ = 13.3, 5.1 Hz, 1H), 4.68 - 4.54 (m, 2H), 4.47 (s, 2H), 3.61 - 3.51 (m, 1H), 3.49 - 3.38 (m, 1H), 3.17 - 3.02 (m, 1H), 3.00 - 2.88 (m, 2H), 2.85 (s, 3H), 2.83 - 2.75 (m, 1H), 2.60 - 2.49 (m, 3H), 2.27 - 2.17 (m, 1H). LCMS (ESI) calcd for $C_{32}H_{30}N_5O_3S^+$ $[M+H]^+$: 564.21, found, 564.2.

Example 204: Preparation of 3-(5-((4-(2-methyl-5,6,7,8-tetrahydrobenzo[4,5]thieno[2,3-d]pyrimidin-4-yl)-3,6-dihydropyridin-1 (2H)-yl)methyl)-1-oxoisoindolin-2-yl)piperidine-2,6-dione (GT-04863)

Referring to the method of Scheme 1, the target compound (GT-04863) was prepared under appropriate conditions as would be understood by those skilled in the art (white solid, 24 mg, yield 33%). $^1$H NMR (400 MHz, DMSO) δ 11.50 (s, 1H), 11.01 (s, 1H), 7.97 (s, 1H), 7.86 (s, 2H), 5.84 (s, 1H), 5.15 (dd, $J$= 13.2, 5.1 Hz, 1H), 4.66 - 4.38 (m, 4H), 3.82 - 3.73 (m, 2H), 3.69 - 3.66 (m, 1H), 3.45 - 3.39 (m, 1H), 3.10 - 2.95 (m, 1H), 2.95 - 2.74 (m, 6H), 2.68 (s, 3H), 2.62 (d, $J$ = 15.5 Hz, 2H), 2.46 - 2.41 (m, 1H), 2.08 - 1.99 (m, 1H), 1.89 - 1.70 (m, 4H). LCMS (ESI) calcd for $C_{30}H_{32}N_5O_3S^+$ $[M+H]^+$: 542.22, found, 542.3.

Example 205: Preparation of 3-(5-((4-(2-methyl-6,7-dihydro-5H-cyclopenta[4,5]thieno[2,3-d]pyrimidin-4-yl)-3,6-dihydropyridin-1(2H)-yl)methyl)-1-oxoisoindolin-2-yl)piperidine-2,6-dione (GT-05211)

Referring to the method of Scheme 1, the target compound (GT-05211) was prepared under appropriate conditions as would be understood by those skilled in the art (white solid, 26 mg, yield 32%). $^1$H NMR (400 MHz, MeOD-d4) δ 7.97 - 7.92 (m, 2H), 7.83 (d, $J$ = 7.9 Hz, 1H), 6.32 (s, 1H), 5.19 (dd, $J$ = 13.3, 5.1 Hz, 1H), 4.76 - 4.67 (m, 2H), 4.66 - 4.52 (m, 2H), 4.11 (s, 2H), 3.93 - 3.78 (m, 1H), 3.64 - 3.46 (m, 1H), 3.21 - 2.97 (m, 6H), 2.91 (s, 3H), 2.84 - 2.74 (m, 1H), 2.61 - 2.46 (m, 3H), 2.45 - 2.36 (m, 1H), 2.26 - 2.13 (m, 1H). LCMS (ESI) calcd for $C_{29}H_{30}N_5O_3S^+$ $[M+H]^+$: 528.21, found, 528.3.

Example 206: Preparation of 3-(5-((4-(2-methylthieno[2,3-d]pyrimidin-4-yl)-3,6-dihydropyridin-1(2H)-yl)methyl)-1-oxoisoindolin-2-yl)piperidine-2,6-dione (GT-04718)

Referring to the method of Scheme 1, the target compound (GT-04718) was prepared under appropriate conditions as would be understood by those skilled in the art (white solid, 3 mg, yield 3%). $^1$H NMR (400 MHz, MeOD) δ 7.99 (d, $J$ = 7.7 Hz, 1H), 7.87 (s, 1H), 7.82 - 7.76 (m, 2H), 7.66 (d, $J$ = 6.1 Hz, 1H), 6.64 (s, 1H), 5.22 (dd, $J$ = 13.3, 5.3 Hz, 1H), 4.89 (s, 2H), 4.85 - 4.85 (m, 2H), 4.71 - 4.57 (m, 4H), 4.10 (s, 2H), 2.94 (d, $J$ = 12.5 Hz, 1H), 2.84 (s, 1H), 2.79 (s, 3H), 2.55 (d, $J$ = 8.9 Hz, 1H), 2.22 (s, 1H). LCMS (ESI) calcd for $C_{26}H_{26}N_5O_3S^+$ $[M+H]^+$: 488.18, found, 488.3.

Example 207: Preparation of 3-(1-oxo-5-((4-(thieno[2,3-d]pyrimidin-2-yl)-3,6-dihydropyridin-1(2H)-yl)methyl)isoindolin-2-yl)piperidine-2,6-dione (GT-04708)

Referring to the method of Scheme 1, the target compound (GT-04708) was prepared under appropriate conditions as would be understood by those skilled in the art (white solid, 17 mg, yield 21%). $^1$H NMR (400 MHz, MeOD) δ 9.29 (s, 1H), 7.98 (d, J = 7.9 Hz, 1H), 7.87 (d, J = 6.4 Hz, 2H), 7.79 (d, J = 8.0 Hz, 1H), 7.56 (d, J = 6.1 Hz, 1H), 7.30 (s, 1H), 5.21 (dd, J = 13.6, 5.3 Hz, 1H), 4.68 (d, J = 5.2 Hz, 2H), 4.62 (d, J = 7.6 Hz, 2H), 4.11 (s, 2H), 3.96 -3.87(m, 1H), 3.50-3.48 (m, 1H), 3.15 - 3.08 (m, 1H), 3.00 - 2.89 (m, 1H), 2.81 (d, J = 15.7 Hz, 1H), 2.55 (dd, J = 13.2, 5.0 Hz, 1H), 2.25 - 2.19 (m, 1H), 1.35 - 1.31 (m, 1H). LCMS (ESI) calcd for $C_{25}H_{24}N_5O_3S^+$ $[M+H]^+$: 474.16, found, 474.1.

Example 208: Preparation of 3-(5-((4-(2-chloro-6,7-dihydrothieno[3,2-d]pyrimidin-4-yl)piperazin-1-yl)methyl)-1-oxoisoindolin-2-yl)piperidine-2,6-dione (GT-04683)

Referring to the method of Scheme 1, the target compound (GT-04683) was prepared under appropriate conditions as would be understood by those skilled in the art (white solid, 40 mg, yield 43%). $^1$H NMR (400 MHz, MeOD) δ 7.98 (d, $J$ = 7.7 Hz, 1H), 7.81 (s, 1H), 7.75 (d, $J$ = 8.1 Hz, 1H), 5.23 (dd, $J$ = 13.2, 5.1 Hz, 1H), 4.66 - 4.57 (m, 6H), 4.55 (s, 2H), 3.63 - 3.56 (m, 2H), 3.47 - 3.43 (m, 4H), 3.29 - 3.22(m, 2H), 3.03 - 2.90 (m, 1H), 2.85 - 2.83 (m, 1H), 2.55 (dd, $J$ = 13.3, 4.5 Hz, 1H), 2.24 - 2.19 (m, 1H). LCMS (ESI) calcd for $C_{24}H_{26}ClN_6O_3S^+$ $[M+H]^+$: 513.15, found, 513.2.

Example 209: Preparation of 3-(5-((4-(2-morpholino-6,7-dihydrothieno[3,2-d]pyrimidin-4-yl)piperazin-1-yl)methyl)-1-oxoisoindolin-2-yl)piperidine-2,6-dione (GT-04710)

Referring to the method of Scheme 1, the target compound (GT-04710) was prepared under appropriate conditions as would be understood by those skilled in the art (white solid, 31 mg, yield 45%). $^1$H NMR (400 MHz, MeOD) δ 7.95 (d, J = 7.8 Hz, 1H), 7.90 (s, 1H), 7.76 (d, J = 7.6 Hz, 1H), 5.20 (dd, J = 13.42 5.2 Hz, 1H), 4.98 - 4.88 (m, 4H), 4.61 - 4.56 (m, 4H), 4.53 - 4.51 (m, 1H), 3.82 - 3.76 (m, 9H), 3.56 - 3.52 (m, 3H), 3.44 - 3.40 (m, 3H), 2.94 - 2.90 (m, 1H), 2.86 - 2.74 (m, 1H), 2.57 -2.52 (m, 1H), 2.23 - 2.19 (m, 1H). LCMS (ESI) calcd for $C_{28}H_{34}N_7O_4S^+$ $[M+H]^+$: 564.24, found, 564.3.

Example 210: Preparation of 3-(1-oxo-5-((4-(4-(piperazin-1-yl)-6,7-dihydrothieno[3,2-d]pyrimidin-2-yl)piperazin-1-yl) methyl)isoindolin-2-yl)piperidine-2,6-dione (GT-04720)

Referring to the method of Scheme 1, the target compound (GT-04720) was prepared under appropriate conditions as would be understood by those skilled in the art (white solid, 25 mg, yield 93%). $^1$H NMR (400 MHz, MeOD) δ 7.94 (s, 2H), 7.80 (d, *J* = 7.4 Hz, 1H), 5.20 (dd, *J* = 13.2, 4.9 Hz, 1H), 4.61 (dd, *J* = 17.9, 6.9 Hz, 6H), 4.43 (s, 1H), 4.22 (t, *J* = 15.2 Hz, 4H), 3.69 (d, *J* = 5.2 Hz, 2H), 3.62 (s, 1H), 3.53 (d, *J* = 7.4 Hz, 4H), 3.43 (d, *J* = 14.5 Hz, 5H), 3.01 - 2.88 (m, 1H), 2.82 (d, *J* = 15.5 Hz, 1H), 2.62 - 2.37 (m, 2H), 2.23 (s, 1H). LCMS (ESI) calcd for $C_{28}H_{35}N_8O_4S^+$ $[M+H]^+$: 563.25, found, 563.3.

Example 211: Preparation of 3-(5-((4-(4-morpholino-6,7-dihydrothieno[3,2-d]pyrimidin-2-yl)piperazin-1-yl)methyl)-1-oxoisoindolin-2-yl)piperidine-2,6-dione (GT-04694)

Referring to the method of Scheme 1, the target compound (GT-04694) was prepared under appropriate conditions as would be understood by those skilled in the art (white solid, 41 mg, yield 47%). $^1$H NMR (400 MHz, MeOD) δ 7.98 - 7.90 (m, 2H), 7.78 (d, *J* = 7.8 Hz, 1H), 5.21 (dd, *J* = 13.4, 5.1 Hz, 1H), 4.83 (d, *J* = 5.0 Hz, 2H), 4.69 - 4.49 (m, 5H), 4.06 - 3.94 (m, 4H), 3.86 - 3.75 (m, 4H), 3.73 - 3.53 (m, 3H), 3.51 (dd, *J* = 12.9, 4.8 Hz, 3H), 3.39 (dd, *J* = 12.7, 4.6 Hz, 3H), 2.95 (ddd, *J* = 18.5, 13.5, 5.3 Hz, 1H), 2.87 - 2.76 (m, 1H), 2.55 (qd, *J* = 13.2, 4.7 Hz, 1H), 2.22 (ddd, *J* = 10.5, 5.3, 3.1 Hz, 1H). LCMS (ESI) calcd for $C_{28}H_{34}N_7O_4S^+$ $[M+H]^+$: 564.24, found, 564.3.

Example 212: Preparation of 3-(1-oxo-5-((4-(thieno[3,2-d]pyrimidin-4-yl)piperazin-1-yl)methyl)isoindolin-2-yl)piperidine-2,6-dione (GT-04681)

Referring to the method of Scheme 1, the target compound (GT-04681) was prepared under appropriate conditions as would be understood by those skilled in the art (white solid, 36 mg, yield 47%). $^1$H NMR (400 MHz, MeOD) δ 8.92 (s, 1H), 8.64 (d, *J* = 5.6 Hz, 1H), 7.99 (d, *J* = 7.8 Hz, 1H), 7.91 (s, 1H), 7.82 (d, *J* = 7.7 Hz, 1H), 7.68 (d, *J* = 5.6 Hz, 1H), 5.24 (dd, *J* = 13.3, 5.1 Hz, 1H), 4.63 - 4.53 (m, 4H), 3.57 - 3.51 (m, 4H), 3.36 - 3.32(m, 4H), 3.04 - 2.91 (m, 1H), 2.82 (d, *J* = 15.5 Hz, 1H), 2.57 (dd, *J* = 13.1, 4.5 Hz, 1H), 2.29 - 2.14 (m, 1H). LCMS (ESI) calcd for $C_{24}H_{25}N_6O_3S^+$ $[M+H]^+$: 477.17, found, 477.2.

Example 213: Preparation of 3-(5-((4-(2-chlorothieno[3,2-d]pyrimidin-4-yl)piperazin-1-yl)methyl)-1-oxoisoindolin-2-yl) piperidine-2,6-dione (GT-04682)

Referring to the method of Scheme 1, the target compound (GT-04682) was prepared under appropriate conditions as would be understood by those skilled in the art (white solid, 60 mg, yield 64%). $^1$H NMR (400 MHz, MeOD) δ 8.29 (d, *J* = 5.5 Hz, 1H), 7.99 (d, *J* = 7.8 Hz, 1H), 7.85 (s, 1H), 7.79 (d, *J* = 7.8 Hz, 1H), 7.48 (d, *J* = 5.5 Hz, 1H), 5.24 (dd, *J* = 13.4, 5.1 Hz, 1H), 5.01 - 4.83 (m, 2H), 4.71 - 4.59 (m, 5H), 3.79 - 3.60 (m, 3H), 3.48 - 3.41 (m, 2H), 3.06 - 2.93 (m, 1H), 2.88 - 2.77 (m, 1H), 2.57 (dd, *J* = 13.3, 4.3 Hz, 1H), 2.30 - 2.14 (m, 1H). LCMS (ESI) calcd for $C_{24}H_{24}ClN_6O_3S^+$ $[M+H]^+$: 511.13, found, 511.1.

Example 214: Preparation of 3-(5-((4-(2-morpholinothieno[3,2-d]pyrimidin-4-yl)piperazin-1-yl)methyl)-1-oxoisoindolin-2-yl)piperidine-2,6-dione (GT-04903)

Referring to the method of Scheme 1, the target compound (GT-04903) was prepared under appropriate conditions as would be understood by those skilled in the art (white solid, 30 mg, yield 35%). $^1$H NMR (400 MHz, MeOD) δ 8.30 (d, *J* = 5.6 Hz, 1H), 7.93 (d, *J* = 8.0 Hz, 2H), 7.81 - 7.74 (m, 1H), 7.41 (d, *J* = 5.6 Hz, 1H), 5.18 (dd, *J* = 13.3, 5.2 Hz, 1H), 5.13 - 4.89 (m, 2H), 4.68 - 4.50 (m, 4H), 3.84 (brs, 10H), 3.75 - 3.40 (m, 4H), 3.01 - 2.85 (m, 1H), 2.82 - 2.76 (m, 1H), 2.52 (qd, *J* = 13.2, 4.7 Hz, 1H), 2.27 - 2.12 (m, 1H). LCMS (ESI) calcd for $C_{28}H_{32}N_7O_4S^+$ $[M+H]^+$: 562.22, found, 562.3.

Example 215: Preparation of 3-(1-oxo-5-((4-(4-(piperazin-1-yl)thieno[3,2-d]pyrimidin-2-yl)piperazin-1-yl)methyl)isoindolin-2-yl)piperidine-2,6-dione (GT-04698)

Referring to the method of Scheme 1, the target compound (GT-04698) was prepared under appropriate conditions as would be understood by those skilled in the art (white solid, 43 mg, yield 62%). $^1H$ NMR (400 MHz, MeOD) δ 8.35 (d, J = 5.6 Hz, 1H), 7.97 (d, J = 7.8 Hz, 1H), 7.92 (s, 1H), 7.79 (d, J = 7.3 Hz, 1H), 7.46 (d, J = 5.6 Hz, 1H), 5.21 (dd, J = 13.4, 5.2 Hz, 1H), 4.63 (dd, J = 14.9, 11.0 Hz, 4H), 4.45 - 4.38 (m, 4H), 4.34 (d, J = 7.0 Hz, 1H), 3.69 (d, J = 5.2 Hz, 3H), 3.61 (d, J = 5.9 Hz, 4H), 3.53 (d, J = 5.0 Hz, 4H), 3.03 - 2.89 (m, 1H), 2.82 (d, J = 15.7 Hz, 1H), 2.63 - 2.47 (m, 1H), 2.22 (d, J = 10.2 Hz, 1H). LCMS (ESI) calcd for $C_{28}H_{33}N_8O_3S^+$ $[M+H]^+$: 561.24, found, 561.3.

Example 216: Preparation of 3-(5-((4-(4-morpholinothieno[3,2-d]pyrimidin-2-yl)piperazin-1-yl)methyl)-1-oxoisoindolin-2-yl)piperidine-2,6-dione (GT-04906)

Referring to the method of Scheme 1, the target compound (GT-04906) was prepared under appropriate conditions as would be understood by those skilled in the art (white solid, 27 mg, yield 32%). $^1H$ NMR (400 MHz, MeOD) δ 8.28 (d, *J* = 5.6 Hz, 1H), 7.94 (d, *J* = 7.9 Hz, 1H), 7.86 (s, 1H), 7.74 (d, *J* = 7.5 Hz, 1H), 7.39 (d, *J* = 5.6 Hz, 1H), 5.19 (dd, *J* = 13.3, 5.1 Hz, 1H), 4.66 - 4.52 (m, 5H), 4.20 - 4.09 (m, 4H), 3.90 - 3.82 (m, 5H), 3.60 - 3.36 (m, 7H), 3.00 - 2.87 (m, 1H), 2.84 - 2.78 (m, 1H), 2.60 - 2.45 (m, 1H), 2.23 - 2.16 (m, 1H). LCMS (ESI) calcd for $C_{28}H_{32}N_7O_4S^+$ $[M+H]^+$: 562.22, found, 562.3.

Example 217: Preparation of 3-(5-((4-(benzo[b]thiophen-4-yl)piperazin-1-yl)methyl)-1-oxoisoindolin-2-yl)piperidine-2,6-dione (GT-04202)

Referring to the method of Scheme 1, the target compound (GT-04202) was prepared under appropriate conditions as would be understood by those skilled in the art (white solid, 30 mg, yield 41%). $^1H$ NMR (400 MHz, MeOD-d4) δ 7.95 (d, *J* = 7.8 Hz, 1H), 7.84 (s, 1H), 7.77 (d, *J* = 7.9 Hz, 1H), 7.66 (d, *J* = 8.1 Hz, 1H), 7.62 (d, *J* = 5.5 Hz, 1H), 7.49 (dd, *J* = 5.6, 0.7 Hz, 1H), 7.32 (t, *J* = 7.9 Hz, 1H), 7.01 (d, *J* = 7.6 Hz, 1H), 5.19 (dd, *J* = 13.4, 5.2 Hz, 1H), 4.60 (q, *J* = 10.0 Hz, 4H), 3.71 - 3.58 (m, 4H), 3.57 - 3.51 (m, 2H), 3.20 - 3.11 (m, 2H), 3.00 - 2.85 (m, 1H), 2.84 - 2.72 (m, 1H), 2.56 - 2.46 (m, 1H), 2.21 - 2.11 (m, 1H). LCMS (ESI) calcd for $C_{26}H_{27}N_4 0_3S^+$ $[M+H]^+$: 475.18, found, 475.2.

Example 218: Preparation of 3-(1-oxo-5-((3-(trifluoromethyl)-5,6-dihydro-[1,2,4]triazolo[4,3-a]pyrazin-7(8H)-yl)methyl) isoindolin-2-yl)piperidine-2,6-dione (GT-04199)

Referring to the method of Scheme 1, the target compound (GT-04199) was prepared under appropriate conditions as would be understood by those skilled in the art (white solid, 16 mg, yield 23%). $^1H$ NMR (400 MHz, MeOD-d4) δ 7.88 (d, *J* = 7.8 Hz, 1H), 7.76 (s, 1H), 7.68 (d, *J* = 7.9 Hz, 1H), 5.17 (dd, *J* = 13.4, 5.2 Hz, 1H), 4.55 (q, *J* = 17.3 Hz, 2H), 4.45 - 4.32 (m, 6H), 3.48 (t, *J* = 5.5 Hz, 2H), 2.99 - 2.86 (m, 1H), 2.85 - 2.74 (m, 1H), 2.56 - 2.45 (m, 1H), 2.26 - 2.13 (m, 1H). LCMS (ESI) calcd for $C_{20}H_{20}F_3N_6O_3S^+$ $[M+H]^+$: 449.15, found, 449.2.

Example 219: Preparation of 3-(5-((4-(2-((2,4-dimethylphenyl)thio)phenyl)piperazin-1-yl)methyl)-1-oxoisoindolin-2-yl) piperidine-2,6-dione (GT-04203)

Referring to the method of Scheme 1, the target compound (GT-04203) was prepared under appropriate conditions as would be understood by those skilled in the art (white solid, 27 mg, yield 40%). $^1H$ NMR (400 MHz, MeOD-d4) δ 7.95 (d, *J* = 7.8 Hz, 1H), 7.81 (s, 1H), 7.73 (d, *J* = 7.8 Hz, 1H), 7.28 (d, *J* = 7.9 Hz, 1H), 7.21 - 7.10 (m, 3H), 7.06 (d, *J* = 6.6 Hz, 1H), 6.99 - 6.91 (m, 1H), 6.57 - 6.49 (m, 1H), 5.20 (dd, *J* = 13.4, 5.1 Hz, 1H), 4.67 - 4.52 (m, 4H), 3.68 - 3.45 (m, 4H), 3.42 - 3.36 (m, 2H), 3.23 - 3.08 (m, 2H), 2.94 - 2.88 (m, 1H), 2.85 - 2.75 (m, 1H), 2.57 - 2.51 (m, 1H), 2.33 (s, 3H), 2.27 (s, 3H), 2.24 - 2.13 (m, 1H). LCMS (ESI) calcd for $C_{32}H_{35}N_4O_3S^+$ $[M+H]^+$: 555.24, found, 555.3.

Example 220: Preparation of 3-(5-((6,7-dihydrothieno[3,2-c]pyridin-5(4H)-yl)methyl)-1-oxoisoindolin-2-yl)piperidine-2,6-dione (GT-04200)

Referring to the method of Scheme 1, the target compound (GT-04200) was prepared under appropriate conditions as would be understood by those skilled in the art (white solid, 17 mg, yield 28%). $^1H$ NMR (400 MHz, MeOD-d4) δ 7.94 (d, *J* = 7.8 Hz, 1H), 7.80 (s, 1H), 7.73 (d, *J* = 7.8 Hz, 1H), 7.40 (d, *J* = 5.2 Hz, 1H), 6.86 (d, *J* = 5.2 Hz, 1H), 5.19 (dd, *J* = 13.4, 5.2 Hz, 1H), 4.65 (s, 2H), 4.5 (q, *J* = 12.2 Hz, 2H), 4.36 (s, 2H), 3.97 - 3.77 (m, 1H), 3.57 - 3.46(m, 1H), 3.25 (t, *J* = 6.1 Hz, 2H), 3.01 - 2.86 (m, 1H), 2.82 - 2.77 (m, 1H), 2.56 - 2.47 (m, 1H), 2.23 - 2.16 (m, 1H). LCMS (ESI) calcd for $C_{21}H_{22}N_3O_3S^+$ $[M+H]^+$: 396.14, found, 396.2.

Example 221: Preparation of 3-(5-((4-(benzo[d]isothiazol-3-yl)piperazin-1-yl)methyl)-1-oxoisoindolin-2-yl)piperidine-2,6-dione (GT-04255)

Referring to the method of Scheme 1, the target compound (GT-04255) was prepared under appropriate conditions as would be understood by those skilled in the art (white solid, 33 mg, yield 47%). $^1$H NMR (400 MHz, MeOD-d4) δ 8.04 (d, *J* = 8.2 Hz, 1H), 7.95 (d, *J* = 8.0 Hz, 2H), 7.82 (s, 1H), 7.75 (d, *J* = 7.6 Hz, 1H), 7.57 (t, *J* = 7.3 Hz, 1H), 7.47 (t, *J* = 7.4 Hz, 1H), 5.19 (dd, *J* = 13.3, 5.2 Hz, 1H), 4.68 - 4.50 (m, 4H), 4.33 - 4.09 (m, 2H), 3.69 - 3.38 (m, 6H), 3.03 - 2.86 (m, 1H), 2.86 - 2.72 (m, 1H), 2.58 - 2.47 (m, 1H), 2.24 - 2.18 (m, 1H). LCMS (ESI) calcd for $C_{25}H_{26}N_5O_3S^+$ [M+H]$^+$: 476.18, found, 476.2.

Example 222: Preparation of 3-(5-((4-(4-((3r,5r,7r)-adamantan-1-yl)benzyl)piperazin-1-yl)methyl)-1-oxoisoindolin-2-yl)piperidine-2,6-dione (GT-03026)

Referring to the method of Scheme 1, the target compound (GT-03026) was prepared under appropriate conditions as would be understood by those skilled in the art (white solid, 20 mg, yield 37%). LCMS (ESI) calcd for $C_{35}H_{43}N_4O_3S^+$ [M+H]$^+$: 567.33, found, 567.3.

Example 223: Preparation of 2-(2,6-dioxopiperidin-3-yl)-5-((4-(3-methylthiophen-2-yl)-3,6-dihydropyridin-1(2H)-yl)methyl)isoindoline-1,3-dione (GT-04732)

Referring to the method of Scheme 1, the target compound (GT-04732) was prepared under appropriate conditions as would be understood by those skilled in the art (white solid, 28 mg, yield 41%). $^1$H NMR (400 MHz, MeOD) δ 8.15 (s, 1H), 8.06 (s, 2H), 7.28 (d, *J*= 5.1 Hz, 1H), 6.90 (d, *J*= 5.1 Hz, 1H), 5.90 (s, 1H), 5.21 (dd, *J*= 12.5, 5.4 Hz, 1H), 4.86 (s, 2H), 4.67 (s, 2H), 3.96 (s, 2H), 2.99 - 2.74 (m, 5H), 2.32 (s, 3H), 2.24 - 2.12 (m, 1H). LCMS (ESI) calcd for $C_{24}H_{24}N_3O_4S^+$ [M+H]$^+$: 450.15, found, 450.1.

Example 224: Preparation of 3-(4-((4-(3-methylthiophen-2-yl)-3,6-dihydropyridin-1(2H)-yl)methyl)-1-oxoisoindolin-2-yl)piperidine-2,6-dione (GT-04728)

Referring to the method of Scheme 1, the target compound (GT-04728) was prepared under appropriate conditions as would be understood by those skilled in the art (white solid, 24 mg, yield 37%). $^1$H NMR (400 MHz, MeOD) δ 8.00 (d, *J* = 7.6 Hz, 1H), 7.89 (d, *J* = 7.6 Hz, 1H), 7.76 - 7.71 (m, 1H), 7.28 (d, *J* = 5.1 Hz, 1H), 6.90 (d, *J* = 5.1 Hz, 1H), 5.91 (s, 1H), 5.25 (dd, *J* = 17.2, 8.2 Hz, 2H), 4.88 (d, *J* = 3.4 Hz, 1H), 4.84 - 4.81 (m, 1H), 4.59 (s, 2H), 4.01 (s, 2H), 8.89 - 1.79 (m, 197H), 2.85 (s, 2H), 2.61 (d, *J* = 8.1 Hz, 2H), 2.53 (dd, *J* = 13.0, 4.5 Hz, 1H), 2.32 (s, 3H), 2.30 - 2.19 (m, 2H). LCMS (ESI) calcd for $C_{24}H_{26}N_3O_3S^+$ [M+H]$^+$: 436.17, found, 436.2.

Example 225: Preparation of 3-(4-fluoro-5-((4-(3-methylthiophen-2-yl)-3,6-dihydropyridin-1(2H)-yl)methyl)-1-oxoisoindolin-2-yl)piperidine-2,6-dione (GT-04729)

Referring to the method of Scheme 1, the target compound (GT-04729) was prepared under appropriate conditions as would be understood by those skilled in the art (white solid, 21 mg, yield 31%). $^1$H NMR (400 MHz, MeOD) δ 7.86 - 7.79 (m, 2H), 7.28 (d, *J* = 5.1 Hz, 1H), 6.90 (d, *J* = 5.1 Hz, 1H), 5.91 (s, 1H), 5.21 (dd, *J* = 13.4, 5.1 Hz, 1H), 4.78 - 4.54 (m, 5H), 4.03 (s, 2H), 2.92 (dd, *J* = 13.2, 5.4 Hz, 2H), 2.84 (s, 2H), 2.60 - 2.51 (m, 1H), 2.32 (s, 3H), 2.23 (d, *J* = 5.2 Hz, 1H). LCMS (ESI) calcd for $C_{24}H_{25}FN_3O_3S^+$ [M+H]$^+$: 454.16, found, 454.2.

Example 226: Preparation of 3-(7-fluoro-5-((4-(3-methylthiophen-2-yl)-3,6-dihydropyridin-1(2H)-yl)methyl)-1-oxoisoindolin-2-yl)piperidine-2,6-dione (GT-04730)

Referring to the method of Scheme 1, the target compound (GT-04730) was prepared under appropriate conditions as would be understood by those skilled in the art (GT-04730) (white solid, 35 mg, yield 51%). $^1$H NMR (400 MHz, MeOD) δ 7.63 (s, 1H), 7.48 (d, *J* = 9.8 Hz, 1H), 7.28 (d, *J* = 5.1 Hz, 1H), 6.90 (d, *J* = 5.1 Hz, 1H), 5.90 (s, 1H), 5.18 (dd, *J* = 13.3, 5.2 Hz, 1H), 4.87 (s, 1H), 4.59 (t, *J* = 12.5 Hz, 4H), 3.96 (s, 2H), 3.78 (s, 1H), 3.00 - 2.89 (m, 2H), 2.82 (dd, *J* = 9.9, 7.7 Hz, 2H), 2.52 (dd, *J* = 13.2, 4.8 Hz, 1H), 2.32 (s, 3H), 2.22 (dd, *J* = 9.1, 3.7 Hz, 1H). LCMS (ESI) calcd for $C_{24}H_{25}FN_3O_3S^+$ [M+H]$^+$: 454.16, found, 454.2.

Example 227: Preparation of 2-(2,6-dioxopiperidin-3-yl)-4-((4-(3-methylthiophen-2-yl)-3,6-dihydropyridin-1(2H)-yl)methyl)isoindoline-1,3-dione (GT-04731)

Referring to the method of Scheme 1, the target compound (GT-04731) was prepared under appropriate

conditions as would be understood by those skilled in the art (white solid, 22 mg, yield 33%). $^1$H NMR (400 MHz, MeOD) δ 8.10 (dd, *J* = 6.1, 2.3 Hz, 1H), 8.04 - 7.98 (m, 2H), 7.28 (d, *J* = 5.1 Hz, 1H), 6.90 (d, *J* = 5.1 Hz, 1H), 5.92 (s, 1H), 5.24 (dd, *J* = 12.6, 5.5 Hz, 1H), 4.92 (d, *J* = 3.0 Hz, 4H), 4.09 (s, 2H), 2.93 - 2.72 (m, 5H), 2.31 (d, *J* = 11.7 Hz, 3H), 2.25 - 2.15 (m, 1H). LCMS (ESI) calcd for $C_{24}H_{24}N_3O_4S^+$ [M+H]$^+$: 450.15, found, 450.2.

Example 228: Preparation of 3-(4-((4-(2,3-dichlorophenyl)piperazin-1-yl)methyl)-1-oxoisoindolin-2-yl)piperidine-2,6-dione (GT-04528)

Referring to the method of Scheme 1, the target compound (GT-04528) was prepared under appropriate conditions as would be understood by those skilled in the art (white solid, 62 mg, yield 79%). $^1$H NMR (400 MHz, DMSO) δ 11.07 (s, 1H), 10.93 (s, 1H), 8.00 (d, *J* = 6.4 Hz, 1H), 7.85 (d, *J* = 7.0 Hz, 1H), 7.67 (d, *J* = 7.2 Hz, 1H), 7.43 - 7.29 (m, 2H), 7.20 (d, *J* = 6.5 Hz, 1H), 5.19 (dd, *J* = 13.2, 5.0 Hz, 1H), 4.84 (d, *J* = 17.6 Hz, 1H), 4.63 - 4.41 (m, 3H), 3.59 - 3.39 (m, 5H), 3.16 - 3.26 (m, 3H), 3.04 - 2.88 (m, 1H), 2.66 (d, *J* = 16.2 Hz, 1H), 2.44 - 2.23 (m, 1H), 2.13 - 1.94 (m, 1H). LCMS (ESI) calcd for $C_{24}H_{25}Cl_2N_4O_3^+$ [M+H]$^+$: 487.13, found, 487.1.

Example 229: Preparation of 3-(5-((4-(2,3-dichlorophenyl)piperazin-1-yl)methyl)-4-fluoro-1-oxoisoindolin-2-yl)piperidine-2,6-dione (GT-04526)

Referring to the method of Scheme 1, the target compound (GT-04526) was prepared under appropriate conditions as would be understood by those skilled in the art (white solid, 30 mg, yield 30%). $^1$H NMR (400 MHz, DMSO) δ 11.03 (s, 1H), 7.88 (s, 1H), 7.73 (d, *J* = 7.8 Hz, 1H), 7.60 (dd, *J* = 7.1, 3.5 Hz, 1H), 7.36 (q, *J* = 7.8 Hz, 2H), 7.20 (dd, *J* = 7.3, 2.2 Hz, 1H), 5.17 - 5.13 (m, 1H), 4.69 - 4.37 (m, 5H), 3.65 - 3.52 (m, 2H), 3.21 - 3.06 (m, 3H), 2.99 - 2.84 (m, 2H), 2.70 - 2.57 (m, 2H), 2.57 - 2.42 (m, 1H), 2.06 - 1.95 (m, 1H). LCMS (ESI) calcd for $C_{24}H_{24}Cl_2FN_4O_3^+$ [M+H]$^+$: 505.12, found, 505.1.

Example 230: Preparation of 3-(5-((4-(2,3-dichlorophenyl)piperazin-1-yl)methyl)-6-fluoro-1-oxoisoindolin-2-yl)piperidine-2,6-dione (GT-04527)

Referring to the method of Scheme 1, the target compound (GT-04527) was prepared under appropriate conditions as would be understood by those skilled in the art (white solid, 48 mg, yield 47%). $^1$H NMR (400 MHz, DMSO) δ 11.02 (s, 1H), 8.02 (s, 1H), 7.70 (d, *J* = 8.0 Hz, 1H), 7.41 - 7.31 (m, 2H), 7.19 (dd, *J* = 7.2, 2.2 Hz, 1H), 5.15 (dd, *J* = 13.2, 5.1 Hz, 1H), 4.56 (brs, 2H), 4.45 (dd, *J* = 50.2, 17.4 Hz, 3H), 3.61 - 3.39 (m, 4H), 3.21 - 3.12 (m, 3H), 3.01 - 2.87 (m, 1H), 2.64 - 2.56 (m, 1H), 2.51 - 2.41 (m, 1H), 2.11 - 1.90 (m, 1H). LCMS (ESI) calcd for $C_{24}H_{24}Cl_2FN_4O_3^+$ [M+H]$^+$: 505.12, found, 505.1.

Example 231: Preparation of 3-(5-((4-(2,3-dichlorophenyl)piperazin-1-yl)methyl)-7-fluoro-1-oxoisoindolin-2-yl)piperidine-2,6-dione (GT-04445)

Referring to the method of Scheme 1, the target compound (GT-04445) was prepared under appropriate conditions as would be understood by those skilled in the art (white solid, 3.3 g, yield 81%). $^1$H NMR (400 MHz, DMSO) δ 11.43 (s, 1H), 11.03 (s, 1H), 7.76 - 7.66 (m, 2H), 7.42 - 7.32 (m, 2H), 7.20 (dd, *J* = 7.3, 2.3 Hz, 1H), 5.12 (dd, *J* = 13.3, 5.1 Hz, 1H), 4.61 - 4.36 (m, 4H), 3.51 - 3.45(m, 4H), 3.33 - 3.15 (m, 4H), 3.01 - 2.84 (m, 1H), 2.61 (d, *J* = 17.9 Hz, 1H), 2.47 - 2.40 (m, 1H), 2.05 - 2.01 (m, 1H). LCMS (ESI) calcd for $C_{24}H_{24}Cl_2FN_4O_3^+$ [M+H]$^+$: 505.12, found, 505.1.

Example 232: Preparation of 3-(5-((4-(5,6-dichloropyridazin-4-yl)piperazin-1-yl)methyl)-1-oxoisoindolin-2-yl)piperidine-2,6-dione (GT-04544)

Referring to the method of Scheme 1, the target compound (GT-04544) was prepared under appropriate conditions as would be understood by those skilled in the art (white solid, 60 mg, yield 62%). $^1$H NMR (400 MHz, DMSO) δ 11.02 (s, 1H), 9.06 (s, 1H), 7.85 (d, *J* = 7.4 Hz, 2H), 7.74 (d, *J* = 8.3 Hz, 1H), 5.15 (dd, *J* = 13.3, 5.1 Hz, 1H), 4.62 - 4.37 (m, 4H), 4.10 - 3.84 (m, 2H), 3.54 - 3.36 (m, 4H), 3.25 - 3.21 (m, 2H), 2.92 - 2.90 (m, 2H), 2.62 (d, *J* = 17.5 Hz, 1H), 2.43 (dd, *J* = 13.2, 4.6 Hz, 1H), 2.10 - 1.92 (m, 1H). LCMS (ESI) calcd for $C_{22}H_{23}Cl_2N_6O_3^+$ [M+H]$^+$: 489.12, found, 489.1.

Example 233: Preparation of 3-(4-((4-(6-fluorobenzo[d]isoxazol-3-yl)piperidin-1-yl)methyl)-1-oxoisoindolin-2-yl)piperidine-2,6-dione (GT-04764)

Referring to the method of Scheme 1, the target compound (GT-04764) was prepared under appropriate conditions as would be understood by those skilled in the art (white solid, 71 mg, yield 61%). $^1$H NMR (400 MHz, DMSO) δ 11.08 (s, 1H), 10.99 (d, J = 45.5 Hz, 1H), 8.30 - 8.16 (m, 1H), 7.99 (d, J = 6.8 Hz, 1H), 7.86 (d, J = 7.5 Hz, 1H), 7.77 - 7.68 (m,

1H), 7.66 (d, J = 7.4 Hz, 1H), 7.35 (d, J = 9.1 Hz, 1H), 5.21 (dd, J = 13.2, 5.1 Hz, 1H), 4.85 (s, 1H), 4.55 (d, J = 17.5 Hz, 1H), 4.45 (s, 2H), 3.56 (dd, J = 25.5, 11.2 Hz, 2H), 3.49 - 3.39 (m, 2H), 3.26 (d, J = 8.8 Hz, 2H), 2.96 (s, 1H), 2.66 (d, J = 15.2 Hz, 1H), 2.42 - 2.34 (m, 2H), 2.21 (d, J = 12.3 Hz, 2H), 2.07 (d, J = 5.7 Hz, 1H). LCMS (ESI) calcd for $C_{26}H_{26}FN_4O_4^+$ [M+H]$^+$: 477.19, found, 477.3.

Example 234: Preparation of 3-(6-((4-(6-fluorobenzo[d]isoxazol-3-yl)piperidin-1-yl)methyl)-1-oxoisoindolin-2-yl)piperidine-2,6-dione (GT-04768)

Referring to the method of Scheme 1, the target compound (GT-04768) was prepared under appropriate conditions as would be understood by those skilled in the art (white solid, 34 mg, yield 29%). $^1$H NMR (400 MHz, DMSO) δ 11.02 (s, 1H), 10.74 (s, 1H), 8.21 - 8.09 (m, 1H), 8.02 (d, J = 12.1 Hz, 1H), 7.90 (d, J = 7.6 Hz, 1H), 7.79 - 7.64 (m, 2H), 7.34 (td, J = 9.1, 2.1 Hz, 1H), 5.14 (dd, J = 13.3, 5.2 Hz, 1H), 4.53 (d, J = 17.6 Hz, 3H), 3.54 (d, J = 10.7 Hz, 2H), 3.45 (s, 1H), 3.13 (s, 2H), 2.99 - 2.86 (m, 1H), 2.72 - 2.57 (m, 2H), 2.47 - 2.37 (m, 1H), 2.25 (s, 4H), 2.06 - 1.96 (m, 1H). LCMS (ESI) calcd for $C_{26}H_{26}FN_4O_4^+$ [M+H]$^+$: 477.19, found, 477.3.

Example 235: Preparation of 3-(4-fluoro-5-((4-(6-fluorobenzo[d]isoxazol-3-yl)piperidin-1-yl)methyl)-1-oxoisoindolin-2-yl)piperidine-2,6-dione (GT-04765)

Referring to the method of Scheme 1, the target compound (GT-04765) was prepared under appropriate conditions as would be understood by those skilled in the art (white solid, 77 mg, yield 64%). $^1$H NMR (400 MHz, DMSO) δ 11.04 (s, 1H), 10.84 (s, 1H), 8.14 (d, J = 3.7 Hz, 1H), 7.93 (s, 1H), 7.79 - 7.68 (m, 2H), 7.34 (td, J = 9.1, 2.0 Hz, 1H), 5.16 (dd, J = 13.3, 5.1 Hz, 1H), 4.53 (dt, J = 50.5, 13.7 Hz, 4H), 3.62 (s, 2H), 3.46 (s, 1H), 3.25 (s, 2H), 3.02 - 2.82 (m, 1H), 2.64 (dd, J = 20.5, 9.6 Hz, 1H), 2.48 - 2.40 (m, 1H), 2.33 (s, 2H), 2.25 (s, 2H), 2.07 - 1.96 (m, 1H). LCMS (ESI) calcd for $C_{26}H_{25}F_2N_4O_4^+$ [M+H]$^+$: 495.18, found, 495.2.

Example 236: Preparation of 3-(6-fluoro-5-((4-(6-fluorobenzo[d]isoxazol-3-yl)piperidin-1-yl)methyl)-1-oxoisoindolin-2-yl)piperidine-2,6-dione (GT-04766)

Referring to the method of Scheme 1, the target compound (GT-04766) was prepared under appropriate conditions as would be understood by those skilled in the art (white solid, 66 mg, yield 55%). $^1$H NMR (400 MHz, DMSO) δ 11.03 (s, 1H), 11.03 - 10.80 (m, 1H), 8.23 - 8.09 (m, 1H), 8.03 (d, J = 16.1 Hz, 1H), 7.71 (dd, J = 11.9, 10.4 Hz, 2H), 7.34 (t, J = 9.1 Hz, 1H), 5.16 (dd, J = 13.2, 5.0 Hz, 1H), 4.64 - 4.47 (m, 3H), 4.39 (d, J = 17.6 Hz, 1H), 3.59 (d, J = 11.6 Hz, 2H), 3.45 (d, J = 12.7 Hz, 2H), 3.24 (d, J = 10.2 Hz, 2H), 3.03 - 2.85 (m, 1H), 2.72 - 2.56 (m, 2H), 2.33 (s, 2H), 2.22 (d, J = 13.7 Hz, 2H), 2.09 - 1.96 (m, 1H). LCMS (ESI) calcd for $C_{26}H_{25}F_2N_4O_4^+$ [M+H]$^+$: 495.18, found, 495.3.

Example 237: Preparation of 3-(7-fluoro-5-((4-(6-fluorobenzo[d]isoxazol-3-yl)piperidin-1-yl)methyl)-1-oxoisoindolin-2-yl)piperidine-2,6-dione (GT-04767)

Referring to the method of Scheme 1, the target compound (GT-04767) was prepared under appropriate conditions as would be understood by those skilled in the art (white solid, 82 mg, yield 68%). $^1$H NMR (400 MHz, DMSO) δ 11.03 (s, 2H), 8.17 (dd, J = 8.8, 5.3 Hz, 1H), 7.75 (t, J = 6.7 Hz, 1H), 7.72 (s, 1H), 7.67 (d, J = 10.0 Hz, 1H), 7.34 (td, J = 9.1, 2.2 Hz, 1H), 5.12 (dd, J = 13.3, 5.1 Hz, 1H), 4.55 - 4.40 (m, 4H), 3.55 (d, J = 11.6 Hz, 2H), 3.46 (s, 1H), 3.14 (d, J = 11.9 Hz, 2H), 2.96 - 2.86 (m, 1H), 2.61 (d, J = 16.8 Hz, 2H), 2.37 - 2.29 (m, 2H), 2.22 (d, J = 12.3 Hz, 2H), 2.05 - 1.96 (m, 1H). LCMS (ESI) calcd for $C_{26}H_{25}F_2N_4O_4^+$ [M+H]$^+$: 495.18, found, 495.2.

Example 238: Preparation of 2-(2,6-dioxopiperidin-3-yl)-5-((4-(6-fluorobenzo[d]isoxazol-3-yl)piperidin-1-yl)methyl)isoindoline-1,3-dione (GT-04771)

Referring to the method of Scheme 1, the target compound (GT-04771) was prepared under appropriate conditions as would be understood by those skilled in the art (white solid, 50 mg, yield 42%). $^1$H NMR (400 MHz, DMSO) δ 11.16 (s, 1H), 11.01 (s, 1H), 8.27 (s, 1H), 8.15 (dd, J = 8.6, 5.3 Hz, 2H), 8.05 (dd, J = 15.9, 7.6 Hz, 1H), 7.72 (dd, J = 9.1, 2.0 Hz, 1H), 7.34 (td, J = 9.2, 2.1 Hz, 1H), 5.20 (dd, J = 12.9, 5.4 Hz, 1H), 4.60 (s, 2H), 3.55 (d, J = 11.3 Hz, 2H), 3.47 - 3.42 (m, 1H), 3.15 (d, J = 11.5 Hz, 2H), 2.90 (dd, J = 15.7, 9.9 Hz, 1H), 2.62 (dd, J = 29.8, 12.4 Hz, 3H), 2.32 (d, J = 6.9 Hz, 1H), 2.27 (d, J = 11.9 Hz, 2H), 2.13 - 2.03 (m, 1H). LCMS(ESI) calcd for $C_{26}H_{24}FN_4O_5^+$ [M+H]$^+$: 491.17, found, 491.2.

Example 239: Preparation of 2-(2,6-dioxopiperidin-3-yl)-4-((4-(6-fluorobenzo[d]isoxazol-3-yl)piperidin-1-yl)methyl)isoindoline-1,3-dione (GT-04770)

Referring to the method of Scheme 1, the target compound (GT-04770) was prepared under appropriate conditions as would be understood by those skilled in the art (white solid, 29 mg, yield 24%). $^1$H NMR (400 MHz, DMSO) δ 11.17 (s, 1H), 10.74 (s, 1H), 8.22 (d, J = 7.5 Hz, 1H), 8.15 (dd, J = 8.4, 5.4 Hz, 1H), 8.07 - 7.95 (m, 2H), 7.72 (d, J = 7.3 Hz, 1H), 7.34 (t, J = 8.1 Hz, 1H), 5.20 (dd, J = 12.8, 5.3 Hz, 1H), 4.80 (s, 2H), 3.63 (s, 2H), 3.54 (s, 1H), 2.97 - 2.83 (m, 1H), 2.60 (dd, J = 26.0, 12.6 Hz, 2H), 2.51 (s, 2H), 2.31 (d, J = 16.1 Hz, 2H), 2.24 (s, 2H), 2.14 - 2.02 (m, 1H). LCMS (ESI) calcd for $C_{26}H_{24}FN_4O_5^+$ [M+H]$^+$: 491.17, found, 491.2.

Example 240: Preparation of 3-(4-((4-(benzo[b]thiophen-4-yl)piperazin-1-yl)methyl)-1-oxoisoindolin-2-yl)piperidine-2,6-dione (GT-04914)

Referring to the method of Scheme 1, the target compound (GT-04914) was prepared under appropriate conditions as would be understood by those skilled in the art (white solid, 51 mg, yield 73%). $^1$H NMR (400 MHz, DMSO) δ 11.08 (s, 2H), 8.04 (d, J = 7.5 Hz, 1H), 7.86 (d, J = 7.3 Hz, 1H), 7.77 (d, J = 5.5 Hz, 1H), 7.68 (dd, J = 15.0, 7.8 Hz, 2H), 7.50 (d, J = 5.5 Hz, 1H), 7.32 (t, J = 7.9 Hz, 1H), 6.96 (d, J = 7.5 Hz, 1H), 5.20 (dd, J = 13.2, 5.1 Hz, 1H), 4.89 (d, J = 17.7 Hz, 1H), 4.58 (d, J = 17.6 Hz, 1H), 4.51 (s, 2H), 3.57 (s, 2H), 3.50 (s, 4H), 3.28 (s, 2H), 3.03 - 2.91 (m, 1H), 2.66 (d, J = 17.2 Hz, 1H), 2.42 - 2.31 (m, 1H), 2.10 - 2.02 (m, 1H). LCMS (ESI) calcd for $C_{26}H_{27}N_4O_3S^+$ [M+H]$^+$: 475.18, found, 475.2.

Example 241: Preparation of 5-((4-(benzo[b]thiophen-4-yl)piperazin-1-yl)methyl)-2-(2,6-dioxopiperidin-3-yl)isoindoline-1,3-dione (GT-04915)

Referring to the method of Scheme 1, the target compound (GT-04915) was prepared under appropriate conditions as would be understood by those skilled in the art (white solid, 46 mg, yield 38%). $^1$H NMR (400 MHz, DMSO) δ 11.32 (s, 1H), 11.16 (s, 1H), 8.29 (s, 1H), 8.17 (d, J = 7.7 Hz, 1H), 8.07 (d, J = 7.6 Hz, 1H), 7.76 (d, J = 5.5 Hz, 1H), 7.70 (d, J = 8.1 Hz, 1H), 7.49 (d, J = 5.5 Hz, 1H), 7.31 (t, J = 7.9 Hz, 1H), 6.96 (d, J = 7.6 Hz, 1H), 5.20 (dd, J = 12.9, 5.3 Hz, 1H), 4.66 (s, 2H), 3.55 (d, J = 12.2 Hz, 2H), 3.47 (s, 2H), 3.22 (t, J = 11.4 Hz, 2H), 2.91 (ddd, J = 13.8, 12.2, 5.2 Hz, 1H), 2.62 (dd, J = 28.3, 10.8 Hz, 2H), 2.54 (s, 2H), 2.14 - 2.03 (m, 1H). LCMS (ESI) calcd for $C_{26}H_{25}N_4O_4S^+$ [M+H]$^+$: 489.16, found, 489.2.

Example 242: Preparation of 3-(1-oxo-4-((3-(trifluoromethyl)-5,6-dihydro-[1,2,4]triazolo[4,3-a]pyrazin-7(8H)-yl)methyl) isoindolin-2-yl)piperidine-2,6-dione (GT-04920)

Referring to the method of Scheme 1, the target compound (GT-04920) was prepared under appropriate conditions as would be understood by those skilled in the art (white solid, 14 mg, yield 19%). $^1$H NMR (400 MHz, DMSO) δ 13.05 (s, 1H), 11.13 (d, J = 7.7 Hz, 1H), 8.50 (d, J = 7.8 Hz, 1H), 8.45 (s, 1H), 8.03 (s, 1H), 7.97 - 7.91 (m, 2H), 7.84 - 7.77 (m, 1H), 7.75 (d, J = 8.3 Hz, 1H), 7.70 (d, J = 7.6 Hz, 2H), 7.50 (s, 1H), 5.21 - 5.12 (m, 1H), 4.75 (s, 1H), 4.42 (s, 2H), 3.69 (d, J = 7.5 Hz, 3H), 3.32 - 3.25 (m, 2H), 3.24 - 3.10 (m, 3H), 2.96 - 2.85 (m, 2H), 2.68 - 2.58 (m, 3H), 2.55 (s, 2H), 2.07 (d, J = 4.6 Hz, 1H), 1.95 (d, J = 12.9 Hz, 2H), 1.86 (s, 1H), 1.77 - 1.53 (m, 4H). LCMS (ESI) calcd for $C_{20}H_{20}F_3N_6O_3^+$ [M+H]$^+$: 449.15, found, 449.2.

Example 243: Preparation of 2-(2,6-dioxopiperidin-3-yl)-5-((3-(trifluoromethyl)-5,6-dihydro-[1,2,4]triazolo[4,3-a]pyrazin-7(8H)-yl)methyl)isoindoline-1,3-dione (GT-04921)

Referring to the method of Scheme 1, the target compound (GT-04921) was prepared under appropriate conditions as would be understood by those skilled in the art (white solid, 33 mg, yield 25%). $^1$H NMR (400 MHz, DMSO) δ 11.13 (s, 1H), 7.98 (s, 1H), 7.96 - 7.90 (m, 2H), 5.16 (dd, J = 12.9, 5.4 Hz, 1H), 4.21 (t, J = 5.4 Hz, 2H), 4.04 (d, J = 25.0 Hz, 4H), 3.07 (t, J = 5.3 Hz, 2H), 2.90 (d, J = 6.6 Hz, 1H), 2.64 - 2.55 (m, 2H), 2.53 (s, 1H), 2.11 - 2.02 (m, 1H), 1.27 (dt, J = 7.4, 4.9 Hz, 1H). LCMS (ESI) calcd for $C_{20}H_{18}F_3N_6O_4^+$ [M+H]$^+$: 463.13, found, 463.1.

Example 244: Preparation of 3-(1-oxo-5-((4-(2-(piperidin-1-yl)-6,7-dihydrothieno[3,2-d]pyrimidin-4-yl)piperazin-1-yl) methyl)isoindolin-2-yl)piperidine-2,6-dione (GT-05380)

Referring to the method of Scheme 1, the target compound (GT-05380) was prepared under appropriate conditions as would be understood by those skilled in the art (white solid, 24 mg, yield 27%). $^1$H NMR (400 MHz, DMSO-d6) δ 11.34 (s, 1H), 11.01 (s, 1H), 7.85 (s, 1H), 7.83 (d, *J* = 7.9 Hz, 1H), 7.74 (d, *J* = 7.6 Hz, 1H), 5.15 (dd, *J*= 13.2, 5.1 Hz, 1H), 4.59 - 4.33 (m, 6H), 3.70 - 3.65 (m, 4H), 3.62 - 3.58 (m, 3H), 3.34 - 3.26 (m, 3H), 3.24 - 3.07 (m, 4H), 2.99 - 2.86 (m, 1H), 2.61 (d, *J*= 17.1 Hz, 1H), 2.47 - 2.37 (m, 1H), 2.09 - 1.96 (m, 1H), 1.67 - 1.61 (m, 2H), 1.56 - 1.48 (m, 4H). LCMS (ESI) calcd for

$C_{29}H_{36}N_7O_3S^+$ [M+H]$^+$: 562.26, found, 562.3.

Example 245: Preparation of 3-(1-oxo-5-((4-(2-(pyrrolidin-1-yl)-6,7-dihydrothieno[3,2-d]pyrimidin-4-yl)piperazin-1-yl) methyl)isoindolin-2-yl)piperidine-2,6-dione (GT-05389)

Referring to the method of Scheme 1, the target compound (GT-05389) was prepared under appropriate conditions as would be understood by those skilled in the art (white solid, 12 mg, yield 13%). $^1$H NMR (400 MHz, DMSO-d6) δ 11.02 (s, 1H), 7.89 (s, 1H), 7.82 (d, *J* = 7.7 Hz, 1H), 7.76 (d, *J*= 7.7 Hz, 1H), 5.15 (dd, *J*= 13.2, 5.0 Hz, 1H), 4.85 - 4.75 (m, 1H), 4.57 - 4.35 (m, 6H), 3.45 - 3.27 (m, 7H), 3.24 - 3.08 (m, 3H), 3.01 - 2.86 (m, 2H), 2.61 (d, *J* = 16.6 Hz, 2H), 2.46 - 2.36 (m, 1H), 2.05 - 1.91 (m, 6H). LCMS (ESI) calcd for $C_{28}H_{34}N_7O_3S^+$ [M+H]$^+$: 548.24, found, 548.3.

Example 246: Preparation of 3-(5-((4-(2-(azetidin-1-yl)-6,7-dihydrothieno[3,2-d]pyrimidin-4-yl)piperazin-1-yl)methyl)-1-oxoisoindolin-2-yl)piperidine-2,6-dione (GT-05900)

Referring to the method of Scheme 1, the target compound (GT-05900) was prepared under appropriate conditions as would be understood by those skilled in the art (white solid, 45 mg, yield 50%). $^1$H NMR (400 MHz, DMSO-d6) δ 12.33 (s, 1H), 11.01 (s, 1H), 7.93 (s, 1H), 7.83 - 7.77 (m, 2H), 5.14 (dd, *J*= 13.3, 5.0 Hz, 1H), 4.63 - 4.39 (m, 6H), 4.05 (t, *J* = 5.6 Hz, 1H), 3.81 - 3.67 (m, 4H), 3.52 - 3.38 (m, 6H), 3.39 - 3.28 (m, 2H), 3.26 - 3.17 (m, 2H), 3.02 - 2.84 (m, 1H), 2.61 (d, *J*= 16.5 Hz, 1H), 2.48 - 2.38 (m, 1H), 2.03 - 1.99 (m, 2H). LCMS (ESI) calcd for $C_{27}H_{32}N_7O_3S^+$ [M+H]$^+$: 534.23, found, 534.3.

Example 247: Preparation of 3-(5-((((3s,5s,7s)-adamantan-1-yl)amino)methyl)-1-oxoisoindolin-2-yl)piperidine-2,6-dione (GT-06913)

Referring to the method of Scheme 1, the target compound (GT-06913) was prepared under appropriate conditions as would be understood by those skilled in the art (white solid, 14 mg, yield 18%). $^1$H NMR (400 MHz, DMSO-d6) δ 11.00 (s, 1H), 9.01 (s, 2H), 7.81 (d, *J* = 7.6 Hz, 2H), 7.71 *(d, J*= 8.3 Hz, 1H), 5.14 (dd, *J*= 13.4, 5.2 Hz, 1H), 4.50 (d, *J* = 17.6 Hz, 1H), 4.36 (d, *J*= 17.6 Hz, 1H), 4.24 (s, 2H), 3.01 - 2.83 (m, 1H), 2.61 (d, *J* = 17.6 Hz, 1H), 2.47 - 2.35 (m, 1H), 2.16 (s, 3H), 2.05 - 2.01 (m, 1H), 1.98 (s, 6H), 1.71 - 1.61 (m, 6H). LCMS (ESI) calcd for $C_{24}H_{30}N_3O_3^+$ [M+H]$^+$: 408.23, found, 408.3.

Example 248: Preparation of (1-((1R,4R)-7,7-dimethyl-2-oxobicyclo[2.2.1]heptan-1-yl)-N-((2-(2,6-dioxopiperidin-3-yl)-1-oxoisoindolin-5-yl)methyl)methanesulfonamide (GT-06927)

Referring to the method of Scheme 1, the target compound (GT-06927) was prepared under appropriate conditions as would be understood by those skilled in the art (white solid, 9 mg, yield 26%). $^1$H NMR (400 MHz, DMSO-d6) δ 10.98 (s, 1H), 7.74 (s, 1H), 7.71 (d, *J*=7.8 Hz, 1H), 7.59 (s, 1H), 7.50 (d, *J*=7.9 Hz, 1H), 5.11 (dd, *J*= 13.3, 5.1 Hz, 1H), 4.53 - 4.27 (m, 4H), 3.02 - 2.82 (m, 2H), 2.60 (d,*J* = 16.1 Hz, 1H), 2.53 - 2.48 (m, 1H), 2.47 - 2.28 (m, 3H), 2.11 - 1.85 (m, 4H), 1.58 - 1.51 (m, 1H), 1.48 - 1.33 (m, 1H), 0.99 (s, 3H), 0.75 (d, *J* = 1.3 Hz, 3H). LCMS (ESI) calcd for $C_{24}H_{30}N_3O_6S^+$ [M+H]$^+$: 488.18, found, 488.2.

Example 249: Preparation of 5-((4-(2,3-dichlorophenyl)-3,6-dihydropyridin-1(2H)-yl)methyl)-2-(2,6-dioxopiperidin-3-yl) isoindoline-1,3-dione (GT-07842)

Referring to the method of Scheme 1, the target compound (GT-07842) was prepared under appropriate conditions as would be understood by those skilled in the art (white solid, 60 mg, yield 74%). $^1$H NMR (400 MHz, DMSO-d6) δ 11.22 (s, 1H), 11.16 (s, 1H), 8.28 (s, 1H), 8.16 (d, J = 7.3 Hz, 1H), 8.06 (d, J = 7.6 Hz, 1H), 7.68 - 7.60 (m, 1H), 7.40 (t, J = 7.9 Hz, 1H), 7.25 (dd, J = 7.6, 1.4 Hz, 1H), 5.74 (s, 1H), 5.20 (dd, J = 12.9, 5.3 Hz, 1H), 4.79 - 4.55 (m, 2H), 3.93 - 3.70 (m, 2H), 3.69 - 3.54 (m, 1H), 3.31 - 3.22 (m, 2H), 2.94 - 2.82 (m, 2H), 2.65 - 2.54 (m, 2H), 2.10 - 2.05 (m, 1H). LCMS (ESI) calcd for $C_{25}H_{22}Cl_2N_3O_4^+$ [M+H]$^+$: 498.10, found, 498.1

Example 250: Preparation of 5-((4-(3-chloropyridin-2-yl)piperazin-1-yl)methyl)-2-(2,6-dioxopiperidin-3-yl)isoindoline-1,3-dione (GT-07843)

Referring to the method of Scheme 1, the target compound (GT-07843) was prepared under appropriate conditions as would be understood by those skilled in the art (white solid, 34 mg, yield 54%). $^1$H NMR (400 MHz, DMSO-d6) δ 11.73 (s, 1H), 11.15 (s, 1H), 8.29 (s, 1H), 8.26 (dd, *J*=4.7, 1.6 Hz, 1H), 8.18 (dd, *J*=7.7, 1.2 Hz, 1H), 8.04 (d, *J*=7.6 Hz, 1H), 7.87 (dd, *J* = 7.8, 1.6 Hz, 1H), 7.10 (dd, *J*= 7.8, 4.7 Hz, 1H), 5.19 (dd, *J*= 12.9, 5.4 Hz, 1H), 4.75 - 4.71 (m, 2H), 3.84 (d, *J* = 12.9 Hz, 2H), 3.44 - 3.33 (m, 4H), 3.31 - 3.13 (m, 2H), 2.95 - 2.86 (m, 1H), 2.62 (d, *J* = 17.7 Hz, 1H), 2.58 - 2.53 (m, 1H), 2.15 - 2.00 (m, 1H). LCMS (ESI) calcd for $C_{23}H_{23}ClN_5O_4^+$ [M+H]$^+$: 468.14, found, 468.1.

Example 251: Preparation of 2-(2,6-dioxopiperidin-3-yl)-5-((4-(pyridazin-3-yl)piperazin-1-yl)methyl)isoindoline-1,3-dione (GT-07844)

Referring to the method of Scheme 1, the target compound (GT-07844) was prepared under appropriate conditions as would be understood by those skilled in the art (white solid, 40 mg, yield 56%). $^1$H NMR (400 MHz, DMSO-d6) δ 12.39 (s, 1H), 11.16 (s, 1H), 8.86 (dd, *J* = 3.9, 1.7 Hz, 1H), 8.30 (s, 1H), 8.18 (dd, *J* = 7.7, 1.2 Hz, 1H), 8.04 (d, *J* = 7.7 Hz, 1H), 7.99 - 7.96 (m, 2H), 5.19 (dd, *J* = 12.8, 5.4 Hz, 1H), 4.61 (s, 2H), 4.51 - 4.45 (m, 3H), 3.53 - 3.08 (m, 5H), 2.91 - 2.88 (m, 1H), 2.62 (d, *J* = 18.0 Hz, 1H), 2.58 - 2.52 (m, 1H), 2.14 - 2.00 (m, 1H). LCMS (ESI) calcd for $C_{22}H_{23}N_6O_4^+$ [M+H]$^+$: 435.18, found, 435.1.

Example 252: Preparation of 5-((4-(4-chloro-3-fluoropyridin-2-yl)piperazin-1-yl)methyl)-2-(2,6-dioxopiperidin-3-yl)isoindoline-1,3-dione (GT-08135)

Referring to the method of Scheme 1, the target compound (GT-08135) was prepared under appropriate conditions as would be understood by those skilled in the art (white solid, 41 mg, yield 66%). $^1$H NMR (400 MHz, DMSO-d6) δ 11.88 (s, 1H), 11.22 (s, 1H), 8.33 (s, 1H), 8.25 - 8.18 (m, 1H), 8.13 - 8.05 (m, 2H), 7.25 (t, *J* = 4.9 Hz, 1H), 5.25 (dd, *J* = 12.9, 5.3 Hz, 1H), 4.64 (s, 2H), 4.15 (d, *J* = 13.4 Hz, 2H), 3.51 (dd, *J* = 32.1, 12.6 Hz, 4H), 3.37 - 3.17 (m, 2H), 3.08 - 2.88 (m, 1H), 2.74 - 2.65 (m, 1H), 2.64 - 2.58 (m, 1H), 2.20 - 2.05 (m, 1H). LCMS (ESI) calcd for $C_{23}H_{22}ClFN_5O_4^+$ [M+H]$^+$: 486.13, found, 486.1.

Example 253: Preparation of 2-(2,6-dioxopiperidin-3-yl)-5-((4-(6-fluoro-3,4-dihydroquinolin-1(2H)-yl)piperidin-1-yl)methyl)isoindoline-1,3-dione (GT-06234)

Referring to the method of Scheme 1, the target compound (GT-06234) was prepared under appropriate conditions as would be understood by those skilled in the art (white solid, 48 mg, yield 60%). $^1$H NMR (400 MHz, DMSO-d6) δ 11.16 (s, 1H), 8.27 (s, 1H), 8.16 (d, J = 7.8 Hz, 1H), 8.04 (d, J = 7.7 Hz, 1H), 6.86 - 6.67 (m, 3H), 5.20 (dd, J = 12.8, 5.3 Hz, 1H), 4.50 (d, J = 3.4 Hz, 2H), 3.97 - 3.81 (m, 3H), 3.46 (d, J = 11.3 Hz, 2H), 3.18 - 3.03 (m, 3H), 2.98 - 2.84 (m, 1H), 2.71 - 2.61 (m, 2H), 2.62 - 2.55 (m, 1H), 2.29 - 2.21 (m, 2H), 2.10 - 2.03 (m, 1H), 1.83 - 1.77 (m, 4H). LCMS (ESI) calcd for $C_{28}H_{30}FN_4O_4^+$ [M+H]$^+$: 505.22, found, 505.3.

Example 254: Preparation of 2-(2,6-dioxopiperidin-3-yl)-5-((4-(7-fluoro-2,3-dihydro-4H-benzo[b][1,4]oxazin-4-yl)piperidin-1-yl)methyl)isoindoline-1,3-dione (GT-06235)

Referring to the method of Scheme 1, the target compound (GT-06235) was prepared under appropriate conditions as would be understood by those skilled in the art (white solid, 42 mg, yield 53%). $^1$H NMR (400 MHz, DMSO-d6) δ 11.43 (s, 1H), 11.16 (s, 1H), 8.26 (s, 1H), 8.15 (d, *J* = 7.6 Hz, 1H), 8.04 (d, *J* = 7.6 Hz, 1H), 6.88 - 6.79 (m, 1H), 6.62 - 6.57 (m, 2H), 5.20 (dd, *J* = 12.9, 5.3 Hz, 1H), 4.50 (d, *J* = 3.9 Hz, 2H), 3.91 (t, *J* = 11.5 Hz, 2H), 3.48 (d, *J* = 11.1 Hz, 2H), 3.21 - 3.13 (m, 2H), 3.11 - 3.00 (m, 2H), 2.99 - 2.82 (m, 1H), 2.62 (d, *J* = 17.9 Hz, 1H), 2.58 - 2.53 (m, 1H), 2.21 - 2.14 (m, 3H), 2.09 - 2.05 (m, 1H), 1.79 (d, *J* = 12.3 Hz, 2H). LCMS (ESI) calcd for $C_{27}H_{28}FN_4O_5^+$ [M+H]$^+$: 507.20, found, 507.2.

Example 255: Preparation of 2-(2,6-dioxopiperidin-3-yl)-5-((4-(7-fluoro-3-oxo-2,3-dihydro-4H-benzo[b][1,4]oxazin-4-yl)piperidin-1-yl)methyl)isoindoline-1,3-dione (GT-06236)

Referring to the method of Scheme 1, the target compound (GT-06236) was prepared under appropriate conditions as would be understood by those skilled in the art (white solid, 53 mg, yield 68%). $^1$H NMR (400 MHz, DMSO-d6) δ 11.16 (s, 1H), 10.83 (s, 1H), 8.23 (s, 1H), 8.08 (dd, *J* = 18.3, 7.7 Hz, 2H), 7.57 - 7.53 (m, 1H), 6.99 (dd, *J* = 9.2, 2.9 Hz, 1H), 6.91 (td, *J* = 8.7, 2.9 Hz, 1H), 5.20 (dd, *J* = 12.9, 5.3 Hz, 1H), 4.52 (d, *J* = 8.2 Hz, 2H), 4.46 - 4.40 (m, 1H), 3.51 (d, *J* = 11.0 Hz, 2H), 3.42 - 3.38 (m, 2H), 3.20 - 3.11 (m, 2H), 3.02 - 2.80 (m, 3H), 2.62 (d, *J* = 17.7 Hz, 1H), 2.58 - 2.52 (m, 1H), 2.15 - 2.01 (m, 1H), 1.91 (d, *J* = 12.4 Hz, 2H). LCMS (ESI) calcd for $C_{27}H_{26}FN_4O_6^+$ [M+H]$^+$: 521.18, found, 521.2.

Example 256: Preparation of 2-(2,6-dioxopiperidin-3-yl)-5-((4-(7-fluorochroman-4-yl)piperidin-1-yl)methyl)isoindoline-1,3-dione (GT-06237)

Referring to the method of Scheme 1, the target compound (GT-06237) was prepared under appropriate conditions as would be understood by those skilled in the art (white solid, 39 mg, yield 49%). $^1$H NMR (400 MHz, DMSO-d6) δ 11.15 (s, 1H), 10.72 (s, 1H), 8.21 (s, 1H), 8.08 (d, *J* = 7.8 Hz, 1H), 8.03 (d, *J* = 7.6 Hz, 1H), 7.17 - 7.08 (m, 1H), 6.67 (td, *J* = 8.5, 2.6 Hz, 1H), 6.59 (dd, *J* = 10.5, 2.6 Hz, 1H), 5.19 (dd, *J* = 12.8, 5.4 Hz, 1H), 4.47 (d, *J* = 4.4 Hz, 2H), 4.29 - 4.05 (m, 2H), 3.47 - 3.31 (m, 2H), 3.00 - 2.79 (m, 3H), 2.76 - 2.66 (m, 1H), 2.61 (d, *J* = 18.3 Hz, 1H), 2.56 (d, *J* = 4.4 Hz, 1H), 2.11 - 2.06 (m,

1H), 1.99 - 1.93 (m, 1H), 1.88 - 1.84 (m, 3H), 1.76 - 1.52 (m, 3H). LCMS (ESI) calcd for $C_{28}H_{29}FN_3O_5^+$ [M+H]$^+$: 506.21, found, 506.2.

Example 257: Preparation of 2-(2,6-dioxopiperidin-3-yl)-5-((4-(7-fluorochroman-4-ylidene)piperidin-1-yl)methyl)isoindoline-1,3-dione (GT-06238)

Referring to the method of Scheme 1, the target compound (GT-06238) was prepared under appropriate conditions as would be understood by those skilled in the art (white solid, 40 mg, yield 50%). $^1$H NMR (400 MHz, DMSO-d6) δ 11.15 (s, 1H), 10.80 (s, 1H), 8.19 (s, 1H), 8.06 (s, 2H), 7.25 - 7.16 (m, 1H), 6.73 - 6.65 (m, 2H), 5.19 (dd, *J* = 12.7, 5.3 Hz, 1H), 4.54 (d, *J* = 4.8 Hz, 2H), 4.34 - 4.29 (m, 1H), 4.24 - 4.19 (m, 1H), 3.53 - 3.39 (m, 2H), 3.11 - 2.85 (m, 4H), 2.70 - 2.64 (m, 2H), 2.61 - 2.53 (m, 2H), 2.49 - 2.37 (m, 3H), 2.12 - 2.03 (m, 1H). LCMS (ESI) calcd for $C_{28}H_{27}FN_3O_5^+$ [M+H]$^+$: 504.19, found, 504.2.

Example 258: Preparation of 2-(2,6-dioxopiperidin-3-yl)-5-((4-(5-fluoronaphthalen-1-yl)piperidin-1-yl)methyl)isoindoline-1,3-dione (GT-06263)

Referring to the method of Scheme 1, the target compound (GT-06263) was prepared under appropriate conditions as would be understood by those skilled in the art (white solid, 51 mg, yield 63%). $^1$H NMR (400 MHz, DMSO-d6) δ 11.21 (s, 1H), 11.16 (s, 1H), 8.30 (s, 1H), 8.18 (d, *J* = 7.7 Hz, 1H), 8.07 (t, *J* = 8.0 Hz, 2H), 7.98 (d, *J* = 8.3 Hz, 1H), 7.66 - 7.59 (m, 1H), 7.59 - 7.52 (m, 1H), 7.48 (d, *J* = 7.2 Hz, 1H), 7.36 (dd, *J*= 10.7, 7.7 Hz, 1H), 5.21 (dd, *J*= 12.9, 5.3 Hz, 1H), 4.59 (d, *J* = 4.1 Hz, 2H), 3.67 (t, *J* = 11.8 Hz, 1H), 3.56 (d, *J* = 11.4 Hz, 2H), 3.27 - 3.21 (m, 2H), 2.95 - 2.87 (m, 1H), 2.62 (d, *J* = 16.8 Hz, 1H), 2.56 (dd, *J* = 13.4, 4.4 Hz, 1H), 2.27 - 2.18 (m, 2H), 2.10 - 2.05 (m, 3H). LCMS (ESI) calcd for $C_{29}H_{27}FN_3O_4^+$ [M+H]$^+$: 500.20, found, 500.2.

Example 259: Preparation of 2-(2,6-dioxopiperidin-3-yl)-5-((4-(7-fluoroquinolin-4-yl)piperidin-1-yl)methyl)isoindoline-1,3-dione (GT-06265)

Referring to the method of Scheme 1, the target compound (GT-06265) was prepared under appropriate conditions as would be understood by those skilled in the art (white solid, 61 mg, yield 76%). $^1$H NMR (400 MHz, DMSO-d6) δ 11.89 (s, 1H), 11.17 (s, 1H), 9.13 (d, *J* = 5.2 Hz, 1H), 8.61 (dd, *J* = 9.5, 5.8 Hz, 1H), 8.34 (s, 1H), 8.23 (d, *J* = 7.8 Hz, 1H), 8.07 (d, *J* = 7.9 Hz, 1H), 8.06 - 8.00 (m, 1H), 7.82 - 7.77 (m, 1H), 7.64 (d, *J* = 5.3 Hz, 1H), 5.21 (dd, *J* = 12.9, 5.3 Hz, 1H), 4.60 (d, *J* = 3.0 Hz, 2H), 3.90 (t, *J* = 12.0 Hz, 1H), 3.59 (d, *J* = 11.4 Hz, 2H), 3.29 - 3.20 (m, 2H), 2.98 - 2.85 (m, 1H), 2.66 - 2.61 (m, 1H), 2.58 - 2.52 (m, 1H), 2.42 - 2.33 (m, 2H), 2.07 (d, *J* = 12.7 Hz, 3H). LCMS (ESI) calcd for $C_{28}H_{26}FN_4O_4^+$ [M+H]$^+$: 501.19, found, 501.2.

Example 260: Preparation of 2-(2,6-dioxopiperidin-3-yl)-5-((4-(isoquinolin-5-yl)piperidin-1-yl)methyl)isoindoline-1,3-dione (GT-06266)

Referring to the method of Scheme 1, the target compound (GT-06266) was prepared under appropriate conditions as would be understood by those skilled in the art (white solid, 51 mg, yield 61%). $^1$H NMR (400 MHz, DMSO-d6) δ 11.96 (s, 1H), 11.17 (s, 1H), 9.89 (s, 1H), 8.76 - 8.68 (m, 2H), 8.47 - 8.41 (m, 1H), 8.36 (s, 1H), 8.25 (d, *J*= 7.7 Hz, 1H), 8.07 (d, *J*= 7.6 Hz, 1H), 8.01 (d, *J* = 4.8 Hz, 2H), 5.21 (dd, *J* = 12.9, 5.3 Hz, 1H), 4.62 (s, 2H), 3.79 (t, *J* = 11.8 Hz, 2H), 3.58 (d, *J*= 11.1 Hz, 2H), 3.33 - 3.20 (m, 3H), 2.98 - 2.85 (m, 1H), 2.63 (d, *J*= 17.2 Hz, 1H), 2.60 - 2.54 (m, 1H), 2.45 - 2.35 (m, 2H), 2.11 - 2.04 (m, 3H). LCMS (ESI) calcd for $C_{28}H_{27}N_4O_4^+$ [M+H]$^+$: 483.20, found, 483.2.

Example 261: Preparation of 2-(2,6-dioxopiperidin-3-yl)-5-((4-(5-fluoronaphthalen-1-yl)-3,6-dihydropyridin-1(2H)-yl) methyl)isoindoline-1,3-dione (GT-06264)

Referring to the method of Scheme 1, the target compound (GT-06264) was prepared under appropriate conditions as would be understood by those skilled in the art (white solid, 55 mg, yield 68%). $^1$H NMR (400 MHz, DMSO-d6) δ 11.53 (s, 1H), 11.16 (s, 1H), 8.35 (s, 1H), 8.23 (d, *J* = 7.5 Hz, 1H), 8.08 (d, *J*= 7.6 Hz, 1H), 8.03 (d, *J* = 8.4 Hz, 1H), 7.99 (d, *J*= 8.6 Hz, 1H), 7.68 - 7.59 (m, 1H), 7.53 - 7.48 (m, 1H), 7.46 (d, *J* = 5.0 Hz, 1H), 7.37 (dd, *J*= 10.8, 7.7 Hz, 1H), 5.75 (s, 1H), 5.21 (dd, *J* = 12.9, 5.3 Hz, 1H), 4.83 - 4.57 (m, 2H), 3.99 - 3.76 (m, 2H), 3.76 - 3.62 (m, 1H), 3.49 - 3.38 (m, 1H), 3.12 - 2.95 (m, 1H), 2.97 - 2.83 (m, 1H), 2.62 (d, *J* = 14.2 Hz, 2H), 2.56 (dd, *J*= 13.3, 4.4 Hz, 1H), 2.19 - 2.00 (m, 1H). LCMS (ESI) calcd for $C_{29}H_{25}FN_3O_4^+$ [M+H]$^+$: 498.18, found, 498.2.

Example 262: Preparation of 2-(2,6-dioxopiperidin-3-yl)-5-((4-(7-fluoroquinolin-4-yl)-3,6-dihydropyridin-1(2H)-yl) methyl)isoindoline-1,3-dione (GT-06267)

Referring to the method of Scheme 1, the target compound (GT-06267) was prepared under appropriate conditions as would be understood by those skilled in the art (white solid, 65 mg, yield 80%). $^1$H NMR (400 MHz, DMSO-d6) δ 12.18 (s, 1H), 11.16 (s, 1H), 9.12 (d, *J* = 5.0 Hz, 1H), 8.54 (dd, *J* = 9.4, 6.0 Hz, 1H), 8.39 (s, 1H), 8.28 (d, *J*= 7.7 Hz, 1H), 8.07 (d, *J* = 7.7 Hz, 1H), 8.01 (dd, *J* = 9.7, 2.6 Hz, 1H), 7.70 (td, *J* = 9.0, 2.6 Hz, 1H), 7.65 (d, *J* = 5.0 Hz, 1H), 5.94 (s, 1H), 5.21 (dd, *J*= 12.9, 5.3 Hz, 1H), 4.73 (q, *J* = 14.3 Hz, 2H), 3.92 - 3.73 (m, 3H), 3.80 - 3.67 (m, 1H), 3.51 - 3.32 (m, 1H), 3.19 - 3.07 (m, 1H), 2.95 - 2.87 (m, 1H), 2.71 (d, *J* = 17.3 Hz, 1H), 2.63 (d, *J* = 16.8 Hz, 1H), 2.56 (dd, *J* = 13.5, 4.5 Hz, 1H), 2.12 - 2.07 (m, 1H). LCMS (ESI) calcd for $C_{28}H_{24}FN_4O_4^+$ [M+H]$^+$: 499.18, found, 499.2.

Example 263: Preparation of 2-(2,6-dioxopiperidin-3-yl)-5-((4-(isoquinolin-5-yl)-3,6-dihydropyridin-1(2H)-yl)methyl)isoindoline-1,3-dione (GT-06268)

Referring to the method of Scheme 1, the target compound (GT-06268) was prepared under appropriate conditions as would be understood by those skilled in the art (white solid, 60 mg, yield 72%). $^1$H NMR (400 MHz, DMSO-d6) δ 12.25 (s, 1H), 11.17 (s, 1H), 9.87 (s, 1H), 8.67 (dd, *J*= 15.3, 6.6 Hz, 2H), 8.48 (dd, *J*= 7.3, 2.0 Hz, 1H), 8.41 (s, 1H), 8.29 (dd, *J*= 7.7, 1.1 Hz, 1H), 8.07 (d, *J*= 7.7 Hz, 1H), 8.03 - 7.98 (m, 2H), 5.83 (s, 1H), 5.21 (dd, *J*= 12.9, 5.3 Hz, 1H), 4.74 (d, *J* = 9.7 Hz, 2H), 3.72 (brs, 2H), 3.43 (brs, 2H), 3.15 - 3.09 (m, 1H), 2.95 - 2.91 (m, 1H), 2.72 - 2.68 (m, 1H), 2.63 (d, *J* = 17.1 Hz, 1H), 2.57 - 2.52 (m, 1H), 2.16 - 2.03 (m, 1H). LCMS (ESI) calcd for $C_{28}H_{25}N_4O_4^+$ [M+H]$^+$: 481.19, found, 481.2.

Example 264: Preparation of 2-(2,6-dioxopiperidin-3-yl)-5-((3-(7-fluorochroman-4-yl)azetidin-1-yl)methyl)isoindoline-1,3-dione (GT-06269)

Referring to the method of Scheme 1, the target compound (GT-06269) was prepared under appropriate conditions as would be understood by those skilled in the art (white solid, 17 mg, yield 17%). $^1$H NMR (400 MHz, DMSO-d6) δ 11.22 (s, 1H), 11.15 (s, 1H), 8.17 (d, *J*= 9.0 Hz, 1H), 8.07 - 8.02 (m, 2H), 7.10 - 7.01 (m, 1H), 6.88 - 6.61 (m, 2H), 5.18 (dd, *J*= 12.8, 5.3 Hz, 1H), 4.62 - 4.58 (m, 2H), 4.45 - 4.37 (m, 1H), 4.23 - 4.10 (m, 2H), 4.10 - 3.98 (m, 2H), 3.25 - 3.17 (m, 2H), 2.99 - 2.81 (m, 2H), 2.67 - 2.58 (m, 1H), 2.59 - 2.53 (m, 1H), 2.15 - 2.02 (m, 1H), 2.01 - 1.87 (m, 1H), 1.74 - 1.65 (m, 1H). LCMS (ESI) calcd for $C_{26}H_{25}FN_3O_5^+$ [M+H]$^+$: 478.18, found, 478.2.

Example 265: Preparation of 2-(2,6-dioxopiperidin-3-yl)-5-((3-(7-fluorochroman-4-ylidene)azetidin-1-yl)methyl)isoindoline-1,3-dione (GT-08134)

Referring to the method of Scheme 1, the target compound (GT-08134) was prepared under appropriate conditions as would be understood by those skilled in the art (white solid, 25 mg, yield 33%). $^1$H NMR (400 MHz, DMSO-d6) δ 11.44 (s, 1H), 11.09 (s, 1H), 8.15 (s, 1H), 8.05 (d, *J* = 7.7 Hz, 1H), 7.98 (d, *J*= 7.7 Hz, 1H), 6.99 - 6.94 (m, 1H), 6.73 - 6.68 (m, 2H), 5.12 (dd, *J*= 12.9, 5.3 Hz, 1H), 5.04 (brs, 2H), 4.94 - 4.75 (m, 2H), 4.66 (s, 2H), 4.22 - 4.08 (m, 2H), 3.35 - 3.32(m, 2H), 2.88 - 2.73 (m, 1H), 2.61 - 2.52 (m, 1H), 2.52 - 2.46 (m, 1H), 2.08 - 1.96 (m, 1H). LCMS (ESI) calcd for $C_{26}H_{23}FN_3O_5^+$ [M+H]$^+$: 476.16, found, 476.1.

Example 266: Preparation of 2-(2,6-dioxopiperidin-3-yl)-5-((3-(7-fluoroquinolin-4-yl)azetidin-1-yl)methyl)isoindoline-1,3-dione (GT-06270)

Referring to the method of Scheme 1, the target compound (GT-06270) was prepared under appropriate conditions as would be understood by those skilled in the art (white solid, 54 mg, yield 63%). $^1$H NMR (400 MHz, DMSO-d6) δ 11.16 (s, 1H), 9.13 (d, *J* = 4.7 Hz, 1H), 8.21 (d, *J*= 7.2 Hz, 1H), 8.13 - 8.08 (m, 1H), 8.03 (d, *J* = 7.5 Hz, 1H), 8.01 - 7.95 (m, 1H), 7.93 - 7.83 (m, 1H), 7.74 (d, *J* = 5.0 Hz, 1H), 7.65 (t, *J*= 7.6 Hz, 1H), 5.18 (dd, *J*= 10.6, 5.3 Hz, 1H), 4.96 - 4.84 (m, 1H), 4.82 - 4.75 (m, 1H), 4.64 (s, 2H), 4.54 - 4.47 (m, 1H), 4.40 - 4.35 (m, 1H), 3.62 - 3.41 (m, 1H), 2.95 - 2.88 (m, 1H), 2.68 - 2.59 (m, 1H), 2.53 -2.48 (m, 1H), 2.10 -2.04 (m, 1H). LCMS (ESI) calcd for $C_{26}H_{22}FN_4O_4^+$ [M+H]$^+$: 473.16, found, 473.1.

Example 267: Preparation of 2-(2,6-dioxopiperidin-3-yl)-5-((3-(5-fluoroindolin-1-yl)azetidin-1-yl)methyl)isoindoline-1,3-dione (GT-06271)

Referring to the method of Scheme 1, the target compound (GT-06271) was prepared under appropriate conditions as would be understood by those skilled in the art (white solid, 38 mg, yield 37%). $^1$H NMR (400 MHz, DMSO-d6) δ 11.15 (s, 1H), 8.21 (d, *J* = 6.5 Hz, 1H), 8.11 (d, *J* = 7.7 Hz, 1H), 8.03 (dd, *J* = 8.0, 3.0 Hz, 1H), 6.97 (d, *J*= 8.5 Hz, 1H), 6.84 - 6.78 (m, 1H), 6.47 - 6.36 (m, 1H), 5.18 (dd, *J* = 12.8, 5.4 Hz, 1H), 4.71 (d, *J* = 6.0 Hz, 1H), 4.62 (d, *J* = 4.6 Hz, 2H), 4.40 - 4.20

(m, 4H), 3.48 (t, *J* = 8.2 Hz, 2H), 3.00 - 2.83 (m, 3H), 2.61 (d, *J*= 17.7 Hz, 1H), 2.58 - 2.52 (m, 1H), 2.15 - 2.00 (m, 1H). LCMS (ESI) calcd for $C_{25}H_{24}FN_4O_4^+$ [M+H]$^+$: 463.18, found, 463.2.

Example 268: Preparation of 2-(2,6-dioxopiperidin-3-yl)-5-((3-(5-fluoro-1H-benzo[d]imidazol-1-yl)azetidin-1-yl)methyl)isoindoline-1,3-dione (GT-06272)

Referring to the method of Scheme 1, the target compound (GT-06272) was prepared under appropriate conditions as would be understood by those skilled in the art (white solid, 26 mg, yield 25%). $^1$H NMR (400 MHz, DMSO-d6) δ 11.14 (s, 1H), 8.71 (s, 1H), 8.14 (s, 1H), 8.00 (d, *J* = 6.8 Hz, 2H), 7.86 (dd, *J* = 9.0, 4.6 Hz, 1H), 7.55 (dd, *J* = 9.4, 2.5 Hz, 1H), 7.23 (td, *J* = 9.3, 2.4 Hz, 1H), 5.53 - 5.42 (m, 1H), 5.17 (dd, *J*= 12.8, 5.4 Hz, 1H), 4.47 - 4.37 (m, 2H), 4.30 - 4.18 (m, 2H), 3.89 - 3.74 (m, 2H), 2.94 - 2.87 (m, 2H), 2.61 (d, *J* = 17.8 Hz, 1H), 2.54 (d, *J* = 14.5 Hz, 1H), 2.14 - 1.99 (m, 1H). LCMS (ESI) calcd for $C_{24}H_{21}FN_5O_4^+$ [M+H]$^+$: 462.16, found, 462.1.

Example 269: Preparation of 2-(2,6-dioxopiperidin-3-yl)-5-((4-(thieno[2,3-d]pyrimidin-4-yl)piperazin-1-yl)methyl)isoindoline-1,3-dione (GT-06303)

Referring to the method of Scheme 1, the target compound (GT-06303) was prepared under appropriate conditions as would be understood by those skilled in the art (white solid, 39 mg, yield 62%). $^1$H NMR (400 MHz, DMSO-d6) δ 12.10 (s, 1H), 11.16 (s, 1H), 8.56 (s, 1H), 8.27 (s, 1H), 8.15 (d, *J* = 7.8 Hz, 1H), 8.05 (d, *J*= 7.6 Hz, 1H), 7.77 (d, *J* = 6.1 Hz, 1H), 7.70 (d, *J* = 6.2 Hz, 1H), 5.19 (dd, *J* = 12.9, 5.4 Hz, 1H), 4.70 (d, *J* = 10.4 Hz, 2H), 4.60 (s, 2H), 3.84 - 3.63 (m, 2H), 3.45 (brs, 2H), 3.26 (brs, 2H), 2.99 - 2.81 (m, 1H), 2.62 (d, *J* = 17.7 Hz, 1H), 2.58 - 2.53 (m, 1H), 2.15 - 1.98 (m, 1H). LCMS (ESI) calcd for $C_{24}H_{23}N_6O_4S^+$ [M+H]$^+$: 491.15, found, 491.2.

Example 270: Preparation of 2-(2,6-dioxopiperidin-3-yl)-5-((4-(5-methylthieno[2,3-d]pyrimidin-4-yl)piperazin-1-yl)methyl)isoindoline-1,3-dione (GT-06304)

Referring to the method of Scheme 1, the target compound (GT-06304) was prepared under appropriate conditions as would be understood by those skilled in the art (white solid, 48 mg, yield 60%). $^1$H NMR (400 MHz, DMSO-d6) δ 11.49 (s, 1H), 11.15 (s, 1H), 8.63 (s, 1H), 8.27 (s, 1H), 8.14 (d, *J* = 7.7 Hz, 1H), 8.06 (d, *J*= 7.5 Hz, 1H), 7.48 (s, 1H), 5.18 (dd, *J* = 12.2, 6.1 Hz, 1H), 4.62 (s, 1H), 3.92 (d, *J* = 13.5 Hz, 1H), 3.83 - 3.72 (m, 4H), 3.52 (d, *J*= 13.2 Hz, 1H), 3.42 (d, *J* = 9.7 Hz, 1H), 3.39 - 3.22 (m, 2H), 2.99 - 2.83 (m, 1H), 2.69 - 2.59 (m, 2H), 2.55 (s, 3H), 2.10 - 2.07 (m, 1H). LCMS (ESI) calcd for $C_{25}H_{25}N_6O_4S^+$ [M+H]$^+$: 505.17, found, 505.2.

Example 271: Preparation of 2-(2,6-dioxopiperidin-3-yl)-5-((4-(6-methylthieno[2,3-d]pyrimidin-4-yl)piperazin-1-yl)methyl)isoindoline-1,3-dione (GT-06305)

Referring to the method of Scheme 1, the target compound (GT-06305) was prepared under appropriate conditions as would be understood by those skilled in the art (white solid, 63 mg, yield 79%). $^1$H NMR (400 MHz, DMSO-d6) δ 12.18 (s, 1H), 11.16 (s, 1H), 8.52 (s, 1H), 8.28 (s, 1H), 8.17 (dd, *J*= 7.7, 1.1 Hz, 1H), 8.05 (d, *J* = 7.6 Hz, 1H), 7.43 (d, *J* = 1.1 Hz, 1H), 5.20 (dd, *J* = 12.9, 5.4 Hz, 1H), 4.65 (s, 1H), 4.61 (s, 3H), 3.71 (brs, 2H), 3.45 (brs, 2H), 3.24 (brs, 2H), 2.95 - 2.87 (m, 1H), 2.62 (d, *J* = 17.7 Hz, 1H), 2.57 (s, 3H), 2.14 - 2.00 (m, 1H). LCMS (ESI) calcd for $C_{25}H_{25}N_6O_4S^+$ [M+H]$^+$: 505.17, found, 505.2.

Example 272: Preparation of 2-(2,6-dioxopiperidin-3-yl)-5-((4-(6-isopropylthieno[2,3-d]pyrimidin-4-yl)piperazin-1-yl)methyl)isoindoline-1,3-dione (GT-06306)

Referring to the method of Scheme 1, the target compound (GT-06306) was prepared under appropriate conditions as would be understood by those skilled in the art (white solid, 56 mg, yield 73%). $^1$H NMR (400 MHz, DMSO-d6) δ 11.94 (s, 1H), 11.16 (s, 1H), 8.49 (s, 1H), 8.27 (s, 1H), 8.16 (d, *J* = 7.8 Hz, 1H), 8.05 (d, *J*= 7.6 Hz, 1H), 7.36 (s, 1H), 5.20 (dd, *J*= 12.9, 5.3 Hz, 1H), 4.61 (s, 4H), 3.67 (brs, 2H), 3.44 (brs, 2H), 3.31 - 3.24 (m, 3H), 3.01 - 2.82 (m, 1H), 2.62 (d, *J*= 17.5 Hz, 1H), 2.58 - 2.53 (m, 1H), 2.17 - 1.96 (m, 1H), 1.34 (d, *J* = 6.8 Hz, 6H). LCMS (ESI) calcd for $C_{27}H_{29}N_6O_4S^+$ [M+H]$^+$: 533.20, found, 533.2.

Example 273: Preparation of 5-((4-(5,6-dimethylthieno[2,3-d]pyrimidin-4-yl)piperazin-1-yl)methyl)-2-(2,6-dioxopiperidin-3-yl)isoindoline-1,3-dione (GT-06307)

Referring to the method of Scheme 1, the target compound (GT-06307) was prepared under appropriate conditions as would be understood by those skilled in the art (white solid, 62 mg, yield 80%). $^1$HNMR (400 MHz, DMSO-d6)

δ 11.72 (s, 1H), 11.15 (s, 1H), 8.57 (s, 1H), 8.28 (s, 1H), 8.20 - 8.11 (m, 1H), 8.05 (d, *J* = 7.7 Hz, 1H), 5.19 (dd, *J* = 12.8, 5.4 Hz, 1H), 4.61 (s, 2H), 3.88 (d, *J* = 13.7 Hz, 2H), 3.50 (t, *J* = 12.1 Hz, 2H), 3.41 (d, *J* = 10.4 Hz, 2H), 3.34 - 3.29 (m, 2H), 2.98 - 2.81 (m, 1H), 2.62 (d, *J* = 17.7 Hz, 1H), 2.57 - 2.53 (m, 1H), 2.46 (s, 3H), 2.42 (s, 3H), 2.13 - 2.01 (m, 1H). LCMS (ESI) calcd for $C_{26}H_{27}N_6O_4S^+$ $[M+H]^+$: 519.18, found, 519.2.

Example 274: Preparation of 2-(2,6-dioxopiperidin-3-yl)-5-((4-(6-phenylthieno[2,3-d]pyrimidin-4-yl)piperazin-1-yl) methyl)isoindoline-1,3-dione (GT-06310)

Referring to the method of Scheme 1, the target compound (GT-06310) was prepared under appropriate conditions as would be understood by those skilled in the art (white solid, 58 mg, yield 80%). $^1$H NMR (400 MHz, DMSO-d6) δ 11.60 (s, 1H), 11.16 (s, 1H), 8.52 (s, 1H), 8.26 (s, 1H), 8.14 (d, *J* = 7.5 Hz, 1H), 8.07 (d, *J*= 7.6 Hz, 1H), 8.01 (s, 1H), 7.91 - 7.82 (m, 2H), 7.51 (t, *J*= 7.5 Hz, 2H), 7.43 (t, *J*= 7.3 Hz, 1H), 5.20 (dd, *J* = 12.9, 5.4 Hz, 1H), 4.73 (d, *J* = 13.7 Hz, 2H), 4.62 (s, 2H), 3.68 (t, *J* = 10.4 Hz, 2H), 3.57 - 3.54 (m, 2H), 3.28 (brs, 2H), 3.01 - 2.82 (m, 1H), 2.62 (d, *J* = 17.3 Hz, 1H), 2.59 - 2.54 (m, 1H), 2.16 - 1.94 (m, 1H). LCMS (ESI) calcd for $C_{30}H_{27}N_6O_4S^+$ $[M+H]^+$: 567.18, found, 567.2.

Example 275: Preparation of 5-((4-(6-bromothieno[2,3-d]pyrimidin-4-yl)piperazin-1-yl)methyl)-2-(2,6-dioxopiperidin-3-yl)isoindoline-1,3-dione (GT-06309)

Referring to the method of Scheme 1, the target compound (GT-06309) was prepared under appropriate conditions as would be understood by those skilled in the art (white solid, 64 mg, yield 89%). $^1$H NMR (400 MHz, DMSO-d6) δ 11.84 (s, 1H), 11.16 (s, 1H), 8.49 (s, 1H), 8.26 (s, 1H), 8.14 (d, *J* = 7.8 Hz, 1H), 8.06 (d, *J* = 7.6 Hz, 1H), 7.94 (s, 1H), 5.20 (dd, *J* = 12.9, 5.4 Hz, 1H), 4.60 (s, 4H), 3.70 - 3.56 (m, 2H), 3.53 - 3.36 (m, 2H), 3.23 (s, 2H), 2.99 - 2.81 (m, 1H), 2.62 (d, *J* = 17.6 Hz, 1H), 2.59 - 2.53 (m, 1H), 2.17 - 1.97 (m, 1H). LCMS (ESI) calcd for $C_{24}H_{22}BrN_6O_4S^+$ $[M+H]^+$: 569.06, found, 569.1.

Example 276: Preparation of 2-(2,6-dioxopiperidin-3-yl)-5-((4-(5-phenylthieno[2,3-d]pyrimidin-4-yl)piperazin-1-yl) methyl)isoindoline-1,3-dione (GT-06345)

Referring to the method of Scheme 1, the target compound (GT-06345) was prepared under appropriate conditions as would be understood by those skilled in the art (white solid, 59 mg, yield 82%). $^1$HNMR (400 MHz, DMSO-d6) δ 11.75 (s, 1H), 11.16 (s, 1H), 8.68 (s, 1H), 8.13 (s, 1H), 8.06 - 7.98 (m, 2H), 7.78 (s, 1H), 7.49 - 7.43 (m, 2H), 7.41 (dd, *J* = 7.5, 2.9 Hz, 3H), 5.20 (dd, *J*= 12.9, 5.3 Hz, 1H), 4.39 (s, 2H), 3.86 - 3.72 (m, 4H), 3.23 - 3.07 (m, 4H), 2.98 - 2.84 (m, 1H), 2.62 (d, *J*= 17.2 Hz, 1H), 2.55 (dd, *J*= 13.2, 4.3 Hz, 1H), 2.11 - 2.07 (m, 1H). LCMS (ESI) calcd for $C_{30}H_{27}N_6O_4S^+$ $[M+H]^+$: 567.18, found, 567.2.

Example 277: Preparation of 2-(2,6-dioxopiperidin-3-yl)-5-((4-(5,6,7,8-tetrahydrobenzo[4,5]thieno[2,3-d]pyrimidin-4-yl) piperazin-1-yl)methyl)isoindoline-1,3-dione (GT-06564)

Referring to the method of Scheme 1, the target compound (GT-06564) was prepared under appropriate conditions as would be understood by those skilled in the art (white solid, 52 mg, yield 79%). $^1$H NMR (400 MHz, DMSO-d6) δ 11.56 (s, 1H), 11.16 (s, 1H), 8.57 (s, 1H), 8.28 (s, 1H), 8.16 (d, *J* = 7.7 Hz, 1H), 8.06 (d, *J* = 7.6 Hz, 1H), 5.19 (dd, *J* = 12.9, 5.3 Hz, 1H), 4.62 (s, 2H), 3.87 (d, *J* = 12.8 Hz, 2H), 3.45 - 3.34 (m, 7H), 2.92 - 2.87 (m, 4H), 2.62 (d, *J* = 17.8 Hz, 1H), 2.58 - 2.52 (m, 1H), 2.13 - 2.01 (m, 1H), 1.90 - 1.85 (m, 2H), 1.84 - 1.68 (m, 2H). LCMS (ESI) calcd for $C_{28}H_{29}N_6O_4S^+$ $[M+H]^+$: 545.20, found, 545.2.

Example 278: Preparation of 5-((4-(6,7-dihydro-5H-cyclopenta[4,5]thieno[2,3-d]pyrimidin-4-yl)piperazin-1-yl) methyl)-2-(2,6-dioxopiperidin-3-yl)isoindoline-1,3-dione (GT-06565)

Referring to the method of Scheme 1, the target compound (GT-06565) was prepared under appropriate conditions as would be understood by those skilled in the art (white solid, 48 mg, yield 74%). $^1$H NMR (400 MHz, DMSO-d6) δ 12.05 (s, 1H), 11.16 (s, 1H), 8.52 (s, 1H), 8.30 (s, 1H), 8.18 (dd, *J*= 7.7, 1.0 Hz, 1H), 8.04 (d, *J*= 7.6 Hz, 1H), 5.19 (dd, *J*= 12.9, 5.3 Hz, 1H), 4.60 (s, 2H), 4.15 (d, *J*= 13.2 Hz, 2H), 3.61 (t, *J* = 11.9 Hz, 2H), 3.43 (d, *J* = 10.4 Hz, 2H), 3.29 (brs, 2H), 3.14 - 2.97 (m, 4H), 2.91 - 2.87 (m, 1H), 2.62 (d, *J* = 17.5 Hz, 1H), 2.58 - 2.53 (m, 1H), 2.47 - 2.33 (m, 2H), 2.11 - 2.07 (m, 1H). LCMS (ESI) calcd for $C_{27}H_{27}N_6O_4S^+$ $[M+H]^+$: 531.18, found, 531.2.

Example 279: Preparation of 2-(2,6-dioxopiperidin-3-yl)-5-((4-(2-methyl-5-phenylthieno[2,3-d]pyrimidin-4-yl)piperazin-1-yl)methyl)isoindoline-1,3-dione (GT-06346)

Referring to the method of Scheme 1, the target compound (GT-06346) was prepared under appropriate

conditions as would be understood by those skilled in the art (white solid, 36 mg, yield 51%). $^1$H NMR (400 MHz, DMSO-d6) δ 11.93 (s, 1H), 11.16 (s, 1H), 8.13 (s, 1H), 8.07 - 7.98 (m, 2H), 7.68 (s, 1H), 7.45 - 7.43 (m, 2H), 7.41 - 7.36 (m, 3H), 5.20 (dd, *J*= 12.9, 5.3 Hz, 1H), 4.38 (s, 2H), 3.79 (d, *J* = 12.2 Hz, 2H), 3.26 - 3.03 (m, 4H), 2.99 - 2.83 (m, 1H), 2.63 (s, 3H), 2.60 - 2.58 (m, 1H), 2.55 - 2.52(m, 1H), 2.50 - 2.43 (m, 2H), 2.15 - 2.00 (m, 1H). LCMS (ESI) calcd for $C_{31}H_{29}N_6O_4S^+$ [M+H]$^+$: 581.20, found, 581.2.

Example 280: Preparation of 2-(2,6-dioxopiperidin-3-yl)-5-((4-(2-methyl-5,6,7,8-tetrahydrobenzo[4,5]thieno[2,3-d]pyrimidin-4-yl)piperazin-1-yl)methyl)isoindoline-1,3-dione (GT-06348)

Referring to the method of Scheme 1, the target compound (GT-06348) was prepared under appropriate conditions as would be understood by those skilled in the art (white solid, 49 mg, yield 72%). $^1$H NMR (400 MHz, DMSO-d6) δ 11.80 (s, 1H), 11.16 (s, 1H), 8.30 (s, 1H), 8.18 (d, *J* = 8.0 Hz, 1H), 8.05 (d, *J*= 7.7 Hz, 1H), 5.19 (dd, *J*= 12.9, 5.3 Hz, 1H), 4.61 (s, 2H), 3.87 - 3.83 (m, 4H), 3.50 - 3.40 (m, 4H), 3.32 - 3.25 (m, 2H), 2.95 - 2.87 (m, 4H), 2.62 (d, *J*= 17.3 Hz, 1H), 2.55 (s, 3H), 2.09 - 2.05 (m, 1H), 1.92 - 1.86 (m, 2H), 1.77 - 1.65 (m, 2H). LCMS (ESI) calcd for $C_{29}H_{31}N_6O_4S^+$ [M+H]$^+$: 559.21, found, 559.3.

Example 281: Preparation of 2-(2,6-dioxopiperidin-3-yl)-5-((4-(2-methyl-6,7-dihydro-5H-cyclopenta[4,5]thieno[2,3-d] pyrimidin-4-yl)piperazin-1-yl)methyl)isoindoline-1,3-dione (GT-06566)

Referring to the method of Scheme 1, the target compound (GT-06566) was prepared under appropriate conditions as would be understood by those skilled in the art (white solid, 46 mg, yield 69%). $^1$H NMR (400 MHz, DMSO-d6) δ 11.94 (s, 1H), 11.16 (s, 1H), 8.29 (s, 1H), 8.17 (d, *J* = 7.7 Hz, 1H), 8.05 (d, *J*= 7.6 Hz, 1H), 5.19 (dd, *J*= 12.9, 5.3 Hz, 1H), 4.60 (s, 2H), 4.16 (d, *J*= 12.7 Hz, 2H), 3.59 (t, *J* = 12.5 Hz, 2H), 3.44 (d, *J* = 11.0 Hz, 2H), 3.27 (brs, 2H), 3.12 - 2.94 (m, 4H), 2.91 - 2.85 (m, 1H), 2.66 - 2.61 (m, 1H), 2.58 (d, *J* = 9.8 Hz, 1H), 2.54 (s, 3H), 2.42 - 2.33 (m, 2H), 2.11 - 1.99 (m, 1H). LCMS (ESI) calcd for $C_{28}H_{29}N_6O_4S^+$ [M+H]$^+$: 545.20, found, 545.2.

Example 282: Preparation of 2-(2,6-dioxopiperidin-3-yl)-5-((4-(2-methylthieno[2,3-d]pyrimidin-4-yl)piperazin-1-yl) methyl)isoindoline-1,3-dione (GT-06347)

Referring to the method of Scheme 1, the target compound (GT-06347) was prepared under appropriate conditions as would be understood by those skilled in the art (white solid, 68 mg, yield 85%). $^1$H NMR (400 MHz, DMSO-d6) δ 12.29 (s, 1H), 11.16 (s, 1H), 8.28 (s, 1H), 8.17 (d, *J* = 7.8 Hz, 1H), 8.05 (d, *J*= 7.6 Hz, 1H), 7.71 (s, 2H), 5.20 (dd, *J* = 12.9, 5.3 Hz, 1H), 4.77 (brs, 2H), 4.61 (brs, 2H), 3.81 (brs, 2H), 3.49 (brs, 2H), 3.27 (brs, 2H), 2.99 - 2.82 (m, 1H), 2.62 (d, *J* = 17.7 Hz, 1H), 2.59 (s, 3H), 2.54 - 2.51(m, 1H), 2.11 - 2.07 (m, 1H). LCMS (ESI) calcd for $C_{25}H_{25}N_6O_4S^+$ [M+H]$^+$: 505.17, found, 505.2.

Example 283: Preparation of 2-(2,6-dioxopiperidin-3-yl)-5-((4-(thieno[2,3-d]pyrimidin-2-yl)piperazin-1-yl)methyl)isoindoline-1,3-dione (GT-06567)

Referring to the method of Scheme 1, the target compound (GT-06567) was prepared under appropriate conditions as would be understood by those skilled in the art (white solid, 40 mg, yield 67%). $^1$H NMR (400 MHz, DMSO-d6) δ 11.59 (s, 1H), 11.16 (s, 1H), 8.97 (s, 1H), 8.25 (s, 1H), 8.12 (d, *J* = 7.7 Hz, 1H), 8.04 (d, *J*= 7.6 Hz, 1H), 7.45 (d, *J* = 5.9 Hz, 1H), 7.33 (d, *J* = 6.0 Hz, 1H), 5.19 (dd, *J* = 12.9, 5.4 Hz, 1H), 4.77 (d, *J* = 14.3 Hz, 2H), 4.56 (s, 2H), 3.52 - 3.43 (m, 4H), 3.18 - 3.06 (m, 2H), 2.99 - 2.82 (m, 1H), 2.62 (d, *J* = 17.8 Hz, 1H), 2.58 - 2.52 (m, 1H), 2.10 - 2.07 (m, 1H). LCMS (ESI) calcd for $C_{24}H_{23}N_6O_4S^+$ [M+H]$^+$: 491.15, found, 491.2.

Example 284: Preparation of 5-((4-(5,6-dimethylthieno[2,3-d]pyrimidin-4-yl)-1,4-diazepan-1-yl)methyl)-2-(2,6-dioxopiperidin-3-yl)isoindoline-1,3-dione (GT-06308)

Referring to the method of Scheme 1, the target compound (GT-06308) was prepared under appropriate conditions as would be understood by those skilled in the art (white solid, 62 mg, yield 81%). $^1$H NMR (400 MHz, DMSO-d6) δ 11.79 (s, 1H), 11.16 (s, 1H), 8.52 (s, 1H), 8.29 (s, 1H), 8.17 (d, *J* = 7.7 Hz, 1H), 8.03 (d, *J* = 7.6 Hz, 1H), 5.19 (dd, *J* = 12.7, 5.3 Hz, 1H), 4.57 (s, 2H), 4.10 - 4.02 (m, 2H), 3.92 - 3.87 (m, 1H), 3.74 - 3.68 (m, 1H), 3.57 - 3.44 (m, 3H), 3.15 (brs, 1H), 2.99 - 2.81 (m, 1H), 2.62 (d, *J*= 18.1 Hz, 1H), 2.56 (d, *J* = 4.1 Hz, 1H), 2.43 (s, 3H), 2.39 - 2.36 (m, 1H), 2.34 (s, 3H), 2.19 - 2.13 (m, 1H), 2.12 - 2.00 (m, 1H). LCMS (ESI) calcd for $C_{27}H_{29}N_6O_4S^+$ [M+H]$^+$: 533.20, found, 533.2.

Example 285: Preparation of 2-(2,6-dioxopiperidin-3-yl)-5-((4-(thieno[2,3-d]pyrimidin-4-yl)piperidin-1-yl)methyl)isoindoline-1,3-dione (GT-06591)

Referring to the method of Scheme 1, the target compound (GT-06591) was prepared under appropriate conditions as would be understood by those skilled in the art (white solid, 43 mg, yield 58%). $^1$H NMR (400 MHz, DMSO-d6) δ 11.53 (s, 1H), 11.17 (s, 1H), 9.03 (s, 1H), 8.33 (s, 1H), 8.21 (dd, *J*= 7.7, 1.0 Hz, 1H), 8.06 (d, *J*= 7.6 Hz, 1H), 8.01 (d, *J*= 6.0 Hz, 1H), 7.84 (d, *J* = 6.1 Hz, 1H), 5.21 (dd, *J*= 12.9, 5.4 Hz, 1H), 4.59 (d, *J* = 4.1 Hz, 2H), 3.64 - 3.54 (m, 3H), 3.19 - 3.11 (m, 2H), 3.01 - 2.82 (m, 1H), 2.63 (d, *J*= 17.3 Hz, 1H), 2.58 - 2.54 (m, 1H), 2.43 - 2.32 (m, 2H), 2.10 - 2.04 (m, 3H). LCMS (ESI) calcd for $C_{25}H_{24}N_5O_4S^+$ $[M+H]^+$: 490.15, found, 490.1.

Example 286: Preparation of 2-(2,6-dioxopiperidin-3-yl)-5-((4-(6-methylthieno[2,3-d]pyrimidin-4-yl)piperidin-1-yl) methyl)isoindoline-1,3-dione (GT-06592)

Referring to the method of Scheme 1, the target compound (GT-06592) was prepared under appropriate conditions as would be understood by those skilled in the art (white solid, 19 mg, yield 25%). $^1$H NMR (400 MHz, DMSO-d6) δ 11.16 (s, 1H), 11.00 (s, 1H), 8.93 (s, 1H), 8.28 (s, 1H), 8.15 (dd, *J*= 7.7, 1.1 Hz, 1H), 8.07 (d, *J*= 7.7 Hz, 1H), 7.50 (d, *J*= 1.3 Hz, 1H), 5.20 (dd, *J*= 12.9, 5.4 Hz, 1H), 4.58 (d, *J*= 4.3 Hz, 2H), 3.55 (d, *J* = 12.5 Hz, 2H), 3.16 - 3.08 (m, 2H), 2.92 - 2.86 (m, 1H), 2.67 - 2.63 (m, 1H), 2.62 (s, 3H), 2.59 (d, *J* = 10.2 Hz, 1H), 2.57 - 2.52 (m, 1H), 2.41 - 2.20 (m, 2H), 2.16 - 1.94 (m, 3H). LCMS (ESI) calcd for $C_{26}H_{26}N_5O_4S^+$ $[M+H]^+$: 504.17, found, 504.2.

Example 287: Preparation of 5-((4-(5,6-dimethylthieno[2,3-d]pyrimidin-4-yl)piperidin-1-yl)methyl)-2-(2,6-dioxopiperidin-3-yl)isoindoline-1,3-dione (GT-06593)

Referring to the method of Scheme 1, the target compound (GT-06593) was prepared under appropriate conditions as would be understood by those skilled in the art (white solid, 54 mg, yield 69%). $^1$HNMR (400 MHz, DMSO-d6) δ 11.28 (s, 1H), 11.16 (s, 1H), 8.91 (s, 1H), 8.30 (s, 1H), 8.17 (d, *J*= 7.8 Hz, 1H), 8.05 (d, *J* = 7.7 Hz, 1H), 5.20 (dd, *J* = 12.9, 5.4 Hz, 1H), 4.56 (d, *J* = 4.6 Hz, 2H), 3.78 (t, *J* = 11.7 Hz, 1H), 3.48 (d, *J* = 11.5 Hz, 2H), 3.28 - 3.20 (m, 2H), 2.91 - 2.88 (m, 1H), 2.68 - 2.59 (m, 1H), 2.58 - 2.54 (m, 1H), 2.52 (s, 3H), 2.71 (s, 3H), 2.41 - 2.26 (m, 2H), 2.16 - 2.05 (m, 1H), 2.01 (d, *J* = 13.6 Hz, 2H). LCMS (ESI) calcd for $C_{27}H_{28}N_5O_4S^+$ $[M+H]^+$: 518.19, found, 518.2.

Example 288: Preparation of 2-(2,6-dioxopiperidin-3-yl)-5-((4-(6-phenylthieno[2,3-d]pyrimidin-4-yl)piperidin-1-yl) methyl)isoindoline-1,3-dione (GT-06594)

Referring to the method of Scheme 1, the target compound (GT-06594) was prepared under appropriate conditions as would be understood by those skilled in the art (white solid, 27 mg, yield 32%). $^1$H NMR (400 MHz, DMSO-d6) δ 11.16 (s, 1H), 9.00 (s, 1H), 8.29 (d, *J*= 8.7 Hz, 2H), 8.18 (d, *J*= 8.0 Hz, 1H), 8.08 (d, *J* = 7.6 Hz, 1H), 7.90 (d, *J* = 7.4 Hz, 2H), 7.54 (t, *J* = 7.5 Hz, 2H), 7.49 (d, *J* = 7.3 Hz, 1H), 5.21 (dd, *J*= 12.9, 5.3 Hz, 1H), 4.61 (d, *J*= 4.2 Hz, 2H), 3.65 - 3.59 (m, 3H), 3.17 - 3.09 (m, 2H), 3.01 - 2.82 (m, 1H), 2.63 (d, *J* = 17.2 Hz, 1H), 2.60 - 2.54 (m, 1H), 2.41 - 2.34 (m, 2H), 2.14 - 2.08 (m, 3H). LCMS (ESI) calcd for $C_{31}H_{28}N_5O_4S^+$ $[M+H]^+$: 566.19, found, 566.2.

Example 289: Preparation of 2-(2,6-dioxopiperidin-3-yl)-5-((4-(5-phenylthieno[2,3-d]pyrimidin-4-yl)piperidin-1-yl) methyl)isoindoline-1,3-dione (GT-06595)

Referring to the method of Scheme 1, the target compound (GT-06595) was prepared under appropriate conditions as would be understood by those skilled in the art (white solid, 65 mg, yield 76%). $^1$H NMR (400 MHz, DMSO-d6) δ 11.16 (s, 1H), 11.08 (s, 1H), 9.07 (s, 1H), 8.19 (s, 1H), 8.10 - 8.01 (m, 2H), 7.84 (s, 1H), 7.52 - 7.50 (m, 2H), 7.49 - 7.42 (m, 3H), 5.21 (dd, *J* = 13.0, 5.3 Hz, 1H), 4.41 (d, *J* = 3.6 Hz, 2H), 3.35 (d, *J* = 6.9 Hz, 2H), 3.01 - 2.84 (m, 1H), 2.79 (brs, 1H), 2.63 (d, *J* = 15.9 Hz, 1H), 2.59 - 2.54 (m, 1H), 2.19 (t, *J* = 9.1 Hz, 4H), 2.10 - 2.06 (m, 1H), 1.82 - 1.74 (m, 2H). LCMS (ESI) calcd for $C_{31}H_{28}N_5O_4S^+$ $[M+H]^+$: 566.19, found, 566.2.

Example 290: Preparation of 2-(2,6-dioxopiperidin-3-yl)-5-((4-(5,6,7,8-tetrahydrobenzo[4,5]thieno[2,3-d]pyrimidin-4-yl) piperidin-1-yl)methyl)isoindoline-1,3-dione (GT-06649)

Referring to the method of Scheme 1, the target compound (GT-06649) was prepared under appropriate conditions as would be understood by those skilled in the art (white solid, 52 mg, yield 63%). $^1$H NMR (400 MHz, DMSO-d6) δ 11.20 (s, 1H), 11.16 (s, 1H), 8.90 (s, 1H), 8.29 (s, 1H), 8.16 (d, *J* = 7.8 Hz, 1H), 8.05 (d, *J*= 7.6 Hz, 1H), 5.20 (dd, *J*= 12.8, 5.3 Hz, 1H), 4.55 (d, *J* = 4.6 Hz, 2H), 3.66 (t, *J* = 11.9 Hz, 1H), 3.47 (d, *J* = 11.3 Hz, 2H), 3.26 - 3.18 (m, 2H), 3.02 (brs, 2H), 2.92 - 2.87 (m, 3H), 2.62 (d, *J* = 17.8 Hz, 1H), 2.58 - 2.53 (m, 1H), 2.41 - 2.29 (m, 2H), 2.10 - 2.07 (m, 1H), 2.00 (d, *J* = 13.5 Hz, 2H),

1.87 (brs, 4H). LCMS (ESI) calcd for $C_{29}H_{30}N_5O_4S^+$ [M+H]$^+$: 544.20, found, 544.2.

Example 291: Preparation of 5-((4-(6,7-dihydro-5H-cyclopenta[4,5]thieno[2,3-d]pyrimidin-4-yl)piperidin-1-yl) methyl)-2-(2,6-dioxopiperidin-3-yl)isoindoline-1,3-dione (GT-06650)

Referring to the method of Scheme 1, the target compound (GT-06650) was prepared under appropriate conditions as would be understood by those skilled in the art (white solid, 56 mg, yield 70%). $^1$HNMR (400 MHz, DMSO-d6) δ 11.44 (s, 1H), 11.16 (s, 1H), 8.91 (s, 1H), 8.31 (s, 1H), 8.19 (d, *J*= 7.8 Hz, 1H), 8.05 (d, *J*= 7.7 Hz, 1H), 5.20 (dd, *J*= 12.9, 5.3 Hz, 1H), 4.56 (d, *J* = 4.4 Hz, 2H), 3.51 - 3.48 (m, 3H), 3.27 - 3.19 (m, 2H), 3.13 (t, *J*= 7.0 Hz, 2H), 3.06 (t, *J* = 7.2 Hz, 2H), 2.99 - 2.83 (m, 1H), 2.62 (d, *J* = 17.7 Hz, 1H), 2.55 (dd, *J* = 13.6, 4.6 Hz, 1H), 2.49 - 2.43 (m, 1H), 2.40 - 2.29 (m, 2H), 2.12 - 2.05 (m, 1H), 2.00 (d, *J*= 13.1 Hz, 2H). LCMS (ESI) calcd for $C_{28}H_{28}N_5O_4S^+$ [M+H]$^+$: 530.19, found, 530.2.

Example 292: Preparation of 2-(2,6-dioxopiperidin-3-yl)-5-((4-(2-methyl-5-phenylthieno[2,3-d]pyrimidin-4-yl)piperidin-1-yl)methyl)isoindoline-1,3-dione (GT-06646)

Referring to the method of Scheme 1, the target compound (GT-06646) was prepared under appropriate conditions as would be understood by those skilled in the art (white solid, 58 mg, yield 66%). $^1$H NMR (400 MHz, DMSO-d6) δ 11.17 (s, 1H), 10.96 (s, 1H), 8.19 (s, 1H), 8.06 (d, *J* = 2.2 Hz, 2H), 7.70 (s, 1H), 7.49 - 7.44 (m, 5H), 5.21 (dd, *J*= 13.0, 5.4 Hz, 1H), 4.41 (d, *J* = 3.7 Hz, 2H), 3.34 (d, *J* = 8.2 Hz, 2H), 3.01 - 2.84 (m, 1H), 2.79 - 2.74 (m, 1H), 2.70 (s, 3H), 2.67 - 2.60 (m, 1H), 2.58 - 2.54 (m, 1H), 2.21 - 2.03 (m, 5H), 1.75 (d, *J* = 9.8 Hz, 2H). LCMS (ESI) calcd for $C_{32}H_{30}N_5O_4S^+$ [M+H]$^+$: 580.20, found, 580.2.

Example 293: Preparation of 2-(2,6-dioxopiperidin-3-yl)-5-((4-(2-methyl-5,6,7,8-tetrahydrobenzo[4,5]thieno[2,3-d]pyrimidin-4-yl)piperidin-1-yl)methyl)isoindoline-1,3-dione (GT-06648)

Referring to the method of Scheme 1, the target compound (GT-06648) was prepared under appropriate conditions as would be understood by those skilled in the art (white solid, 21 mg, yield 25%). $^1$H NMR (400 MHz, DMSO-d6) δ 11.16 (s, 1H), 10.87 (s, 1H), 8.27 (s, 1H), 8.14 (dd, *J*= 7.3, 1.4 Hz, 1H), 8.05 (d, *J* = 7.7 Hz, 1H), 5.20 (dd, *J* = 12.9, 5.4 Hz, 1H), 4.55 (d, *J* = 4.7 Hz, 2H), 3.62 (t, *J*= 11.6 Hz, 1H), 3.47 (d, *J* = 11.0 Hz, 2H), 3.28 - 3.19 (m, 2H), 2.99 (brs, 2H), 2.90 - 2.85 (m, 3H), 2.63 (s, 3H), 2.61 - 2.55 (m, 2H), 2.33 - 2.24 (m, 3H), 2.13 - 2.05 (m, 2H), 1.99 (d, *J* = 13.0 Hz, 3H). LCMS (ESI) calcd for $C_{30}H_{32}N_5O_4S^+$ [M+H]$^+$: 558.22, found, 558.2.

Example 294: Preparation of 2-(2,6-dioxopiperidin-3-yl)-5-((4-(2-methyl-6,7-dihydro-5H-cyclopenta[4,5]thieno[2,3-d] pyrimidin-4-yl)piperidin-1-yl)methyl)isoindoline-1,3-dione (GT-06651)

Referring to the method of Scheme 1, the target compound (GT-06651) was prepared under appropriate conditions as would be understood by those skilled in the art (white solid, 48 mg, yield 58%). $^1$H NMR (400 MHz, DMSO-d6) δ 11.16 (s, 2H), 11.13 (s, 1H), 8.29 (s, 1H), 8.17 (dd, *J*= 7.7, 0.9 Hz, 1H), 8.05 (d, *J*= 7.6 Hz, 1H), 5.20 (dd, *J*= 12.9, 5.4 Hz, 1H), 4.56 (d, *J* = 4.5 Hz, 2H), 3.49 - 3.42 (m, 3H), 3.27 - 3.18 (m, 2H), 3.10 (t, *J* = 7.0 Hz, 2H), 3.02 (t, *J*= 7.2 Hz, 2H), 2.97 - 2.85 (m, 1H), 2.66 (s, 3H), 2.63 - 2.58 (m, 1H), 2.57 - 2.52 (m, 1H), 2.49 - 2.43 (m, 1H), 2.33 - 2.25 (m, 3H), 2.11 - 2.06 (m, 1H), 1.99 (d, *J* = 13.1 Hz, 2H). LCMS (ESI) calcd for $C_{29}H_{30}N_5O_4S^+$ [M+H]$^+$: 544.20, found, 544.2.

Example 295: Preparation of 2-(2,6-dioxopiperidin-3-yl)-5-((4-(2-methylthieno[2,3-d]pyrimidin-4-yl)piperidin-1-yl) methyl)isoindoline-1,3-dione (GT-06647)

Referring to the method of Scheme 1, the target compound (GT-06647) was prepared under appropriate conditions as would be understood by those skilled in the art (white solid, 39 mg, yield 51%). $^1$H NMR (400 MHz, DMSO-d6) δ 11.32 (s, 1H), 11.16 (s, 1H), 8.31 (s, 1H), 8.19 (d, *J* = 7.7 Hz, 1H), 8.06 (d, *J* = 7.7 Hz, 1H), 7.87 (d, *J* = 6.0 Hz, 1H), 7.78 (dd, *J*= 6.0, 1.2 Hz, 1H), 5.20 (dd, *J*= 12.9, 5.3 Hz, 1H), 4.58 (d, *J* = 4.2 Hz, 2H), 3.55 (d, *J* = 11.6 Hz, 3H), 3.19 - 3.10 (m, 2H), 2.99 - 2.85 (m, 1H), 2.70 (s, 3H), 2.62 (d, *J*= 17.4 Hz, 1H), 2.55 - 2.50 (m, 1H), 2.38 -2.29 (m, 2H), 2.10 - 2.02 (m, 3H). LCMS (ESI) calcd for $C_{26}H_{26}N_5O_4S^+$ [M+H]$^+$: 504.17, found, 504.2.

Example 296: Preparation of 2-(2,6-dioxopiperidin-3-yl)-5-((4-(thieno[2,3-d]pyrimidin-2-yl)piperidin-1-yl)methyl)isoindoline-1,3-dione (GT-06652)

Referring to the method of Scheme 1, the target compound (GT-06652) was prepared under appropriate conditions as would be understood by those skilled in the art (white solid, 18 mg, yield 24%). $^1$H NMR (400 MHz, DMSO-d6) δ 11.16 (s, 1H), 11.12 (s, 1H), 9.30 (s, 1H), 8.29 (s, 1H), 8.17 (d,*J* = 7.8 Hz, 1H), 8.05 (d, *J* = 7.7 Hz, 1H), 7.93 (d, *J* = 5.9 Hz,

1H), 7.56 (d, *J* = 5.9 Hz, 1H), 5.20 (dd, *J* = 13.4, 5.9 Hz, 1H), 4.56 (d, *J* = 4.7 Hz, 2H), 3.50 (d, *J* = 11.2 Hz, 2H), 3.30 - 3.13 (m, 3H), 2.94 - 2.87 (m, 2H), 2.64 - 2.60 (m, 1H), 2.56 - 2.53 (m, 1H), 2.29 - 2.20 (m, 4H), 2.11 - 2.02 (m, 2H). LCMS (ESI) calcd for $C_{25}H_{24}N_5O_4S^+$ [M+H]+: 490.15, found, 490.2.

Example 297: Preparation of 2-(2,6-dioxopiperidin-3-yl)-5-((4-(thieno[2,3-d]pyrimidin-4-yl)-3,6-dihydropyridin-1(2H)-yl) methyl)isoindoline-1,3-dione (GT-06653)

Referring to the method of Scheme 1, the target compound (GT-06653) was prepared under appropriate conditions as would be understood by those skilled in the art (white solid, 43 mg, yield 58%). $^1$H NMR (400 MHz, DMSO-d6) δ 11.79 (s, 1H), 11.16 (s, 1H), 9.08 (s, 1H), 8.36 (s, 1H), 8.08 (s, 1H), 8.06 (s, 1H), 8.04 (s, 1H), 7.77 (d, *J* = 6.1 Hz, 1H), 6.66 (s, 1H), 5.26 - 5.15 (m, 2H), 4.73 - 4.69 (m, 2H), 3.73 - 3.70 (m, 1H), 3.34 (brs, 1H), 3.05 - 3.02 (m, 1H), 2.93 - 2.86 (m, 2H), 2.62 (d, *J*= 14.3 Hz, 1H), 2.56 (d, *J*= 8.7 Hz, 1H), 2.10 - 2.07 (m, 2H). LCMS (ESI) calcd for $C_{25}H_{22}N_5O_4S^+$ [M+H]+: 488.14, found, 488.1.

Example 298: Preparation of 2-(2,6-dioxopiperidin-3-yl)-5-((4-(5-methylthieno[2,3-d]pyrimidin-4-yl)-3,6-dihydropyridin-1(2H)-yl)methyl)isoindoline-1,3-dione (GT-06654)

Referring to the method of Scheme 1, the target compound (GT-06654) was prepared under appropriate conditions as would be understood by those skilled in the art (white solid, 43 mg, yield 56%). $^1$H NMR (400 MHz, DMSO-d6) δ 11.85 (s, 1H), 11.16 (s, 1H), 9.03 (s, 1H), 8.35 (s, 1H), 8.24 (d, *J* = 7.8 Hz, 1H), 8.07 (d, *J* = 7.7 Hz, 1H), 7.65 (s, 1H), 5.91 (s, 1H), 5.20 (dd, *J* = 12.9, 5.4 Hz, 1H), 4.75 - 4.64 (m, 2H), 3.99 - 3.80 (m, 2H), 3.77 - 3.64 (m, 1H), 3.45 - 3.25 (m, 1H), 3.17 - 3.06 (m, 1H), 3.00 - 2.83 (m, 2H), 2.62 (d, *J* = 17.4 Hz, 1H), 2.55 - 2.51 (m, 1H), 2.43 (s, 3H), 2.10 - 2.04 (m, 1H). LCMS (ESI) calcd for $C_{26}H_{24}N_5O_4S^+$ [M+H]+: 502.15, found, 502.2.

Example 299: Preparation of 2-(2,6-dioxopiperidin-3-yl)-5-((4-(6-methylthieno[2,3-d]pyrimidin-4-yl)-3,6-dihydropyridin-1(2H)-yl)methyl)isoindoline-1,3-dione (GT-06655)

Referring to the method of Scheme 1, the target compound (GT-06655) was prepared under appropriate conditions as would be understood by those skilled in the art (white solid, 37 mg, yield 48%). $^1$H NMR (400 MHz, DMSO-d6) δ 11.87 (s, 1H), 11.16 (s, 1H), 8.98 (s, 1H), 8.37 (s, 1H), 8.25 (dd, *J*= 7.7, 1.1 Hz, 1H), 8.07 (d, *J* = 7.7 Hz, 1H), 7.49 (d, *J* = 1.2 Hz, 1H), 6.62 (s, 1H), 5.21 (dd, *J* = 12.9, 5.3 Hz, 1H), 4.78 - 4.65 (m, 2H), 4.02 - 3.91 (m, 2H), 3.72 - 3.70 (m, 1H), 3.32 (brs, 1H), 3.08 - 2.88 (m, 3H), 2.63 (s, 3H), 2.59 (d, *J* = 9.6 Hz, 1H), 2.54 (d, *J* = 4.4 Hz, 1H), 2.14 - 2.07 (m, 1H). LCMS (ESI) calcd for $C_{26}H_{24}N_5O_4S^+$ [M+H]+: 502.15, found, 502.2.

Example 300: Preparation of 2-(2,6-dioxopiperidin-3-yl)-5-((4-(6-isopropylthieno[2,3-d]pyrimidin-4-yl)-3,6-dihydropyridin-1(2H)-yl)methyl)isoindoline-1,3-dione (GT-06656)

Referring to the method of Scheme 1, the target compound (GT-06656) was prepared under appropriate conditions as would be understood by those skilled in the art (white solid, 59 mg, yield 73%). $^1$H NMR (400 MHz, DMSO-d6) δ 11.70 (s, 1H), 11.16 (s, 1H), 8.99 (s, 1H), 8.35 (s, 1H), 8.23 (d, *J* = 7.7 Hz, 1H), 8.07 (d, *J* = 7.7 Hz, 1H), 7.48 (s, 1H), 6.64 (s, 1H), 5.20 (dd, *J* = 13.0, 5.3 Hz, 1H), 4.78 - 4.62 (m, 2H), 4.07 - 3.88 (m, 2H), 3.74 - 3.69 (m, 1H), 3.35 - 3.32 (m, 2H), 3.10 - 3.03 (m, 2H), 2.93 - 2.83 (m, 1H), 2.63 (d, *J*= 16.5 Hz, 1H), 2.56 (dd, *J* = 13.3, 4.2 Hz, 1H), 2.12 - 2.06 (m, 1H), 1.37 (s, 3H), 1.35 (s, 3H). LCMS (ESI) calcd for $C_{28}H_{28}N_5O_4S^+$ [M+H]+: 530.19, found, 530.2.

Example 301: Preparation of 5-((4-(5,6-dimethylthieno[2,3-d]pyrimidin-4-yl)-3,6-dihydropyridin-1 (2H)-yl) methyl)-2-(2,6-dioxopiperidin-3-yl)isoindoline-1,3-dione (GT-06657)

Referring to the method of Scheme 1, the target compound (GT-06657) was prepared under appropriate conditions as would be understood by those skilled in the art (white solid, 37 mg, yield 47%). $^1$H NMR (400 MHz, DMSO-d6) δ 11.49 (s, 1H), 11.16 (s, 1H), 8.94 (s, 1H), 8.31 (s, 1H), 8.19 (d, *J*= 7.7 Hz, 1H), 8.07 (d, *J*= 7.6 Hz, 1H), 5.84 (s, 1H), 5.20 (dd, *J*= 12.8, 5.4 Hz, 1H), 4.79 - 4.61 (m, 2H), 3.97 - 3.81 (m, 3H), 3.77 - 3.65 (m, 1H), 3.46 - 3.30 (m, 1H), 3.10 - 3.02 (m, 1H), 2.95 - 2.88 (m, 2H), 2.71 - 2.58 (m, 1H), 2.57 (d, *J* = 4.3 Hz, 1H), 2.52 (s, 3H), 2.29 (s, 3H), 2.12 - 2.04 (m, 1H). LCMS (ESI) calcd for $C_{27}H_{26}N_5O_4S^+$ [M+H]+: 516.17, found, 516.2.

Example 302: Preparation of 2-(2,6-dioxopiperidin-3-yl)-5-((4-(6-phenylthieno[2,3-d]pyrimidin-4-yl)-3,6-dihydropyridin-1(2H)-yl)methyl)isoindoline-1,3-dione (GT-06675)

Referring to the method of Scheme 1, the target compound (GT-06675) was prepared under appropriate

conditions as would be understood by those skilled in the art (white solid, 42 mg, yield 61%). $^1$H NMR (400 MHz, DMSO-d6) δ 11.16 (s, 1H), 11.14 (s, 1H), 9.06 (s, 1H), 8.31 (s, 1H), 8.18 (d, *J* = 7.0 Hz, 1H), 8.13 (s, 1H), 8.10 (d, *J* = 7.7 Hz, 1H), 7.94 - 7.86 (m, 2H), 7.57 - 7.52 (m, 2H), 7.50 - 7.47 (m, 1H), 6.80 (s, 1H), 5.21 (dd, *J* = 12.9, 5.4 Hz, 1H), 4.84 - 4.61 (m, 2H), 4.15 - 3.91 (m, 2H), 3.88 - 3.72 (m, 1H), 3.32 - 3.29 (m, 1H), 3.15 - 3.00 (m, 2H), 2.99 - 2.84 (m, 1H), 2.67 - 2.58 (m, 1H), 2.56 - 2.52 (m, 1H), 2.20 - 1.97 (m, 1H). LCMS (ESI) calcd for $C_{31}H_{26}N_5O_4S^+$ [M+H]$^+$: 564.17, found, 564.2.

Example 303: Preparation of 2-(2,6-dioxopiperidin-3-yl)-5-((4-(5-phenylthieno[2,3-d]pyrimidin-4-yl)-3,6-dihydropyridin-1(2H)-yl)methyl)isoindoline-1,3-dione (GT-06676)

Referring to the method of Scheme 1, the target compound (GT-06676) was prepared under appropriate conditions as would be understood by those skilled in the art (white solid, 41 mg, yield 60%). $^1$H NMR (400 MHz, DMSO-d6) δ 11.32 (s, 1H), 11.17 (s, 1H), 9.12 (s, 1H), 8.23 (s, 1H), 8.10 (d, *J* = 2.4 Hz, 2H), 7.96 (s, 1H), 7.42 - 7.29 (m, 4H), 7.18 (t, *J* = 6.6 Hz, 1H), 5.32 - 5.18 (m, 2H), 4.56 - 4.40 (m, 2H), 3.60 - 3.54 (m, 1H), 3.30 - 3.26 (m, 1H), 3.14 - 3.05 (m, 1H), 3.00 - 2.86 (m, 1H), 2.79 - 2.52 (m, 5H), 2.12 - 2.08 (m, 1H). LCMS (ESI) calcd for $C_{31}H_{26}N_5O_4S^+$ [M+H]$^+$: 564.17, found, 564.2.

Example 304: Preparation of 2-(2,6-dioxopiperidin-3-yl)-5-((4-(5,6,7,8-tetrahydrobenzo[4,5]thieno[2,3-d]pyrimidin-4-yl)-3,6-dihydropyridin-1(2H)-yl)methyl)isoindoline-1,3-dione (GT-06680)

Referring to the method of Scheme 1, the target compound (GT-06680) was prepared under appropriate conditions as would be understood by those skilled in the art (white solid, 60 mg, yield 73%). $^1$H NMR (400 MHz, DMSO-d6) δ 11.80 (s, 1H), 11.16 (s, 1H), 8.94 (s, 1H), 8.34 (s, 1H), 8.24 (d, *J* = 7.7 Hz, 1H), 8.07 (d, *J* = 7.7 Hz, 1H), 5.86 (s, 1H), 5.20 (dd, *J* = 12.9, 5.4 Hz, 1H), 4.83 - 4.58 (m, 2H), 3.86 - 3.80 (m, 2H), 3.74 - 3.69 (m, 1H), 3.45 - 3.25 (m, 1H), 3.11 - 3.07 (m, 1H), 2.98 - 2.77 (m, 6H), 2.64 (d, *J* = 2.6 Hz, 1H), 2.59 - 2.53 (m, 1H), 2.11 - 2.07 (m, 1H), 1.90 - 1.64 (m, 4H). LCMS (ESI) calcd for $C_{29}H_{28}N_5O_4S^+$ [M+H]$^+$: 542.19, found, 542.2.

Example 305: Preparation of 5-((4-(6,7-dihydro-5H-cyclopenta[4,5]thieno[2,3-d]pyrimidin-4-yl)-3,6-dihydropyridin-1(2H)-yl)methyl)-2-(2,6-dioxopiperidin-3-yl)isoindoline-1,3-dione (GT-06681)

Referring to the method of Scheme 1, the target compound (GT-06681) was prepared under appropriate conditions as would be understood by those skilled in the art (white solid, 61 mg, yield 76%). $^1$H NMR (400 MHz, DMSO-d6) δ 11.80 (s, 1H), 11.16 (s, 1H), 8.95 (s, 1H), 8.33 (s, 1H), 8.23 (d, *J* = 7.7 Hz, 1H), 8.07 (d, *J* = 7.7 Hz, 1H), 6.09 (s, 1H), 5.20 (dd, *J* = 12.9, 5.4 Hz, 1H), 4.73 - 4.63 (m, 2H), 4.03 - 3.98 (m, 1H), 3.73 - 3.70 (m, 2H), 3.35 - 3.29 (m, 1H), 3.14 - 3.01 (m, 4H), 2.99 - 2.95 (m, 1H), 2.93 - 2.83 (m, 2H), 2.62 (d, *J* = 17.3 Hz, 1H), 2.55 (dd, *J* = 13.6, 4.6 Hz, 1H), 2.44 - 2.31 (m, 2H), 2.14 - 2.01 (m, 1H). LCMS (ESI) calcd for $C_{28}H_{26}N_5O_4S^+$ [M+H]$^+$: 528.17, found, 528.2.

Example 306: Preparation of 2-(2,6-dioxopiperidin-3-yl)-5-((4-(2-methyl-5-phenylthieno[2,3-d]pyrimidin-4-yl)-3,6-dihydropyridin-1(2H)-yl)methyl)isoindoline-1,3-dione (GT-06677)

Referring to the method of Scheme 1, the target compound (GT-06677) was prepared under appropriate conditions as would be understood by those skilled in the art (white solid, 54 mg, yield 77%). $^1$H NMR (400 MHz, DMSO-d6) δ 11.44 (s, 1H), 11.17 (s, 1H), 8.24 (s, 1H), 8.13 - 8.08 (m, 2H), 7.82 (s, 1H), 7.40 - 7.27 (m, 4H), 7.17 - 7.13 (m, 1H), 5.23 (dd, *J* = 13.0, 5.4 Hz, 2H), 4.62 - 4.38 (m, 2H), 3.62 - 3.45 (m, 1H), 3.28 (d, *J* = 16.7 Hz, 1H), 3.14 - 2.99 (m, 1H), 2.99 - 2.85 (m, 1H), 2.75 (s, 3H), 2.69 - 2.59 (m, 4H), 2.20 - 2.00 (m, 1H). LCMS (ESI) calcd for $C_{32}H_{28}N_5O_4S^+$ [M+H]$^+$: 578.19, found, 578.2.

Example 307: Preparation of 2-(2,6-dioxopiperidin-3-yl)-5-((4-(2-methyl-5,6,7,8-tetrahydrobenzo[4,5]thieno[2,3-d]pyrimidin-4-yl)-3,6-dihydropyridin-1(2H)-yl)methyl)isoindoline-1,3-dione (GT-06679)

Referring to the method of Scheme 1, the target compound (GT-06679) was prepared under appropriate conditions as would be understood by those skilled in the art (white solid, 44 mg, yield 52%). $^1$H NMR (400 MHz, DMSO-d6) δ 11.71 (s, 1H), 11.16 (s, 1H), 8.34 (s, 1H), 8.24 (d, *J* = 7.7 Hz, 1H), 8.07 (d, *J* = 7.7 Hz, 1H), 5.84 (s, 1H), 5.20 (dd, *J* = 12.9, 5.4 Hz, 1H), 4.75 - 4.66 (m, 2H), 3.95 - 2.79 (m, 2H), 3.70 - 3.67 (m, 1H), 3.47 - 3.27 (m, 1H), 3.10 - 2.97 (m, 1H), 2.95 - 2.83 (m, 4H), 2.80 - 2.76 (m, 1H), 2.68 (s, 3H), 2.66 - 2.60 (m, 1H), 2.57 - 2.53 (m, 1H), 2.15 - 2.01 (m, 1H), 1.87 - 1.70 (m, 4H). LCMS (ESI) calcd for $C_{30}H_{30}N_5O_4S^+$ [M+H]$^+$: 556.20, found, 556.2.

Example 308: Preparation of 2-(2,6-dioxopiperidin-3-yl)-5-((4-(2-methyl-6,7-dihydro-5H-cyclopenta[4,5]thieno[2,3-d]pyrimidin-4-yl)-3,6-dihydropyridin-1(2H)-yl)methyl)isoindoline-1,3-dione (GT-06682)

Referring to the method of Scheme 1, the target compound (GT-06682) was prepared under appropriate conditions as would be understood by those skilled in the art (white solid, 45 mg, yield 55%). $^1$H NMR (400 MHz, DMSO-d6) δ 11.83 (s, 1H), 11.16 (s, 1H), 8.34 (s, 1H), 8.24 (d, *J* = 7.6 Hz, 1H), 8.07 (d, *J* = 7.7 Hz, 1H), 6.05 (s, 1H), 5.20 (dd, *J* = 12.9, 5.4 Hz, 1H), 4.79 - 4.59 (m, 2H), 3.85 - 3.81 (m, 2H), 3.71 (d, *J* = 11.4 Hz, 1H), 3.33 (brs, 1H), 3.03 - 2.92 (m, 4H), 2.88 - 2.82 (m, 2H), 2.68 (s, 3H), 2.64 - 2.61 (m, 1H), 2.55 (dd, *J* = 13.6, 4.7 Hz, 1H), 2.40 - 2.33 (m, 2H), 2.11 - 2.05 (m, 1H). LCMS (ESI) calcd for $C_{29}H_{28}N_5O_4S^+$ [M+H]$^+$: 542.19, found, 542.2.

Example 309: Preparation of 2-(2,6-dioxopiperidin-3-yl)-5-((4-(2-methylthieno[2,3-d]pyrimidin-4-yl)-3,6-dihydropyridin-1(2H)-yl)methyl)isoindoline-1,3-dione (GT-06678)

Referring to the method of Scheme 1, the target compound (GT-06678) was prepared under appropriate conditions as would be understood by those skilled in the art (white solid, 39 mg, yield 51%). $^1$H NMR (400 MHz, DMSO-d6) δ 11.77 (s, 1H), 11.16 (s, 1H), 8.36 (s, 1H), 8.24 (dd, *J* = 7.7, 1.1 Hz, 1H), 8.07 (d, *J* = 7.7 Hz, 1H), 7.91 (d, *J* = 6.1 Hz, 1H), 7.69 (d, *J* = 6.1 Hz, 1H), 6.59 (s, 1H), 5.20 (dd, *J* = 12.9, 5.4 Hz, 1H), 4.81 - 4.61 (m, 2H), 4.02 - 3.92 (m, 2H), 3.77 - 3.64 (m, 1H), 3.33 (brs, 1H), 3.02 - 2.96 (m, 2H), 2.94 - 2.87 (m, 1H), 2.72 (s, 3H), 2.66 (d, *J* = 14.3 Hz, 1H), 2.59 - 2.53 (m, 1H), 2.11 - 2.07 (m, 1H). LCMS (ESI) calcd for $C_{26}H_{24}N_5O_4S^+$ [M+H]$^+$: 502.15, found, 502.2.

Example 310: Preparation of 2-(2,6-dioxopiperidin-3-yl)-5-((4-(thieno[2,3-d]pyrimidin-2-yl)-3,6-dihydropyridin-1(2H)-yl)methyl)isoindoline-1,3-dione (GT-06683)

Referring to the method of Scheme 1, the target compound (GT-06683) was prepared under appropriate conditions as would be understood by those skilled in the art (white solid, 17 mg, yield 23%). $^1$H NMR (400 MHz, DMSO-d6) δ 11.26 (s, 1H), 11.15 (s, 1H), 9.34 (s, 1H), 8.30 (s, 1H), 8.18 (d, *J* = 7.8 Hz, 1H), 8.07 (d, *J* = 7.7 Hz, 1H), 7.97 (d, *J* = 5.9 Hz, 1H), 7.59 (d, *J* = 5.9 Hz, 1H), 7.20 (s, 1H), 5.20 (dd, *J* = 12.9, 5.4 Hz, 1H), 4.79 - 4.57 (m, 2H), 4.04 - 3.86 (m, 2H), 3.80 - 3.67 (m, 1H), 3.36 - 3.26 (m, 1H), 3.17 - 2.98 (m, 2H), 2.97 - 2.84 (m, 1H), 2.62 (d, *J* = 17.2 Hz, 1H), 2.55 (dd, *J* = 13.2, 4.3 Hz, 1H), 2.17 - 2.02 (m, 1H). LCMS (ESI) calcd for $C_{25}H_{22}N_5O_4S^+$ [M+H]$^+$: 488.14, found, 488.2.

Example 311: Preparation of 2-(2,6-dioxopiperidin-3-yl)-5-((4-(2-(piperidin-1-yl)-6,7-dihydrothieno[3,2-d]pyrimidin-4-yl)piperazin-1-yl)methyl)isoindoline-1,3-dione (GT-08003)

Referring to the method of Scheme 1, the target compound (GT-08003) was prepared under appropriate conditions as would be understood by those skilled in the art (white solid, 37 mg, yield 69%). $^1$H NMR (400 MHz, DMSO-d6) δ 11.97 (s, 1H), 11.15 (s, 1H), 8.24 (s, 1H), 8.12 (d, J = 8.5 Hz, 1H), 8.03 (d, J = 7.6 Hz, 1H), 5.19 (dd, J = 12.9, 5.4 Hz, 1H), 4.55 - 4.44 (m, 4H), 3.75 - 3.66 (m, 5H), 3.37 - 3.30 (m, 4H), 3.29 - 3.19 (m, 3H), 3.17 - 3.10 (m, 2H), 2.95 - 2.86 (m, 1H), 2.62 (d, J = 18.0 Hz, 1H), 2.59 - 2.52 (m, 1H), 2.15 - 2.01 (m, 1H), 1.66 - 1.60 (m, 2H), 1.54 - 1.51 (m, 4H). LCMS (ESI) calcd for $C_{29}H_{34}N_7O_4S^+$ [M+H]$^+$: 576.24, found, 576.2.

Example 312: Preparation of 2-(2,6-dioxopiperidin-3-yl)-5-((4-(2-(pyrrolidin-1-yl)-6,7-dihydrothieno[3,2-d]pyrimidin-4-yl)piperazin-1-yl)methyl)isoindoline-1,3-dione (GT-08004)

Referring to the method of Scheme 1, the target compound (GT-08004) was prepared under appropriate conditions as would be understood by those skilled in the art (white solid, 27 mg, yield 50%). $^1$H NMR (400 MHz, DMSO-d6) δ 12.12 (s, 1H), 11.15 (s, 1H), 8.24 (s, 1H), 8.12 (d, *J* = 7.5 Hz, 1H), 8.03 (d, *J* = 7.6 Hz, 1H), 5.19 (dd, *J* = 12.8, 5.4 Hz, 1H), 4.54 (brs, 4H), 3.61 - 3.53 (m, 7H), 3.35 - 3.10 (m, 7H), 2.91 - 2.85 (m, 1H), 2.62 (d, *J* = 18.0 Hz, 1H), 2.58 - 2.52 (m, 1H), 2.11 - 2.95 (m, 1H), 2.03 - 1.86 (m, 4H). LCMS (ESI) calcd for $C_{28}H_{32}N_7O_4S^+$ [M+H]$^+$: 562.22, found, 562.2.

Example 313: Preparation of 5-((4-(2-(azetidin-1-yl)-6,7-dihydrothieno[3,2-d]pyrimidin-4-yl)piperazin-1-yl)methyl)-2-(2,6-dioxopiperidin-3-yl)isoindoline-1,3-dione (GT-08005)

Referring to the method of Scheme 1, the target compound (GT-08005) was prepared under appropriate conditions as would be understood by those skilled in the art (white solid, 22 mg, yield 39%). $^1$H NMR (400 MHz, DMSO-d6) δ 12.18 (s, 1H), 11.15 (s, 1H), 8.24 (s, 1H), 8.13 (d, *J* = 7.7 Hz, 1H), 8.03 (d, *J* = 7.6 Hz, 1H), 5.19 (dd, *J* = 12.8, 5.4 Hz, 1H), 4.54 (brs, 4H), 4.12 (t, *J* = 7.4 Hz, 3H), 3.77 - 3.54 (m, 5H), 3.31 - 3.03 (m, 7H), 2.98 - 2.82 (m, 1H), 2.62 (d, *J* = 17.8 Hz, 1H), 2.58 - 2.52 (m, 1H), 2.41 - 2.28 (m, 1H), 2.10 - 2.05 (m, 1H). LCMS (ESI) calcd for $C_{27}H_{30}N_7O_4S^+$ [M+H]$^+$: 548.21, found, 548.2.

Example 314: Preparation of 5-((4-(2-((2,4-dimethylphenyl)thio)phenyl)piperazin-1-yl)methyl)-2-(2,6-dioxopiperidin-3-yl)isoindoline-1,3-dione (GT-06761)

Referring to the method of Scheme 1, the target compound (GT-06761) was prepared under appropriate conditions as would be understood by those skilled in the art (white solid, 60 mg, yield 70%). $^1$H NMR (400 MHz, DMSO-d6) δ 11.50 (s, 1H), 11.16 (s, 1H), 8.30 (s, 1H), 8.18 (d, *J* = 7.4 Hz, 1H), 8.05 (d, *J*= 7.6 Hz, 1H), 7.33 (d, *J*= 7.5 Hz, 1H), 7.24 (d, *J* = 9.8 Hz, 1H), 7.15 (d, *J* = 3.6 Hz, 2H), 7.10 (dd, *J* = 7.2, 5.7 Hz, 1H), 7.02 - 6.93 (m, 1H), 6.42 (d, *J*= 7.6 Hz, 1H), 5.20 (dd, *J*= 12.9, 5.4 Hz, 1H), 4.64 (s, 2H), 3.46 (brs, 2H), 3.39 (brs, 2H), 3.22 (brs, 4H), 2.99 - 2.83 (m, 1H), 2.64 (d, *J* = 2.7 Hz, 1H), 2.55 (dd, *J*= 13.5, 4.6 Hz, 1H), 2.32 (s, 3H), 2.23 (s, 3H), 2.12 - 2.05 (m, 1H). LCMS (ESI) calcd for $C_{32}H_{33}N_4O_4S^+$ [M+H]$^+$: 569.22, found, 569.3.

Example 315: Preparation of 5-((6,7-dihydrothieno[3,2-c]pyridin-5(4H)-yl)methyl)-2-(2,6-dioxopiperidin-3-yl)isoindoline-1,3-dione (GT-06762)

Referring to the method of Scheme 1, the target compound (GT-06762) was prepared under appropriate conditions as would be understood by those skilled in the art (white solid, 40 mg, yield 63%). $^1$H NMR (400 MHz, DMSO-d6) δ 11.62 (s, 1H), 11.15 (s, 1H), 8.30 (s, 1H), 8.19 (d, *J* = 6.9 Hz, 1H), 8.05 (d, *J*= 7.6 Hz, 1H), 7.45 (d, *J*= 5.1 Hz, 1H), 6.88 (d, *J*= 5.2 Hz, 1H), 5.20 (dd, *J*= 12.9, 5.3 Hz, 1H), 4.70 (dd, *J*= 46.0, 13.1 Hz, 2H), 4.21 (brs, 2H), 3.78 - 3.75 (m, 1H), 3.30 - 3.26 (m, 2H), 3.19 - 3.07 (m, 1H), 2.99 - 2.83 (m, 1H), 2.62 (d, *J* = 17.6 Hz, 1H), 2.55 (dd, *J* = 13.2, 4.4 Hz, 1H), 2.19 - 1.96 (m, 1H). LCMS (ESI) calcd for $C_{21}H_{20}N_3O_4S^+$ [M+H]$^+$: 410.12, found, 410.2.

Example 316: Preparation of 5-((4-(benzo[d]isothiazol-3-yl)piperazin-1-yl)methyl)-2-(2,6-dioxopiperidin-3-yl)isoindoline-1,3-dione (GT-06763)

Referring to the method of Scheme 1, the target compound (GT-06763) was prepared under appropriate conditions as would be understood by those skilled in the art (white solid, 46 mg, yield 62%). $^1$H NMR (400 MHz, DMSO-d6) δ 11.59 (s, 1H), 11.16 (s, 1H), 8.28 (s, 1H), 8.17 - 8.05 (m, 4H), 7.59 (t *J* = 11.2 Hz, 1H), 7.46 (t, *J* = 11.2 Hz, 1H), 5.20 (dd, *J* = 12.9, 5.4 Hz, 1H), 4.65 (s, 2H), 4.08 (d, *J* = 12.9 Hz, 2H), 3.60 - 3.42 (m, 4H), 3.28 - 3,24 (m, 2H), 2.99 - 2.83 (m, 1H), 2.62 (d, *J* = 17.5 Hz, 1H), 2.55 (dd, *J*= 13.6, 4.6 Hz, 1H), 2.17 - 1.96 (m, 1H). LCMS (ESI) calcd for $C_{25}H_{24}N_5O_4S^+$ [M+H]$^+$: 490.15, found, 490.2.

Example 317: Preparation of 5-((4-(4-((3r,5r,7r)-adamantan-1-yl)benzyl)piperazin-1-yl)methyl)-2-(2,6-dioxopiperidin-3-yl)isoindoline-1,3-dione (GT-06810)

Referring to the method of Scheme 1, the target compound (GT-06810) was prepared under appropriate conditions as would be understood by those skilled in the art (white solid, 15 mg, yield 21%). $^1$H NMR (400 MHz, DMSO-d6) δ 11.14 (s, 1H), 8.00 (s, 1H), 7.98 - 7.95 (m, 2H), 7.50 (d, *J*= 6.7 Hz, 2H), 7.43 (d, *J* = 8.0 Hz, 2H), 5.16 (dd, *J* = 12.9, 5.4 Hz, 1H), 4.28 (brs, 2H), 3.68 - 3.62 (m, 4H), 3.42 - 3.29 (m, 2H), 3.20 - 3.05 (m, 3H), 2.94 - 2.87 (m, 2H), 2.61 (d, *J*= 17.9 Hz, 1H), 2.57 - 2.52 (m, 1H), 2.06 (s, 4H), 1.86 (s, 6H), 1.74 (s, 6H). LCMS (ESI) calcd for $C_{35}H_{41}N_4O_4^+$ [M+H]$^+$: 581.31, found, 581.3.

Example 318: Preparation of 5-((((3s,5s,7s)-adamantan-1-yl)amino)methyl)-2-(2,6-dioxopiperidin-3-yl)isoindoline-1,3-dione (GT-06912)

Referring to the method of Scheme 1, the target compound (GT-06912) was prepared under appropriate conditions as would be understood by those skilled in the art (white solid, 17 mg, yield 26%). $^1$H NMR (400 MHz, DMSO-d6) δ 11.14 (s, 1H), 9.20 (s, 2H), 8.20 (s, 1H), 8.09 (d, *J* = 7.8 Hz, 1H), 8.01 (d, *J*= 7.7 Hz, 1H), 5.18 (dd, *J*= 13.0, 5.4 Hz, 1H), 4.34 (s, 2H), 2.95 - 2.86 (m, 1H), 2.62 (d, *J*= 16.0 Hz, 1H), 2.56 (dd, *J*= 13.5, 4.5 Hz, 1H), 2.17 (s, 3H), 2.14 - 2.05 (m, 1H), 1.99 (s, 6H), 1.72 - 1.61 (m, 6H). LCMS (ESI) calcd for $C_{24}H_{28}N_3O_4^+$ [M+H]$^+$: 422.21, found, 422.3.

Example 319: Preparation of 1-((1R,4R)-7,7-dimethyl-2-oxobicyclo[2.2.1]heptan-1-yl)-N-((2-(2,6-dioxopiperidin-3-yl)-1,3-dioxoisoindolin-5-yl)methyl)methanesulfonamide (GT-07997)

Referring to the method of Scheme 1, the target compound (GT-07997) was prepared under appropriate conditions as would be understood by those skilled in the art (white solid, 35 mg, yield 70%). $^1$H NMR (400 MHz, DMSO-d6) δ 11.12 (s, 1H), 7.94 - 7.90 (m, 2H), 7.86 - 7.81 (m, 2H), 5.16 (dd, *J* = 12.9, 5.3 Hz, 1H), 4.43 (d, *J*= 6.3 Hz, 2H), 3.35 (d, *J* = 14.9 Hz, 1H), 2.95 (d, *J* = 14.9 Hz, 1H), 2.92 - 2.82 (m, 1H), 2.61 (d, *J*= 16.6 Hz, 1H), 2.57 - 2.52 (m, 1H), 2.41 - 2.29 (m, 2H), 2.13 - 2.03 (m, 2H), 2.00 - 1.88 (m, 2H), 1.61 - 1.52 (m, 1H), 1.46 - 1.34 (m, 1H), 1.01 (s, 3H), 0.78 (s, 3H). LCMS (ESI) calcd

for $C_{24}H_{28}N_3O_7S^+$ [M+H]+: 502.16, found, 502.1.

Example 320: Preparation of 2-(2,6-dioxopiperidin-3-yl)-5-((4-(4-methylthiophen-3-yl)piperazin-1-yl)methyl)isoindoline-1,3-dione (GT-06260)

Referring to the method of Scheme 1, the target compound (GT-06260) was prepared under appropriate conditions as would be understood by those skilled in the art (white solid, 30 mg, yield 54%). $^1$HNMR(400 MHz, DMSO-d6) δ 11.31 (s, 1H), 11.15 (s, 1H), 8.27 (s, 1H), 8.16 (d, *J* = 7.7 Hz, 1H), 8.05 (d, *J*= 7.7 Hz, 1H), 7.13 (d, *J*= 2.2 Hz, 1H), 6.81 (d, *J*= 3.2 Hz, 1H), 5.19 -5.15 (m, *J* = 12.9, 5.3 Hz, 1H), 4.61 (s, 2H), 3.38 - 3.19 (m, 6H), 3.07 - 2.85 (m, 3H), 2.59-2.43 (m, 2H), 2.14 - 2.04 (m, 4H). LCMS (ESI) calcd for $C_{23}H_{25}N_4O_4S^+$ [M+H]+: 453.16, found, 453.2.

Example 321: Preparation of 2-(2,6-dioxopiperidin-3-yl)-5-((4-(thiophen-3-yl)-3,6-dihydropyridin-1(2H)-yl)methyl)isoindoline-1,3-dione (GT-06261)

Referring to the method of Scheme 1, the target compound (GT-06261) was prepared under appropriate conditions as would be understood by those skilled in the art (white solid, 34 mg, yield 63%). $^1$H NMR (400 MHz, DMSO-d6) δ 11.15 (s, 1H), 10.85 (s, 1H), 8.34 - 7.94 (m, 3H), 7.59 - 7.52 (m, 2H), 7.37-7.12 (m, 1H), 6.15 (s, 1H), 5.19 -4.90(m, 1H), 4.62 (d, *J*= 13.4 Hz, 2H), 3.70 (d, *J*= 62.8 Hz, 3H), 3.37 (d, *J* = 4.1 Hz, 1H), 3.02 - 2.53 (m, 5H), 2.07-1.80 (m, 1H). LCMS (ESI) calcd for $C_{23}H_{22}N_3O_4S^+$ [M+H]+: 436.13, found, 436.2.

Example 322: Preparation of 2-(2,6-dioxopiperidin-3-yl)-5-((4-(5-methylthiophen-2-yl)piperidin-1-yl)methyl)isoindoline-1,3-dione (GT-06262)

Referring to the method of Scheme 1, the target compound (GT-06262) was prepared under appropriate conditions as would be understood by those skilled in the art (light yellow solid, 24 mg, yield 44%). $^1$H NMR (400 MHz, DMSO-d6) δ 11.15 (s, 1H), 10.91 (s, 1H), 8.24 (s, 1H), 8.18 - 7.99 (m, 2H), 6.72 - 6.58 (m, 2H), 5.18 (s, 1H), 4.52 (s, 2H), 3.40 (s, 2H), 3.06 (d, *J*= 11.6 Hz, 4H), 2.71 - 2.55 (m, 2H), 2.38 (s, 3H), 2.14 - 1.88 (m, 5H). LCMS (ESI) calcd for $C_{24}H_{26}N_3O_4S^+$ [M+H]+: 452.16, found, 452.2.

Example 323: Preparation of 5-((4-(2,3-dichlorophenyl)piperazin-1-yl)methyl)-2-(2,6-dioxopiperidin-3-yl)isoindoline-1,3-dione (GT-06273)

Referring to the method of Scheme 1, the target compound (GT-06273) was prepared under appropriate conditions as would be understood by those skilled in the art (white solid, 35 mg, yield 57%). $^1$H NMR (400 MHz, DMSO-d6) δ 11.15 (s, 2H), 8.06 (s, 3H), 7.36 (d, *J*= 7.2 Hz, 2H), 7.20 - 7.24 (m, *J* = 7.3, 2.1 Hz, 1H), 5.26 - 5.11 (m, 1H), 4.63 (s, 2H), 3.44 (s, 6H), 3.15 (s, 2H), 3.00 - 2.84 (m, 1H), 2.59-2.23 (m, 2H), 2.13 - 2.01 (m, 1H). LCMS (ESI) calcd for $C_{24}H_{23}Cl_2N_4O_4^+$ [M+H]+: 501.11, found, 501.1.

Example 324: Preparation of 5-((4-(2-chlorophenyl)piperazin-1-yl)methyl)-2-(2,6-dioxopiperidin-3-yl)isoindoline-1,3-dione (GT-06274)

Referring to the method of Scheme 1, the target compound (GT-06274) was prepared under appropriate conditions as would be understood by those skilled in the art (white solid, 34 mg, yield 59%). $^1$H NMR (400 MHz, DMSO-d6) δ 11.15 (s, 2H), 8.26 (s, 1H), 8.18 - 8.01 (m, 2H), 7.45 (d, *J* = 7.7 Hz, 1H), 7.34 (t, *J* = 7.3 Hz, 1H), 7.20 (d, *J* = 7.6 Hz, 1H), 7.11 (t, *J* = 7.5 Hz, 1H), 5.19-4.82 (m, 1H), 3.42 (d,*J* = 12.4 Hz, 6H), 3.30 - 3.23 (m, 2H), 3.14 (s, 2H), 2.89 (d, *J* = 12.2 Hz, 1H), 2.59-2.20 (m, 2H), 2.13 - 2.03 (m, 1H). LCMS (ESI) calcd for $C_{24}H_{24}ClN_4O_4^+$ [M+H]+: 467.14, found, 467.1.

Example 325: Preparation of 5-((4-(4-chlorophenyl)piperazin-1-yl)methyl)-2-(2,6-dioxopiperidin-3-yl)isoindoline-1,3-dione (GT-06275)

Referring to the method of Scheme 1, the target compound (GT-06275) was prepared under appropriate conditions as would be understood by those skilled in the art (white solid, 43 mg, yield 75%). $^1$H NMR (400 MHz, DMSO-d6) δ 11.37 (s, 1H), 11.16 (s, 1H), 8.25 (s, 1H), 8.13 (d, *J* = 7.8 Hz, 1H), 8.05 (d, *J* = 7.6 Hz, 1H), 7.29 (d, *J* = 8.9 Hz, 2H), 7.00 (d, *J* = 9.0 Hz, 2H), 5.19- 5.16 (m, *J* = 12.9, 5.3 Hz, 1H), 4.59 (s, 2H), 3.82 (s, 2H), 3.39 (s, 1H), 3.18 (s, 4H), 2.91 (s, 1H), 2.59-2.23 (m, 2H), 2.08 (s, 1H). LCMS (ESI) calcd for $C_{24}H_{24}ClN_4O_4^+$ [M+H]+: 467.15, found, 467.1.

Example 326: Preparation of 2-(2,6-dioxopiperidin-3-yl)-5-((4-(m-tolyl)piperazin-1-yl)methyl)isoindoline-1,3-dione (GT-06276)

Referring to the method of Scheme 1, the target compound (GT-06276) was prepared under appropriate conditions as would be understood by those skilled in the art (white solid, 47 mg, yield 85%). $^1$H NMR (400 MHz, DMSO-d6) δ 11.35 (s, 1H), 11.16 (s, 1H), 8.27 (s, 1H), 8.15 (d, *J* = 7.7 Hz, 1H), 8.06 (d, *J* = 7.6 Hz, 1H), 7.14 (t, *J* = 7.8 Hz, 1H), 6.82 - 6.74 (m, 2H), 6.69 (d, *J* = 7.4 Hz, 1H), 5.25 - 5.15 (m, 1H), 4.60 (s, 2H), 3.80 (d, *J* = 10.4 Hz, 2H), 3.39 (s, 2H), 3.15 (d, *J* = 9.8 Hz, 4H), 2.97 - 2.86 (m, 1H), 2.68 - 2.55 (m, 2H), 2.26 (s, 3H), 2.13 - 2.03 (m, 1H). LCMS (ESI) calcd for $C_{25}H_{27}N_4O_4^+$ [M+H]$^+$: 447.20, found, 447.3.

Example 327: Preparation of 2-(2,6-dioxopiperidin-3-yl)-5-((4-(p-tolyl)piperazin-1-yl)methyl)isoindoline-1,3-dione (GT-06277)

Referring to the method of Scheme 1, the target compound (GT-06277) was prepared under appropriate conditions as would be understood by those skilled in the art (white solid, 33 mg, yield 61%). $^1$H NMR (400 MHz, DMSO-d6) δ 11.20 (s, 1H), 11.16 (s, 1H), 8.26 (s, 1H), 8.13 (d, *J* = 7.7 Hz, 1H), 8.06 (d, *J*= 7.7 Hz, 1H), 7.08 (d, *J* = 8.4 Hz, 2H), 6.88 (d, *J*= 8.4 Hz, 2H), 5.20-4.82 (m, 1H), 4.60 (s, 2H), 3.74 (d, *J* = 12.8 Hz, 2H), 3.59 (s, 1H), 3.41 (d, *J* = 10.9 Hz, 3H), 3.10 (d, *J*= 12.3 Hz, 2H), 2.97 - 2.83 (m, 1H), 2.59-2.21 (m, 2H), 2.22 (s, 3H), 2.14 - 2.04 (m, 1H). LCMS (ESI) calcd for $C_{25}H_{27}N_4O_4^+$ [M+H]$^+$: 447.20, found, 447.3.

Example 328: Preparation of 5-((4-(2,4-dichlorophenyl)piperazin-1-yl)methyl)-2-(2,6-dioxopiperidin-3-yl)isoindoline-1,3-dione (GT-06355)

Referring to the method of Scheme 1, the target compound (GT-06355) was prepared under appropriate conditions as would be understood by those skilled in the art (white solid, 35 mg, yield 58%). $^1$H NMR (400 MHz, DMSO-d6) δ 11.05 (d, *J* = 22.6 Hz, 2H), 8.18 (s, 1H), 8.02 (d, *J* = 21.9 Hz, 2H), 7.53 (s, 1H), 7.34-7.11 (m, 1H), 7.15 (d, *J* = 8.7 Hz, 1H), 5.12 -4.81(m, 1H), 4.56 (s, 2H), 3.34 (d, *J* = 14.8 Hz, 4H), 3.24 - 3.17 (m, 2H), 3.06 (s, 2H), 2.85 - 2.77 (m, 1H), 2.52 -2.21(m, 2H), 2.07 - 1.94 (m, 1H). LCMS (ESI) calcd for $C_{24}H_{23}Cl_2N_4O_4^+$ [M+H]$^+$: 501.11, found, 501.1.

Example 329: Preparation of 5-((4-(2,5-dichlorophenyl)piperazin-1-yl)methyl)-2-(2,6-dioxopiperidin-3-yl)isoindoline-1,3-dione (GT-06356)

Referring to the method of Scheme 1, the target compound (GT-06356) was prepared under appropriate conditions as would be understood by those skilled in the art (white solid, 34 mg, yield 55%). $^1$H NMR (400 MHz, DMSO-d6) δ 11.08 (s, 2H), 8.18 (s, 1H), 8.10 - 7.94 (m, 2H), 7.41 (d, *J*= 8.5 Hz, 1H), 7.25 - 7.01 (m, 2H), 5.12 -4.81(m, 1H), 4.56 (s, 2H), 3.38 (s, 3H), 3.29 (s, 1H), 3.11 (d, *J* = 11.2 Hz, 4H), 2.82-2.65 (m, 1H), 2.62 - 2.45 (m, 2H), 2.05 - 1.96 (m, 1H). LCMS (ESI) calcd for $C_{24}H_{23}Cl_2N_4O_4^+$ [M+H]$^+$: 501.11, found, 501.1.

Example 330: Preparation of 2-(2,6-dioxopiperidin-3-yl)-5-((4-(3-(trifluoromethyl)phenyl)piperidin-1-yl)methyl)isoindoline-1,3-dione (GT-06357)

Referring to the method of Scheme 1, the target compound (GT-06357) was prepared under appropriate conditions as would be understood by those skilled in the art (white solid, 40 mg, yield 65%). $^1$H NMR (400 MHz, DMSO-d6) δ 11.13 (s, 1H), 8.26 (s, 1H), 8.10- 8.06 (m, 2H), 7.74 (t, *J*= 8.2 Hz, 2H), 7.57 (d, *J*= 7.7 Hz, 1H), 7.48 (t, *J*= 7.7 Hz, 1H), 5.21-5.18 (m, 1H), 4.56 (s, 2H), 3.49 (d, *J* = 10.9 Hz, 2H), 3.24 - 3.07 (m, 3H), 2.95 - 2.87 (m, 1H), 2.69 - 2.55 (m, 2H), 2.26 (d, *J*= 12.3 Hz, 2H), 2.10 -1.94(m, 1H), 1.90 (d, *J* = 13.2 Hz, 2H). LCMS (ESI) calcd for $C_{26}H_{25}F_3N_3O_4^+$ [M+H]$^+$: 500.18, found, 500.3.

Example 331: Preparation of 5-((4-(2,3-dichlorophenyl)piperidin-1-yl)methyl)-2-(2,6-dioxopiperidin-3-yl)isoindoline-1,3-dione (GT-06358)

Referring to the method of Scheme 1, the target compound (GT-06358) was prepared under appropriate conditions as would be understood by those skilled in the art (white solid, 41 mg, yield 67%). $^1$H NMR (400 MHz, DMSO-d6) δ 11.17 (s, 1H), 10.97 (s, 1H), 8.26 (s, 1H), 8.10 (d, *J* = 18.7 Hz, 2H), 7.58 (d, *J* = 7.8 Hz, 1H), 7.47 - 7.28 (m, 2H), 5.21-4.91(m, 1H), 3.58 - 3.40 (m, 3H), 3.26 - 3.12 (m, 2H), 2.99 - 2.85 (m, 1H), 2.71 - 2.55 (m, 2H), 2.08-1.83 (m, 5H), 1.31 - 1.26 (m, 2H). LCMS (ESI) calcd for $C_{25}H_{24}Cl_2N_3O_4^+$ [M+H]$^+$: 500.11, found, 500.1.

Example 332: Preparation of 5-((4-(2,3-difluorophenyl)piperidin-1-yl)methyl)-2-(2,6-dioxopiperidin-3-yl)isoindoline-1,3-dione (GT-06359)

Referring to the method of Scheme 1, the target compound (GT-06359) was prepared under appropriate conditions as would be understood by those skilled in the art (white solid, 40 mg, yield 69%). $^1$H NMR (400 MHz, DMSO-d6) δ 11.16 (s, 1H), 10.85 (s, 1H), 8.24 (s, 1H), 8.09 (dd, *J* = 25.1, 7.6 Hz, 2H), 7.38 - 7.28 (m, 1H), 7.27 - 7.18 (m, 1H), 7.10 (t, *J*= 7.0 Hz, 1H), 5.20 -4.97(m, 1H), 3.48 (d, *J*= 10.5 Hz, 2H), 3.38 (s, 1H), 3.33 (s, 1H), 3.15 (d, *J* = 10.7 Hz, 3H), 2.90-2.71 (m, 1H), 2.67 - 2.53 (m, 2H), 2.05- 2.00 (m, 5H). LCMS (ESI) calcd for $C_{25}H_{24}F_2N_3O_4{}^+$[M+H]$^+$: 468.17, found, 468.2.

Example 333: Preparation of 5-((4-(2-chloro-3-fluorophenyl)piperidin-1-yl)methyl)-2-(2,6-dioxopiperidin-3-yl)isoindoline-1,3-dione (GT-06360)

Referring to the method of Scheme 1, the target compound (GT-06360) was prepared under appropriate conditions as would be understood by those skilled in the art (white solid, 42 mg, yield 71%). $^1$H NMR (400 MHz, DMSO-d6) δ 11.16 (s, 1H), 10.94 (s, 1H), 8.24 (s, 1H), 8.09- 8.01 (m, 2H), 7.49 - 7.39 (m, 1H), 7.33 (t, *J* = 8.2 Hz, 1H), 7.17 (d, *J* = 7.8 Hz, 1H), 5.20-4.96 (m, 1H), 4.55 (s, 2H), 3.49 (d, *J* = 11.1 Hz, 2H), 3.42 - 3.37 (m, 1H), 3.32 (s, 1H), 3.30 - 3.12 (m, 3H), 2.91-2.73 (m, 1H), 2.58- 2.54 (m, 2H), 2.20 - 1.84 (m, 5H). LCMS (ESI) calcd for $C_{25}H_{24}ClFN_3O_4{}^+$ [M+H]$^+$: 484.14, found, 484.2.

Example 334: Preparation of 5-((4-(2-chloro-3-fluorophenyl)-3,6-dihydropyridin-1(2H)-yl)methyl)-2-(2,6-dioxopiperidin-3-yl)isoindoline-1,3-dione (GT-06429)

Referring to the method of Scheme 1, the target compound (GT-06429) was prepared under appropriate conditions as would be understood by those skilled in the art (white solid, 33 mg, yield 56%). $^1$H NMR (400 MHz, DMSO-d6) δ 11.06 (s, 1H), 8.33 - 7.90 (m, 3H), 7.46 - 7.24 (m, 2H), 7.19 - 6.97 (m, 1H), 5.72 (s, 1H), 5.13-4.89 (m, 1H), 4.59 (s, 2H), 3.92 - 3.40 (m, 3H), 3.24 - 3.20 (m, 1H), 2.95 - 2.72 (m, 2H), 2.50-2.32 (m, 3H), 2.07 - 1.96 (m, 1H). LCMS (ESI) calcd for $C_{25}H_{23}ClFN_3O_4{}^+$[M+H]$^+$: 482.13, found, 482.1.

Example 335: Preparation of 2-(2,6-dioxopiperidin-3-yl)-5-((4-(pyridin-2-yl)piperazin-1-yl)methyl)isoindoline-1,3-dione (GT-06521)

Referring to the method of Scheme 1, the target compound (GT-06521) was prepared under appropriate conditions as would be understood by those skilled in the art (white solid, 27 mg, yield 69%). $^1$NMR (400 MHz, DMSO-d6) δ 11.16 (s,1H), 8.25 (s,1H), 8.13 (dd, *J*= 11.2, 6.0 Hz,2H), 8.05 (d, *J*= 7.6 Hz,1H), 7.79 (t, *J* = 7.6 Hz,1H), 7.11 (d, *J* = 8.8 Hz,1H), 6.95 - 6.79 (m,1H), 5.20 -5.03(m, 1H), 4.58 (s, 2H), 4.41 (s, 2H), 3.60 (s, 2H), 3.17 (s, 4H), 2.93 - 2.84 (m,1H), 2.98-2.56 (m,2H), 2.14 - 2.04 (m, 1H). LCMS (ESI) calcd for $C_{23}H_{24}N_5O_4{}^+$[M+H]$^+$: 434.18, found, 434.2.

Example 336: Preparation of 2-(2,6-dioxopiperidin-3-yl)-5-((4-(6-methylpyridin-2-yl)piperazin-1-yl)methyl)isoindoline-1,3-dione (GT-06522)

Referring to the method of Scheme 1, the target compound (GT-06522) was prepared under appropriate conditions as would be understood by those skilled in the art (white solid, 27 mg, yield 67%). $^1$NMR (400 MHz, DMSO-d6) δ 11.35 (s, 2H), 8.25 (s,1H), 8.20-8.09 (m, 2H), 7.68 (s, 1H), 7.09 - 6.55 (m, 2H), 5.20-5.09 (m, 1H), 4.58 (s, 2H), 4.40 (s, 2H), 3.67 (s, 2H), 3.15 (s, 3H), 2.89 (d, *J* = 12.0 Hz, 1H), 2.67 - 2.53 (m, 3H), 2.42 (s, 3H), 2.15 - 2.01 (m, 1H). LCMS (ESI) calcd for $C_{24}H_{26}N_5O_4{}^+$[M+H]$^+$: 448.20, found, 448.2.

Example 337: Preparation of 5-((4-(6-chloropyridin-2-yl)piperazin-1-yl)methyl)-2-(2,6-dioxopiperidin-3-yl)isoindoline-1,3-dione (GT-06523)

Referring to the method of Scheme 1, the target compound (GT-06523) was prepared under appropriate conditions as would be understood by those skilled in the art (light yellow solid, 30 mg, yield 70%). $^1$NMR (400 MHz, DMSO-d6) δ 11.10 (s, 1H), 8.20 (s, 1H), 8.06 (s, 2H), 7.65 (t, *J* = 7.9 Hz, 1H), 6.90 (d, *J* = 8.3 Hz, 1H), 6.79 (d, *J* = 7.5 Hz, 1H), 5.35-5.23 (m, 1H), 4.57 (s, 2H), 4.34 (s, 2H), 3.56 (s, 2H), 3.13 (s, 3H), 2.93 - 2.84 (m, 1H), 2.65 (s, 1H), 2.60 (s, 1H), 2.58 - 2.54 (m, 1H), 2.15 - 2.00 (m, 1H). LCMS (ESI) calcd for $C_{23}H_{23}ClN_5O_4{}^+$[M+H]$^+$: 468.14, found, 468.2.

Example 338: Preparation of 5-((4-(5-chloropyridin-2-yl)piperazin-1-yl)methyl)-2-(2,6-dioxopiperidin-3-yl)isoindoline-1,3-dione (GT-06524)

Referring to the method of Scheme 1, the target compound (GT-06524) was prepared under appropriate

conditions as would be understood by those skilled in the art (white solid, 32 mg, yield 74%). [1]NMR (400 MHz, DMSO-d6) δ 11.24 (s, 2H), 8.23 (s, 1H), 8.18 (d, *J*= 2.6 Hz, 1H), 8.11 (d, *J*= 7.7 Hz, 1H), 8.05 (d, *J*= 7.6 Hz, 1H), 7.77-7.65 (m, 1H), 6.99 (d, *J* = 9.1 Hz, 1H), 5.28-5.12 (m, 1H), 4.57 (s, 2H), 4.35 (d, *J* = 12.9 Hz, 2H), 3.31 (s, 3H), 3.12 (s, 2H), 2.97 - 2.83 (m, 2H), 2.64 (s, 1H), 2.60 (s, 1H), 2.57 (d, *J*= 4.4 Hz, 1H), 2.16 - 2.01 (m, 1H). LCMS (ESI) calcd for $C_{23}H_{23}ClN_5O_4^+[M+H]^+$: 468.14, found, 468.2.

Example 339: Preparation of 5-((4-(4-chloropyridin-2-yl)piperazin-1-yl)methyl)-2-(2,6-dioxopiperidin-3-yl)isoindoline-1,3-dione (GT-06525)

Referring to the method of Scheme 1, the target compound (GT-06525) was prepared under appropriate conditions as would be understood by those skilled in the art (light yellow solid, 30 mg, yield 70%). $^1$H NMR (400 MHz, DMSO-d6) δ 11.10 (s, 1H), 8.10 (d, *J*= 22.6 Hz, 3H), 7.99 - 7.77 (m, 1H), 7.07 (s, 1H), 6.85 (s, 1H), 5.28-5.15 (m, 1H), 4.52 (d, *J*= 52.9 Hz, 3H), 3.78 (s, 1H), 3.66-3.54 (m, 1H), 3.40 (s, 2H), 3.21-3.10 (m, 2H), 2.95 - 2.86 (m, 1H), 2.68 (d, *J* = 13.5 Hz, 1H), 2.62 (s, 1H), 2.58 (d, *J*= 4.4 Hz, 1H), 2.15 - 2.05 (m, 1H). LCMS (ESI) calcd for $C_{23}H_{23}ClN_5O_4^+[M+H]^+$: 468.14, found, 468.2.

Example 340: Preparation of 2-(2,6-dioxopiperidin-3-yl)-5-((4-(3-fluoropyridin-2-yl)piperazin-1-yl)methyl)isoindoline-1,3-dione (GT-06526)

Referring to the method of Scheme 1, the target compound (GT-06526) was prepared under appropriate conditions as would be understood by those skilled in the art (white solid, 33 mg, yield 60%). $^1$H NMR (400 MHz, DMSO-d6) δ 11.56 (s, 1H), 11.16 (s, 1H), 8.27 (s, 1H), 8.14 (d, *J*= 7.7 Hz, 1H), 8.13-8.01 (m, 2H), 7.69 - 7.54 (m, 1H), 7.08-6.96 (m, 1H), 5.23-5.16 (m, 1H), 4.59 (s, 2H), 4.05 (d, *J*= 13.0 Hz, 2H), 3.46 - 3.33 (m, 4H), 3.23 (s, 2H), 2.98 - 2.84 (m, 1H), 2.62 (d, *J* = 17.9 Hz, 1H), 2.58 - 2.52 (m, 1H), 2.14-2.03 (m, 1H). LCMS (ESI) calcd for $C_{23}H_{23}FN_5O_4^+[M+H]^+$: 452.17, found, 452.2.

Example 341: Preparation of 2-(2,6-dioxopiperidin-3-yl)-5-((4-(6-fluoropyridin-2-yl)piperazin-1-yl)methyl)isoindoline-1,3-dione (GT-06527)

Referring to the method of Scheme 1, the target compound (GT-06527) was prepared under appropriate conditions as would be understood by those skilled in the art (white solid, 19 mg, yield 35%). $^1$H NMR (400 MHz, DMSO-d6) δ 11.16 (s, 2H), 8.21 (s, 1H), 8.06 (t, *J* = 6.1 Hz, 2H), 7.77 (q, *J* = 8.3 Hz, 1H), 6.80 (d, *J* = 6.7 Hz, 1H), 6.41 (d, *J* = 5.4 Hz, 1H), 5.25-5.12 (m, 1H), 4.58 (s, 2H), 4.32 (s, 2H), 3.50 - 3.39 (m, 2H), 3.33 - 3.28 (m, 2H), 3.12 (s, 2H), 2.93 - 2.84 (m, 1H), 2.62 (d, *J* = 18.2 Hz, 1H), 2.58 - 2.53 (m, 1H), 2.14 - 2.02 (m, 1H). LCMS (ESI) calcd for $C_{23}H_{23}FN_5O_4^+[M+H]^+$: 452.17, found, 452.2.

Example 342: Preparation of 2-(2,6-dioxopiperidin-3-yl)-5-((4-(5-fluoropyridin-2-yl)piperazin-1-yl)methyl)isoindoline-1,3-dione (GT-06528)

Referring to the method of Scheme 1, the target compound (GT-06528) was prepared under appropriate conditions as would be understood by those skilled in the art (white solid, 36 mg, yield 67%). $^1$H NMR (400 MHz, DMSO-d6) δ 11.66 (s, 1H), 11.16 (s, 1H), 8.27 (s, 1H), 8.15 (t, *J* = 5.5 Hz, 2H), 8.04 (d, *J*= 7.6 Hz, 1H), 7.67-7.54 (m, 1H), 7.07-6.96 (m, 1H), 5.23-5.16(m, 1H), 4.58 (s, 2H), 4.29 (d, *J*= 13.5 Hz, 2H), 3.40 (d, *J* = 11.2 Hz, 2H), 3.32 (t, *J* = 12.7 Hz, 2H), 3.12 (s, 2H), 2.97 - 2.85 (m, 1H), 2.62 (d, *J* = 17.7 Hz, 1H), 2.58 - 2.52 (m, 1H), 2.14 - 1.97 (m, 1H). LCMS (ESI) calcd for $C_{23}H_{23}FN_5O_4^+[M+H]^+$: 452.17, found, 452.2.

Example 343: Preparation of 2-(2,6-dioxopiperidin-3-yl)-5-((4-(3-(trifluoromethyl)pyridin-2-yl)piperazin-1-yl)methyl)isoindoline-1,3-dione (GT-06529)

Referring to the method of Scheme 1, the target compound (GT-06529) was prepared under appropriate conditions as would be understood by those skilled in the art (white solid, 35 mg, yield 57%). NMR (400 MHz, DMSO-d6) δ 11.68 (s, 1H), 11.16 (s, 1H), 8.60 (d, *J* = 4.1 Hz, 1H), 8.31 (s, 1H), 8.19 (d, *J*= 7.7 Hz, 1H), 8.15 (d, *J*= 7.9 Hz, 1H), 8.04 (d, *J* = 7.6 Hz, 1H), 7.33 (d, *J*= 2.9 Hz, 1H), 5.19 (d, *J*= 7.5 Hz ,1H), 4.62 (s, 2H), 3.51 (s, 3H), 3.45 (s, 3H), 3.23 (s, 2H), 2.89 (d, *J* = 11.8 Hz, 1H), 2.66 - 2.55 (m, 2H), 2.09 (d, *J* = 5.3 Hz, 1H). LCMS (ESI) calcd for $C_{24}H_{23}F_3N_5O_4^+[M+H]^+$: 502.17, found, 502.2.

Example 344: Preparation of 2-(2,6-dioxopiperidin-3-yl)-5-((4-(6-(trifluoromethyl)pyridin-2-yl)piperazin-1-yl)methyl)isoindoline-1,3-dione (GT-06530)

Referring to the method of Scheme 1, the target compound (GT-06530) was prepared under appropriate conditions as would be understood by those skilled in the art (white solid, 32 mg, yield 52%). $^1$NMR (400 MHz, DMSO-d6) δ 11.33 (s, 1H), 11.09 (s, 1H), 8.16 (s, 1H), 7.99 (t, *J*= 10.6 Hz, 2H), 7.78 (t, *J* = 7.9 Hz, 1H), 7.16-7.06 (m, 2H), 5.16-5.08 (m, 1H), 4.43 (d, *J* = 62.3 Hz, 4H), 3.35 (s, 2H), 3.27 - 3.21 (m, 2H), 3.06 (s, 2H), 2.86 - 2.76 (m, 1H), 2.57-2.44 (m, 2H), 2.00 (d, *J* = 5.1 Hz, 1H). LCMS (ESI) calcd for $C_{24}H_{23}F_3N_5O_4^+[M+H]^+$: 502.17, found, 502.2.

Example 345: Preparation of 2-(2,6-dioxopiperidin-3-yl)-5-((4-(5-(trifluoromethyl)pyridin-2-yl)piperazin-1-yl)methyl)isoindoline-1,3-dione (GT-06531)

Referring to the method of Scheme 1, the target compound (GT-06531) was prepared under appropriate conditions as would be understood by those skilled in the art (white solid, 45 mg, yield 74%). $^1$NMR (400 MHz, DMSO-d6) δ 11.55 (s, 1H), 11.16 (s, 1H), 8.49 (s, 1H), 8.24 (s, 1H), 8.11 (d, *J* = 7.8 Hz, 1H), 8.05 (d, *J*= 7.6 Hz, 1H), 7.95-7.88 (m, 1H), 7.08 (d, *J* = 9.1 Hz, 1H), 5.23-5.16 (m, 1H), 4.57 (s, 4H), 3.43 (s, 4H), 3.12 (s, 2H), 2.89 (d, *J*= 11.9 Hz, 1H), 2.64-2.55 (m, 2H), 2.14 - 2.01 (m, 1H). LCMS (ESI) calcd for $C_{24}H_{23}F_3N_5O_4^+[M+H]^+$: 502.17, found, 502.2.

Example 346: Preparation of 5-((4-(3,5-dichloropyridin-2-yl)piperazin-1-yl)methyl)-2-(2,6-dioxopiperidin-3-yl)isoindoline-1,3-dione (GT-06532)

Referring to the method of Scheme 1, the target compound (GT-06532) was prepared under appropriate conditions as would be understood by those skilled in the art (white solid, 39 mg, yield 64%). $^1$NMR (400 MHz, DMSO-d6) δ 11.50 (s, 1H), 11.08 (s, 1H), 8.26 (d, *J*= 2.1 Hz, 1H), 8.19 (s, 1H), 8.06 (d, *J*= 1.9 Hz, 2H), 7.97 (d, *J*= 7.6 Hz, 1H), 5.16-5.08 (m, 1H), 4.53 (s, 2H), 3.77 (d, *J*= 12.7 Hz, 2H), 3.33 (s, 2H), 3.26 - 3.22 (m, 2H), 3.17 (s, 2H), 2.90 - 2.77 (m, 1H), 2.55-2.43 (m, 2H), 2.01 (s, 1H), 1.90(s, 1H). LCMS (ESI) calcd for $C_{23}H_{22}Cl_2N_5O_4^+[M+H]^+$: 502.10, found, 502.1.

Example 347: Preparation of 2-(2,6-dioxopiperidin-3-yl)-5-((4-(3-fluoro-4-iodopyridin-2-yl)piperazin-1-yl)methyl)isoindoline-1,3-dione (GT-06533)

Referring to the method of Scheme 1, the target compound (GT-06533) was prepared under appropriate conditions as would be understood by those skilled in the art (white solid, 47 mg, yield 67%). $^1$NMR (400 MHz, DMSO-d6) δ 11.55 (s, 1H), 11.07 (s, 1H), 8.18 (s, 1H), 8.05 (d, *J* = 8.5 Hz, 1H), 7.96 (d, *J* = 7.7 Hz, 1H), 7.64 (d, *J*= 5.1 Hz, 1H), 7.36-7.27 (m, 1H), 5.15-5.03 (m, 1H), 4.49 (s, 2H), 3.96 (d, *J*= 13.3 Hz, 2H), 3.39 - 3.23 (m, 4H), 3.12 (s, 2H), 2.89 - 2.72 (m, 1H), 2.53 (d, *J*= 17.8 Hz, 1H), 2.48 (d, *J* = 4.3 Hz, 1H), 2.02-1.94 (m, 1H). LCMS (ESI) calcd for $C_{23}H_{22}FIN_5O_4^+[M+H]^+$: 578.07, found, 578.1.

Example 348: Preparation of 5-((4-(2,3-dichloropyridin-4-yl)piperazin-1-yl)methyl)-2-(2,6-dioxopiperidin-3-yl)isoindoline-1,3-dione (GT-06534)

Referring to the method of Scheme 1, the target compound (GT-06534) was prepared under appropriate conditions as would be understood by those skilled in the art (white solid, 39 mg, yield 63%). $^1$NMR (400 MHz, DMSO-d6) δ 12.05 (s, 1H), 11.16 (s, 1H), 8.31 (s, 1H), 8.26-8.14 (m, 2H), 8.04 (d, *J*= 7.6 Hz, 1H), 7.21 (d, *J* = 5.5 Hz, 1H), 5.24-5.16 (m, 1H), 4.63 (s, 2H), 3.77 (s, 2H), 3.41 (d, *J* = 13.1 Hz, 3H), 3.28 (s, 2H), 2.93 (d, *J* = 13.8 Hz, 1H), 2.68 - 2.56 (m, 2H), 2.14 - 2.02 (m, 1H). LCMS (ESI) calcd for $C_{23}H_{22}Cl_2N_5O_4^+[M+H]^+$: 502.10, found, 502.2.

Example 349: Preparation of 5-((4-(2,3-difluoropyridin-4-yl)piperazin-1-yl)methyl)-2-(2,6-dioxopiperidin-3-yl)isoindoline-1,3-dione (GT-06535)

Referring to the method of Scheme 1, the target compound (GT-06535) was prepared under appropriate conditions as would be understood by those skilled in the art (white solid, 38 mg, yield 65%). $^1$NMR (400 MHz, DMSO-d6) δ 11.56 (s, 1H), 11.16 (s, 1H), 8.24 (s, 1H), 8.12 (d, *J* = 7.5 Hz, 1H), 8.06 (d, *J*= 7.6 Hz, 1H), 7.81 (d, *J*= 5.7 Hz, 1H), 7.05 (t, *J* = 6.0 Hz, 1H), 5.23-5.14 (m, 1H), 4.59 (s, 2H), 3.91 (s, 2H), 3.50 (s, 2H), 3.27 (s, 3H), 2.94-2.88 (m, 1H), 2.61 (t, *J* = 14.5 Hz, 2H), 2.54 (s, 1H), 2.08 (s, 1H). LCMS (ESI) calcd for $C_{23}H_{22}F_2N_5O_4^+[M+H]^+$: 470.16, found, 470.2.

Example 350: Preparation of 5-((4-(3-bromo-5-chloropyridin-4-yl)piperazin-1-yl)methyl)-2-(2,6-dioxopiperidin-3-yl)isoindoline-1,3-dione (GT-06599)

Referring to the method of Scheme 1, the target compound (GT-06599) was prepared under appropriate conditions as would be understood by those skilled in the art (white solid, 31 mg, yield 47%). $^1$NMR (400 MHz, DMSO-d6) δ 11.58 (s, 1H), 11.16 (s, 1H), 8.64 (s, 1H), 8.54 (s, 1H), 8.31 (s, 1H), 8.19 (d, *J* = 7.7 Hz, 1H), 8.06 (d, *J* = 7.7 Hz, 1H), 5.24-5.16 (m, 1H), 4.65 (s, 2H), 3.81 (s, 2H), 3.42 (d, *J* = 9.6 Hz, 4H), 3.25 (s, 2H), 2.91 (s, 1H), 2.66 - 2.55 (m, 2H), 2.15 - 2.01 (m, 1H). LCMS (ESI) calcd for $C_{23}H_{22}BrClN_5O_4^+$[M+H]$^+$: 546.05, found, 546.0.

Example 351: Preparation of 5-((4-(3-chloro-5-fluoropyridin-4-yl)piperazin-1-yl)methyl)-2-(2,6-dioxopiperidin-3-yl)isoindoline-1,3-dione (GT-06600)

Referring to the method of Scheme 1, the target compound (GT-06600) was prepared under appropriate conditions as would be understood by those skilled in the art (white solid, 29mg, yield 48%). $^1$NMR (400 MHz, DMSO-d6) δ 11.75 (s, 1H), 11.16 (s, 1H), 8.48 (d, *J* = 3.3 Hz, 1H), 8.43 (s, 1H), 8.30 (s, 1H), 8.17 (d, *J*= 7.7 Hz, 1H), 8.05 (d, *J* = 7.6 Hz, 1H), 5.25-5.17 (m, 1H), 4.63 (s, 2H), 3.68 (s, 2H), 3.59 - 3.56 (m, 1H), 3.43 (d, *J* = 9.9 Hz, 2H), 3.22 (s, 2H), 2.90 (d, *J* = 3.2 Hz, 1H), 2.62 (d, *J* = 17.7 Hz, 2H), 2.54 (s, 1H), 2.16 - 2.01 (m, 1H). LCMS (ESI) calcd for $C_{23}H_{22}ClFN_5O_4^+$[M+H]$^+$: 486.13, found, 486.1.

Example 352: Preparation of 5-((4-(3-bromo-4-chloropyridin-2-yl)piperazin-1-yl)methyl)-2-(2,6-dioxopiperidin-3-yl)isoindoline-1,3-dione (GT-06601)

Referring to the method of Scheme 1, the target compound (GT-06601) was prepared under appropriate conditions as would be understood by those skilled in the art (white solid, 29 mg, yield 48%). $^1$NMR (400 MHz, DMSO-d6) δ 11.75 (s, 1H), 11.16 (s, 1H), 8.48 (d, *J* = 3.3 Hz, 1H), 8.43 (s, 1H), 8.30 (s, 1H), 8.17 (d, *J*= 7.7 Hz, 1H), 8.05 (d, *J*= 7.6 Hz, 1H), 5.24-5.17(m, 1H), 4.63 (s, 2H), 3.68 (s, 2H), 3.59 - 3.56 (m, 2H), 3.43 (d, *J*= 9.9 Hz, 2H), 3.22 (s, 2H), 2.90 (d, *J* = 3.2 Hz, 2H), 2.62 (d, *J*= 17.7 Hz, 1H), 2.54 (s, 2H), 2.16 - 2.01 (m, 1H). LCMS (ESI) calcd for $C_{23}H_{22}ClFN_5O_4^+$[M+H]$^+$: 486.13, found, 486.1.

Example 353: Preparation of 5-((4-(3,5-dichloropyridin-4-yl)piperazin-1-yl)methyl)-2-(2,6-dioxopiperidin-3-yl)isoindoline-1,3-dione (GT-06602)

Referring to the method of Scheme 1, the target compound (GT-06602) was prepared under appropriate conditions as would be understood by those skilled in the art (white solid, 27 mg, yield 48%). $^1$NMR (400 MHz, DMSO-d6) δ 12.05 (s, 1H), 11.22 (s, 1H), 8.57 (s, 2H), 8.40 (s, 1H), 8.28 (d, *J*= 7.5 Hz, 1H), 8.10 (d, *J*= 7.7 Hz, 1H), 5.28-5.34 (m, 1H), 4.70 (s, 2H), 3.98 (s, 2H), 3.50 (d, *J*= 13.3 Hz, 4H), 3.29 (s, 2H), 3.06 - 2.89 (m, 1H), 2.64 (d, *J* = 10.4 Hz, 2H), 2.21 - 2.09 (m, 1H). LCMS (ESI) calcd for $C_{23}H_{22}Cl_2N_5O_4^+$[M+H]$^+$: 502.10, found, 502.1.

Example 354: Preparation of 5-((4-(2,6-dimethylpyridin-4-yl)piperazin-1-yl)methyl)-2-(2,6-dioxopiperidin-3-yl)isoindoline-1,3-dione (GT-06603)

Referring to the method of Scheme 1, the target compound (GT-06603) was prepared under appropriate conditions as would be understood by those skilled in the art (white solid, 42mg, yield 74%). $^1$NMR (400 MHz, DMSO-d6) δ 13.92 (s, 1H), 12.47 (s, 1H), 11.16 (s, 1H), 8.25 (s, 1H), 8.13 (d, *J*= 7.4 Hz, 1H), 8.03 (d, *J*= 7.6 Hz, 1H), 7.05 (s, 2H), 5.19 (dd, *J* = 12.8, 5.4 Hz, 1H), 4.56 (s, 2H), 4.35 (s, 2H), 3.80 - 3.58 (m, 2H), 3.43 (s, 2H), 3.24 - 3.05 (m, 2H), 2.89 (d, *J* = 11.8 Hz, 1H), 2.63-2.57 (m, 2H), 2.53 (s, 1H), 2.48 (s, 5H), 2.21 - 1.93 (m, 1H). LCMS (ESI) calcd for $C_{25}H_{28}N_5O_4^+$[M+H]$^+$: 462.21, found, 462.3.

Example 355: Preparation of 2-(2,6-dioxopiperidin-3-yl)-5-((4-(perfluoropyridin-4-yl)piperazin-1-yl)methyl)isoindoline-1,3-dione (GT-06604)

Referring to the method of Scheme 1, the target compound (GT-06604) was prepared under appropriate conditions as would be understood by those skilled in the art (white solid, 40mg, yield 66%). $^1$HNMR (400 MHz, DMSO-d6) δ 11.08 (s, 1H), 8.17 (s, 1H), 8.04 (s, 1H), 7.97 (d, *J*= 7.5 Hz, 1H), 5.13 (d, *J* = 12.9 Hz, 1H), 4.51 (s, 2H), 3.68 (s, 4H), 3.35 (s, 1H), 3.18 (s, 2H), 2.83 (d, *J* = 4.8 Hz, 1H), 2.57 (s, 1H), 2.51 (d, *J* = 10.5 Hz, 1H), 2.47 (s, 1H), 2.01 (d, *J* = 5.6 Hz, 1H). LCMS (ESI) calcd for $C_{23}H_{20}F_4N_5O_4^+$[M+H]$^+$: 506.14, found, 506.1.

Example 356: Preparation of 5-((4-(6-chloropyridazin-3-yl)piperazin-1-yl)methyl)-2-(2,6-dioxopiperidin-3-yl)isoindoline-1,3-dione (GT-06605)

Referring to the method of Scheme 1, the target compound (GT-06605) was prepared under appropriate conditions as would be understood by those skilled in the art (white solid, 32 mg, yield 56%). $^1$NMR (400 MHz, DMSO-d6) δ 11.16 (s, 1H), 8.26 (s, 1H), 8.13 (d, *J*= 8.6 Hz, 1H), 8.05 (d, *J*= 7.6 Hz, 1H), 7.67 (d, *J* = 9.6 Hz, 1H), 7.49 (d, *J* = 9.6 Hz, 1H), 5.20 (dd, *J*= 12.8, 5.4 Hz, 1H), 4.58 (s, 2H), 4.45 (d, *J* = 13.2 Hz, 2H), 3.45 (s, 4H), 3.16 (s, 2H), 2.91 (s, 1H), 2.63-2.55 (m, 2H), 2.08 (s, 1H). LCMS (ESI) calcd for $C_{22}H_{22}ClN_6O_4^+$ $[M+H]^+$: 469.14, found, 469.2.

Example 357: Preparation of 2-(2,6-dioxopiperidin-3-yl)-5-((4-(pyrimidin-2-yl)piperazin-1-yl)methyl)isoindoline-1,3-dione (GT-06606)

Referring to the method of Scheme 1, the target compound (GT-06606) was prepared under appropriate conditions as would be understood by those skilled in the art (white solid, 38mg, yield 71%). $^1$NMR (400 MHz, DMSO-d6) δ 12.05 (s, 1H), 11.16 (s, 1H), 8.45 (d, *J* = 4.8 Hz, 2H), 8.29 (s, 1H), 8.17 (d, *J*= 7.7 Hz, 1H), 8.03 (d, *J* = 7.6 Hz, 1H), 6.78 (t, *J* = 4.8 Hz, 1H), 5.23-5.16 (m, 1H), 4.69 (d, *J* = 13.3 Hz, 2H), 4.58 (s, 2H), 3.57 - 3.34 (m, 2H), 3.10 (d, *J* = 3.9 Hz, 4H), 2.99 - 2.84 (m, 1H), 2.60 (t, *J*= 14.3 Hz, 2H), 2.07 (d, *J* = 5.2 Hz, 1H). LCMS (ESI) calcd for $C_{22}H_{23}N_6O_4^+$ $[M+H]^+$: 435.18, found, 435.2.

Example 358: Preparation of 5-((4-(3,4-dichloropyridin-2-yl)piperidin-1-yl)methyl)-2-(2,6-dioxopiperidin-3-yl)isoindoline-1,3-dione (GT-06607)

Referring to the method of Scheme 1, the target compound (GT-06607) was prepared under appropriate conditions as would be understood by those skilled in the art (white solid, 29 mg, yield 48%). $^1$NMR (400 MHz, DMSO-d6) δ 11.16 (s, 1H), 8.48 (d, *J*= 5.2 Hz, 1H), 8.27 (s, 1H), 8.14 (d, *J*= 7.7 Hz, 1H), 8.05 (d, *J*= 7.6 Hz, 1H), 7.66 (d, *J*= 5.2 Hz, 1H), 5.23-5.17 (m, 1H), 4.54 (d, *J* = 4.4 Hz, 2H), 3.48 (d, *J* = 11.6 Hz, 2H), 3.17 (d, *J*= 10.4 Hz, 4H), 2.97 - 2.85 (m, 2H), 2.60 (t, *J*= 14.5 Hz, 1H), 2.19 (d, *J* = 12.7 Hz, 2H), 2.12 - 2.04 (m, 1H). LCMS (ESI) calcd for $C_{24}H_{23}Cl_2N_4O_4^+$ $[M+H]^+$: 501.11, found, 501.1.

Example 359: Preparation of 5-((3,4-dichloro-3',6'-dihydro-[2,4'-bipyridin]-1'(2'H)-yl)methyl)-2-(2,6-dioxopiperidin-3-yl) isoindoline-1,3-dione (GT-06608)

Referring to the method of Scheme 1, the target compound (GT-06608) was prepared under appropriate conditions as would be understood by those skilled in the art (light yellow solid, 24mg, yield 40%). $^1$NMR (400 MHz, DMSO-d6) δ 11.43 (s, 2H), 11.16 (s, 1H), 8.50 (d, *J*= 5.2 Hz, 1H), 8.31 (s, 1H), 8.19 (d, *J* = 6.8 Hz, 1H), 8.06 (d, *J* = 7.7 Hz, 1H), 7.72 (d, *J* = 5.2 Hz, 1H), 6.20 (s, 1H), 5.24-5.17 (m, 1H), 4.68 (s, 2H), 3.90 (s,3H), 3.83 (s, 2H), 3.31 (s, 1H), 2.93 - 2.85 (m, 2H), 2.73 (s, 2H), 2.66 - 2.55 (m, 1H), 2.15 - 2.00 (m, 2H). LCMS (ESI) calcd for $C_{24}H_{21}Cl_2N_4O_4^+$ $[M+H]^+$: 499.09, found, 499.1.

Example 360: Preparation of 5-((4,5-dichloro-3',6'-dihydro-[3,4'-bipyridin]-1'(2'H)-yl)methyl)-2-(2,6-dioxopiperidin-3-yl) isoindoline-1,3-dione (GT-06609)

Referring to the method of Scheme 1, the target compound (GT-06609) was prepared under appropriate conditions as would be understood by those skilled in the art (white solid, 26 mg, yield 49%). $^1$NMR (400 MHz, DMSO-d6) δ 11.63 (s, 1H), 11.16 (s, 1H), 8.76 (s, 1H), 8.40 (s, 1H), 8.32 (s, 1H), 8.21 (d, *J* = 7.8 Hz, 1H), 8.06 (d, *J* = 7.6 Hz, 1H), 5.91 (s, 1H), 5.20 (dd, *J*= 12.9, 5.3 Hz, 1H), 4.68 (s, 2H), 3.83 (d, *J* = 10.8 Hz, 2H), 3.72 (s, 2H), 3.30 (s, 1H), 2.90 (d, *J* = 12.1 Hz, 1H), 2.64 (s, 1H), 2.59 (d, *J* = 10.4 Hz, 2H), 2.14 - 2.03 (m, 1H). LCMS (ESI) calcd for $C_{24}H_{21}Cl_2N_4O_4^+$ $[M+H]^+$: 499.09, found, 499.1.

Example 361: Preparation of 5-((5-chloro-3',6'-dihydro-[3,4'-bipyridin]-1'(2'H)-yl)methyl)-2-(2,6-dioxopiperidin-3-yl)isoindoline-1,3-dione (GT-06611)

Referring to the method of Scheme 1, the target compound (GT-06611) was prepared under appropriate conditions as would be understood by those skilled in the art (light yellow solid, 26mg, yield 45%). ' NMR (400 MHz, DMSO-d6) δ 11.82 (s, 1H), 11.16 (s, 1H), 8.70 (d, *J* = 1.4 Hz, 1H), 8.61 (d, *J* = 2.0 Hz, 1H), 8.35 (s, 1H), 8.23 (d, *J* = 7.7 Hz, 1H), 8.09 (s, 1H), 8.04 (d, *J* = 7.6 Hz, 1H), 6.41 (s, 1H), 5.20 (dd, *J*= 12.8, 5.3 Hz, 1H), 4.66 (s, 2H), 3.84 (d, *J* = 22.4 Hz, 2H), 3.63 (s, 1H), 3.28 (s, 1H), 2.99 - 2.76 (m,3H), 2.69 - 2.55 (m, 2H), 2.15 - 1.95 (m, 1H). LCMS (ESI) calcd for $C_{24}H_{22}ClN_4O_4^+$ $[M+H]^+$: 465.13, found, 465.1.

Example 362: Preparation of 2-(2,6-dioxopiperidin-3-yl)-5-((3-fluoro-3',6'-dihydro-[2,4'-bipyridin]-1'(2'H)-yl)methyl)isoindoline-1,3-dione (GT-06612)

Referring to the method of Scheme 1, the target compound (GT-06612) was prepared under appropriate conditions as would be understood by those skilled in the art (white solid, 19 mg, yield 34%). $^1$NMR (400 MHz, DMSO-d6) δ 11.16 (s, 1H), 8.46 (dd, *J* = 2.9, 1.6 Hz, 1H), 8.33 (s, 1H), 8.22 (d, *J*= 7.7 Hz, 1H), 8.05 (d, *J* = 7.7 Hz, 1H), 7.82-7.76 (m, 1H), 7.48 - 7.39 (m, 1H), 6.55 (s, 1H), 5.19 (s, 1H), 4.67 (s, 2H), 3.83 - 3.78 (m, 2H), 3.64 (t, *J*= 12.9 Hz, 1H), 3.28 (s, 1H), 2.96 (s, 3H), 2.60 (s, 2H), 2.17 - 1.96 (m, 1H). LCMS (ESI) calcd for $C_{24}H_{22}FN_4O_4^+$ [M+H]$^+$: 449.16, found, 449.2.

Example 363: Preparation of 5-((4-(2,3-difluoropyridin-4-yl)piperidin-1-yl)methyl)-2-(2,6-dioxopiperidin-3-yl)isoindoline-1,3-dione (GT-06613)

Referring to the method of Scheme 1, the target compound (GT-06613) was prepared under appropriate conditions as would be understood by those skilled in the art (white solid, 23 mg, yield 40%). $^1$NMR (400 MHz, DMSO-d6) δ 11.18 (s, 1H), 11.09 (s, 1H), 8.19 (s, 1H), 8.07 (d, *J*= 7.5 Hz, 1H), 8.02 - 7.89 (m, 2H), 7.22 (t, *J* = 4.8 Hz, 1H), 5.15-5.06 (m, 1H), 4.47 (s, 2H), 3.42 (d, *J* = 11.3 Hz, 2H), 3.18 (s, 1H), 3.07 (d, *J* = 10.5 Hz, 2H), 2.91 - 2.77 (m, 1H), 2.53 (t, *J* = 14.2 Hz, 2H), 2.10 (d, *J* = 12.2 Hz, 2H), 2.01 (s, 1H), 1.92 (d, *J* = 12.8 Hz, 2H). LCMS (ESI) calcd for $C_{24}H_{23}F_2N_4O_4^+$ [M+H]$^+$: 469.17, found, 470.3.

Example 364: Preparation of 5-((2',3'-difluoro-3,6-dihydro-[4,4'-bipyridin]-1(2H)-yl)methyl)-2-(2,6-dioxopiperidin-3-yl) isoindoline-1,3-dione (GT-06614)

Referring to the method of Scheme 1, the target compound (GT-06614) was prepared under appropriate conditions as would be understood by those skilled in the art (white solid, 33 mg, yield 58%). $^1$NMR (400 MHz, DMSO-d6) δ 11.32 (s, 1H), 11.09 (s, 1H), 8.21 (s, 1H), 8.09 (d, *J* = 7.3 Hz, 1H), 8.02 - 7.93 (m, 2H), 7.35 (t, *J*= 5.1 Hz, 1H), 6.27 (s, 1H), 5.15-5.06 (m, 1H), 4.59 (s, 2H), 3.79 (s, 2H), 3.56 (d, *J*= 21.2 Hz, 1H), 3.22 - 3.13 (m, 1H), 2.82 (d, *J* = 11.9 Hz, 2H), 2.66 (s, 1H), 2.57-2.48 (m, 2H), 2.02 (d, *J*= 5.4 Hz, 1H). LCMS (ESI) calcd for $C_{24}H_{21}F_2N_4O_4^+$ [M+H]$^+$: 469.15, found, 470.3.

Example 365: Preparation of 5-((4-(2,3-dichloropyridin-4-yl)piperidin-1-yl)methyl)-2-(2,6-dioxopiperidin-3-yl)isoindoline-1,3-dione (GT-06615)

Referring to the method of Scheme 1, the target compound (GT-06615) was prepared under appropriate conditions as would be understood by those skilled in the art (white solid, 27 mg, yield 44%). $^1$NMR (400 MHz, DMSO-d6) δ 11.29 (s, 1H), 11.08 (s, 1H), 8.32 (d, *J*= 5.0 Hz, 1H), 8.19 (s, 1H), 8.07 (d, *J*= 7.6 Hz, 1H), 7.97 (d, *J* = 7.7 Hz, 1H), 7.30 (d, *J*= 5.1 Hz, 1H), 5.15-5.06 (m, 1H), 4.47 (s, 2H), 3.43 (d, *J* = 11.0 Hz, 2H), 3.23 (s, 1H), 3.10 (d, *J* = 10.3 Hz, 2H), 2.88-2.79 (m, 1H), 2.53 (t, *J*= 14.3 Hz, 2H), 2.06 (d, *J* = 12.4 Hz, 2H), 2.00 (d, *J* = 5.1 Hz, 1H), 1.92 (d, *J* = 12.9 Hz, 2H). LCMS (ESI) calcd for $C_{24}H_{23}Cl_2N_4O_4^+$ [M+H]$^+$: 501.11, found, 501.1.

Example 366: Preparation of 5-((2',3'-dichloro-3,6-dihydro-[4,4'-bipyridin]-1(2H)-yl)methyl)-2-(2,6-dioxopiperidin-3-yl) isoindoline-1,3-dione (GT-06616)

Referring to the method of Scheme 1, the target compound (GT-06616) was prepared under appropriate conditions as would be understood by those skilled in the art (white solid, 31mg, yield 50%). $^1$NMR (400 MHz, DMSO-d6) δ 11.37 (s, 1H), 11.09 (s, 1H), 8.33 (d, *J*= 4.9 Hz, 1H), 8.22 (s, 1H), 8.10 (d, *J*= 7.5 Hz, 1H), 7.98 (d, *J* = 7.6 Hz, 1H), 7.29 (d, *J* = 4.9 Hz, 1H), 5.85 (s, 1H), 5.13 (dd, *J* = 12.8, 5.3 Hz, 1H), 4.59 (s, 2H), 3.74 (s, 2H), 3.56 (s, 1H), 3.23 - 3.15 (m, 1H), 2.82 (d, *J* = 12.0 Hz, 2H), 2.55 (d, *J* = 17.6 Hz, 2H), 2.02 (d, *J* = 5.4 Hz, 1H). LCMS (ESI) calcd for $C_{24}H_{21}Cl_2N_4O_4^+$ [M+H]$^+$: 499.09, found, 499.2.

Example 367: Preparation of 5-((3-(2,3-dichlorophenyl)-3,6-diazabicyclo[3.1.1]heptan-6-yl)methyl)-2-(2,6-dioxopiperidin-3-yl)isoindoline-1,3-dione (GT-06688)

Referring to the method of Scheme 1, the target compound (GT-06688) was prepared under appropriate conditions as would be understood by those skilled in the art (white solid, 27 mg, yield 46%). $^1$NMR (400 MHz, DMSO-d6) δ 11.07 (s, 1H), 8.12-8.06 (m, 3H), 7.28 (t, *J* = 8.0 Hz, 2H), 5.14-5.05 (m, 1H), 4.74 (d, *J* = 40.5 Hz, 1H), 4.10 (d, *J* = 154.6 Hz, 2H), 3.61 (d, *J* = 61.9 Hz, 2H), 3.30 (s, 2H), 3.23 (s, 1H), 3.17 (s, 1H), 2.81 (d, *J*= 11.9 Hz, 1H), 2.54 (d, *J*= 17.0 Hz, 2H), 2.45 (s, 1H), 2.00 (d, *J*= 5.3 Hz, 1H). LCMS (ESI) calcd for $C_{25}H_{23}Cl_2N_4O_4^+$ [M+H]$^+$: 513.11, found, 512.9.

Example 368: Preparation of 5-((6-(2,3-dichlorophenyl)-3,6-diazabicyclo[3.1.1]heptan-3-yl)methyl)-2-(2,6-dioxopiperidin-3-yl)isoindoline-1,3-dione (GT-06689)

Referring to the method of Scheme 1, the target compound (GT-06689) was prepared under appropriate conditions as would be understood by those skilled in the art (white solid, 37 mg, yield 60%). $^1$NMR (400 MHz, DMSO-d6) δ 12.26 (s, 1H), 11.11 (s, 1H), 7.95 (d, *J* = 22.2 Hz, 1H), 7.76 (d, *J*= 7.3 Hz, 1H), 6.90 (s, 1H), 6.82 (d, *J*= 7.5 Hz, 1H), 6.47 (d, *J*= 7.7 Hz, 1H), 5.13 (d, *J*= 5.1 Hz, 1H), 4.56 (s, 2H), 4.28 (s, 2H), 3.49 (s, 2H), 3.36 (s, 1H), 2.85 (s, 1H), 2.65 (s, 1H), 2.57 (d, *J* = 17.5 Hz, 2H), 2.52 (s, 2H), 2.09 - 2.00 (m, 1H). LCMS (ESI) calcd for $C_{25}H_{23}Cl_2N_4O_4^+$ [M+H]$^+$: 513.11, found, 512.9.

Example 369: Preparation of 5-(((1R,4R)-5-(2,3-dichlorophenyl)-2,5-diazabicyclo[2.2.1]heptan-2-yl)methyl)-2-(2,6-dioxopiperidin-3-yl)isoindoline-1,3-dione (GT-06690)

Referring to the method of Scheme 1, the target compound (GT-06690) was prepared under appropriate conditions as would be understood by those skilled in the art (white solid, 18 mg, yield 29%). $^1$NMR (400 MHz, DMSO-d6) δ 11.08 (s, 1H), 10.56 (s, 1H), 8.26 (s, 1H), 8.14 (d, *J* = 7.6 Hz, 1H), 7.96 (d, *J* = 7.6 Hz, 1H), 7.09 (d, *J* = 8.1 Hz, 1H), 7.04 (d, *J*= 7.4 Hz, 1H), 6.91 (d, *J* = 8.1 Hz, 1H), 5.11 (dd, *J* = 12.8, 5.4 Hz, 1H), 4.68 (d, *J* = 4.0 Hz, 1H), 4.46 (s, 2H), 4.34 (s, 1H), 3.86 (d, *J* = 9.4 Hz, 1H), 3.72 (d, *J*= 11.1 Hz, 1H), 3.53 (d, *J* = 4.1 Hz, 1H), 3.30 (s, 1H), 3.19 (s, 1H), 2.90 - 2.76 (m, 1H), 2.52 (s, 2H), 2.10 (d, *J* = 11.4 Hz, 1H), 2.04 - 1.94 (m, 1H). LCMS (ESI) calcd for $C_{25}H_{23}Cl_2N_4O_4^+$ [M+H]$^+$: 513.11, found, 512.9.

Example 370: Preparation of 5-(((1S,4S)-5-(2,3-dichlorophenyl)-2,5-diazabicyclo[2.2.1]heptan-2-yl)methyl)-2-(2,6-dioxopiperidin-3-yl)isoindoline-1,3-dione (GT-06691)

Referring to the method of Scheme 1, the target compound (GT-06691) was prepared under appropriate conditions as would be understood by those skilled in the art (white solid, 32 mg, yield 51%). $^1$NMR (400 MHz, DMSO-d6) δ 11.08 (s, 1H), 10.50 (s, 1H), 8.25 (s, 1H), 8.14 (d, *J* = 7.6 Hz, 1H), 7.97 (d, *J* = 7.6 Hz, 1H), 7.10 (s, 1H), 7.04 (s, 1H), 6.91 (d, *J* = 8.1 Hz, 1H), 5.16-5.07 (m, 1H), 4.70 (d, *J* = 8.7 Hz, 1H), 4.46 (s, 2H), 4.34 (s, 1H), 3.85 (s, 1H), 3.73 (s, 1H), 3.19 (s, 2H), 2.82 (s, 1H), 2.52 (s, 3H), 2.10 (d, *J* = 11.8 Hz, 1H), 2.04 - 1.97 (m, 1H). LCMS (ESI) calcd for $C_{25}H_{23}Cl_2N_4O_4^+$ [M+H]$^+$: 513.11, found, 512.9.

Example 371: Preparation of 5-((5-(2,3-dichlorophenyl)-2,5-diazabicyclo[2.2.2]octan-2-yl)methyl)-2-(2,6-dioxopiperidin-3-yl)isoindoline-1,3-dione (GT-06692)

Referring to the method of Scheme 1, the target compound (GT-06692) was prepared under appropriate conditions as would be understood by those skilled in the art (white solid, 27 mg, yield 42%). $^1$H NMR (400 MHz, DMSO-d6) δ 11.11 (s, 1H), 8.35 (d, *J* = 8.8 Hz, 1H), 8.24 (m, *J* = 15.1, 7.7 Hz, 1H), 7.97 (t, *J* = 7.4 Hz, 1H), 7.28 (m, *J* = 15.9, 8.1 Hz, 1H), 7.21 - 7.12 (m, 2H), 5.16 - 5.08 (m, 1H), 4.76 - 4.49 (m, 2H), 3.73 (s, 1H), 3.64-3.53(m, 2H), 3.41 (d, *J* = 12.1 Hz, 1H), 3.14 (d, *J* = 12.3 Hz, 1H), 2.86 - 2.77 (m, 1H), 2.57 (s, 1H), 2.51 (d, *J* = 11.0 Hz, 1H), 2.28 (d, *J* = 12.2 Hz, 1H), 2.05 (s, 1H), 2.03-1.94 (m, 2H), 1.89 - 1.79 (m, 1H), 1.23 - 1.18 (m, 1H). LCMS (ESI) calcd for $C_{26}H_{25}Cl_2N_4O_4^+$ [M+H]$^+$: 527.12, found, 526.9.

Example 372: Preparation of 5-((8-(2,3-dichlorophenyl)-3,8-diazabicyclo[3.2.1]octan-3-yl)methyl)-2-(2,6-dioxopiperidin-3-yl)isoindoline-1,3-dione (GT-06693)

Referring to the method of Scheme 1, the target compound (GT-06693) was prepared under appropriate conditions as would be understood by those skilled in the art (white solid, 32 mg, yield 50%). $^1$H NMR (400 MHz, DMSO-d6) δ 11.08 (s, 1H), 10.56 (s, 1H), 8.35 (s, 1H), 8.22 (s, 1H), 7.95 (s, 1H), 7.19 (s, 2H), 6.99 (s, 1H), 5.11 (d, *J* = 8.0 Hz, 1H), 4.51 (s, 2H), 4.10 (s, 2H), 3.25 - 3.15 (m, 2H), 2.82 (d, *J* = 13.4 Hz, 1H), 2.56-2.48 (m, 2H), 2.13 (s, 2H), 2.05 - 1.96 (m, 2H), 1.92 (s, 2H), 1.26 - 1.13 (m, 1H). LCMS (ESI) calcd for $C_{26}H_{25}Cl_2N_4O_4^+$ [M+H]$^+$: 527.12, found, 526.9.

Example 373: Preparation of 5-((3-(2,3-dichlorophenyl)-3,8-diazabicyclo[3.2.1]octan-8-yl)methyl)-2-(2,6-dioxopiperidin-3-yl)isoindoline-1,3-dione (GT-06694)

Referring to the method of Scheme 1, the target compound (GT-06694) was prepared under appropriate conditions as would be understood by those skilled in the art (white solid, 30 mg, yield 47%). $^1$H NMR (400 MHz, DMSO-d6) δ 11.08 (s, 1H), 8.34 (s, 1H), 8.24 (d, *J* = 7.5 Hz, 1H), 7.99 (d, *J* = 7.6 Hz, 1H), 7.35 - 7.24 (m, 2H), 7.11 (d, *J* = 7.6 Hz, 1H), 5.16-5.09 (m, 1H), 4.41 (d, *J* = 4.9 Hz, 2H), 3.92 (s, 2H), 3.51 (d, *J* = 12.2 Hz, 2H), 3.14 (d, *J* = 11.6 Hz, 2H), 2.86 - 2.77 (m, 1H), 2.57 (s, 1H), 2.53 (s, 1H), 2.30 - 2.21 (m, 3H), 2.03 - 1.96 (m, 1H), 1.25-1.17 (m, 1H). LCMS (ESI) calcd for $C_{26}H_{25}Cl_2N_4O_4^+$[M+H]$^+$: 527.12, found, 526.9.

Example 374: Preparation of 5-((4-(2,3-dichlorophenyl)-1,4-diazepan-1-yl)methyl)-2-(2,6-dioxopiperidin-3-yl)isoindoline-1,3-dione (GT-06695)

Referring to the method of Scheme 1, the target compound (GT-06695) was prepared under appropriate conditions as would be understood by those skilled in the art (white solid, 30 mg, yield 48%). $^1$H NMR (400 MHz, DMSO-d6) δ 11.21 (s, 1H), 11.08 (s, 1H), 8.23 (d, *J* = 14.9 Hz, 1H), 8.12 (d, *J* = 7.6 Hz, 1H), 7.97 (d, *J* = 7.7 Hz, 1H), 7.24 (m, *J* = 7.1, 6.2 Hz, 2H), 7.15 (p, *J* = 3.9 Hz, 1H), 5.12 (m, *J* = 12.8, 5.3 Hz, 1H), 4.59 (d, *J* = 22.2 Hz, 2H), 3.47 (dd, *J* = 17.8, 9.1 Hz, 3H), 3.18 (m, *J* = 13.9, 6.9 Hz, 2H), 2.86 - 2.77 (m, 1H), 2.52 (m, *J* = 22.3, 11.2 Hz, 2H), 2.16 (d, *J* = 18.4 Hz, 2H), 1.25 - 1.16 (m, 4H). LCMS (ESI) calcd for $C_{25}H_{25}Cl_2N_4O_4^+$ [M+H]$^+$: 515.12, found, 514.9.

Example 375: Preparation of 5-([3,4'-bipiperidin]-1-ylmethyl)-2-(2,6-dioxopiperidin-3-yl)isoindoline-1,3-dione (GT-06881)

Referring to the method of Scheme 1, the target compound (GT-06881) was prepared under appropriate conditions as would be understood by those skilled in the art (light yellow solid, 21mg, yield 67%). $^1$H NMR (400 MHz, DMSO-d6) δ 11.21 (s, 1H), 11.08 (s, 1H), 8.23 (d, *J* = 14.9 Hz, 1H), 8.12 (d, *J* = 7.6 Hz, 1H), 7.97 (d, *J* = 7.7 Hz, 1H), 7.28-7.19 (m, 2H), 7.15 (d, *J* = 3.9 Hz, 1H), 5.16-5.10 (m, 1H), 4.59 (d, *J* = 22.2 Hz, 2H), 3.51-3.44 (m, 3H), 3.22-3.17 (m, 2H), 2.86 - 2.77 (m, 1H), 2.57-2.40 (m, 2H), 2.16 (d, *J* = 18.4 Hz, 2H), 1.25 - 1.16 (m, 4H). LCMS (ESI) calcd for $C_{23}H_{24}F_2N_5O_3^+$ [M+H]$^+$: 439.23, found, 439.3.

Example 376: Preparation of 2-(2,6-dioxopiperidin-3-yl)-5-((4-(thiophen-2-yl)piperazin-1-yl)methyl)isoindoline-1,3-dione (GT-06212)

Referring to the method of Scheme 1, the target compound (GT-06212) was prepared under appropriate conditions as would be understood by those skilled in the art (brown solid, 18.6 mg, yield 34 %). $^1$H NMR (400 MHz, DMSO-d6) δ 11.43 (s, 1H), 11.14 (s, 1H), 9.36 (s, 1H), 8.28 - 8.16 (m, 1H), 8.16 - 7.96 (m, 3H), 5.26 - 5.17 (m, 1H), 4.68 - 4.20 (m, 3H), 4.01 - 3.64 (m, 2H), 3.23 (s, 3H), 2.94 - 2.86 (m, 2H), 2.84 - 2.70 (m, 1H), 2.65 - 2.57 (m, 2H), 2.10 - 2.05 (m, 1H). LCMS (ESI) calcd for $C_{22}H_{23}N_4O_4S$ $^+$ [M+H]$^+$: 439.14, found, 439.1.

Example 377: Preparation of 2-(2,6-dioxopiperidin-3-yl)-5-((4-(5-methylthiophen-2-yl)piperazin-1-yl)methyl)isoindoline-1,3-dione (GT-06213)

Referring to the method of Scheme 1, the target compound (GT-06213) was prepared under appropriate conditions as would be understood by those skilled in the art (white solid, 12.3 mg, yield 22 %). $^1$H NMR (400 MHz, DMSO-d6) δ 11.15 (s, 1H), 9.30 (s, 1H), 8.28 - 8.10 (m, 2H), 8.09 - 7.97 (m, 3H), 5.26 - 5.11 (m, 2H), 4.64 - 4.24 (m, 3H), 3.15 - 3.06 (m, 3H), 2.98 - 2.87 (m, 3H), 2.63 - 2.56 (m, 2H), 2.39 - 2.28 (m, 2H), 2.12 - 2.04 (m, 2H), 1.28 (dd, *J* = 11.0, 6.4 Hz, 1H). LCMS (ESI) calcd for $C_{23}H_{25}N_4O_4S^+$ [M+H]$^+$: 453.16, found, 453.2.

Example 378: Preparation of 2-(2,6-dioxopiperidin-3-yl)-5-((4-(3-methylthiophen-2-yl)piperazin-1-yl)methyl)isoindoline-1,3-dione (GT-06214)

Referring to the method of Scheme 1, the target compound (GT-06214) was prepared under appropriate conditions as would be understood by those skilled in the art (white solid, 5.3 mg, yield 10 %). $^1$H NMR (400 MHz, DMSO-d6) δ 11.09 (s, 1H), 8.04 - 7.96 (m, 2H), 7.87 - 7.70 (m, 2H), 7.02 (d, *J* = 5.5 Hz, 1H), 6.71 (d, *J* = 5.5 Hz, 1H), 5.16 - 5.06 (m, 2H), 4.54 (s, 2H), 3.13 (d, *J* = 11.5 Hz, 4H), 2.92 - 2.78 (m, 3H), 2.61 - 2.46 (m, 4H), 2.01 (s, 3H). LCMS (ESI) calcd for $C_{23}H_{25}N_4O_4S$ $^+$ [M+H]$^+$: 453.16, found, 453.2.

Example 379: Preparation of 2-(2,6-dioxopiperidin-3-yl)-5-((4-(thiophen-3-yl)piperazin-1-yl)methyl)isoindoline-1,3-dione (GT-06215)

Referring to the method of Scheme 1, the target compound (GT-06215) was prepared under appropriate conditions as would be understood by those skilled in the art (white solid, 16 mg, yield 29 %). $^1$H NMR (400 MHz, DMSO-d6) δ 11.16 (s, 1H), 8.15 (d, 1H), 8.03 - 7.99 (m, 2H), 7.47 (dd, *J* = 5.1, 3.0 Hz, 1H), 6.98 (d, *J* = 5.3 Hz, 1H), 6.50 (d, *J* = 1.4 Hz, 1H), 5.20 - 5.13 (m, 2H), 4.59 (s, 2H), 4.35 (s, 2H), 3.67 (d, *J* = 11.6 Hz, 2H), 3.16 - 3.11 (m, 2H), 2.93 - 2.86 (m, 2H), 2.59 (s, 2H), 2.10 - 2.06 (m, 2H). LCMS (ESI) calcd for $C_{22}H_{23}N_4O_4S$ $^+$ [M+H]$^+$: 439.14, found, 439.1.

Example 380: Preparation of 2-(2,6-dioxopiperidin-3-yl)-5-((4-(5-methylthiophen-3-yl)piperazin-1-yl)methyl)isoindoline-1,3-dione (GT-06218)

Referring to the method of Scheme 1, the target compound (GT-06218) was prepared under appropriate conditions as would be understood by those skilled in the art (white solid, 18 mg, yield 32 %). $^1$H NMR (400 MHz, DMSO-d6) δ 11.21 (s, 1H), 11.15 (s, 1H), 9.43 (s, 1H), 8.23 (d, *J* = 22.5 Hz, 1H), 8.14 - 8.05 (m, 1H), 8.02 (t, *J* = 6.8 Hz, 1H), 5.26 - 5.12 (m, 1H), 4.50 (d, *J* = 67.1 Hz, 2H), 3.69 - 3.55 (m, 2H), 3.32 - 3.01 (m, 7H), 2.94 - 2.86 (m, 1H), 2.65 - 2.54 (m, 2H), 2.44 - 2.29 (m, 2H), 2.12 - 2.04 (m, 1H). LCMS (ESI) calcd for $C_{23}H_{25}N_4O_4S^+$ [M+H]$^+$: 453.16, found, 453.2.

Example 381: Preparation of 2-(2,6-dioxopiperidin-3-yl)-5-((4-(thiophen-2-yl)-3,6-dihydropyridin-1(2H)-yl)methyl)isoindoline-1,3-dione (GT-06219)

Referring to the method of Scheme 1, the target compound (GT-06219) was prepared under appropriate conditions as would be understood by those skilled in the art (white solid, 36 mg, yield 67 %). $^1$H NMR (400 MHz, DMSO-d6) δ 11.16 (s, 1H), 11.11 (s, 1H), 8.27 (s, 1H), 8.16 (d, 1H), 8.05 (d, *J* = 7.6 Hz, 1H), 7.49 (d, *J* = 5.0 Hz, 1H), 7.18 (d, *J* = 3.4 Hz, 1H), 7.13 - 7.02 (m, 1H), 6.01 (s, 1H), 5.20 (dd,*J* = 12.8, 5.4 Hz, 1H), 4.72 - 4.52 (m, 2H), 3.90 - 3.58 (m, 3H), 3.31 - 3.27 (m, 1H), 2.95 - 2.80 (m, 3H), 2.65 - 2.54 (m, 2H), 2.11 - 2.00 (m, 1H). LCMS (ESI) calcd for $C_{23}H_{22}N_3O_4S^+$ [M+H]$^+$: 436.13, found, 436.2.

Example 382: Preparation of 2-(2,6-dioxopiperidin-3-yl)-5-((4-(5-methylthiophen-2-yl)-3,6-dihydropyridin-1(2H)-yl) methyl)isoindoline-1,3-dione (GT-06220)

Referring to the method of Scheme 1, the target compound (GT-06220) was prepared under appropriate conditions as would be understood by those skilled in the art (white solid, 20 mg, yield 36 %). $^1$H NMR (400 MHz, DMSO-d6) δ 11.16 (s, 1H), 10.95 (s, 1H), 8.25 (s, 1H), 8.09 (dd, *J* = 28.2, 7.5 Hz, 2H), 6.96 (s, 1H), 6.75 (s, 1H), 5.90 (s, 1H), 5.20 (dd, *J* = 12.7, 5.2 Hz, 1H), 4.62 (d, *J* = 17.9 Hz, 2H), 3.90 - 3.54 (m, 3H), 3.30 - 3.26 (m, 1H), 2.96 - 2.88 (m, 1H), 2.87 - 2.73 (m, 2H), 2.66 - 2.54 (m, 2H), 2.42 (s, 3H), 2.13 - 2.03 (m, 1H). LCMS (ESI) calcd for $C_{24}H_{24}N_3O_4S^+$ [M+H]$^+$: 450.15, found, 450.2.

Example 383: Preparation of 2-(2,6-dioxopiperidin-3-yl)-5-((4-(4-methylthiophen-3-yl)-3,6-dihydropyridin-1(2H)-yl) methyl)isoindoline-1,3-dione (GT-06221)

Referring to the method of Scheme 1, the target compound (GT-06221) was prepared under appropriate conditions as would be understood by those skilled in the art (white solid, 33 mg, yield 60 %). $^1$H NMR (400 MHz, DMSO-d6) δ 11.16 (s, 1H), 11.04 (s, 1H), 8.27 (s, 1H), 8.15 (d, *J* = 7.7 Hz, 1H), 8.06 (d, *J* = 7.6 Hz, 1H), 7.43 (d, *J* = 3.2 Hz, 1H), 7.20 (d, *J* = 3.2, 0.9 Hz, 1H), 5.80 (s, 1H), 5.20 (dd, *J* = 12.9, 5.4 Hz, 1H), 4.74 - 4.54 (m, 2H), 3.90 - 3.67 (m, 2H), 3.61 (s, 1H), 3.29 - 3.21 (m, 1H), 2.96 - 2.79 (m, 2H), 2.68 - 2.53 (m, 3H), 2.24 (s, 3H), 2.13 - 2.03 (m, 1H). LCMS (ESI) $C_{24}H_{24}N_3O_4S^+$ [M+H]$^+$: 450.15, found, 450.2.

Example 384: Preparation of 2-(2,6-dioxopiperidin-3-yl)-5-((4-(5-methylthiophen-3-yl)-3,6-dihydropyridin-1(2H)-yl) methyl)isoindoline-1,3-dione (GT-06279)

Referring to the method of Scheme 1, the target compound (GT-06279) was prepared under appropriate conditions as would be understood by those skilled in the art (white solid, 32 mg, yield 58 %). $^1$H NMR (400 MHz, DMSO-d6) δ 11.10 (s, 1H), 10.82 (s, 1H), 8.25 - 7.90 (m, 4H), 7.21 (s, 1H), 6.99 (s, 1H), 5.99 (s, 1H), 5.12 (dd, 1H), 4.54 (d, *J* = 19.7 Hz, 2H), 3.75 - 3.61 (m, 4H), 2.90 - 2.78 (m, 2H), 2.72 - 2.59 (m, 3H), 2.35 (s, 3H), 2.05 - 1.99 (m, 1H). LCMS (ESI) calcd for $C_{24}H_{24}N_3O_4S^+$ [M+H]$^+$: 450.15, found, 450.

Example 385: Preparation of 2-(2,6-dioxopiperidin-3-yl)-5-((4-(furan-2-yl)-3,6-dihydropyridin-1(2H)-yl)methyl)isoindoline-1,3-dione (GT-06222)

Referring to the method of Scheme 1, the target compound (GT-06222) was prepared under appropriate conditions as would be understood by those skilled in the art (white solid, 7 mg, yield 13 %). $^1$H NMR (400 MHz, DMSO-d6) δ 11.16 (s, 1H), 11.06 (s, 1H), 8.29 - 8.21 (m, 1H), 8.14 - 8.04 (m, 2H), 7.68 (s, 1H), 6.59 - 6.46 (m, 2H), 6.09 (s, 1H), 5.20 (dd, *J* = 12.8, 5.3 Hz, 1H), 4.78 - 4.58 (m, 2H), 3.71 (d, *J* = 59.4 Hz, 3H), 3.25 - 3.18 (m, 1H), 2.95 - 2.87 (m, 1H), 2.81 - 2.62 (m, 3H), 2.10 - 2.04 (m, 1H), 1.28 (dd, *J* = 10.4, 6.5 Hz, 1H). LCMS (ESI) calcd for $C_{23}H_{22}N_3O_5^+$ [M+H]$^+$: 420.16, found, 420.1.

Example 386: Preparation of 2-(2,6-dioxopiperidin-3-yl)-5-((4-(furan-3-yl)-3,6-dihydropyridin-1(2H)-yl)methyl)isoindoline-1,3-dione (GT-06223)

Referring to the method of Scheme 1, the target compound (GT-06223) was prepared under appropriate conditions as would be understood by those skilled in the art (white solid, 15 mg, yield 29 %). $^1$H NMR (400 MHz, DMSO-d6) δ 11.09 (s, 1H), 10.90 (s, 1H), 8.19 (s, 1H), 8.06 (d, *J* = 7.2 Hz, 1H), 7.98 (d, *J* = 7.4 Hz, 1H), 7.77 (s, 1H), 7.61 (s, 1H), 6.68 (s, 1H), 5.91 (s, 1H), 5.12 (dd, *J* = 12.7, 5.1 Hz, 1H), 4.63 - 4.48 (m, 2H), 3.82 - 3.49 (m, 4H), 3.21 - 3.18 (m, 1H), 2.87 - 2.81 (m, 1H), 2.71 - 2.55 (m, 3H), 2.03 - 1.97 (m, 1H). LCMS (ESI) calcd for $C_{23}H_{22}N_3O_5{}^+$ [M+H]$^+$: 420.16, found, 420.2.

Example 387: Preparation of 2-(2,6-dioxopiperidin-3-yl)-5-((4-(thiophen-2-yl)piperidin-1-yl)methyl)isoindoline-1,3-dione (GT-06224)

Referring to the method of Scheme 1, the target compound (GT-06224) was prepared under appropriate conditions as would be understood by those skilled in the art (white solid, 24 mg, yield 44 %). $^1$H NMR (400 MHz, DMSO-d6) δ 11.16 (s, 1H), 10.92 (s, 1H), 8.25 (s, 1H), 8.13 (d, *J* = 7.6 Hz, 1H), 8.05 (d, *J* = 7.5 Hz, 1H), 7.38 (d, *J* = 5.1 Hz, 1H), 6.98 (dd, *J* = 5.0, 3.6 Hz, 1H), 6.91 (d, *J* = 3.3 Hz, 1H), 5.23 - 5.17 (m, 1H), 4.54 (d, *J* = 4.2 Hz, 2H), 3.45 (d, *J* = 11.3 Hz, 3H), 3.14 - 3.05 (m, 3H), 2.94 - 2.86 (m, 1H), 2.67 -2.53 (m, 3H), 2.14 -2.04 (m, 3H). LCMS (ESI) calcd for $C_{23}H_{24}N_3O_4S^+$ [M+H]$^+$: 438.15, found, 438.2.

Example 388: Preparation of 2-(2,6-dioxopiperidin-3-yl)-5-((4-(3-methylthiophen-2-yl)piperidin-1-yl)methyl)isoindoline-1,3-dione (GT-06280)

Referring to the method of Scheme 1, the target compound (GT-06280) was prepared under appropriate conditions as would be understood by those skilled in the art (white solid, 36 mg, yield 65 %). $^1$H NMR (400 MHz, DMSO-d6) δ 11.17 (s, 1H), 10.73 (s, 1H), 8.23 (s, 1H), 8.08 (dd, *J* = 21.7, 7.6 Hz, 2H), 7.27 (d, *J* = 5.0 Hz, 1H), 6.83 (d, *J* = 5.0 Hz, 1H), 5.20 (dd, *J* = 12.7, 5.3 Hz, 1H), 4.53 (s, 2H), 3.20 - 3.07 (m, 3H), 2.95 - 2.85 (m, 1H), 2.65 - 2.53 (m, 2H), 2.15 (s, 3H), 2.12 - 1.91 (m, 5H). LCMS (ESI) calcd for $C_{24}H_{26}N_3O_4S^+$ [M+H]$^+$: 452.16, found, 452.2.

Example 389: Preparation of 2-(2,6-dioxopiperidin-3-yl)-5-((4-(thiophen-3-yl)piperidin-1-yl)methyl)isoindoline-1,3-dione (GT-06281)

Referring to the method of Scheme 1, the target compound (GT-06281) was prepared under appropriate conditions as would be understood by those skilled in the art (white solid, 16 mg, yield 30 %). $^1$H NMR (400 MHz, DMSO-d6) δ 11.10 (s, 1H), 10.57 (s, 1H), 8.19 (d, *J* = 17.5 Hz, 1H), 8.01 (dd, *J* = 23.2, 7.6 Hz, 2H), 7.44 (dd, *J* = 4.9, 2.9 Hz, 1H), 7.16 (s, 1H), 6.98 (d, *J* = 5.0 Hz, 1H), 5.12 (dd, *J* = 12.7, 5.3 Hz, 1H), 4.46 (s, 2H), 3.38 (d, *J* = 11.8 Hz, 2H), 3.04 - 2.95 (m, 2H), 2.87 - 2.76 (m, 2H), 2.60 - 2.47 (m, 2H), 2.05 - 1.98 (m, 3H), 1.84 (dd, *J* = 24.4, 11.8 Hz, 2H). LCMS (ESI) calcd for $C_{23}H_{24}N_3O_4S^+$ [M+H]$^+$: 438.15, found, 438.2.

Example 390: Preparation of 2-(2,6-dioxopiperidin-3-yl)-5-((4-(4-methylthiophen-3-yl)piperidin-1-yl)methyl)isoindoline-1,3-dione (GT-06282)

Referring to the method of Scheme 1, the target compound (GT-06282) was prepared under appropriate conditions as would be understood by those skilled in the art (white solid, 33 mg, yield 59 %). $^1$H NMR (400 MHz, DMSO-d6) δ 11.09 (s, 1H), 10.73 (s, 1H), 8.17 (s, 1H), 8.01 (dd, *J* = 26.6, 7.7 Hz, 2H), 7.05 (d, *J* = 3.4 Hz, 2H), 5.12 (dd, *J* = 12.8, 5.3 Hz, 1H), 4.46 (d, *J* = 3.7 Hz, 2H), 3.38 (d, *J* = 11.2 Hz, 2H), 3.08 - 2.96 (m, 2H), 2.88 - 2.79 (m, 1H), 2.76 - 2.67 (m, 1H), 2.59 - 2.47 (m, a2H), 2.10 (s, 3H), 2.05 - 1.99 (m, 1H), 1.94 - 1.82 (m, 4H). LCMS (ESI) calcd for $C_{24}H_{26}N_3O_4S^+$ [M+H]$^+$: 452.16, found, 452.2.

Example 391: Preparation of 2-(2,6-dioxopiperidin-3-yl)-5-((4-(5-methylthiophen-3-yl)piperidin-1-yl)methyl)isoindoline-1,3-dione (GT-06283)

Referring to the method of Scheme 1, the target compound (GT-06283) was prepared under appropriate conditions as would be understood by those skilled in the art (white solid, 32 mg, yield 58 %). $^1$H NMR (400 MHz, DMSO-d6) δ 11.16 (s, 1H), 10.75 (s, 1H), 8.24 (s, 1H), 8.12 (d, *J* = 7.6 Hz, 1H), 8.05 (d, *J* = 7.6 Hz, 1H), 6.93 (s, 1H), 6.72 (s, 1H), 5.19 (dd, *J* = 12.7, 5.3 Hz, 1H), 4.58 (d, *J* = 47.7 Hz, 2H), 3.43 (d, *J* = 12.2 Hz, 2H), 3.11 - 2.98 (m, 2H), 2.95 - 2.84 (m, 1H), 2.77 (t, *J* = 12.1 Hz, 1H), 2.68 - 2.53 (m, 2H), 2.42 (s, 3H), 2.12 - 2.00 (m, 3H), 2.00 - 1.76 (m, 2H). LCMS (ESI) calcd for $C_{24}H_{26}N_3O_4S^+$ [M+H]$^+$: 452.16, found, 452.2.

Example 392: Preparation of 2-(2,6-dioxopiperidin-3-yl)-5-((4-(furan-2-yl)piperidin-1-yl)methyl)isoindoline-1,3-dione (GT-06284)

Referring to the method of Scheme 1, the target compound (GT-06284) was prepared under appropriate conditions as would be understood by those skilled in the art (white solid, 27 mg, yield 52 %). $^1$H NMR (400 MHz, DMSO-d6) δ 11.16 (s, 1H), 10.81 (s, 1H), 8.29 - 8.18 (m, 1H), 8.12 (d, *J* = 7.8 Hz, 1H), 8.07 - 7.98 (m, 1H), 7.63 - 7.51 (m, 1H), 6.43 - 6.29 (m, 1H), 6.14 (d, *J* = 3.1 Hz, 1H), 5.19 (dd, *J* = 12.7, 5.3 Hz, 1H), 4.52 (s, 2H), 3.44 (d, *J* = 11.2 Hz, 2H), 3.14 - 3.01 (m, 2H), 2.97 - 2.84 (m, 2H), 2.68 - 2.54 (m, 2H), 2.20 - 2.07 (m, 3H), 2.05 - 1.80 (m, 2H). LCMS (ESI) calcd for $C_{23}H_{24}N_3O_5{}^+$ $[M+H]^+$: 422.17, found, 422.2.

Example 393: Preparation of 2-(2,6-dioxopiperidin-3-yl)-5-((4-(furan-3-yl)piperidin-1-yl)methyl)isoindoline-1,3-dione (GT-06285)

Referring to the method of Scheme 1, the target compound (GT-06285) was prepared under appropriate conditions as would be understood by those skilled in the art (white solid, 35 mg, yield 67 %). $^1$H NMR (400 MHz, DMSO-d6) δ 11.16 (s, 1H), 10.52 (s, 1H), 8.28 - 8.17 (m, 1H), 8.07 (dd, *J* = 19.1, 7.6 Hz, 2H), 7.72 - 7.57 (m, 1H), 7.50 (s, 1H), 6.43 (s, 1H), 5.19 (dd, *J* = 12.8, 5.3 Hz, 1H), 4.71 - 4.42 (m, 2H), 3.43 (s, 2H), 3.13 - 2.99 (m, 2H), 2.98 - 2.87 (m, 1H), 2.76 - 2.54 (m, 3H), 2.11 - 2.01 (m, 3H), 2.01 - 1.68 (m, 2H). LCMS (ESI) calcd for $C_{23}H_{24}N_3O_5{}^+$ $[M+H]^+$: 422.17, found, 422.2.

Example 394: Preparation of 2-(2,6-dioxopiperidin-3-yl)-5-((4-phenylpiperazin-1-yl)methyl)isoindoline-1,3-dione (GT-06286)

Referring to the method of Scheme 1, the target compound (GT-06286) was prepared under appropriate conditions as would be understood by those skilled in the art (white solid, 32 mg, yield 60 %). $^1$H NMR (400 MHz, DMSO-d6) δ 11.17 (s, 2H), 8.26 (s, 1H), 8.13 (d, *J* = 7.7 Hz, 1H), 8.06 (d, *J* = 7.6 Hz, 1H), 7.27 (t, *J* = 7.9 Hz, 2H), 6.98 (d, *J* = 8.1 Hz, 2H), 6.87 (t, *J* = 7.3 Hz, 1H), 5.20 (dd, *J* = 12.8, 5.4 Hz, 1H), 4.61 (s, 2H), 3.82 (d, *J* = 11.8 Hz, 2H), 3.39 - 3.36 (m, 1H), 3.33 - 3.06 (m, 5H), 2.98 - 2.83 (m, 1H), 2.67 - 2.53 (m, 2H), 2.17 - 2.01 (m, 1H). LCMS (ESI) calcd for $C_{24}H_{25}N_4O_4{}^+$ $[M+H]^+$: 433.19, found, 433.3.

Example 395: Preparation of 2-(2,6-dioxopiperidin-3-yl)-5-((4-(2-methoxyphenyl)piperazin-1-yl)methyl)isoindoline-1,3-dione (GT-06287)

Referring to the method of Scheme 1, the target compound (GT-06287) was prepared under appropriate conditions as would be understood by those skilled in the art (white solid, 32 mg, yield 56 %). $^1$H NMR (400 MHz, DMSO-d6) δ 11.44 (s, 1H), 11.22 (s, 1H), 8.34 (s, 1H), 8.23 (d, *J* = 7.7 Hz, 1H), 8.10 (d, *J* = 7.7 Hz, 1H), 7.12 - 7.02 (m, 2H), 7.02 - 6.91 (m, 2H), 5.25 (dd, *J* = 12.8, 5.3 Hz, 1H), 4.66 (s, 2H), 3.84 (s, 3H), 3.54 (s, 2H), 3.45 (s, 2H), 3.31 (s, 2H), 3.12 (t, *J* = 11.9 Hz, 2H), 3.03 - 2.92 (m, 1H), 2.74 - 2.59 (m, 2H), 2.19 - 2.00 (m, 1H). LCMS (ESI) $C_{25}H_{27}N_4O_5{}^+$ $[M+H]^+$: 463.20, found, 463.2.

Example 396: Preparation of 2-(2,6-dioxopiperidin-3-yl)-5-((4-(3-methoxyphenyl)piperazin-1-yl)methyl)isoindoline-1,3-dione (GT-06288)

Referring to the method of Scheme 1, the target compound (GT-06288) was prepared under appropriate conditions as would be understood by those skilled in the art (white solid, 41 mg, yield 72 %). $^1$H NMR (400 MHz, DMSO-d6) δ 11.31 (s, 1H), 11.10 (s, 1H), 8.19 (s, 1H), 8.07 (d, *J* = 7.7 Hz, 1H), 7.98 (d, *J* = *7.6* Hz, 1H), 7.09 (t, *J* = 8.2 Hz, 1H), 6.53 - 6.34 (m, 3H), 5.12 (dd, *J* = 12.8, 5.4 Hz, 1H), 4.53 (s, 2H), 3.75 (d, *J* = 8.8 Hz, 2H), 3.65 (s, 3H), 3.31 (s, 2H), 3.16 - 2.97 (m, 4H), 2.89 - 2.78 (m, 1H), 2.63 - 2.46 (m, 2H), 2.04 - 1.95 (m, 1H). LCMS (ESI) calcd for $C_{25}H_{27}N_4O_5{}^+$ $[M+H]^+$: 463.20, found, 463.2.

Example 397: Preparation of 5-((4-(3-bromophenyl)piperazin-1-yl)methyl)-2-(2,6-dioxopiperidin-3-yl)isoindoline-1,3-dione (GT-06289)

Referring to the method of Scheme 1, the target compound (GT-06289) was prepared under appropriate conditions as would be understood by those skilled in the art (white solid, 38 mg, yield 61 %). $^1$H NMR (400 MHz, DMSO-d6) δ 11.27 (s, 1H), 11.16 (s, 1H), 8.24 (s, 1H), 8.12 (d, *J* = 7.4 Hz, 1H), 8.05 (d, *J*= 7.6 Hz, 1H), 7.25 - 7.14 (m, 2H), 7.03 - 6.94 (m, 2H), 5.20 (dd, *J*= 12.7, 5.3 Hz, 1H), 4.59 (s, 2H), 3.87 (s, 2H), 3.39 (s, 2H), 3.18 (s, 4H), 2.95 - 2.83 (m, 1H), 2.68 - 2.53 (m, 2H), 2.16 - 1.96 (m, 1H). LCMS (ESI) calcd for $C_{24}H_{24}BrN_4O_4{}^+$ $[M+H]^+$: 511.10, found, 511.1.

Example 398: Preparation of 2-(2,6-dioxopiperidin-3-yl)-5-((4-(4-(trifluoromethyl)phenyl)piperazin-1-yl)methyl)isoindoline-1,3-dione (GT-06290)

Referring to the method of Scheme 1, the target compound (GT-06290) was prepared under appropriate conditions as would be understood by those skilled in the art (white solid, 45 mg, yield 74 %). $^1$H NMR (400 MHz, DMSO-d6) δ 11.16 (s, 1H), 10.95 (s, 1H), 8.20 (s, 1H), 8.06 (q, *J* = 7.8 Hz, 2H), 7.57 (d, *J* = 8.7 Hz, 2H), 7.13 (d, *J* = 8.7 Hz, 2H), 5.19 (dd, *J* = 12.7, 5.4 Hz, 1H), 4.57 (s, 2H), 4.00 (s, 2H), 3.35 - 3.15 (m, 6H), 2.97 - 2.87 (m, 1H), 2.66 - 2.53 (m, 2H), 2.12 - 2.02 (m, 1H). LCMS (ESI) calcd for $C_{25}H_{24}F_3N_4O_4^+$ [M+H]$^+$: 501.17, found, 501.2.

Example 399: Preparation of 2-(2,6-dioxopiperidin-3-yl)-5-((4-(3-fluorophenyl)piperazin-1-yl)methyl)isoindoline-1,3-dione (GT-06291)

Referring to the method of Scheme 1, the target compound (GT-06291) was prepared under appropriate conditions as would be understood by those skilled in the art (white solid, 34 mg, yield 61 %). $^1$H NMR (400 MHz, DMSO-d6) δ 11.40 (s, 1H), 11.16 (s, 1H), 8.26 (s, 1H), 8.20 - 8.10 (m, 1H), 8.05 (d, *J* = 7.7 Hz, 1H), 7.27 (dd, *J* = 16.1, 8.0 Hz, 1H), 6.85 - 6.75 (m, 2H), 6.69 - 6.56 (m, 1H), 5.19 (dd, *J* = 12.8, 5.4 Hz, 1H), 4.60 (s, 2H), 3.87 (s, 2H), 3.41 - 3.34 (m, 2H), 3.15 (s, 4H), 2.98 - 2.81 (m, 1H), 2.68 - 2.52 (m, 2H), 2.11 - 1.93 (m, 1H). LCMS (ESI) calcd for $C_{24}H_{24}FN_4O_4^+$ [M+H]$^+$: 451.18, found, 451.1.

Example 400: Preparation of 2-(2,6-dioxopiperidin-3-yl)-5-((4-(4-fluorophenyl)piperazin-1-yl)methyl)isoindoline-1,3-dione (GT-06292)

Referring to the method of Scheme 1, the target compound (GT-06292) was prepared under appropriate conditions as would be understood by those skilled in the art (white solid, 30 mg, yield 54 %). $^1$H NMR (400 MHz, DMSO-d6) δ 11.16 (s, 2H), 8.25 (s, 1H), 8.13 (d, *J* = 7.5 Hz, 1H), 8.05 (d, *J* = 7.6 Hz, 1H), 7.10 (t, *J* = 8.8 Hz, 2H), 7.04 - 6.93 (m, 2H), 5.19 (dd, *J* = 12.8, 5.4 Hz, 1H), 4.60 (s, 2H), 3.71 (s, 2H), 3.24 (d, *J* = 58.9, 25.4 Hz, 6H), 2.98 - 2.87 (m, 1H), 2.70 - 2.53 (m, 2H), 2.13 - 2.02 (m, 1H). LCMS (ESI) calcd for $C_{24}H_{24}FN_4O_4^+$ [M+H]$^+$: 451.18, found, 451.2.

Example 401: Preparation of 5-((4-(2-bromophenyl)piperazin-1-yl)methyl)-2-(2,6-dioxopiperidin-3-yl)isoindoline-1,3-dione (GT-06293)

Referring to the method of Scheme 1, the target compound (GT-06293) was prepared under appropriate conditions as would be understood by those skilled in the art (white solid, 33 mg, yield 53 %). $^1$H NMR (400 MHz, DMSO) δ 11.21 - 10.89 (m, 2H), 8.26 (s, 1H), 8.14 (d, *J* = 7.2 Hz, 1H), 8.05 (d, *J* = 7.6 Hz, 1H), 7.63 (dd, *J = 7.9,* 1.4 Hz, 1H), 7.42 - 7.30 (m, 1H), 7.21 (d, *J* = 8.0, 1.4 Hz, 1H), 7.13 - 7.00 (m, 1H), 5.19 (dd, *J* = 12.8, 5.4 Hz, 1H), 4.63 (s, 2H), 3.44 (s, 2H), 3.32 - 3.27 (m, 2H), 3.17 (s, 4H), 2.97 - 2.82 (m, 1H), 2.69 - 2.53 (m, 2H), 2.14 - 2.01 (m, 1H). LCMS (ESI) calcd for $C_{24}H_{24}BrN_4O_4^+$ [M+H]$^+$: 511.10, found, 511.1.

Example 402: Preparation of 2-(2,6-dioxopiperidin-3-yl)-5-((4-(4-methoxyphenyl)piperazin-1-yl)methyl)isoindoline-1,3-dione (GT-06294)

Referring to the method of Scheme 1, the target compound (GT-06294) was prepared under appropriate conditions as would be understood by those skilled in the art (white solid, 38 mg, yield 67 %). $^1$H NMR (400 MHz, DMSO-d6) δ 11.22 (s, 2H), 8.33 (s, 1H), 8.21 (d, *J* = 7.8 Hz, 1H), 8.11 (d, *J* = 7.6 Hz, 1H), 6.96 (dd, *J* = 36.1, 9.1 Hz, 4H), 5.25 (dd, *J* = 12.8, 5.4 Hz, 1H), 4.66 (s, 2H), 3.75 (s, 4H), 3.23 (d, *J* = 49.8 Hz, 4H), 3.02 - 2.89 (m, 1H), 2.74 - 2.58 (m, 2H), 2.23 - 2.04 (m, 1H). LCMS (ESI) calcd for $C_{25}H_{27}N_4O_5{}^+$ [M+H]$^+$: 463.20, found, 463.2.

Example 403: Preparation of 5-((4-(4-bromophenyl)piperazin-1-yl)methyl)-2-(2,6-dioxopiperidin-3-yl)isoindoline-1,3-dione (GT-06295)

Referring to the method of Scheme 1, the target compound (GT-06295) was prepared under appropriate conditions as would be understood by those skilled in the art (yellow solid, 47 mg, yield 75 %). $^1$H NMR (400 MHz, DMSO-d6) δ 11.16 (s, 1H), 10.91 (s, 1H), 8.21 (s, 1H), 8.07 (q, *J* = 7.7 Hz, 2H), 7.41 (d, *J* = 8.9 Hz, 2H), 6.95 (d, *J* = 9.0 Hz, 2H), 5.19 (dd, *J* = 12.7, 5.4 Hz, 1H), 4.58 (s, 2H), 3.81 (s, 2H), 3.34 - 3.28 (m, 2H), 3.24 - 3.03 (m, 4H), 2.96 - 2.84 (m, 1H), 2.69 - 2.53 (m, 2H), 2.16 - 2.00 (m, 1H). LCMS (ESI) calcd for $C_{24}H_{24}BrN_4O_4{}^+$ [M+H]$^+$: 511.10, found, 511.1.

Example 404: Preparation of 2-(2,6-dioxopiperidin-3-yl)-5-((4-(o-tolyl)piperazin-1-yl)methyl)isoindoline-1,3-dione (GT-06296)

Referring to the method of Scheme 1, the target compound (GT-06296) was prepared under appropriate conditions as would be understood by those skilled in the art (white solid, 23 mg, yield 42 %). $^1$H NMR (400 MHz, DMSO-d6) δ 11.26 (d, *J* = 30.6 Hz, 2H), 8.34 (s, 1H), 8.23 (d, *J* = 7.7 Hz, 1H), 8.11 (d, *J* = 7.6 Hz, 1H), 7.24 (t, *J* = 8.1 Hz, 2H), 7.12 - 7.01 (m, 2H), 5.25 (dd, *J* = 12.8, 5.4 Hz, 1H), 4.68 (s, 2H), 3.48 - 3.43 (m, 2H), 3.33 (s, 2H), 3.27 - 3.14 (m, 4H), 3.04 - 2.90 (m, 1H), 2.73 - 2.58 (m, 2H), 2.31 (s, 3H), 2.21 - 2.05 (m, 1H). LCMS (ESI) calcd for $C_{25}H_{27}N_4O_4$ $^+$ [M+H]$^+$: 447.20, found, 447.2.

Example 405: Preparation of 5-((4-(2,6-dichlorophenyl)piperazin-1-yl)methyl)-2-(2,6-dioxopiperidin-3-yl)isoindoline-1,3-dione (GT-06297)

Referring to the method of Scheme 1, the target compound (GT-06297) was prepared under appropriate conditions as would be understood by those skilled in the art (white solid, 41 mg, yield 66.87 %). $^1$H NMR (400 MHz, DMSO-d6) δ 11.09 (s, 1H), 10.87 (s, 1H), 8.19 (s, 1H), 8.07 (d, *J* = 7.4 Hz, 1H), 7.98 (d, *J* = 7.6 Hz, 1H), 7.40 (d, *J* = 28.4 Hz, 2H), 7.16 (t, *J* = 8.1 Hz, 1H), 5.12 (dd, *J* = 12.8, 5.3 Hz, 1H), 4.56 (s, 2H), 3.68 (s, 2H), 3.29 - 3.22 (m, 2H), 3.12 (d, *J* = 30.1 Hz, 4H), 2.90 - 2.76 (m, 1H), 2.61 - 2.45 (m, 2H), 2.06 - 1.93 (m, 1H). LCMS (ESI) calcd for $C_{24}H_{23}Cl_2N_4O_4^+$ [M+H]$^+$: 501.11, found, 501.1.

Example 406: Preparation of 5-((4-(3,4-dichlorophenyl)piperazin-1-yl)methyl)-2-(2,6-dioxopiperidin-3-yl)isoindoline-1,3-dione (GT-06298)

Referring to the method of Scheme 1, the target compound (GT-06298) was prepared under appropriate conditions as would be understood by those skilled in the art (white solid, 44 mg, yield 72 %). $^1$H NMR (400 MHz, DMSO-d6) δ 11.16 (s, 1H), 10.89 (s, 1H), 8.19 (s, 1H), 8.06 (s, 2H), 7.46 (d, *J* = 9.0 Hz, 1H), 7.23 (d, *J* = 2.6 Hz, 1H), 6.99 (dd, *J* = 9.0, 2.8 Hz, 1H), 5.19 (dd, *J* = 12.7, 5.4 Hz, 1H), 4.56 (s, 2H), 4.02 - 3.69 (m, 2H), 3.35 - 3.31 (m, 2H), 3.26 - 3.10 (m, 4H), 2.98 - 2.87 (m, 1H), 2.71 - 2.53 (m, 2H), 2.15 - 1.97 (m, 1H). LCMS (ESI) calcd for $C_{24}H_{23}Cl_2N_4O_4$ $^+$ [M+H]$^+$: 501.11, found, 501.1.

Example 407: Preparation of 5-((4-(3,5-dichlorophenyl)piperazin-1-yl)methyl)-2-(2,6-dioxopiperidin-3-yl)isoindoline-1,3-dione (GT-06299)

Referring to the method of Scheme 1, the target compound (GT-06299) was prepared under appropriate conditions as would be understood by those skilled in the art (white solid, 39 mg, yield 64%). $^1$H NMR (400 MHz, DMSO-d6) δ 11.09 (s, 1H), 11.07 - 10.75 (m, 1H), 8.11 (s, 1H), 8.05 - 7.88 (m, 2H), 6.95 (s, 2H), 6.88 (s, 1H), 5.12 (dd, *J* = 12.7, 5.4 Hz, 1H), 4.54 (s, 2H), 3.86 (s, 2H), 3.27 - 3.20 (m, 2H), 3.19 - 2.91 (m, 4H), 2.86 - 2.78 (m, 1H), 2.68 - 2.47 (m, 2H), 2.05 - 1.95 (m, 1H). LCMS (ESI) calcd for $C_{24}H_{23}Cl_2N_4O_4$ $^+$ [M+H]$^+$: 501.11, found, 501.1.

Example 408: Preparation of 5-((4-(2,3-difluorophenyl)piperazin-1-yl)methyl)-2-(2,6-dioxopiperidin-3-yl)isoindoline-1,3-dione (GT-06300)

Referring to the method of Scheme 1, the target compound (GT-06300) was prepared under appropriate conditions as would be understood by those skilled in the art (white solid, 27 mg, yield 47%). $^1$H NMR (400 MHz, DMSO-d6) δ 11.16 (s, 1H), 11.05 (s, 1H), 8.23 (s, 1H), 8.08 (dd, *J* = 22.9, 7.7 Hz, 2H), 7.21 - 6.99 (m, 2H), 6.93 (t, *J* = 7.9 Hz, 1H), 5.19 (dd, *J* = 12.8, 5.4 Hz, 1H), 4.60 (s, 2H), 3.62 - 3.50 (m, 2H), 3.30 - 3.09 (m, 4H), 2.96 - 2.82 (m, 1H), 2.66 - 2.53 (m, 2H), 2.15 - 2.02 (m, 1H). LCMS (ESI) calcd for $C_{24}H_{23}F_2N_4O_4^+$ [M+H]$^+$: 469.17, found, 469.2.

Example 409: Preparation of 5-((4-(2,4-difluorophenyl)piperazin-1-yl)methyl)-2-(2,6-dioxopiperidin-3-yl)isoindoline-1,3-dione (GT-06361)

Referring to the method of Scheme 1, the target compound (GT-06361) was prepared under appropriate conditions as would be understood by those skilled in the art (white solid, 29 mg, yield 50 %). $^1$H NMR (400 MHz, DMSO) δ 11.16 (s, 2H), 8.27 - 8.17 (m, 1H), 8.13 (d, 1H), 8.05 (d, *J* = 7.6 Hz, 1H), 7.31 - 7.21 (m, 1H), 7.19 - 7.10 (m, 1H), 7.04 (dd, *J* = 11.7, 5.4 Hz, 1H), 5.19 (dd, *J* = 12.7, 5.4 Hz, 1H), 4.61 (s, 2H), 3.47 (s, 2H), 3.35 - 3.23 (m, 4H), 3.17 (s, 2H), 2.96 - 2.83 (m, 1H), 2.70 - 2.53 (m, 2H), 2.13 - 2.02 (m, 1H). LCMS (ESI) calcd for $C_{24}H_{23}F_2N_4O_4$ $^+$ [M+H]$^+$: 469.17, found, 469.2.

Example 410: Preparation of 5-((4-(2,5-difluorophenyl)piperazin-1-yl)methyl)-2-(2,6-dioxopiperidin-3-yl)isoindoline-1,3-dione (GT-06362)

Referring to the method of Scheme 1, the target compound (GT-06362) was prepared under appropriate conditions as would be understood by those skilled in the art (white solid, 32 mg, yield 56%). $^1$H NMR (400 MHz, DMSO-d6) δ 11.16 (s, 1H), 10.86 (s, 1H), 8.29 - 8.13 (m, 1H), 8.07 (q, *J* = 7.7 Hz, 2H), 7.33 - 7.12 (m, 1H), 7.08 - 6.91 (m, 1H), 6.89 - 6.68 (m, 1H), 5.19 (dd, *J* = 12.7, 5.4 Hz, 1H), 4.58 (s, 2H), 3.60 - 3.49 (m, 2H), 3.32 - 3.27 (m, 2H), 3.19 (s, 4H), 2.96 - 2.85 (m, 1H), 2.69 - 2.53 (m, 2H), 2.14 - 2.02 (m, 1H). LCMS (ESI) calcd for $C_{24}H_{23}F_2N_4O_4^+$ [M+H]$^+$: 469.17, found, 469.2.

Example 411: Preparation of 5-((4-(2,6-difluorophenyl)piperazin-1-yl)methyl)-2-(2,6-dioxopiperidin-3-yl)isoindoline-1,3-dione (GT-06363)

Referring to the method of Scheme 1, the target compound (GT-06363) was prepared under appropriate conditions as would be understood by those skilled in the art (white solid, 28 mg, yield 49%). $^1$H NMR (400 MHz, DMSO-d6) δ 11.16 (s, 1H), 10.99 (s, 1H), 8.27 - 8.18 (m, 1H), 8.12 (d, *J* = 7.7 Hz, 1H), 8.05 (d, *J* = 7.6 Hz, 1H), 7.23 - 7.13 (m, 1H), 7.12 - 6.97 (m, 2H), 5.19 (dd, *J* = 12.7, 5.4 Hz, 1H), 4.60 (s, 2H), 3.55 - 3.47 (m, 2H), 3.36 - 3.33 (m, 2H), 3.31 - 3.10 (m, 4H), 2.94 - 2.84 (m, 1H), 2.66 - 2.52 (m, 2H), 2.13 - 2.03 (m, 1H). LCMS (ESI) calcd for $C_{24}H_{23}F_2N_4O_4^+$ [M+H]$^+$: 469.17, found, 469.2.

Example 412: Preparation of 5-((4-(3,4-difluorophenyl)piperazin-1-yl)methyl)-2-(2,6-dioxopiperidin-3-yl)isoindoline-1,3-dione (GT-06364)

Referring to the method of Scheme 1, the target compound (GT-06364) was prepared under appropriate conditions as would be understood by those skilled in the art (white solid, 31 mg, yield 54%). $^1$H NMR (400 MHz, DMSO-d6) δ 11.36 (s, 1H), 11.16 (s, 1H), 8.26 (s, 1H), 8.14 (d, *J* = 7.8 Hz, 1H), 8.08 - 8.00 (m, 1H), 7.39 - 7.21 (m, 1H), 7.08 (ddd, *J* = 13.9, 6.9, 2.9 Hz, 1H), 6.78 (d, *J* = 8.9 Hz, 1H), 5.19 (dd, *J* = 12.8, 5.4 Hz, 1H), 4.59 (s, 2H), 3.79 (s, 2H), 3.42 - 3.37 (m, 2H), 3.18 (s, 4H), 2.95 - 2.85 (m, 1H), 2.68 - 2.52 (m, 2H), 2.13 - 2.01 (m, 1H). LCMS (ESI) calcd for $C_{24}H_{23}F_2N_4O_4^+$ [M+H]$^+$: 469.17, found, 469.2.

Example 413: Preparation of 5-((4-(3,5-difluorophenyl)piperazin-1-yl)methyl)-2-(2,6-dioxopiperidin-3-yl)isoindoline-1,3-dione (GT-06365)

Referring to the method of Scheme 1, the target compound (GT-06365) was prepared under appropriate conditions as would be understood by those skilled in the art (white solid, 42 mg, yield 73%). $^1$H NMR (400 MHz, DMSO-d6) δ 11.16 (s, 1H), 11.12 - 10.74 (m, 1H), 8.20 (s, 1H), 8.06 (q, *J* = 7.7 Hz, 2H), 6.71 (d, *J* = 9.2 Hz, 2H), 6.58 (t, *J* = 9.2 Hz, 1H), 5.19 (dd, *J* = 12.7, 5.4 Hz, 1H), 4.55 (s, 2H), 3.91 (s, 2H), 3.32 - 3.26 (m, 2H), 3.26 - 3.07 (m, 4H), 2.97 - 2.87 (m, 1H), 2.69 - 2.53 (m, 2H), 2.13 - 2.02 (m, 1H). LCMS (ESI) calcd for $C_{24}H_{23}F_2N_4O_4^+$ [M+H]$^+$: 469.17, found, 469.2.

Example 414: Preparation of 5-((4-(2,4-dimethylphenyl)piperazin-1-yl)methyl)-2-(2,6-dioxopiperidin-3-yl)isoindoline-1,3-dione (GT-06366)

Referring to the method of Scheme 1, the target compound (GT-06366) was prepared under appropriate conditions as would be understood by those skilled in the art (white solid, 36 mg, yield 64%). $^1$H NMR (400 MHz, DMSO-d6) δ 11.16 (s, 1H), 11.07 (s, 1H), 8.27 (s, 1H), 8.15 (d, *J* = 8.6 Hz, 1H), 8.05 (d, *J* = 7.7 Hz, 1H), 7.02 - 6.95 (m, 2H), 6.92 (d, *J* = 8.0 Hz, 1H), 5.19 (dd, *J* = 12.8, 5.4 Hz, 1H), 4.62 (s, 2H), 3.61 - 3.52 (m, 2H), 3.26 (s, 2H), 3.15 - 3.02 (m, 4H), 2.96 - 2.84 (m, 1H), 2.72 - 2.53 (m, 2H), 2.21 (s, 6H), 2.12 - 2.03 (m, 1H). LCMS (ESI) calcd for $C_{26}H_{29}N_4O_4{}^+$ [M+H]$^+$: 461.22, found, 461.3.

Example 415: Preparation of 5-((4-(5-chloro-2-methylphenyl)piperazin-1-yl)methyl)-2-(2,6-dioxopiperidin-3-yl)isoindoline-1,3-dione (GT-06367)

Referring to the method of Scheme 1, the target compound (GT-06367) was prepared under appropriate conditions as would be understood by those skilled in the art (white solid, 31 mg, yield 53%). $^1$H NMR (400 MHz, DMSO-d6) δ 11.16 (s, 2H), 8.26 (s, 1H), 8.15 (d, *J* = 7.7 Hz, 1H), 8.05 (d, *J* = 7.6 Hz, 1H), 7.22 (d, *J* = 8.1 Hz, 1H), 7.10 - 7.02 (m, 2H), 5.19 (dd, *J* = 12.8, 5.4 Hz, 1H), 4.62 (s, 2H), 3.35 - 3.30 (m, 2H), 3.30 - 3.18 (m, 4H), 3.17 - 3.09 (m, 2H), 2.94 - 2.84 (m, 1H), 2.67 - 2.52 (m, 2H), 2.23 (s, 3H), 2.14 - 2.04 (m, 1H). LCMS (ESI) calcd for $C_{25}H_{26}ClN_4O_4{}^+$ [M+H]$^+$: 481.16, found, 481.2.

Example 416: Preparation of 2-(2,6-dioxopiperidin-3-yl)-5-((4-phenylpiperidin-1-yl)methyl)isoindoline-1,3-dione (GT-06368)

Referring to the method of Scheme 1, the target compound (GT-06368) was prepared under appropriate conditions as would be understood by those skilled in the art (white solid, 32 mg, yield 60%). $^1$H NMR (400 MHz, DMSO-d6) δ 11.16 (s, 1H), 10.72 (s, 1H), 8.29 - 8.20 (m, 1H), 8.12 (d, *J* = 7.8 Hz, 1H), 8.05 (d, *J* = 7.6 Hz, 1H), 7.36 - 7.29 (m, 2H), 7.29 - 7.21 (m, 3H), 5.19 (dd, *J* = 12.8, 5.3 Hz, 1H), 4.54 (s, 2H), 3.53 - 3.46 (m, 2H), 3.17 - 3.05 (m, 2H), 2.95 - 2.78 (m, 2H), 2.66 - 2.52 (m, 2H), 2.03 (dt, *J* = 28.0, 9.2 Hz, 5H). LCMS (ESI) calcd for $C_{25}H_{26}N_3O_4$ $^+$ [M+H]$^+$: 432.19, found, 432.2.

Example 417: Preparation of 5-((4-(3-bromophenyl)piperidin-1-yl)methyl)-2-(2,6-dioxopiperidin-3-yl)isoindoline-1,3-dione (GT-06369)

Referring to the method of Scheme 1, the target compound (GT-06369) was prepared under appropriate conditions as would be understood by those skilled in the art (white solid, 38 mg, yield 61%). $^1$H NMR (400 MHz, DMSO) δ 11.16 (s, 1H), 10.44 (s, 1H), 8.27 - 8.15 (m, 1H), 8.08 (q, *J* = 16.4, 7.7 Hz, 2H), 7.49 - 7.39 (m, 2H), 7.31 (t, *J* = 7.7 Hz, 1H), 7.25 (d, *J* = 7.7 Hz, 1H), 5.19 (dd, *J* = 12.8, 5.4 Hz, 1H), 4.55 (s, 2H), 3.48 - 3.46 (m, 2H), 3.08 (s, 2H), 2.95 - 2.82 (m, 2H), 2.67 - 2.54 (m, 2H), 2.13 - 2.04 (m, 1H), 2.04 - 1.84 (m, 4H). LCMS (ESI) calcd for $C_{25}H_{25}BrN_3O_4$ $^+$ [M+H]$^+$: 510.10, found, 510.1.

Example 418: Preparation of 5-((4-(4-bromophenyl)piperidin-1-yl)methyl)-2-(2,6-dioxopiperidin-3-yl)isoindoline-1,3-dione (GT-06370)

Referring to the method of Scheme 1, the target compound (GT-06370) was prepared under appropriate conditions as would be understood by those skilled in the art (white solid, 35 mg, yield 56%). $^1$H NMR (400 MHz, DMSO-d6) δ 11.16 (s, 1H), 10.78 (s, 1H), 8.22 (s, 1H), 8.12 (d, *J* = 7.7 Hz, 1H), 8.06 (d, *J* = 7.7 Hz, 1H), 7.53 (d, *J* = 8.4 Hz, 2H), 7.20 (d, 2H), 5.20 (dd, *J* = 12.8, 5.4 Hz, 1H), 4.55 (d, *J* = 4.2 Hz, 2H), 3.47 (d, *J* = 11.5 Hz, 2H), 3.07 (d, *J* = 11.1 Hz, 2H), 2.97 - 2.78 (m, 2H), 2.66 - 2.53 (m, 2H), 2.14 - 2.01 (m, 2H), 1.97 (s, 3H), 1.35 - 1.22 (m, 1H). LCMS (ESI) calcd for $C_{25}H_{25}BrN_3O_4$ $^+$ [M+H]$^+$: 510.10, found, 510.1.

Example 419: Preparation of 5-((4-(2-chlorophenyl)piperidin-1-yl)methyl)-2-(2,6-dioxopiperidin-3-yl)isoindoline-1,3-dione (GT-06371)

Referring to the method of Scheme 1, the target compound (GT-06371) was prepared under appropriate conditions as would be understood by those skilled in the art (white solid, 36 mg, yield 63%). $^1$H NMR (400 MHz, DMSO-d6) δ 11.16 (s, 1H), 10.83 (s, 1H), 8.24 (s, 1H), 8.09 (dd, *J* = 22.2, 7.6 Hz, 2H), 7.46 (d, *J* = 7.9 Hz, 1H), 7.40 - 7.23 (m, 3H), 5.20 (dd, *J* = 12.8, 5.3 Hz, 1H), 4.55 (d, *J* = 4.3 Hz, 2H), 3.49 (d, *J* = 10.9 Hz, 2H), 3.27 - 3.14 (m, 3H), 2.96 - 2.84 (m, 1H), 2.71 - 2.53 (m, 2H), 2.16 - 2.00 (m, 3H), 2.00 - 1.90 (m, 2H). LCMS (ESI) calcd for $C_{25}H_{25}ClN_3O_4^+$ [M+H]$^+$: 466.15, found, 466.2.

Example 420: Preparation of 5-((4-(4-chlorophenyl)piperidin-1-yl)methyl)-2-(2,6-dioxopiperidin-3-yl)isoindoline-1,3-dione (GT-06372)

Referring to the method of Scheme 1, the target compound (GT-06372) was prepared under appropriate conditions as would be understood by those skilled in the art (white solid, 32 mg, yield 56%). $^1$H NMR (400 MHz, DMSO-d6) δ 11.16 (s, 1H), 10.79 (s, 1H), 8.25 (s, 1H), 8.12 (d, *J* = 7.6 Hz, 1H), 8.06 (d, *J* = 7.6 Hz, 1H), 7.42 (d, 2H), 7.26 (d, *J* = 8.5 Hz, 2H), 5.20 (dd, *J*= 12.9, 5.3 Hz, 1H), 4.55 (d, *J* = 4.5 Hz, 2H), 3.47 (d, *J* = 11.5 Hz, 2H), 3.15 - 2.98 (m, 2H), 2.98 - 2.75 (m, 2H), 2.70 - 2.53 (m, 2H), 2.14 - 1.84 (m, 5H). LCMS (ESI) calcd for $C_{25}H_{25}ClN_3O_4$ $^+$ [M+H]$^+$: 466.15, found, 466.2.

Example 421: Preparation of 2-(2,6-dioxopiperidin-3-yl)-5-((4-(2-fluorophenyl)piperidin-1-yl)methyl)isoindoline-1,3-dione (GT-06373)

Referring to the method of Scheme 1, the target compound (GT-06373) was prepared under appropriate conditions as would be understood by those skilled in the art (white solid, 29 mg, yield 52%). $^1$H NMR (400 MHz, DMSO-d6) δ 11.16 (s, 1H), 10.81 (s, 1H), 8.24 (s, 1H), 8.12 (d, *J* = 7.8 Hz, 1H), 8.05 (d, *J* = 7.6 Hz, 1H), 7.38 - 7.25 (m, 2H), 7.24 - 7.09 (m, 2H), 5.20 (dd, *J* = 12.8, 5.3 Hz, 1H), 4.55 (d, *J* = 4.2 Hz, 2H), 3.48 (d, *J* = 11.5 Hz, 2H), 3.21 - 3.05 (m, 3H), 2.97 - 2.87 (m, 1H), 2.67 - 2.53 (m, 2H), 2.16 - 2.03 (m, 3H), 1.94 (d, *J* = 12.8 Hz, 2H). LCMS (ESI) calcd for $C_{25}H_{25}FN_3O_4^+$ [M+H]$^+$: 450.18, found, 450.3.

Example 422: Preparation of 2-(2,6-dioxopiperidin-3-yl)-5-((4-(4-fluorophenyl)piperidin-1-yl)methyl)isoindoline-1,3-dione (GT-06374)

Referring to the method of Scheme 1, the target compound (GT-06374) was prepared under appropriate conditions as would be understood by those skilled in the art (white solid, 19 mg, yield 34%). $^1H$ NMR (400 MHz, DMSO-d6) δ 11.16 (s, 1H), 10.74 (s, 1H), 8.25 (s, 1H), 8.12 (d, *J* = 7.6 Hz, 1H), 8.06 (d, *J* = 7.7 Hz, 1H), 7.27 (q, *J* = 8.7, 5.6 Hz, 2H), 7.16 (t, *J* = 8.8 Hz, 2H), 5.20 (dd, *J* = 12.8, 5.4 Hz, 1H), 4.55 (d, *J* = 4.5 Hz, 2H), 3.47 (d, *J* = 11.3 Hz, 2H), 3.14 - 3.00 (m, 2H), 2.97 - 2.78 (m, 2H), 2.67 - 2.54 (m, 2H), 2.12 - 1.87 (m, 5H). LCMS (ESI) calcd for $C_{25}H_{25}FN_3O_4{}^+$ [M+H]$^+$: 450.18, found, 450.2.

Example 423: Preparation of 2-(2,6-dioxopiperidin-3-yl)-5-((4-(2-(trifluoromethyl)phenyl)piperidin-1-yl)methyl)isoindoline-1,3-dione (GT-06375)

Referring to the method of Scheme 1, the target compound (GT-06375) was prepared under appropriate conditions as would be understood by those skilled in the art (white solid, 33 mg, yield 54%). $^1H$ NMR (400 MHz, DMSO-d6) δ 11.08 (s, 1H), 10.69 (s, 1H), 8.16 (s, 1H), 8.02 (dd, *J* = 27.0, 7.6 Hz, 2H), 7.60 - 7.42 (m, 4H), 5.12 (dd, *J* = 12.9, 5.3 Hz, 1H), 4.49 (d, *J* = 4.0 Hz, 2H), 3.38 (d, *J* = 30.6, 7.3 Hz, 2H), 3.06 - 2.92 (m, 2H), 2.93 - 2.74 (m, 2H), 2.58 - 2.45 (m, 2H), 2.07 - 1.76 (m, 5H). LCMS (ESI) calcd for $C_{26}H_{25}F_3N_3O_4{}^+$ [M+H]$^+$: 500.18, found, 500.2.

Example 424: Preparation of 5-((4-(3-chloro-5-(trifluoromethyl)pyridin-2-yl)piperazin-1-yl)methyl)-2-(2,6-dioxopiperidin-3-yl)isoindoline-1,3-dione (GT-06548)

Referring to the method of Scheme 1, the target compound (GT-06548) was prepared under appropriate conditions as would be understood by those skilled in the art (white solid, 28 mg, yield 43%). $^1H$ NMR (400 MHz, DMSO-d6) δ 11.45 (s, 1H), 11.16 (s, 1H), 8.63 (s, 1H), 8.28 (d, *J* = 21.1 Hz, 2H), 8.09 (dd, *J* = 28.9, 7.6 Hz, 2H), 5.20 (dd, *J* = 12.8, 5.3 Hz, 1H), 4.60 (s, 2H), 4.17 - 4.03 (m, 2H), 3.74 - 3.59 (m, 3H), 3.26 (s, 2H), 2.98 - 2.88 (m, 1H), 2.68 - 2.53 (m, 3H), 2.13 - 2.03 (m, 1H). LCMS (ESI) calcd for $C_{24}H_{22}ClF_3N_5O_4{}^+$ [M+H]$^+$: 536.13, found, 536.2.

Example 425: Preparation of 2-(2,6-dioxopiperidin-3-yl)-5-((4-(pyridin-3-yl)piperazin-1-yl)methyl)isoindoline-1,3-dione (GT-06549)

Referring to the method of Scheme 1, the target compound (GT-06549) was prepared under appropriate conditions as would be understood by those skilled in the art (white solid, 3.6 mg, yield 7%). $^1H$ NMR (400 MHz, DMSO-d6) δ 11.90 (s, 1H), 11.16 (s, 1H), 8.52 *(d, J=* 2.6 Hz, 1H), 8.26 (s, 2H), 8.14 (d, *J* = 7.7 Hz, 1H), 8.10 - 7.98 (m, 2H), 7.81 (dd, *J=* 8.8, 5.4 Hz, 1H), 5.20 (dd, *J=* 12.7, 5.3 Hz, 1H), 4.58 (s, 2H), 4.07 (s, 2H), 3.80 - 3.66 (m, 3H), 3.25 - 3.20 (m, 2H), 2.96 - 2.87 (m, 1H), 2.69 - 2.53 (m, 3H), 2.11 - 2.04 (m, 1H). LCMS (ESI) calcd for $C_{23}H_{24}N_5O_4{}^+$ [M+H]$^+$: 434.18, found, 434.2.

Example 426: Preparation of 5-((4-(6-chloropyridin-3-yl)piperazin-1-yl)methyl)-2-(2,6-dioxopiperidin-3-yl)isoindoline-1,3-dione (GT-06550)

Referring to the method of Scheme 1, the target compound (GT-06550) was prepared under appropriate conditions as would be understood by those skilled in the art (white solid, 29 mg, yield 50%). $^1H$ NMR (400 MHz, DMSO-d6) δ 11.36 (s, 1H), 11.16 (s, 1H), 8.24 (s, 1H), 8.09 (dd, *J* = 26.2, 9.3 Hz, 3H), 7.49 (d, *J* = 8.8 Hz, 1H), 7.37 (d, *J* = 8.8 Hz, 1H), 5.20 (dd, *J* = 12.7, 5.0 Hz, 1H), 4.59 (s, 2H), 3.90 (s, 2H), 3.73 - 3.65 (m, 2H), 3.29 - 3.15 (m, 4H), 2.94 - 2.85 (m, 1H), 2.66 - 2.56 (m, 2H), 2.15 - 2.01 (m, 1H). LCMS (ESI) calcd for $C_{23}H_{23}ClN_5O_4{}^+$ [M+H]$^+$: 468.14, found, 468.2.

Example 427: Preparation of 5-((4-(4,5-dichloropyridin-3-yl)piperazin-1-yl)methyl)-2-(2,6-dioxopiperidin-3-yl)isoindoline-1,3-dione (GT-06551)

Referring to the method of Scheme 1, the target compound (GT-06551) was prepared under appropriate conditions as would be understood by those skilled in the art (white solid, 29 mg, yield 47%). $^1H$ NMR (400 MHz, DMSO-d6) δ 11.64 (s, 1H), 11.16 (s, 1H), 8.52 (s, 1H), 8.41 (s, 1H), 8.29 (s, 1H), 8.19 - 8.12 (m, 1H), 8.08 - 8.00 (m, 1H), 5.20 (dd, *J=* 12.8, 5.3 Hz, 1H), 4.64 (s, 2H), 3.56 - 3.53 (m, 2H), 3.45 (d, *J* = 10.2 Hz, 2H), 3.41 - 3.26 (m, 4H), 2.98 - 2.84 (m, 1H), 2.67 - 2.53 (m, 2H), 2.13 - 2.02 (m, 1H). LCMS (ESI) calcd for $C_{23}H_{22}Cl_2N_5O_4{}^+$ [M+H]$^+$: 502.10, found, 502.1.

Example 428: Preparation of 5-((4-(2,4-dichloropyridin-3-yl)piperazin-1-yl)methyl)-2-(2,6-dioxopiperidin-3-yl)isoindoline-1,3-dione (GT-06552)

Referring to the method of Scheme 1, the target compound (GT-06552) was prepared under appropriate

conditions as would be understood by those skilled in the art (white solid, 25 mg, yield 41%). $^1H$ NMR (400 MHz, DMSO-d6) δ 11.16 (s, 2H), 8.29 (s, 1H), 8.22 - 8.13 (m, 2H), 8.06 (d, *J* = 7.6 Hz, 1H), 7.63 (d, *J* = 21.0 Hz, 1H), 5.20 (dd, *J* = 12.8, 5.4 Hz, 1H), 4.64 (s, 2H), 3.74 (s, 2H), 3.27 - 3.16 (m, 4H), 2.95 - 2.84 (m, 1H), 2.66 - 2.53 (m, 3H), 2.13 - 2.02 (m, 1H), 1.30 - 1.26 (m, 1H). LCMS (ESI) calcd for $C_{23}H_{22}Cl_2N_5O_4{}^+$ $[M+H]^+$: 502.10, found, 502.2.

Example 429: Preparation of 2-(2,6-dioxopiperidin-3-yl)-5-((4-(pyridin-4-yl)piperazin-1-yl)methyl)isoindoline-1,3-dione (GT-06553)

Referring to the method of Scheme 1, the target compound (GT-06553) was prepared under appropriate conditions as would be understood by those skilled in the art (white solid, 18 mg, yield 34%). $^1H$ NMR (400 MHz, DMSO-d6) δ 14.14 (s, 1H), 11.16 (s, 1H), 9.46 (s, 1H), 8.36 (d, *J* = 7.2 Hz, 1H), 8.27 (s, 1H), 8.15 (d, *J* = 7.8 Hz, 1H), 8.03 (d, 1H), 7.28 (t, *J* = 12.8 Hz, 2H), 5.19 (dd, *J* = 12.8, 5.4 Hz, 1H), 4.50 (d, *J* = 70.8 Hz, 3H), 3.64 - 3.53 (m, 4H), 3.14 - 3.06 (m, 3H), 2.98 - 2.83 (m, 1H), 2.69 - 2.53 (m, 2H), 2.14 - 1.95 (m, 1H). LCMS (ESI) calcd for $C_{23}H_{24}N_5O_4{}^+$ $[M+H]^+$: 434.18, found, 434.2.

Example 430: Preparation of 2-(2,6-dioxopiperidin-3-yl)-5-((4-(2-methylpyridin-4-yl)piperazin-1-yl)methyl)isoindoline-1,3-dione (GT-06554)

Referring to the method of Scheme 1, the target compound (GT-06554) was prepared under appropriate conditions as would be understood by those skilled in the art (white solid, 28 mg, yield 51%). $^1H$ NMR (400 MHz, DMSO-d6) δ 14.06 (s, 1H), 11.16 (s, 1H), 8.25 (s, 2H), 8.13 (d, *J* = 7.6 Hz, 1H), 8.04 (d, *J* = 7.6 Hz, 1H), 7.19 (s, 1H), 7.16 - 7.09 (m, 1H), 5.19 (dd, *J* = 12.7, 5.4 Hz, 1H), 4.47 (d, *J* = 73.7 Hz, 4H), 3.68 (s, 6H), 3.33 - 3.13 (m, 3H), 2.97 - 2.86 (m, 1H), 2.67 - 2.55 (m, 2H), 2.15 - 1.97 (m, 1H). LCMS (ESI) calcd for $C_{24}H_{26}N_5O_4{}^+$ $[M+H]^+$: 448.20, found, 448.2.

Example 431: Preparation of 5-((4-(3,4-difluoropyridin-2-yl)piperazin-1-yl)methyl)-2-(2,6-dioxopiperidin-3-yl)isoindoline-1,3-dione (GT-06555)

Referring to the method of Scheme 1, the target compound (GT-06555) was prepared under appropriate conditions as would be understood by those skilled in the art (white solid, 30 mg, yield 52%). $^1H$ NMR (400 MHz, DMSO-d6) δ 11.71 (s, 1H), 11.16 (s, 1H), 8.27 (s, 1H), 8.15 (d, *J* = 7.6 Hz, 1H), 8.05 (t, *J* = 6.4 Hz, 2H), 7.17 - 7.03 (m, 1H), 5.19 (dd, *J* = 12.8, 5.3 Hz, 1H), 4.58 (s, 2H), 4.11 (d, *J* = 12.0 Hz, 2H), 3.50 - 3.39 (m, 4H), 3.22 (s, 2H), 2.95 - 2.85 (m, 1H), 2.66 - 2.53 (m, 2H), 2.12 - 2.04 (m, 1H). LCMS (ESI) calcd for $C_{23}H_{22}F_2N_5O_4{}^+$ $[M+H]^+$: 470.16, found, 470.2.

Example 432: Preparation of 5-((4-(3-bromopyridin-4-yl)piperazin-1-yl)methyl)-2-(2,6-dioxopiperidin-3-yl)isoindoline-1,3-dione (GT-06556)

Referring to the method of Scheme 1, the target compound (GT-06556) was prepared under appropriate conditions as would be understood by those skilled in the art (white solid, 28 mg, yield 45%). $^1H$ NMR (400 MHz, DMSO-d6) δ 11.62 (s, 1H), 11.16 (s, 1H), 8.25 (s, 1H), 8.13 (d, *J* = 7.7, 1.1 Hz, 1H), 8.03 (dd, *J* = 18.8, 6.6 Hz, 2H), 7.15 - 7.06 (m, 1H), 5.19 (dd, *J* = 12.8, 5.4 Hz, 1H), 4.59 (s, 2H), 3.86 (s, 2H), 3.44 - 3.33 (m, 4H), 3.26 (s, 2H), 3.00 - 2.83 (m, 1H), 2.66 - 2.52 (m, 2H), 2.13 - 2.00 (m, 1H). LCMS (ESI) calcd for $C_{23}H_{23}BrN_5O_4{}^+$ $[M+H]^+$: 512.09, found, 512.2.

Example 433: Preparation of 2-(2,6-dioxopiperidin-3-yl)-5-((4-(3-fluoropyridin-4-yl)piperazin-1-yl)methyl)isoindoline-1,3-dione (GT-06617)

Referring to the method of Scheme 1, the target compound (GT-06617) was prepared under appropriate conditions as would be understood by those skilled in the art (white solid, 13 mg, yield 23%). $^1H$ NMR (400 MHz, DMSO-d6) δ 12.72 - 11.49 (m, 1H), 11.16 (s, 1H), 8.72 (d, *J* = 8.2 Hz, 1H), 8.36 (d, *J* = 6.8 Hz, 1H), 8.24 (s, 1H), 8.09 (dd, *J* = 28.1, 7.7 Hz, 2H), 7.49 - 7.35 (m, 1H), 5.19 (dd, *J* = 12.8, 5.4 Hz, 1H), 4.55 (s, 2H), 4.19 - 4.05 (m, 2H), 3.76 - 3.67 (m, 4H), 2.96 - 2.83 (m, 2H), 2.68 - 2.55 (m, 3H), 2.13 - 2.02 (m, 1H). LCMS (ESI) calcd for $C_{23}H_{23}FN_5O_4{}^+$ $[M+H]^+$: 452.17, found, 452.2.

Example 434: Preparation of 5-((4-(3-chloropyridin-4-yl)piperazin-1-yl)methyl)-2-(2,6-dioxopiperidin-3-yl)isoindoline-1,3-dione (GT-06618)

Referring to the method of Scheme 1, the target compound (GT-06618) was prepared under appropriate conditions as would be understood by those skilled in the art (white solid, 36 mg, yield 50%). $^1H$ NMR (400 MHz, DMSO-d6) δ 12.20 (s, 1H), 11.16 (s, 1H), 8.74 (s, 1H), 8.50 (d, *J* = 6.4 Hz, 1H), 8.29 (s, 1H), 8.17 (d, *J* = 7.7 Hz, 1H), 8.03 (d, *J* = 22.6, 7.6 Hz, 1H), 7.44 *(d, J* = 6.6 Hz, 1H), 5.20 (dd, *J* = 12.9, 5.3 Hz, 1H), 4.63 (s, 2H), 4.02 (s, 2H), 3.88 - 3.79 (m, 4H), 3.40 - 3.34 (m, 2H), 2.96 - 2.86 (m, 1H), 2.67 - 2.55 (m, 2H), 2.18 - 2.02 (m, 1H). LCMS (ESI) calcd for $C_{23}H_{23}ClN_5O_4{}^+$ $[M+H]^+$: 468.14,

found, 468.2.

Example 435: Preparation of 5-((4-(2-chloropyridin-4-yl)piperazin-1-yl)methyl)-2-(2,6-dioxopiperidin-3-yl)isoindoline-1,3-dione (GT-06619)

Referring to the method of Scheme 1, the target compound (GT-06619) was prepared under appropriate conditions as would be understood by those skilled in the art (white solid, 52 mg, yield 73%). $^1$H NMR (400 MHz, DMSO-d6) δ 11.95 (s, 1H), 11.16 (s, 1H), 8.25 (s, 1H), 8.17 - 8.07 (m, 2H), 8.04 (d, *J* = 7.7 Hz, 1H), 7.08 (d, *J* = 2.3 Hz, 1H), 6.98 (dd, *J* = 6.4, 2.4 Hz, 1H), 5.19 (dd, *J* = 12.8, 5.4 Hz, 1H), 4.58 (s, 2H), 4.19 (s, 2H), 3.54 - 3.33 (m, 4H), 3.16 (s, 2H), 2.98 - 2.84 (m, 1H), 2.66 - 2.53 (m, 2H), 2.13 - 1.97 (m, 1H). LCMS (ESI) calcd for $C_{23}H_{23}ClN_5O_4{}^+$ $[M+H]^+$: 468.14, found, 468.1.

Example 436: Preparation of 5-((4-(2-chloro-3-fluoropyridin-4-yl)piperazin-1-yl)methyl)-2-(2,6-dioxopiperidin-3-yl)isoindoline-1,3-dione (GT-06620)

Referring to the method of Scheme 1, the target compound (GT-06620) was prepared under appropriate conditions as would be understood by those skilled in the art (white solid, 35 mg, yield 47%). $^1$H NMR (400 MHz, DMSO-d6) δ 11.47 (s, 1H), 11.16 (s, 1H), 8.20 (d, *J* = 24.2 Hz, 1H), 8.14 - 8.09 (m, 1H), 8.04 (dd, *J*= 14.2, 6.6 Hz, 2H), 7.17 - 7.08 (m, 1H), 5.20 (dd, *J* = 12.8, 5.4 Hz, 1H), 4.64 (s, 2H), 3.88 (s, 2H), 3.27 (s, 6H), 2.96 - 2.81 (m, 1H), 2.67 - 2.53 (m, 2H), 2.14 - 2.03 (m, 1H). LCMS(ESI) calcd for $C_{23}H_{22}ClFN_5O_4{}^+$ $[M+H]^+$: 486.13, found, 486.1.

Example 437: Preparation of 5-((4-(2-bromo-3-fluoropyridin-4-yl)piperazin-1-yl)methyl)-2-(2,6-dioxopiperidin-3-yl)isoindoline-1,3-dione (GT-06621)

Referring to the method of Scheme 1, the target compound (GT-06621) was prepared under appropriate conditions as would be understood by those skilled in the art (white solid, 46 mg, yield 57%). $^1$H NMR (400 MHz, DMSO-d6) δ 11.16 (s, 1H), 8.83 (s, 1H), 8.53 (d, *J* = 6.4 Hz, 1H), 8.30 (s, 1H), 8.18 (d, *J* = 8.6 Hz, 1H), 8.05 (d, *J* = 7.6 Hz, 1H), 7.41 (d, *J* = 6.4 Hz, 1H), 5.20 (dd, *J* = 12.8, 5.4 Hz, 1H), 4.64 (s, 2H), 3.98 (s, 2H), 3.49 - 3.42 (m, 4H), 3.35 - 3.27 (m, 2H), 2.94 - 2.84 (m, 1H), 2.68 - 2.54 (m, 2H), 2.15 - 2.02 (m, 1H). LCMS (ESI) calcd for $C_{23}H_{22}BrFN_5O_4{}^+$ $[M+H]^+$: 530.08, found, 530.1.

Example 438: Preparation of 5-((4-(3-bromo-2-chloropyridin-4-yl)piperazin-1-yl)methyl)-2-(2,6-dioxopiperidin-3-yl)isoindoline-1,3-dione (GT-06622)

Referring to the method of Scheme 1, the target compound (GT-06622) was prepared under appropriate conditions as would be understood by those skilled in the art (white solid, 40 mg, yield 49%). $^1$H NMR (400 MHz, DMSO-d6) δ 11.45 (s, 1H), 11.16 (s, 1H), 8.30 - 8.20 (m, 2H), 8.14 (d, *J* = 7.7 Hz, 1H), 8.04 (d, 1H), 7.18 (d, *J* = 5.5 Hz, 1H), 5.20 (dd, *J*= 12.8, 5.4 Hz, 1H), 4.64 (s, 2H), 3.69 (s, 2H), 3.29 (s, 6H), 2.94 - 2.84 (m, 1H), 2.65 - 2.54 (m, 2H), 2.14 - 1.99 (m, 1H). LCMS (ESI) calcd for $C_{23}H_{22}BrClN_5O_4{}^+$ $[M+H]^+$: 546.05, found, 546.1.

Example 439: Preparation of 5-((4-(5,6-dichloropyrimidin-4-yl)piperazin-1-yl)methyl)-2-(2,6-dioxopiperidin-3-yl)isoindoline-1,3-dione (GT-06623)

Referring to the method of Scheme 1, the target compound (GT-06623) was prepared under appropriate conditions as would be understood by those skilled in the art (white solid, 36 mg, yield 47%). $^1$H NMR (400 MHz, DMSO-d6) δ 11.44 (s, 1H), 11.16 (s, 1H), 8.53 (s, 1H), 8.22 (s, 1H), 8.07 (dd, *J*= 19.6, 7.7 Hz, 2H), 5.19 (dd, *J* = 12.8, 5.4 Hz, 1H), 4.57 (s, 2H), 4.38 (s, 2H), 3.74 - 3.44 (m, 4H), 3.22 (s, 2H), 3.00 - 2.84 (m, 1H), 2.69 - 2.53 (m, 2H), 2.16 - 2.01 (m, 1H). LCMS (ESI) calcd for $C_{22}H_{21}Cl_2N_6O_4{}^+$ $[M+H]^+$: 503.10, found, 503.1.

Example 440: Preparation of 5-((4-(3,6-dichloropyridazin-4-yl)piperazin-1-yl)methyl)-2-(2,6-dioxopiperidin-3-yl)isoindoline-1,3-dione (GT-06625)

Referring to the method of Scheme 1, the target compound (GT-06625) was prepared under appropriate conditions as would be understood by those skilled in the art (white solid, 24 mg, yield 31%). $^1$H NMR (400 MHz, DMSO-d6) δ 11.59 (d, *J* = 80.5 Hz, 1H), 11.16 (s, 1H), 8.24 (d, *J* = 9.9 Hz, 1H), 8.15 - 8.09 (m, 1H), 8.05 (q, *J* = 7.6, 4.1 Hz, 1H), 7.59 (s, 1H), 5.19 (dd, *J* = 12.8, 5.4 Hz, 1H), 4.59 (d, *J* = 20.3 Hz, 2H), 4.45 (s, 1H), 3.92 (s, 1H), 3.70 - 3.54 (m, 2H), 3.35 - 3.15 (m, 4H), 2.95 - 2.84 (m, 1H), 2.65 - 2.53 (m, 2H), 2.12 - 2.05 (m, 1H). LCMS (ESI) calcd for $C_{22}H_{21}Cl_2N_6O_4{}^+$ $[M+H]^+$: 503.10, found, 503.1.

Example 441: Preparation of 5-((4-(1,3,5-triazin-2-yl)piperazin-1-yl)methyl)-2-(2,6-dioxopiperidin-3-yl)isoindoline-1,3-dione (GT-06626)

Referring to the method of Scheme 1, the target compound (GT-06626) was prepared under appropriate conditions as would be understood by those skilled in the art (white solid, 40 mg, yield 60%). $^1$H NMR (400 MHz, DMSO-d6) δ 11.57 (s, 1H), 11.09 (s, 1H), 8.63 (s, 2H), 8.16 (s, 1H), 8.07 - 7.94 (m, 2H), 5.12 (dd, *J* = 12.8, 5.4 Hz, 1H), 4.58 (d, *J* = 69.4 Hz, 4H), 3.62 - 3.51 (m, 2H), 3.09 (s, 4H), 2.90 - 2.76 (m, 1H), 2.61 - 2.47 (m, 2H), 2.08 - 1.97 (m, 1H). LCMS (ESI) calcd for $C_{21}H_{22}N_7O_4^+$ [M+H]$^+$: 436.17, found, 436.2.

Example 442: Preparation of 5-((4-(benzo[d]isoxazol-3-yl)piperidin-1-yl)methyl)-2-(2,6-dioxopiperidin-3-yl)isoindoline-1,3-dione (GT-06627)

Referring to the method of Scheme 1, the target compound (GT-06627) was prepared under appropriate conditions as would be understood by those skilled in the art (white solid, 28 mg, yield 48%). $^1$H NMR (400 MHz, DMSO-d6) δ 11.13 (d, *J*= 25.2 Hz, 2H), 8.28 (s, 1H), 8.16 - 8.06 (m, 2H), 7.76 (d, 1H), 7.67 (t, *J* = 7.7 Hz, 1H), 7.41 (t, *J* = 7.4 Hz, 1H), 5.20 (dd, *J* = 12.8, 5.3 Hz, 1H), 4.60 (s, 2H), 3.63 - 3.43 (m, 3H), 3.25 - 3.12 (m, 2H), 2.97 - 2.83 (m, 1H), 2.67 - 2.53 (m, 2H), 2.41 - 2.18 (m, 4H), 2.15 - 2.00 (m, 1H). LCMS (ESI) calcd for $C_{26}H_{25}N_4O_5^+$ [M+H]$^+$: 473.18, found, 473.3.

Example 443: Preparation of 2-(2,6-dioxopiperidin-3-yl)-5-((4-(isoxazol-3-yl)piperidin-1-yl)methyl)isoindoline-1,3-dione (GT-06628)

Referring to the method of Scheme 1, the target compound (GT-06628) was prepared under appropriate conditions as would be understood by those skilled in the art (white solid, 3.7 mg, yield 7%). $^1$H NMR (400 MHz, DMSO-d6) δ 11.16 (s, 1H), 10.68 (s, 1H), 8.88 (d, *J*= 14.7 Hz, 1H), 8.23 (s, 1H), 8.16 - 7.99 (m, 2H), 6.55 (s, 1H), 5.19 (dd, *J*= 12.7, 5.3 Hz, 1H), 4.54 (s, 2H), 3.52 - 3.45 (m, 3H), 3.18 - 3.02 (m, 3H), 2.95 - 2.85 (m, 1H), 2.66 - 2.56 (m, 2H), 2.19 - 1.96 (m, 4H). LCMS (ESI) calcd for $C_{22}H_{23}N_4O_5^+$ [M+H]$^+$: 423.17, found, 423.2.

Example 444: Preparation of 2-(2,6-dioxopiperidin-3-yl)-5-((4-(6-fluorobenzo[d]isoxazol-3-yl)piperazin-1-yl)methyl)isoindoline-1,3-dione (GT-06629)

Referring to the method of Scheme 1, the target compound (GT-06629) was prepared under appropriate conditions as would be understood by those skilled in the art (white solid, 26 mg, yield 43%). $^1$H NMR (400 MHz, DMSO-d6) δ 11.16 (s, 2H), 8.28 - 7.99 (m, 4H), 7.67 - 7.53 (m, 1H), 7.26 (t, *J* = 9.1, 2.1 Hz, 1H), 5.20 (dd, *J*= 12.9, 5.4 Hz, 1H), 4.62 (s, 2H), 4.11 (s, 2H), 3.59 - 3.47 (m, 4H), 3.47 - 3.44 (m, 2H), 2.94 - 2.86 (m, 1H), 2.68 - 2.56 (m, 2H), 2.16 - 1.98 (m, 1H). LCMS (ESI) calcd for $C_{25}H_{23}FN_5O_5$ $^+$ [M+H]$^+$: 492.17, found, 492.2.

Example 445: Preparation of 2-(2,6-dioxopiperidin-3-yl)-5-((4-(isoxazolo[4,5-c]pyridin-3-yl)piperazin-1-yl)methyl)isoindoline-1,3-dione (GT-06630)

Referring to the method of Scheme 1, the target compound (GT-06630) was prepared under appropriate conditions as would be understood by those skilled in the art (white solid, 30 mg, yield 52%). $^1$H NMR (400 MHz, DMSO-d6) δ 11.91 (s, 1H), 11.15 (d, *J* = 8.7 Hz, 1H), 9.55 (s, 1H), 8.77 (d, *J* = 6.2 Hz, 1H), 8.28 (s, 1H), 8.16 (d, *J* = 7.7, 1.2 Hz, 1H), 8.07 (d, *J* = 7.6 Hz, 1H), 7.94 (d, *J* = 6.2 Hz, 1H), 5.20 (dd, *J* = 12.8, 5.4 Hz, 1H), 4.64 (s, 2H), 4.20 (s, 2H), 3.71 - 3.66 (m, 2H), 3.43 - 3.28 (m, 4H), 2.94 - 2.86 (m, 1H), 2.67 - 2.55 (m, 2H), 2.12 - 2.05 (m, 1H). LCMS (ESI) calcd for $C_{24}H_{23}N_6O_5$ $^+$ [M+H]$^+$: 475.17, found, 475.3.

Example 446: Preparation of 2-(2,6-dioxopiperidin-3-yl)-5-((4-(6-fluorobenzo[d]isothiazol-3-yl)piperazin-1-yl)methyl) isoindoline-1,3-dione (GT-06631)

Referring to the method of Scheme 1, the target compound (GT-06631) was prepared under appropriate conditions as would be understood by those skilled in the art (white solid, 26 mg, yield 42%). $^1$H NMR (400 MHz, DMSO-d6) δ 11.36 - 11.06 (m, 2H), 8.27 - 7.98 (m, 5H), 7.35 (td, *J* = 8.8, 2.3 Hz, 1H), 5.20 (dd, *J* = 12.8, 5.3 Hz, 1H), 4.65 (s, 2H), 4.05 (s, 2H), 3.51 - 3.44 (m, 4H), 3.34 - 3.31 (m, 2H), 2.96 - 2.86 (m, 1H), 2.68 - 2.56 (m, 2H), 2.14 - 2.03 (m, 1H). LCMS(ESI) calcd for $C_{25}H_{23}FN_5O_4S$ $^+$ [M+H]$^+$: 508.14, found, 508.2.

Example 447: Preparation of 2-(2,6-dioxopiperidin-3-yl)-5-((4-(6-fluoro-1H-indazol-3-yl)piperazin-1-yl)methyl)isoindoline-1,3-dione (GT-06632)

Referring to the method of Scheme 1, the target compound (GT-06632) was prepared under appropriate conditions as would be understood by those skilled in the art (yellow solid, 37 mg, yield 62%). $^1$H NMR (400 MHz, DMSO-d6) δ 12.20 (s, 1H), 11.07 (s, 1H), 10.93 (s, 1H), 8.16 (s, 1H), 8.01 (dd, 2H), 7.75 (dd, *J* = 9.0, 5.2 Hz, 1H), 7.08 (d, *J* = 9.7, 2.2 Hz, 1H), 6.81 (td, *J* = 9.2, 2.3 Hz, 1H), 5.11 (dd, *J* = 12.8, 5.4 Hz, 1H), 4.54 (s, 2H), 3.87 (s, 2H), 3.34 (s, 2H), 3.25 - 3.18 (m, 4H), 2.87 - 2.75 (m, 1H), 2.59 - 2.44 (m, 2H), 2.08 - 1.94 (m, 1H). LCMS (ESI) calcd for $C_{25}H_{24}FN_6O_4^+$ [M+H]$^+$: 491.18, found, 491.2.

Example 448: Preparation of 2-(2,6-dioxopiperidin-3-yl)-5-((4-(6-fluoro-1H-indol-3-yl)piperidin-1-yl)methyl)isoindoline-1,3-dione (GT-06633)

Referring to the method of Scheme 1, the target compound (GT-06633) was prepared under appropriate conditions as would be understood by those skilled in the art (white solid, 34 mg, yield 57%). $^1$H NMR (400 MHz, DMSO-d6) δ 11.06 (s, 1H), 10.89 (s, 1H), 10.58 (s, 1H), 8.20 (d, *J* = 9.6 Hz, 1H), 8.02 (dd, 2H), 7.61 - 7.49 (m, 1H), 7.05 (td, *J* = 5.1, 2.3 Hz, 2H), 6.84 - 6.70 (m, 1H), 5.13 (dd, *J* = 12.9, 5.3 Hz, 1H), 4.53 (s, 2H), 3.39 (d, *J* = 11.8 Hz, 2H), 3.16 - 2.92 (m, 3H), 2.89 - 2.79 (m, 1H), 2.61 - 2.47 (m, 2H), 2.11 - 1.88 (m, 5H). LCMS (ESI) calcd for $C_{27}H_{26}FN_4O_4^+$ [M+H]$^+$: 489.19, found, 489.2.

Example 449: Preparation of 2-(2,6-dioxopiperidin-3-yl)-5-((4-(6-fluoro-1H-indol-3-yl)-3,6-dihydropyridin-1(2H)-yl)methyl)isoindoline-1,3-dione (GT-06634)

Referring to the method of Scheme 1, the target compound (GT-06634) was prepared under appropriate conditions as would be understood by those skilled in the art (white solid, 13 mg, yield 22%). $^1$H NMR (400 MHz, DMSO-d6) δ 11.31 (s, 1H), 11.08 (s, 2H), 10.77 (d, *J* = 98.3 Hz, 1H), 8.24 - 8.17 (m, 1H), 8.05 - 7.95 (m, 2H), 7.64 (d, *J* = 45.6 Hz, 1H), 7.29 - 7.00 (m, 2H), 6.69 (t, *J* = 9.3 Hz, 1H), 5.11 (dd, *J* = 12.9, 5.3 Hz, 2H), 4.62 - 4.31 (m, 3H), 2.93 - 2.80 (m, 3H), 2.78 - 2.70 (m, 1H), 2.61 - 2.54 (m, 2H), 2.04 - 1.95 (m, 2H). LCMS (ESI) calcd for $C_{27}H_{24}FN_4O_4^+$ [M+H]$^+$: 487.18, found, 487.3.

Example 450: Preparation of 2-(2,6-dioxopiperidin-3-yl)-5-((4-(6-fluoro-1-methyl-1H-indol-3-yl)piperidin-1-yl)methyl)isoindoline-1,3-dione (GT-06635)

Referring to the method of Scheme 1, the target compound (GT-06635) was prepared under appropriate conditions as would be understood by those skilled in the art (white solid, 38 mg, yield 62%). $^1$H NMR (400 MHz, DMSO-d6) δ 11.09 (s, 1H), 10.54 (s, 1H), 8.18 (s, 1H), 8.03 (dd, *J* = 23.7, 7.5 Hz, 2H), 7.59 (dd, *J* = 8.6, 5.4 Hz, 1H), 7.19 (dd, *J* = 10.3, 2.1 Hz, 1H), 7.05 (s, 1H), 6.81 (t, *J* = 13.0, 5.3 Hz, 1H), 5.13 (dd, *J* = 12.8, 5.3 Hz, 1H), 4.53 (s, 2H), 3.63 (s, 3H), 3.41 (d, *J* = 11.5 Hz, 2H), 3.12 - 3.00 (m, 2H), 2.97 - 2.77 (m, 2H), 2.62 - 2.48 (m, 2H), 2.11 - 1.86 (m, 5H). LCMS (ESI) calcd for $C_{28}H_{28}FN_4O_4^+$ [M+H]$^+$: 503.21, found, 503.2.

Example 451: Preparation of 2-(2,6-dioxopiperidin-3-yl)-5-((4-(6-fluoro-1-methyl-1H-indol-3-yl)-3,6-dihydropyridin-1(2H)-yl)methyl)isoindoline-1,3-dione (GT-06636)

Referring to the method of Scheme 1, the target compound (GT-06636) was prepared under appropriate conditions as would be understood by those skilled in the art (yellow solid, 15 mg, yield 24%). $^1$H NMR (400 MHz, DMSO-d6) δ 11.08 (s, 1H), 10.66 (s, 1H), 8.17 (s, 1H), 8.03 (dd, *J* = 22.6, 7.4 Hz, 2H), 7.74 (dd, *J* = 8.9, 5.3 Hz, 1H), 7.47 (s, 1H), 7.29 (dd, *J* = 10.1, 2.3 Hz, 1H), 6.89 (td, *J* = 9.4, 2.4 Hz, 1H), 6.06 (s, 1H), 5.13 (dd, *J* = 12.8, 5.4 Hz, 1H), 4.71 - 4.45 (m, 2H), 3.87 - 3.57 (m, 6H), 2.90 - 2.69 (m, 3H), 2.61 - 2.45 (m, 3H), 2.08 - 2.00 (m, 1H). LCMS (ESI) calcd for $C_{28}H_{26}FN_4O_4^+$ [M+H]$^+$: 501.19, found, 501.2.

Example 452: Preparation of 2-(2,6-dioxopiperidin-3-yl)-5-((4-(5-fluoroindolin-1-yl)piperidin-1-yl)methyl)isoindoline-1,3-dione (GT-06637)

Referring to the method of Scheme 1, the target compound (GT-06637) was prepared under appropriate conditions as would be understood by those skilled in the art (yellow solid, 23 mg, yield 38%). $^1$H NMR (400 MHz, DMSO-d6) δ 11.22 (s, 1H), 8.37 - 8.07 (m, 4H), 7.11 - 6.81 (m, 2H), 6.58 - 6.48 (m, 1H), 5.26 (dd, *J* = 8.5, 4.2 Hz, 1H), 4.77 - 4.53 (m, 3H), 3.68 - 3.63 (m, 2H), 3.35 (t, *J* = 8.4 Hz, 2H), 3.19 - 3.03 (m, 2H), 3.01 - 2.89 (m, 3H), 2.73 - 2.64 (m, 2H), 2.26 - 2.02 (m, 4H), 1.89 (d, *J* = 11.8 Hz, 1H). LCMS (ESI) calcd for $C_{27}H_{28}FN_4O_4^+$ [M+H]$^+$: 491.21, found, 491.2.

Example 453: Preparation of 2-(2,6-dioxopiperidin-3-yl)-5-((4-(5-fluoro-1H-benzo[d]imidazol-1-yl)piperidin-1-yl)methyl) isoindoline-1,3-dione (GT-06638)

Referring to the method of Scheme 1, the target compound (GT-06638) was prepared under appropriate conditions as would be understood by those skilled in the art (white solid, 39 mg, yield 65%). $^1$H NMR (400 MHz, DMSO-d6) δ 11.87 (s, 1H), 11.22 (s, 1H), 9.24 (s, 1H), 8.41 - 8.01 (m, 5H), 7.73 (dd, *J* = 8.9, 2.2 Hz, 1H), 7.48 (t, *J* = 9.3 Hz, 1H), 5.26 (dd, *J* = 12.8, 5.3 Hz, 1H), 4.95 (s, 1H), 4.67 (s, 2H), 3.27 (s, 4H), 3.06 - 2.86 (m, 2H), 2.76 - 2.68 (m, 3H), 2.38 (d, *J* = 11.7 Hz, 2H), 2.17 - 2.10 (m, 1H). LCMS (ESI) calcd for $C_{26}H_{25}FN_5O_4^+$ [M+H]$^+$: 490.19, found, 490.2.

Example 454: Preparation of 2-(2,6-dioxopiperidin-3-yl)-5-((4-(5-fluoro-2-oxo-2,3-dihydro-1H-benzo[d]imidazol-1-yl)piperidin-1-yl)methyl)isoindoline-1,3-dione (GT-06704)

Referring to the method of Scheme 1, the target compound (GT-06704) was prepared under appropriate conditions as would be understood by those skilled in the art (white solid, 43 mg, yield 70%). $^1$H NMR (400 MHz, DMSO-d6) δ 11.13 (d, *J* = 21.3 Hz, 2H), 10.98 (s, 1H), 8.25 (s, 1H), 8.10 (dd, *J* = 25.6, 7.6 Hz, 2H), 7.54 - 7.41 (m, 1H), 6.92 - 6.77 (m, 2H), 5.20 (dd, *J* = 12.8, 5.3 Hz, 1H), 4.53 (d, *J* = 19.5 Hz, 3H), 3.53 (d, *J* = 11.0 Hz, 2H), 3.28 - 3.05 (m, 3H), 2.95 - 2.86 (m, 1H), 2.82 - 2.69 (m, 2H), 2.69 - 2.54 (m, 3H), 2.13 - 2.03 (m, 1H), 1.89 (d, *J*= 11.2 Hz, 2H). LCMS (ESI) calcd for $C_{26}H_{25}FN_5O_5^+$ [M+H]$^+$: 506.18, found, 506.1.

Example 455: Preparation of 5-((4-(1H-pyrrolo[2,3-b]pyridin-3-yl)piperidin-1-yl)methyl)-2-(2,6-dioxopiperidin-3-yl)isoindoline-1,3-dione (GT-06705)

Referring to the method of Scheme 1, the target compound (GT-06705) was prepared under appropriate conditions as would be understood by those skilled in the art (white solid, 27 mg, yield 47%). $^1$H NMR (400 MHz, DMSO-d6) δ 12.02 (s, 1H), 11.34 (s, 1H), 11.09 (s, 1H), 8.45 (d, *J* = 7.8 Hz, 1H), 8.28 - 8.24 (m, 2H), 8.12 (d, *J* = 7.7, 1.1 Hz, 1H), 7.99 (d, *J* = 7.7 Hz, 1H), 7.33 (d, *J* = 2.2 Hz, 1H), 7.20 (dd, *J* = 7.9, 5.3 Hz, 1H), 5.13 (dd, *J* = 12.9, 5.3 Hz, 1H), 4.50 (d, *J* = 4.4 Hz, 2H), 3.41 (d, *J* = 11.2 Hz, 3H), 3.15 - 2.94 (m, 4H), 2.87 - 2.80 (m, 1H), 2.58 - 2.47 (m, 2H), 2.21 - 2.13 (m, 2H), 2.02 (d, *J* = 11.0 Hz, 3H). LCMS (ESI) calcd for $C_{26}H_{26}N_5O_4^+$ [M+H]$^+$: 472.20, found, 472.2.

Example 456: Preparation of 5-((4-(1H-pyrrolo[2,3-b]pyridin-3-yl)-3,6-dihydropyridin-1(2H)-yl)methyl)-2-(2,6-dioxopiperidin-3-yl)isoindoline-1,3-dione (GT-06706)

Referring to the method of Scheme 1, the target compound (GT-06706) was prepared under appropriate conditions as would be understood by those skilled in the art (yellow solid, 20 mg, yield 35%). $^1$H NMR (400 MHz, DMSO-d6) δ 11.96 (s, 1H), 11.09 (s, 1H), 10.92 (s, 1H), 8.27 - 8.18 (m, 3H), 8.09 (d, *J*= 7.8 Hz, 1H), 8.00 (d, *J* = 7.6 Hz, 1H), 7.64 (d, *J* = 2.6 Hz, 1H), 7.11 (dd, *J* = 7.9, 4.9 Hz, 1H), 6.11 (s, 1H), 5.13 (dd, *J* = 12.9, 5.3 Hz, 1H), 4.69 - 4.52 (m, 2H), 3.88 - 3.68 (m, 2H), 3.59 (d, 1H), 3.26 (s, 1H), 2.92 - 2.77 (m, 3H), 2.61 - 2.48 (m, 2H), 2.05 - 1.96 (m, 1H). LCMS (ESI) calcd for $C_{26}H_{24}N_5O_4{}^+$ [M+H]$^+$: 470.18, found, 470.2.

Example 457: Preparation of 5-((4-(1H-pyrrolo[2,3-b]pyridin-3-yl)piperazin-1-yl)methyl)-2-(2,6-dioxopiperidin-3-yl)isoindoline-1,3-dione (GT-06707)

Referring to the method of Scheme 1, the target compound (GT-06707) was prepared under appropriate conditions as would be understood by those skilled in the art (yellow solid, 22 mg, yield 38%). $^1$H NMR (400 MHz, DMSO-d6) δ 11.68 (s, 1H), 11.39 (s, 1H), 11.16 (s, 1H), 8.31 - 8.21 (m, 3H), 8.17 (d, *J* = 7.7 Hz, 1H), 8.09 - 8.03 (m, 1H), 7.27 - 7.07 (m, 2H), 5.20 (dd, *J* = 12.8, 5.2 Hz, 1H), 4.64 (s, 2H), 3.41 - 3.27 (m, 5H), 3.18 - 3.06 (m, 2H), 2.95 - 2.87 (m, 1H), 2.69 - 2.55 (m, 3H), 2.11 - 2.01 (m, 1H). LCMS (ESI) calcd for $C_{25}H_{25}N_6O_4{}^+$ [M+H]$^+$: 473.19, found, 473.0.

Example 458: Preparation of 5-((4-(2-chloro-6,7-dihydrothieno[3,2-d]pyrimidin-4-yl)piperazin-1-yl)methyl)-2-(2,6-dioxopiperidin-3-yl)isoindoline-1,3-dione (GT-06710)

Referring to the method of Scheme 1, the target compound (GT-06710) was prepared under appropriate conditions as would be understood by those skilled in the art (white solid, 38 mg, yield 59%). $^1$H NMR (400 MHz, DMSO-d6) δ 11.33 (s, 1H), 11.16 (s, 1H), 8.20 (s, 1H), 8.13 - 7.95 (m, 2H), 5.20 (dd, *J* = 12.9, 5.4 Hz, 1H), 4.54 (s, 2H), 4.40 (s, 1H), 3.64 - 3.46 (m, 3H), 3.37 - 3.27 (m, 4H), 3.22 (t, *J*= 8.2 Hz, 2H), 3.19 - 3.12 (m, 1H), 2.96 - 2.85 (m, 1H), 2.68 - 2.52 (m, 3H), 2.12 - 2.02 (m, 1H). LCMS (ESI) calcd for $C_{24}H_{24}ClN_6O_4S{}^+$ [M+H]$^+$: 527.13, found, 526.9.

Example 459: Preparation of 2-(2,6-dioxopiperidin-3-yl)-5-((4-(2-morpholino-6,7-dihydrothieno[3,2-d]pyrimidin-4-yl)piperazin-1-yl)methyl)isoindoline-1,3-dione (GT-06712)

Referring to the method of Scheme 1, the target compound (GT-06712) was prepared under appropriate conditions as would be understood by those skilled in the art (white solid, 28 mg, yield 40%). $^1$H NMR (400 MHz, DMSO-d6) δ 11.83 (s, 1H), 11.14 (d, *J* = 12.0 Hz, 1H), 8.24 (s, 1H), 8.12 (d, *J* = 7.7 Hz, 1H), 8.04 (d, *J* = 7.7 Hz, 1H), 5.19 (dd, *J* = 12.8, 5.3 Hz, 1H), 4.56 (s, 2H), 4.46 (s, 1H), 3.66 (s, 12H), 3.32 (t, *J*= 7.9 Hz, 3H), 3.19 (t, *J* = 8.1 Hz, 3H), 2.96 - 2.86 (m, 1H), 2.73 - 2.53 (m, 3H), 2.13 - 2.03 (m, 1H). LCMS (ESI) calcd for $C_{28}H_{32}N_7O_5S^+$ [M+H]$^+$: 578.22, found, 578.0.

Example 460: Preparation of 2-(2,6-dioxopiperidin-3-yl)-5-((4-(4-morpholino-6,7-dihydrothieno[3,2-d]pyrimidin-2-yl)piperazin-1-yl)methyl)isoindoline-1,3-dione (GT-06713)

Referring to the method of Scheme 1, the target compound (GT-06713) was prepared under appropriate conditions as would be understood by those skilled in the art (yellow solid, 35 mg, yield 50%). $^1$H NMR (400 MHz, DMSO-d6) δ 11.35 (s, 1H), 11.16 (s, 1H), 8.22 (s, 1H), 8.06 (dd, 2H), 5.19 (dd, *J* = 12.7, 5.3 Hz, 1H), 4.55 (s, 3H), 3.65 (s, 10H), 3.40 - 3.32 (m, 4H), 3.27 (t, *J*= 8.1 Hz, 2H), 3.11 (t, *J* = 8.1 Hz, 3H), 2.97 - 2.86 (m, 1H), 2.65 - 2.56 (m, 2H), 2.14 - 1.98 (m, 1H). LCMS (ESI) calcd for $C_{28}H_{32}N_7O_5S^+$ [M+H]$^+$: 578.22, found, 578.0.

Example 461: Preparation of 2-(2,6-dioxopiperidin-3-yl)-5-((4-(thieno[3,2-d]pyrimidin-4-yl)piperazin-1-yl)methyl)isoindoline-1,3-dione (GT-06714)

Referring to the method of Scheme 1, the target compound (GT-06714) was prepared under appropriate conditions as would be understood by those skilled in the art (white solid, 40 mg, yield 67%). $^1$H NMR (400 MHz, DMSO-d6) δ 11.97 (s, 1H), 11.09 (s, 1H), 8.74 (s, 1H), 8.45 (d, *J* = 5.5 Hz, 1H), 8.17 (s, 1H), 8.05 (d, *J* = 7.3 Hz, 1H), 7.98 (d, *J* = 7.6 Hz, 1H), 7.53 (d, *J* = 5.6 Hz, 1H), 5.13 (dd, *J*= 12.9, 5.3 Hz, 1H), 4.76 (s, 2H), 4.50 (s, 2H), 3.60 (s, 6H), 2.88 - 2.80 (m, 1H), 2.61 - 2.50 (m, 2H), 2.06 - 1.98 (m, 1H). LCMS (ESI) calcd for $C_{24}H_{23}N_6O_4S^+$ [M+H]$^+$: 491.15, found, 491.0.

Example 462: Preparation of 5-((4-(2-chlorothieno[3,2-d]pyrimidin-4-yl)piperazin-1-yl)methyl)-2-(2,6-dioxopiperidin-3-yl)isoindoline-1,3-dione (GT-06715)

Referring to the method of Scheme 1, the target compound (GT-06715) was prepared under appropriate conditions as would be understood by those skilled in the art (white solid, 39 mg, yield 61%). $^1$H NMR (400 MHz, DMSO-d6) δ 11.74 (s, 1H), 11.16 (s, 1H), 8.39 (d, *J* = 5.5 Hz, 1H), 8.21 (s, 1H), 8.08 (dd, *J* = 21.4, 7.7 Hz, 2H), 7.47 (d, *J* = 5.5 Hz, 1H), 5.20 (dd, *J* = 12.8, 5.4 Hz, 1H), 4.64 (d, *J* = 59.9 Hz, 4H), 3.76 (s, 2H), 3.29 (s, 3H), 2.98 - 2.83 (m, 1H), 2.70 - 2.52 (m, 3H), 2.13 - 2.02 (m, 1H). LCMS (ESI) calcd for $C_{24}H_{22}ClN_6O_4S^+$ [M+H]$^+$: 525.11, found, 524.9.

Example 463: Preparation of 2-(2,6-dioxopiperidin-3-yl)-5-((4-(2-morpholinothieno[3,2-d]pyrimidin-4-yl)piperazin-1-yl)methyl)isoindoline-1,3-dione (GT-06717)

Referring to the method of Scheme 1, the target compound (GT-06717) was prepared under appropriate conditions as would be understood by those skilled in the art (white solid, 44 mg, yield 63%). $^1$H NMR (400 MHz, DMSO-d6) δ 12.19 (s, 1H), 11.16 (s, 1H), 8.43 - 7.95 (m, 4H), 7.57 (s, 1H), 5.20 (dd, *J* = 12.9, 5.4 Hz, 1H), 4.66 (d, *J* = 64.8 Hz, 4H), 3.75 (dd, *J* = 28.4, 23.7 Hz, 10H), 3.33 - 3.13 (m, 4H), 2.95 - 2.86 (m, 1H), 2.70 - 2.52 (m, 3H), 2.13 - 2.02 (m, 1H). LCMS (ESI) calcd for $C_{28}H_{30}N_7O_5S^+$ [M+H]$^+$: 576.20, found, 576.0 .

Example 464: Preparation of 2-(2,6-dioxopiperidin-3-yl)-5-((4-(4-morpholinothieno[3,2-d]pyrimidin-2-yl)piperazin-1-yl)methyl)isoindoline-1,3-dione (GT-06718)

Referring to the method of Scheme 1, the target compound (GT-06718) was prepared under appropriate conditions as would be understood by those skilled in the art (white solid, 26 mg, yield 37%). $^1$H NMR (400 MHz, DMSO-d6) δ 11.69 (s, 1H), 11.16 (s, 1H), 8.26 (d, *J* = 18.9 Hz, 2H), 8.08 (dd, *J*= 25.4, 7.5 Hz, 2H), 7.45 (s, 1H), 5.20 (dd, *J* = 12.8, 5.4 Hz, 1H), 4.74 (s, 2H), 4.56 (s, 2H), 3.96 (s, 4H), 3.83 - 3.63 (m, 6H), 3.21 - 3.09 (m, 3H), 2.93 - 2.86 (m, 1H), 2.69 - 2.54 (m, 3H), 2.13 - 2.05 (m, 1H). LCMS (ESI) calcd for $C_{28}H_{30}N_7O_5S^+$ [M+H]$^+$: 576.20, found, 576.0.

Example 465: Preparation of 2-(2,6-dioxopiperidin-3-yl)-5-((4-(4-(piperazin-1-yl)-6,7-dihydrothieno[3,2-d]pyrimidin-2-yl)piperazin-1-yl)methyl)isoindoline-1,3-dione (GT-06799)

Referring to the method of Scheme 1, the target compound (GT-06799) was prepared under appropriate

conditions as would be understood by those skilled in the art (white solid, 26 mg, yield 68%). $^{1}H$ NMR (400 MHz, DMSO-d6) δ 11.86 (s, 1H), 11.16 (s, 1H), 9.47 (s, 1H), 8.26 (s, 1H), 8.16 (d, 1H), 8.04 (d, *J* = 7.6 Hz, 1H), 5.19 (dd, *J* = 12.9, 5.1 Hz, 1H), 4.66 (d, *J* = 75.4 Hz, 4H), 4.17 (d, *J* = 37.4 Hz, 3H), 3.85 (s, 4H), 3.39 (s, 4H), 3.30 (s, 1H), 3.21 - 3.07 (m, 6H), 2.97 - 2.86 (m, 1H), 2.66 - 2.54 (m, 2H). LCMS (ESI) calcd for $C_{28}H_{33}N_8O_4S^+$ [M+H]+: 577.23, found, 577.3.

Example 466: Preparation of 2-(2,6-dioxopiperidin-3-yl)-5-((4-(4-(piperazin-1-yl)thieno[3,2-d]pyrimidin-2-yl)piperazin-1-yl)methyl)isoindoline-1,3-dione (GT-06800)

Referring to the method of Scheme 1, the target compound (GT-06800) was prepared under appropriate conditions as would be understood by those skilled in the art (white solid, 34 mg, yield 89%). $^{1}H$ NMR (400 MHz, DMSO-d6) δ 11.75 (s, 1H), 11.16 (s, 1H), 9.49 (s, 2H), 8.25 (s, 2H), 8.13 (d, *J* = 7.8 Hz, 1H), 8.05 (d, *J* = 7.6 Hz, 1H), 7.39 (s, 1H), 5.20 (dd, *J* = 12.8, 5.3 Hz, 1H), 4.73 (s, 2H), 4.58 (s, 2H), 4.14 (s, 4H), 3.27 (s, 6H), 3.19 - 2.97 (m, 3H), 2.96 - 2.87 (m, 1H), 2.69 - 2.56 (m, 2H), 2.11 - 2.01 (m, 1H), 1.29 - 1.22 (m, 1H). LCMS (ESI) calcd for $C_{28}H_{31}N_8O_4S^+$ [M+H]+: 575.22, found, 575.3.

Example 467: Preparation of 5-((4-(5,6-dichloropyridazin-4-yl)piperazin-1-yl)methyl)-2-(2,6-dioxopiperidin-3-yl)isoindoline-1,3-dione (GT-06624)

Referring to the method of Scheme 1, the target compound (GT-06624) was prepared under appropriate conditions as would be understood by those skilled in the art (white solid, 36 mg, yield 47%). $^{1}H$ NMR (400 MHz, DMSO-d6) δ 11.73 (s, 1H), 11.16 (s, 1H), 9.06 (s, 1H), 8.25 (s, 1H), 8.13 (d, *J* = 7.8 Hz, 1H), 8.05 (d, *J* = 7.6 Hz, 1H), 5.19 (dd, *J* = 12.8, 5.4 Hz, 1H), 4.61 (s, 2H), 3.96 (s, 2H), 3.56 (s, 2H), 3.36 - 3.21 (m, 4H), 2.95 - 2.85 (m, 1H), 2.66 - 2.53 (m, 2H), 2.10 - 2.03 (m, 1H). LCMS (ESI) calcd for $C_{22}H_{21}Cl_2N_6O_4^+$ [M+H]+: 503.10, found, 503.1.

Biological activity assay

Reagents and Materials

| Reagents and materials | Suppliers or Source |
|---|---|
| Human multiple myeloma cell line: MM.1S | ATCC (American Type Culture Collection) |
| Human multiple myeloma cell line resistant to dexamethasone: MM.1R | ATCC |
| Human non-small cell lung cancer cell line: NCI-H1975 | ATCC |
| Human prostate cancer cell line: Vcap | ATCC |
| Human T cell acute lymphoblastic leukemia cell line: CCRF-CEM | Dalian Meilun Biotech Co., Ltd. |
| Human diffuse large B-cell lymphoma cell line: TMD8 | Kyinno Biotechnology Co., Ltd |
| Human colon cancer cell line: SW620 | ATCC |
| Human monocytic leukemia cell line: THP-1 | ATCC |
| Human T lymphocyte leukemia cell line: Jurkat | ATCC |
| Human thyroid carcinoma cell line: CAL-62 | Dalian Meilun Biotech Co., Ltd. |
| Human breast cancer cell line: MDA-MB-231 | National Collection of Authenticated Cell Cultures (NCACC) |
| Human acute T lymphocytic leukemia cell line: Molt-4 | National Collection of Authenticated Cell Cultures |
| Human gastric cancer cell line: MGC803 | Dalian Meilun Biotech Co., Ltd. |
| Human large cell immunoblastic lymphoma cell line: SR | Dalian Meilun Biotech Co., Ltd. |
| Human myeloid monocytic leukemia cell line: MV-4-11 | ATCC |
| RPMI-1640 Medium | ATCC |
| DMEM high-glucose medium | ATCC |
| IMDM Medium | Gibco Company |
| L-15 Medium | ATCC |
| CellTiter-Meiluncell Luminescent Cell Viability Assay Kit | Dalian Meilun Biotech Co., Ltd. |
| Fetal bovine serum (FBS) | Sunrise Science Products Company |
| Penicillin-Streptomycin | Beijing TransGen Biotech Co., Ltd. |

(continued)

| Reagents and materials | Suppliers or Source |
| --- | --- |
| Mycoplasma detection kit | Beijing TransGen Biotech Co., Ltd. |
| STR genotype detection | Shanghai Biowing Applied Biotechnology Co. Ltd. |

Methods:

Cell cultures

The tumor cells used herein were cultured in a cell culture medium listed in table 2 at 37°C in an incubator with 5% $CO_2$. Prior to experimentation, all cell lines used were correctly identified by STR profiling, and tested negative for mycoplasma contamination using a mycoplasma detection kit through routine screenings.

Table 2. Tumor cells and cell culture medium used in the present disclosure

| Cell line | Cell culture medium used |
| --- | --- |
| MM.1S | RPMI 1640 culture medium supplemented with 10% FBS, as well as penicillin at a final concentration of 100 U/mL and streptomycin at a final concentration of 100 μg/mL |
| MM.1R | |
| NCI-H1975 | |
| CCRF-CEM | |
| TMD8 | |
| THP-1 | |
| Jurkat | |
| MDA-MB-231 | |
| MGC803 | |
| SR | |
| Molt-4 | |
| Vcap | DMEM high-glucose medium supplemented with 10% FBS, as well as penicillin at a final concentration of 100 U/mL and streptomycin at a final concentration of 100 μg/mL |
| CAL-62 | |
| MV-4-11 | IMDM culture medium supplemented with 10% FBS, as well as penicillin at a final concentration of 100 U/mL and streptomycin at a final concentration of 100 μg/mL |
| SW620 | L-15 culture medium supplemented with 10% FBS, as well as penicillin at a final concentration of 100 U/mL and streptomycin at a final concentration of 100 μg/mL |

**I. Determination of half inhibitory concentration ($IC_{50}$) of the compounds against tumor cells:**

The $IC_{50}$ values of the compounds of the present disclosure (including the compounds in Table 1, and Examples 1-467) were determined using the CellTiter-Meiluncell Luminescent Cell Viability Assay Kit from Dalian Meilun Biotech Co., Ltd. The detailed procedure is as follows: Tumor cells were seeded in 96-well cell culture plates at the densities specified in Table 3. On the following day, the test compounds of the present disclosure were subjected to serial dilution. The dilution can be performed according to one of the following two methods:

(1) The first dilution method: The test compounds of the present disclosure (including the compounds in Table 1, and Examples 1-467) were subjected to 5-fold serial dilutions starting from a highest concentration of 2000 μM, resulting in a total of 9 concentrations from high to low (2000, 400, 80, 16, 3.2, 0.64, 0.128, 0.0256, 0.00512 μM). Subsequently, 0.5 μL of the test compound dilution was transferred into the 96-well plate containing the cells, with each well containing 100 μL of cell culture medium. This resulted in final test compound concentrations of 10, 2, 0.4, 0.08, 0.016, 0.0032, 0.00064, 0.000128, and 0.0000256 μM, respectively, or

(2) As an alternative, a second dilution method can be used: The test compounds of the present disclosure (including the compounds in Table 1, and Examples 1-467) were subjected to a 50-fold gradient dilution starting from a highest concentration of 100 μM, resulting in 2 concentrations (100 μM, 2 μM). Into the 96-well plate, the diluted test

compounds and corresponding detection reagents were added, resulting in final test compound concentrations of 500 nM and 10 nM, respectively. The experimental procedure is described below using the diluted 2 μM test compound as an example.

0.5 μL of the aforementioned diluted compound of the present disclosure (including the compounds in Table 1, and Examples 1-468) was added to the seeded cells in 100 μL of medium, yielding a final test compound concentration of 10 nM. After the cells were treated with the compounds of the present disclosure for a specified period (the treatment durations were shown in Table 3), the cell viability assay reagent was added to the culture medium according to the kit's instructions to determine cell viability. The negative control was DMSO, and the reference controls were corresponding commercial inhibitors immunomodulatory agents (including lenalidomide and pomalidomide). All controls were treated using the same procedure as the compounds of the present disclosure. The growth inhibition curves for the compounds of the present disclosure were plotted using Prism GraphPad software, and the $IC_{50}$ values were calculated therefrom. The results were listed in Tables 4-8 below.

Table 3. Seeding protocol and treatment time for tumor cells

| Cells | Cells seeding protocol | The times for treating cells with the compounds of the present disclosure (hours) |
|---|---|---|
| MM.1S | cells were seeded at a density of 10,000 cells per well in 100 μL of serum-containing RPMI-1640 medium per well | 96 hours |
| MM.1R | | |
| TMD8 | | |
| THP-1 | | |
| SR | | |
| Jurkat | | |
| Molt-4 | cells were seeded at a density of 6,000 cells per well in 100 μL of serum-containing RPMI-1640 medium per well | 96 hours |
| NCI-H1975 | cells were seeded at a density of 4,000 cells per well in 100 μL of serum-containing RPMI-1640 medium per well | 96 hours |
| CCRF-CEM | | |
| MGC803 | cells were seeded at a density of 2,000 cells per well in 100 μL of serum-containing RPMI-1640 medium per well | 96 hours |
| MV-4-11 | cells were seeded at a density of 10,000 cells per well in 100 μL of serum-containing IMDM medium per well | 96 hours |
| Vcap | cells were seeded at a density of 4,000 cells per well in 100 μL of serum-containing DMEM medium per well | 96 hours |
| MDA-MB-231 | | |
| CAL-62 | cells were seeded at a density of 2,000 cells per well in 100 μL of serum-containing DMEM medium per well | 96 hours |
| SW620 | cells were seeded at a density of 4,000 cells per well in 100 μL of serum-containing L-15 medium per well | 96 hours |

Table 4. The inhibitory activity ($IC_{50}$, nM) of the compounds of the present disclosure against tumor cells proliferation

| Com p. No. | MDA -MB-231 | Molt 4 | H197 5 | CCR F- CEM | CAL-62 | Jurka t | MGC 803 | MM. 1R | TMD 8 | Vcap | SW6 20 | SR |
|---|---|---|---|---|---|---|---|---|---|---|---|---|
| lenali domi de | J | J | J | J | J | J | J | J | J | J | J | J |
| poma lidom ide | J | J | J | J | J | J | J | J | I | J | J | J |
| GT-0420 1 | - | - | - | B | B | B | D | - | D | C | E | - |
| GT-0420 2 | - | - | - | B | B | B | D | - | D | B | B | - |
| GT-0420 3 | - | - | - | C | C | C | F | - | D | C | D | - |
| GT-0425 4 | - | - | - | C | D | D | F | - | F | D | D | - |
| GT-0425 5 | - | - | - | D | E | F | - | - | - | G | F | - |
| GT-0443 0 | - | D | - | D | E | D | - | E | - | F | - | - |
| GT-0443 1 | E | D | E | B | C | B | E | C | - | D | E | F |
| GT-0443 2 | G | C | F | C | D | B | F | C | - | D | F | - |
| GT-0443 7 | F | B | E | C | D | B | E | C | - | D | E | F |
| GT-0443 8 | - | D | G | D | D | D |  | D | - | E | F | - |
| GT-0444 0 | G | C | G | D | D | D | G | D | - | E | G | - |
| GT-0444 5 | - | F | - | F | - | F | - | - | - | - | - | - |
| GT-0444 6 | F | B | E | C | C | B | F | C | - | D | E | F |
| GT-0444 7 | - | F | - | F | - | F | - | F | - | - | - | - |
| GT-0444 8 | G | D | F | D | D | D | G | D | - | E | F | - |
| GT-0445 2 | F | C | F | C | D | C | F | D | - | D | F | F |
| GT-0445 3 | F | D | F | C | D | C | F | D | - | E | F | G |
| GT-0445 4 | F | D | F | D | D | C | F | D | - | D | F | F |
| GT-0445 5 | E | B | E | B | C | B | E | C | - | D | D | F |
| GT-0445 6 | F | D | F | C | D | C | F | D | - | E | F | G |
| GT-0446 1 | - | D | - | D | E | D | - | D | - | F | - | - |
| GT-0446 2 | D | B | D | B | D | D | D | B | - | C | D | D |
| GT-0446 3 | F | C | F | C | D | C | F | C | - | D | F | G |
| GT-0446 5 | - | D | G | D | D | D | F | D | - | E | F |  |
| GT-0446 6 | D | B | D | B | C | B | E | B | - | D | D | E |
| GT-0446 7 | D | B | D | B | B | B | D | B | - | C | D | D |
| GT-0446 8 | - | C | F | C | D | C | G | D | - | E | F | G |
| GT-0446 9 | - | D | - | C | D | D | G | D | - | E | G |  |
| GT-0447 0 | - | D | G | D | D | C | F | D | - | E | F | G |
| GT-0447 1 | - | D | - | D | F | D | - | E | - | F | - | - |
| GT-0447 2 | - | B | D | B | C | B | E | B | - | D | D | E |
| GT-0448 5 | F | C | F | C | D | B | F | C | - | D | F | F |
| GT-0448 7 | F | C | E | C | D | C | E | D | - | D | F | F |
| GT-0449 0 | - | F | - | E | G | E | - | F | - | G | - | - |

(continued)

| Com p. No. | MDA -MB-231 | Molt 4 | H197 5 | CCR F-CEM | CAL-62 | Jurka t | MGC 803 | MM. 1R | TMD 8 | Vcap | SW6 20 | SR |
|---|---|---|---|---|---|---|---|---|---|---|---|---|
| GT-0449 1 | F | C | F | C | D | C | G | D | - | D | F | F |
| GT-0449 3 | - | - | - | D | D | D | - | D | - | F | - | - |
| GT-0449 4 | D | B | D | B | B | A | D | A | - | B | C | C |
| GT-0449 5 | B | A | B | A | A | A | D | - | - | B | B | B |
| GT-0449 6 | F | C | F | C | D | C | F | D | - | D | F | F |
| GT-0449 7 | D | B | D | B | B | B | E | B | - | C | D | D |
| GT-0449 8 | - | D | - | D | E | D | - | E | - | F | G | - |
| GT-0450 2 | - | D | - | D | D | D | - | D | - | E | F | - |
| GT-0450 3 | B | A | B | A | A | A | C | B | - | B | B | B |
| GT-0450 4 | - | D | - | D | D | D | - | D | - | E | F | - |
| GT-0451 5 | E | C | E | - | D | E | - | C | - | - | - | - |
| GT-0452 6 | D | B | D | - | C | - | - | B | - | - | - | - |
| GT-0452 7 | D | B | D | - | B | G | - | C | - | - | - | - |
| GT-0452 9 | - | D | - | - | F | D | - | E | - | - | - | - |
| GT-0453 0 | E | D | F | - | D | C | - | D | - | - | - | - |
| GT-0453 1 | F | D | F | - | D | C | - | D | - | - | - | - |
| GT-0453 2 | - | D | - | - | D | D | - | D | - | - | - | - |
| GT-0453 3 | E | B | E | - | C | B | - | B | - | - | - | - |
| GT-0453 8 | - | E | - | - | F | E | - | F | - | - | - | - |
| GT-0453 9 | - | D | - | - | E | D | - | D | - | - | - | - |
| GT-0454 0 | - | E | - | - | F | E | - | F | - | - | - | - |
| GT-0454 4 | - | - | - | - | E | - | - | - | - | - | - | - |
| GT-0454 5 | - | - | - | - | F | - | - | - | - | - | - | - |
| GT-0454 6 | - | F | - | - | D | E | - | F | - | - | - | - |
| GT-0454 7 | - | F | - | - | - | F | - | F | - | - | - | - |
| GT-0454 8 | G | D | G | - | - | D | - | D | - | - | - | - |
| GT-0454 9 | F | C | F | - | D | C | - | F | - | - | - | - |
| GT-0455 0 | B | A | B | - | A | A | - | A | - | - | - | - |
| GT-0455 1 | D | B | C | - | B | A | - | B | - | - | - | - |
| GT-0455 2 | E | B | D | - | C | B | - | C | - | - | - | - |
| GT-0455 3 | D | B | D | - | B | B | - | B | - | - | - | - |
| GT-0455 4 | B | A | B | - | A | A | - | A | - | - | - | - |
| GT-0455 5 | G | D | F | - | D | C | - | C | - | - | - | - |
| GT-0455 8 | C | B | C | - | B | A | - | A | - | - | - | - |
| GT-0455 9 | D | B | D | - | B | B | - | B | - | - | - | - |
| GT-0456 0 | - | D | - | - | D | D | - | D | - | - | - | - |
| GT-0457 0 | B | B | D | - | - | - | - | - | D | - | - | - |
| GT-0457 1 | D | C | E | - | - | - | - | - | F | - | - | - |

(continued)

| Com p. No. | MDA -MB-231 | Molt 4 | H197 5 | CCR F-CEM | CAL-62 | Jurka t | MGC 803 | MM. 1R | TMD 8 | Vcap | SW6 20 | SR |
|---|---|---|---|---|---|---|---|---|---|---|---|---|
| GT-0457 2 | D | B | D | - | - | - | - | - | D | - | - | - |
| GT-0457 3 | D | C | E | - | - | - | - | - | E | - | - | - |
| GT-0458 2 | - | E | - | - | - | - | - | - | - | - | - | - |
| GT-0458 5 | C | B | D | - | - | - | - | - | D | - | - | - |
| GT-0458 6 | D | C | F | - | - | - | - | - | F | - | - | - |
| GT-0458 7 | B | B | C | - | - | - | - | - | D | - | - | - |
| GT-0458 8 | D | C | E | - | - | - | - | - | F | - | - | - |
| GT-0458 9 | F | D | F | - | - | - | - | - | G | - | - | - |
| GT-0460 1 | G | F | - | - | - | - | - | - | G | - | - | - |
| GT-0460 2 | D | B | D | - | - | - | - | - | D | - | - | - |
| GT-0460 3 | F | D | F | - | - | - | - | - | - | - | - | - |
| GT-0461 0 | - | G | - | - | - | - | - | - | - | - | - | - |
| GT-0461 4 | D | B | D | - | - | - | - | - | D | - | - | - |
| GT-0461 5 | F | D | F | - | - | - | - | - | G | - | - | - |
| GT-0461 7 | D | C | F | - | - | - | - | - | F | - | - | - |
| GT-0461 8 | - | E | - | - | - | - | - | - | - | - | - | - |
| GT-0462 0 | D | B | D | - | - | - | - | - | D | - | - | - |
| GT-0465 0 | E | D | E | - | - | - | - | - | F | - | - | - |
| GT-0465 1 | - | G | - | - | - | - | - | - | - | - | - | - |
| GT-0466 4 | - | F | - | - | - | - | - | - | - | - | - | - |
| GT-0466 6 | C | B | D | - | - | - | - | - | D | - | - | - |
| GT-0467 7 | - | - | - | - | - | - | - | - | - | - | - | - |
| GT-0468 9 | D | C | E | - | - | - | - | - | E | - | - | - |
| GT-0469 0 | E | C | F | - | - | - | - | - | G | - | - | - |
| GT-0469 1 | G | D | G | - | - | - | - | - | - | - | - | - |
| GT-0469 2 | D | B | E | - | - | - | - | - | D | - | - | - |
| GT-0470 1 | D | B | E | - | - | - | - | - | E | - | - | - |
| GT-0470 3 | - | F | - | - | - | - | - | - | - | - | - | - |
| GT-0470 4 | D | B | D | - | - | - | - | - | D | - | - | - |
| GT-0470 8 | C | B | D | - | - | - | - | - | D | - | - | - |
| GT-0471 0 | - | G | - | - | - | - | - | - | - | - | - | - |
| GT-0471 2 | E | D | F | - | - | - | - | - | - | - | - | - |
| GT-0471 3 | B | B | C | - | - | - | - | - | D | - | - | - |
| GT-0471 6 | - | F | - | - | - | - | - | - | - | - | - | - |
| GT-0471 8 | C | B | D | - | - | - | - | - | D | - | - | - |
| GT-0472 2 | F | E | F | - | - | - | - | - | - | - | - | - |
| GT-0472 3 | C | B | D | - | - | - | - | - | D | - | - | - |
| GT-0472 4 | D | C | E | - | - | - | - | - | E | - | - | - |

(continued)

| Com p. No. | MDA -MB-231 | Molt 4 | H197 5 | CCR F-CEM | CAL-62 | Jurka t | MGC 803 | MM. 1R | TMD 8 | Vcap | SW6 20 | SR |
|---|---|---|---|---|---|---|---|---|---|---|---|---|
| GT-0472 6 | - | D | - | - | - | - | - | - | - | - | - | - |
| GT-0472 7 | F | D | - | - | - | - | - | - | G | - | - | - |
| GT-0472 9 | B | B | D | - | - | - | - | - | D | - | - | - |
| GT-0473 0 | D | B | D | - | - | - | - | - | D | - | - | - |
| GT-0473 1 | - | G | - | - | - | - | - | - | - | - | - | - |
| GT-0473 2 | G | E | - | - | - | - | - | - | - | - | - | - |
| GT-0476 5 | D | C | E | - | - | - | - | - | F | - | - | - |
| GT-0476 6 | D | B | D | - | - | - | - | - | D | - | - | - |

Table 5. The inhibitory activity ($IC_{50}$, nM) of the compounds of the present disclosure against tumor cells proliferation

| Comp. No. | MM.1S | MV-4-11 | Molt-4 | H1975 | CCRF-CEM | Jurkat | TMD8 | SR |
|---|---|---|---|---|---|---|---|---|
| lenalidomide | H | J | J | J | J | J | J | J |
| pomalidomide | G | J | J | J | J | J | I | J |
| GT-04855 | C | - | - | E | V | E | - | G |
| GT-04856 | D | - | - | - | - | - | - | - |
| GT-04858 | A | - | - | B | - | B | - | C |
| GT-04859 | E | - | - | - | - | - | - | - |
| GT-04863 | D | - | - | - | - | - | - | - |
| GT-04898 | C | - | - | - | - | - | - | - |
| GT-04900 | D | - | - | - | - | - | - | - |
| GT-04905 | B | - | - | F | - | F | - | - |
| GT-04901 | D | - | - | - | - | - | - | - |
| GT-04902 | A | - | - | B | - | B | - | C |
| GT-04903 | D | - | - | - | - | - | - | - |
| GT-04907 | B | - | - | E | - | E | - | - |
| GT-04930 | D | - | - | - | - | - | - | - |
| GT-04934 | A | - | - | E | - | D | - | F |
| GT-04935 | A | - | - | B | - | B | - | D |
| GT-05101 | A | - | - | B | - | B | - | C |
| GT-04915 | F | - | - | G | - | F | - | - |
| GT-06652 | - | - | - | - | - | F | - | - |
| GT-06656 | F | D | D | - | D | E | - | - |
| GT-06675 | F | E | D | F | D | E | G | G |
| GT-06678 | - | F | F | - | F | - | - | - |
| GT-06679 | - | F | - | - | - | - | - | - |
| GT-06680 | F | D | F | - | F | - | - | - |
| GT-06681 | - | G | - | - | - | - | - | - |
| GT-06683 | - | F | G | - | G | - | - | - |

(continued)

| Comp. No. | MM.1S | MV-4-11 | Molt-4 | H1975 | CCRF-CEM | Jurkat | TMD8 | SR |
|---|---|---|---|---|---|---|---|---|
| GT-06761 | F | D | F | - | F | - | - | - |
| GT-06620 | - | F | - | - | - | - | - | - |
| GT-06623 | - | F | - | - | - | - | - | - |
| GT-06625 | - | F | - | - | - | - | - | - |
| GT-06626 | - | E | - | - | - | - | - | - |
| GT-06631 | - | F | - | - | - | - | - | - |
| GT-06632 | G | D | F | - | E | F | - | - |
| GT-06633 | F | E | F | - | F | F | - | - |
| GT-06635 | F | D | E | - | E | F | - | - |
| GT-06636 | F | D | D | G | D | E | - | - |
| GT-06637 | - | E | - | - | - | - | - | - |
| GT-06638 | - | G | - | - | - | - | - | - |
| GT-06706 | - | F | G | - | G | - | - | - |
| GT-06712 | - | F | - | - | - | - | - | - |
| GT-06717 | - | D | - | - | - | - | - | - |
| GT-06718 | - | F | - | - | - | - | - | - |
| GT-06601 | - | F | - | - | - | - | - | - |
| GT-06608 | F | D | F | - | F | F | - | - |
| GT-06609 | - | F | F | - | F | F | - | - |
| GT-06611 | - | F | G | - | F | - | - | - |
| GT-06612 | - | G | G | - | G | - | - | - |
| GT-06614 | - | D | - | - | - | - | - | - |
| GT-06616 | - | F | G | - | G | - | - | - |
| GT-06694 | - | F | G | - | G | - | - | - |
| GT-05211 | E | - | - | - | - | - | - | - |
| GT-05264 | B | - | - | - | - | - | D | - |
| GT-05266 | C | - | - | - | - | - | D | - |
| GT-05380 | B | - | - | - | - | - | D | - |

Note: The symbol "-" indicates "not detected";
In Tables 4-5, the $IC_{50}$ data of the compounds of the present disclosure for inhibiting tumor cell proliferation are classified into the following activity levels:
0 nM<level A≤1 nM; 1 nM<level B≤5 nM; 5 nM<level C≤10nM; 10 nM < level D≤50nM; 50 nM < level E≤100nM; 100 nM<level F≤500 nM; 500 nM<level G≤1000 nM; 1000 nM<level H≤5000nM; 5000 nM<level I≤10000 nM; 10000 nM<level J.

Table 6. Inhibitory activity of the compounds of the present disclosure against tumor cells proliferation

| Comp. No. | CCRF-CEM | | Kasumi-1 | |
|---|---|---|---|---|
| | Inhibition rate (%) at 500 nM | Inhibition rate (%) at 10 nM | Inhibition rate (%) at 500 nM | Inhibition rate (%) at 10 nM |
| lenalidomide | 6a3 | 6a3 | 6a1 | 6a2 |
| GT-07842 | 6a11 | 6a5 | 6a7 | 6a6 |

(continued)

| Comp. No. | CCRF-CEM | | Kasumi-1 | |
|---|---|---|---|---|
| | Inhibition rate (%) at 500 nM | Inhibition rate (%) at 10 nM | Inhibition rate (%) at 500 nM | Inhibition rate (%) at 10 nM |
| GT-08003 | 6a11 | 6a5 | 6a10 | 6a2 |
| GT-08134 | 6a9 | 6a4 | 6a9 | 6a3 |
| Note: in table 6, the symbols 6a1, 6a2, 6a3, 6a4, 6a5, 6a6, 6a7, 6a8, 6a9, 6a10, and 6a11 are defined as follows: 0% < 6a1 < 5%, 5% ≤ 6a2 < 10%, 10% ≤ 6a3 < 15%, 15% ≤ 6a4 < 20%, 20% ≤ 6a5 < 30%, 30% ≤ 6a6 < 40%, 40% ≤ 6a7 < 50%, 50% ≤ 6a8 < 60%, 60% ≤ 6a9 < 80%, 80% ≤ 6a10 < 90%, 90% ≤ 6a11 ≤ 100%. The symbol "-" indicates "not detected". | | | | |

Table 7. Inhibitory activity of the compounds of the present disclosure on the Proliferation of Human acute T lymphocytic leukemia cells (Molt-4)

| Comp. No. | Inhibition rate (%) at 500 nM | Inhibition rate (%) at 10 nM |
|---|---|---|
| lenalidomide | 7a3 | 7a4 |
| GT-07842 | 7a10 | 7a5 |
| GT-08003 | 7a11 | 7a5 |
| Note: in table 7, the symbols 7a1, 7a2, 7a3, 7a4, 7a5, 7a6, 7a7, 7a8, 7a9, 7a10, and 7a11 are defined as follows: 0% < 7a1 < 5%, 5% ≤ 7a2 < 10%, 10% ≤ 7a3 < 15%, 15% ≤ 7a4 < 20%, 20% ≤ 7a5 < 30%, 30% ≤ 7a6 < 40%, 40% ≤ 7a7 < 50%, 50% ≤ 7a8 < 60%, 60% ≤ 7a9 < 80%, 80% ≤ 7a10 < 90%, 90% ≤ 7a11 ≤ 100%. The symbol "-" indicates "not detected". | | |

Table 8. Inhibitory activity of the compounds of the present disclosure on the Proliferation of Human breast cancer cells (MDA-MB-231)

| Comp. No. | Inhibition rate (%) at 500 nM | Inhibition rate (%) at 10 nM | Comp. No. | Inhibition rate (%) at 500 nM | Inhibition rate (%) at 10 nM |
|---|---|---|---|---|---|
| lenalidomide | 8a3 | 8a4 | GT-08004 | 8a7 | 8a6 |
| GT-07842 | 8a5 | 8a5 | GT-08005 | 8a6 | 8a5 |
| GT-07843 | 8a6 | 8a6 | GT-08134 | 8a5 | 8a6 |
| GT-08003 | 8a6 | 8a6 | | | |
| Note: in table 8, the symbols 8a1, 8a2, 8a3, 8a4, 8a5, 8a6, 8a7, 8a8, 8a9, 8a10, and 8a11 are defined as follows: 0% < 8a1 < 5%, 5% ≤ 8a2 < 10%, 10% ≤ 8a3 < 15%, 15% ≤ 8a4 < 20%, 20% ≤ 8a5 < 30%, 30% ≤ 8a6 < 40%, 40% ≤ 8a7 < 50%, 50% ≤ 8a8 < 60%, 60% ≤ 8a9 < 80%, 80% ≤ 8a10 < 90%, 90% ≤ 8a11 ≤ 100%. The symbol "-" indicates "not detected". | | | | | |

The experimental results demonstrated that the compounds of the present disclosure (including those listed in Table 1 and in Examples 1-467) exhibited significant inhibitory effects on tumor cell proliferation (as shown in Tables 4-8), which were superior to those of the corresponding positive control drugs.

**II. Western-blot assay**

Conventional Western blot assay was performed to evaluate the effects of the compounds of the present disclosure (including those listed in Table 1 and in Examples 1-467) on the expression of target proteins in cells.

(1) Cell Seeding and Protein Harvesting: Human peripheral blood mononuclear cells (hPBMCs) at a density of $1\times10^6$ cells/mL were thawed according to the PBMC Recovery Protocol (Milecell Biotechnologies Inc, Shanghai). The cells were then treated with the compounds of the present disclosure (including the compounds listed in Table 1, and the Examples 1-467 compounds) at concentrations of 50 nM and 500 nM. Controls included a negative control (DMSO) and a positive control (the commercial immunomodulatory drug lenalidomide), both processed following the identical

experimental protocol as the compounds of the present disclosure. After 24 hours of compounds treatment, cells were harvested and lysed in RIPA protein lysate containing protease inhibitors on ice for 30 min, followed by centrifugation. The supernatant was aspirated as the extracted total cell protein. The protein concentration of the supernatant was determined using the conventional BCA (Bicinchoninic Acid) assay.

(2) To the collected supernatant, 5X SDS sample loading buffer was added, followed by denaturation at 95°C for 5 minutes. The denatured samples were then separated by SDS-PAGE electrophoresis on a polyacrylamide gel, transferred onto a nitrocellulose membrane (0.45 μm NC membrane), blocked with blocking buffer at room temperature for 1 hour, and subjected to antibody incubation and development procedures according to the antibody manufacturer's instructions (Cell Signaling Technology Company).

Half-Maximal Degradation Concentration value (the drug concentration required for degrading proteins by 50%, abbreviated as $DC_{50}$) reads method: comparing the grayscale values of Western blot bands from drug-treated samples with those from blank DMSO-treated controls, and identifying the drug concentration range at which the grayscale value is half that of the DMSO-treated control bands.

$DC_{50}$ values can be calculated using ImageJ software to measure grayscale values of Western blot bands from drug-treated samples. The concentration-response curve is fitted to estimate the drug concentration corresponding to half the grayscale value.

Target protein remaining (%) refers to the percentage of target protein remaining in cells after treatment with protein degrader compounds.

Calculation method for target protein remaining (%): Use ImageJ software to measure grayscale values of Western blot bands from drug (protein degrader)-treated samples. The grayscale value of drug-treated bands is divided by that of internal control protein bands, and the resulting quotient is normalized to obtain the relative percentage of remaining target protein after protein degrader treatment.

$$\text{Target protein degradation rate (\%)} = 1 - \text{target protein remaining (\%)}.$$

The effects of the compounds of the present disclosure (including the compounds listed in Table 1, and the Example 1-467 compounds) on substrate proteins expression in cells were shown in Table II-1 below.

Table II-1 Degradation of substrate proteins WEE1, IKZF1, IKZF2, IKZF3, CK1α, and GSPT1 by the compounds of the present disclosure

| Comp. No./names | Weel | IKZF 1 | IKZF2 | IKZF3 | GSPT1 | CK1a |
|---|---|---|---|---|---|---|
| Lenalidomide | B | A | B | A | B | B |
| GT-03026 | B | A | A | A | A | A |
| GT-04199 | A | B | A | A | B | B |
| GT-04200 | A | B | A | A | B | B |
| GT-04201 | A | B | A | B | A | A |
| GT-04202 | A | A | A | A | A | A |
| GT-04203 | A | A | A | A | A | A |
| GT-04254 | A | A | A | A | A | A |
| GT-04255 | A | A | A | A | A | A |
| GT-04428 | B | B | B | B | A | B |
| GT-04430 | B | A | A | A | A | B |
| GT-04431 | A | A | A | A | A | A |
| GT-04432 | A | A | A | A | A | B |
| GT-04433 | B | B | B | B | A | B |
| GT-04434 | B | B | B | B | A | B |
| GT-04437 | A | A | A | A | A | A |
| GT-04438 | A | A | A | A | A | A |

(continued)

| Comp. No./names | Weel | IKZF 1 | IKZF2 | IKZF3 | GSPT1 | CK1a |
|---|---|---|---|---|---|---|
| GT-04439 | A | A | A | A | A | A |
| GT-04440 | A | A | A | A | A | A |
| GT-04445 | B | A | A | A | A | B |
| GT-04446 | A | A | A | A | A | A |
| GT-04447 | B | A | A | A | A | B |
| GT-04448 | A | A | A | A | A | A |
| GT-04449 | A | A | A | A | A | A |
| GT-04450 | B | B | B | B | A | B |
| GT-04451 | B | B | B | B | A | B |
| GT-04452 | A | B | B | B | A | A |
| GT-04453 | A | A | A | A | A | A |
| GT-04454 | A | A | A | A | A | A |
| GT-04455 | A | A | A | A | A | A |
| GT-04456 | A | A | A | A | A | A |
| GT-04461 | A | A | A | A | A | B |
| GT-04462 | A | A | A | A | A | A |
| GT-04463 | A | A | A | A | A | A |
| GT-04464 | B | B | A | B | A | B |
| GT-04465 | A | A | A | A | A | A |
| GT-04466 | A | A | A | A | A | A |
| GT-04467 | A | A | A | A | A | A |
| GT-04468 | A | A | A | A | A | A |
| GT-04469 | A | A | A | A | A | A |
| GT-04470 | A | A | A | A | A | A |
| GT-04471 | A | A | A | A | A | B |
| GT-04472 | A | A | A | A | A | B |
| GT-04473 | A | A | B | B | A | B |
| GT-04479 | B | B | A | A | A | B |
| GT-04480 | A | A | A | A | A | A |
| GT-04481 | A | A | A | B | A | A |
| GT-04482 | A | B | A | B | A | A |
| GT-04483 | A | B | A | B | A | A |
| GT-04484 | A | B | A | B | A | A |
| GT-04485 | B | B | B | B | A | A |
| GT-04486 | A | A | A | B | A | A |
| GT-04487 | B | B | A | B | A | A |
| GT-04488 | B | B | A | B | A | A |
| GT-04490 | B | B | A | B | A | A |
| GT-04491 | B | B | A | B | A | A |

(continued)

| Comp. No./names | Weel | IKZF 1 | IKZF2 | IKZF3 | GSPT1 | CK1a |
|---|---|---|---|---|---|---|
| GT-04492 | B | B | B | B | A | A |
| GT-04493 | A | B | A | B | A | A |
| GT-04494 | A | B | A | B | A | A |
| GT-04495 | B | B | A | B | A | A |
| GT-04496 | B | B | A | B | A | A |
| GT-04497 | A | A | A | A | A | A |
| GT-04498 | A | A | A | A | A | A |
| GT-04499 | A | A | A | A | A | A |
| GT-04500 | A | A | A | A | A | A |
| GT-04501 | A | A | A | A | A | A |
| GT-04502 | A | A | A | A | A | A |
| GT-04503 | A | A | A | A | A | A |
| GT-04504 | A | A | A | A | A | B |
| GT-04508 | A | A | A | A | A | B |
| GT-04515 | A | A | A | A | A | A |
| GT-04526 | A | A | A | A | A | A |
| GT-04527 | A | B | A | A | A | A |
| GT-04528 | A | B | B | B | B | B |
| GT-04529 | A | A | A | A | A | A |
| GT-04530 | A | A | A | A | A | A |
| GT-04531 | A | A | A | A | A | A |
| GT-04532 | A | A | A | A | A | A |
| GT-04533 | A | A | A | A | A | A |
| GT-04537 | A | A | A | A | A | A |
| GT-04538 | A | A | A | A | A | A |
| GT-04539 | A | A | A | A | A | A |
| GT-04540 | A | A | A | A | A | B |
| GT-04541 | A | A | A | A | B | B |
| GT-04542 | A | A | A | A | A | B |
| GT-04543 | A | A | A | A | A | B |
| GT-04544 | A | A | A | A | A | A |
| GT-04545 | A | A | A | A | A | A |
| GT-04546 | A | A | A | A | A | A |
| GT-04547 | A | A | A | A | A | A |
| GT-04548 | A | A | A | A | A | A |
| GT-04549 | A | A | A | A | A | A |
| GT-04550 | A | A | A | A | A | A |
| GT-04551 | A | A | A | A | A | A |
| GT-04552 | A | A | A | A | A | A |

(continued)

| Comp. No./names | Weel | IKZF 1 | IKZF2 | IKZF3 | GSPT1 | CK1a |
|---|---|---|---|---|---|---|
| GT-04553 | A | A | A | A | A | A |
| GT-04554 | A | A | A | A | A | A |
| GT-04555 | A | A | A | A | A | A |
| GT-04556 | A | A | A | A | A | A |
| GT-04557 | A | A | A | A | A | A |
| GT-04558 | A | A | A | A | A | A |
| GT-04559 | A | A | A | A | A | A |
| GT-04560 | A | A | A | A | A | A |
| GT-04570 | A | A | A | A | A | A |
| GT-04571 | A | A | A | A | A | A |
| GT-04572 | A | A | A | A | A | A |
| GT-04573 | A | A | A | A | A | A |
| GT-04578 | B | B | A | B | A | A |
| GT-04579 | B | B | B | B | B | A |
| GT-04580 | A | A | A | A | B | A |
| GT-04581 | B | B | A | A | B | B |
| GT-04582 | A | A | A | A | A | B |
| GT-04585 | A | A | A | A | A | A |
| GT-04586 | B | B | A | B | A | B |
| GT-04587 | A | A | A | A | A | B |
| GT-04588 | B | B | A | B | A | B |
| GT-04589 | B | B | A | B | A | B |
| GT-04590 | B | B | A | B | A | B |
| GT-04591 | B | B | B | B | A | A |
| GT-04600 | A | A | A | A | A | B |
| GT-04601 | A | A | B | A | A | A |
| GT-04602 | A | A | A | A | A | A |
| GT-04603 | A | A | A | A | A | A |
| GT-04610 | A | A | A | A | A | A |
| GT-04611 | A | B | B | B | A | B |
| GT-04612 | A | B | B | B | A | B |
| GT-04613 | A | B | B | B | A | B |
| GT-04614 | A | B | B | B | A | B |
| GT-04615 | A | A | A | A | A | A |
| GT-04616 | B | B | B | B | A | B |
| GT-04617 | A | A | A | A | A | B |
| GT-04618 | A | A | A | A | A | B |
| GT-04620 | A | A | A | A | A | B |
| GT-04648 | B | A | A | A | A | B |

(continued)

| Comp. No./names | Weel | IKZF 1 | IKZF2 | IKZF3 | GSPT1 | CK1a |
|---|---|---|---|---|---|---|
| GT-04649 | B | A | A | A | A | B |
| GT-04650 | A | A | A | A | A | B |
| GT-04651 | B | A | A | A | A | A |
| GT-04664 | A | A | A | A | A | A |
| GT-04665 | A | A | A | A | A | A |
| GT-04666 | A | A | A | A | A | A |
| GT-04667 | A | A | A | A | A | B |
| GT-04677 | A | A | A | A | A | B |
| GT-04678 | A | A | A | A | A | B |
| GT-04679 | B | B | B | B | A | A |
| GT-04680 | B | B | B | B | A | A |
| GT-04681 | B | B | B | B | A | A |
| GT-04682 | B | B | B | B | A | A |
| GT-04689 | A | A | A | A | A | A |
| GT-04690 | A | A | A | A | A | A |
| GT-04691 | A | A | A | A | A | A |
| GT-04692 | A | A | A | A | A | A |
| GT-04693 | A | B | A | A | A | B |
| GT-04694 | A | B | A | A | A | B |
| GT-04698 | A | A | A | A | A | B |
| GT-04700 | B | A | A | A | A | B |
| GT-04701 | A | A | A | A | A | A |
| GT-04702 | B | A | A | A | A | B |
| GT-04703 | B | A | A | A | A | A |
| GT-04704 | A | A | A | A | A | A |
| GT-04705 | B | A | A | A | A | A |
| GT-04706 | B | A | A | A | A | A |
| GT-04707 | B | A | A | A | A | A |
| GT-04708 | A | B | B | B | A | A |
| GT-04709 | B | B | B | A | A | A |
| GT-04710 | B | B | B | A | A | A |
| GT-04712 | B | B | B | A | A | A |
| GT-04713 | A | A | A | A | A | A |
| GT-04714 | A | A | A | A | A | A |
| GT-04715 | A | A | A | A | A | A |
| GT-04716 | B | B | B | B | A | B |
| GT-04717 | B | B | A | A | A | B |
| GT-04718 | A | A | A | A | A | B |
| GT-04722 | B | B | B | A | A | A |

(continued)

| Comp. No./names | Weel | IKZF 1 | IKZF2 | IKZF3 | GSPT1 | CK1a |
|---|---|---|---|---|---|---|
| GT-04723 | A | A | A | A | A | A |
| GT-04724 | A | A | A | A | A | A |
| GT-04725 | B | B | B | B | A | B |
| GT-04726 | A | A | A | A | A | A |
| GT-04727 | A | A | B | A | A | A |
| GT-04728 | B | B | B | B | A | B |
| GT-04729 | A | A | A | A | A | B |
| GT-04730 | A | A | A | A | A | A |
| GT-04731 | B | B | B | B | A | B |
| GT-04732 | A | B | B | B | A | A |
| GT-04764 | A | B | B | B | A | A |
| GT-04765 | A | A | A | A | A | A |
| GT-04770 | B | B | B | B | A | B |
| GT-04771 | A | B | B | B | A | A |
| GT-04855 | A | A | A | A | A | B |
| GT-04856 | A | A | A | A | A | B |
| GT-04858 | A | A | A | A | A | B |
| GT-04859 | B | A | A | A | A | B |
| GT-04860 | B | A | A | A | A | B |
| GT-04861 | A | A | A | A | A | A |
| GT-04862 | A | A | A | A | A | A |
| GT-04863 | A | A | A | A | A | A |
| GT-04897 | B | B | B | B | A | B |
| GT-04898 | B | A | A | A | A | B |
| GT-04899 | B | A | B | B | B | B |
| GT-04900 | B | A | A | A | B | A |
| GT-04908 | A | A | A | A | A | B |
| GT-04905 | B | B | A | A | A | B |
| GT-04901 | B | A | A | A | B | B |
| GT-04902 | A | A | A | A | A | A |
| GT-04903 | B | B | A | A | A | A |
| GT-04904 | B | A | B | B | B | B |
| GT-04906 | B | A | B | B | B | B |
| GT-04907 | A | A | A | A | A | B |
| GT-04928 | B | B | B | B | A | B |
| GT-04929 | B | A | B | B | A | B |
| GT-04930 | A | A | A | A | A | B |
| GT-04933 | A | A | A | A | A | A |
| GT-04934 | A | A | A | A | A | A |

(continued)

| Comp. No./names | Weel | IKZF 1 | IKZF2 | IKZF3 | GSPT1 | CK1a |
|---|---|---|---|---|---|---|
| GT-04935 | A | A | A | A | A | A |
| GT-04936 | A | A | A | A | A | A |
| GT-04993 | A | A | A | A | A | A |
| GT-04994 | A | A | A | A | B | B |
| GT-04995 | A | A | A | A | B | B |
| GT-04996 | A | A | A | A | A | A |
| GT-05034 | B | A | A | A | A | A |
| GT-05101 | A | A | A | A | A | B |
| GT-05102 | A | A | A | B | A | B |
| GT-04914 | A | A | B | B | A | A |
| GT-04915 | A | A | A | A | A | A |
| GT-04920 | A | A | A | A | A | B |
| GT-04921 | A | A | B | B | B | B |
| GT-05157 | B | A | A | A | A | A |
| GT-05158 | B | B | A | A | A | B |
| GT-05159 | B | A | A | A | A | B |
| GT-05209 | A | A | A | A | B | B |
| GT-05210 | A | A | A | A | B | A |
| GT-05211 | A | A | A | A | A | A |
| GT-05264 | A | A | A | A | A | A |
| GT-05265 | B | A | A | A | A | A |
| GT-05266 | A | A | A | A | A | A |
| GT-05332 | B | B | A | B | B | B |
| GT-05380 | A | A | A | A | A | A |
| GT-05389 | B | A | A | B | B | B |
| GT-06234 | B | B | B | B | A | B |
| GT-06235 | B | B | B | B | A | B |
| GT-06236 | B | B | A | B | A | B |
| GT-06237 | B | B | B | B | A | B |
| GT-06238 | B | B | A | A | A | B |
| GT-06263 | B | B | B | A | A | A |
| GT-06264 | A | A | A | A | A | A |
| GT-06265 | B | B | A | B | B | B |
| GT-06267 | A | B | A | A | A | A |
| GT-06266 | B | B | A | B | B | B |
| GT-06268 | A | B | A | A | A | A |
| GT-06269 | B | B | A | A | A | B |
| GT-06270 | B | B | B | B | A | B |
| GT-06271 | A | A | A | B | A | B |

(continued)

| Comp. No./names | Weel | IKZF 1 | IKZF2 | IKZF3 | GSPT1 | CK1a |
|---|---|---|---|---|---|---|
| GT-06303 | B | A | B | B | B | B |
| GT-06304 | B | A | A | B | B | B |
| GT-06305 | B | B | B | B | B | A |
| GT-06306 | B | A | A | A | A | A |
| GT-06307 | B | A | A | B | A | A |
| GT-06308 | B | A | A | B | B | B |
| GT-06309 | B | A | A | A | B | B |
| GT-06310 | B | B | A | A | A | A |
| GT-06347 | B | A | A | A | B | B |
| GT-06348 | A | A | A | A | A | A |
| GT-06564 | A | A | A | A | A | A |
| GT-06565 | A | A | A | A | A | A |
| GT-06566 | A | A | A | A | A | A |
| GT-06567 | A | B | B | B | A | A |
| GT-06212 | B | B | A | B | A | B |
| GT-06213 | B | B | A | B | A | B |
| GT-06214 | B | B | A | B | A | B |
| GT-06215 | A | B | B | A | B | A |
| GT-06218 | A | B | B | A | A | A |
| GT-06219 | A | B | B | A | A | A |
| GT-06220 | A | A | A | A | A | A |
| GT-06221 | A | A | A | A | A | A |
| GT-06279 | A | A | A | A | A | A |
| GT-06222 | A | A | A | A | A | A |
| GT-06223 | A | A | B | A | A | A |
| GT-06224 | A | A | B | A | A | A |
| GT-06280 | B | B | B | B | A | B |
| GT-06281 | B | B | B | B | A | B |
| GT-06282 | B | B | B | B | A | B |
| GT-06283 | B | B | B | B | A | B |
| GT-06284 | B | B | B | B | A | B |
| GT-06285 | B | B | B | B | A | B |
| GT-06286 | B | B | B | B | A | B |
| GT-06287 | B | B | B | B | A | B |
| GT-06288 | B | B | B | B | A | B |
| GT-06289 | B | B | B | B | A | B |
| GT-06290 | B | B | B | B | A | B |
| GT-06291 | B | B | B | B | A | B |
| GT-06292 | B | B | B | B | A | B |

(continued)

| Comp. No./names | Weel | IKZF 1 | IKZF2 | IKZF3 | GSPT1 | CK1a |
|---|---|---|---|---|---|---|
| GT-06293 | B | B | B | B | A | B |
| GT-06295 | B | B | B | B | A | B |
| GT-06296 | B | B | B | B | A | B |
| GT-06297 | B | B | B | B | A | B |
| GT-06298 | B | B | B | B | A | B |
| GT-06299 | B | B | B | B | A | B |
| GT-06300 | B | B | B | B | A | B |
| GT-06361 | B | B | B | B | A | B |
| GT-06362 | B | B | B | B | A | B |
| GT-06363 | B | A | B | B | A | B |
| GT-06364 | B | B | B | B | A | B |
| GT-06365 | B | B | B | B | A | B |
| GT-06366 | B | B | A | A | A | B |
| GT-06367 | A | B | A | A | A | B |
| GT-06368 | A | B | A | A | A | A |
| GT-06369 | A | A | A | A | A | A |
| GT-06370 | A | B | A | A | A | A |
| GT-06371 | B | A | B | B | A | B |
| GT-06372 | A | A | A | B | A | B |
| GT-06373 | A | A | A | B | A | B |
| GT-06374 | A | A | A | B | A | B |
| GT-06375 | A | A | A | B | A | B |
| GT-06552 | B | B | A | B | A | B |
| GT-06553 | B | B | A | B | A | B |
| GT-06554 | B | B | A | B | A | B |
| GT-06555 | B | B | A | B | A | B |
| GT-06556 | B | B | A | B | A | B |
| GT-06260 | B | B | B | B | A | B |
| GT-06261 | A | A | A | A | A | A |
| GT-06262 | B | B | A | B | A | A |
| GT-06273 | A | A | A | A | A | A |
| GT-06274 | B | B | B | B | A | B |
| GT-06275 | B | B | B | B | A | B |
| GT-06276 | B | A | A | B | A | B |
| GT-06277 | B | B | B | B | A | B |
| GT-06355 | B | B | B | B | A | B |
| GT-06356 | B | B | B | B | A | B |
| GT-06358 | A | A | A | B | A | B |
| GT-06359 | A | A | A | B | A | B |

(continued)

| Comp. No./names | Weel | IKZF 1 | IKZF2 | IKZF3 | GSPT1 | CK1a |
|---|---|---|---|---|---|---|
| GT-06360 | A | A | A | B | A | B |
| GT-06429 | A | A | A | A | A | A |
| GT-06521 | B | B | B | B | A | B |
| GT-06522 | B | B | B | B | A | B |
| GT-06523 | B | A | B | B | A | B |
| GT-06524 | B | A | B | B | A | B |
| GT-06525 | A | B | A | A | A | B |
| GT-06526 | A | B | A | A | A | B |
| GT-06527 | A | B | A | A | A | B |
| GT-06528 | A | B | A | A | A | B |
| GT-06529 | A | B | A | A | A | B |
| GT-06530 | B | B | B | B | A | B |
| GT-06531 | B | B | B | B | A | B |
| GT-06532 | B | B | B | B | A | B |
| GT-06533 | B | B | B | B | A | B |
| GT-06534 | B | B | B | B | A | B |
| GT-06535 | B | B | A | A | B | B |
| GT-06591 | A | B | B | B | A | B |
| GT-06592 | A | A | A | A | A | B |
| GT-06593 | A | A | A | A | A | B |
| GT-06594 | A | A | A | A | A | B |
| GT-06646 | B | B | B | B | A | B |
| GT-06647 | A | B | A | B | A | A |
| GT-06648 | A | B | A | B | A | A |
| GT-06649 | A | B | A | A | A | A |
| GT-06650 | A | B | B | B | B | B |
| GT-06651 | A | B | A | B | B | B |
| GT-06652 | A | B | A | B | A | B |
| GT-06653 | A | A | B | A | A | B |
| GT-06654 | A | B | B | B | A | B |
| GT-06655 | A | A | A | A | A | B |
| GT-06656 | A | A | A | A | A | B |
| GT-06657 | A | A | B | B | A | B |
| GT-06675 | B | B | B | B | B | A |
| GT-06676 | A | B | B | B | B | A |
| GT-06677 | B | B | B | B | B | A |
| GT-06678 | A | A | B | A | A | A |
| GT-06679 | A | B | B | A | B | B |
| GT-06680 | A | A | A | A | A | B |

(continued)

| Comp. No./names | Weel | IKZF 1 | IKZF2 | IKZF3 | GSPT1 | CK1a |
|---|---|---|---|---|---|---|
| GT-06681 | A | A | A | A | A | B |
| GT-06682 | A | B | A | A | A | B |
| GT-06683 | A | A | A | A | A | B |
| GT-06761 | A | A | A | A | A | A |
| GT-06762 | A | A | B | B | B | A |
| GT-06763 | A | A | B | B | A | A |
| GT-06810 | B | A | A | A | A | A |
| GT-06912 | A | A | A | B | A | A |
| GT-06913 | A | A | A | B | A | A |
| GT-06927 | B | B | B | B | B | A |
| GT-06619 | B | B | B | A | A | B |
| GT-06620 | B | B | B | A | A | B |
| GT-06625 | B | B | B | B | A | B |
| GT-06626 | B | B | B | B | A | B |
| GT-06627 | B | B | B | B | A | B |
| GT-06628 | B | B | B | B | A | B |
| GT-06629 | A | B | A | B | A | A |
| GT-06630 | A | B | A | B | A | A |
| GT-06631 | A | B | B | B | A | A |
| GT-06632 | A | A | A | A | A | A |
| GT-06633 | A | A | A | A | A | A |
| GT-06634 | A | B | A | A | A | A |
| GT-06635 | A | A | A | A | A | A |
| GT-06636 | A | A | A | A | A | A |
| GT-06637 | A | A | A | A | A | A |
| GT-06638 | A | B | B | B | A | A |
| GT-06704 | B | B | B | A | A | B |
| GT-06705 | A | B | B | B | A | B |
| GT-06706 | A | A | A | A | A | A |
| GT-06707 | A | B | B | A | A | A |
| GT-06710 | B | B | A | B | A | B |
| GT-06712 | B | B | B | B | A | B |
| GT-06717 | A | A | B | B | A | B |
| GT-06718 | B | B | B | B | A | B |
| GT-06599 | B | B | B | B | A | B |
| GT-06600 | B | B | B | B | A | B |
| GT-06601 | B | B | B | B | A | B |
| GT-06607 | B | B | B | B | A | B |
| GT-06608 | B | B | B | B | A | B |

(continued)

| Comp. No./names | Weel | IKZF 1 | IKZF2 | IKZF3 | GSPT1 | CK1a |
|---|---|---|---|---|---|---|
| GT-06609 | A | A | A | A | A | B |
| GT-06611 | A | A | A | A | A | A |
| GT-06612 | A | A | A | A | A | A |
| GT-06613 | B | B | B | B | A | B |
| GT-06614 | B | B | B | B | A | A |
| GT-06615 | A | B | B | A | A | A |
| GT-06616 | A | A | B | A | A | A |
| GT-06688 | B | B | B | B | A | B |
| GT-06690 | B | B | B | A | A | B |
| GT-06691 | A | B | A | B | A | B |
| GT-06692 | A | B | A | B | A | B |
| GT-06693 | A | B | A | B | A | B |
| GT-06694 | A | B | A | B | A | B |
| GT-06695 | B | B | A | B | A | B |
| GT-06881 | B | B | B | B | A | A |
| GT-07060 | A | A | A | A | A | A |
| GT-07842 | B | A | A | A | A | B |
| GT-07844 | B | A | B | A | B | B |
| GT-08003 | B | A | A | A | A | A |
| GT-08004 | B | A | A | A | A | B |
| GT-08005 | B | A | A | A | B | B |
| GT-08134 | B | A | A | A | A | B |
| GT-05900 | B | B | A | A | B | B |
| **Note:** In Table II-1, "A" is defined as 0% ≤ substrate protein remaining % ≤ 80%, and "B" is defined as 80%< substrate protein remaining %≤ 100%. | | | | | | |

The degradation effects of the compounds of the present invention on the target substrate proteins were shown in Table II-1 and Figure 1. From the experimental results in Table II-1 and Figure 1, it can be concluded that the compounds of the present invention significantly degrade the target substrate proteins (e.g., proteins WEE1, IKZF1/2/3, CK1α, and GSPT1). Compared to lenalidomide, the compounds of the present invention exhibited significantly enhanced efficiency in inducing substrate protein degradation and also showed selective induction of degradation, thereby potentially enabling their use in treating indications associated with these substrate proteins.

**III. Determination of CRBN-binding affinity of compounds:**

The CEREBLON BINDING kits (specification: 10,000 tests; product number: Catalog # 64BDCRBNPEH; CISBIO company) was used to determine the CRBN binding ability of the compounds to be tested through a HTRF method (homogeneous time-resolved fluorescence). The specific methods are as follows:

**1.** According to the instructions of the CEREBLON BINDING KITS, serial dilutions were performed for the test compounds of the present disclosure (including the compounds in Table 1, and Examples 1-467) and for lenalidomide. Specifically, diluent #9 (1X) solution was used as the diluent, and multiple concentration gradients were set. The dilution operation can be performed according to one of the following two methods:

(1) The first dilution method: The test compounds of the present disclosure (including the compounds in Table 1,

and Examples 1-467) were subjected to 5-fold serial dilutions starting from a highest concentration of 40 μM, resulting in a total of 7 concentrations from high to low (40, 8, 1.6, 0.32, 0.064, 0.0128, 0.00256 μM). Subsequently, the diluted test compounds and corresponding detection reagents were added to each well of a 96-well plate, resulting in final test compound concentrations of 10, 2, 0.4, 0.08, 0.016, 0.0032, and 0.00064 μM, respectively.

(2) As an alternative, a second dilution method can be used: The test compounds of the present disclosure (including the compounds in Table 1, and Examples 1-467) were subjected to 4-fold gradient dilutions starting from a highest concentration of 8 μM, resulting in 2 concentrations (8 μM, 2 μM). Into the 96-well plate, the diluted test compounds and corresponding detection reagents were added, resulting in final test compound concentrations of 2 μM and 0.5 μM, respectively. The experimental procedure is described below using the diluted 8 μM test compound and lenalidomide solutions as examples.

**2.** 2.5 μL of the above 8 μM of the tested compounds and lenalidomide solution, as well as the same volume of diluent #9 (1X) solution (solvent control group, Std0) were added to each well of a 96-well plate, respectively. Then, 2.5 μL of human Cereblon WT GST-tagged protein solution was added to each well. Finally, 5 μL of the thoroughly mixed Thalidomide-Re reagent and GST Eu antibody working solution were added to each of the aforementioned wells. The final concentration of the tested compounds and lenalidomide in each well was 2 μM.

**3.** The blank control wells were sequentially added with 2.5 μL of diluent #9 (1X) solution, 2.5 μL of binding buffer, and 5 μL of thoroughly mixed Thalidomide-Re reagent and GST Eu antibody working solution.

**4.** After sealing and incubating the solutions in the aforementioned wells at room temperature for 3 hours, the absorbance values at emission wavelengths of 620 nm and 665 nm were detected by the HTRF method using a Spark microplate reader (V3.1 SP1).

The corresponding CRBN binding inhibition rate was calculated using the following method:

$$R_{compound} = OD\ 665\ nm_{compound} / OD\ 620\ nm_{compound} - OD\ 665\ nm_{control} / OD\ 620\ nm_{control}$$

$$R_{Std0} = OD\ 665\ nm_{Std0} / OD\ 620\ nm_{Std0} - OD\ 665\ nm_{control} / OD\ 620\ nm_{control}$$

$$\text{inhibition rate } (\%) = (1 - R_{compound} / R_{Std0}) * 100$$

Note: OD 665 nm $_{compound}$ is the absorbance value of each well of the compounds to be tested of the present disclosure at an emission wavelength of 665 nm.

OD 620 nm $_{compound}$ is the absorbance value of each well of the compounds to be tested of the present disclosure at an emission wavelength of 620 nm.

OD 665 nm $_{control}$ is the absorbance value of the blank control well at an emission wavelength of 665 nm.

OD 620 nm $_{control}$ is the absorbance value of the blank control well at an emission wavelength of 620 nm.

OD 665 nm $_{Std0}$ is the absorbance value of the solvent control well at an emission wavelength of 665 nm.

OD 620 nm $_{Std0}$ is the absorbance value of the solvent control well at an emission wavelength of 620 nm.

The test results were shown in Table III-1 below.

**Table III-1.** HTRF inhibitory activity ($IC_{50}$, μM) of the compounds of the present disclosure (including but not limited to the compounds in Table 1, and the Examples 1-467 compounds)

| Comp. No. | HTRF $IC_{50}$ (μM) | Comp. No. | HTRF $IC_{50}$ (μM) | Comp. No. | HTRF $IC_{50}$ (μM) | Comp. No. | HTRF $IC_{50}$ (μM) |
|---|---|---|---|---|---|---|---|
| Lenalidomide | e | GT-04553 | c | GT-04856 | b | GT-04508 | b |
| GT-04200 | b | GT-04554 | b | GT-04858 | a | GT-04515 | b |
| GT-04201 | c | GT-04555 | b | GT-04859 | a | GT-04526 | b |
| GT-04202 | b | GT-04556 | b | GT-04860 | a | GT-04528 | b |
| GT-04203 | b | GT-04557 | b | GT-04861 | a | GT-04529 | d |
| GT-04254 | b | GT-04558 | a | GT-04862 | a | GT-04530 | b |
| GT-04255 | b | GT-04559 | a | GT-04863 | a | GT-04531 | b |

(continued)

| Comp. No. | HTRF $IC_{50}$ (μM) | Comp. No. | HTRF $IC_{50}$ (μM) | Comp. No. | HTRF $IC_{50}$ (μM) | Comp. No. | HTRF $IC_{50}$ (μM) |
|---|---|---|---|---|---|---|---|
| GT-04428 | a | GT-04560 | b | GT-04896 | b | GT-04532 | b |
| GT-04430 | b | GT-04570 | b | GT-04897 | d | GT-04533 | b |
| GT-04431 | b | GT-04571 | c | GT-04898 | a | GT-04537 | b |
| GT-04432 | b | GT-04572 | b | GT-04899 | b | GT-04538 | a |
| GT-04433 | c | GT-04573 | c | GT-04900 | a | GT-04539 | b |
| GT-04434 | b | GT-04578 | c | GT-04908 | a | GT-04540 | b |
| GT-04435 | a | GT-04579 | d | GT-04905 | a | GT-04541 | b |
| GT-04436 | a | GT-04580 | b | GT-04901 | a | GT-04542 | a |
| GT-04437 | b | GT-04581 | c | GT-04902 | a | GT-04543 | b |
| GT-04438 | b | GT-04582 | a | GT-04903 | a | GT-04544 | b |
| GT-04439 | c | GT-04585 | a | GT-04904 | a | GT-04545 | a |
| GT-04440 | b | GT-04586 | b | GT-04906 | a | GT-04546 | b |
| GT-04445 | b | GT-04587 | b | GT-04907 | a | GT-04547 | b |
| GT-04446 | b | GT-04588 | c | GT-04928 | a | GT-04548 | b |
| GT-04447 | c | GT-04589 | a | GT-04929 | b | GT-04549 | b |
| GT-04448 | c | GT-04590 | c | GT-04930 | a | GT-04550 | a |
| GT-04449 | b | GT-04591 | d | GT-04933 | b | GT-04551 | b |
| GT-04450 | b | GT-04600 | b | GT-04934 | a | GT-04552 | c |
| GT-04451 | c | GT-04601 | a | GT-04935 | a | GT-04709 | a |
| GT-04452 | b | GT-04602 | a | GT-04936 | c | GT-04710 | a |
| GT-04453 | c | GT-04603 | b | GT-04993 | a | GT-04712 | b |
| GT-04454 | b | GT-04610 | b | GT-04994 | a | GT-04713 | b |
| GT-04455 | b | GT-04611 | b | GT-04995 | a | GT-04714 | b |
| GT-04456 | b | GT-04612 | b | GT-04996 | a | GT-04715 | d |
| GT-04461 | c | GT-04613 | c | GT-05034 | a | GT-04716 | b |
| GT-04462 | b | GT-04614 | b | GT-05100 | a | GT-04717 | b |
| GT-04463 | c | GT-04615 | b | GT-05101 | b | GT-04718 | b |
| GT-04464 | c | GT-04616 | a | GT-05102 | a | GT-04720 | a |
| GT-04465 | c | GT-04617 | a | GT-05157 | a | GT-04722 | a |
| GT-04466 | b | GT-04618 | b | GT-05158 | a | GT-04723 | a |
| GT-04467 | b | GT-04620 | b | GT-05159 | a | GT-04724 | a |
| GT-04468 | b | GT-04648 | b | GT-05209 | a | GT-04725 | a |
| GT-04469 | b | GT-04649 | a | GT-05210 | b | GT-04726 | b |
| GT-04470 | b | GT-04650 | a | GT-05211 | a | GT-04727 | a |
| GT-04471 | b | GT-04651 | a | GT-05264 | b | GT-04728 | c |
| GT-04472 | b | GT-04664 | b | GT-05265 | a | GT-04729 | b |
| GT-04473 | b | GT-04665 | a | GT-05266 | a | GT-04730 | b |
| GT-04479 | b | GT-04666 | a | GT-05332 | a | GT-04732 | b |
| GT-04480 | d | GT-04667 | b | GT-05380 | b | GT-04765 | c |

(continued)

| Comp. No. | HTRF $IC_{50}$ (μM) | Comp. No. | HTRF $IC_{50}$ (μM) | Comp. No. | HTRF $IC_{50}$ (μM) | Comp. No. | HTRF $IC_{50}$ (μM) |
|---|---|---|---|---|---|---|---|
| GT-04481 | b | GT-04677 | a | GT-05389 | a | GT-04766 | d |
| GT-04482 | b | GT-04678 | b | GT-06238 | c | GT-04767 | b |
| GT-04483 | b | GT-04679 | b | GT-06304 | b | GT-04768 | d |
| GT-04484 | b | GT-04680 | b | GT-06306 | d | GT-04855 | a |
| GT-04485 | b | GT-04681 | b | GT-06309 | d | GT-06651 | c |
| GT-04486 | b | GT-04682 | b | GT-06345 | c | GT-06653 | b |
| GT-04487 | b | GT-04683 | b | GT-06346 | c | GT-06655 | b |
| GT-04488 | b | GT-04689 | a | GT-06348 | c | GT-06656 | c |
| GT-04490 | b | GT-04690 | b | GT-06564 | b | GT-06657 | c |
| GT-04491 | b | GT-04691 | b | GT-06565 | c | GT-06675 | b |
| GT-04492 | b | GT-04692 | a | GT-06566 | b | GT-06676 | c |
| GT-04493 | b | GT-04693 | a | GT-06567 | c | GT-06677 | b |
| GT-04494 | a | GT-04694 | a | GT-06212 | a | GT-06679 | d |
| GT-04495 | a | GT-04698 | b | GT-06213 | d | GT-06680 | d |
| GT-04496 | b | GT-04700 | b | GT-06215 | b | GT-06681 | d |
| GT-04497 | b | GT-04701 | a | GT-06220 | d | GT-06682 | d |
| GT-04498 | b | GT-04702 | b | GT-06222 | c | GT-06913 | d |
| GT-04499 | b | GT-04703 | b | GT-06223 | c | GT-06927 | b |
| GT-04500 | b | GT-04704 | a | GT-06593 | d | GT-06632 | d |
| GT-04501 | a | GT-04705 | b | GT-06595 | c | GT-06634 | b |
| GT-04502 | b | GT-04706 | b | GT-06646 | b | GT-06636 | c |
| GT-04503 | a | GT-04707 | a | GT-06649 | d | GT-06705 | d |
| GT-04504 | b | GT-04708 | b | GT-06650 | d | GT-06713 | d |
| GT-06718 | c | GT-06605 | d | GT-06608 | c | GT-06609 | b |
| GT-06616 | d | GT-05900 | a | | | | |
| Note: in Table III-1, the symbols a, b, c, d, and e are defined as follows: 0<a≤0.5μM, 0.5μM<b≤1μM, 1μM<c≤1.5μM, 1.5μM<d<1.9μM, 1.9μM ≤ e ≤10μM. | | | | | | | |

The results in Table III-1 demonstrate that the compounds of the present disclosure (including the compounds in Table 1, and Examples 1-467) exhibit lower or comparable $IC_{50}$ values, or higher inhibition rates compared to lenalidomide, indicating stronger or comparable binding affinity to CRBN.

**IV. TNF-α Activity Inhibition Assay**

PBMCs cells were cultured at 37°C with 5% $CO_2$ and seeded in 96-well plates at $1\times10^7$ cells/well. The compounds to be tested (including those listed in Table 1, and Examples 1-467 compounds) were dissolved in DMSO and diluted to appropriate concentrations, ensuring that the final DMSO concentration in cell culture did not exceed 0.5%. After 1-hour incubation in medium with or without the compounds, the cells were stimulated with Lipopolysaccharide (LPS, 1 ng/ml) and continued to be cultured for 18-20 hours. Culture supernatants were then collected, diluted with serum-free medium, and analyzed for TNF-α levels using an ELISA kit. $IC_{50}$ values were calculated using GraphPad Prism 7.0.
TNF-α downregulation is a key mechanism for the anti-tumor action of immunomodulators. The compounds of the present disclosure demonstrated dose-dependent inhibition of TNF-α levels.

### V. Pharmacokinetic Study of the compounds of the present disclosure

The pharmacokinetic properties of the compounds of the present disclosure were evaluated using conventional pharmacokinetic experimental methods.

The test compounds of the present disclosure (including the compounds in Table 1, and the Examples 1-467 compounds) were orally administered to experimental animals to evaluate their pharmacokinetic properties. The experimental animals used may be those commonly used in pharmacokinetic experiments, e.g., adult and healthy rodents (e.g., mice, rats (such as Sprague-Dawley (SD) rats), guinea pigs) or non-rodents (rabbits, dogs, and monkeys). The experimental animals used in this study were mice.

The experimental animals were divided into two groups: a control group (for blank plasma collection) and an oral administration group (dosed at 10 mg/kg or 30mg/kg). Blood samples were collected at predetermined time points after administration, such as 0.25h, 0.5h, 1h, 2h, 4h, 8h, and 24h post-dose. Blank plasma was collected from the control group.

Prior to analysis, plasma samples were processed as follows: To 10 μL of plasma sample were added 10 μL of 50% acetonitrile and 200 μL of internal standard solution (containing 50 ng/mL dexamethasone in acetonitrile). For blank control plasma samples, no internal standard was added, instead an equivalent volume of acetonitrile was supplemented. The processed samples were vortex-mixed, centrifuged, and the supernatant was subjected to LC-MS/MS analysis.

After preparation of calibration curves and QC samples, the concentrations of compounds in plasma samples were determined by LC-MS/MS analysis. The pharmacokinetic parameters of the test compounds were calculated using the pharmacokinetic calculation software WinNonlin v8.1 with non-compartmental analysis based on the plasma concentration data obtained from LC-MS/MS.

The results demonstrated that the compounds of the present disclosure administered via oral route exhibited favorable drug metabolism and pharmacokinetic (DMPK) properties, indicating their potential as therapeutic agents for cancer patients.

### VI. Effects of the compounds of the present disclosure on TNF-α, IL-10, IL-6, IL-1β, IFN-γ, GM-CSF, IL-2, and IL-12p40 release from PBMCs induced by Lipopolysaccharide (LPS) or Phytohemagglutinin (PHA)

The following materials were used in this study:

VI.1 Reagents and Consumable materials

| Name | Supplier or Source |
|---|---|
| Human PBMC cell | Shanghai Sai Li |
| Lipopolysaccharide (LPS) | Sigma |
| Phytohemagglutinin (PHA) | MCE |
| RPMI 1640 Medium | Gibco |
| Human TNF-alpha DuoSet ELISA | R&D Systems |
| Human IL-6 DuoSet ELISA | R&D Systems |
| Human IL-10 DuoSet ELISA | R&D Systems |
| Human IFN-gamma DuoSet ELISA | R&D Systems |
| Human IL-2 DuoSet ELISA | R&D Systems |
| Human IL-1 beta ELISA Kit | Abclonal |
| Human GM-CSF ELISA Kit | Abclonal |
| Human IL-12/IL-23 p40 ELISA Kit | Abclonal |

VI.2 Methods

VI.2.1 Cell Thawing and Plating

A complete medium was prepared and pre-warmed to 37°C. Cryovials containing the cells were retrieved from liquid nitrogen, and the area immediately below the cap-notch interface was firmly gripped with forceps. The cryovials were immersed in a 37°C water bath, and shook occasionally to facilitate thawing. The cell suspension was aspirated and transferred to a centrifuge tube containing 10 mL complete medium, followed by thorough mixing and centrifugation at 400

× g for 10 min at room temperature. The supernatant was discarded, and the pellet was resuspended in fresh complete medium. After cell counting and the cell density adjustment, the cell suspension was seeded in 96-well plates at a density of $1\times10^5$ cells/well (75 μL/well) and incubated in an incubator for 2 h at 37°C with 5% $CO_2$ under high humidity conditions.

VI.2.2 Compound Preparation and Addition to Cell Plates

1) The test compounds were prepared as 10 mM stock solutions in DMSO.
2) The test compounds were serially diluted 5-fold in DMSO starting from 1 mM (highest concentration) to generate 9 concentration gradients.
3) The test compounds were further diluted in culture medium to five times their final working concentration intended for use.
4) The diluted compounds were added to designated wells according to the plate layout at 25 μL/well, achieving final concentrations starting from 1 μM with 5-fold serial dilution (9 concentration points in total).
5) After 1 h incubation, LPS or PHA (25 μL/well) was added to the cell wells to reach final concentrations of: 1 μg/mL LPS (for TNF-α, IL-10, IL-6, GM-CSF and IL-12p40), 5 μg/mL LPS (IL-1β), 50 μg/mL LPS (IFN-γ), or 1 μg/mL PHA (IL-2).
6) The plates were incubated in an incubator for 24 h at 37°C with 5% $CO_2$ under high humidity. Cell plate layout:

| | 1 | 2 | 3 | 4 | 5 | 6 | 7 | 8 | 9 | 10 | 11 | 12 |
|---|---|---|---|---|---|---|---|---|---|---|---|---|
| A | $H_2O$ | $H_2O$ | $H_2O$ | $H_2O$ | $H_2O$ | $H_2O$ | $H_2O$ | $H_2O$ | $H_2O$ | $H_2O$ | $H_2O$ | $H_2O$ |
| B | $H_2O$ | Cmpd 1# (1 μM Starting, 5-fold dilution, 9 points) | | | | | | | | | LPS/PHA | $H_2O$ |
| C | $H_2O$ | | | | | | | | | | | $H_2O$ |
| D | $H_2O$ | Cmpd 2# (1 μM Starting, 5-fold dilution, 9 points) | | | | | | | | | | $H_2O$ |
| E | $H_2O$ | | | | | | | | | | | $H_2O$ |
| F | $H_2O$ | Cmpd 3# (1 μM Starting, 5-fold dilution, 9 points) | | | | | | | | | Blank | $H_2O$ |
| G | $H_2O$ | | | | | | | | | | | $H_2O$ |
| H | $H_2O$ | $H_2O$ | $H_2O$ | $H_2O$ | $H_2O$ | $H_2O$ | $H_2O$ | $H_2O$ | $H_2O$ | $H_2O$ | $H_2O$ | $H_2O$ |

VI.2.3 Detection

(1) The cell plate was centrifuged and the culture supernatant was aspirated. TNF-α, IL-10, IL-6, IL-1β, IFN-γ, GM-CSF, IL-2, and IL-12p40 cytokines in the culture supernatant were detected according to the ELISA kit instructions.

The concentrations of TNF-α, IL-10, IL-6, IL-1β, IFN-γ, GM-CSF, IL-2, and IL-12p40 in the samples were calculated based on the standard curve. Inhibition rate % = [(Ac-As)/(Ac-Ab)]×100%

As: OA of the samples (cells + LPS/PHA + test compound)

Ac: OA of positive cell control (cells + LPS/PHA + DMSO)

Ab: OA of blank control (cells + culture medium + DMSO)

(2) $IC_{50}$ value was calculated as follows: using GraphPad Prism 6 software and applying the calculation formula XY-analysis/Nonlinear regression (curve fit)/Dose response-Inhibition/log (inhibitor) vs. response-Variable slope (four parameters) to perform $IC_{50}$ curve fitting and determine the $IC_{50}$ values.

The experimental results demonstrated that the compounds of the present disclosure can modulate the production levels of cytokines associated with autoimmune diseases and may be used for the treatment of autoimmune diseases.

**VII. Efficacy experiment of the compounds of the present disclosure on Psoriasis-like skin lesions induced by Imiquimod in Mice**

The compounds of the present disclosure (test compounds, including the compounds in Table 1, and Examples) were administered orally via gavage once daily for 7 consecutive days to mice with psoriasis-like skin lesions induced by imiquimod, to investigate their efficacy on such lesions.

A total of 60 female C57BL/6J mice (purchased from Shanghai SLAC Laboratory Animal Co., Ltd.) were randomly divided into 6 groups (n=10 per group) based on body weight:

| Group | Drug | Number of Animals | Dose (mg/kg) | Administration volume (mL/kg) | Administration concentration (mg/mL) | Route and Duration |
|---|---|---|---|---|---|---|
| 1 | Control: Vehicle | 10 | NA | 10 | NA | ig. qd. 7 days |
| 2 | Model: Vehicle | 10 | NA | 10 | NA | ig. qd. 7 days |
| 3 | Positive Control | 10 | 3 | 10 | 0.3 | sc. qd. 7 days |
| 4 | Low-dose test compounds group | 10 | 3 | 10 | 0.3 | ig. qd. 7 days |
| 5 | Medium-dose test compounds group | 10 | 10 | 10 | | ig. qd. 7 days |
| 6 | High-dose test compounds group | 10 | 30 | 10 | 3 | ig. qd. 7 days |
| Note: ig., oral gavage; sc., subcutaneous injection; qd., once daily. | | | | | | |

After grouping the experimental animals, the backs of all mice were shaved. Except for the Control group, the remaining groups were topically treated with imiquimod cream once daily for 7 consecutive days. Simultaneously, each group was administered with a vehicle control, a positive control drug, or the test compounds, according to the above table, once daily for 7 consecutive days.

All clinical symptoms of each animal were observed at the beginning and throughout the experiment. The animals' weights were measured and recorded every 2 days.

Scoring

Gross Observation Scoring of Skin Lesions (PASI Method): during the last 3 days of modeling and drug administration, the changes in skin lesions of mice in each group were observed once daily. PASI Method: the erythema, scaling, and thickening/infiltration of the lesions were scored separately, and the scores were summed to obtain the total score. PASI Scoring Criteria: 0 = none; 1 = mild; 2 = moderate; 3 = severe; 4 = very severe.

The skin lesions were photographed once daily during the last 3 days of modeling and drug administration.

After the experiment, the mice were euthanized by $CO_2$ inhalation. Three different areas of skin from the lesion site on the back were collected using a 6 mm punch, weighed, and recorded, and the average weight was calculated.

The skin tissue from the lesion site was homogenized and the levels of IL-23p19, IL-12p40, IL-17, and TNF-$\alpha$ were determined.

The local lesion tissue was fixed, stained with HE, and subjected to pathological scoring (based on epidermal thickness, degree of epidermal keratinization, thickness of the basal layer, and degree of dermal inflammatory cell infiltration). Scoring Criteria: 0 = none; 1 = mild; 2 = moderate; 3 = severe; 4 = very severe.

Experimental data were presented as Mean + SD. Statistical analysis between two groups was performed using SPSS, with $p < 0.05$ considered statistically significant.

The experimental results demonstrated that the compounds of the present disclosure improve psoriasis-like skin lesions on the back of mice induced by imiquimod and may be used for the treatment of psoriasis.

**VIII. Efficacy Experiment of the compounds of the present disclosure on the CIA Model in Rats Induced by Type II Collagen**

The compounds of the present disclosure (test compounds, including the compounds in Table 1, and Examples 1-467) were administered orally via gavage once daily for 21 consecutive days to Wistar rats with collagen-induced arthritis (CIA) that was induced using bovine type II collagen (Chondrex, Inc.), in order to investigate their efficacy in the rat arthritis model.

Preparation of the Modeling Agent:

10 mg of CII (Immunization Grade Bovine Type II Collagen, purchased from Chondrex, Inc.) was dissolved in 5 mL of 0.05M acetic acid solution, allowing it to be fully dissolved, thereby preparing a solution with a concentration of 2 mg/mL, which was then stored at 4°C in the dark overnight. On the day of the experiment, the 2 mg/mL CII solution was mixed with an equal volume of a 4 mg/mL solution of Complete Freund's Adjuvant (CFA, also purchased from Chondrex, Inc.) on ice, and was thoroughly emulsified into an emulsion.

On day 0, 10 Wistar rats (purchased from Beijing Vital River Laboratory Animal Technology Co., Ltd.) were randomly selected as the blank control group and were not immunized. The remaining 100 Wistar rats were intradermally injected at the base of the tail with an emulsion prepared by mixing equal volumes of CII (2 mg/mL) and CFA (4 mg/mL) for the first immunization. A second booster immunization was administered to them on day 7. Between days 10 and 14, i.e., 3 to 7 days after the second booster immunization, 50 rats with an arthritis index (AI) score ranging from 1 to 2 were selected from the modeled animals and were divided into 5 groups based on their body weight, foot volume, and AI score, with 10 rats per group:

| Group | Drug | Number of Animals | Dose (mg/kg) | Administration volume (mL/kg) | Administration concentration (mg/mL) | Route and Duration |
|---|---|---|---|---|---|---|
| 1 | Control: Vehicle | 10 | NA | 10 | NA | ig. qd. 21 days |
| 2 | Model: Vehicle | 10 | NA | 10 | NA | ig. qd. 21 days |
| 3 | Positive Control | 10 | 0.1 | 10 | 0.01 | sc. qd. 21 days |
| 4 | Low-dose test compounds group | 10 | 3 | 10 | 0.3 | ig. qd. 21 days |
| 5 | Medium-dose test compounds group | 10 | 10 | 10 | | ig. qd. 21 days |
| 6 | High-dose test compounds group | 10 | 30 | 10 | 3 | ig. qd. 21 days |
| Note: ig. = oral gavage; sc. = subcutaneous injection | | | | | | |

Administration commenced between 3 and 7 days after the second booster immunization, once the AI score of the affected paws reached 1-2. According to the table above, animals in each group received daily oral gavage (or subcutaneous injection) of either the vehicle control, positive control drug, or the test compounds for 21 consecutive days.

Beginning on day 3 after the second booster immunization, the volume of both hind paws (or toes) was measured and recorded twice weekly.

Foot Volume Measurement: Before the measurement, a line was marked on the rat's ankle joint with a marker pen to indicate the position. After clean water was added to the instrument, the instrument's reading was reset to zero in preparation for the measurement. The hind limb of the rat was placed in the water so that the marked line at the ankle joint was at the surface of the liquid. The reading obtained by pressing the foot pedal at this time was the foot volume of the rat. After the measurement was completed, the foot pedal was pressed again to reset the instrument to zero, preparing it for the measurement of the next rat.

Scoring

Arthritis Index (AI): The foot volume was measured twice a week, and the swelling of the four toes was scored simultaneously. Each foot was scored on a scale from 0 to 4, with a maximum score of 16 for each rat.

Arthritis Index (AI) Scoring Criteria:

| Score | Degree |
|---|---|
| 0 | No swelling, normal appearance |
| 1 | Mild swelling or redness of the ankle, wrist, or finger joints |
| 2 | Moderate swelling or redness of the ankle, wrist, or finger joints |
| 3 | Severe swelling or redness of the ankle, wrist, or finger joints |
| 4 | Very severe swelling or redness of the ankle, wrist, or finger joints |

The day of starting drug administration is recorded as Day 0. Five days after starting drug administration, i.e., 2 hours after Day 5 administration, blood was collected from the jugular vein of the experimental animals, allowed to stand for more than 30 minutes, centrifuged to separate the serum, which was stored at -80°C. The levels of TNF-α, IL-1β, and IL-6 in serum were measured by ELISA.

After the experiment, the animals were euthanized by $CO_2$ inhalation. The spleen and thymus of the animals were collected and weighed, and the organ coefficients were calculated.

Pathological Examination: One side of the foot joint tissue was fixed in 10% neutral formalin solution, followed by decalcification and paraffin embedding to prepare pathological sections. The sections were then stained with hematoxylin and eosin (HE) for pathological observation.

Observation Indices:

1. Infiltration of synovial inflammatory cells (lymphocytes, plasma cells);
2. Synovial tissue hyperplasia;
3. Synovial pannus;
4. Fibrinoid necrosis on the synovial surface.

Scoring and Counting Method: 0, 1, 2, 3, and 4 levels, where "0" indicates no observation; "1" is mild; "2" is moderate; "3" is severe; and "4" is extremely severe.

Experimental data were presented as Mean ± SEM. Data between two groups were analyzed using Excel-T test, with $p<0.05$ considered statistically significant. Scoring data were analyzed using the non-parametric Mann-Whitney U test in SPSS, with $p<0.05$ considered statistically significant.

The experimental results demonstrated that the compounds of the present disclosure can regulate the serum levels of inflammatory cytokines in rats with collagen-induced arthritis (CIA) model that was induced using bovine type II collagen, and significantly improve the swelling of rat limbs, and thus may be used for the treatment of arthritis.

Although the preferred embodiments of the present invention have been described in detail herein, it is to be understood that the invention is not limited to the specific structures and steps explicitly described and illustrated. Other modifications and variations may be made by those skilled in the art without departing from the spirit and scope of the invention.

## Claims

**1.** A compound of Formula (I):

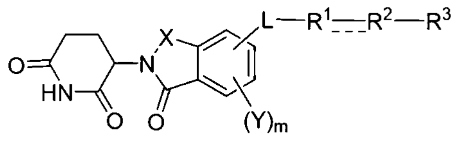

(I)

or salts, enantiomers, stereoisomers, solvates, isotopically enriched analogs, prodrugs, or polymorphs thereof, wherein $\overline{----}$ represents a single or double bond;

X represents $CH_2$ or C(=O);

$(Y)_m$ indicates that benzene ring is optionally substituted with m Y groups, wherein Y represents deuterium, $C_1$-$C_4$ alkyl, halogen or halogenated $C_1$-$C_2$ alkyl, and m represents an integer of 0, 1, 2 or 3;

L represents methylene or linear or branched $C_2$-$C_6$ alkylene, wherein the methylene or linear or branched $C_2$-$C_6$ alkylene is optionally substituted with one or more substituents selected from the group consisting of: deuterium, halogen, $C_1$-$C_3$ alkyl, $C_1$-$C_3$ alkoxy, and halogenated $C_1$-$C_3$ alkyl;

$R^1$ represents a bond, O, $N(R^a)$, $N(R^a)S(O)_2$, $S(O)_2N(R^a)$, nitrogen-containing heterocyclylene or nitrogen-containing heterocyclylylidene, wherein $R^a$ represents hydrogen, deuterium or $C_1$-$C_6$ alkyl, and the nitrogen-containing heterocyclylene or nitrogen-containing heterocyclylylidene is optionally substituted with one or more substituents selected from the group consisting of: deuterium, hydroxy, amino, mercapto, =O, halogen, cyano, $C_1$-$C_6$ alkyl, halogenated $C_1$-$C_6$ alkyl, $C_1$-$C_6$ alkoxy and halogenated $C_1$-$C_6$ alkoxy;

$R^2$ represents heteroarylylidene, heteroarylene, heterocyclylene, $(CH_2)_n$-arylene or $(CH_2)_n$-cycloalkylene, wherein n represents an integer of 0, 1 or 2, and the heteroarylylidene, heteroarylene, heterocyclylene, arylene or cycloalkylene is optionally substituted with one or more substituents selected from the group consisting of: deuterium, hydroxy, amino, mercapto, =O, halogen, cyano, $C_1$-$C_6$ alkyl, halogenated $C_1$-$C_6$ alkyl, $C_1$-$C_6$ alkoxy, and halogenated $C_1$-$C_6$ alkoxy;

$R^3$ represents hydrogen, deuterium, $SR^b$, $R^b$, halogenated $C_1$-$C_6$ alkyl, cycloalkyl or nitrogen-containing heterocyclyl, wherein $R^b$ represents aryl, and the aryl, cycloalkyl or nitrogen-containing heterocyclyl is optionally substituted with one or more substituents selected from the group consisting of: deuterium, hydroxy, amino, mercapto, =O, halogen, cyano, $C_1$-$C_6$ alkyl, halogenated $C_1$-$C_6$ alkyl, $C_1$-$C_6$ alkoxy, and halogenated $C_1$-$C_6$ alkoxy;

wherein when $\overline{----}$ represents a double bond, $R^1$ represents nitrogen-containing heterocyclylylidene, and $R^2$ represents heteroarylylidene;

when $\overline{----}$ represents a single bond, $R^1$ represents a bond, O, $N(R^a)$, $N(R^a)S(O)_2$, $S(O)_2N(R^a)$ or nitrogen-containing heterocyclylene, and $R^2$ represents heteroarylene, heterocyclylene, $(CH_2)_n$-arylene or $(CH_2)_n$-cycloalkylene.

2. The compound of Formula (I) or salts, enantiomers, stereoisomers, solvates, isotopically enriched analogs, prodrugs, or polymorphs thereof as claimed in claim 1, wherein the structure of Formula (I) is also represented by the following formula:

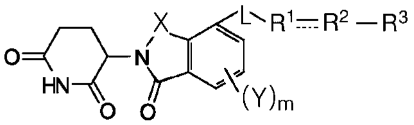
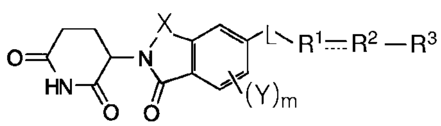

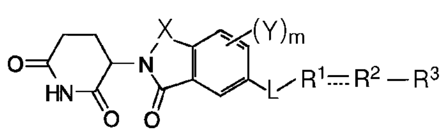
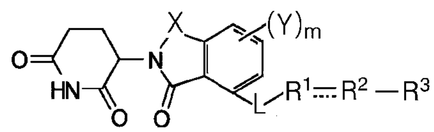

(I-c)

wherein $\overline{----}$, X, Y, L, m, $R^1$, $R^2$, and $R^3$ are as defined in claim 1.

3. The compound of Formula (I) or salts, enantiomers, stereoisomers, solvates, isotopically enriched analogs, prodrugs, or polymorphs thereof as claimed in claim 1, wherein the structure of Formula (I) is also represented by the following

formula:

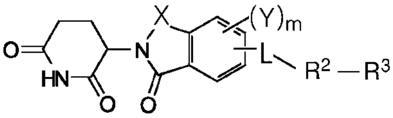

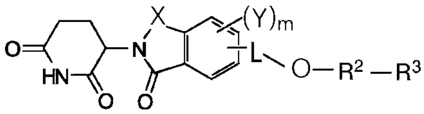

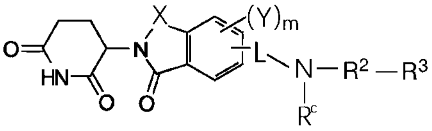

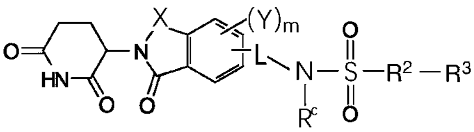

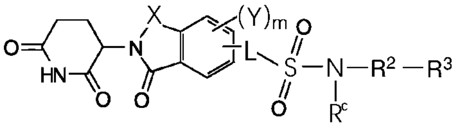

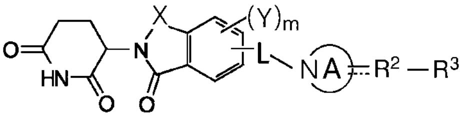

wherein $\overline{----}$, X, Y, L, m, $R^2$, and $R^3$ are as defined in claim 1;
$R^c$ represents hydrogen, deuterium or $C_1$-$C_6$ alkyl;
ring A represents nitrogen-containing heterocyclylene or nitrogen-containing heterocyclylylidene, wherein the nitrogen-containing heterocyclylene or nitrogen-containing heterocyclylylidene is optionally substituted with one or more substituents selected from the group consisting of: deuterium, hydroxy, amino, mercapto, =O, halogen, cyano, $C_1$-$C_6$ alkyl, halogenated $C_1$-$C_6$ alkyl, $C_1$-$C_6$ alkoxy, and halogenated $C_1$-$C_6$ alkoxy.

**4.** The compound of Formula (I) or salts, enantiomers, stereoisomers, solvates, isotopically enriched analogs, prodrugs, or polymorphs thereof as claimed in claim 1, wherein the structure of Formula (I) is also represented by the following formula:

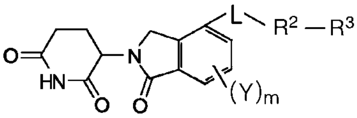

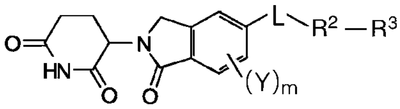

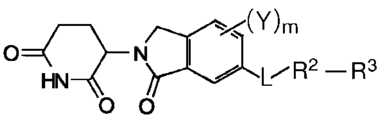

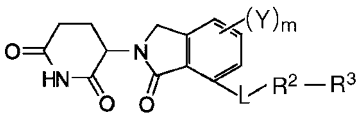

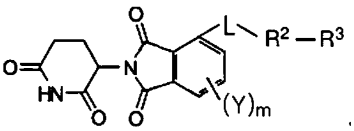

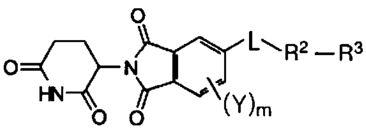

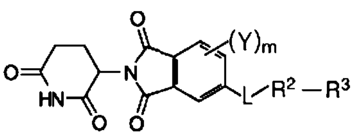

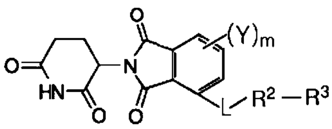

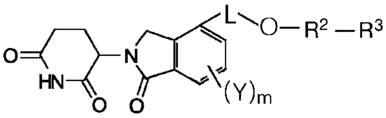

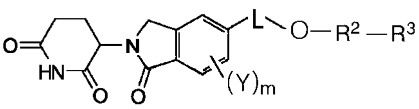

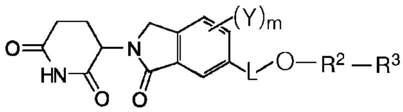
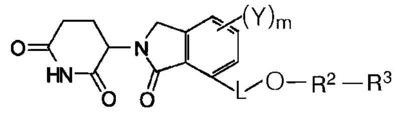

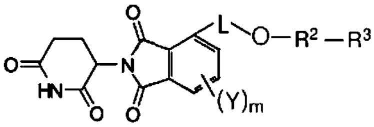
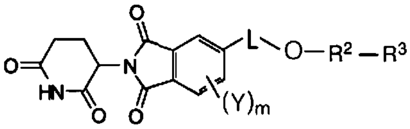

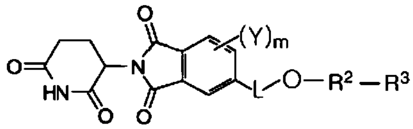
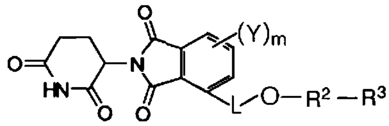

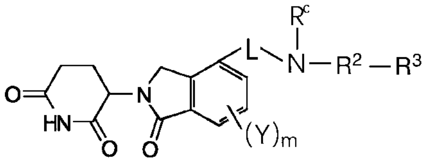
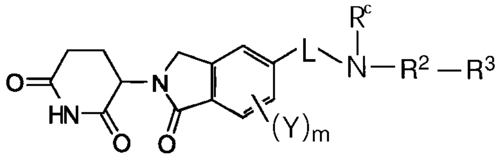

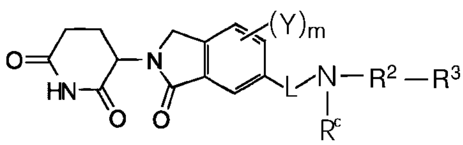
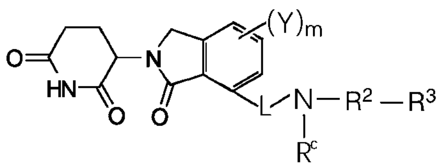

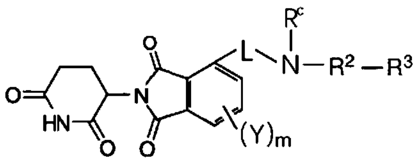
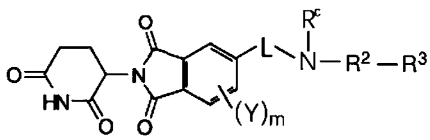

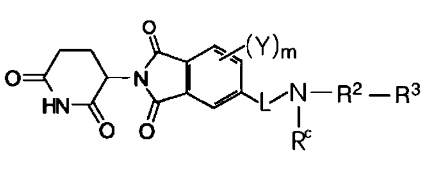
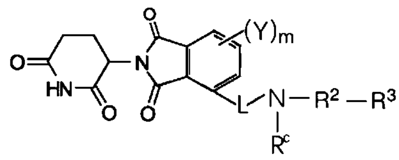

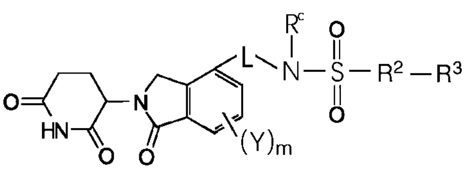
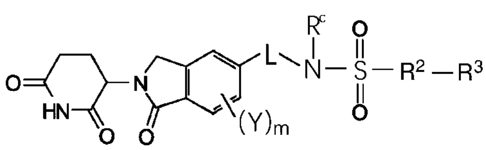

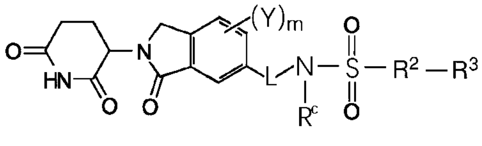
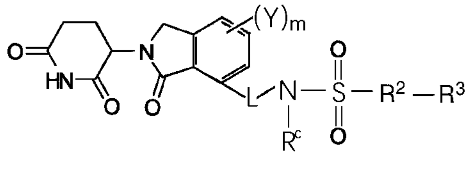

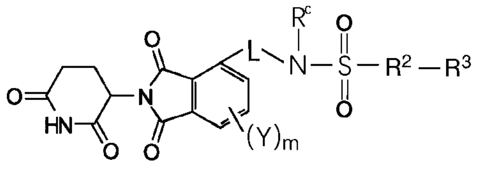
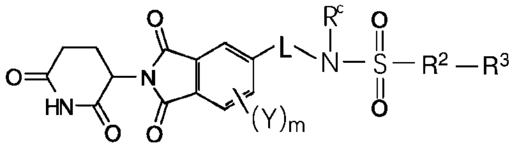

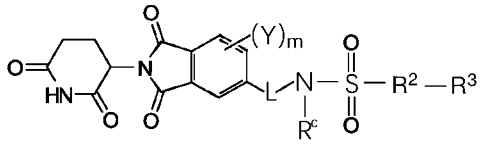
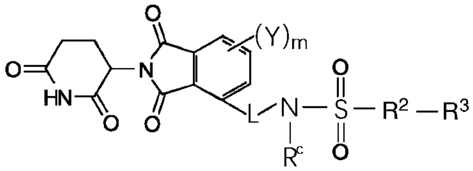

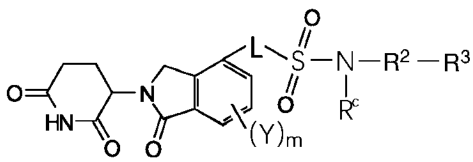
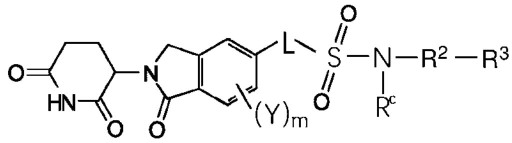

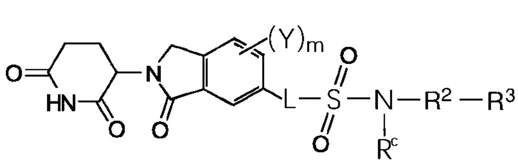
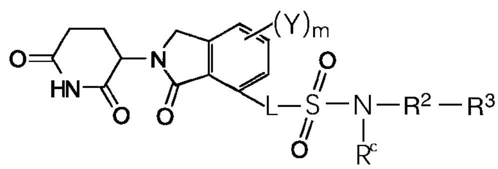

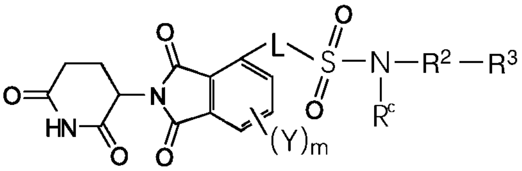
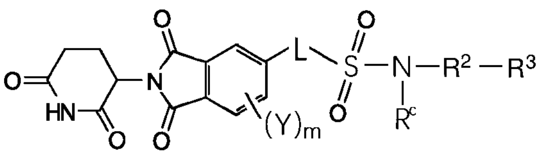

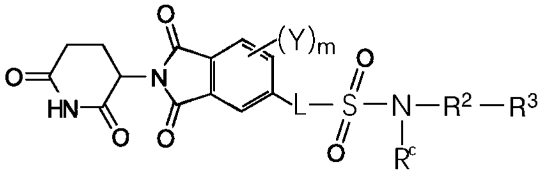
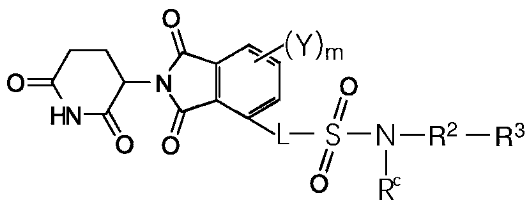

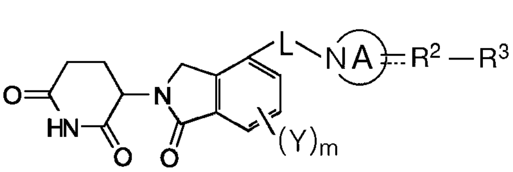
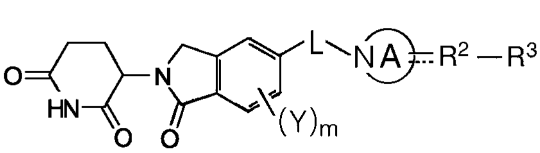

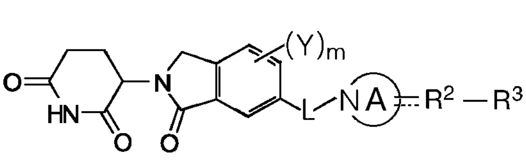
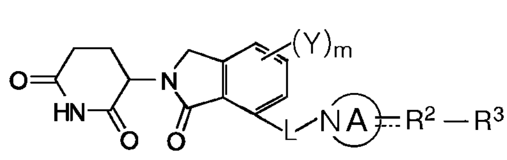

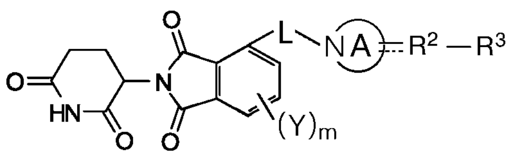
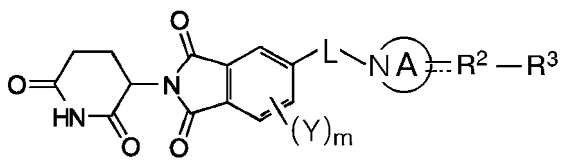

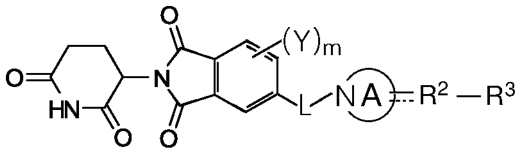 or 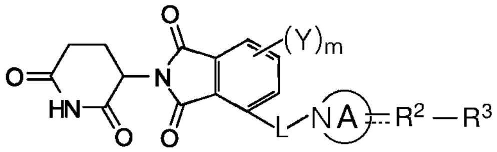 ;

wherein , Y, L, m, $R^2$, and $R^3$ are as defined in claim 1;
$R^c$ represents hydrogen, deuterium or $C_1$-$C_6$ alkyl;
ring A represents nitrogen-containing heterocyclylene or nitrogen-containing heterocyclylylidene, wherein the nitrogen-containing heterocyclylene or nitrogen-containing heterocyclylylidene is optionally substituted with one or more substituents selected from the group consisting of: deuterium, hydroxy, amino, mercapto, =O, halogen, cyano, $C_1$-$C_6$ alkyl, halogenated $C_1$-$C_6$ alkyl, $C_1$-$C_6$ alkoxy, and halogenated $C_1$-$C_6$ alkoxy.

**5.** The compound of Formula (I) or salts, enantiomers, stereoisomers, solvates, isotopically enriched analogs, prodrugs, or polymorphs thereof as claimed in any one of claims 1-4, wherein Y represents D, F, Cl, Br, I, $CF_3$, $CH_2F$, $CHF_2$, $CH_2Cl$, $CHCl_2$, $CF_2CF_3$, $CHFCF_3$, $CF_2CHF_2$, $CHFCHF_2$, $CH_2CF_3$ or $CH_2CH_2Cl$.

**6.** The compound of Formula (I) or salts, enantiomers, stereoisomers, solvates, isotopically enriched analogs, prodrugs, or polymorphs thereof as claimed in any one of claims 1-5, wherein the nitrogen-containing heterocyclylene comprises: 4- to 30-membered nitrogen-containing heterocyclylene, 4- to 20-membered nitrogen-containing heterocyclylene, 4- to 15-membered nitrogen-containing heterocyclylene, 5- to 30-membered nitrogen-containing heterocyclylene, 5 to 20-membered nitrogen-containing heterocyclylene or 5-to 15-membered nitrogen-containing heterocyclylene, wherein the nitrogen-containing heterocyclylene is optionally substituted with one or more substituents selected from the group consisting of: deuterium, hydroxy, amino, mercapto, =O, halogen, cyano, $C_1$-$C_6$ alkyl, halogenated $C_1$-$C_6$ alkyl, $C_1$-$C_6$ alkoxy, and halogenated $C_1$-$C_6$ alkoxy.

**7.** The compound of Formula (I) or salts, enantiomers, stereoisomers, solvates, isotopically enriched analogs, prodrugs, or polymorphs thereof as claimed in any one of claims 1-6, wherein the nitrogen-containing heterocyclylylidene comprises: 4- to 30-membered nitrogen-containing heterocyclylylidene, 4- to 20-membered nitrogen-containing heterocyclylylidene, 4- to 15-membered nitrogen-containing heterocyclylylidene, 5- to 30-membered nitrogen-containing heterocyclylylidene, 5 to 20-membered nitrogen-containing heterocyclylylidene or 5- to 15-membered nitrogen-containing heterocyclylylidene, wherein the nitrogen-containing heterocyclylylidene is optionally substituted with one or more substituents selected from the group consisting of: deuterium, hydroxy, amino, mercapto, =O, halogen, cyano, $C_1$-$C_6$ alkyl, halogenated $C_1$-$C_6$ alkyl, $C_1$-$C_6$ alkoxy, and halogenated $C_1$-$C_6$ alkoxy.

**8.** The compound of Formula (I) or salts, enantiomers, stereoisomers, solvates, isotopically enriched analogs, prodrugs, or polymorphs thereof as claimed in any one of claims 1-7, wherein the structure of the nitrogen-containing heterocyclylene comprises:

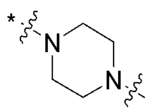 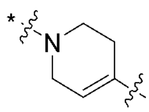 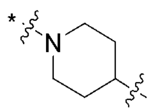 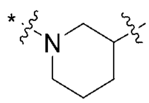 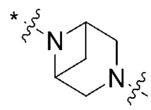 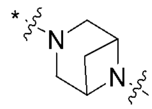

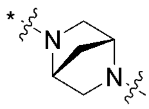 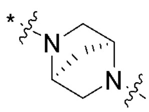 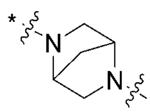 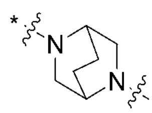 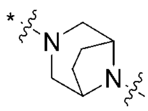 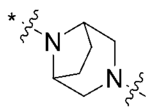

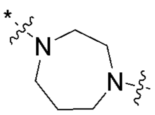 or 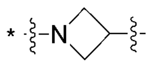 ;

wherein symbol * indicates the point of attachment to L.

**9.** The compound of Formula (I) or salts, enantiomers, stereoisomers, solvates, isotopically enriched analogs, prodrugs,

or polymorphs thereof as claimed in any one of claims 1-8, wherein $R^2$ represents heteroarylylidene, arylene, heteroarylene, heterocyclylene, cycloalkylene, -$CH_2$-arylene, -$CH_2$-cycloalkylene, -$(CH_2)_2$-arylene or -$(CH_2)_2$-cycloalkylene, wherein the heteroarylylidene, arylene, heteroarylene, heterocyclylene or cycloalkylene is optionally substituted with one or more substituents selected from the group consisting of: deuterium, hydroxy, amino, mercapto, =O, halogen, cyano, $C_1$-$C_6$ alkyl, halogenated $C_1$-$C_6$ alkyl, $C_1$-$C_6$ alkoxy, and halogenated $C_1$-$C_6$ alkoxy.

**10.** The compound of Formula (I) or salts, enantiomers, stereoisomers, solvates, isotopically enriched analogs, prodrugs, or polymorphs thereof as claimed in any one of claims 1-8, wherein $R^2$ represents 7- to 30-membered heteroarylylidene, arylene, 5- to 30-membered heteroarylene, 4- to 30-membered heterocyclylene, cycloalkylene, -$CH_2$-arylene, -$CH_2$-cycloalkylene, -$(CH_2)_2$-arylene or -$(CH_2)_2$-cycloalkylene, wherein the arylene comprises $C_{5-30}$ arylene, and the cycloalkylene comprises $C_{3-30}$ cycloalkylene; and wherein the heteroarylylidene, arylene, heteroarylene, heterocyclylene or cycloalkylene is optionally substituted with one or more substituents selected from the group consisting of: deuterium, hydroxy, amino, mercapto, =O, halogen, cyano, $C_1$-$C_6$ alkyl, halogenated $C_1$-$C_6$ alkyl, $C_1$-$C_6$ alkoxy, and halogenated $C_1$-$C_6$ alkoxy.

**11.** The compound of Formula (I) or salts, enantiomers, stereoisomers, solvates, isotopically enriched analogs, prodrugs, or polymorphs thereof as claimed in any one of claims 1-10, wherein

the heteroarylylidene is chromanylylidene or 2,3-dihydroquinolin-4(1H)-ylylidene, wherein the heteroarylylidene is optionally substituted with one or more substituents selected from the group consisting of: deuterium, hydroxy, amino, mercapto, =O, halogen, cyano, $C_1$-$C_6$ alkyl, halogenated $C_1$-$C_6$ alkyl, $C_1$-$C_6$ alkoxy, and halogenated $C_1$-$C_6$ alkoxy;
the arylene is selected from the group consisting of: phenylene or naphthylene, wherein the arylene is optionally substituted with one or more substituents selected from the group consisting of: deuterium, hydroxy, amino, mercapto, =O, halogen, cyano, $C_1$-$C_6$ alkyl, halogenated $C_1$-$C_6$ alkyl, $C_1$-$C_6$ alkoxy, and halogenated $C_1$-$C_6$ alkoxy;
the heteroarylene is selected from the group consisting of: furanylene, oxazolylene, isoxazolylene, oxadiazolylene, thienylene, isothiazolylene, thiadiazolylene, pyrrolylene, imidazolylene, pyrazolylene, triazolylene, pyridylene, pyrimidinylene, pyridazinylene, pyrazinylene, indolylene, isoindolylene, indazolylene, quinolinylene, isoquinolinylene, quinazolinylene, triazinylene, isoxazolo[4,5-c]pyridinylene, pyrrolo[2,3-b]pyridinylene, indolinylene, benzothiazolylene, benzofuranylene, isobenzofuranylene, benzothienylene, benzimidazolylene, 2,3-dihydro-1H-benzo[d]imidazolylene, pyrrolo[2,3-b]pyridinylene, 3,4-dihydroquinolinylene, 2,3-dihydro-4H-benzo[b][1,4]oxazinylene, chromanylene, thieno[2,3-d]pyrimidinylene, thieno[3,2-d]pyrimidinylene, tetrahydrobenzo[4,5]thieno[2,3-d]pyrimidinylene, 6,7-dihydro-5H-cyclopenta[4,5]thieno[2,3-d]pyrimidinylene, dihydrothieno[3,2-d]pyrimidinylene, 5,6-dihydro-[1,2,4]triazolo[4,3-a]pyrazinylene, dihydrothieno[3,2-c]pyridinylene, benzoxazolylene, benzisoxazolylene, benzisothiazolylene, benzotriazolylene, benzo[2,1,3]oxadiazolylene, benzo[2,1,3]thiadiazolylene, benzo[1,2,3]thiadiazolylene, pyrazolo[1,5-a]pyridylene, pyrazolo[1,5-a]pyrimidinylene, imidazo[1,2-a]pyridylene, 1H-pyrrolo[3,2-b]thiazolylene, or benzo[2,1-b]thiazolylene, wherein the heteroarylene is optionally substituted with one or more substituents selected from the group consisting of: deuterium, hydroxy, amino, mercapto, =O, halogen, cyano, $C_1$-$C_6$ alkyl, halogenated $C_1$-$C_6$ alkyl, $C_1$-$C_6$ alkoxy, and halogenated $C_1$-$C_6$ alkoxy;
the heterocyclylene is selected from the group consisting of: azetidinylene, oxetanylene, pyrrolidinylene, imidazolidinylene, pyrrolidinylene, tetrahydrofuranylene, tetrahydropyranylene, tetrahydrothienylene, tetrahydrothiopyranylene, oxazolidinylene, thiazolidinylene, piperidinylene, piperazinylene, morpholinylene, thiomorpholinylene, dioxacyclohexylene, or diazacycloheptanylene, wherein the heterocyclylene is optionally substituted with one or more substituents selected from the group consisting of: deuterium, hydroxy, amino, mercapto, =O, halogen, cyano, $C_1$-$C_6$ alkyl, halogenated $C_1$-$C_6$ alkyl, $C_1$-$C_6$ alkoxy, and halogenated $C_1$-$C_6$ alkoxy;
the cycloalkylene is selected from the group consisting of: cyclopropylene, cyclobutylene, cyclopentylene, cyclopentenylene, cyclohexylene, cyclohexenylene, cycloheptylene, cyclooctylene, decalinylene, octahydropentalenylene, octahydro-1H-indenylene, $C_{5-15}$ spiro-cycloalkylene, adamantanylene, noradamantanylene, bornylene, bicyclo[2.2.1]heptylene, or bicyclo[2.2.1]heptenylene, wherein the cycloalkylene is optionally substituted with one or more substituents selected from the group consisting of: deuterium, hydroxy, amino, mercapto, =O, halogen, cyano, $C_1$-$C_6$ alkyl, halogenated $C_1$-$C_6$ alkyl, $C_1$-$C_6$ alkoxy, and halogenated $C_1$-$C_6$ alkoxy.

**12.** The compound of Formula (I) or salts, enantiomers, stereoisomers, solvates, isotopically enriched analogs, prodrugs, or polymorphs thereof as claimed in any one of claims 1-11, wherein

the aryl is selected from the group consisting of: phenyl and naphthyl, wherein the aryl is optionally substituted with one or more substituents selected from the group consisting of: deuterium, hydroxy, amino, mercapto, =O, halogen, cyano, $C_1$-$C_6$ alkyl, halogenated $C_1$-$C_6$ alkyl, $C_1$-$C_6$ alkoxy, and halogenated $C_1$-$C_6$ alkoxy;
the cycloalkyl is selected from the group consisting of: cyclopropyl, cyclobutyl, cyclopentyl, cyclopentenyl, cyclohexyl, cyclohexenyl, cycloheptyl, cyclooctyl, decalinyl, octahydropentalenyl, octahydro-1H-indenyl, $C_{5-15}$ spiro-cycloalkyl, adamantanyl, noradamantanyl, bornyl, bicyclo[2.2.1]heptylene, or bicyclo[2.2.1]heptenylene, wherein the cycloalkyl is optionally substituted with one or more substituents selected from the group consisting of: deuterium, hydroxy, amino, mercapto, =O, halogen, cyano, $C_1$-$C_6$ alkyl, halogenated $C_1$-$C_6$ alkyl, $C_1$-$C_6$ alkoxy, and halogenated $C_1$-$C_6$ alkoxy;
the nitrogen-containing heterocyclyl is selected from the group consisting of: azetidinyl, pyrrolidinyl, imidazolidinyl, diazacycloheptanyl, morpholinyl, piperazinyl, piperidinyl, pyrrolidinyl, and azetidinyl, wherein the nitrogen-containing heterocyclyl is optionally substituted with one or more substituents selected from the group consisting of: deuterium, hydroxy, amino, mercapto, =O, halogen, cyano, $C_1$-$C_6$ alkyl, halogenated $C_1$-$C_6$ alkyl, $C_1$-$C_6$ alkoxy, and halogenated $C_1$-$C_6$ alkoxy.

**13.** The compound of Formula (I) or salts, enantiomers, stereoisomers, solvates, isotopically enriched analogs, prodrugs, or polymorphs thereof as claimed in claim 1, wherein when $R^1$ represents O, NH, $NHS(O)_2$ or $S(O)_2NH$, $R^2$ represents $-(CH_2)_n$-cycloalkylene, and $R^3$ represents hydrogen, wherein the cycloalkylene is optionally substituted with one or more substituents selected from the group consisting of: deuterium, hydroxy, amino, mercapto, =O, halogen, cyano, $C_1$-$C_6$ alkyl, halogenated $C_1$-$C_6$ alkyl, $C_1$-$C_6$ alkoxy, and halogenated $C_1$-$C_6$ alkoxy.

**14.** The compound of Formula (I) or salts, enantiomers, stereoisomers, solvates, isotopically enriched analogs, prodrugs, or polymorphs thereof as claimed in claim 1, wherein when $\overline{- - - -}$ represents a double bond, $R^1$ represents piperidinylylidene or azetidinylylidene, $R^2$ represents chromanylylidene, and $R^3$ represents hydrogen.

**15.** The compound of Formula (I) or salts, enantiomers, stereoisomers, solvates, isotopically enriched analogs, prodrugs, or polymorphs thereof as claimed in any one of claims 1-12, which is selected from the group consisting of:

3-(1-oxo-5-((4-(thiophen-2-yl)piperazin-1-yl)methyl)isoindolin-2-yl)piperidine-2,6-dione;
3-(5-((4-(5-methylthiophen-2-yl)piperazin-1-yl)methyl)-1-oxoisoindolin-2-yl)piperidine-2,6-dione;
3-(5-((4-(3-methylthiophen-2-yl)piperazin-1-yl)methyl)-1-oxoisoindolin-2-yl)piperidine-2,6-dione;
3-(1-oxo-5-((4-(thiophen-3-yl)piperazin-1-yl)methyl)isoindolin-2-yl)piperidine-2,6-dione;
3-(5-((4-(4-methylthiophen-3-yl)piperazin-1-yl)methyl)-1-oxoisoindolin-2-yl)piperidine-2,6-dione;
3-(5-((4-(5-methylthiophen-3-yl)piperazin-1-yl)methyl)-1-oxoisoindolin-2-yl)piperidine-2,6-dione;
3-(1-oxo-5-((4-(thiophen-2-yl)-3,6-dihydropyridin-1(2H)-yl)methyl)isoindolin-2-yl)piperidine-2,6-dione;
3-(5-((4-(5-methylthiophen-2-yl)-3,6-dihydropyridin-1(2H)-yl)methyl)-1-oxoisoindolin-2-yl)piperidine-2,6-dione;
3-(5-((4-(3-methylthiophen-2-yl)-3,6-dihydropyridin-1(2H)-yl)methyl)-1-oxoisoindolin-2-yl)piperidine-2,6-dione;
3-(1-oxo-5-((4-(thiophen-3-yl)-3,6-dihydropyridin-1(2H)-yl)methyl)isoindolin-2-yl)piperidine-2,6-dione;
3-(5-((4-(4-methylthiophen-3-yl)-3,6-dihydropyridin-1(2H)-yl)methyl)-1-oxoisoindolin-2-yl)piperidine-2,6-dione;
3-(5-((4-(5-methylthiophen-3-yl)-3,6-dihydropyridin-1(2H)-yl)methyl)-1-oxoisoindolin-2-yl)piperidine-2,6-dione;
3-(5-((4-(furan-2-yl)-3,6-dihydropyridin-1(2H)-yl)methyl)-1-oxoisoindolin-2-yl)piperidine-2,6-dione;
3-(5-((4-(furan-3-yl)-3,6-dihydropyridin-1(2H)-yl)methyl)-1-oxoisoindolin-2-yl)piperidine-2,6-dione;
3-(1-oxo-5-((4-(thiophen-2-yl)piperidin-1-yl)methyl)isoindolin-2-yl)piperidine-2,6-dione;
3-(5-((4-(5-methylthiophen-2-yl)piperidin-1-yl)methyl)-1-oxoisoindolin-2-yl)piperidine-2,6-dione;
3-(5-((4-(3-methylthiophen-2-yl)piperidin-1-yl)methyl)-1-oxoisoindolin-2-yl)piperidine-2,6-dione;
3-(1-oxo-5-((4-(thiophen-3-yl)piperidin-1-yl)methyl)isoindolin-2-yl)piperidine-2,6-dione;
3-(5-((4-(5-methylthiophen-3-yl)piperidin-1-yl)methyl)-1-oxoisoindolin-2-yl)piperidine-2,6-dione;
3-(5-((4-(4-methylthiophen-3-yl)piperidin-1-yl)methyl)-1-oxoisoindolin-2-yl)piperidine-2,6-dione;
3-(5-((4-(furan-2-yl)piperidin-1-yl)methyl)-1-oxoisoindolin-2-yl)piperidine-2,6-dione;
3-(5-((4-(furan-3-yl)piperidin-1-yl)methyl)-1-oxoisoindolin-2-yl)piperidine-2,6-dione;
3-(5-((4-(2,3-dichlorophenyl)piperazin-1-yl)methyl)-1-oxoisoindolin-2-yl)piperidine-2,6-dione;
3-(1-oxo-5-((4-phenylpiperazin-1-yl)methyl)isoindolin-2-yl)piperidine-2,6-dione;
3-(5-((4-(2-methoxyphenyl)piperazin-1-yl)methyl)-1-oxoisoindolin-2-yl)piperidine-2,6-dione;
3-(5-((4-(3-methoxyphenyl)piperazin-1-yl)methyl)-1-oxoisoindolin-2-yl)piperidine-2,6-dione;
3-(5-((4-(4-methoxyphenyl)piperazin-1-yl)methyl)-1-oxoisoindolin-2-yl)piperidine-2,6-dione;
3-(1-oxo-5-((4-(4-(trifluoromethyl)phenyl)piperazin-1-yl)methyl)isoindolin-2-yl)piperidine-2,6-dione;
3-(5-((4-(3-fluorophenyl)piperazin-1-yl)methyl)-1-oxoisoindolin-2-yl)piperidine-2,6-dione;
3-(5-((4-(4-fluorophenyl)piperazin-1-yl)methyl)-1-oxoisoindolin-2-yl)piperidine-2,6-dione;

3-(5-((4-(2-chlorophenyl)piperazin-1-yl)methyl)-1-oxoisoindolin-2-yl)piperidine-2,6-dione;
3-(5-((4-(4-chlorophenyl)piperazin-1-yl)methyl)-1-oxoisoindolin-2-yl)piperidine-2,6-dione;
3-(5-((4-(2-bromophenyl)piperazin-1-yl)methyl)-1-oxoisoindolin-2-yl)piperidine-2,6-dione;
3-(5-((4-(3-bromophenyl)piperazin-1-yl)methyl)-1-oxoisoindolin-2-yl)piperidine-2,6-dione;
3-(5-((4-(4-bromophenyl)piperazin-1-yl)methyl)-1-oxoisoindolin-2-yl)piperidine-2,6-dione;
3-(1-oxo-5-((4-(o-tolyl)piperazin-1-yl)methyl)isoindolin-2-yl)piperidine-2,6-dione;
3-(1-oxo-5-((4-(m-tolyl)piperazin-1-yl)methyl)isoindolin-2-yl)piperidine-2,6-dione;
3-(1-oxo-5-((4-(p-tolyl)piperazin-1-yl)methyl)isoindolin-2-yl)piperidine-2,6-dione;
3-(5-((4-(2,4-dichlorophenyl)piperazin-1-yl)methyl)-1-oxoisoindolin-2-yl)piperidine-2,6-dione;
3-(5-((4-(2,5-dichlorophenyl)piperazin-1-yl)methyl)-1-oxoisoindolin-2-yl)piperidine-2,6-dione;
3-(5-((4-(2,6-dichlorophenyl)piperazin-1-yl)methyl)-1-oxoisoindolin-2-yl)piperidine-2,6-dione;
3-(5-((4-(3,4-dichlorophenyl)piperazin-1-yl)methyl)-1-oxoisoindolin-2-yl)piperidine-2,6-dione;
3-(5-((4-(3,5-dichlorophenyl)piperazin-1-yl)methyl)-1-oxoisoindolin-2-yl)piperidine-2,6-dione;
3-(5-((4-(2,3-difluorophenyl)piperazin-1-yl)methyl)-1-oxoisoindolin-2-yl)piperidine-2,6-dione;
3-(5-((4-(2,4-difluorophenyl)piperazin-1-yl)methyl)-1-oxoisoindolin-2-yl)piperidine-2,6-dione;
3-(5-((4-(2,5-difluorophenyl)piperazin-1-yl)methyl)-1-oxoisoindolin-2-yl)piperidine-2,6-dione;
3-(5-((4-(2,6-difluorophenyl)piperazin-1-yl)methyl)-1-oxoisoindolin-2-yl)piperidine-2,6-dione;
3-(5-((4-(3,4-difluorophenyl)piperazin-1-yl)methyl)-1-oxoisoindolin-2-yl)piperidine-2,6-dione;
3-(5-((4-(3,5-difluorophenyl)piperazin-1-yl)methyl)-1-oxoisoindolin-2-yl)piperidine-2,6-dione;
3-(5-((4-(2,4-dimethylphenyl)piperazin-1-yl)methyl)-1-oxoisoindolin-2-yl)piperidine-2,6-dione;
3-(5-((4-(5-chloro-2-methylphenyl)piperazin-1-yl)methyl)-1-oxoisoindolin-2-yl)piperidine-2,6-dione;
3-(1-oxo-5-((4-phenylpiperidin-1-yl)methyl)isoindolin-2-yl)piperidine-2,6-dione;
3-(5-((4-(3-bromophenyl)piperidin-1-yl)methyl)-1-oxoisoindolin-2-yl)piperidine-2,6-dione;
3-(5-((4-(4-bromophenyl)piperidin-1-yl)methyl)-1-oxoisoindolin-2-yl)piperidine-2,6-dione;
3-(5-((4-(2-chlorophenyl)piperidin-1-yl)methyl)-1-oxoisoindolin-2-yl)piperidine-2,6-dione;
3-(5-((4-(4-chlorophenyl)piperidin-1-yl)methyl)-1-oxoisoindolin-2-yl)piperidine-2,6-dione;
3-(5-((4-(2-fluorophenyl)piperidin-1-yl)methyl)-1-oxoisoindolin-2-yl)piperidine-2,6-dione;
3-(5-((4-(4-fluorophenyl)piperidin-1-yl)methyl)-1-oxoisoindolin-2-yl)piperidine-2,6-dione;
3-(1-oxo-5-((4-(2-(trifluoromethyl)phenyl)piperidin-1-yl)methyl)isoindolin-2-yl)piperidine-2,6-dione;
3-(1-oxo-5-((4-(3-(trifluoromethyl)phenyl)piperidin-1-yl)methyl)isoindolin-2-yl)piperidine-2,6-dione;
3-(5-((4-(2,3-dichlorophenyl)piperidin-1-yl)methyl)-1-oxoisoindolin-2-yl)piperidine-2,6-dione;
3-(5-((4-(2-chloro-3-fluorophenyl)piperidin-1-yl)methyl)-1-oxoisoindolin-2-yl)piperidine-2,6-dione;
3-(5-((4-(2,3-difluorophenyl)piperidin-1-yl)methyl)-1-oxoisoindolin-2-yl)piperidine-2,6-dione;
3-(5-((4-(2,3-dichlorophenyl)-3,6-dihydropyridin-1(2H)-yl)methyl)-1-oxoisoindolin-2-yl)piperidine-2,6-dione;
3-(5-((4-(2-chloro-3-fluorophenyl)-3,6-dihydropyridin-1(2H)-yl)methyl)-1-oxoisoindolin-2-yl)piperidine-2,6-dione;
3-(1-oxo-5-((4-(pyridin-2-yl)piperazin-1-yl)methyl)isoindolin-2-yl)piperidine-2,6-dione;
3-(5-((4-(6-methylpyridin-2-yl)piperazin-1-yl)methyl)-1-oxoisoindolin-2-yl)piperidine-2,6-dione;
3-(5-((4-(6-chloropyridin-2-yl)piperazin-1-yl)methyl)-1-oxoisoindolin-2-yl)piperidine-2,6-dione;
3-(5-((4-(5-chloropyridin-2-yl)piperazin-1-yl)methyl)-1-oxoisoindolin-2-yl)piperidine-2,6-dione;
3-(5-((4-(4-chloropyridin-2-yl)piperazin-1-yl)methyl)-1-oxoisoindolin-2-yl)piperidine-2,6-dione;
3-(5-((4-(3-chloropyridin-2-yl)piperazin-1-yl)methyl)-1-oxoisoindolin-2-yl)piperidine-2,6-dione;
3-(5-((4-(3-fluoropyridin-2-yl)piperazin-1-yl)methyl)-1-oxoisoindolin-2-yl)piperidine-2,6-dione;
3-(5-((4-(6-fluoropyridin-2-yl)piperazin-1-yl)methyl)-1-oxoisoindolin-2-yl)piperidine-2,6-dione;
3-(5-((4-(5-fluoropyridin-2-yl)piperazin-1-yl)methyl)-1-oxoisoindolin-2-yl)piperidine-2,6-dione;
3-(1-oxo-5-((4-(3-(trifluoromethyl)pyridin-2-yl)piperazin-1-yl)methyl)isoindolin-2-yl)piperidine-2,6-dione;
3-(1-oxo-5-((4-(6-(trifluoromethyl)pyridin-2-yl)piperazin-1-yl)methyl)isoindolin-2-yl)piperidine-2,6-dione;
3-(1-oxo-5-((4-(5-(trifluoromethyl)pyridin-2-yl)piperazin-1-yl)methyl)isoindolin-2-yl)piperidine-2,6-dione;
3-(5-((4-(3,4-dichloropyridin-2-yl)piperazin-1-yl)methyl)-1-oxoisoindolin-2-yl)piperidine-2,6-dione;
3-(5-((4-(3,5-dichloropyridin-2-yl)piperazin-1-yl)methyl)-1-oxoisoindolin-2-yl)piperidine-2,6-dione;
3-(5-((4-(4,5-dichloropyridin-2-yl)piperazin-1-yl)methyl)-1-oxoisoindolin-2-yl)piperidine-2,6-dione;
3-(5-((4-(3,4-difluoropyridin-2-yl)piperazin-1-yl)methyl)-1-oxoisoindolin-2-yl)piperidine-2,6-dione;
3-(5-((4-(4-chloro-3-fluoropyridin-2-yl)piperazin-1-yl)methyl)-1-oxoisoindolin-2-yl)piperidine-2,6-dione;
3-(5-((4-(3-fluoro-4-iodopyridin-2-yl)piperazin-1-yl)methyl)-1-oxoisoindolin-2-yl)piperidine-2,6-dione;
3-(5-((4-(3-bromo-4-chloropyridin-2-yl)piperazin-1-yl)methyl)-1-oxoisoindolin-2-yl)piperidine-2,6-dione;
3-(5-((4-(3-chloro-5-(trifluoromethyl)pyridin-2-yl)piperazin-1-yl)methyl)-1-oxoisoindolin-2-yl)piperidine-2,6-dione;
3-(5-((4-(4-chloro-6-methylpyridin-2-yl)piperazin-1-yl)methyl)-1-oxoisoindolin-2-yl)piperidine-2,6-dione;

3-(1-oxo-5-((4-(pyridin-3-yl)piperazin-1-yl)methyl)isoindolin-2-yl)piperidine-2,6-dione;
3-(5-((4-(6-chloropyridin-3-yl)piperazin-1-yl)methyl)-1-oxoisoindolin-2-yl)piperidine-2,6-dione;
3-(5-((4-(4,5-dichloropyridin-3-yl)piperazin-1-yl)methyl)-1-oxoisoindolin-2-yl)piperidine-2,6-dione;
3-(5-((4-(2,4-dichloropyridin-3-yl)piperazin-1-yl)methyl)-1-oxoisoindolin-2-yl)piperidine-2,6-dione;
3-(1-oxo-5-((4-(pyridin-4-yl)piperazin-1-yl)methyl)isoindolin-2-yl)piperidine-2,6-dione;
3-(5-((4-(2-methylpyridin-4-yl)piperazin-1-yl)methyl)-1-oxoisoindolin-2-yl)piperidine-2,6-dione;
3-(5-((4-(3-bromopyridin-4-yl)piperazin-1-yl)methyl)-1-oxoisoindolin-2-yl)piperidine-2,6-dione;
3-(5-((4-(3-fluoropyridin-4-yl)piperazin-1-yl)methyl)-1-oxoisoindolin-2-yl)piperidine-2,6-dione;
3-(5-((4-(3-chloropyridin-4-yl)piperazin-1-yl)methyl)-1-oxoisoindolin-2-yl)piperidine-2,6-dione;
3-(5-((4-(2-chloropyridin-4-yl)piperazin-1-yl)methyl)-1-oxoisoindolin-2-yl)piperidine-2,6-dione;
3-(5-((4-(2-chloro-3-fluoropyridin-4-yl)piperazin-1-yl)methyl)-1-oxoisoindolin-2-yl)piperidine-2,6-dione;
3-(5-((4-(2-bromo-3-fluoropyridin-4-yl)piperazin-1-yl)methyl)-1-oxoisoindolin-2-yl)piperidine-2,6-dione;
3-(5-((4-(3-bromo-2-chloropyridin-4-yl)piperazin-1-yl)methyl)-1-oxoisoindolin-2-yl)piperidine-2,6-dione;
3-(5-((4-(2,3-dichloropyridin-4-yl)piperazin-1-yl)methyl)-1-oxoisoindolin-2-yl)piperidine-2,6-dione;
3-(5-((4-(2,3-difluoropyridin-4-yl)piperazin-1-yl)methyl)-1-oxoisoindolin-2-yl)piperidine-2,6-dione;
3-(5-((4-(3-fluoro-2-hydroxypyridin-4-yl)piperazin-1-yl)methyl)-1-oxoisoindolin-2-yl)piperidine-2,6-dione;
3-(5-((4-(3-bromo-5-chloropyridin-4-yl)piperazin-1-yl)methyl)-1-oxoisoindolin-2-yl)piperidine-2,6-dione;
3-(5-((4-(3-chloro-5-fluoropyridin-4-yl)piperazin-1-yl)methyl)-1-oxoisoindolin-2-yl)piperidine-2,6-dione;
3-(5-((4-(3,5-dichloropyridin-4-yl)piperazin-1-yl)methyl)-1-oxoisoindolin-2-yl)piperidine-2,6-dione;
3-(5-((4-(2,6-dimethylpyridin-4-yl)piperazin-1-yl)methyl)-1-oxoisoindolin-2-yl)piperidine-2,6-dione;
3-(1-oxo-5-((4-(perfluoropyridin-4-yl)piperazin-1-yl)methyl)isoindolin-2-yl)piperidine-2,6-dione;
3-(1-oxo-5-((4-(pyridazin-3-yl)piperazin-1-yl)methyl)isoindolin-2-yl)piperidine-2,6-dione;
3-(5-((4-(6-chloropyridazin-3-yl)piperazin-1-yl)methyl)-1-oxoisoindolin-2-yl)piperidine-2,6-dione;
3-(1-oxo-5-((4-(pyrimidin-2-yl)piperazin-1-yl)methyl)isoindolin-2-yl)piperidine-2,6-dione;
3-(1-oxo-5-((4-(pyrazin-2-yl)piperazin-1-yl)methyl)isoindolin-2-yl)piperidine-2,6-dione;
3-(5-((4-(5,6-dichloropyrimidin-4-yl)piperazin-1-yl)methyl)-1-oxoisoindolin-2-yl)piperidine-2,6-dione;
3-(5-((4-(3,6-dichloropyridazin-4-yl)piperazin-1-yl)methyl)-1-oxoisoindolin-2-yl)piperidine-2,6-dione;
3-(5-((4-(1,3,5-triazin-2-yl)piperazin-1-yl)methyl)-1-oxoisoindolin-2-yl)piperidine-2,6-dione;
3-(5-((4-(3,4-dichloropyridin-2-yl)piperidin-1-yl)methyl)-1-oxoisoindolin-2-yl)piperidine-2,6-dione;
3-(5-((4-(2,3-dichloropyridin-4-yl)piperidin-1-yl)methyl)-1-oxoisoindolin-2-yl)piperidine-2,6-dione;
3-(5-((4-(2,3-difluoropyridin-4-yl)piperidin-1-yl)methyl)-1-oxoisoindolin-2-yl)piperidine-2,6-dione;
3-(5-([3,4'-bipiperidin]-1-ylmethyl)-1-oxoisoindolin-2-yl)piperidine-2,6-dione;
3-(5-((3,4-dichloro-3',6'-dihydro-[2,4'-bipyridin]-1'(2'H)-yl)methyl)-1-oxoisoindolin-2-yl)piperidine-2,6-dione;
3-(5-((4,5-dichloro-3',6'-dihydro-[3,4'-bipyridin]-1'(2'H)-yl)methyl)-1-oxoisoindolin-2-yl)piperidine-2,6-dione;
3-(5-((5-chloro-3',6'-dihydro-[3,4'-bipyridin]-1'(2'H)-yl)methyl)-1-oxoisoindolin-2-yl)piperidine-2,6-dione;
3-(5-((2',3'-dichloro-3,6-dihydro-[4,4'-bipyridin]-1 (2H)-yl)methyl)-1-oxoisoindolin-2-yl)piperidine-2,6-dione;
3-(5-((3-fluoro-3',6'-dihydro-[2,4'-bipyridin]-1'(2'H)-yl)methyl)-1-oxoisoindolin-2-yl)piperidine-2,6-dione;
3-(5-((2',3'-difluoro-3,6-dihydro-[4,4'-bipyridin]-1(2H)-yl)methyl)-1-oxoisoindolin-2-yl)piperidine-2,6-dione;
3-(5-((3-(2,3-dichlorophenyl)-3,6-diazabicyclo[3.1.1]heptan-6-yl)methyl)-1-oxoisoindolin-2-yl)piperidine-2,6-dione;
3-(5-((6-(2,3-dichlorophenyl)-3,6-diazabicyclo[3.1.1]heptan-3-yl)methyl)-1-oxoisoindolin-2-yl)piperidine-2,6-dione;
3-(5-(((1R,4R)-5-(2,3-dichlorophenyl)-2,5-diazabicyclo[2.2.1]heptan-2-yl)methyl)-1-oxoisoindolin-2-yl)piperidine-2,6-dione;
3-(5-(((1S,4S)-5-(2,3-dichlorophenyl)-2,5-diazabicyclo[2.2.1]heptan-2-yl)methyl)-1-oxoisoindolin-2-yl)piperidine-2,6-dione;
3-(5-((5-(2,3-dichlorophenyl)-2,5-diazabicyclo[2.2.2]octan-2-yl)methyl)-1-oxoisoindolin-2-yl)piperidine-2,6-dione;
3-(5-((8-(2,3-dichlorophenyl)-3,8-diazabicyclo[3.2.1]octan-3-yl)methyl)-1-oxoisoindolin-2-yl)piperidine-2,6-dione;
3-(5-((3-(2,3-dichlorophenyl)-3,8-diazabicyclo[3.2.1]octan-8-yl)methyl)-1-oxoisoindolin-2-yl)piperidine-2,6-dione;
3-(5-((4-(2,3-dichlorophenyl)-1,4-diazepan-1-yl)methyl)-1-oxoisoindolin-2-yl)piperidine-2,6-dione;
3-(5-((4-(6-fluorobenzo[d]isoxazol-3-yl)piperidin-1-yl)methyl)-1-oxoisoindolin-2-yl)piperidine-2,6-dione;
3-(5-((4-(benzo[d]isoxazo1-3-yl)piperidin-1-yl)methyl)-1-oxoisoindolin-2-yl)piperidine-2,6-dione;
3-(5-((4-(isoxazol-3-yl)piperidin-1-yl)methyl)-1-oxoisoindolin-2-yl)piperidine-2,6-dione;
3-(5-((4-(6-fluorobenzo[d]isoxazol-3-yl)piperazin-1-yl)methyl)-1-oxoisoindolin-2-yl)piperidine-2,6-dione;
3-(5-((4-(isoxazolo[4,5-c]pyridin-3-yl)piperazin-1-yl)methyl)-1-oxoisoindolin-2-yl)piperidine-2,6-dione;

3-(5-((4-(6-fluorobenzo[d]isothiazol-3-yl)piperazin-1-yl)methyl)-1-oxoisoindolin-2-yl)piperidine-2,6-dione;
3-(5-((4-(6-fluoro-1H-indazol-3-yl)piperazin-1-yl)methyl)-1-oxoisoindolin-2-yl)piperidine-2,6-dione;
3-(5-((4-(1H-pyrrolo[2,3-b]pyridin-3-yl)piperazin-1-yl)methyl)-1-oxoisoindolin-2-yl)piperidine-2,6-dione;
3-(5-((4-(6-fluoro-1H-indol-3-yl)piperidin-1-yl)methyl)-1-oxoisoindolin-2-yl)piperidine-2,6-dione;
3-(5-((4-(6-fluoro-1-methyl-1H-indol-3-yl)piperidin-1-yl)methyl)- 1-oxoisoindolin-2-yl)piperidine-2,6-dione;
3-(5-((4-(5-fluoroindolin-1-yl)piperidin-1-yl)methyl)-1-oxoisoindolin-2-yl)piperidine-2,6-dione;
3-(5-((4-(5-fluoro-1H-benzo[d]imidazol-1-yl)piperidin-1-yl)methyl)-1-oxoisoindolin-2-yl)piperidine-2,6-dione;
3-(5-((4-(5-fluoro-2-oxo-2,3-dihydro-1H-benzo[d]imidazol-1-yl)piperidin-1-yl)methyl)-1-oxoisoindolin-2-yl)piperidine-2,6-dione;
3-(5-((4-(1H-pyrrolo[2,3-b]pyridin-3-yl)piperidin-1-yl)methyl)-1-oxoisoindolin-2-yl)piperidine-2,6-dione;
3-(5-((4-(1H-pyrrolo[2,3-b]pyridin-3-yl)-3,6-dihydropyridin-1(2H)-yl)methyl)-1-oxoisoindolin-2-yl)piperidine-2,6-dione;
3-(5-((4-(6-fluoro-1H-indol-3-yl)-3,6-dihydropyridin-1(2H)-yl)methyl)-1-oxoisoindolin-2-yl)piperidine-2,6-dione;
3-(5-((4-(6-fluoro-1-methyl-1H-indol-3-yl)-3,6-dihydropyridin-1(2H)-yl)methyl)-1-oxoisoindolin-2-yl)piperidine-2,6-dione;
3-(5-((4-(6-fluoro-3,4-dihydroquinolin-1(2H)-yl)piperidin-1-yl)methyl)-1-oxoisoindolin-2-yl)piperidine-2,6-dione;
3-(5-((4-(7-fluoro-2,3-dihydro-4H-benzo[b][1,4]oxazin-4-yl)piperidin-1-yl)methyl)-1-oxoisoindolin-2-yl)piperidine-2,6-dione;
3-(5-((4-(7-fluoro-3-oxo-2,3-dihydro-4H-benzo[b] [1,4] oxazin-4-yl)piperidin-1-yl)methyl)-1-oxoisoindolin-2-yl) piperidine-2,6-dione;
3-(5-((4-(7-fluorochroman-4-yl)piperidin-1-yl)methyl)-1-oxoisoindolin-2-yl)piperidine-2,6-dione;
3-(5-((4-(7-fluorochroman-4-ylidene)piperidin-1-yl)methyl)-1-oxoisoindolin-2-yl)piperidine-2,6-dione;
3-(5-((4-(5-fluoronaphthalen-1-yl)piperidin-1-yl)methyl)-1-oxoisoindolin-2-yl)piperidine-2,6-dione;
3-(5-((4-(7-fluoroquinolin-4-yl)piperidin-1-yl)methyl)-1-oxoisoindolin-2-yl)piperidine-2,6-dione;
3-(5-((4-(isoquinolin-5-yl)piperidin-1-yl)methyl)-1-oxoisoindolin-2-yl)piperidine-2,6-dione;
3-(5-((4-(5-fluoronaphthalen-1-yl)-3,6-dihydropyridin-1(2H)-yl)methyl)-1-oxoisoindolin-2-yl)piperidine-2,6-dione;
3-(5-((4-(isoquinolin-5-yl)-3,6-dihydropyridin-1 (2H)-yl)methyl)-1-oxoisoindolin-2-yl)piperidine-2,6-dione;
3-(5-((3-(7-fluorochroman-4-yl)azetidin-1-yl)methyl)-1-oxoisoindolin-2-yl)piperidine-2,6-dione;
3-(5-((3-(7-fluorochroman-4-ylidene)azetidin-1-yl)methyl)-1-oxoisoindolin-2-yl)piperidine-2,6-dione;
3-(5-((3-(7-fluoroquinolin-4-yl)azetidin-1-yl)methyl)-1-oxoisoindolin-2-yl)piperidine-2,6-dione;
3-(5-((3-(5-fluoroindolin-1-yl)azetidin-1-yl)methyl)-1-oxoisoindolin-2-yl)piperidine-2,6-dione;
3-(5-((3-(5-fluoro-1H-benzo[d]imidazol-1-yl)azetidin-1-yl)methyl)-1-oxoisoindolin-2-yl)piperidine-2,6-dione;
3-(1-oxo-5-((4-(thieno[2,3-d]pyrimidin-4-yl)piperazin-1-yl)methyl)isoindolin-2-yl)piperidine-2,6-dione;
3-(5-((4-(5-methylthieno[2,3-d]pyrimidin-4-yl)piperazin-1-yl)methyl)-1-oxoisoindolin-2-yl)piperidine-2,6-dione;
3-(5-((4-(6-methylthieno[2,3-d]pyrimidin-4-yl)piperazin-1-yl)methyl)-1-oxoisoindolin-2-yl)piperidine-2,6-dione;
3-(5-((4-(6-isopropylthieno[2,3-d]pyrimidin-4-yl)piperazin-1-yl)methyl)-1-oxoisoindolin-2-yl)piperidine-2,6-dione;
3-(5-((4-(5,6-dimethylthieno[2,3-d]pyrimidin-4-yl)piperazin-1-yl)methyl)-1-oxoisoindolin-2-yl)piperidine-2,6-dione;
3-(1-oxo-5-((4-(6-phenylthieno[2,3-d]pyrimidin-4-yl)piperazin-1-yl)methyl)isoindolin-2-yl)piperidine-2,6-dione;
3-(5-((4-(6-bromothieno[2,3-d]pyrimidin-4-yl)piperazin-1-yl)methyl)-1-oxoisoindolin-2-yl)piperidine-2,6-dione;
3-(1-oxo-5-((4-(5-phenylthieno[2,3-d]pyrimidin-4-yl)piperazin-1-yl)methyl)isoindolin-2-yl)piperidine-2,6-dione;
3-(1-oxo-5-((4-(5,6,7,8-tetrahydrobenzo[4,5]thieno[2,3-d]pyrimidin-4-yl)piperazin-1-yl)methyl)isoindolin-2-yl) piperidine-2,6-dione;
3-(5-((4-(6,7-dihydro-5H-cyclopenta[4,5]thieno[2,3-d]pyrimidin-4-yl)piperazin-1-yl)methyl)-1-oxoisoindolin-2-yl)piperidine-2,6-dione;
3-(5-((4-(2-methyl-5-phenylthieno[2,3-d]pyrimidin-4-yl)piperazin-1-yl)methyl)-1-oxoisoindolin-2-yl)piperidine-2,6-dione;
3-(5-((4-(2-methyl-5,6,7,8-tetrahydrobenzo[4,5]thieno[2,3-d]pyrimidin-4-yl)piperazin-1-yl)methyl)-1-oxoisoindolin-2-yl)piperidine-2,6-dione;
3-(5-((4-(2-methyl-6,7-dihydro-5H-cyclopenta[4,5]thieno[2,3-d]pyrimidin-4-yl)piperazin-1-yl)methyl)-1-oxoisoindolin-2-yl)piperidine-2,6-dione;
3-(5-((4-(2-methylthieno[2,3-d]pyrimidin-4-yl)piperazin-1-yl)methyl)-1-oxoisoindolin-2-yl)piperidine-2,6-dione;
3-(1-oxo-5-((4-(thieno[2,3-d]pyrimidin-2-yl)piperazin-1-yl)methyl)isoindolin-2-yl)piperidine-2,6-dione;
3-(5-((4-(5,6-dimethylthieno[2,3-d]pyrimidin-4-yl)-1,4-diazepan-1-yl)methyl)-1-oxoisoindolin-2-yl)piperidine-2,6-dione;
3-(1-oxo-5-((4-(thieno[2,3-d]pyrimidin-4-yl)piperidin-1-yl)methyl)isoindolin-2-yl)piperidine-2,6-dione;

3-(5-((4-(6-methylthieno[2,3-d]pyrimidin-4-yl)piperidin-1-yl)methyl)-1-oxoisoindolin-2-yl)piperidine-2,6-dione;
3-(5-((4-(5,6-dimethylthieno[2,3-d]pyrimidin-4-yl)piperidin-1-yl)methyl)-1-oxoisoindolin-2-yl)piperidine-2,6-dione;
3-(1-oxo-5-((4-(6-phenylthieno[2,3-d]pyrimidin-4-yl)piperidin-1-yl)methyl)isoindolin-2-yl)piperidine-2,6-dione;
3-(1-oxo-5-((4-(5-phenylthieno[2,3-d]pyrimidin-4-yl)piperidin-1-yl)methyl)isoindolin-2-yl)piperidine-2,6-dione;
3-(1-oxo-5-((4-(5,6,7,8-tetrahydrobenzo[4,5]thieno[2,3-d]pyrimidin-4-yl)piperidin-1-yl)methyl)isoindolin-2-yl)piperidine-2,6-dione;
3-(5-((4-(6,7-dihydro-5H-cyclopenta[4,5]thieno[2,3-d]pyrimidin-4-yl)piperidin-1-yl)methyl)-1-oxoisoindolin-2-yl)piperidine-2,6-dione;
3-(5-((4-(2-methyl-5-phenylthieno[2,3-d]pyrimidin-4-yl)piperidin-1-yl)methyl)-1-oxoisoindolin-2-yl)piperidine-2,6-dione;
3-(5-((4-(2-methyl-5,6,7,8-tetrahydrobenzo[4,5]thieno[2,3-d]pyrimidin-4-yl)piperidin-1-yl)methyl)-1-oxoisoindolin-2-yl)piperidine-2,6-dione;
3-(5-((4-(2-methyl-6,7-dihydro-5H-cyclopenta[4,5]thieno[2,3-d]pyrimidin-4-yl)piperidin-1-yl)methyl)-1-oxoisoindolin-2-yl)piperidine-2,6-dione;
3-(5-((4-(2-methylthieno[2,3-d]pyrimidin-4-yl)piperidin-1-yl)methyl)-1-oxoisoindolin-2-yl)piperidine-2,6-dione;
3-(1-oxo-5-((4-(thieno[2,3-d]pyrimidin-2-yl)piperidin-1-yl)methyl)isoindolin-2-yl)piperidine-2,6-dione;
3-(5-((4-(5-ethylpyrimidin-2-yl)piperidin-1-yl)methyl)-1-oxoisoindolin-2-yl)piperidine-2,6-dione;
3-(1-oxo-5-((4-(thieno[2,3-d]pyrimidin-4-yl)-3,6-dihydropyridin-1(2H)-yl)methyl)isoindolin-2-yl)piperidine-2,6-dione;
3-(5-((4-(5-methylthieno[2,3-d]pyrimidin-4-yl)-3,6-dihydropyridin-1(2H)-yl)methyl)-1-oxoisoindolin-2-yl)piperidine-2,6-dione;
3-(5-((4-(6-methylthieno[2,3-d]pyrimidin-4-yl)-3,6-dihydropyridin-1(2H)-yl)methyl)-1-oxoisoindolin-2-yl)piperidine-2,6-dione;
3-(5-((4-(6-isopropylthieno[2,3-d]pyrimidin-4-yl)-3,6-dihydropyridin-1(2H)-yl)methyl)-1-oxoisoindolin-2-yl)piperidine-2,6-dione;
3-(5-((4-(5,6-dimethylthieno[2,3-d]pyrimidin-4-yl)-3,6-dihydropyridin-1(2H)-yl)methyl)-1-oxoisoindolin-2-yl)piperidine-2,6-dione;
3-(1-oxo-5-((4-(6-phenylthieno[2,3-d]pyrimidin-4-yl)-3,6-dihydropyridin-1(2H)-yl)methyl)isoindolin-2-yl)piperidine-2,6-dione;
3-(1-oxo-5-((4-(5-phenylthieno[2,3-d]pyrimidin-4-yl)-3,6-dihydropyridin-1(2H)-yl)methyl)isoindolin-2-yl)piperidine-2,6-dione;
3-(1-oxo-5-((4-(5,6,7,8-tetrahydrobenzo[4,5]thieno[2,3-d]pyrimidin-4-yl)-3,6-dihydropyridin-1(2H)-yl)methyl)isoindolin-2-yl)piperidine-2,6-dione;
3-(5-((4-(6,7-dihydro-5H-cyclopenta[4,5]thieno[2,3-d]pyrimidin-4-yl)-3,6-dihydropyridin-1(2H)-yl)methyl)-1-oxoisoindolin-2-yl)piperidine-2,6-dione;
3-(5-((4-(2-methyl-5-phenylthieno[2,3-d]pyrimidin-4-yl)-3,6-dihydropyridin-1(2H)-yl)methyl)-1-oxoisoindolin-2-yl)piperidine-2,6-dione;
3-(5-((4-(2-methyl-5,6,7,8-tetrahydrobenzo[4,5]thieno[2,3-d]pyrimidin-4-yl)-3,6-dihydropyridin-1(2H)-yl)methyl)-1-oxoisoindolin-2-yl)piperidine-2,6-dione;
3-(5-((4-(2-methyl-6,7-dihydro-5H-cyclopenta[4,5]thieno[2,3-d]pyrimidin-4-yl)-3,6-dihydropyridin-1(2H)-yl)methyl)-1-oxoisoindolin-2-yl)piperidine-2,6-dione;
3-(5-((4-(2-methylthieno[2,3-d]pyrimidin-4-yl)-3,6-dihydropyridin-1(2H)-yl)methyl)-1-oxoisoindolin-2-yl)piperidine-2,6-dione;
3-(1-oxo-5-((4-(thieno[2,3-d]pyrimidin-2-yl)-3,6-dihydropyridin-1(2H)-yl)methyl)isoindolin-2-yl)piperidine-2,6-dione;
3-(5-((4-(2-chloro-6,7-dihydrothieno[3,2-d]pyrimidin-4-yl)piperazin-1-yl)methyl)-1-oxoisoindolin-2-yl)piperidine-2,6-dione;
3-(5-((4-(2-morpholino-6,7-dihydrothieno[3,2-d]pyrimidin-4-yl)piperazin-1-yl)methyl)-1-oxoisoindolin-2-yl)piperidine-2,6-dione;
3-(1-oxo-5-((4-(4-(piperazin-1-yl)-6,7-dihydrothieno[3,2-d]pyrimidin-2-yl)piperazin-1-yl)methyl)isoindolin-2-yl)piperidine-2,6-dione;
3-(5-((4-(4-morpholino-6,7-dihydrothieno[3,2-d]pyrimidin-2-yl)piperazin-1-yl)methyl)-1-oxoisoindolin-2-yl)piperidine-2,6-dione;
3-(1-oxo-5-((4-(2-(piperidin-1-yl)-6,7-dihydrothieno[3,2-d]pyrimidin-4-yl)piperazin-1-yl)methyl)isoindolin-2-yl)piperidine-2,6-dione;
3-(1-oxo-5-((4-(2-(pyrrolidin-1-yl)-6,7-dihydrothieno[3,2-d]pyrimidin-4-yl)piperazin-1-yl)methyl)isoindolin-2-yl)piperidine-2,6-dione;

3-(5-((4-(2-(azetidin-1-yl)-6,7-dihydrothieno[3,2-d]pyrimidin-4-yl)piperazin-1-yl)methyl)-1-oxoisoindolin-2-yl)piperidine-2,6-dione;
3-(1-oxo-5-((4-(thieno[3,2-d]pyrimidin-4-yl)piperazin-1-yl)methyl)isoindolin-2-yl)piperidine-2,6-dione;
3-(5-((4-(2-chlorothieno[3,2-d]pyrimidin-4-yl)piperazin-1-yl)methyl)-1-oxoisoindolin-2-yl)piperidine-2,6-dione;
3-(5-((4-(2-morpholinothieno[3,2-d]pyrimidin-4-yl)piperazin-1-yl)methyl)-1-oxoisoindolin-2-yl)piperidine-2,6-dione;
3-(1-oxo-5-((4-(4-(piperazin-1-yl)thieno[3,2-d]pyrimidin-2-yl)piperazin-1-yl)methyl)isoindolin-2-yl)piperidine-2,6-dione;
3-(5-((4-(4-morpholinothieno[3,2-d]pyrimidin-2-yl)piperazin-1-yl)methyl)-1-oxoisoindolin-2-yl)piperidine-2,6-dione;
3-(5-((4-(benzo[b]thiophen-4-yl)piperazin-1-yl)methyl)-1-oxoisoindolin-2-yl)piperidine-2,6-dione;
3-(1-oxo-5-((3-(trifluoromethyl)-5,6-dihydro-[1,2,4]triazolo[4,3-a]pyrazin-7(8H)-yl)methyl)isoindolin-2-yl)piperidine-2,6-dione;
3-(5-((4-(2-((2,4-dimethylphenyl)thio)phenyl)piperazin-1-yl)methyl)-1-oxoisoindolin-2-yl)piperidine-2,6-dione;
3-(5-((6,7-dihydrothieno[3,2-c]pyridin-5(4H)-yl)methyl)-1-oxoisoindolin-2-yl)piperidine-2,6-dione;
3-(5-((4-(benzo[d]isothiazol-3-yl)piperazin-1-yl)methyl)-1-oxoisoindolin-2-yl)piperidine-2,6-dione;
3-(5-((4-(4-((3r,5r,7r)-adamantan-1-yl)benzyl)piperazin-1-yl)methyl)-1-oxoisoindolin-2-yl)piperidine-2,6-dione;
3-(5-((((3s,5s,7s)-adamantan-1-yl)amino)methyl)-1-oxoisoindolin-2-yl)piperidine-2,6-dione;
(1-((1R,4R)-7,7-dimethyl-2-oxobicyclo[2.2.1]heptan-1-yl)-N-((2-(2,6-dioxopiperidin-3-yl)-1-oxoisoindolin-5-yl)methyl)methanesulfonamide;
3-(5-((((1R,4S)-bicyclo[2.2.1]heptan-2-yl)oxy)methyl)-1-oxoisoindolin-2-yl)piperidine-2,6-dione;
2-(2,6-dioxopiperidin-3-yl)-5-((4-(thiophen-2-yl)piperazin-1-yl)methyl)isoindoline-1,3-dione;
2-(2,6-dioxopiperidin-3-yl)-5-((4-(5-methylthiophen-2-yl)piperazin-1-yl)methyl)isoindoline-1,3-dione;
2-(2,6-dioxopiperidin-3-yl)-5-((4-(3-methylthiophen-2-yl)piperazin-1-yl)methyl)isoindoline-1,3-dione;
2-(2,6-dioxopiperidin-3-yl)-5-((4-(thiophen-3-yl)piperazin-1-yl)methyl)isoindoline-1,3-dione;
2-(2,6-dioxopiperidin-3-yl)-5-((4-(4-methylthiophen-3-yl)piperazin-1-yl)methyl)isoindoline-1,3-dione;
2-(2,6-dioxopiperidin-3-yl)-5-((4-(5-methylthiophen-3-yl)piperazin-1-yl)methyl)isoindoline-1,3-dione;
2-(2,6-dioxopiperidin-3-yl)-5-((4-(thiophen-2-yl)-3,6-dihydropyridin-1(2H)-yl)methyl)isoindoline-1,3-dione;
2-(2,6-dioxopiperidin-3-yl)-5-((4-(5-methylthiophen-2-yl)-3,6-dihydropyridin-1(2H)-yl)methyl)isoindoline-1,3-dione;
2-(2,6-dioxopiperidin-3-yl)-5-((4-(3-methylthiophen-2-yl)-3,6-dihydropyridin-1(2H)-yl)methyl)isoindoline-1,3-dione;
2-(2,6-dioxopiperidin-3-yl)-5-((4-(thiophen-3-yl)-3,6-dihydropyridin-1(2H)-yl)methyl)isoindoline-1,3-dione;
2-(2,6-dioxopiperidin-3-yl)-5-((4-(4-methylthiophen-3-yl)-3,6-dihydropyridin-1(2H)-yl)methyl)isoindoline-1,3-dione;
2-(2,6-dioxopiperidin-3-yl)-5-((4-(5-methylthiophen-3-yl)-3,6-dihydropyridin-1(2H)-yl)methyl)isoindoline-1,3-dione;
2-(2,6-dioxopiperidin-3-yl)-5-((4-(furan-2-yl)-3,6-dihydropyridin-1(2H)-yl)methyl)isoindoline-1,3-dione;
2-(2,6-dioxopiperidin-3-yl)-5-((4-(furan-3-yl)-3,6-dihydropyridin-1(2H)-yl)methyl)isoindoline-1,3-dione;
2-(2,6-dioxopiperidin-3-yl)-5-((4-(thiophen-2-yl)piperidin-1-yl)methyl)isoindoline-1,3-dione;
2-(2,6-dioxopiperidin-3-yl)-5-((4-(5-methylthiophen-2-yl)piperidin-1-yl)methyl)isoindoline-1,3-dione;
2-(2,6-dioxopiperidin-3-yl)-5-((4-(3 -methylthiophen-2-yl)piperidin-1-yl)methyl)isoindoline-1,3-dione;
2-(2,6-dioxopiperidin-3-yl)-5-((4-(thiophen-3-yl)piperidin-1-yl)methyl)isoindoline-1,3-dione;
2-(2,6-dioxopiperidin-3-yl)-5-((4-(5-methylthiophen-3-yl)piperidin-1-yl)methyl)isoindoline-1,3-dione;
2-(2,6-dioxopiperidin-3-yl)-5-((4-(4-methylthiophen-3-yl)piperidin-1-yl)methyl)isoindoline-1,3-dione;
2-(2,6-dioxopiperidin-3-yl)-5-((4-(furan-2-yl)piperidin-1-yl)methyl)isoindoline-1,3-dione;
2-(2,6-dioxopiperidin-3-yl-5-((4-(furan-3-yl)piperidin-1-yl)methyl)isoindoline-1,3-dione;
5-((4-(2,3-dichlorophenyl)piperazin-1-yl)methyl)-2-(2,6-dioxopiperidin-3-yl)isoindoline-1,3-dione;
2-(2,6-dioxopiperidin-3-yl)-5-((4-phenylpiperazin-1-yl)methyl)isoindoline-1,3-dione;
2-(2,6-dioxopiperidin-3-yl)-5-((4-(2-methoxyphenyl)piperazin-1-yl)methyl)isoindoline-1,3-dione;
2-(2,6-dioxopiperidin-3-yl)-5-((4-(3-methoxyphenyl)piperazin-1-yl)methyl)isoindoline-1,3-dione;
2-(2,6-dioxopiperidin-3-yl)-5-((4-(4-methoxyphenyl)piperazin-1-yl)methyl)isoindoline-1,3-dione;
2-(2,6-dioxopiperidin-3-yl)-5-((4-(4-(trifluoromethyl)phenyl)piperazin-1-yl)methyl)isoindoline-1,3-dione;
2-(2,6-dioxopiperidin-3-yl)-5-((4-(3-fluorophenyl)piperazin-1-yl)methyl)isoindoline-1,3-dione;
2-(2,6-dioxopiperidin-3-yl)-5-((4-(4-fluorophenyl)piperazin-1-yl)methyl)isoindoline-1,3-dione;
5-((4-(2-chlorophenyl)piperazin-1-yl)methyl)-2-(2,6-dioxopiperidin-3-yl)isoindoline-1,3-dione;
5-((4-(4-chlorophenyl)piperazin-1-yl)methyl)-2-(2,6-dioxopiperidin-3-yl)isoindoline-1,3-dione;
5-((4-(2-bromophenyl)piperazin-1-yl)methyl)-2-(2,6-dioxopiperidin-3-yl)isoindoline-1,3-dione;

5-((4-(3-bromophenyl)piperazin-1-yl)methyl)-2-(2,6-dioxopiperidin-3-yl)isoindoline-1,3-dione;
5-((4-(4-bromophenyl)piperazin-1-yl)methyl)-2-(2,6-dioxopiperidin-3-yl)isoindoline-1,3-dione;
2-(2,6-dioxopiperidin-3-yl)-5-((4-(o-tolyl)piperazin-1-yl)methyl)isoindoline-1,3-dione;
2-(2,6-dioxopiperidin-3-yl)-5-((4-(m-tolyl)piperazin-1-yl)methyl)isoindoline-1,3-dione;
2-(2,6-dioxopiperidin-3-yl)-5-((4-(p-tolyl)piperazin-1-yl)methyl)isoindoline-1,3-dione;
5-((4-(2,4-dichlorophenyl)piperazin-1-yl)methyl)-2-(2,6-dioxopiperidin-3-yl)isoindoline-1,3-dione;
5-((4-(2,5-dichlorophenyl)piperazin-1-yl)methyl)-2-(2,6-dioxopiperidin-3-yl)isoindoline-1,3-dione;
5-((4-(2,6-dichlorophenyl)piperazin-1-yl)methyl)-2-(2,6-dioxopiperidin-3-yl)isoindoline-1,3-dione;
5-((4-(3,4-dichlorophenyl)piperazin-1-yl)methyl)-2-(2,6-dioxopiperidin-3-yl)isoindoline-1,3-dione;
5-((4-(3,5-dichlorophenyl)piperazin-1-yl)methyl)-2-(2,6-dioxopiperidin-3-yl)isoindoline-1,3-dione;
5-((4-(2,3-difluorophenyl)piperazin-1-yl)methyl)-2-(2,6-dioxopiperidin-3-yl)isoindoline-1,3-dione;
5-((4-(2,4-difluorophenyl)piperazin-1-yl)methyl)-2-(2,6-dioxopiperidin-3-yl)isoindoline-1,3-dione;
5-((4-(2,5-difluorophenyl)piperazin-1-yl)methyl)-2-(2,6-dioxopiperidin-3-yl)isoindoline-1,3-dione;
5-((4-(2,6-difluorophenyl)piperazin-1-yl)methyl)-2-(2,6-dioxopiperidin-3-yl)isoindoline-1,3-dione;
5-((4-(3,4-difluorophenyl)piperazin-1-yl)methyl)-2-(2,6-dioxopiperidin-3-yl)isoindoline-1,3-dione;
5-((4-(3,5-difluorophenyl)piperazin-1-yl)methyl)-2-(2,6-dioxopiperidin-3-yl)isoindoline-1,3-dione;
5-((4-(2,4-dimethylphenyl)piperazin-1-yl)methyl)-2-(2,6-dioxopiperidin-3-yl)isoindoline-1,3-dione;
5-((4-(5-chloro-2-methylphenyl)piperazin-1-yl)methyl)-2-(2,6-dioxopiperidin-3-yl)isoindoline-1,3-dione;
2-(2,6-dioxopiperidin-3-yl)-5-((4-phenylpiperidin-1-yl)methyl)isoindoline-1,3-dione;
5-((4-(3-bromophenyl)piperidin-1-yl)methyl)-2-(2,6-dioxopiperidin-3-yl)isoindoline-1,3-dione;
5-((4-(4-bromophenyl)piperidin-1-yl)methyl)-2-(2,6-dioxopiperidin-3-yl)isoindoline-1,3-dione;
5-((4-(2-chlorophenyl)piperidin-1-yl)methyl)-2-(2,6-dioxopiperidin-3-yl)isoindoline-1,3-dione;
5-((4-(4-chlorophenyl)piperidin-1-yl)methyl)-2-(2,6-dioxopiperidin-3-yl)isoindoline-1,3-dione;
2-(2,6-dioxopiperidin-3-yl)-5-((4-(2-fluorophenyl)piperidin-1-yl)methyl)isoindoline-1,3-dione;
2-(2,6-dioxopiperidin-3-yl)-5-((4-(4-fluorophenyl)piperidin-1-yl)methyl)isoindoline-1,3-dione;
2-(2,6-dioxopiperidin-3-yl)-5-((4-(2-(trifluoromethyl)phenyl)piperidin-1-yl)methyl)isoindoline-1,3-dione;
2-(2,6-dioxopiperidin-3-yl)-5-((4-(3-(trifluoromethyl)phenyl)piperidin-1-yl)methyl)isoindoline-1,3-dione;
5-((4-(2,3-dichlorophenyl)piperidin-1-yl)methyl)-2-(2,6-dioxopiperidin-3-yl)isoindoline-1,3-dione;
5-((4-(2-chloro-3-fluorophenyl)piperidin-1-yl)methyl)-2-(2,6-dioxopiperidin-3-yl)isoindoline-1,3-dione;
5-((4-(2,3-difluorophenyl)piperidin-1-yl)methyl)-2-(2,6-dioxopiperidin-3-yl)isoindoline-1,3-dione;
5-((4-(2,3-dichlorophenyl)-3,6-dihydropyridin-1(2H)-yl)methyl)-2-(2,6-dioxopiperidin-3-yl)isoindoline-1,3-dione;
5-((4-(2-chloro-3-fluorophenyl)-3,6-dihydropyridin-1(2H)-yl)methyl)-2-(2,6-dioxopiperidin-3-yl)isoindoline-1,3-dione;
2-(2,6-dioxopiperidin-3-yl)-5-((4-(pyridin-2-yl)piperazin-1-yl)methyl)isoindoline-1,3-dione;
2-(2,6-dioxopiperidin-3-yl)-5-((4-(6-methylpyridin-2-yl)piperazin-1-yl)methyl)isoindoline-1,3-dione;
5-((4-(6-chloropyridin-2-yl)piperazin-1-yl)methyl)-2-(2,6-dioxopiperidin-3-yl)isoindoline-1,3-dione;
5-((4-(5-chloropyridin-2-yl)piperazin-1-yl)methyl)-2-(2,6-dioxopiperidin-3-yl)isoindoline-1,3-dione;
5-((4-(4-chloropyridin-2-yl)piperazin-1-yl)methyl)-2-(2,6-dioxopiperidin-3-yl)isoindoline-1,3-dione;
5-((4-(3-chloropyridin-2-yl)piperazin-1-yl)methyl)-2-(2,6-dioxopiperidin-3-yl)isoindoline-1,3-dione;
2-(2,6-dioxopiperidin-3-yl)-5-((4-(3-fluoropyridin-2-yl)piperazin-1-yl)methyl)isoindoline-1,3-dione;
2-(2,6-dioxopiperidin-3-yl)-5-((4-(6-fluoropyridin-2-yl)piperazin-1-yl)methyl)isoindoline-1,3-dione;
2-(2,6-dioxopiperidin-3-yl)-5-((4-(5-fluoropyridin-2-yl)piperazin-1-yl)methyl)isoindoline-1,3-dione;
2-(2,6-dioxopiperidin-3-yl)-5-((4-(3-(trifluoromethyl)pyridin-2-yl)piperazin-1-yl)methyl)isoindoline-1,3-dione;
2-(2,6-dioxopiperidin-3-yl)-5-((4-(6-(trifluoromethyl)pyridin-2-yl)piperazin-1-yl)methyl)isoindoline-1,3-dione;
2-(2,6-dioxopiperidin-3-yl)-5-((4-(5-(trifluoromethyl)pyridin-2-yl)piperazin-1-yl)methyl)isoindoline-1,3-dione;
5-((4-(3,4-dichloropyridin-2-yl)piperazin-1-yl)methyl)-2-(2,6-dioxopiperidin-3-yl)isoindoline-1,3-dione;
5-((4-(3,5-dichloropyridin-2-yl)piperazin-1-yl)methyl)-2-(2,6-dioxopiperidin-3-yl)isoindoline-1,3-dione;
5-((4-(4,5-dichloropyridin-2-yl)piperazin-1-yl)methyl)-2-(2,6-dioxopiperidin-3-yl)isoindoline-1,3-dione;
5-((4-(3,4-difluoropyridin-2-yl)piperazin-1-yl)methyl)-2-(2,6-dioxopiperidin-3-yl)isoindoline-1,3-dione;
5-((4-(4-chloro-3-fluoropyridin-2-yl)piperazin-1-yl)methyl)-2-(2,6-dioxopiperidin-3-yl)isoindoline-1,3-dione;
2-(2,6-dioxopiperidin-3-yl)-5-((4-(3-fluoro-4-iodopyridin-2-yl)piperazin-1-yl)methyl)isoindoline-1,3-dione;
5-((4-(3-bromo-4-chloropyridin-2-yl)piperazin-1-yl)methyl)-2-(2,6-dioxopiperidin-3-yl)isoindoline-1,3-dione;
5-((4-(3-chloro-5-(trifluoromethyl)pyridin-2-yl)piperazin-1-yl)methyl)-2-(2,6-dioxopiperidin-3-yl)isoindoline-1,3-dione;
5-((4-(4-chloro-6-methylpyridin-2-yl)piperazin-1-yl)methyl)-2-(2,6-dioxopiperidin-3-yl)isoindoline-1,3-dione;
2-(2,6-dioxopiperidin-3-yl)-5-((4-(pyridin-3-yl)piperazin-1-yl)methyl)isoindoline-1,3-dione;
5-((4-(6-chloropyridin-3-yl)piperazin-1-yl)methyl)-2-(2,6-dioxopiperidin-3-yl)isoindoline-1,3-dione;

5-((4-(4,5-dichloropyridin-3-yl)piperazin-1-yl)methyl)-2-(2,6-dioxopiperidin-3-yl)isoindoline-1,3-dione;
5-((4-(2,4-dichloropyridin-3-yl)piperazin-1-yl)methyl)-2-(2,6-dioxopiperidin-3-yl)isoindoline-1,3-dione;
2-(2,6-dioxopiperidin-3-yl)-5-((4-(pyridin-4-yl)piperazin-1-yl)methyl)isoindoline-1,3-dione;
2-(2,6-dioxopiperidin-3-yl)-5-((4-(2-methylpyridin-4-yl)piperazin-1-yl)methyl)isoindoline-1,3-dione;
5-((4-(3-bromopyridin-4-yl)piperazin-1-yl)methyl)-2-(2,6-dioxopiperidin-3-yl)isoindoline-1,3-dione;
2-(2,6-dioxopiperidin-3-yl)-5-((4-(3-fluoropyridin-4-yl)piperazin-1-yl)methyl)isoindoline-1,3-dione;
5-((4-(3-chloropyridin-4-yl)piperazin-1-yl)methyl)-2-(2,6-dioxopiperidin-3-yl)isoindoline-1,3-dione;
5-((4-(2-chloropyridin-4-yl)piperazin-1-yl)methyl)-2-(2,6-dioxopiperidin-3-yl)isoindoline-1,3-dione;
5-((4-(2-chloro-3-fluoropyridin-4-yl)piperazin-1-yl)methyl)-2-(2,6-dioxopiperidin-3-yl)isoindoline-1,3-dione;
5-((4-(2-bromo-3-fluoropyridin-4-yl)piperazin-1-yl)methyl)-2-(2,6-dioxopiperidin-3-yl)isoindoline-1,3-dione;
5-((4-(3-bromo-2-chloropyridin-4-yl)piperazin-1-yl)methyl)-2-(2,6-dioxopiperidin-3-yl)isoindoline-1,3-dione;
5-((4-(2,3-dichloropyridin-4-yl)piperazin-1-yl)methyl)-2-(2,6-dioxopiperidin-3-yl)isoindoline-1,3-dione;
5-((4-(2,3-difluoropyridin-4-yl)piperazin-1-yl)methyl)-2-(2,6-dioxopiperidin-3-yl)isoindoline-1,3-dione;
2-(2,6-dioxopiperidin-3-yl)-5-((4-(3-fluoro-2-hydroxypyridin-4-yl)piperazin-1-yl)methyl)isoindoline-1,3-dione;
5-((4-(3-bromo-5-chloropyridin-4-yl)piperazin-1-yl)methyl)-2-(2,6-dioxopiperidin-3-yl)isoindoline-1,3-dione;
5-((4-(3-chloro-5-fluoropyridin-4-yl)piperazin-1-yl)methyl)-2-(2,6-dioxopiperidin-3-yl)isoindoline-1,3-dione;
5-((4-(3,5-dichloropyridin-4-yl)piperazin-1-yl)methyl)-2-(2,6-dioxopiperidin-3-yl)isoindoline-1,3-dione;
5-((4-(2,6-dimethylpyridin-4-yl)piperazin-1-yl)methyl)-2-(2,6-dioxopiperidin-3-yl)isoindoline-1,3-dione;
2-(2,6-dioxopiperidin-3-yl)-5-((4-(perfluoropyridin-4-yl)piperazin-1-yl)methyl)isoindoline-1,3-dione;
2-(2,6-dioxopiperidin-3-yl)-5-((4-(pyridazin-3-yl)piperazin-1-yl)methyl)isoindoline-1,3-dione;
5-((4-(6-chloropyridazin-3-yl)piperazin-1-yl)methyl)-2-(2,6-dioxopiperidin-3-yl)isoindoline-1,3-dione;
2-(2,6-dioxopiperidin-3-yl)-5-((4-(pyrimidin-2-yl)piperazin-1-yl)methyl)isoindoline-1,3-dione;
2-(2,6-dioxopiperidin-3-yl)-5-((4-(pyrazin-2-yl)piperazin-1-yl)methyl)isoindoline-1,3-dione;
5-((4-(5,6-dichloropyrimidin-4-yl)piperazin-1-yl)methyl)-2-(2,6-dioxopiperidin-3-yl)isoindoline-1,3-dione;
5-((4-(3,6-dichloropyridazin-4-yl)piperazin-1-yl)methyl)-2-(2,6-dioxopiperidin-3-yl)isoindoline-1,3-dione;
5-((4-(1,3,5-triazin-2-yl)piperazin-1-yl)methyl)-2-(2,6-dioxopiperidin-3-yl)isoindoline-1,3-dione;
5-((4-(3,4-dichloropyridin-2-yl)piperidin-1-yl)methyl)-2-(2,6-dioxopiperidin-3-yl)isoindoline-1,3-dione;
5-((4-(2,3-dichloropyridin-4-yl)piperidin-1-yl)methyl)-2-(2,6-dioxopiperidin-3-yl)isoindoline-1,3-dione;
5-((4-(2,3-difluoropyridin-4-yl)piperidin-1-yl)methyl)-2-(2,6-dioxopiperidin-3-yl)isoindoline-1,3-dione;
5-([3,4'-bipiperidin]-1-ylmethyl)-2-(2,6-dioxopiperidin-3-yl)isoindoline-1,3-dione;
5-((3,4-dichloro-3',6'-dihydro-[2,4'-bipyridin]-1'(2'H)-yl)methyl)-2-(2,6-dioxopiperidin-3-yl)isoindoline-1,3-dione;
5-((4,5-dichloro-3',6'-dihydro-[3,4'-bipyridin]-1'(2'H)-yl)methyl)-2-(2,6-dioxopiperidin-3-yl)isoindoline-1,3-dione;
5-((5-chloro-3',6'-dihydro-[3,4'-bipyridin]-1'(2'H)-yl)methyl)-2-(2,6-dioxopiperidin-3-yl)isoindoline-1,3-dione;
5-((2',3'-dichloro-3,6-dihydro-[4,4'-bipyridin]-1(2H)-yl)methyl)-2-(2,6-dioxopiperidin-3-yl)isoindoline-1,3-dione;
2-(2,6-dioxopiperidin-3-yl)-5-((3-fluoro-3',6'-dihydro-[2,4'-bipyridin]-1'(2'H)-yl)methyl)isoindoline-1,3-dione;
5-((2',3'-difluoro-3,6-dihydro-[4,4'-bipyridin]-1 (2H)-yl)methyl)-2-(2,6-dioxopiperidin-3-yl)isoindoline-1,3-dione;
5-((3-(2,3-dichlorophenyl)-3,6-diazabicyclo[3.1.1]heptan-6-yl)methyl)-2-(2,6-dioxopiperidin-3-yl)isoindoline-1,3-dione;
5-((6-(2,3-dichlorophenyl)-3,6-diazabicyclo[3.1.1]heptan-3-yl)methyl)-2-(2,6-dioxopiperidin-3-yl)isoindoline-1,3-dione;
5-(((1R,4R)-5-(2,3-dichlorophenyl)-2,5-diazabicyclo[2.2.1]heptan-2-yl)methyl)-2-(2,6-dioxopiperidin-3-yl)isoindoline-1,3-dione;
5-(((1S,4S)-5-(2,3-dichlorophenyl)-2,5-diazabicyclo[2.2.1]heptan-2-yl)methyl)-2-(2,6-dioxopiperidin-3-yl)isoindoline-1,3-dione;
5-((5-(2,3-dichlorophenyl)-2,5-diazabicyclo[2.2.2]octan-2-yl)methyl)-2-(2,6-dioxopiperidin-3-yl)isoindoline-1,3-dione;
5-((8-(2,3-dichlorophenyl)-3,8-diazabicyclo[3.2.1]octan-3-yl)methyl)-2-(2,6-dioxopiperidin-3-yl)isoindoline-1,3-dione;
5-((3-(2,3-dichlorophenyl)-3,8-diazabicyclo[3.2.1]octan-8-yl)methyl)-2-(2,6-dioxopiperidin-3-yl)isoindoline-1,3-dione;
5-((4-(2,3-dichlorophenyl)-1,4-diazepan-1-yl)methyl)-2-(2,6-dioxopiperidin-3-yl)isoindoline-1,3-dione;
2-(2,6-dioxopiperidin-3-yl)-5-((4-(6-fluorobenzo[d]isoxazol-3-yl)piperidin-1-yl)methyl)isoindoline-1,3-dione;
5-((4-(benzo[d]isoxazol-3-yl)piperidin-1-yl)methyl)-2-(2,6-dioxopiperidin-3-yl)isoindoline-1,3-dione;
2-(2,6-dioxopiperidin-3-yl)-5-((4-(isoxazol-3-yl)piperidin-1-yl)methyl)isoindoline-1,3-dione;
2-(2,6-dioxopiperidin-3-yl)-5-((4-(6-fluorobenzo[d]isoxazol-3-yl)piperazin-1-yl)methyl)isoindoline-1,3-dione;
2-(2,6-dioxopiperidin-3-yl)-5-((4-(isoxazolo[4,5-c]pyridin-3-yl)piperazin-1-yl)methyl)isoindoline-1,3-dione;

2-(2,6-dioxopiperidin-3-yl)-5-((4-(6-fluorobenzo[d]isothiazol-3-yl)piperazin-1-yl)methyl)isoindoline-1,3-dione;
2-(2,6-dioxopiperidin-3-yl)-5-((4-(6-fluoro-1H-indazol-3-yl)piperazin-1-yl)methyl)isoindoline-1,3-dione;
5-((4-(1H-pyrrolo[2,3-b]pyridin-3-yl)piperazin-1-yl)methyl)-2-(2,6-dioxopiperidin-3-yl)isoindoline-1,3-dione;
2-(2,6-dioxopiperidin-3-yl)-5-((4-(6-fluoro-1H-indol-3-yl)piperidin-1-yl)methyl)isoindoline-1,3-dione;
2-(2,6-dioxopiperidin-3-yl)-5-((4-(6-fluoro-1-methyl-1H-indol-3-yl)piperidin-1-yl)methyl)isoindoline-1,3-dione;
2-(2,6-dioxopiperidin-3-yl)-5-((4-(5-fluoroindolin-1-yl)piperidin-1-yl)methyl)isoindoline-1,3-dione;
2-(2,6-dioxopiperidin-3-yl)-5-((4-(5-fluoro-1H-benzo[d]imidazol-1-yl)piperidin-1-yl)methyl)isoindoline-1,3-dione;
2-(2,6-dioxopiperidin-3-yl)-5-((4-(5-fluoro-2-oxo-2,3-dihydro-1H-benzo[d]imidazol-1-yl)piperidin-1-yl)methyl)isoindoline-1,3-dione;
5-((4-(1H-pyrrolo[2,3-b]pyridin-3-yl)piperidin-1-yl)methyl)-2-(2,6-dioxopiperidin-3-yl)isoindoline-1,3-dione;
5-((4-(1H-pyrrolo[2,3-b]pyridin-3-yl)-3,6-dihydropyridin-1(2H)-yl)methyl)-2-(2,6-dioxopiperidin-3-yl)isoindoline-1,3-dione;
2-(2,6-dioxopiperidin-3-yl)-5-((4-(6-fluoro-1H-indol-3-yl)-3,6-dihydropyridin-1(2H)-yl)methyl)isoindoline-1,3-dione;
2-(2,6-dioxopiperidin-3-yl)-5-((4-(6-fluoro-1-methyl-1H-indol-3-yl)-3,6-dihydropyridin-1(2H)-yl)methyl)isoindoline-1,3-dione;
2-(2,6-dioxopiperidin-3-yl)-5-((4-(6-fluoro-3,4-dihydroquinolin-1(2H)-yl)piperidin-1-yl)methyl)isoindoline-1,3-dione;
2-(2,6-dioxopiperidin-3-yl)-5-((4-(7-fluoro-2,3-dihydro-4H-benzo[b][1,4]oxazin-4-yl)piperidin-1-yl)methyl)isoindoline-1,3-dione;
2-(2,6-dioxopiperidin-3-yl)-5-((4-(7-fluoro-3-oxo-2,3-dihydro-4H-benzo[b][1,4]oxazin-4-yl)piperidin-1-yl)methyl)isoindoline-1,3-dione;
2-(2,6-dioxopiperidin-3-yl)-5-((4-(7-fluorochroman-4-yl)piperidin-1-yl)methyl)isoindoline-1,3-dione;
2-(2,6-dioxopiperidin-3-yl)-5-((4-(7-fluorochroman-4-ylidene)piperidin-1-yl)methyl)isoindoline-1,3-dione;
2-(2,6-dioxopiperidin-3-yl)-5-((4-(5-fluoronaphthalen-1-yl)piperidin-1-yl)methyl)isoindoline-1,3-dione;
2-(2,6-dioxopiperidin-3-yl)-5-((4-(7-fluoroquinolin-4-yl)piperidin-1-yl)methyl)isoindoline-1,3-dione;
2-(2,6-dioxopiperidin-3-yl)-5-((4-(isoquinolin-5-yl)piperidin-1-yl)methyl)isoindoline-1,3-dione;
2-(2,6-dioxopiperidin-3-yl)-5-((4-(5-fluoronaphthalen-1-yl)-3,6-dihydropyridin-1(2H)-yl)methyl)isoindoline-1,3-dione;
2-(2,6-dioxopiperidin-3-yl)-5-((4-(isoquinolin-5-yl)-3,6-dihydropyridin-1(2H)-yl)methyl)isoindoline-1,3-dione;
2-(2,6-dioxopiperidin-3-yl)-5-((3-(7-fluorochroman-4-yl)azetidin-1-yl)methyl)isoindoline-1,3-dione;
2-(2,6-dioxopiperidin-3-yl)-5-((3-(7-fluorochroman-4-ylidene)azetidin-1-yl)methyl)isoindoline-1,3-dione;
2-(2,6-dioxopiperidin-3-yl)-5-((3-(7-fluoroquinolin-4-yl)azetidin-1-yl)methyl)isoindoline-1,3-dione;
2-(2,6-dioxopiperidin-3-yl)-5-((3-(5-fluoroindolin-1-yl)azetidin-1-yl)methyl)isoindoline-1,3-dione;
2-(2,6-dioxopiperidin-3-yl)-5-((3-(5-fluoro-1H-benzo[d]imidazol-1-yl)azetidin-1-yl)methyl)isoindoline-1,3-dione;
2-(2,6-dioxopiperidin-3-yl)-5-((4-(thieno[2,3-d]pyrimidin-4-yl)piperazin-1-yl)methyl)isoindoline-1,3-dione;
2-(2,6-dioxopiperidin-3-yl)-5-((4-(5-methylthieno[2,3-d]pyrimidin-4-yl)piperazin-1-yl)methyl)isoindoline-1,3-dione;
2-(2,6-dioxopiperidin-3-yl)-5-((4-(6-methylthieno[2,3-d]pyrimidin-4-yl)piperazin-1-yl)methyl)isoindoline-1,3-dione;
2-(2,6-dioxopiperidin-3-yl)-5-((4-(6-isopropylthieno[2,3-d]pyrimidin-4-yl)piperazin-1-yl)methyl)isoindoline-1,3-dione;
5-((4-(5,6-dimethylthieno[2,3-d]pyrimidin-4-yl)piperazin-1-yl)methyl)-2-(2,6-dioxopiperidin-3-yl)isoindoline-1,3-dione;
2-(2,6-dioxopiperidin-3-yl)-5-((4-(6-phenylthieno[2,3-d]pyrimidin-4-yl)piperazin-1-yl)methyl)isoindoline-1,3-dione;
5-((4-(6-bromothieno[2,3-d]pyrimidin-4-yl)piperazin-1-yl)methyl)-2-(2,6-dioxopiperidin-3-yl)isoindoline-1,3-dione;
2-(2,6-dioxopiperidin-3-yl)-5-((4-(5-phenylthieno[2,3-d]pyrimidin-4-yl)piperazin-1-yl)methyl)isoindoline-1,3-dione;
2-(2,6-dioxopiperidin-3-yl)-5-((4-(5,6,7,8-tetrahydrobenzo[4,5]thieno[2,3-d]pyrimidin-4-yl)piperazin-1-yl)methyl)isoindoline-1,3-dione;
5-((4-(6,7-dihydro-5H-cyclopenta[4,5]thieno[2,3-d]pyrimidin-4-yl)piperazin-1-yl)methyl)-2-(2,6-dioxopiperidin-3-yl)isoindoline-1,3-dione;
2-(2,6-dioxopiperidin-3-yl)-5-((4-(2-methyl-5-phenylthieno[2,3-d]pyrimidin-4-yl)piperazin-1-yl)methyl)isoindoline-1,3-dione;
2-(2,6-dioxopiperidin-3-yl)-5-((4-(2-methyl-5,6,7,8-tetrahydrobenzo[4,5]thieno[2,3-d]pyrimidin-4-yl)piperazin-1-yl)methyl)isoindoline-1,3-dione;
2-(2,6-dioxopiperidin-3-yl)-5-((4-(2-methyl-6,7-dihydro-5H-cyclopenta[4,5]thieno[2,3-d]pyrimidin-4-yl)piperazin-1-yl)methyl)isoindoline-1,3-dione;
2-(2,6-dioxopiperidin-3-yl)-5-((4-(2-methylthieno[2,3-d]pyrimidin-4-yl)piperazin-1-yl)methyl)isoindoline-1,3-dione;
2-(2,6-dioxopiperidin-3-yl)-5-((4-(thieno[2,3-d]pyrimidin-2-yl)piperazin-1-yl)methyl)isoindoline-1,3-dione;
5-((4-(5,6-dimethylthieno[2,3-d]pyrimidin-4-yl)-1,4-diazepan-1-yl)methyl)-2-(2,6-dioxopiperidin-3-yl)isoindoline-1,3-dione;
2-(2,6-dioxopiperidin-3-yl)-5-((4-(thieno[2,3-d]pyrimidin-4-yl)piperidin-1-yl)methyl)isoindoline-1,3-dione;
2-(2,6-dioxopiperidin-3-yl)-5-((4-(6-methylthieno[2,3-d]pyrimidin-4-yl)piperidin-1-yl)methyl)isoindoline-1,3-dione;
5-((4-(5,6-dimethylthieno[2,3-d]pyrimidin-4-yl)piperidin-1-yl)methyl)-2-(2,6-dioxopiperidin-3-yl)isoindoline-1,3-dione;
2-(2,6-dioxopiperidin-3-yl)-5-((4-(6-phenylthieno[2,3-d]pyrimidin-4-yl)piperidin-1-yl)methyl)isoindoline-1,3-dione;
2-(2,6-dioxopiperidin-3-yl)-5-((4-(5-phenylthieno[2,3-d]pyrimidin-4-yl)piperidin-1-yl)methyl)isoindoline-1,3-dione;
2-(2,6-dioxopiperidin-3-yl)-5-((4-(5,6,7,8-tetrahydrobenzo[4,5]thieno[2,3-d]pyrimidin-4-yl)piperidin-1-yl)methyl)isoindoline-1,3-dione;
5-((4-(6,7-dihydro-5H-cyclopenta[4,5]thieno[2,3-d]pyrimidin-4-yl)piperidin-1-yl)methyl)-2-(2,6-dioxopiperidin-3-yl)isoindoline-1,3-dione;
2-(2,6-dioxopiperidin-3-yl)-5-((4-(2-methyl-5-phenylthieno[2,3-d]pyrimidin-4-yl)piperidin-1-yl)methyl)isoindoline-1,3-dione;
2-(2,6-dioxopiperidin-3-yl)-5-((4-(2-methyl-5,6,7,8-tetrahydrobenzo[4,5]thieno[2,3-d]pyrimidin-4-yl)piperidin-1-yl)methyl)isoindoline-1,3-dione;
2-(2,6-dioxopiperidin-3-yl)-5-((4-(2-methyl-6,7-dihydro-5H-cyclopenta[4,5]thieno[2,3-d]pyrimidin-4-yl)piperidin-1-yl)methyl)isoindoline-1,3-dione;
2-(2,6-dioxopiperidin-3-yl)-5-((4-(2-methylthieno[2,3-d]pyrimidin-4-yl)piperidin-1-yl)methyl)isoindoline-1,3-dione;
2-(2,6-dioxopiperidin-3-yl)-5-((4-(thieno[2,3-d]pyrimidin-2-yl)piperidin-1-yl)methyl)isoindoline-1,3-dione;
2-(2,6-dioxopiperidin-3-yl)-5-((4-(5-ethylpyrimidin-2-yl)piperidin-1-yl)methyl)isoindoline-1,3-dione;
2-(2,6-dioxopiperldin-3-yl)-5-((4-(thieno[2,3-d]pyrimidin-4-yl)-3,6-dihydropyridin-1(2H)-yl)methyl)isoindoline-1,3-dione;
2-(2,6-dioxopiperidin-3-yl)-5-((4-(5-methylthieno[2,3-d]pyrimidin-4-yl)-3,6-dihydropyridin-1(2H)-yl)methyl)isoindoline-1,3-dione;
2-(2,6-dioxopiperidin-3-yl)-5-((4-(6-methylthieno[2,3-d]pyrimidin-4-yl)-3,6-dihydropyridin-1(2H)-yl)methyl)isoindoline-1,3-dione;
2-(2,6-dioxopiperidin-3-yl)-5-((4-(6-isopropylthieno[2,3-d]pyrimidin-4-yl)-3,6-dihydropyridin-1(2H)-yl)methyl)isoindoline-1,3-dione;
5-((4-(5,6-dimethylthieno[2,3-d]pyrimidin-4-yl)-3,6-dihydropyridin-1(2H)-yl)methyl)-2-(2,6-dioxopiperidin-3-yl)isoindoline-1,3-dione;
2-(2,6-dioxopiperidin-3-yl)-5-((4-(6-phenylthieno[2,3-d]pyrimidin-4-yl)-3,6-dihydropyridin-1(2H)-yl)methyl)isoindoline-1,3-dione;
2-(2,6-dioxopiperidin-3-yl)-5-((4-(5-phenylthieno[2,3-d]pyrimidin-4-yl)-3,6-dihydropyridin-1(2H)-yl)methyl)isoindoline-1,3-dione;
2-(2,6-dioxopiperidin-3-yl)-5-((4-(5,6,7,8-tetrahydrobenzo[4,5]thieno[2,3-d]pyrimidin-4-yl)-3,6-dihydropyridin-1(2H)-yl)methyl)isoindoline-1,3-dione;
5-((4-(6,7-dihydro-5H-cyclopenta[4,5]thieno[2,3-d]pyrimidin-4-yl)-3,6-dihydropyridin-1(2H)-yl)methyl)-2-(2,6-dioxopiperidin-3-yl)isoindoline-1,3-dione;
2-(2,6-dioxopiperidin-3-yl)-5-((4-(2-methyl-5-phenylthieno[2,3-d]pyrimidin-4-yl)-3,6-dihydropyridin-1(2H)-yl)methyl)isoindoline-1,3-dione;
2-(2,6-dioxopiperidin-3-yl)-5-((4-(2-methyl-5,6,7,8-tetrahydrobenzo[4,5]thieno[2,3-d]pyrimidin-4-yl)-3,6-dihydropyridin-1(2H)-yl)methyl)isoindoline-1,3-dione;
2-(2,6-dioxopiperidin-3-yl)-5-((4-(2-methyl-6,7-dihydro-5H-cyclopenta[4,5]thieno[2,3-d]pyrimidin-4-yl)-3,6-dihydropyridin-1(2H)-yl)methyl)isoindoline-1,3-dione;
2-(2,6-dioxopiperidin-3-yl)-5-((4-(2-methylthieno[2,3-d]pyrimidin-4-yl)-3,6-dihydropyridin-1(2H)-yl)methyl)isoindoline-1,3-dione;
2-(2,6-dioxopiperidin-3-yl)-5-((4-(thieno[2,3-d]pyrimidin-2-yl)-3,6-dihydropyridin-1(2H)-yl)methyl)isoindoline-1,3-dione;
5-((4-(2-chloro-6,7-dihydrothieno[3,2-d]pyrimidin-4-yl)piperazin-1-yl)methyl)-2-(2,6-dioxopiperidin-3-yl)isoindoline-1,3-dione;
2-(2,6-dioxopiperidin-3-yl)-5-((4-(2-morpholino-6,7-dihydrothieno[3,2-d]pyrimidin-4-yl)piperazin-1-yl)methyl)isoindoline-1,3-dione;
2-(2,6-dioxopiperidin-3-yl)-5-((4-(4-(piperazin-1-yl)-6,7-dihydrothieno[3,2-d]pyrimidin-2-yl)piperazin-1-yl)methyl)isoindoline-1,3-dione;
2-(2,6-dioxopiperidin-3-yl)-5-((4-(4-morpholino-6,7-dihydrothieno[3,2-d]pyrimidin-2-yl)piperazin-1-yl)methyl)isoindoline-1,3-dione;
2-(2,6-dioxopiperidin-3-yl)-5-((4-(2-(piperidin-1-yl)-6,7-dihydrothieno[3,2-d]pyrimidin-4-yl)piperazin-1-yl)methyl)isoindoline-1,3-dione;
2-(2,6-dioxopiperidin-3-yl)-5-((4-(2-(pyrrolidin-1-yl)-6,7-dihydrothieno[3,2-d]pyrimidin-4-yl)piperazin-1-yl)methyl)isoindoline-1,3-dione;
5-((4-(2-(azetidin-1-yl)-6,7-dihydrothieno[3,2-d]pyrimidin-4-yl)piperazin-1-yl)methyl)-2-(2,6-dioxopiperidin-3-yl)isoindoline-1,3-dione;
2-(2,6-dioxopiperidin-3-yl)-5-((4-(thieno[3,2-d]pyrimidin-4-yl)piperazin-1-yl)methyl)isoindoline-1,3-dione;
5-((4-(2-chlorothieno[3,2-d]pyrimidin-4-yl)piperazin-1-yl)methyl)-2-(2,6-dioxopiperidin-3-yl)isoindoline-1,3-dione;
2-(2,6-dioxopiperidin-3-yl)-5-((4-(2-morpholinothieno[3,2-d]pyrimidin-4-yl)piperazin-1-yl)methyl)isoindoline-1,3-dione;
2-(2,6-dioxopiperidin-3-yl)-5-((4-(4-(piperazin-1-yl)thieno[3,2-d]pyrimidin-2-yl)piperazin-1-yl)methyl)isoindoline-1,3-dione;
2-(2,6-dioxopiperidin-3-yl)-5-((4-(4-morpholinothieno[3,2-d]pyrimidin-2-yl)piperazin-1-yl)methyl)isoindoline-1,3-dione;
5-((4-(benzo[b]thiophen-4-yl)piperazin-1-yl)methyl)-2-(2,6-dioxopiperidin-3-yl)isoindoline-1,3-dione;
2-(2,6-dioxopiperidin-3-yl)-5-((3-(trifluoromethyl)-5,6-dihydro-[1,2,4]triazolo[4,3-a]pyrazin-7(8H)-yl)methyl)isoindoline-1,3-dione;
5-((4-(2-((2,4-dimethylphenyl)thio)phenyl)piperazin-1-yl)methyl)-2-(2,6-dioxopiperidin-3-yl)isoindoline-1,3-dione;
5-((6,7-dihydrothieno[3,2-c]pyridin-5(4H)-yl)methyl)-2-(2,6-dioxopiperidin-3-yl)isoindoline-1,3-dione;
5-((4-(benzo[d]isothiazol-3-yl)piperazin-1-yl)methyl)-2-(2,6-dioxopiperidin-3-yl)isoindoline-1,3-dione;
5-((4-(4-((3r,5r,7r)-adamantan-1-yl)benzyl)piperazin-1-yl)methyl)-2-(2,6-dioxopiperidin-3-yl)isoindoline-1,3-dione;
5-((((3s,5s,7s)-adamantan-1-yl)amino)methyl)-2-(2,6-dioxopiperidin-3-yl)isoindoline-1,3-dione;
1-((1R,4R)-7,7-dimethyl-2-oxobicyclo[2.2.1]heptan-1-yl)-N-((2-(2,6-dioxopiperidin-3-yl)-1,3-dioxoisoindolin-5-yl)methyl)methanesulfonamide;
5-((((1R,4S)-bicyclo[2.2.1]heptan-2-yl)oxy)methyl)-2-(2,6-dioxopiperidin-3-yl)isoindoline-1,3-dione;
3-(4-((4-(3-methylthiophen-2-yl)-3,6-dihydropyridin-1(2H)-yl)methyl)-1-oxoisoindolin-2-yl)piperidine-2,6-dione;
3-(4-fluoro-5-((4-(3-methylthiophen-2-yl)-3,6-dihydropyridin-1(2H)-yl)methyl)-1-oxoisoindolin-2-yl)piperidine-2,6-dione;
3-(7-fluoro-5-((4-(3-methylthiophen-2-yl)-3,6-dihydropyridin-1(2H)-yl)methyl)- 1 -oxoisoindolin-2-yl)piperidine-2,6-dione;
2-(2,6-dioxopiperidin-3-yl)-4-((4-(3-methylthiophen-2-yl)-3,6-dihydropyridin-1(2H)-yl)methyl)isoindoline-1,3-dione;
3-(4-((4-(2,3-dichlorophenyl)piperazin-1-yl)methyl)-1-oxoisoindolin-2-yl)piperidine-2,6-dione;
3-(5-((4-(2,3-dichlorophenyl)piperazin-1-yl)methyl)-4-fluoro-1-oxoisoindolin-2-yl)piperidine-2,6-dione;
3-(5-((4-(2,3-dichlorophenyl)piperazin-1-yl)methyl)-6-fluoro-1-oxoisoindolin-2-yl)piperidine-2,6-dione;
3-(5-((4-(2,3-dichlorophenyl)piperazin-1-yl)methyl)-7-fluoro-1-oxoisoindolin-2-yl)piperidine-2,6-dione;
3-(4-((4-(6-fluorobenzo[d]isoxazol-3-yl)piperidin-1-yl)methyl)-1-oxoisoindolin-2-yl)piperidine-2,6-dione;
3-(6-((4-(6-fluorobenzo[d]isoxazol-3-yl)piperidin-1-yl)methyl)-1-oxoisoindolin-2-yl)piperidine-2,6-dione;
3-(4-fluoro-5-((4-(6-fluorobenzo[d]isoxazol-3-yl)piperidin-1-yl)methyl)-1-oxoisoindolin-2-yl)piperidine-2,6-dione;
3-(6-fluoro-5-((4-(6-fluorobenzo[d]isoxazol-3-yl)piperidin-1-yl)methyl)-1-oxoisoindolin-2-yl)piperidine-2,6-dione;
3-(7-fluoro-5-((4-(6-fluorobenzo[d]isoxazol-3-yl)piperidin-1-yl)methyl)-1-oxoisoindolin-2-yl)piperidine-2,6-dione;
2-(2,6-dioxopiperidin-3-yl)-4-((4-(6-fluorobenzo[d]isoxazol-3-yl)piperidin-1-yl)methyl)isoindoline-1,3-dione;
3-(4-((4-(benzo[b]thiophen-4-yl)piperazin-1-yl)methyl)-1-oxoisoindolin-2-yl)piperidine-2,6-dione;

3-(1-oxo-4-((3-(trifluoromethyl)-5,6-dihydro-[1,2,4]triazolo[4,3-a]pyrazin-7(8H)-yl)methyl)isoindolin-2-yl)piperidine-2,6-dione;
3-(5-((4-(5,6-dichloropyridazin-4-yl)piperazin-1-yl)methyl)-1-oxoisoindolin-2-yl)piperidine-2,6-dione;
2-(2,6-dioxopiperidin-3-yl)-5-((4-(7-fluoroquinolin-4-yl)-3,6-dihydropyridin-1(2H)-yl)methyl)isoindoline-1,3-dione; and
5-((4-(5,6-dichloropyridazin-4-yl)piperazin-1-yl)methyl)-2-(2,6-dioxopiperidin-3-yl)isoindoline-1,3-dione.

**16.** The compound of Formula (I) or salts, enantiomers, stereoisomers, solvates, isotopically enriched analogs, prodrugs, or polymorphs thereof as claimed in any one of claims 1-15, which is a salt of the compound of Formula (I) selected from: hydrohalides (including hydrochloride and hydrobromide), sulfate, citrate, maleate, sulfonate, methanesulfonate, ethanesulfonate, edisylate, formate, acetate, 2,2-dichloroacetate, trimethylacetate, propionate, valerate, citrate, lactate, lactobionate, L-tartrate, fumarate, L-malate, L-lactate, α-ketoglutarate, hippurate, D-glucuronate, D-gluconate, α-D-glucoheptonate, glycolate, mucate, L-ascorbate, orotate, picrate, glycinate, alaninate, arginate, cinnamate, laurate, pamoate, sebacate, besylate, palmitate, triphenylacetate, 2-ethyl-butanedioate, iodate, nicotinate, L-pyroglutamate, L-prolinate, ferulate, 2-hydroxyethanesulfonate, undecenoate, camphorate, camphorsulfonate, dodecylsulfonate, phosphate, thiocyanate, dihydrophosphate, pyrophosphate, metaphosphate, oxalate, malonate, carbonate, malonate, benzoate, mandelate, succinate, trifluoroacetate, pyruvate, *p*-chlorobenzenesulfonate, 1,5-naphthalenedisulfonate, 3-hydroxy-2-naphthoate, 1-hydroxy-2-naphthoate, 2-naphthalenesulfonate, glycolate, and tosylate.

**17.** A pharmaceutical composition, comprising the compound of Formula (I) or pharmaceutically acceptable salts thereof as claimed in any one of claims 1-16, and at least one pharmaceutically acceptable carrier.

**18.** The pharmaceutical composition as claimed in claim 17, further comprising at least one additional therapeutic agent.

**19.** The pharmaceutical composition as claimed in claim 18, further comprising at least one second therapeutic agent, e.g., a therapeutic agent for treating tumors or cancers.

**20.** A kit or reagent kit, comprising the compound of Formula (I) or pharmaceutically acceptable salts thereof as claimed in any one of claims 1 to 16, or the pharmaceutical composition as claimed in claim 17.

**21.** Use of the compound of Formula (I) or pharmaceutically acceptable salts thereof as claimed in any one of claims 1-16, or of the pharmaceutical composition as claimed in any one of claims 17-19, for the manufacture of a medicament for the prevention or treatment of a disease or disorder selected from the group consisting of: tumors, cancers, infectious diseases, inflammatory diseases, autoimmune diseases, anemia, hemorrhagic shock, transplant rejection, multiple organ dysfunction syndrome (MODS), sarcoidosis, adult respiratory distress syndrome, cardiovascular diseases, Richter syndrome (RS), acute liver failure, and diabetes.

**22.** A method for treating or preventing a disease or disorder in a subject, comprising administering to the subject a therapeutically effective amount of the compound of Formula (I) or pharmaceutically acceptable salts thereof as claimed in any one of claims 1-16, or the pharmaceutical composition as claimed in claim 17, wherein the disease or disorder is selected from the group consisting of: tumors, cancers, infectious diseases, inflammatory diseases, autoimmune diseases, anemia, hemorrhagic shock, transplant rejection, multiple organ dysfunction syndrome (MODS), sarcoidosis, adult respiratory distress syndrome, cardiovascular diseases, Richter syndrome (RS), acute liver failure, and diabetes.

**23.** The use as claimed in claim 21, or the method as claimed in claim 22, wherein the disease or disorder is selected from the group consisting of: myeloma, including multiple myeloma, plasma cell myeloma, smoldering myeloma, smoldering multiple myeloma; myelofibrosis; bone marrow disease; myelodysplastic syndrome (MDS); previously treated myelodysplastic syndrome; transplantation-related cancer; neutropenia; leukemia, including acute myeloid leukemia (AML), chronic myeloid leukemia (CML), chronic myelogenous leukemia, acute lymphoblastic leukemia (ALL), chronic lymphocytic leukemia (CLL), B-cell chronic lymphocytic leukemia, leukemia-associated anemia, monocytic leukemia, myelomonocytic leukemia, T-lymphocytic leukemia, acute T-lymphocytic leukemia; lymphoma, including diffuse large B-cell lymphoma, non-Hodgkin's lymphoma, Hodgkin's lymphoma, anaplastic lymphoma, anaplastic large cell lymphoma, immunoblastic T-Cell lymphoma, CD20 positive lymphoma, mantle cell lymphoma, follicular lymphoma (FL), Burkitt's lymphoma, marginal zone lymphoma (MZL), primary lymphoma, B-cell lymphoma, recurrent B-cell non-Hodgkin's lymphoma, recurrent diffuse large B-cell lymphoma, recurrent mediastinal (thymic) large B-cell lymphoma, primary mediastinal (thymic) large B-cell lymphoma, recurrent transformed non-Hodgkin's lymphoma,

refractory B-cell non-Hodgkin's lymphoma, refractory diffuse large B-cell lymphoma, refractory primary mediastinal (thymic) large B-cell lymphoma, refractory transformed non-Hodgkin's lymphoma; thyroid cancer; melanoma; lung cancer, including lung adenocarcinoma, lung squamous cell carcinoma, non-small cell lung cancer, and small cell lung cancer; inflammatory myofibroblastoma; colorectal cancer; intestinal cancer; brain glioma; astroblastoma; ovarian cancer; bronchial cancer; prostate cancer; breast cancer, including triple negative breast cancer, sporadic breast cancer, ductal carcinoma of the breast, and patients with Cowden syndrome; pancreatic cancer; central nervous system tumor; neuroblastoma; glioma; peripheral neuroepithelioma; extramedullary plasmacytoma; plasmacytoma; gastric cancer; gastrointestinal stromal tumors; esophageal cancer; colorectal adenocarcinoma; esophageal squamous cell carcinoma; liver cancer; renal cell carcinoma; bladder cancer; endometrial cancer; metrocarcinoma; head and neck cancer; brain cancer; oral cancer; sarcoma, including rhabdomyosarcoma, various lipogenic tumors, Ewing's sarcoma/primitive neuroectodermal tumors (Ewing/PNETs), and leiomyosarcoma; urothelial carcinoma; basal cell carcinoma; oral squamous cell carcinoma; cholangiocarcinoma; bone cancer; cervical cancer; skin cancer; Richter syndrome (RS); sepsis syndrome; autoimmune diseases, including rheumatoid arthritis, autoimmune encephalomyelitis, ankylosing spondylitis, psoriasis, systemic lupus erythematosus, multiple sclerosis, recurrent oral ulcers, Kawasaki disease, polymyositis/dermatomyositis, Sjogren's syndrome, and atopic dermatitis; keratoconjunctivitis; inflammatory diseases, including Crohn's disease and ulcerative colitis, pneumonia, osteoarthritis, synovitis, systemic inflammatory response syndrome, airway inflammation, bronchitis; cerebral malaria; infectious diseases, including viral pneumonia, Acquired immunodeficiency syndrome (AIDS), COVID-19 novel coronavirus infection, gram-negative bacteria infection, gram-positive bacteria infection, tuberculosis, etc; septic shock; tuberculosis; bacterial meningitis; chronic obstructive pulmonary disease; asthma; hemorrhagic shock; organ (including kidney, heart, lung) or tissue transplantation rejection; diabetes; sarcoidosis; adult respiratory distress syndrome; anemia; pediatric aplastic anemia; cardiovascular diseases (e.g., coronary heart disease, congestive heart failure, myocardial infarction, atherosclerosis); multiple organ dysfunction caused by cachexia and septic shock; and acute liver failure.

**24.** The method as claimed in claim 22, wherein the compound of Formula (I) or pharmaceutically acceptable salts thereof as claimed in any one of claims 1-16, or the pharmaceutical composition as claimed in any one of claims 17-19 is administered to the subject through at least one mode of administration selected from the group consisting of nasal administration, inhalation administration, topical administration, oral administration, oral mucosal administration, rectal administration, pleural cavity administration, peritoneal administration, vaginal administration, intramuscular administration, subcutaneous, transdermal, epidural, intrathecal, and intravenous administration.

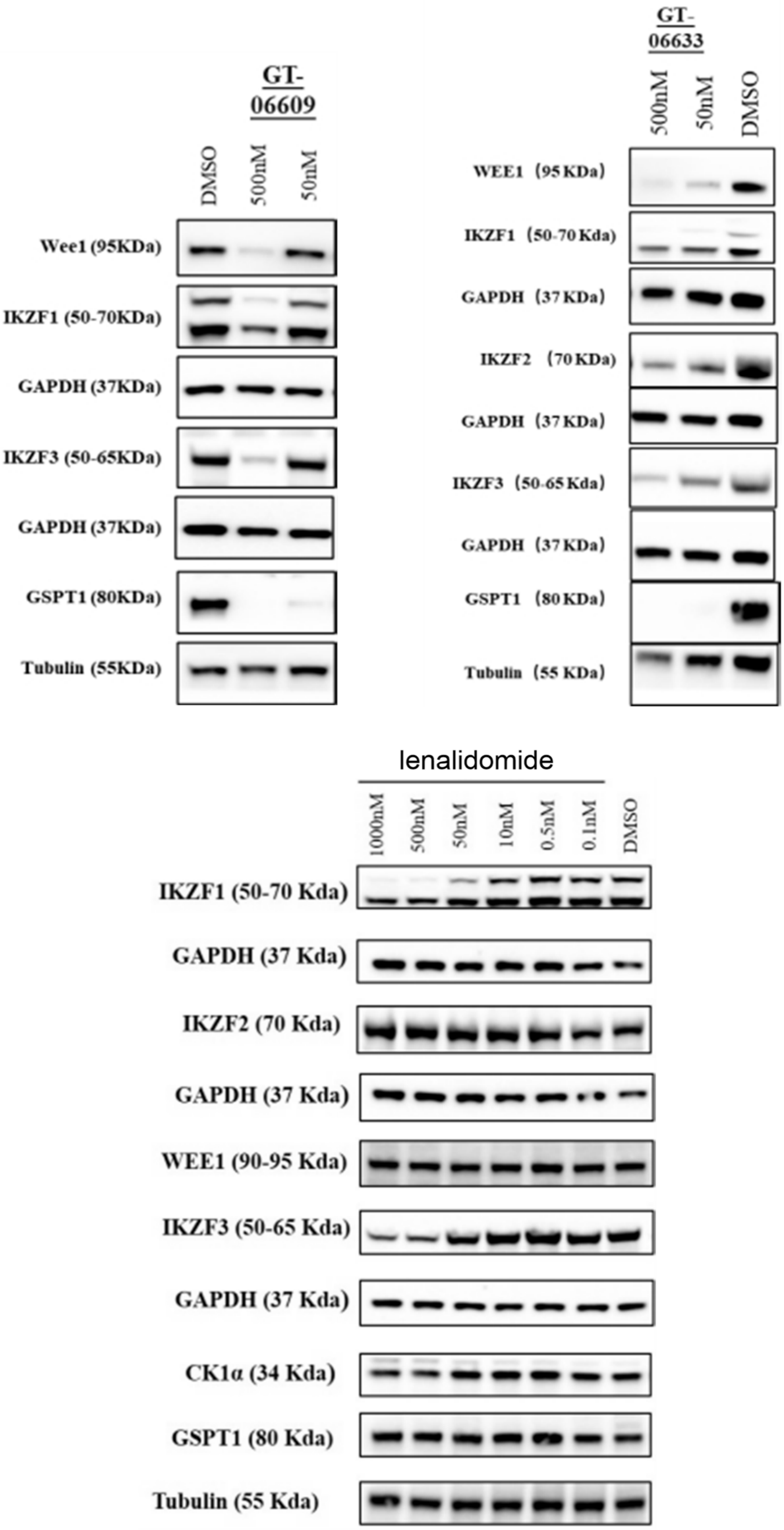
GT-06609
DMSO
500nM
50nM
Wee1 (95KDa)
IKZF1 (50-70KDa)
GAPDH (37KDa)
IKZF3 (50-65KDa)
GAPDH (37KDa)
GSPT1 (80KDa)
Tubulin (55KDa)
GT-06633
500nM
50nM
DMSO
WEE1 (95 KDa)
IKZF1 (50-70 Kda)
GAPDH (37 KDa)
IKZF2 (70 KDa)
GAPDH (37 KDa)
IKZF3 (50-65 Kda)
GAPDH (37 KDa)
GSPT1 (80 KDa)
Tubulin (55 KDa)
lenalidomide
1000nM
500nM
50nM
10nM
0.5nM
0.1nM
DMSO
IKZF1 (50-70 Kda)
GAPDH (37 Kda)
IKZF2 (70 Kda)
GAPDH (37 Kda)
WEE1 (90-95 Kda)
IKZF3 (50-65 Kda)
GAPDH (37 Kda)
CK1α (34 Kda)
GSPT1 (80 Kda)
Tubulin (55 Kda)

Figure 1

# INTERNATIONAL SEARCH REPORT

International application No.
**PCT/CN2024/105487**

**A. CLASSIFICATION OF SUBJECT MATTER**

C07D409/14(2006.01)i; C07D401/04(2006.01)i; C07D401/14(2006.01)i; C07D405/14(2006.01)i; C07D413/14(2006.01)i; C07D417/14(2006.01)i; A61K 31/4545(2006.01)i; A61K31/496(2006.01)i; A61K31/517(2006.01)i; A61P35/00(2006.01)i

According to International Patent Classification (IPC) or to both national classification and IPC

**B. FIELDS SEARCHED**

Minimum documentation searched (classification system followed by classification symbols)

IPC: C07D A61K A61P

Documentation searched other than minimum documentation to the extent that such documents are included in the fields searched

Electronic data base consulted during the international search (name of data base and, where practicable, search terms used)

CNTXT, CNKI, DWPI, WPABS, REGISTRY(STN), CAPLUS(STN): 戊二酰亚胺, 异吲哚啉酮, 泛素连接酶, 癌, 肿瘤, glutarimide, isoindolinone, cancer, ubiquitin ligase, cereblon, CRBN, tumor, tumour, 结构式检索, structural formula search

**C. DOCUMENTS CONSIDERED TO BE RELEVANT**

| Category* | Citation of document, with indication, where appropriate, of the relevant passages | Relevant to claim No. |
|---|---|---|
| X | CN 110963994 A (SHANGHAI INSTITUTE OF MATERIA MEDICA, CHINESE ACADEMY OF SCIENCES) 07 April 2020 (2020-04-07) abstract, and claims 1, 12 and 14-18 | 1-24 |
| X | CN 111285850 A (SHANGHAI INSTITUTE OF MATERIA MEDICA, CHINESE ACADEMY OF SCIENCES) 16 June 2020 (2020-06-16) abstract, and claims 1, 8 and 10-15 | 1-24 |
| X | CN 102264720 A (CELGENE CORP.) 30 November 2011 (2011-11-30) abstract, and claims 16-17 and 20-30 | 1-24 |
| X | WO 2023025091 A1 (BIOFRONT LTD. (CAYMAN) et al.) 02 March 2023 (2023-03-02) abstract, and claims 1 and 5-11 | 1-24 |
| X | WO 2023015283 A1 (CELGENE CORPORATION) 09 February 2023 (2023-02-09) claims 94-96 | 1-24 |

☑ Further documents are listed in the continuation of Box C. ☑ See patent family annex.

* Special categories of cited documents:
- "A" document defining the general state of the art which is not considered to be of particular relevance
- "D" document cited by the applicant in the international application
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **09 October 2024** | **15 October 2024** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | Telephone No. |

Form PCT/ISA/210 (second sheet) (July 2022)

**INTERNATIONAL SEARCH REPORT**

International application No.
**PCT/CN2024/105487**

**C. DOCUMENTS CONSIDERED TO BE RELEVANT**

| Category* | Citation of document, with indication, where appropriate, of the relevant passages | Relevant to claim No. |
|---|---|---|
| X | ACS. "RN 2915679-98-8 etc." *STN Registry,* 24 March 2023 (2023-03-24), pp. 1-10<br>pages 1-10, structure formulae | 1-14 |
| PX | WO 2024032689 A1 (GLUETACS THERAPEUTICS (SHANGHAI) CO., LTD.) 15 February 2024 (2024-02-15)<br>claims 1 and 22-34 | 1-24 |
| PX | WO 2024140637 A1 (GLUETACS THERAPEUTICS (SHANGHAI) CO., LTD.) 04 July 2024 (2024-07-04)<br>claims 1 and 9-19 | 1-24 |

Form PCT/ISA/210 (second sheet) (July 2022)

**INTERNATIONAL SEARCH REPORT**

International application No.
**PCT/CN2024/105487**

**Box No. II Observations where certain claims were found unsearchable (Continuation of item 2 of first sheet)**

This international search report has not been established in respect of certain claims under Article 17(2)(a) for the following reasons:

1. ☑ Claims Nos.: **22-24**
because they relate to subject matter not required to be searched by this Authority, namely:
The method of claims 22-24 is a method for treatment of a disease; however, the present search report is provided on the basis of the corresponding pharmaceutical use.

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

Form PCT/ISA/210 (continuation of first sheet) (July 2022)

**INTERNATIONAL SEARCH REPORT**
**Information on patent family members**

International application No.
**PCT/CN2024/105487**

| Patent document cited in search report | Publication date (day/month/year) | Patent family member(s) | Publication date (day/month/year) |
|---|---|---|---|
| CN 110963994 A | 07 April 2020 | BR 112021006458 A2 | 06 July 2021 |
| | | KR 20210069085 A | 10 June 2021 |
| | | KR 102672549 B1 | 10 June 2024 |
| | | CA 3121667 A1 | 02 April 2020 |
| | | AU 2019348094 A1 | 27 May 2021 |
| | | AU 2019348094 B2 | 22 December 2022 |
| | | WO 2020064002 A1 | 02 April 2020 |
| | | EP 3862348 A1 | 11 August 2021 |
| | | EP 3862348 A4 | 22 June 2022 |
| | | US 2022041576 A1 | 10 February 2022 |
| | | JP 2022503942 A | 12 January 2022 |
| | | JP 7168773 B2 | 09 November 2022 |
| CN 111285850 A | 16 June 2020 | CA 3122317 A1 | 11 June 2020 |
| | | CA 3122317 C | 03 October 2023 |
| | | AU 2019392231 A1 | 29 July 2021 |
| | | AU 2019392231 B2 | 20 October 2022 |
| | | WO 2020114482 A1 | 11 June 2020 |
| | | EP 3896062 A1 | 20 October 2021 |
| | | EP 3896062 A4 | 15 June 2022 |
| | | US 2022041578 A1 | 10 February 2022 |
| CN 102264720 A | 30 November 2011 | TW 201022227 A | 16 June 2010 |
| | | TWI 469974 B | 21 January 2015 |
| | | US 2016115161 A1 | 28 April 2016 |
| | | US 9550766 B2 | 24 January 2017 |
| | | IL 234164 A | 31 July 2016 |
| | | AU 2009311477 A1 | 14 May 2010 |
| | | AU 2009311477 B2 | 30 October 2014 |
| | | KR 20170007531 A | 18 January 2017 |
| | | ES 2662407 T3 | 06 April 2018 |
| | | MX 361467 B | 06 December 2018 |
| | | WO 2010053732 A1 | 14 May 2010 |
| | | BRPI 0919942 A2 | 23 December 2014 |
| | | BRPI 0919942 B1 | 19 February 2019 |
| | | EP 2985281 A2 | 17 February 2016 |
| | | EP 2985281 A3 | 30 March 2016 |
| | | EP 2985281 B1 | 27 December 2017 |
| | | US 2012252843 A1 | 04 October 2012 |
| | | US 8318773 B2 | 27 November 2012 |
| | | IL 234167 A | 28 September 2017 |
| | | CA 2957226 A1 | 14 May 2010 |
| | | CA 2957226 C | 14 August 2018 |
| | | CA 2741299 A1 | 14 May 2010 |
| | | CA 2741299 C | 28 March 2017 |
| | | RU 2011121567 A | 10 December 2012 |
| | | RU 2527952 C2 | 10 September 2014 |
| | | HK 1154859 A1 | 04 May 2012 |
| | | JP 2015227343 A | 17 December 2015 |
| | | JP 6034924 B2 | 30 November 2016 |
| | | SMT 201600025 B | 25 February 2016 |
| | | IL 212435 A0 | 30 June 2011 |

Form PCT/ISA/210 (patent family annex) (July 2022)

**INTERNATIONAL SEARCH REPORT**
**Information on patent family members**

International application No.
**PCT/CN2024/105487**

| Patent document cited in search report | Publication date (day/month/year) | Patent family member(s) | | | Publication date (day/month/year) |
|---|---|---|---|---|---|
| | | IL | 212435 | A | 30 June 2015 |
| | | IL | 234162 | A | 30 June 2016 |
| | | US | 2017088538 | A1 | 30 March 2017 |
| | | US | 9796698 | B2 | 24 October 2017 |
| | | UA | 107783 | C2 | 25 February 2015 |
| | | NI | 201100082 | A | 26 October 2011 |
| | | AR | 106942 | A2 | 07 March 2018 |
| | | SI | 2358697 | T1 | 29 February 2016 |
| | | PE | 20110547 | A1 | 04 August 2011 |
| | | JP | 2014224119 | A | 04 December 2014 |
| | | JP | 5998179 | B2 | 28 September 2016 |
| | | IL | 234165 | A | 31 August 2017 |
| | | MX | 2011004470 | A | 31 May 2011 |
| | | US | 2013045976 | A1 | 21 February 2013 |
| | | US | 9227954 | B2 | 05 January 2016 |
| | | IL | 228597 | A0 | 31 December 2013 |
| | | IL | 228597 | A | 31 January 2017 |
| | | KR | 20180000750 | A | 03 January 2018 |
| | | CO | 6361916 | A2 | 20 January 2012 |
| | | AR | 106945 | A2 | 07 March 2018 |
| | | ES | 2559388 | T3 | 11 February 2016 |
| | | US | 2012122865 | A1 | 17 May 2012 |
| | | US | 8222249 | B2 | 17 July 2012 |
| | | EP | 2358697 | A1 | 24 August 2011 |
| | | EP | 2358697 | B1 | 21 October 2015 |
| | | AR | 106963 | A2 | 07 March 2018 |
| | | IL | 234168 | A | 31 July 2016 |
| | | CL | 2011000957 | A1 | 16 September 2011 |
| | | IL | 234163 | A | 30 June 2016 |
| | | HUE | 026105 | T2 | 30 May 2016 |
| | | DK | 2358697 | T3 | 25 January 2016 |
| | | PL | 2358697 | T3 | 29 April 2016 |
| | | SG | 10201703508 | QA | 29 June 2017 |
| | | IL | 234166 | B | 30 April 2018 |
| | | SG | 195613 | A1 | 30 December 2013 |
| | | HRP | 20151366 | T1 | 15 January 2016 |
| | | PT | 2358697 | E | 03 February 2016 |
| | | AR | 106943 | A2 | 07 March 2018 |
| | | ZA | 201102941 | B | 25 July 2012 |
| | | EP | 3354646 | A1 | 01 August 2018 |
| | | KR | 20110089162 | A | 04 August 2011 |
| | | KR | 101696938 | B1 | 16 January 2017 |
| | | PE | 20140963 | A1 | 06 August 2014 |
| | | AR | 106944 | A2 | 07 March 2018 |
| | | NZ | 592425 | A | 26 April 2013 |
| | | AR | 074001 | A1 | 15 December 2010 |
| | | JP | 2012507541 | A | 29 March 2012 |
| | | JP | 5603876 | B2 | 08 October 2014 |
| | | US | 2010204227 | A1 | 12 August 2010 |
| | | US | 8129375 | B2 | 06 March 2012 |

Form PCT/ISA/210 (patent family annex) (July 2022)

**INTERNATIONAL SEARCH REPORT**
**Information on patent family members**

International application No.
**PCT/CN2024/105487**

| Patent document cited in search report | Publication date (day/month/year) | Patent family member(s) | Publication date (day/month/year) |
|---|---|---|---|
| WO 2023025091 A1 | 02 March 2023 | WO 2023023941 A1 | 02 March 2023 |
| | | CA 3226670 A1 | 02 March 2023 |
| | | JP 2024531439 A | 29 August 2024 |
| | | KR 20240052001 A | 22 April 2024 |
| | | EP 4392420 A1 | 03 July 2024 |
| | | AU 2022334511 A1 | 01 February 2024 |
| WO 2023015283 A1 | 09 February 2023 | JP 2024528988 A | 01 August 2024 |
| | | AU 2022324621 A1 | 18 January 2024 |
| | | CL 2024000305 A1 | 14 June 2024 |
| | | KR 20240043780 A | 03 April 2024 |
| | | MX 2024000901 A | 06 February 2024 |
| | | IL 309653 A | 01 February 2024 |
| | | EP 4380582 A1 | 12 June 2024 |
| | | CO 2024001133 A2 | 07 March 2024 |
| | | US 2023095912 A1 | 30 March 2023 |
| | | AR 126718 A1 | 08 November 2023 |
| | | TW 202321278 A | 01 June 2023 |
| | | CA 3223636 A1 | 09 February 2023 |
| WO 2024032689 A1 | 15 February 2024 | None | |
| WO 2024140637 A1 | 04 July 2024 | None | |

Form PCT/ISA/210 (patent family annex) (July 2022)